(19) Europäisches Patentamt
European Patent Office
Office européen des brevets

(11) EP 2 252 591 B1

(12) EUROPÄISCHE PATENTSCHRIFT

(45) Veröffentlichungstag und Bekanntmachung des Hinweises auf die Patenterteilung:
**22.06.2011 Patentblatt 2011/25**

(21) Anmeldenummer: **09707966.9**

(22) Anmeldetag: **30.01.2009**

(51) Int Cl.:
***C07D 233/72** (2006.01)* ***A61K 31/4166** (2006.01)*
***A61P 25/32** (2006.01)* ***A61P 25/34** (2006.01)*
***A61P 3/04** (2006.01)* ***A61P 3/10** (2006.01)*

(86) Internationale Anmeldenummer:
**PCT/EP2009/000592**

(87) Internationale Veröffentlichungsnummer:
**WO 2009/097999 (13.08.2009 Gazette 2009/33)**

# (54) ARYL-SUBSTITUIERTE IMIDAZOLIDIN-2,4-DIONE, VERFAHREN ZU IHRER HERSTELLUNG, DIESE VERBINDUNGEN ENTHALTENDE ARZNEIMITTEL UND IHRE VERWENDUNG

ARYL-SUBSTITUTED IMIDAZOLIDINE-2,4-DIONES, METHOD FOR THE PRODUCTION THEREOF, MEDICAMENTS CONTAINING SAID COMPOUNDS AND USE THEREOF

IMIDAZOLIDINE-2,4-DIONES SUBSTITUÉS PAR UN ARYLE, PROCÉDÉ DE PRODUCTION, MÉDICAMENTS CONTENANT CES COMPOSÉS ET LEUR UTILISATION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **07.02.2008 EP 08290130**

(43) Veröffentlichungstag der Anmeldung:
**24.11.2010 Patentblatt 2010/47**

(73) Patentinhaber: **Sanofi-Aventis**
**75013 Paris (FR)**

(72) Erfinder:
- **JAEHNE, Gerhard**
  **65926 Frankfurt am Main (DE)**
- **STENGELIN, Siegfried**
  **65926 Frankfurt am Main (DE)**
- **GOSSEL, Matthias**
  **65926 Frankfurt am Main (DE)**
- **KLABUNDE, Thomas**
  **65926 Frankfurt am Main (DE)**
- **WINKLER, Irvin**
  **65926 Frankfurt am Main (DE)**

(74) Vertreter: **Fischer, Hans-Jürgen et al**
**Sanofi-Aventis Deutschland GmbH**
**Patent- und Lizenzabteilung**
**Industriepark Höchst**
**Gebaüde K 801**
**65926 Frankfurt am Main (DE)**

(56) Entgegenhaltungen:
**EP-A1- 0 761 221** **WO-A-2004/101529**

EP 2 252 591 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft Imidazolidin-2,4-dione, die mit einem Aralkylrest substituiert sind sowie ihre physiologisch verträglichen Salze.

Es sind bereits strukturähnliche Imidazolin-2,4-dione beschrieben (US 5,411,981). Strukturähnliche stickstoffhaltige Heterocyclusderivate sind auch in US 2007/0010529 beschrieben, diese Verbindungen eignen sich zur Behandlung von entzündlichen Erkankungen.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung des metabolischen Syndroms, des Diabetes Typ II und der Adipositas geeignet sind.

Die Erfindung betrifft daher Verbindungen der Formel I,

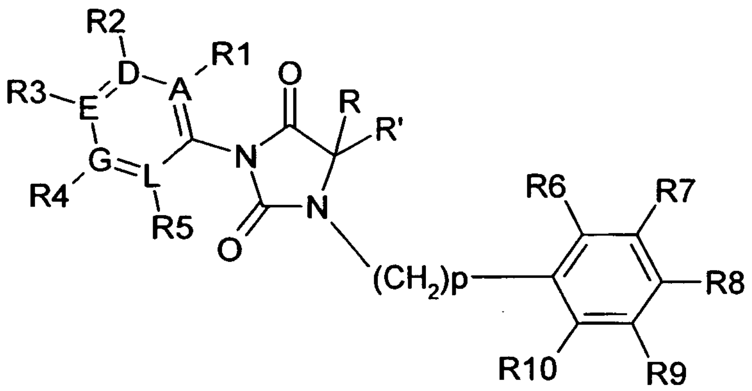

I

worin bedeuten

| | |
|---|---|
| R, R' | unabhängig voneinander H, $(CH_2)_n$-Aryl, $(C_1$-$C_6)$-Alkyl, wobei $(C_1$-$C_6)$-Alkyl oder der Arylrest substituiert sein kann mit Halogen, O-R14, $S(O)_m$-R12 oder NR13R15; |
| oder R und R' | bilden gemeinsam einen Ring mit drei bis acht Kohlenstoffatomen, wobei ein Kohlenstoffatom durch O, $S(O)_m$, NR13 oder NR15 ersetzt sein kann; |
| m | 0, 1, 2; |
| n | 0, 1, 2, 3, 4; |
| p | 1, 2, 3, 4, 5; |
| q | 1, 2, 3, 4; |
| r | 2, 3, 4, 5, 6; |
| v | 0, 1, 2, 3, 4; |
| A, D, E, G, L | unabhängig voneinander C oder N, wobei bei der Bedeutung N der entsprechende Substituent R1, R2, R3, R4, R5 entfällt, oder R2-D=E-R3 oder R4-G=L-R5 haben die Bedeutung S oder O und wobei der Fünf- oder Sechsring mit $-(CH_2)_3-$ oder $-(CH_2)_4-$ oder -CH=CH-CH=CH- zu einem Bicyclus anelliert sein kann; |
| R1, R2, R3, R4, R5 | unabhängig voneinander H, F, Cl, Br, J, CN, $N_3$, NC, $NO_2$, $CF_3$, $(C_1$-$C_8)$- Alkyl, $(C_3$-$C_8)$-Cycloalkyl, $(CH_2)_q$-[$(C_3$-$C_8)$-Cycloalkyl], $(CH_2)_n$-[$(C_3$-$C_8)$- Cycloalkenyl], $(CH_2)_n$-[$(C_7$-$C_{12})$-Bicycloal- |

kyl], $(CH_2)_n$-[($C_7$-$C_{12}$)-Tricycloalkyl], Adamantan-1-yl, Adamantan-2-yl, $(CH_2)_n$-Aryl, $(CH_2)_n$-Heteroaryl, $OCF_3$, O- R11, NR13R15, NH-CN, $S(O)_m$-R12, $SO_2$-$NH_2$, $SO_2$-N=CH-N$(CH_3)_2$, $SO_2$-NH- [($C_1$-$C_8$)-Alkyl], $SO_2$-NH-[($C_3$-$C_8$)-Cycloalkyl], $SO_2$-NH-$(CH_2)_n$-Aryl, $SO_2$-NH- $(CH_2)_n$-Heteroaryl, $SO_2$-N[($C_1$-$C_8$)-Alkyl]$_2$, $SO_2$-R16, $SF_5$, CO-O[($C_1$-$C_8$)- Alkyl], CO-O[($C_3$-$C_8$)-Cycloalkyl], CO-O-$(CH_2)_n$-Aryl, CO-O-$(CH_2)_n$-Heteroaryl, CO- $NH_2$, CO-NH-CN, CO-NH-[($C_1$-$C_8$)-Alkyl], CO-N[($C_1$-$C_8$)-Alkyl]$_2$, CO-NH- [($C_3$-$C_8$)-Cycloalkyl], CO-N[($C_3$-$C_8$)-Cycloalkyl]$_2$, C(=NH)-O-[($C_1$-$C_6$-Alkyl)], C(=NH)-$NH_2$, C(=NH)-R16, C(=NR13)-NR12R13, $(CH_2)_n$-C(=$NSO_2$-R12)$NH_2$, CO-R16, COOH, CO-($C_1$-$C_8$)-Alkyl, CO-($C_3$-$C_8$)-Cycloalkyl, CO-Aryl, CO- Heteroaryl, CH(OH)-Aryl, CH(OH)-Heteroaryl, CH[O-($C_1$-$C_6$)-Alkyl]-Aryl, CH[O-($C_1$-$C_6$)-Alkyl]-Heteroaryl, CHF-Aryl, CHF-Heteroaryl, $CF_2$-Aryl, $CF_2$- Heteroaryl, CHO, $CH_2$-OH, $CH_2$-CN, $CH_2$-O-R12, $CH_2$-O-$(CH_2)_n$-CO-O[($C_1$- $C_8$)-Alky!], $CH_2$-O-$(CH_2)_n$-CO-$NH_2$, $CH_2$-O-$(CH_2)_q$-COOH, wobei die Alkyl-, Cycloalkyl-, Cycloalkenyl-, Bicycloalkyl- und Tricycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, $OCF_3$, OH, O-$(CH_2)_n$-Aryl, $(CH_2)_n$-Aryl, $S(O)_m$-($C_1$-$C_6$)-Alkyl, $SO_2$-$NH_2$, SH, NR12R13, NH-CO-[($C_1$-$C_6$)-Alkyl], NH-CO-$(CH_2)_n$-Aryl, $(CH_2)_n$-COOH, $(CH_2)_n$-$CONH_2$, $(CH_2)_n$-CO-O($C_1$-$C_6$)-Alkyl, $(CH_2)_n$-CO-($C_1$- $C_6$)-Alkyl substituiert sein können und wobei die Alkylreste mit Fluoratomen substituiert sein können;

R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder 6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -($C_1$-$C_6$)-Alkyl substituiert sein kann und wobei der bicyclische Heterocyclus 9 bis 12 Ringglieder enthalten kann und bis zu fünf CH- bzw. $CH_2$- Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit

R11, F, Cl, Br, J, CN, $N_3$, NC, $NO_2$, $CF_3$, $(CH_2)_n$-O-R11, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O($C_1$-$C_6$)-Alkyl]$_2$, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N$(R13)_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, $(CH_2)_n$-NR12-CO-N$(R12)_2$, $(CH_2)$n-NR12-CO-NHR11, $(CH_2)_n$-NH-C(=NH)-$NH_2$, $(CH_2)_n$-NH-C(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$ -CO-NH-[($C_1$-$C_8$)-Alkyl], $(CH_2)_n$-NH-$(CH_2)_n$ -CO-NH-$(CH_2)_r$-OH, $(CH_2)_n$-NH-$(CH_2)_n$ -CO-N[(C1-C8)-Alkyl]$_2$, $(CH_2)_n$-NH-$(CH_2)_n$ -CO-NH-[($C_3$-$C_8$)-Cycloalkyl], $(CH_2)_n$-NH-$(CH_2)_n$ -CO-N[($C_3$-$C_8$)-Cycloalkyl]$_2$, $(CH_2)_n$-NH-C$(CH_3)_2$-CO-O($C_1$-$C_8$)-Alkyl, $(CH_2)_n$-NH-C$(CH_3)_2$-CO-O($C_3$-$C_8$)-Cycloalkyl, $(CH_2)_n$-NH-C$(CH_3)_2$-CO-O-$(CH_2)_r$-$NH_2$, $(CH_2)_n$-NH-C$(CH_3)_2$-CO-O-$(CH_2)_n$-Aryl, $(CH_2)_n$-NH-C$(CH_3)_2$-CO-O-$(CH_2)_n$-Heteroaryl, $(CH_2)_n$-NH-C$(CH_3)_2$-CO-$NH_2$, $(CH_2)_n$-NH-C$(CH_3)_2$-CO-NH-[($C_1$-$C_8$)-Alkyl], $(CH_2)_n$-NH-C$(CH_3)_2$-CO-NH-$(CH_2)_r$-OH, $(CH_2)_n$-NH-C$(CH_3)_2$-CO-N[($C_1$-$C_8$)-Alkyl]$_2$, $(CH_2)_n$-NH-$(CH_3)_2$-CO-NH-[($C_3$-$C_8$)-Cycloalkyl], $(CH_2)_n$-NH-C$(CH_3)_2$-CO-N[($C_3$-$C_8$)-Cycloalkyl]$_2$, $(CH_2)_n$-NH-C$(CH_3)_2$-COOH, $S(O)_m$-R12, $SO_2$-R16, $SO_2$-N=CH-N$(CH_3)_2$,

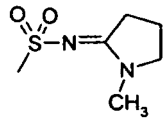,

$SO_2$-NH-CO-R12, $SO_2$-NHR12, $SO_2$-NH-$(CH_2)_r$-OH, $SO_2$-N[($C_1$-$C_8$)-Alkyl]$_2$, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH-piperidin-1-yl, $(CH_2)_n$-CO-NH-$SO_2$-NHR12, $(CH_2)_n$-CO-NH-$SO_2$-R18, $(CH_2)_n$-CHO, $(CH_2)_n$-C(=NH)$NH_2$, $(CH_2)_n$-C(=NH)-NHOH, $(CH_2)_n$-C(=NH)-(NH-O-($C_1$-$C_6$)-Alkyl], $(CH_2)_n$-C(=NH)(R16), $(CH_2)_n$-C(=NR13)NHR12, $(CH_2)_n$-C(=NR12)NR12R13, $(CH_2)_n$-C(=$NSO_2$-R12)$NH_2$, $(CH_2)_n$-C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, $(C_1-C_6)$-Alkyl, $(C_3-C_6)$-Cycloalkyl, O-$(C_1-C_6)$-Alkyl, $S(O)_m$-$(C_1-C_6)$-Alkyl, $SO_2$-$NH_2$, COOH, $CONH_2$, CO-O$(C_1-C_6)$-Alkyl, CO-$(C_1-C_6)$-Alkyl substituiert sein können und wobei die Alkylreste mit Fluoratomen substituiert sein können,

und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff verschiedenen Substituenten versehen ist;

H, F, Cl, Br, J, CN, $N_3$, NC, $NO_2$, $CF_3$,
$(C_1-C_8)$-Alkyl, $(C_2-C_{10})$-Alkenyl, $(C_2-C_{10})$-Alkinyl, $(C_3-C_8)$-Cycloalkyl, Aryl, Heteroaryl, $(CH_2)_n$-CO-[O-$(C_1-C_8)$-Alkyl], $(CH_2)_n$-CO-[O-$(C_3-C_8)$-Cycloalkyl], $(CH_2)_n$-CO-[$(C_1-C_8)$-Alkyl], $(CH_2)_n$-CO-[$(C_3-C_8)$-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-$(C_1-C_6)$-Alkyl], $(CH_2)_n$-P(O)[O-$(C_1-C_6)$-Alkyl]$_2$, $(CH_2)_n$-P(O)(OH)(O-$CH_2$-Aryl), $(CH_2)_n$-P(O)(O-$CH_2$-Aryl)$_2$, $(CH_2)_n$-P(O)$(OH)_2$, $(CH_2)_n$-$SO_3H$, $(CH_2)_n$-$SO_2$-$NH_2$, $(CH_2)_n$-CO-NH-[$(C_1-C_8)$-Alkyl], $(CH_2)_n$-CO-N[$(C_1-C_8)$-Alkyl]$_2$, $(CH_2)_n$-CO-NH-[$(C_3-C_8)$-Cycloalkyl],
$(C_2-C_{10})$-Alkenyl-CO-O[$(C_1-C_6)$-Alkyl], $(C_2-C_{10})$-Alkenyl-$CONH_2$, $(C_2-C_{10})$-Alkenyl-COOH, $(C_2-C_{10})$-Alkinyl-CO-O[$(C_1-C_6)$-Alkyl], $(C_2-C_{10})$-Alkinyl-$CONH_2$, $(C_2-C_{10})$-Alkinyl-COOH, $(CH_2)_n$-CO-R16,
$(CH_2)_n$-OH, $(CH_2)_n$-O-$(C_1-C_8)$-Alkyl, $(CH_2)_n$-O-$(C_2-C_{10})$-Alkenyl, $(CH_2)_n$-O-$(C_2-C_{10})$-Alkinyl, $(CH_2)_n$-O-$(C_3-C_8)$-Cycloalkyl, $(CH_2)_n$-O-$(CH_2)_q$-[$(C_3-C_8)$-Cycloalkyl], $(CH_2)_n$-O-$(CH_2)_n$-CO-[O-$(C_1-C_8)$-Alkyl], $(CH_2)_n$-O-$(CH_2)_n$-CO-[O-$(C_3-C_8)$-Cycloalkyl], $(CH_2)_n$-O-$(CH_2)_n$-CO-[$(C_1-C_8)$-Alkyl], $(CH_2)_n$-O-$(CH_2)_n$-CO-[$(C_3-C_8)$-Cycloalkyl], $(CH_2)_n$-O-$(CH_2)_n$-CO-[O-$(CH_2)_v$-Aryl], $(CH_2)_n$-O-$(CH_2)_n$-CO-[O-$(CH_2)_v$-Heteroaryl], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P(O)(OH)[O-$(C_1-C_6)$-Alkyl], $(CH_2)_n$-O-$(CH_2)_n$-P(O)[O-$(C_1-C_6)$-Alkyl]2, $(CH_2)_n$-O-$(CH_2)_n$-P(O)(OH)(O-$CH_2$-Aryl), $(CH_2)_n$-O-$(CH_2)_n$-P(O)(O-$CH_2$-Aryl)$_2$, $(CH_2)_n$-O-$(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$-O-$(CH_2)_n$-CO-NH-[$(C_1-C_8)$-Alkyl], $(CH_2)_n$-O-$(CH_2)_n$-CO-N[$(C_1-C_8)$-Alkyl]$_2$, $(CH_2)_n$-O-$(CH_2)_n$-CO-NH-[$(C_3-C_8)$-Cycloalkyl], $(CH_2)_n$-O-$(CH_2)_n$-CR21R22-CO-O[$(C_1-C_6)$-Alkyl], $(CH_2)_n$-O-$(CH_2)_n$-CR21R22-$CONH_2$, $(CH_2)_n$-O-$(CH_2)_n$-CR21R22-COOH, $(CH_2)_n$-O-$(CH_2)_n$-CO-R16, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$,
$(CH_2)_n$-$NH_2$, $(CH_2)_n$-NH-$(C_1-C_8)$-Alkyl, $(CH_2)_n$-NH-$(C_3-C_8)$-Cycloalkyl,
$(CH_2)_n$-NH-$(CH_2)_n$-CO-[O-$(C_3-C_8)$-Cycloalkyl], $(CH_2)_n$-NH-$(CH_2)_n$-CO-[$(C_1-C_8)$-Alkyl], $(CH_2)_n$-NH-$(CH_2)_n$-CO-[$(C_3-C_8)$-Cycloalkyl], $(CH_2)_n$-NH-$(CH_2)_n$-CO-[O-$(CH_2)_v$-Aryl], $(CH_2)_n$-NH-$(CH_2)_n$-CO-[O-$(CH_2)_n$-Heteroaryl], $(CH_2)_n$-NH-$(CH_2)_q$-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[$(C_1-C_6)$-Alkyl], $(CH_2)_n$-NH-$(CH_2)_n$-CR21 R22-$CONH_2$,
$(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[$(C_1-C_8)$-Alkyl], $(CH_2)_n$-NH- $(CH_2)_n$-$SO_2$-[$(C_3-C_8)$-Cycloalkyl], $(CH_2)_n$-NH-$SO_2$-$(CH_2)_n$-$NH_2$, $(CH_2)_n$-NH-$SO_2$-$(CH_2)_n$-NH-$(C_1-C_8)$-Alkyl, $(CH_2)_n$-NH-$SO_2$-$(CH_2)_n$-NH-$(C_3-C_8)$-Cycloalkyl, $(CH_2)_n$-NH-$SO_2$-$(CH_2)_n$-N[$(C_1-C_8)$-Alkyl]$_2$,
$(CH_2)_n$-NH-CN, $(CH_2)_n$-NH-$SO_2$-R16,
$(CH_2)_n$-NR12-CO-NH-$(C_1-C_8)$-Alkyl, $(CH_2)_n$-NR12-CO-NH-$(C_3-C_8)$-Cycloalkyl, $(CH_2)_n$-NR12-CO-$NH_2$, $(CH_2)_n$-NR12-CO-NH-$SO_2$-$(C_1-C_8)$-Alkyl, $(CH_2)_n$-NR12-CO-NH-$SO_2$-$(C_3-C_8)$-Cycloalkyl, $(CH_2)_n$-NR12-CO-N[$(C_1-C_8)$-Alkyl]$_2$, $(CH_2)_n$-NH-CO-NH-$(CH_2)_n$-CO-[O-$(C_1-C_8)$-Alkyl], $(CH_2)$n-NH-CO-NH-$(CH_2)_q$-CO-$NH_2$, $(CH_2)_n$-NH-CO-NH-$(CH_2)_q$-COOH, $(CH_2)_n$-NH-C(=NH)-$NH_2$, $(CH_2)_n$-NH-C(=NH)-R16, $(CH_2)_n$-NH-C(=NH)-NH[$(C_1-C_8)$-Alkyl], $(CH_2)_n$-NH-C(=N-$SO_2$-$(C_1-C_8)$-Alkyl)-$NH_2$, $(CH_2)_n$-NH-C(=N-$SO_2$-$(C_1-C_8)$-Alkyl)-NH[$(C_1-C_8)$-Alkyl], $(CH_2)_n$-NH-C(=N-$SO_2$-$NH_2$)-$NH_2$, $(CH_2)_n$-NH-C(=N-$SO_2$-$NH_2$)-NH[$(C_1-C_8)$-Alkyl], $(CH_2)_n$-NH-C(=NH)-N[$(C_1-C_8)$-Alkyl]$_2$, $(CH_2)_n$-NH-C(=N-$SO_2$-$(C_1-C_8)$-Alkyl)-N[$(C_1-C_8)$-Alkyl]$_2$, $(CH_2)_n$-NH-$(C_{H2})_n$-CO-O-$(CH_2)_r$-$NH_2$, $(CH_2)_n$-NH-$(CH_2)_n$ -CO-NH-[$(C_1-C_8)$-Alkyl], $(CH_2)_n$-NH-$(CH_2)_n$ -CO-NH-$(CH_2)_r$-OH, $(CH_2)_n$-NH-$(CH_2)_n$ -CO-N[$(C_1-C_8)$-Alkyl]$_2$, $(CH_2)_n$-NH-$(CH_2)_n$ -CO-NH-[($C_3$-

$C_8$)-Cycloalkyl], $(CH_2)_n$-NH-$C(CH_3)_2$-CO-O($C_3$-$C_8$)-Cycloalkyl, $(CH_2)_n$-NH-$C(CH_3)_2$-CO-O-$(CH_2)_r$-$NH_2$, $(CH_2)_n$-NH-$C(CH_3)_2$-CO-O-$(CH_2)_n$-Aryl, $(CH_2)_n$-NH-$C(CH_3)_2$-CO-O-$(CH_2)_n$-Heteroaryl, $(CH_2)_n$-NH-$C(CH_3)_2$-CO-NH-[($C_1$-$C_8$)-Alkyl], $(CH_2)_n$-NH-$C(CH_3)_2$-CO-NH-$(CH_2)_r$-OH, $(CH_2)_n$-NH-$C(CH_3)_2$-CO-N[($C_1$-$C_8$)-Alkyl]$_2$, $(CH_2)_n$-NH-$(CH_3)_2$-CO-NH-[($C_3$-$C_8$)-Cycloalkyl], $(CH_2)_n$-NH-$C(CH_3)_2$-CO-N[($C_3$-$C_8$)-Cycloalkyl]$_2$, $(CH_2)_n$-S(O)$_m$-($C_1$-$C_8$)-Alkyl, $(CH_2)_n$-S(O)$_m$-($C_3$-$C_8$)-Cycloalkyl, $(CH_2)_n$-$SO_2$-R16, $SO_2$-N=CH-N$(CH_3)_2$,

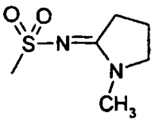
,

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)-Alkyl, $(CH_2)_n$-$SO_2$-NH-($C_3$-$C_8$)-Cycloalkyl, $(CH_2)_n$-$SO_2$-N[($C_1$-$C_8$)-Alkyl]$_2$, $SO_2$-NH-$(CH_2)_r$-OH, $SO_2$-NH-$(CH_2)_r$-$NH_2$, $SF_5$,
$(CH_2)_q$-CN,
$(CH_2)_n$-CO-NH-piperidin-1-yl,
$(CH_2)_n$-CO-NH-$SO_2$-NHR12, $(CH_2)_n$-CO-NH-$SO_2$-($C_1$-$C_8$)-Alkyl, (CH2)n-CO-NH-$SO_2$-($C_3$-$C_8$)-Cycloalkyl,
$(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, S(O)$_m$-($C_1$-$C_6$)-Alkyl, $SO_2$-$NH_2$, COOH, $CONH_2$, CO-[O($C_1$-$C_6$)-Alkyl], CO-($C_1$-$C_6$)-Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und
wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, ($C_1$-$C_8$)-Alkyl oder =O substituiert sein können;

T NR23-CO-NR24, NR23-$SO_2$-NR24, $SO_2$-NR23-$SO_2$, CO-NR23-CO, NR23- C(=NR13)-NR24, NR23-C(=NR22)-NR24, CO-NR23-CR22R23, CO- CR22R23-CO, CR22R23-CO-CR22R24, NR23-CO-CR22R24, NR23-$SO_2$- CR22R24, CR22R24-CO-NR23, CR22R24-$SO_2$-NR23, CR22R23-NR23-$SO_2$, $SO_2$-CR22R23-NR23, $SO_2$-NR23-CR22R23-, NR23-CR22R23-$SO_2$, CO-NR23- $SO_2$, $SO_2$-NR23-CO, CO-CR22R23-$SO_2$, $SO_2$-CR22R23-CO, CR23R24- CR23R24-CR23R24, CR23R24-NR23-CR23R24;

R11 H, ($C_1$-$C_8$)-Alkyl, ($C_2$-$C_{10}$)-Alkenyl, ($C_2$-$C_{10}$)-Alkinyl, ($C_3$-$C_8$)-Cycloalkyl, $(CH_2)_q$-[($C_3$-$C_8$)-Cycloalkyl], $(CH_2)_n$-Aryl, $(CH_2)_n$-CO-[O-($C_1$-$C_8$)-Alkyl], $(CH_2)_n$-CO-[O-($C_3$-$C_8$)-Cycloalkyl], $(CH_2)_n$-CO-[($C_1$-$C_8$)-Alkyl], $(CH_2)_n$-CO- [($C_3$-$C_8$)-Cycloalkyl], $(CH_2)_n$-CO-Aryl, $(CH_2)_n$-CO-Heteroaryl, $(CH_2)_q$-CO-$NH_2$, $(CH_2)_q$-COOH, $(CH_2)_n$P(O)(OH)[O-($C_1$-$C_6$)-Alkyl], $(CH_2)_n$-P(O)[O-($C_1$-$C_6$)- Alkyl]$_2$, $(CH_2)_n$-P(O)(OH)(O-$CH_2$-Aryl), $(CH_2)_n$-P(O)(O-$CH_2$-Aryl)$_2$, $(CH_2)_n$- P(O)(OH)$_2$, $(CH_2)_n$-$SO_3H$, $(CH_2)_n$-$SO_2$-$NH_2$, $(CH_2)_n$-CO-NH-[($C_1$-$C_8$)-Alkyl], $(CH_2)_n$-CO-N[($C_1$-$C_8$)-Alkyl]$_2$, $(CH_2)_n$-CO-NH-[($C_3$-$C_8$)-Cycloalkyl], ($C_2$-$C_{10}$)- Alkenyl-CO-O[($C_1$-$C_6$)-Alkyl], ($C_2$-$C_{10}$)-Alkenyl-$CONH_2$, ($C_2$-$C_{10}$)-Alkenyl- COOH, ($C_2$-$C_{10}$)-Alkinyl-CO-O[($C_1$-$C_6$)-Alkyl], ($C_2$-$C_{10}$)-Alkinyl-$CONH_2$, ($C_2$- $C_{10}$)-Alkinyl-COOH, $(CH_2)_n$-CR21[(CO-O($C_1$-$C_6$)-Alkyl)]$_2$, $(CH_2)_n$-CR21($CONH_2$)$_2$, $(CH_2)_n$-CR21(COOH)$_2$, $(CH_2)_n$-CR21R22-CO-O[($C_1$-$C_6$)- Alkyl], $(CH_2)_n$-CR21R22-$CONH_2$, $(CH_2)_n$-CR21R22-CO-NH-[($C_1$-$C_8$)-Alkyl], $(CH_2)_n$-CR21R22-CO-N[($C_1$-$C_8$)-Alkyl]$_2$, $(CH_2)_n$-CR21R22-COOH, $(CH_2)_n$-CO-R16, $(CH_2)_n$-CO-NH-$C(CH_3)_2$-CO-O[($C_1$-$C_8$)-Alkyl], $(CH_2)_n$-CO-NH-$C(CH_3)_2$-$CONH_2$, $(CH_2)_n$-CO-NH-$C(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, ($C_1$-$C_6$)-Alkyl, ($C_3$- $C_6$)-Cycloalkyl, O-($C_1$-$C_6$)-Alkyl, S(O)$_m$-($C_1$-$C_6$)-Alkyl, $SO_2$-$NH_2$, COOH, $CONH_2$, CO-O($C_1$-$C_6$)-Alkyl, CO-($C_1$-$C_6$)-Alkyl substituiert sein kann und wobei die Alkylreste mit Fluoratomen substituiert sein können;

R12 H, ($C_1$-$C_8$)-Alkyl, ($C_3$-$C_8$)-Cycloalkyl, $(CH_2)_n$-Aryl, $(CH_2)_n$-Heteroaryl, wobei die Alkyl- oder Cycloalkylreste

mit Fluoratomen substituiert sein können, und wobei der Aryl- oder Heteroarylrest mit Halogen, CN, $(C_1-C_6)$-Alkyl, O-$(C_1-C_6)$-Alkyl, $SO_2$-$NH_2$, COOH, $CONH_2$, CO-O$(C_1-C_6)$-Alkyl, CO-$(C_1-C_6)$- Alkyl substituiert sein kann und wobei die Alkylreste mit Fluoratomen substituiert sein können;

R13 H, $SO_2$-[$(C_1-C_8)$-Alkyl], $SO_2$-[$(C_3-C_8)$-Cycloalkyl], $SO_2$-$(CH_2)_n$-Aryl, $SO_2$-$(CH2)_n$-Heteroaryl, wobei die Alkyl- und Cycloalkylreste mit Fluoratomen substituiert sein können und wobei der Aryl- oder Heteroarylrest mit Halogen, CN, $CF_3$, $(C_1-C_6)$-Alkyl, $(C_3-C_6)$-Cycloalkyl, O-[$(C_1-C_6)$-Alkyl), $S(O)_m$-[$(C_1-C_6)$-Alkyl], $SO_2$-$NH_2$, COOH, $CONH_2$, CO-[O$(C_1-C_6)$-Alkyl], CO-$(C_1-C_6)$-Alkyl substituiert sein kann und wobei die Alkylreste mit Fluoratomen substituiert sein können;

R14 H, $(C_1-C_8)$-Alkyl, $(C_3-C_8)$-Cycloalkyl, $(CH_2)_n$-Aryl, $(CH_2)_n$-Heteroaryl, $(CH_2)_n$- CO-[O-$(C_1-C_8)$-Alkyl], $(CH_2)_n$-CO-[O-$(CH_2)_n$-AryL], $(CH_2)_N$-CO-[$(C_1-C_8)$-Alkyl], $(CH_2)_n$-CO-[$(C_3-C_8)$-Cycloalkyl], $(CH_2)_n$-CO-Aryl, $(CH_2)_n$-CO-Heteroaryl, $(CH_2)_q$-COOH,
wobei die Alkyl- und Cycloalkylreste mit Fluoratomen substituiert sein können und wobei der Aryl- oder Heteroarylrest mit Halogen, CN, $(C_1-C_6)$-Alkyl, $(C_3$- $C_6)$-Cycloalkyl, O-$(C_1-C_6)$-Alkyl, $S(O)_m$-$(C_1-C_6)$-Alkyl, $SO_2$-$NH_2$, COOH, $CONH_2$, CO-O$(C_1-C_6)$-Alkyl, CO-$(C_1-C_6)$-Alkyl substituiert sein kann und wobei die Alkylreste mit Fluoratomen substituiert sein können;

R15 H, $(C_1-C_8)$-Alkyl, $(C_3-C_8)$-Cycloalkyl, $(CH_2)_n$-Aryl, $(CH_2)_n$-Heteroaryl, $(CH_2)_n$-CO-[O-$(C_1-C_8)$-Alkyl], CO-[$(C_1-C_8)$-Alkyl], CO-[$(C_3-C_8)$-Cycloalkyl], CO-Aryl, CO-Heteroaryl, $(CH_2)_n$-CO-$NH_2$, $(CH_2)_q$-COOH, $(CH_2)_n$-$SO_2$-$NH_2$, $(CH_2)_n$-$C(CH_3)_2$-COOH,
wobei die Alkyl- und Cycloalkylreste mit Fluoratomen substituiert sein können und wobei der Aryl- oder Heteroarylrest mit Halogen, CN, $(C_1-C_6)$-Alkyl, O- $(C_1-C_6)$-Alkyl, $SO_2$-$NH_2$, COOH, $CONH_2$, CO-O$(C_1-C_6)$-Alkyl, CO-$(C_1-C_6)$- Alkyl substituiert sein kann und wobei die Alkylreste mit Fluoratomen substituiert sein können;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[$(C_1-C_6)$- Alkyl]piperazin-1-yl, Thiomorpholin-4-yl, Thiomorpholin-1,1-Dioxid-4-yl, NH- $(CH_2)_r$-OH, NH-$CH(CH_2OH)_2$, NH-$C(CH_2OH)_3$, N[$(C_1-C_6)$-Alkyl-OH]$_2$, N[$(C_1$- $C_6)$-Alkyl][$(C_1-C_6)$-Alkyl-OH], D-Glucamin-N-yl, N-Methyl-D-Glucamin-N-yl, NH-[$(C_1-C_8)$-Alkyl]-CO-O$(C_1-C_6)$-Alkyl, NH-[$(C_1-C_8)$-Alkyl]-COOH, NH-[$(C_1$- $C_8)$-Alkyl]-$CONH_2$, N[( $C_1-C_6)$-Alkyl][$(C_1$- $C_8)$-Alkyl]-CO-O$(C_1-C_6)$-Alkyl, N[( $C_1-C_6)$-Alkyl][$(C_1-C_8)$-Alkyl]-COOH, N[$(C_1-C_6)$-Alkyl][$(C_1-C_8)$-Alkyl]- $CONH_2$, NH-[C(H)(Aryl)]-CO-O$(C_1-C_6)$-Alkyl, NH-[C(H)(Aryl)]-COOH, NH- [C(H)(Aryl)]-$CONH_2$, N[$(C_1-C_6)$-Alkyl][C(H)(Aryl)]-CO-O$(C_1-C_6)$-Alkyl, N[$(C_1-C_6)$-Alkyl][C(H)(Aryl)]-COOH, N[$(C_1-C_6)$-Alkyl][C(H)(Aryl)]-$CONH_2$, NH-[C(H)(Heteroaryl)]-CO-O$(C_1-C_6)$-Alkyl, NH-[C(H)(Heteroaryl)]-COOH, NH-[C(H)(Heteroaryl)]-$CONH_2$, N[( $C_1-C_6)$-Alkyl][C(H)(Heteroaryl)]-CO- O$(C_1-C_6)$-Alkyl, N[$(C_1-C_6)$-Alkyl][C(H)(Heteroaryl)]-COOH, N[$(C_1-C_6)$- Alkyl][C(H)(Heteroaryl)]-$CONH_2$, N(($C_1-C_6)$-Alkyl][$(C_3-C_8)$-Cycloalkyl]-CO-O$(C_1-C_6)$-Alkyl, N[$(C_1-C_6)$-Alkyl][$(C_3-C_8)$-Cycloalkyl]-COOH, N[$(C_1-C_6)$- Alkyl][$(C_3-C_8)$-Cycloalkyl]-$CONH_2$, NH-[$(C_3-C_8)$-Cycloalkyl]-CO-O$(C_1-C_6)$- Alkyl, NH-[$(C_3-C_8)$-Cycloalkyl]-COOH, NH-[$(C_3-C_8)$-Cycloalkyl]-$CONH_2$, NH-$(C_1-C_8)$-Alkyl-OH, NH-[( $C_1-C_6)$-Alkyl]-$SO_2$-$(C_1-C_6)$-Alkyl, NH-[( $C_1-C_6)$- Alkyl]-$SO_3H$, NH-[( $C_1-C_6)$-Alkyl]-$SO_2$-$NH_2$, N[$(C_1-C_6)$-Alkyl]{[$(C_1-C_6)$-Alkyl]-$SO_3H$},
wobei die Alkohol (OH) - Funktionen durch F ersetzt sein können und wobei der Aryl- oder Heteroarylrest mit Halogen, CN, $(C_1-C_6)$-Alkyl, O-$(C_1-C_6)$-Alkyl, OH, $SO_2$-$NH_2$, COOH, $CONH_2$, CO-O$(C_1-C_6)$-Alkyl, CO-$(C_1-C_6)$-Alkyl substituiert sein kann;

R18 $(CH_2)_n$-CR25R26-CO-O$(C_1-C_6)$-Alkyl, $(CH_2)_n$-CR25R26-CO-$NH_2$, $(CH_2)_n$- CR25R26-COOH;

R20 H, $(C_1-C_6)$-Alkyl, $(C_3-C_8)$-Cycloalkyl, Aryl, [$(C_1-C_6)$-Alkyl]-Aryl;

R21 H, F, $CF_3$, $(C_1-C_6)$-Alkyl, $(C_3-C_8)$-Cycloalkyl, OH, O-$(C_1-C_6)$-Alkyl, O-$(C_3-C_8)$- Cycloalkyl, O-$(CH_2)_n$-Aryl, O-(CO)-$(C_1-C_6)$-Alkyl, O-(CO)-$(C_3-C_8)$-Cycloalkyl, O-(CO)-O-$(C_1-C_6)$-Alkyl, O-(CO)-O-$(C_3-C_8)$-Cycloalkyl, NH-[$(C_1-C_6)$-Alkyl]- Aryl, $NH_2$, NH-$(C_1-C_6)$-Alkyl, NH-(CO)-$(C_1-C_6)$-Alkyl;

R22 H, $CF_3$, $(C_1-C_6)$-Alkyl, Aryl, [$(C_1-C_6)$-Alkyl]-Aryl;

R23, R24 unabhängig voneinander H, $(C_1-C_6)$-Alkyl, $(C_3-C_8)$-Cycloalkyl, [$(C_1-C_6)$-Alkyl]- [$(C_3-C_8)$-Cycloalkyl], Aryl, [$(C_1-C_6)$-Alkyl]-Aryl
oder R23 und R24 bilden zusammen eine -CH=CH-, -$CH_2$-$CH_2$-, -$CH_2$-$CH_2$- $CH_2$-, oder

$-CH_2-CH_2-CH_2-CH_2-$ Einheit, worin eine $CH_2$-Gruppierung durch C=O, CHF oder $CF_2$ ersetzt sein kann, und worin bis zu vier Wasserstoffatome durch einen $(C_1-C_6)$-Alkylrest ersetzt sein können;

R25, R26 unabhängig voneinander H, F, $(C_1-C_6)$-Alkyl, Aryl, $[(C_1-C_6)$-Alkyl]-Aryl, wobei der Aryl mit Halogen, CN, OH, O-$(C_1-C_6)$-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N$[(C_1-C_6)$-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

Bevorzugt sind Verbindungen der Formel I, worin ein oder mehrere Reste die folgenden Bedeutungen haben:

R, R' unabhängig voneinander H, $(CH_2)_n$-Aryl, $(C_1-C_6)$-Alkyl, wobei $(C_1-C_6)$-Alkyl oder der Arylrest substituiert sein kann mit Halogen;

oder R und R' bilden gemeinsam einen Ring mit drei bis acht Kohlenstoffatomen, wobei ein Kohlenstoffatom durch O, $S(O)_m$, NR13 oder NR15 ersetzt sein kann;

m 0, 1, 2;

n 0, 1, 2, 3;

p 1,2,3,4;

q 1,2,3;

r 2, 3, 4, 5;

v 0, 1, 2, 3;

A, D, E, G, L unabhängig voneinander C oder N, wobei bei der Bedeutung N der entsprechende Substituent R1, R2, R3, R4, R5 entfällt,
oder R2-D=E-R3 oder R4-G=L-R5 haben die Bedeutung S oder O und wobei der Fünf- oder Sechsring mit $-(CH_2)_3-$ oder $-(CH_2)_4-$ oder -CH=CH-CH=CH- zu einem Bicyclus anelliert sein kann;

R1, R2, R3, R4, R5 unabhängig voneinander H, F, Cl, Br, J, CN, $CF_3$, $(C_1 -C_8)$-Alkyl, $(C_3- C_8)$-Cycloaikyl, $(CH_2)_q$-$[(C_3-C_8)$-Cycloalkyl], $(CH_2)_n$-$[(C_7-C_{12})$-Bicycloalkyl], $(CH_2)_n$-$[(C_7-C_{12})$-Tricycloalkyl], Adamantan-1-yl, Adamantan-2-yl, $(CH_2)_n$- Aryl, $(CH_2)_n$-Heteroaryl, $OCF_3$, O-R11, NR13R15, NH-CN, $S(O)_m$-R12, $SO_2$- $NH_2$, $SO_2$-N=CH-N$(CH_3)_2$, $SO_2$-NH-$[(C_1-C_8)$-Alkyl], $SO_2$-NH-$[(C_3-C_8)$- Cycloalkyl], $SO_2$-NH-$(CH_2)_n$-Aryl, $SO_2$-NH-$(CH_2)_n$-Heteroaryl, $SO_2$-N$[(C_1-C_8)$- Alkyl$]_2$, $SO_2$-R16, $SF_5$, CO-O$[(C_1-C_8)$-Alkyl], CO-O$[(C_3-C_8)$-Cycloalkyl], CO- O-$(CH_2)_n$-Aryl, CO-O-$(CH_2)_n$-Heteroaryl, CO-$NH_2$, CO-NH-CN, CO-NH-$[(C_1- C_8)$-Alkyl], CO-N$[(C_1-C_8)$-Alkyl$]_2$, CO-NH-$[(C_3-C_8)$-Cycloalkyl], C(=NH)-O- $[(C_1-C_6$-Alkyl)], C(=NH)-$NH_2$, C(=NH)-R16, C(=NR13)-NR12R13, $(CH_2)_n$- C(=$NSO_2$-R12)$NH_2$, CO-R16, COOH, CO-$(C_1-C_8)$-Alkyl, CO-$(C_3-C_8)$- Cycloalkyl, CO-Aryl, CO-Heteroaryl, CH(OH)-Aryl, CH(OH)-Heteroaryl, CH[O-$(C_1-C_6)$-Alkyl]-Aryl, CH[O-$(C_1-C_6)$-Alkyl]-Heteroaryl, CHF-Aryl, CHF- Heteroaryl, $CF_2$-Aryl, $CF_2$-Heteroaryl, CHO, $CH_2$-OH, $CH_2$-CN, $CH_2$-O-R12, $CH_2$-O-$(CH_2)_q$-COOH,
wobei die Alkyl-, Cycloalkyl-, Cycloalkenyl-, Bicycloalkyl- und Tricycloalkylreste mit Fluoratomen substituiert sein können und wobei die Aryl- oder Heteroarylreste mit Halogen, CN, $(C_1-C_6)$-Alkyl, $(C_3-C_6)$-Cycloalkyl, O-$(C_1-C_6)$-Alkyl, $OCF_3$, OH, O-$(CH_2)_n$-Aryl, $(CH_2)_n$-Aryl, $S(O)_m$-$(C_1-C_6)$-Alkyl, $SO_2$-$NH_2$, SH, NR12R13, NH-CO-$[(C_1-C_6)$-Alkyl], NH-CO-$(CH_2)_n$-Aryl, $(CH_2)_n$-COOH, $(CH_2)_n$-$CONH_2$, $(CH_2)_n$-CO-O$(C_1-C_6)$-Alkyl, $(CH_2)_n$-CO-$(C_1- C_6)$-Alkyl substituiert sein können und wobei die Alkylreste mit Fluoratomen substituiert sein können;

R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder

6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -($C_1$-$C_6$)-Alkyl substituiert sein kann und wobei der bicyclische Heterocyclus 9 bis 12 Ringglieder enthalten kann und bis zu fünf CH- bzw. $CH_2$-Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit

R11, F, Cl, Br, J, CN, $N_3$, NC, $NO_2$, $CF_3$, $(CH_2)_n$-O-R11, $(CH_2)_n$-O-$(CH_2)_r$-OH, $(CH_2)_n$-O-$CH(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O($C_1$-$C_6$)-Alkyl$]_2$, $(CH_2)_n$-N[$(CH_2)_q$-COOH$]_2$, $(CH_2)_n$-N[$(CH_2)_q$-$CONH_2]_2$, $(CH_2)_n$-NH-R13, $(CH_2)_n$-$N(R13)_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, $(CH_2)_n$-NR12-CO-$N(R12)_2$, $(CH_2)_n$-NR12-CO-NHR11, $(CH_2)_n$-NH-C(=NH)-$NH_2$, $(CH_2)_n$-NH-C(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_1)_n$-NH-$(CH_2)_n$ -CO-NH-[($C_1$-$C_8$)-Alkyl], $(CH_2)_n$-NH-$(CH_2)_n$ -CO-NH-$(CH_2)_r$-OH, $(CH_2)_n$-NH-$(CH_2)_n$ -CO-N[(C1-C8)-Alkyl$]_2$, $(CH_2)_n$-NH-$(CH_2)_n$ -CO-NH-[($C_3$-$C_8$)-Cycloalkyl], $(CH_2)_n$-NH-$(CH_2)_n$ -CO-N[($C_3$-$C_8$)-Cycloalkyl$]_2$, $(CH_2)_n$-NH-$C(CH_3)_2$-CO-O($C_1$-$C_8$)-Alkyl, $(CH_2)_n$-NH-$C(CH_3)_2$-CO-O($C_3$-$C_8$)-Cycloalkyl, $(CH_2)_n$-NH-$C(CH_3)_2$-CO-O-$(CH_2)_r$-$NH_2$, $(CH_2)_n$-NH-$C(CH_3)_2$-CO-O-$(CH_2)_n$-Aryl, $(CH_2)_n$-NH-$C(CH_3)_2$-CO-O-$(CH_2)_n$-Heteroaryl, $(CH_2)_n$-NH-$C(CH_3)_2$-CO-$NH_2$, $(CH_2)_n$-NH-$C(CH_3)_2$-CO-NH-[($C_1$-$C_8$)-Alkyl], $(CH_2)_n$-NH-$C(CH_3)_2$-CO-NH-$(CH_2)_r$-OH, $(CH_2)_n$-NH-$C(CH_3)_2$-CO-N[($C_1$-$C_8$)-Alkyl$]_2$, $(CH_2)_n$-NH-$(CH_3)_2$-CO-NH-[($C_3$-$C_8$)-Cycloalkyl], $(CH_2)_n$-NH-$C(CH_3)_2$-CO-N[($C_3$-$C_8$)-Cycloalkyl$]_2$, $(CH_2)_n$-NH-$C(CH_3)_2$-COOH, $S(O)_m$-R12, $SO_2$-R16, $SO_2$-N=CH-$N(CH_3)_2$,

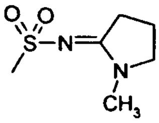
,

$SO_2$-NH-CO-R12, $SO_2$-NHR12, $SO_2$-NH-$(CH_2)_r$-OH, $SO_2$-N[($C_1$-$C_8$)-Alkyl$]_2$, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH-piperidin-l-yl, $(CH_2)_n$-CO-NH-$SO_2$-NHR12, $(CH_2)_n$-CO-NH-$SO_2$-R18, $(CH_2)_n$-CHO, $(CH_2)_n$-C(=NH)$NH_2$, $(CH_2)_n$-C(=NH)-NHOH, $(CH_2)_n$-C(=NH)-[NH-O-($C_1$-$C_6$)-Alkyl], $(CH_2)_n$-C(=NH)(R16), $(CH_2)_n$-C(=NR13)NHR12, $(CH_2)_n$-C(=NR12)NR12R13, $(CH_2)_n$-C(=$NSO_2$-R12)$NH_2$, $(CH_2)_n$-C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, $S(O)_m$-($C_1$-$C_6$)-Alkyl, $SO_2$-$NH_2$, COOH, $CONH_2$, CO-O($C_1$-$C_6$)-Alkyl, CO-($C_1$-$C_6$)-Alkyl substituiert sein können und wobei die Alkylreste mit Fluoratomen substituiert sein können,
und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff verschiedenen Substituenten versehen ist;

H, F, Cl, Br, J, CN, $N_3$, NC, $NO_2$ $CF_3$,
($C_1$-$C_8$)-Alkyl, ($C_2$-$C_{10}$)-Alkenyl, ($C_2$-$C_{10}$)-Alkinyl, ($C_3$-$C_8$)-Cycloalkyl, Aryl, Heteroaryl,
$(CH_2)_n$-CO-[O-($C_1$-$C_8$)-Alkyl], $(CH_2)_n$-CO-[O-($C_3$-$C_8$)-Cycloalkyl], $(CH_2)_n$-CO-[($C_1$-$C_8$)-Alkyl], $(CH_2)_n$-CO-[($C_3$-$C_8$)-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-($C_1$-$C_6$)-Alkyl], $(CH_2)_n$-P(O)[O-($C_1$-$C_6$)-Alkyl$]_2$, $(CH_2)_n$-P(O)(OH)(O-$CH_2$-Aryl), $(CH_2)_n$-P(O)(O-$CH_2$-Aryl$)_2$, $(CH_2)_n$-$P(O)(OH)_2$, $(CH_2)_n$-$SO_3H$, $(CH_2)_n$-$SO_2$-$NH_2$, $(CH_2)_n$-CO-NH-[($C_1$-$C_8$)-Alkyl], $(CH_2)_n$-CO-N[($C_1$-$C_8$)-Alkyl$]_2$, $(CH_2)_n$-CO-NH-[($C_3$-$C_8$)-Cycloalkyl],
($C_2$-$C_{10}$)-Alkenyl-CO-O[($C_1$-$C_6$)-Alkyl], ($C_2$-$C_{10}$)-Alkenyl-$CONH_2$, ($C_2$-$C_{10}$)-Alkenyl-COOH, ($C_2$-$C_{10}$)-Alkinyl-CO-O[($C_1$-$C_6$)-Alkyl], ($C_2$-$C_{10}$)-Alkinyl-$CONH_2$, ($C_2$-$C_{10}$)-Alkinyl-COOH,

$(CH_2)_n$-CO-R16,
$(CH_2)_n$-OH, $(CH_2)_n$-O-($C_1$-$C_8$)-Alkyl, $(CH_2)_n$-O-($C_2$-$C_{10}$)-Alkenyl, $(CH_2)_n$-O-($C_2$-$C_{10}$)-Alkinyl, $(CH_2)_n$-O-($C_3$-$C_8$)-Cycloalkyl, $(CH_2)_n$-O-$(CH_2)_q$-[($C_3$-$C_8$)-Cycloalkyl], $(CH_2)_n$-O-$(CH_2)_n$-CO-[O-($C_1$-$C_8$)-Alkyl], $(CH_2)_n$-O-$(CH_2)_n$-CO-[O-($C_3$-$C_8$)-Cycloalkyl], $(CH_2)_n$-O-$(CH_2)_n$-CO-[($C_1$-$C_8$)-Alkyl], $(CH_2)_n$-O-$(CH_2)_n$-CO-[($C_3$-$C_8$)-Cycloalkyl], $(CH_2)_n$-O-$(CH_2)_n$-CO-[O-$(CH_2)_v$-Aryl], $(CH_2)_n$-O-$(CH_2)_n$-CO-[O-$(CH_2)_v$-Heteroaryl], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P(O)(OH)[O-($C_1$-$C_6$)-Alkyl], $(CH_2)_n$-O-$(CH_2)_n$-P(O)[O-($C_1$-$C_6$)-Alkyl]$_2$, $(CH_2)_n$-O-$(CH_2)_n$-P(O)(OH)(O-$CH_2$-Aryl), $(CH_2)_n$-O-$(CH_2)_n$-P(O)(O-$CH_2$-Aryl)$_2$, $(CH_2)_n$-O-$(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$-O-$(CH_2)_n$-CO-NH-[($C_1$-$C_8$)-Alkyl], $(CH_2)_n$-O-$(CH_2)_n$-CO-N[($C_1$-$C_8$)-Alkyl]$_2$, $(CH_2)_n$-O-$(CH_2)_n$-CO-NH-[($C_3$-$C_8$)-Cycloalkyl], $(CH_2)_n$-O-$(CH_2)_n$-CR21R22-CO-O[($C_1$-$C_6$)-Alkyl], $(CH_2)_n$-O-$(CH_2)_n$-CR21R22-CONH2, $(CH_2)_n$-O-$(CH_2)_n$-CR21R22-COOH, $(CH_2)_n$-O-$(CH_2)_n$-CO-R16, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$,
$(CH_2)_n$-$NH_2$, $(CH_2)_n$-NH-($C_1$-$C_8$)-Alkyl, $(CH_2)_n$-NH-($C_3$-$C_8$)-Cycloalkyl,
$(CH_2)_n$-NH-$(CH_2)_n$-CO-[O-($C_3$-$C_8$)-Cycloalkyl], $(CH_2)_n$-NH-$(CH_2)_n$-CO-[($C_1$-$C_8$)-Alkyl], $(CH_2)_n$-NH-$(CH_2)_n$-CO-[($C_3$-$C_8$)-Cycloalkyl], $(CH_2)_n$-NH-$(C_{H2})_n$-CO-[O-$(CH_2)_v$-Aryl], $(CH_2)_n$-NH-$(CH_2)_n$-CO-[O-$(CH_2)_v$-Heteroaryl], $(CH_2)_n$-NH-$(CH_2)$q-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[($C_1$-$C_6$)-Alkyl], $(CH_2)_n$-NH-$(CH_2)_n$-CR21R22-$CONH_2$,
$(CH_2)_n$-NH-$(CH_2)_n$-CR2 R22-COOH,
$(CH_2)_n$-NH-$(CH_2)_n$-CO-R16,
$(CH_2)_n$-NH-$(CH_2)_n$-$SO_2$-[($C_1$-$C_8$)-Alkyl], $(CH_2)_n$-NH- $(CH_2)_n$-$SO_2$-[($C_3$-$C_8$)-Cycloalkyl], $(CH_2)_n$-NH-$SO_2$-$(CH_2)_n$-$NH_2$, $(CH_2)_n$-NH-$SO_2$-$(CH_2)_n$-NH-($C_1$-$C_8$)-Alkyl, $(CH_2)_n$-NH-$SO_2$-$(CH_2)_n$-NH-($C_3$-$C_8$)-Cycloalkyl, $(CH_2)_n$-NH-$SO_2$-$(CH_2)_n$-N[($C_1$-$C_8$)-Alkyl]$_2$,
$(CH_2)_n$-NH-CN, $(CH_2)_n$-NH-$SO_2$-R16,
$(CH_2)_n$-NR12-CO-NH-($C_1$-$C_8$)-Alkyl, $(CH_2)_n$-NR12-CO-NH-($C_3$-$C_8$)-Cycloalkyl, $(CH_2)_n$-NR12-CO-$NH_2$, $(CH_2)_n$-NR12-CO-NH-$SO_2$-($C_1$-$C_8$)-Alkyl, $(CH_2)_n$-NR12-CO-NH-$SO_2$-($C_3$-$C_8$)-Cycloalkyl, $(CH_2)_n$-NR 12-CO-N[($C_1$-$C_8$)-Alkyl]$_2$, $(CH_2)$n-NH-CO-NH-$(CH_2)_n$-CO-[O-($C_1$-$C_8$)-Alkyl], $(CH_2)$n-NH-CO-NH-$(CH_2)$q-CO-$NH_2$, $(CH_2)$n-NH-CO-NH-$(CH_2)_q$-COOH, $(CH_2)$n-NH-C(=NH)-$NH_2$, $(CH_2)_n$-NH-C(=NH)-R16, $(CH_2)_n$-NH-C(=NH)-NH[($C_1$-$C_8$)-Alkyl], $(CH_2)_n$-NH-C(=N-$SO_2$-($C_1$-$C_8$)-Alky1)-$NH_2$, $(CH_2)_n$-NH-C(=N-$SO_2$-($C_1$-$C_8$)-Alkyl)-NH[($C_1$-$C_8$)-Alkyl], $(CH_2)_n$-NH-C(=N-$SO_2$-$NH_2$)-$NH_2$, $(CH_2)_n$-NH-C(=N-$SO_2$-$NH_2$)-NH[($C_1$-$C_8$)-Alkyl], $(CH_2)_n$-NH-C(=NH)-N[($C_1$-$C_8$)-Alkyl], $(CH_2)_n$-NH-C(=N-$SO_2$-($C_1$-$C_8$)-Alkyl)-N[($C_1$-$C_8$)-Alkyl]$_2$, $(CH_2)_n$-NH-$(CH_2)_n$-CO-O-$(CH_2)_r$$NH_2$, $(CH_2)_n$-NH-$(CH_2)_n$ -CO-NH-[($C_1$-$C_8$)-Alkyl], $(CH_2)_n$-NH-$(CH_2)_n$ -CO-NH-$(CH_2)_r$-OH, $(CH_2)_n$-NH-$(CH_2)_n$ -CO-N[($C_1$-$C_8$)-Alkyl]$_2$, $(CH_2)_n$-NH-$(CH_2)_n$ -CO-NH-[($C_3$-$C_8$)-Cycloalkyl], $(CH_2)_n$-NH-C$(CH_3)_2$-CO-O($C_3$-$C_8$)-Cycloalkyl, $(CH_2)_n$-NH-C$(CH_3)_2$-CO-O-$(CH_2)_r$-$NH_2$, $(CH_2)_n$-NH-C$(CH_3)_2$-CO-O-$(CH_2)_n$-Aryl, $(CH_2)_n$-NH-C$(CH_3)_2$-CO-O-$(CH_2)_n$-Heteroaryl, $(CH_2)_n$-NH-C$(CH_3)_2$-CO-NH-[($C_1$-$C_8$)-Alkyl], $(CH_2)_n$-NH-C$(CH_3)_2$-CO-NH-$(CH_2)_r$-OH, $(CH_2)_n$-NH-C$(CH_3)_2$-CO-N[($C_1$-$C_8$)-Alkyl]$_2$, $(CH_2)_n$-NH-$(CH_3)_2$-CO-NH-[($C_3$-$C_8$)-Cycloalkyl], $(CH_2)_n$-NH-C$(CH_3)_2$-CO-N[($C_3$-$C_8$)-Cycloalkyl]$_2$, $(CH_2)_n$-S$(O)_m$-($C_1$-$C_8$)-Alkyl, $(CH_2)_n$-S$(O)_m$-($C_3$-$C_8$)-Cycloalkyl, $(CH_2)_n$-$SO_2$-R16, $SO_2$-N=CH-N$(CH_3)_2$,

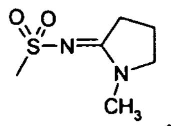,

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)-Alkyl, $(CH_2)_n$-$SO_2$-NH-($C_3$-$C_8$)-Cycloalkyl, $(CH_2)_n$-$SO_2$-N[($C_1$-$C_8$)-Alkyl]$_2$, $SO_2$-NH-$(CH_2)_r$-OH, $SO_2$-NH-$(CH_2)_r$-$NH_2$, SFs,
$(CH_2)_q$-CN,
$(CH_2)_n$-CO-NH-piperidin-1-yl,
$(CH_2)_n$-CO-NH-$SO_2$-NHR12, $(CH_2)_n$-CO-NH-$SO_2$-($C_1$-$C_8$)-Alkyl, $(CH_2)_n$-CO-NH-$SO_2$-($C_3$-$C_8$)-Cycloalkyl,

$(CH_2)_n$-CHO, $(CH_2)$n-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[$(C_1$-$C_6)$-Alkyl], wobei die Alkyl- und Cycloalkylreste mit Fluoratomen substituiert sein können und wobei die Aryl- oder Heteroarylreste mit Halogen, CN, $(C_1$-$C_6)$-Alkyl, $(C_3$-$C_6)$-Cycloalkyl, O-$(C_1$-$C_6)$-Alkyl, $S(O)_m$-$(C_1$-$C_6)$-Alkyl, $SO_2$-$NH_2$, COOH, $CONH_2$, CO-[O$(C_1$-$C_6)$-Alkyl], CO-$(C_1$-$C_6)$-Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und

wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, $(C_1$-$C_8)$-Alkyl oder =O substituiert sein können;

T NR23-CO-NR24, NR23-$SO_2$-NR24, $SO_2$-NR23-$SO_2$, CO-NR23-CO, NR23- C(=NR13)-NR24, NR23-C(=NR22)-NR24, CO-NR23-CR22R23, CO- CR22R23-CO, CR22R23-CO-CR22R24, NR23-CO-CR22R24, NR23-$SO_2$- CR22R24, CR22R24-CO-NR23, CR22R24-$SO_2$-NR23, CR22R23-NR23-$SO_2$, $SO_2$-CR22R23-NR23, $SO_2$-NR23-CR22R23-, NR23-CR22R23-$SO_2$, CO-NR23- $SO_2$, $SO_2$-NR23-CO, CO-CR22R23-$SO_2$, $SO_2$-CR22R23-CO, CR23R24- CR23R24-CR23R24, CR23R24-NR23-CR23R24;

R11 H, $(C_1$-$C_8)$-Alkyl, $(C_2$-$C_{10})$-Alkenyl, $(C_2$-$C_{10})$-Alkinyl, $(C_3$-$C_8)$-Cycloalkyl, $(CH_2)_q$-[$(C_3$-$C_8)$-Cycloalkyl], $(CH_2)_n$-Aryl, $(CH_2)_n$-CO-[O-$(C_1$-$C_8)$-Alkyl], $(CH_2)$,-CO-[O-$(C_3$-$C_8)$-Cycloalkyl], $(CH_2)_n$-CO-[$(C_1$-$C_8)$-Alkyl], $(CH_2)_n$-CO- [$(C_3$-$C_8)$-Cycloalkyl], $(CH_2)_n$-CO-Aryl, $(CH_2)_n$-CO-Heteroaryl, $(CH_2)_q$-CO-$NH_2$, $(CH_2)_q$-COOH, $(CH_2)_n$-P(O)(OH)[O-$(C_1$-$C_6)$-Alkyl], $(CH_2)_n$-P(O)[O-$(C_1$-$C_6)$- Alkyl$]_2$, $(CH_2)_n$-P(O)(OH)(O-$CH_2$-Aryl), $(CH_2)_n$-P(O)(O-$CH_2$-Aryl$)_2$, $(CH_2)_n$- $P(O)(OH)_2$, $(CH_2)_n$-$SO_3H$, $(CH_2)_n$-$SO_2$-$NH_2$, $(CH_2)_n$-CO-NH-[$(C_1$-$C_8)$-Alkyl], $(CH_2)_n$-CO-N[$(C_1$-$C_8)$-Alkyl$]_2$, $(CH_2)_n$-CO-NH-[$(C_3$-$C_8)$-Cycloalkyl], $(C_2$-$C_{10})$- Alkenyl-CO-O[$(C_1$-$C_6)$-Alkyl], $(C_2$-$C_{10})$-Alkenyl-$CONH_2$, $(C_2$-$C_{10})$-Alkenyl- COOH, $(C_2$-$C_{10})$-Alkinyl-CO-O[$(C_1$-$C_6)$-Alkyl], $(C_2$-$C_{10})$-Alkinyl-$CONH_2$, $(C_2$- $C_{10})$-Alkinyl-COOH, $(CH_2)_n$-CR21 [(CO-O$(C_1$-$C_6)$-Alkyl)$]_2$, $(CH_2)_n$- CR21 $(CONH_2)_2$, $(CH_1)_n$-CR21 $(COOH)_2$, $(CH_2)_n$-CR21 R22-CO-O[$(C_1$-$C_6)$- Alkyl], $(CH_2)_n$-CR21R22-$CONH_2$, $(CH_2)_n$-CR21R22-CO-NH-[$(C_1$-$C_8)$-Alkyl], $(CH_2)_n$-CR21 R22-CO-N[$(C_1$-$C_8)$-Alkyl], $(CH_2)_n$-CR21 R22-COOH, $(CH_2)_n$-CO-R16, $(CH_2)_n$CO-NH-C$(CH_3)_2$-CO-O[$(C_1$-$C_8)$-Alkyl], $(CH_2)_n$-CO-NH-C$(CH_3)_2$-$CONH_2$, $(CH_2)_n$-CO-NH-C$(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, $(C_1$-$C_6)$-Alkyl, $(C_3$- $C_6)$-Cycloalkyl, O-$(C_1$-$C_6)$-Alkyl, $S(O)_m$-$(C_1$-$C_6)$-Alkyl, $SO_2$-$NH_2$, COOH, $CONH_2$, CO-O$(C_1$-$C_6)$-Alkyl, CO-$(C_1$-$C_6)$-Alkyl substituiert sein kann und wobei die Alkylreste mit Fluoratomen substituiert sein können;

R12 H, $(C_1$-$C_8)$-Alkyl, $(C_3$-$C_8)$-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, $(C_1$-$C_6)$-Alkyl, 0-$(C_1$-$C_6)$-Alkyl, $SO_2NH_2$, COOH, $CONH_2$, CO-O$(C_1$-$C_6)$-Alkyl, CO-$(C_1$-$C_6)$- Alkyl substituiert sein kann und wobei die Alkylreste mit Fluoratomen substituiert sein können;

R13 H, $S0_2$-[$(C_1$-$C_8)$-Alkyl], $SO_2$-[$(C_3$-$C_8)$-Cycloalkyl], $SO_2$-$(CH_2)_n$-Aryl, $SO_2$-$(CH_2)_n$-Heteroaryl,
wobei die Alkyl- und Cycloalkylreste mit Fluoratomen substituiert sein können und wobei der Aryl- oder Heteroarylrest mit Halogen, CN, $CF_3$, $(C_1$-$C_6)$-Alkyl, $(C_3$-$C_6)$-Cycloalkyl, O-[$(C_1$-$C_6)$-Alkyl], $S(O)_m$-[$(C_1$-$C_6)$-Alkyl], $SO_2$-$NH_2$, COOH, $CONH_2$, CO-[O$(C_1$-$C_6)$-Alkyl], CO-$(C_1$-$C_6)$-Alkyl substituiert sein kann und wobei die Alkylreste mit Fluoratomen substituiert sein können;

R15 $(C_1$-$C_8)$-Alkyl,
wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[$(C_1$-$C_6)$- Alkyl]piperazin-1-yl, Thiomorpholin-4-yl, Thiomorpholin-1,1-Dioxid-4-yl, NH- $(CH_2)_r$-OH, NH-CH$(CH_2OH)_2$, NH-C$(CH_2OH)_3$, N[$(C_1$-$C_6)$-Alkyl-OH$]_2$, D- Glucamin-N-yl, N-Methyl-D-Glucamin-N-yl, NH-[$(C_1$-$C_8)$-Alkyl]-CO-O$(C_1$- C6)-Alkyl,NH-[$(C_1$-$C_8)$-Alkyl]-COOH, NH-[$(C_1$-$C_8)$-Alkyl]-$CONH_2$, N[$(C_1$- $C_6)$-Alkyl][$(C_1$-$C_8)$-Alkyl]-COOH, NH-[C(H)(Aryl)]-CO-O$(C_1$-$C_6)$-Alkyl, NH-[C(H)(Aryl)]-COOH, NH-[C(H)(Aryl)]-$CONH_2$, NH-[C(H)(Heteroaryl)]-CO- O$(C_1$-$C_6)$-Alkyl, NH-[C(H)(Heteroaryl)]-COOH, NH-[C(H)(Heteroaryl)]- $CONH_2$NH-[$(C_3$-$C_8)$-Cycloalkyl]-CO-O$(C_1$-$C_6)$-Alkyl, NH-[$(C_3$-$C_8)$-

Cycloalkyl]-COOH, NH-[($C_3$-$C_8$)-Cycloalkyl]-$CONH_2$, NH-($C_1$-$C_6$)-Alkyl-OH, NH-[($C_1$-$C_6$)-Alkyl]-$SO_2$-($C_1$-$C_6$)-Alkyl, NH-[($C_1$-$C_6$)-Alkyl]-$SO_3$H, NH-[($C_1$- $C_6$)-Alkyl]-$SO_2$-$NH_2$,
wobei die Alkohol (OH) - Funktionen durch F ersetzt sein können und wobei der Aryl- oder Heteroarylrest mit Halogen, CN, ($C_1$-$C_6$)-Alkyl, O-($C_1$-$C_6$)-Alkyl, OH, $SO_2$-$NH_2$, COOH, $CONH_2$, CO-O($C_1$-$C_6$)-Alkyl, CO-($C_1$-$C_6$)-Alkyl substituiert sein kann;

R18 $(CH_2)_n$-CR25R26-CO-O($C_1$-$C_4$)-Alkyl, $(CH_2)_n$-CR25R26-CO-$NH_2$, $(CH_2)_n$- CR25R26-COOH;

R20 H, ($C_1$-$C_6$)-Alkyl, ($C_3$-$C_8$)-Cycloalkyl, Aryl, [($C_1$-$C_6$)-Alkyl]-Aryl;

R21 H, F, $CF_3$, ($C_1$-$C_6$)-Alkyl, ($C_3$-$C_8$)-Cycloalkyl, OH, O-($C_1$-$C_6$)-Alkyl, O-($C_3$-$C_8$)- Cycloalkyl, O-$(CH_2)_n$-Aryl, O-(CO)-($C_1$-$C_6$)-Alkyl, O-(CO)-($C_3$-$C_8$)-Cycloalkyl, O-(CO)-O-($C_1$-$C_6$)-Alkyl, O-(CO)-O-($C_3$-$C_8$)-Cycloalkyl, NH-[($C_1$-$C_6$)-Alkyl]- Aryl, $NH_2$, NH-($C_1$-$C_6$)-Alkyl, NH-(CO)-($C_1$-$C_6$)-Alkyl;

R22 H, $CF_3$, ($C_1$-$C_6$)-Alkyl, Aryl, [($C_1$-$C_6$)-Alkyl]-Aryl;

R23, R24 unabhängig voneinander H, ($C_1$-$C_6$)-Alkyl, ($C_3$-$C_6$)-Cycloalkyl, [($C_1$-$C_4$)-Alkyl]- [($C_3$-$C_6$)-Cycloalkyl], Aryl, [($C_1$-$C_4$)-Alkyl]-Aryl
oder R23 und R24 bilden zusammen eine -CH=CH-, -$CH_2$-$CH_2$-, -$CH_2$-$CH_2$- $CH_2$-, oder -$CH_2$-$CH_2$-$CH_2$-$CH_2$- Einheit, worin eine $CH_2$-Gruppierung durch C=O, CHF oder $CF_2$ ersetzt sein kann, und worin bis zu vier Wasserstoffatome durch einen ($C_1$-$C_4$)-Alkylrest ersetzt sein können;

R25, R26 unabhängig voneinander H, F, ($C_1$-$C_4$)-Alkyl, Aryl, [($C_1$-$C_4$)-Alkyl]-Aryl, wobei der Aryl mit Halogen, CN, OH, O-($C_1$-$C_4$)-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[($C_1$-$C_4$)-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

Besonders bevorzugt sind Verbindungen der Formel I, worin ein oder mehrere Reste die folgenden Bedeutungen haben:

R, R' unabhängig voneinander H, $(CH_2)_n$-Aryl, ($C_1$-$C_6$)-Alkyl, wobei ($C_1$-$C_6$)-Alkyl oder der Arylrest substituiert sein kann mit Halogen;

oder R und R' bilden gemeinsam einen Ring mit drei bis acht Kohlenstoffatomen, wobei ein Kohlenstoffatom durch O, $S(O)_m$, NR13 oder NR15 ersetzt sein kann;

m 0, 1, 2;

n 0, 1, 2;

p 1, 2, 3;

q 1, 2;

r 2, 3, 4;

v 0, 1, 2;

A, D, E, G, L unabhängig voneinander C oder N, wobei bei der Bedeutung N der entsprechende Substituent R1, R2, R3, R4, R5 entfällt, oder R2-D=E-R3 oder R4-G=L-R5 haben die Bedeutung S oder O und wobei der Fünf- oder Sechsring mit -$CH_2)_3$. oder -CH=CH-CH=CH- oder zu einem Bicyclus anelliert sein kann;

R1, R2, R3, R4, R5 unabhängig voneinander H, F, Cl, Br, J, CN, $CF_3$, ($C_1$-$C_8$)-Alkyl, ($C_3$- $C_8$)-Cycloalkyl, Adamantan-1-yl, Adamantan-2-yl, $(CH_2)_n$-Aryl, $(CH_2)_n$- Heteroaryl, $OCF_3$, O-R11, NR13R15,

$S(O)_m$-R12, $SO_2NH_2$, $SO_2$-N=CH- $N(CH_3)_2$, $SO_2$-NH-[($C_1$-$C_8$)-Alkyl], $SO_2$-NH-[($C_3$-$C_8$)-Cycloalkyl], $SO_2$NH- $(CH_2)_n$-Aryl, $SO_2$-NH-$(CH_2)_n$-Heteroaryl, $SO_2$-N[($C_1$-$C_8$)-Alkyl]$_2$, $SO_2$-R16, $SF_5$, CO-O[($C_1$-$C_8$)-Alkyl], CO-O[($C_3$-$C_6$)-Cycloalkyl], CO-$NH_2$, CO-NH-[($C_1$-$C_4$)-Alkyl], CO-N[($C_1$-$C_4$)- Alkyl]$_2$, CO-NH-[($C_3$-$C_6$)-Cycloalkyl], C(=NH)-O-[($C_1$-$C_6$-Alkyl)], C(=NH)- $NH_2$, C(=NH)-R16, C(=NR13)-NR12R13, $(CH_2)_n$-C(=$NSO_2$-R12)$NH_2$, CO-R16, COOH, CO-($C_1$-$C_4$)-Alkyl, CO-($C_3$-$C_6$)-Cycloalkyl, CO-Aryl, CO-Heteroaryl, CH(OH)-Aryl, CH(OH)-Heteroaryl, CHF-Aryl, CHF-Heteroaryl, $CF_2$-Aryl, $CF_2$-Heteroaryl, $CH_2$-OH, $CH_2$-CN, $CH_2$-O-R12, $CH_2$-O-$(CH_2)_q$-COOH, wobei die Alkyl- und Cycloalkylreste mit Fluoratomen substituiert sein können und wobei die Aryl- oder Heteroarylreste mit Halogen, CN, ($C_1$-$C_4$)-Alkyl, ($C_3$- $C_6$)-Cycloalkyl, O-($C_1$-$C_4$)-Alkyl, $OCF_3$, OH, O-$(CH_2)_n$-Aryl, $(CH_2)_n$-Aryl, $S(O)_m$-($C_1$-$C_4$)-Alkyl, $SO_2$-$NH_2$, SH, NR12R13, NH-CO-[($C_1$-$C_4$)-Alkyl], NH- CO-$(CH_2)_n$-Aryl, $(CH_2)_n$-COOH, $(CH_2)_n$-$CONH_2$, $(CH_2)_n$-CO-O($C_1$-$C_4$)-Alkyl, $(CH_2)_n$-CO-($C_1$-$C_4$)-Alkyl substituiert sein können und wobei die Alkylreste mit Fluoratomen substituiert sein können;

R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder 6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -($C_1$-$C_6$)-Alkyl substituiert sein kann und wobei der bicyclische Heterocyclus 9 bis 12 Ringglieder enthalten kann und bis zu fünf CH- bzw. $CH_2$-Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit

R11, F, Cl, Br, J, CN, $N_3$, NC, $NO_2$, $CF_3$, $(CH_2)_n$-O-R11, $(CH_2)_n$-O-$(CH_2)_r$-OH, $(CH_2)_n$-O-CH($CH_2$OH)$_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O($C_1$-$C_6$)-Alkyl]2, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N(R13)$_2$, $(CH_2)_n$-NH-CN, $(CH_2)$,-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, $(CH_2)_n$-NR12-CO-N(R12)$_2$, (CH2)n-NR12-CO-NHR11, $(CH_2)_n$-NH-C(=NH)-$NH_2$, $(CH_2)_n$-NH-C(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_n$-$NH_2$, $(CH_2)_n$-NH-$(CH_2)_n$ -CO-NH-[($C_1$-$C_8$)-Alkyl], $(CH_2)_n$-NH-$(CH_2)_n$ -CO-NH-$(CH_2)_r$-OH, $(CH_2)_n$-NH-$(CH_2)_n$ -CO-N[($C_1$-$C_8$)-Alkyl]$_2$, $(CH_2)_n$-NH-$(CH_2)_n$ -CO-NH-[($C_3$-$C_8$)-Cycloalkyl], $(CH_2)_n$-NH-$(CH_2)$'' -CO-N[($C_3$-$C_8$)-Cycloalkyl]$_2$, $(CH_2)_n$-NH-C$(CH_3)_2$-CO-O($C_1$-$C_8$)-Alkyl, $(CH_2)_n$-NH-C$(CH_3)_2$-CO-O($C_3$-$C_8$)-Cycloalkyl, $(CH_2)_n$-NH-C$(CH_3)_2$-CO-O-$(CH_2)_r$-$NH_2$, $(CH_2)_n$-NH-C$(CH_3)_2$-CO-O-$(CH_2)_n$-$Aryl_3$ $(CH_2)_n$-NH-C$(CH_3)_2$-CO-O-$(CH_2)_n$-Heteroaryl, $(CH_2)_n$-NH-C$(CH_3)_2$-CO-$NH_2$, $(CH_2)_n$-NH-C$(CH_3)_2$-CO-NH-[($C_1$-$C_8$)-Alkyl], $(CH_2)_n$-NH-C$(CH_3)_2$-CO-NH-$(CH_2)_r$-OH, $(CH_2)_n$-NH-C$(CH_3)_2$-CO-N[($C_1$-$C_8$)-Alkyl]$_2$, $(CH_2)_n$-NH-$(CH_3)_2$-CO-NH-[($C_3$-$C_8$)-Cycloalkyl], $(CH_2)_n$-NH-C$(CH_3)_2$-CO-N[($C_3$-$C_8$)-Cycloalkyl]$_2$, $(CH_2)_n$-NH-C$(CH_3)_2$-COOH, $S(O)_m$ R12, $SO_2$-R16, $SO_2$-N=CH-N$(CH_3)_2$,

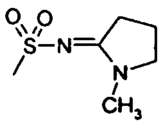

,

$SO_2$-NH-CO-R12, $SO_2$NHR12, $SO_2$-NH-$(CH_2)_r$-OH, $SO_2$-N[($C_1$-$C_8$)-Alkyl]$_2$, $SO_2$-NH-$(CH_2)_r$$NH_2$, $SF_5$, COOH, CO-$NH_2$, $(CH_2)_q$-CN, $(CH_2)_n$-CO-NH-CN, $(CH_2)_n$-CO-NH-piperidin-1-yl, $(CH_2)_n$-CO-NH-$SO_2$-NHR12, $(CH_2)_n$-CO-NH-$SO_2$-R18, $(CH_2)_n$-CHO, (CH2)n-C(=NH)$NH_2$, $(CH_2)_n$-C(=NH)-NHOH, $(CH_2)_n$-C(=NH)-[NH-O-($C_1$-$C_6$)-Alkyl], (CH2)n-C(=NH)(R16), $(CH_2)_n$-C(=NR13)NHR12, $(CH_2)_n$-C(=NR12)NR12R13, $(CH_2)_n$-C(=$NSO_2$-R12)NH2, $(CH_2)_n$-C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, $S(O)_m$($C_1$-$C_6$)-Alkyl, $SO_2$-$NH_2$, COOH, $CONH_2$, CO-O($C_1$-$C_6$)-Alkyl, CO-($C_1$-$C_6$)-Alkyl substituiert sein können und wobei die Alkylreste mit Fluoratomen substituiert sein können,

und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff verschiedenen Substituenten versehen ist;

H, F, Cl, Br, J, CN, $N_3$, NC, $NO_2$, $CF_3$,
($C_1$-$C_8$)-Alkyl, ($C_2$-$C_{10}$)-Alkenyl, ($C_2$-$C_{10}$)-Alkinyl, ($C_3$-$C_8$)-Cycloalkyl,
Aryl, Heteroaryl,
$(CH_2)_n$-CO- [O-($C_1$-$C_8$)-Alkyl], $(CH_2)_n$-CO-[O-($C_3$-$C_8$)-Cycloalkyl], $(CH_2)_n$-CO-[($C_1$-$C_8$)-Alkyl], $(CH_2)_n$-CO-[($C_3$-$C_8$)-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-($C_1$-$C_6$)-Alkyl], $(CH_2)_n$-P(O)[O-($C_1$-$C_6$)-Alkyl]$_2$, $(CH_2)_n$-P(O)(OH)(O-$CH_2$-Aryl), $(CH_2)_n$-P(O)(O-$CH_2$-Aryl)$_2$, $(CH2)_n$-P(O)(OH)$_2$,
$(CH_2)_n$-$SO_3$H, $(CH_2)_n$-$SO_2$-$NH_2$,
$(CH_2)_n$-CO-NH-[($C_1$-$C_8$)-Alkyl], $(CH_2)_n$-CO-N[($C_1$-$C_8$)-Alkyl], $(CH_2)_n$-CO-NH-[($C_3$-$C_8$)-Cycloalkyl],
($C_2$-$C_{10}$)-Alkenyl-CO-O[($C_1$-$C_6$)-Alkyl], ($C_2$-$C_{10}$)-Alkenyl-CONH$_2$, ($C_2$-$C_{10}$)-Alkenyl-COOH, ($C_2$-$C_{10}$)-Alkinyl-CO-O[($C_1$-$C_6$)-Alkyl], ($C_2$-$C_{10}$)-Alkinyl-CONH$_2$, ($C_2$-$C_{10}$)-Alkinyl-COOH,
$(CH_2)_n$-CO-R16,
$(CH_2)_n$-OH, $(CH_2)_n$-O-($C_1$-$C_8$)-Alkyl, $(CH_2)_n$-O-($C_2C_{10}$)-Alkenyl, $(CH_2)_n$-O-($C_2$-$C_{10}$)-Alkinyl, $(CH_2)_n$-O-($C_3$-$C_8$)-Cycloalkyl, $(CH_2)_n$-O-$(CH_2)_q$-[($C_3$-$C_8$)-Cycloalkyl], $(CH_2)_n$-O-$(CH_2)_n$-CO-[O-($C_1$-$C_8$)-Alkyl], $(CH_2)_n$-O-$(CH_2)_n$-CO-[O-($C_3$-$C_8$)-Cycloalkyl], $(CH_2)_n$-O-$(CH_2)_n$CO-[($C_1$-$C_8$)-Alkyl], $(CH_2)_n$-O-$(CH_2)_n$-CO-[($C_3$-$C_8$)-Cycloalkyl], $(CH_2)_n$-O-$(CH_2)_n$-CO-[O-$(CH_2)_v$-Aryl], $(CH_2)_n$-O-$(CH_2)_n$-CO-[O-$(CH_2)_v$-Heteroaryl], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P(O)(OH)[O-($C_1$-$C_6$)-Alkyl], $(CH_2)_n$-O-$(CH_2)_n$-P(O)[O-($C_1$-$C_6$)-Alkyl]$_2$, $(CH_2)_n$-O-$(CH_2)_n$-P(O)(OH)(O-$CH_2$-Aryl), $(CH_2)_n$-O-$(CH_2)_n$-P(O)(O-$CH_2$Aryl)$_2$, $(CH_2)_n$-O-$(CH_2)_n$-P(O)(OH)$_2$, $(CH_2)_n$-O-$(CH_2)_n$-$SO_3$H, $(CH_2)_n$-O-$(CH_2)_n$-$SO_2$-$NH_2$, $(CH_2)_n$-O-$(CH_2)_n$-CO-NH-[($C_1$-$C_8$)-Alkyl], $(CH_2)_n$-O-$(CH_2)_n$-CO-N[($C_1$-$C_8$)-Alkyl]$_2$, $(CH_2)_n$-O-$(CH_2)_n$-CO-NH-[($C_3$-$C_8$)-Cycloalkyl], $(CH_2)_n$-O-$(CH_2)_n$-CR21 R22-CO-O[($C_1$-$C_6$)-Alkyl], $(CH_2)_n$-O-$(CH_2)_n$CR21R22-CONH$_2$, $(CH_2)_n$-O-$(CH_2)_n$-CR21R22-COOH, $(CH_2)_n$-O-$(CH_2)$n-CO-R16, $(CH_2)_n$-O-$(CH_2)_r$-OH, $(CH_2)_n$-O-CH($CH_2$OH)$_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$,
$(CH_2)_n$-$NH_2$, $(CH_2)_n$-NH-($C_1$-$C_8$)-Alkyl, $(CH_2)_n$-NH-($C_3$-$C_8$)-Cycloalkyl, $(CH_2)_n$-NH-$(CH_2)_n$-CO-[O-($C_3$-$C_8$)-Cycloalkyl], $(CH_2)_n$-NH-$(CH_2)$n-CO-[($C_1$-$C_8$)-Alkyl], $(CH_2)_n$-NH-$(CH_2)_n$-CO-[($C_3$-$C_8$)-Cycloalkyl], $(CH_2)_n$-NH-$(CH_2)_n$-CO-[O-$(CH_2)_v$-Aryl], $(CH_2)_n$-NH-$(CH_2)_n$-CO-[O-$(CH_2)_v$-Heteroaryl], $(CH_2)_n$-NH-$(CH_2)_q$-CO-NH-CN,
$(CH_2)$n-NH-$(CH_2)_n$-P(O)(OH)$_2$,
$(CH_2)_n$-NH-$(CH_2)_n$-$SO_3$H,
$(CH_2)_n$-NH-$(CH_2)_n$-$SO_2$-$NH_2$,
$(CH_2)_n$-NH-$(CH_2)_n$-CR21R22-CO-O[($C_1$-$C_6$)-Alkyl], $(CH_2)_n$-NH-$(CH_2)_n$-CR21R22-CONH$_2$,
$(CH_2)_n$-NH-$(CH_2)_n$-CR21 R22-COOH,
$(CH_2)_n$-NH-$(CH_2)_n$-CO-R16,
$(CH_2)_n$-NH-$(CH_2)_n$-$SO_2$-[($C_1$-$C_8$)-Alkyl], $(CH_2)_n$-NH- $(CH_2)_n$-$SO_2$-[($C_3$-$C_8$)-Cycloalkyl], $(CH_2)_n$NH-$SO_2$-$(CH_2)_n$-$NH_2$, $(CH_2)_n$-NH-$SO_2$-$(CH_2)_n$-NH-($C_1$-$C_8$)-Alkyl, $(CH_2)_n$-NH-$SO_2$-$(CH_2)_n$-NH-($C_3$-$C_8$)-Cycloalkyl, $(CH_2)_n$-NH-$SO_2$-$(CH_2)_n$-N[($C_1$-$C_8$)-Alkyl]$_2$,
$(CH_2)_n$-NH-CN, $(CH_2)_n$-NH-$SO_2$-R16,
$(CH_2)_n$-NR12-CO-NH-($C_1$-$C_8$)-Alkyl, $(CH_2)_n$-NR12-CO-NH-($C_3$-$C_8$)-Cycloalkyl, $(CH_2)_n$-NK12-CO-$NH_2$, $(CH_2)_n$-NR12-CO-NH-$SO_2$-($C_1$-$C_8$)-Alkyl, $(CH_2)_n$-NR12-CO-NH-$SO_2$-($C_3$-$C_8$)-Cyclo alkyl, $(CH_2)_n$-NR12-CO-N[($C_1$-$C_8$)-Alkyl]$_2$, $(CH_2)_n$-NH-CO-NH-$(CH_2)_n$-CO-[O-($C_1$-$C_8$)-Alkyl], $(CH_2)_n$-NH-CO-NH-$(CH_2)_q$-CO-$NH_2$, $(CH_2)$n-NH-CO-NH-$(CH_2)_q$-COOH, $(CH_2)_n$-NH-C(=NH)-$NH_2$, $(CH_2)$n-NH-C(=NH)-R16, $(CH_2)_n$-NH-C(-NH)-NH[($C_1$-$C_8$)-Alkyl], $(CH_2)_n$-NH-C(=N-$SO_2$-($C_1$-$C_8$)-Alkyl)-$NH_2$, $(CH_2)_n$-NH-C(=N-SO2-($C_1$-$C_8$)-Alkyl)-NH[($C_1$-$C_8$)-Alkyl], $(CH_2)_n$-NH-C(=N-$SO_2$-$NH_2$)-$NH_2$, $(CH_2)_n$-NH-C(=N-$SO_2$-$NH_2$)-NH[($C_1$-$C_8$)-Alkyl], $(CH_2)_n$-NH-C(=NH)-N[($C_1$-$C_8$)-Alkyl]$_2$, $(CH_2)_n$-NH-C(=N-$SO_2$-(C1-$C_8$)-Alkyl)-N[($C_1$-$C_8$)-Alkyl], $(CH_2)_n$-NH-$(CH_2)_n$-CO-O-$(CH_2)_r$-$NH_2$, $(CH_2)_n$-NH-$(CH_2)_n$ -CO-NH-[($C_1$-$C_8$)-Alkyl], $(CH_2)_n$-NH-$(CH_2)_n$ -CO-NH-$(CH_2)_r$-OH, $(CH_2)_n$-NH-$(CH_2)_n$ -CO-N[($C_1$-$C_8$)-Alkyl]$_2$, $(CH_2)_n$-NH-$(CH_2)_n$ -CO-NH-[($C_3$-$C_8$)-Cycloalkyl], $(CH_2)_n$-NH-C($CH_3$)$_2$-CO-O($C_3$-$C_8$)-Cycloalkyl, $(CH_2)_n$-NH-C($CH_3$)$_2$-CO-O-$(CH_2)_r$-$NH_2$, $(CH_2)_n$-NH-C($CH_3$)$_2$-CO-O-$(CH_2)_n$-Aryl, $(CH_2)_n$-NH-C($CH_3$)$_2$-CO-O-

$(CH_2)_n$-Heteroaryl, $(CH_2)_n$-NH-C$(CH_3)_2$-CO-NH-[($C_1$-$C_8$)-Alkyl], $(CH_2)_n$-NH-C$(CH_3)_2$-CO-NH-$(CH_2)_r$-OH, $(CH_2)_n$-NH-C$(CH_3)_2$-CO-N[($C_1$-$C_8$)-Alkyl]$_2$, $(CH_2)_n$-NH-$(CH_3)_2$-CO-NH-[($C_3$-$C_8$)-Cycloalkyl], $(CH_2)_n$-NH-C$(CH_3)_2$-CO-N[($C_3$-$C_8$)-Cycloalkyl]$_2$, $(CH_2)_n$-S(O)$_m$-($C_1$-$C_8$)-Alkyl, $(CH_2)_n$-S(O)$_m$-($C_3$-$C_8$)-Cycloalkyl, $(CH_2)_n$-$SO_2$-R16, $SO_2$-N=CH-N$(CH_3)_2$,

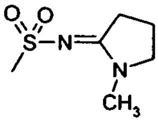,

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)-Alkyl, $(CH_2)_n$-$SO_2$-NH-($C_3$-$C_8$)-Cycloalkyl, $(CH_2)_n$-$SO_2$-N[($C_1$-$C_8$)-Alkyl]$_2$, $SO_2$-NH $(CH_2)_r$-OH, $SO_2$-NH-$(CH_2)_r$-$NH_2$, $SF_5$,
$(CH_2)_q$-CN,
$(CH_2)_n$-CO-NH-piperidin-1-yl,
$(CH_2)_n$-CO-NH-$SO_2$-NHR12, $(CH_2)_n$-CO-NH-$SO_2$($C_1$-$C_8$)-Alkyl, $(CH_2)_n$-CO-NH-$SO_2$-($C_3$-$C_3$)-Cycloalkyl,
$(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl,($C_3$-$C_6$)-Cycloalkyl, 0-($C_1$-$C_6$)-Alkyl, S(O)$_m$-(C1-$C_6$)-Alkyl, $SO_2$-$NH_2$, COOH, $CONH_2$, CO-[O($C_1$-$C_6$)-Alkyl], CO-($C_1$-$C_6$)-Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und
wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, ($C_1$-$C_8$)-Alkyl oder =O substituiert sein können;

T NR23-CO-NR24, NR23-$SO_2$-NR24, $SO_2$-NR23-$SO_2$ CO-NR23-CO, NR23- C(=NR13)-NR24, NR23-C(=NR22)-NR24, CO-NR23-CR22R23, NR23-CO- CR22R24, NR23-$SO_2$-CR22R24, CR22R24-CO-NR23, CR22R24-$SO_2$-NR23, CR22R23-NR23-$SO_2$, $SO_2$CR22R23-NR23, $SO_2$-NR23-CR22R23-, NR23- CR22R23-$SO_2$, CR23R24-CR23R24-CR23R24, CR23R24-NR23-CR23R24;

R11 H, ($C_1$-$C_8$)-Alkyl, ($C_2$-$C_{10}$)-Alkinyl, ($C_3$-$C_6$)-Cycloalkyl, $(CH_2)_n$-Aryl, $(CH_2)_n$- CO-[O-($C_1$-$C_6$)-Alkyl], $(CH_2)_n$-CO-[O-($C_3$-$C_6$)-Cycloalkyl], (CH2)n-CO-[($C_1$- $C_6$)-Alkyl], $(CH_2)_n$-CO-[($C_3$-$C_6$)-Cycloalkyl], $(CH_2)_n$-CO-Aryl, $(CH_2)_n$-CO- Heteroaryl, $(CH_2)_q$-CO-$NH_2$, $(CH_2)_q$-COOH, $(CH_2)_n$-P(O)(OH)[O-($C_1$-$C_6$)- Alkyl], $(CH_2)_n$-P(O)[O-($C_1$-$C_4$)-Alkyl]$_2$, $(CH_2)_n$-P(O)(O-$CH_2$-Aryl)$_2$, $(CH_2)_n$- P(O)$(OH)_2$, $(CH_2)_n$-$SO_3H$, $(CH_2)_n$-$SO_2$-$NH_2$, $(CH_2)_n$-CO-NH-[($C_1$-$C_4$)-Alkyl], $(CH_2)_n$-CO-N[($C_1$-$C_4$)-Alkyl]$_2$, $(CH_2)_n$-CO-NH-[($C_3$-$C_6$)-Cycloalkyl], ($C_2$-$C_6$)- Alkenyl-CO-O[($C_1$-$C_4$)-Alkyl], ($C_2$-$C_6$)-Alkenyl-$CONH_2$, ($C_2$-$C_6$)-Alkenyl- COOH, ($C_2$-$C_6$)-Alkinyl-CO-O[($C_1$-$C_4$)-Alkyl], ($C_2$-$C_6$)-Alkinyl-$CONH_2$, ($C_2$- $C_6$)-Alkinyl-COOH, $(CH_2)_n$-CR21[(CO-O($C_1$-$C_4$)-Alkyl)]$_2$, $(CH_2)_n$- CR21$(CONH_2)_2$, $(CH_2)_n$-CR21$(COOH)_2$, $(CH_2)_n$-CR21R22-CO-O[($C_1$-$C_4$)- Alkyl], $(CH_2)_n$-CR21R22-$CONH_2$, $(CH_2)_n$-CR21R22-CO-NH-[($C_1$-$C_4$)-Alkyl], $(CH_2)_n$-CR21 R22-CO-N[($C_1$ -$C_4$)-Alkyl]$_2$, $(CH_2)_n$-CR21 R22-COOH, $(CH_2)_n$-CO-R16, $(CH_2)_n$-CO-NH-C$(CH_3)_2$-CO-O[($C_1$-$C_8$)-Alkyl], $(CH_2)_n$-CO-NH-C$(CH_3)_2$-CC)$NH_2$, $(CH_2)_n$-CO-NH-C$(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, ($C_1$-$C_4$)-Alkyl, O- ($C_1$-$C_4$)-Alkyl, S(O)m-($C_1$-$C_4$)-Alkyl, $SO_2$-$NH_2$, COOH, $CONH_2$, CO-O($C_1$-$C_4$)- Alkyl substituiert sein kann und wobei die Alkylreste mit Fluoratomen substituiert sein können;

R12 H, ($C_1$-$C_8$)-Alkyl, ($C_3$-$C_8$)-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, ($C_1$-$C_4$)-Alkyl, O-($C_1$-$C_4$)-Alkyl, $SO_2$-$NH_2$, COOH, $CONH_2$, CO-0($C_1$-$C_4$)-Alkyl substituiert sein kann und wobei die Alkylreste mit Fluoratomen substituiert sein können;

R13 H, $SO_2$-[($C_1$-$C_8$)-Alkyl], $SO_2$-[($C_3$-$C_8$)-Cycloalkyl], $SO_2$-$(CH_2)_n$-Aryl, $SO_2$-$(CH_2)_n$-Heteroaryl,

wobei die Alkyl- und Cycloalkylreste mit Fluoratomen substituiert sein können und wobei der Aryl- oder Heteroarylrest mit Halogen, CN, $CF_3$, $(C_1-C_4)$-Alkyl, O-[$(C_1-C_4)$-Alkyl], S(O)m-[$(C_1-C_6)$-Alkyl], $SO_2$-$NH_2$, COOH, $CONH_2$, CO- [O$(C_1-C_4)$-Alkyl] substituiert sein kann und wobei die Alkylreste mit Fluoratomen substituiert sein können;

R15 $(C_1-C_6)$-Alkyl,
wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[$(C_1-C_6)$- Alkyl]piperazin-1-yl, Thiomorpholin-1,1-Dioxid-4-yl, NH-$(CH_2)_r$-OH, NH- $CH(CH_2OH)_2$, NH-$C(CH_2OH)_3$, N[$(C_1-C_4)$-Alkyl-OH]$_2$, D-Glucamin-N-yl, N- Methyl-D-Glucamin-N-yl, NH-[$(C_1-C_4)$-Alkyl]-COOH, NH-[$(C_1-C_4)$-Alkyl]- $CONH_2$, N[$(C_1-C_4)$-Alkyl][$(C_1-C_4)$-Alkyl]-COOH, NH-[C(H)(Aryl)]-CO-O$(C_1- C_4)$-Alkyl, NH-[C(H)(Aryl)]-COOH, NH-[C(H)(Aryl)]-$CONH_2$, NH- [C(H)(Heteroaryl)]-CO-O$(C_1-C_4)$-Alkyl, NH-[C(H)(Heteroaryl)]-COOH, NH- [C(H)(Heteroaryl)]-$CONH_2$, NH-[$(C_3-C_6)$-Cycloalkyl]-CO-O$(C_1-C_4)$-Alkyl, NH- [$(C_3-C_6)$-Cycloalkyl]-COOH, NH-[$(C_3-C_6)$-Cycloalkyl]-$CONH_2$, NH-$(C_1-C_4)$-Alkyl-OH, NH-[( $C_1-C_4$)-Alkyl]-$SO_2$-$(C_1$- C4)-Alkyl, NH-[$(C_1-C_4)$-Alkyl]- $SO_3H$, NH-[$(C_1-C_4)$-Alkyl]-$SO_2$-$NH_2$, wobei die Alkohol (OH) - Funktionen durch F ersetzt sein können und wobei der Aryl- oder Heteroarylrest mit Halogen, CN, $(C_1-C_4)$-Alkyl, 0-$(C_1-C_4)$-Alkyl, OH, $SO_2$-$NH_2$, COOH, $CONH_2$, CO-O$(C_1-C_4)$-Alkyl substituiert sein kann;

R18 $(CH_2)_n$-CR25R26-CO-O$(C_1-C_4)$-Alkyl, $(CH_2)_n$-CR25R26-CO-$NH_2$, $(CH_2)_n$- CR25R26-COOH;

R20 H, $(C_1-C_4)$-Alkyl, $(C_3-C_6)$-Cycloalkyl, Aryl, [$(C_1-C_4)$-Alkyl]-Aryl;

R21 H, F, $CF_3$, $(C_1-C_4)$-Alkyl, $(C_3-C_6)$-Cycloalkyl, OH, O-$(C_1-C_4)$-Alkyl, O-$(C_3-C_6)$- Cycloalkyl, O-$(CH_2)_n$-Aryl, O-(CO)-$(C_1-C_4)$-Alkyl, O-(CO)-$(C_3-C_6)$-Cycloalkyl, O-(CO)-O-$(C_1-C_4)$-Alkyl, O-(CO)-O-$(C_3-C_6)$-Cycloalkyl, NH-[$(C_1-C_4)$-Alkyl]- Aryl, $NH_2$, NH-$(C_1-C_4)$-Alkyl, NH-(CO)-$(C_1-C_4)$-Alkyl;

R22 H, $CF_3$, $(C_1-C_4)$-Alkyl, Aryl, [$(C_1-C_4)$-Alkyl]-Aryl;

R23, R24 unabhängig voneinander H, $(C_1-C_4)$-Alkyl, $(C_3-C_6)$-Cycloalkyl, [$(C_1-C_4)$-Alkyl]- [$(C_3-C_6)$-Cycloalkyl], Aryl, [$(C_1-C_4)$-Alkyl]-Aryl
oder R23 und R24 bilden zusammen eine -CH=CH-, -$CH_2$-$CH_2$-, -$CH_2$-$CH_2$- $CH_2$-, oder -$CH_2$-$CH_2$-$CH_2$-$CH_2$- Einheit, worin eine $CH_2$-Gruppierung durch C=O, CHF oder $CF_2$ ersetzt sein kann, und worin bis zu vier Wasserstoffatome durch einen $(C_1-C_4)$-Alkylrest ersetzt sein können;

R25, R26 unabhängig voneinander H, F, $(C_1-C_4)$-Alkyl, Aryl, [$(C_1-C_4)$-Alkyl]-Aryl,
wobei der Aryl mit Halogen, CN, OH, O-$(C_1-C_4)$-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[$(C_1-C_4)$-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

Ganz besonders bevorzugt sind Verbindungen der Formel I, worin ein oder mehrere Reste die folgenden Bedeutungen haben:

R, R' unabhängig voneinander H, Aryl, $(C_1-C_4)$-Alkyl, wobei $(C_1-C_4)$-Alkyl oder der Arylrest substituiert sein kann mit Halogen;
oder R und R' bilden gemeinsam einen Ring mit drei bis acht Kohlenstoffatomen, wobei ein Kohlenstoffatom durch O, $S(O)_m$, NR13 oder NR15 ersetzt sein kann;
m 0, 1, 2;
n 0, 1, 2;
p 1, 2, 3;
q 1, 2;
r 2, 3, 4;
v 0, 1, 2;
A, D, E, G, L unabhängig voneinander C oder N, wobei bei der Bedeutung N der entsprechende Substituent

R1, R2, R3, R4, R5 entfällt, oder R2-D=E-R3 oder R4-G=L-R5 haben die Bedeutung S oder O und wobei der Fünf- oder Sechsring mit -$(CH_2)_3$- oder -$(CH_2)_4$- oder -CH=CH-CH=CH- zu einem Bicyclus anelliert sein kann;

R1, R2, R3, R4, R5 unabhängig voneinander H, F, Cl, Br, J, CN, $CF_3$, ($C_1$-$C_8$)-Alkyl, ($C_3$- $C_8$)-Cycloalkyl, $(CH_2)_n$-Aryl, $(CH_2)_n$-Heteroaryl, $OCF_3$, O-R11, NR13R15, $S(O)_m$-R12, $SO_2$-$NH_2$, $SO_2$-NH-[($C_1$-$C_8$)-Alkyl], $SO_2$-NH-[($C_3$-$C_8$)-Cycloalkyl], $SO_2$-NH-$(CH_2)_n$-Aryl, $SO_2$-NH-$(CH_2)_n$Heteroaryl, $SO_2$-N[($C_1$-$C_8$)-Alkyl]$_2$, $SO_2$- R16, $SF_5$, CO-O[($C_1$-$C_8$)-Alkyl],
CO-O[($C_3$-$C_6$)-Cycloalkyl], CO-$NH_2$, CO-NH-[($C_1$-$C_4$)-Alkyl], CO-N[($C_1$-$C_4$)- Alkyl)$_2$,C(=NH)-$NH_2$, C(=NH)-R16, $(CH_2)_n$-C(=$NSO_2$-R12)$NH_2$, CO-R16, COOH, CO-($C_1$-$C_4$)-Alkyl, CO-($C_3$-$C_6$)-Cycloalkyl, CO-Aryl, CO-Heteroaryl, CH(OH)-Aryl, CH(OH)-Heteroaryl, CHF-Aryl, CHF-Heteroaryl, $CF_2$-Aryl, $CF_2$-Heteroaryl, $CH_2$-OH, $CH_2$-CN, $CH_2$-O-R12, $CH_2$-O-$(CH_2)_q$-COOH,
wobei die Alkyl- und Cycloalkylreste mit Fluoratomen substituiert sein können und wobei die Aryl- oder Heteroarylreste mit Halogen, CN, ($C_1$-$C_4$)-Alkyl, O- ($C_1$-$C_4$)-Alkyl, $OCF_3$, OH, O-$(CH_2)_n$-Aryl, $(CH_2)_n$-Aryl, $S(O)_m$-($C_1$-$C_4$)-Alkyl, $SO_2$-$NH_2$, SH, NR12R13, NH-CO-[($C_1$-$C_4$)-Alkyl], NH-CO-$(CH_2)_n$-Aryl, $(CH_2)_n$-COOH, $(CH_2)_n$-$CONH_2$, $(CH_2)_n$CO-O($C_1$-$C_4$)-Alkyl, $(CH_2)_n$-CO-($C_1$- $C_4$)-Alkyl substituiert sein können und wobei die Alkylreste mit Fluoratomen substituiert sein können;

R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder 6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -($C_1$-$C_6$)-Alkyl substituiert sein kann und wobei der bicyclische Heterocyclus 9 bis 12 Ringglieder enthalten kann und bis zu fünf CH- bzw. $CH_2$- Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit

R11, F, Cl, Br, J, CN, $N_3$, NC, $NO_2$, $CF_3$, $(CH_2)_n$-O-R11, $(CH_2)_n$-O- $(CH_2)_r$-OH, $(CH_Z)_n$-O-CH($CH_2$OH)$_2$, $(CH_2)_n$-O-$(CH_2)_n$-CO-O-$(CH_2)_r$- $NH_2$, $(CH_2)_n$-O-($CH_2$)n-CO-NH-$(CH_2)_r$-OH, O-R13, $OCF_3$, $(CH_2)_n$-O- $(CH_2)_r$$NH_2$, $(CH_2)_n$-NH-R11, $(CH_2)_n$-N[$(CH_2)_q$-CO-O($C_1$-$C_6$)-Alkyl]$_2$, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N(R13)$_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, ($CH_2$)n-NR12-CO-N(R12)$_2$, $(CH_2)_n$-NR12-CO-NHR11, $(CH_2)_n$-NH-C(=NH)-$NH_2$, $(CH_2)_n$-NH-C(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$ -CO-NH-[($C_1$-$C_8$)-Alkyl], $(CH_2)_n$-NH-$(CH_2)_n$ -CO-NH-$(CH_2)_r$-OH, $(CH_2)_n$-NH-$(CH_2)_n$ -CO-N[(C1-C8)-Alkyl]$_2$, $(CH_2)_n$-NH-$(CH_2)_n$ -CO-NH-[($C_3$-$C_8$)-Cycloalkyl], $(CH_2)_n$-NH-$(CH_2)_n$ -CO-N[($C_3$-$C_8$)-Cycloalkyl]$_2$, $(CH_2)_n$-NH-C$(CH_3)_2$-CO-O($C_1$-$C_8$)-Alkyl, $(CH_2)_n$-NH-C$(CH_3)_2$-CO-O($C_3$-$C_8$)-Cycloalkyl, $(CH_2)_n$-NH-C$(CH_3)_2$-CO-O-$(CH_2)_r$-$NH_2$, $(CH_2)_n$-NH-C$(CH_3)_2$-CO-O-$(CH_2)_n$-Aryl, $(CH_2)_n$-NH-C$(CH_3)_2$-CO-O-$(CH_2)_n$-Heteroaryl, $(CH_2)_n$-NH-C$(CH_3)_2$-CO-$NH_2$, $(CH_2)_n$-NH-C$(CH_3)_2$-CO-NH-[($C_1$-$C_8$)-Alkyl], $(CH_2)_n$-NH-C$(CH_3)_2$-CO-NH-$(CH_2)_r$-OH, $(CH_2)_n$-NH-C$(CH_3)_2$-CO-N[($C_1$-$C_8$)-Alkyl]$_2$, $(CH_2)_n$-NH-$(CH_3)_2$-CO-NH-[($C_3$-$C_8$)-Cycloalkyl], $(CH_2)_n$-NH-C$(CH_3)_2$-CO-N[($C_3$-$C_8$)-Cycloalkyl]$_2$, $(CH_2)_n$-NH-C$(CH_3)_2$-COOH, $S(O)_m$-R12, $SO_2$-R16, $SO_2$-N=CH-N$(CH_3)_2$,

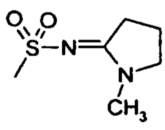
,

$SO_2$-NH-CO-R12, $SO_2$-NHR12, $SO_2$-NH-($CH_2$)r-OH, $SO_2$-N[($C_1$-$C_8$)-Alkyl]$_2$, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH-piperidin-1-yl, $(CH_2)_n$-CO-NH-$SO_2$-NHR12, $(CH_2)_n$-CO-NH-$SO_2$-R18, $(CH_2)_n$-CHO, $(CH_2)_n$-C(=NH)$NH_2$, $(CH_2)_n$-C(=NH)-NHOH, $(CH_2)_n$-C(=NH)-[NH-O-($C_1$-$C_6$)-Alkyl], $(CH_2)_n$-C(=NH)(R16), $(CH_2)_n$-C(=NR13)NHR12, $(CH_2)_n$-C(=NR12)NR12R13, $(CH_2)_n$-C(=$NSO_2$-R12)$NH_2$, $(CH_2)_n$-C(=NH)O[($C_1$-$C_6$)-Alkyl], wobei die Alkyl- und Cycloalkylreste mit Fluoratomen

substituiert sein können und wobei die Aryl- oder Heteroarylreste mit Halogen, CN, $(C_1\text{-}C_6)$-Alkyl, $(C_3\text{-}C_6)$-Cycloalkyl, O-$(C_1\text{-}C_6)$-Alkyl, S(O)m-$(C_1\text{-}C_6)$-Alkyl, $SO_2$-$NH_2$, COOH, $CONH_2$, CO-O$(C_1\text{-}C_6)$-Alkyl, CO-$(C_1\text{-}C_6)$-Alkyl substituiert sein können und wobei die Alkylreste mit Fluoratomen substituiert sein können, und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff verschiedenen Substituenten versehen ist;

H, F, Cl, Br, J, CN, $N_3$, NC, $NO_2$, $CF_3$,
$(C_1\text{-}C_8)$-Alkyl, $(C_2\text{-}C_{10})$-Alkenyl, $(C_2\text{-}C_{10})$-Alkinyl, $(C_3\text{-}C_8)$-Cycloalkyl, Aryl, Heteroaryl, $(CH_2)_n$-CO-[O-$(C_1\text{-}C_8)$-Alkyl], $(CH_2)_n$-CO-[O$(C_3\text{-}C_8)$-Cycloalkyl], $(CH_2)_n$-CO-[$(C_1\text{-}C_8)$-Alkyl], $(CH_2)_n$-CO-[ $(C_3\text{-}C_8)$-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-$(C_1\text{-}C_6)$-Alkyl], $(CH_2)_n$-P(O)[O-$(C_1\text{-}C_6)$-Alkyl]$_2$, $(CH_2)_n$-P(O)(OH)(O-$CH_2$-Aryl), $(CH_2)_n$-P(O)(O-$CH_2$-Aryl)$_2$, $(CH_2)_n$-P(O)(OH)$_2$,
$(CH_2)_n$-$SO_3H$, $(CH_2)_n$-$SO_2$-$NH_2$,
$(CH_2)_n$-CO-NH-[$(C_1\text{-}C_8)$-Alkyl], $(CH_2)_n$-CO-N[$(C_1\text{-}C_8)$-Alkyl]$_2$, $(CH_2)_n$-CO-NH-[$(C_3\text{-}C_8)$-Cycloalkyl],
$(C_2\text{-}C_{10})$-Alkenyl-CO-O[$(C_1\text{-}C_6)$-Alkyl], $(C_2\text{-}C_{10})$-Alkenyl-$CONH_2$, $(C_2\text{-}C_{10})$-Alkenyl-COOH, $(C_2\text{-}C_{10})$-Alkinyl-CO-O[$(C_1\text{-}C_6)$-Alkyl], $(C_2\text{-}C_{10})$-Alkinyl-$CONH_2$, $(C_2\text{-}C_{10})$-Alkinyl-COOH,
$(CH_2)_n$-CO-R16,
$(CH_2)_n$-OH, $(CH_2)_n$-O-$(C_1\text{-}C_8)$-Alkyl, $(CH_2)_n$-O-$(C_2\text{-}C_{10})$-Alkenyl, $(CH_2)_n$-O-$(C_2\text{-}C_{10})$-Alkinyl, $(CH_2)_n$-O-$(C_3\text{-}C_8)$-Cycloalkyl, $(CH_2)_n$-O-$(CH_2)_q$-[$(C_3\text{-}C_8)$-Cycloalkyl], $(CH_2)_n$-O-$(CH_2)_n$-CO-[O-$(C_1\text{-}C_8)$-Alkyl], $(CH_2)_n$-O-$(CH_2)_n$-CO-[O-$(C_3\text{-}C_8)$-Cycloalkyl), $(CH_2)_n$-O-$(CH_2)_n$-CO-[$(C_1\text{-}C_8)$-Alkyl], $(CH_2)_n$-O-$(CH_2)_n$-CO-[$(C_3\text{-}C_8)$-Cycloalkyl], $(CH_2)_n$-O-$(CH_2)_n$-CO-[O-$(CH_2)_v$-Aryl], $(CH_2)_n$-O-$(CH_2)_n$-CO-[O-$(CH_2)_v$-Heteroaryl], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P(O)(OH)[O-$(C_1\text{-}C_6)$-Alkyl], $(CH_2)_n$-O-$(CH_2)_n$-P(O)[O-$(C_1\text{-}C_6)$-Alkyl]$_2$, $(CH_2)_n$-O-$(CH_2)_n$-P(O)(OH)(O-$CH_2$-Aryl), $(CH_2)_n$-O-$(CH_2)_n$-P(O)(O-$CH_2$-Aryl)$_2$, $(CH_2)_n$-O-$(CH_2)_n$-P(O)(OH)$_2$, $(CH_2)_n$-O-$(CH_2)_n$-$SO_3H$, $(CH_2)_n$-O-$(CH_2)_n$-$SO_2$-$NH_2$, $(CH_2)_n$-O-$(CH_2)_n$-CO-NH-[$(C_1\text{-}C_8)$-Alkyl], $(CH_2)_n$-O-$(CH_2)_n$-CO-N[$(C_1\text{-}C_8)$-Alkyl]$_2$, $(CH_2)_n$-O-$(CH_2)_n$-CO-NH-[$(C_3\text{-}C_8)$-Cycloalkyl], $(CH_2)_n$-O-(CH2)$_n$-CR21R22-CO-O[$(C_1\text{-}C_6)$-Alkyl], $(CH_2)_n$-O-$(CH_2)_n$-CR21R22-$CONH_2$, $(CH_2)_n$-O-$(CH_2)_n$-CR21R22-COOH, $(CH_2)_n$-O-$(CH_2)_n$-CO-R16, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$,
$(CH_2)_n$-$NH_2$, $(CH_2)_n$-NH-$(C_1\text{-}C_8)$-Alkyl, $(CH_2)_n$-NH-$(C_3\text{-}C_8)$-Cycloalkyl, $(CH_2)_n$-NH-$(CH_2)_n$-CO-[O-$(C_3\text{-}C_8)$-Cycloalkyl], $(CH_2)_n$-NH-$(CH_2)_n$-CO-[(C1-$C_8$)-Alkyl], $(CH_2)_n$-NH-$(CH_2)_n$-CO-[$(C_3\text{-}C_8)$-Cycloalkyl], $(CH_2)_n$-NH-$(CH_2)_n$-CO-[O-$(CH_2)_v$-Aryl], $(CH_2)_n$-NH-$(CH_2)_n$-CO-[O-$(CH_2)_v$-Heteroaryl], $(CH_2)_n$-NH-$(CH_2)_q$-CO-NH-CN,
$(CH_2)_n$-NH-$(CH_2)_n$-P(O)(OH)$_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[$(C_1\text{-}C_6)$-Alkyl], $(CH_2)_n$-NH-$(CH_2)_n$-CR21 R22-$CONH_2$, $(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[$(C_1\text{-}C_8)$-Alkyl], $(CH_2)_n$-NH- $(CH_2)_n$-$SO_2$-[$(C_3\text{-}C_8)$-Cycloalkyl], $(CH_2)_n$-NH-$SO_2$-$(CH_2)_n$-$NH_2$, $(CH_2)_n$-NH-$SO_2$-$(CH_2)_n$-NH-$(C_1\text{-}C_8)$-Alkyl, $(CH_2)_n$-NH-$SO_2$-$(CH_2)_n$-NH-$(C_3\text{-}C_8)$-Cycloalkyl, $(CH_2)_n$-NH-$SO_2$-$(CH_2)_n$-N[$(C_1\text{-}C_8)$-Alkyl]$_2$,
$(CH_2)_n$-NH-CN, $(CH_2)_n$-NH-$SO_2$-R16,
$(CH_2)_n$-NR12-CO-NH-$(C_1\text{-}C_8)$-Alkyl, $(CH_2)_n$-NR12-CO-NH-$(C_3\text{-}C_8)$-Cycloalkyl, $(CH_2)_n$-NR12-CO-$NH_2$, $(CH_2)_n$-NR12-CO-NH-$SO_2$-$(C_1\text{-}C_8)$-Alkyl, $(CH_2)_n$-NR12-CO-NH-$SO_2$-$(C_3\text{-}C_8)$-Cycloalkyl, $(CH_2)_n$-NR12-CO-N[$(C_1\text{-}C_8)$-Alkyl]$_2$, $(CH_2)_n$-NH-CO-NH-$(CH_2)_n$-CO-[O-$(C_1\text{-}C_8)$-Alkyl], $(CH_2)_n$-NH-CO-NH-$(CH_2)_q$-CO-$NH_2$, $(CH_2)_n$-NH-CO-NH-$(CH_2)_q$-COOH, $(CH_2)_n$-NH-C(=NH)-$NH_2$, $(CH_2)_n$-NH-C(=NH)-R16, $(CH_2)_n$-NH-C(=NH)-NH[$(C_1\text{-}C_8)$-Alkyl], $(CH_2)_n$-NH-C(=N-$SO_2$-$(C_1\text{-}C_8)$-Alkyl)-$NH_2$, $(CH_2)_n$-NH-C(=N-$SO_2$-$(C_1\text{-}C_8)$-Alkyl)-NH[$(C_1\text{-}C_8)$-Alkyl], $(CH_2)_n$-NH-C(=N-$SO_2$-$NH_2$)-$NH_2$, $(CH_2)_n$-NH-C(=N-$SO_2$-$NH_2$)-NH[$(C_1\text{-}C_8)$-Alkyl], $(CH_2)_n$-NH-C(=NH)-N[$(C_1\text{-}C_8)$-Alkyl]$_2$, $(CH_2)_n$-NH-C(=N-$SO_2$-$(C_1\text{-}C_8)$-Alkyl)-N[$(C_1\text{-}C_8)$-Alkyl]$_2$, $(CH_2)_n$-NH-$(CH_2)_n$-CO-O-$(CH_2)_r$-$NH_2$, $(CH_2)_n$-NH-$(CH_2)_n$ -CO-NH-[$(C_1\text{-}C_8)$-Alkyl], $(CH_2)_n$-NH-$(CH_2)_n$- CO-NH-$(CH_2)_r$-OH, $(CH_2)_n$-NH-$(CH_2)_n$ -CO-N[$(C_1\text{-}C_8)$-Alkyl]$_2$, $(CH_2)_n$-NH-$(CH_2)_n$ -CO-NH-

$[(C_3-C_8)$-Cycloalkyl], $(CH_2)_n$-NH-$C(CH_3)_2$-CO-O$(C_3-C_8)$-Cycloalkyl, $(CH_2)_n$-NH-$C(CH_3)_2$-CO-O-$(CH_2)_r$-$NH_2$, $(CH_2)_n$-NH-$C(CH_3)_2$-CO-O-$(CH_2)_n$-Aryl, $(CH_2)_n$-NH-$C(CH_3)_2$-CO-O-$(CH_2)_n$-Heteroaryl, $(CH_2)_n$-NH-$C(CH_3)_2$-CO-NH-$[(C_1-C_8)$-Alkyl], $(CH_2)_n$-NH-$C(CH_3)_2$-CO-NH-$(CH_2)_r$-OH, $(CH_2)_n$NH-$C(CH_3)_2$-CO-N$[(C_1-C_8)$-Alkyl$]_2$, $(CH_2)_n$-NH-$(CH_3)_2$-CO-NH-$[(C_3-C_8)$-Cycloalkyl], $(CH_2)_n$-NH-$C(CH_3)_2$-CO-N$[(C_3-C_8)$-Cycloalkyl$]_2$, $(CH_2)_n$-$S(O)_m$-$(C_1-C_8)$-Alkyl, $(CH_2)_n$-$S(O)_m$-$(C_3-C_8)$-Cycloalkyl, $(CH_2)_n$-$SO_2$-R16, $SO_2$-N=CH-N$(CH_3)_2$,

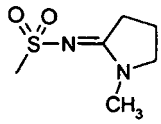,

$(CH_2)_n$-$SO_2$-NH-CO-$(C_1-C_8)$-Alkyl, $(CH_2)_n$-$SO_2$-NH-CO-$(C_3-C_8)$-Cycloalkyl, $(CH_2)_n$-$SO_2$-NH-$(C_1-C_8)$-Alkyl, $(CH_2)_n$-$SO_2$-NH-$(C_3-C_8)$-Cycloalkyl, $(CH_2)_n$-$SO_2$-N$[(C_1-C_8)$-Alkyl$]_2$, $SO_2$-NH-$(CH_2)_r$-OH, $SO_2$-NH-$(CH_2)_r$-$NH_2$, $SF_5$,
$(CH_2)_q$-CN,
$(CH_2)_n$-CO-NH-piperidin-1-yl,
$(CH_2)_n$-CO-NH-$SO_2$-NHR 12, $(CH_2)_n$-CO-NH-$SO_2$-(C1-$C_8$)-Alkyl, $(CH_2)_n$-CO-NH-$SO_2$-$(C_3-C_8)$-Cycloalkyl,
$(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O$[(C_1-C_6)$-Alkyl], wobei die Alkyl- und Cycloalkylreste mit Fluoratomen substituiert sein können und wobei die Aryl- oder Heteroarylreste mit Halogen, CN, $(C_1-C_6)$-Alkyl, $(C_3-C_6)$-Cycloalkyl, O-$(C_1-C_6)$-Alkyl, $S(O)_m$-$(C_1-C_6)$-Alkyl, $SO_2$-$NH_2$, COOH, $CONH_2$, CO-[O$(C_1-C_6)$-Alkyl], CO-$(C_1-C_6)$-Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und
wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, $(C_1-C_8)$-Alkyl oder =O substituiert sein können;

T NR23-CO-NR24, NR23-$SO_2$-NR24, $SO_2$-NR23-$SO_2$, CO-NR23-CO, NR23- C(=NR13)-NR24, NR23-C(=NR22)-NR24, CO-NR23-CR22R23, NR23-$SO_2$- CR22R24, CR22R24-$SO_2$-NR23, CR22R23-NR23-$SO_2$, $SO_2$-CR22R23-NR23, $SO_2$-NR23-CR22R23-, NR23-CR22R23-$SO_2$, CR23R24-CR23R24-CR23R24;

R11 H, $(C_1-C_8)$-Alkyl,$(C_3-C_6)$-Cycloalkyl, $(CH_2)_n$-Aryl, $(CH_2)_n$-CO-[O-$(C_1-C_6)$- Alkyl], $(CH_2)_n$-CO-[O-$(C_3-C_6)$-Cycloalkyl], $(CH_2)_n$-CO-$[(C_1-C_6)$-Alkyl], $(CH_2)_n$- CO-$[(C_3-C_6)$-Cycloalkyl], $(CH_2)_n$-CO-Aryl, $(CH_2)_n$-CO-Heteroaryl, $(CH_2)_q$-CO- $NH_2$, $(CH_2)_q$-COOH, $(CH_2)_n$-P(O)(OH)[O-$(C_1-C_4)$-Alkyl], $(CH_2)_n$-P(O)[O-$(C_1$- $C_4)$-Alkyl$]_2$, $(CH_2)_n$-P(O)(O-$CH_2$-Aryl$)_2$, $(CH_2)_n$-$P(O)(OH)_2$, $(CH_2)_n$-$SO_3H$, $(CH_2)_n$-$SO_2$-$NH_2$, $(CH_2)_n$-CO-NH-$[(C_1-C_4)$-Alkyl], $(CH_2)_n$-CO-N$[(C_1-C_4)$- Alkyl$]_2$, $(CH_2)_n$-CO-NH-$[(C_3-C_6)$-Cycloalkyl], $(C_2-C_6)$-Alkenyl-CO-O$[(C_1-C_4)$- Alkyl], $(C_2-C_6)$-Alkenyl-$CONH_2$, $(C_2-C_6)$-Alkenyl-COOH, $(C_2-C_6)$-Alkinyl-CO- O$[(C_1$-$C_4)$-Alkyl], $(C_2-C_6)$-Alkinyl-$CONH_2$, $(C_2-C_6)$-Alkinyl-COOH, $(CH_2)_n$- CR21 [(CO-O$(C_1-C_4)$-Alkyl)$]_2$, $(CH_2)_n$-CR21 $(CONH_2)_2$, $(CH_2)_n$-CR21$(COOH)_2$, $(CH_2)_n$-CR21R22-CO-O($(C_1-C_4)$-Alkyl], $(CH_2)_n$-CR21R22-$CONH_2$, $(CH_2)_n$- CR21R22-CO-NH-$[(C_1-C_4)$-Alkyl], $(CH_2)_n$-CR21R22-CO-N($(C_1-C_4)$-Alkyl$]_2$, $(CH_2)_n$-CR21R22-COOH, $(CH_2)_n$-CO-R16, $(CH_2)_n$-CO-NH-$C(CH_3)_2$-CO-O$[(C_1$- $C_8)$-Alkyl], $(CH_2)_n$-CO-NH-$C(CH_3)_2$-$CONH_2$, $(CH_2)_n$-CO-NH-$C(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, $(C_1-C_4)$-Alkyl, O-$(C_1-C_4)$-Alkyl, $S(O)_m$-$(C_1-C_4)$-Alkyl, $SO_2$-$NH_2$, COOH, $CONH_2$, CO-O$(C_1-C_4)$-Alkyl substituiert sein kann und wobei die Alkylreste mit Fluoratomen substituiert sein können;

R12 H, $(C_1-C_4)$-Alkyl, $(C_3-C_6)$-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, $(C_1-C_4)$-Alkyl, O-$(C_1-C_4)$-Alkyl, $SO_2$-$NH_2$, COOH, $CONH_2$, CO-O$(C_1-C_4)$-Alkyl substituiert sein kann und wobei die Alkylreste mit Fluoratomen substituiert sein können;

R13 H, $SO_2$-[($C_1$-$C_4$)-Alkyl], $SO_2$-[($C_3$-$C_6$)-Cyclalkyl], $SO_2$-$(CH_2)_n$-Aryl, $SO_2$-$(CH_2)_n$-Heteroaryl, wobei die Alkyl- und Cycloalkylreste mit Fluoratomen substituiert sein können und wobei der Aryl- oder Heteroarylrest mit Halogen, CN, $CF_3$, ($C_1$-$C_4$)-Alkyl, O-[($C_1$-$C_4$)-Alkyl], $S(O)_m$-[($C_1$-$C_6$)-Alkyl], $SO_2$-$NH_2$, COOH, $CONH_2$, CO- [O($C_1$-$C_4$)-Alkyl], substituiert sein kann und wobei die Alkylreste mit Fluoratomen substituiert sein können;

R15 ($C_1$-$C_6$)-Alkyl, wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[($C_1$-$C_6$)- Alkyl]piperazin-1-yl, Thiomorpholin-1,1-Dioxid-4-yl, NH-$(CH_2)_r$-OH, NH- CH$(CH_2OH)_2$, NH-C$(CH_2OH)_3$, N[($C_1$-$C_4$)-Alkyl-OH$]_2$, D-Glucamin-N-yl, N- Methyl-D-Glucamin-N-yl, NH-[($C_1$-$C_4$)-Alkyl]-COOH, NH-[($C_1$-$C_4$)-Alkyl]- $CONH_2$, N[( $C_1$-$C_4$)-Alkyl][($C_1$-$C_4$)-Alkyl]-COOH, NH-[C(H)(Aryl)]-CO-O($C_1$- $C_4$)-Alkyl, NH-[C(H)(Aryl)]-COOH, NH-[C(H)(Aryl)]-$CONH_2$, NH- [C(H)(Heteroaryl)]-CO-O($C_1$-$C_4$)-Alkyl, NH-[C(H)(Heteroaryl)]-COOH, NH- [C(H)(Heteroaryl)]-$CONH_2$, NH-[($C_3$-$C_6$)-Cycloalkyl]-CO-O($C_1$-$C_4$)-Alkyl, NH- [($C_3$-$C_6$)-Cycloalkyl]-COOH, NH-[($C_3$-$C_6$)-Cycloalkyl]-$CONH_2$, NH-($C_1$-$C_4$)-Alkyl-OH, NH-[($C_1$-$C_4$)-Alkyl]-$SO_2$-($C_1$-$C_4$)-Alkyl, NH-[($C_1$-$C_4$)-Alkyl]- $SO_3H$, NH-[($C_1$-$C_4$)-Alkyl]-$SO_2$-$NH_2$, wobei die Alkohol (OH) - Funktionen durch F ersetzt sein können und wobei der Aryl- oder Heteroarylrest mit Halogen, CN, ($C_1$-$C_4$)-Alkyl, O-($C_1$-$C_4$)-Alkyl, OH, $SO_2$-$NH_2$, COOH, $CONH_2$, CO-O($C_1$-$C_4$)-Alkyl substituiert sein kann;

R18 $(CH_2)_n$-CR25R26-CO-O($C_1$-$C_4$)-Alkyl, $(CH_2)_n$-CR25R26-CO-$NH_2$, $(CH_2)_n$- CR25R26-COOH;

R20 H, ($C_1$-$C_4$)-Alkyl, ($C_3$-$C_6$)-Cycloalkyl, Aryl, [($C_1$-$C_4$)-Alkyl]-Aryl;

R21 H, F, $CF_3$, ($C_1$-$C_4$)-Alkyl, ($C_3$-$C_6$)-Cycloalkyl, OH, O-($C_1$-$C_4$)-Alkyl, O-($C_3$-$C_6$)- Cycloalkyl, O-$(CH_2)_n$-Aryl, O-(CO)-($C_1$-$C_4$)-Alkyl, O-(CO)-($C_3$-$C_6$)-Cycloalkyl, O-(CO)-O-($C_1$-$C_4$)-Alkyl, O-(CO)-O-($C_3$-$C_6$)-Cycloalkyl, NH-(($C_1$-$C_4$)-Alkyl]- Aryl, $NH_2$, NH-($C_1$-$C_4$)-Alkyl, NH-(CO)-($C_1$-$C_4$)-Alkyl;

R22 H, $CF_3$, ($C_1$-$C_4$)-Alkyl, Aryl, [($C_1$-$C_4$)-Alkyl]-Aryl;

R23, R24 unabhängig voneinander H, ($C_1$-$C_4$)-Alkyl, ($C_3$-$C_6$)-Cycloalkyl, [($C_1$-$C_4$)-Alkyl]- [($C_3$-$C_6$)-Cycloalkyl], Aryl, [($C_1$-$C_4$)-Alkyl]-Aryl
oder R23 und R24 bilden zusammen eine -CH=CH-, -$CH_2$-$CH_2$-, -$CH_2$-$CH_2$- $CH_2$-, oder -$CH_2$-$CH_2$-$CH_2$-$CH_2$- Einheit, worin eine $CH_2$-Gruppierung durch C=O, CHF oder $CF_2$ ersetzt sein kann, und worin bis zu vier Wasserstoffatome durch einen ($C_1$-$C_4$)-Alkylrest ersetzt sein können;

R25, R26 unabhängig voneinander H, F, ($C_1$-$C_4$)-Alkyl, Aryl, [($C_1$-$C_4$)-Alkyl]-Aryl, wobei der Aryl mit Halogen, CN, OH, O-($C_1$-$C_4$)-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[($C_1$-$C_4$)-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl])phenyl)harnstoff ausgenommen ist,
sowie deren physiologisch verträgliche Salze.

Weiter bevorzugt sind Verbindungen der Formel Ia

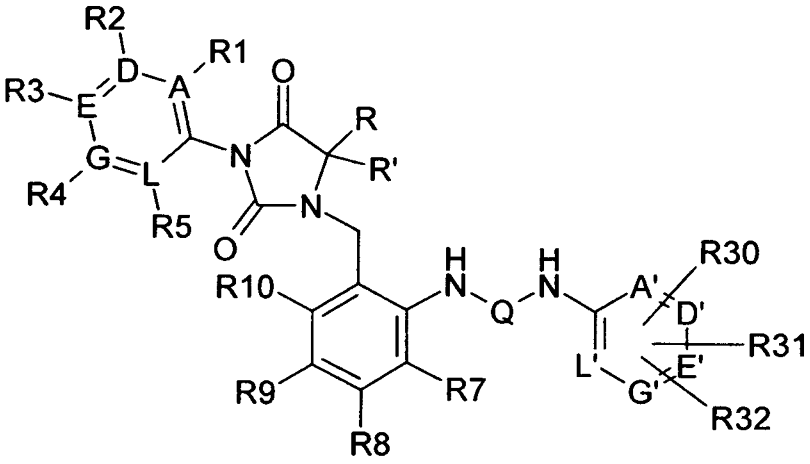

Ia

worin bedeuten

| | |
|---|---|
| R, R' | unabhängig voneinander H, Aryl, $(C_1-C_4)$-Alkyl, wobei $(C_1-C_4)$-Alkyl oder der Arylrest substituiert sein kann mit Halogen; |
| oder R und R' | bilden gemeinsam einen Ring mit drei bis acht Kohlenstoffatomen, wobei ein Kohlenstoffatom durch O, $S(O)_m$, NR13 oder NR15 ersetzt sein kann; |
| m | 0, 1, 2; |
| n | 0, 1, 2; |
| q | 1, 2; |
| r | 2, 3; |
| v | 0, 1, 2; |
| A, D, E, G, L | unabhängig voneinander C oder N, wobei bei der Bedeutung N der entsprechende Substituent R1, R2, R3, R4, R5 entfällt,<br>oder R2-D=E-R3 oder R4-G=L-R5 haben die Bedeutung S oder O und wobei der Fünf- oder Sechsring mit $-(CH_2)_3-$ oder $-(CH_2)_4-$ oder -CH=CH-CH=CH- zu einem Bicyclus anelliert sein kann; |
| Q | C=O, $SO_2$; |
| R1, R2, R3, R4, R5 | unabhängig voneinander H, F, Cl, Br, J, CN, $CF_3$, $(C_1-C_8)$-Alkyl, $(C_3-C_8)$-Cycloalkyl, $(CH_2)_n$-Aryl, $(CH_2)_n$-Heteroaryl, $OCF_3$, O-R11, NR13R15, $S(O)_m$-R12, $SO_2-NH_2$, $SO_2$-NH-[$(C_1-C_8)$-Alkyl], $SO_2$-NH-[$(C_3-C_8)$-Cycloalkyl], $SO_2$-NH-$(CH_2)_n$-Aryl, $SO_2$-NH-$(CH_2)_n$-Heteroaryl, $SO_2$-N[$(C_1-C_8)$-Alkyl]$_2$, $SO_2$- R16, $SF_5$, CO-O[$(C_1-C_8)$-Alkyl],<br>CO-O[$(C_3-C_6)$-Cycloalkyl], CO-$NH_2$, CO-NH-[$(C_1-C_4)$-Alkyl], CO-N[$(C_1-C_4)$- Alkyl]$_2$, C(=NH)-$NH_2$, C(=NH)-R16, $(CH_2)^n$-C(=$NSO_2$-R12)NH2, CO-R16, COOH, CO-$(C_1-C_4)$-Alkyl, CO-$(C_3-C_6)$-Cycloalkyl, CO-Aryl, CO-Heteroaryl, CH(OH)-Aryl, CH(OH)-Heteroaryl, CHF-Aryl, CHF-Heteroaryl, $CF_2$-Aryl, $CF_2$-Heteroaryl, $CH_2$-OH, $CH_2$-CN, $CH_2$-O-R12, $CH_2$-O-$(CH_2)_q$-COOH,<br>wobei die Alkyl- und Cycloalkylreste mit Fluoratomen substituiert sein können und wobei die Aryl- oder Heteroarylreste mit Halogen, CN, $(C_1-C_4)$-Alkyl, O- $(C_1-C_4)$-Alkyl, $OCF_3$, OH, O-$(CH_2)_n$-Aryl, $(CH_2)_n$-Aryl, $S(O)_m$-$(C_1-C_4)$-Alkyl, $SO_2-NH_2$, SH, NR12R13, NH-CO-[$(C_1-C_4)$-Alkyl], NH-CO-$(CH_2)_n$-Aryl, $(CH_2)_n$-COOH, $(CH_2)_n$-$CONH_2$, $(CH_2)_n$-CO-O$(C_1-C_4)$-Alkyl, $(CH_2)_n$-CO-$(C_1- C_4)$-Al- |

kyl substituiert sein können und wobei die Alkylreste mit Fluoratomen substituiert sein können;

R7, R8, R9, R10 unabhängig voneinander H, F, Cl, Br, J, CN, $N_3$, NC, $NO_2$, $CF_3$, $(C_1-C_8)$-Alkyl, $(C_2-C_{10})$-Alkenyl, $(C_2-C_{10})$-Alkinyl, $(C_3-C_8)$-Cycloalkyl, Aryl, Heteroaryl,
$(CH_2)_n$-CO-[O-$(C_1-C_8)$-Alkyl], $(CH_2)_n$-CO-[O-$(C_3-C_8)$-Cycloalkyl], $(CH_2)_n$-CO- [$(C_1-C_8)$-Alkyl], $(CH_2)_n$-CO-[$(C_3-C_8)$-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-$(C_1-C_6)$-Alkyl], $(CH_2)_n$-P(O)[O-$(C_1-C_6)$-Alkyl]$_2$, $(CH_2)_n$- P(O)(OH)(O-$CH_2$-Aryl), $(CH_2)_n$-P(O)(O-$CH_2$-Aryl)$_2$, $(CH_2)_n$-P(O)$(OH)_2$, $(CH_2)$n-$SO_3H$, $(CH_2)_n$-$SO_2$-$NH_2$,
$(CH_2)_n$-CO-NH-[$(C_1-C_8)$-Alkyl], $(CH_2)_n$-CO-N[$(C_1-C_8)$-Alkyl]$_2$, $(CH_2)_n$-CO-NH- [$(C_3-C_8)$-Cycloalkyl],
$(C_2-C_{10})$-Alkenyl-CO-O[$(C_1-C_6)$-Alkyl], $(C_2-C_{10})$-Alkenyl-$CONH_2$, $(C_2-C_{10})$- Alkenyl-COOH, $(C_2-C_{10})$-Alkinyl-CO-O[$(C_1-C_6)$-Alkyl], $(C_2-C_{10})$-Alkinyl- $CONH_2$, $(C_2-C_{10})$-Alkinyl-COOH,
$(CH_2)_n$-CO-R16,
$(CH_2)_n$-OH, $(CH_2)_n$-O-$(C_1-C_8)$-Alkyl, $(CH_2)_n$-O-$(C_2-C_{10})$-Alkenyl, $(CH_2)_n$-O-$(C_2$- $C_{10})$-Alkinyl, $(CH_2)_n$-O-$(C_3-C_8)$-Cycloalkyl, $(CH_2)_n$-O-$(CH_2)_q$-[$(C_3-C_8)$- Cycloalkyl], $(CH_2)_n$-O-$(CH_2)_n$-CO-[O-$(C_1-C_8)$-Alkyl], $(CH_2)_n$-O-$(CH_2)_n$-CO-[O- $(C_3-C_8)$-Cycloalkyl], $(CH_2)_n$-O-$(CH_2)_n$-CO-[$(C_1-C_8)$-Alkyl], $(CH_2)_n$-O-$(CH_2)_n$- CO-[$(C_3-C_8)$-Cycloalkyl], $(CH_2)_n$-O-$(CH_2)_n$-CO-[O-$(CH_2)_v$-Aryl], $(CH_2)_n$-O- $(CH_2)_n$-CO-[O-$(CH_2)_v$-Heteroaryl], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P(O)(OH)[O- $(C_1-C_6)$-Alkyl], $(CH_2)_n$-O-$(CH_2)_n$-P(O)[O-$(C_1-C_6)$-Alkyl]$_2$, $(CH_2)_n$-O-$(CH_2)_n$- P(O)(OH)(O-$CH_2$-Aryl), $(CH_2)_n$-O-$(CH_2)_n$-P(O)(O-$CH_2$-Aryl)$_2$, $(CH_2)_n$-O- $(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$- O-$(CH_2)_n$-CO-NH-[$(C_1-C_8)$-Alkyl], $(CH_2)_n$-O-$(CH_2)_n$-CO-N[$(C_1-C_8)$-Alkyl]$_2$, $(CH_2)_n$-O-$(CH_2)_n$-CO-NH-[$(C_3-C_8)$-Cycloalkyl], $(CH_2)_n$-O-$(CH_2)_n$-CR21R22- CO-O[$(C_1-C_6)$-Alkyl], $(CH_2)_n$-O-$(CH_2)_n$-CR21R22-CONH2, $(CH_2)_n$-O-$(CH_2)_n$- CR21R22-COOH, $(CH_2)_n$-O-$(CH_2)_n$-CO-R16, $(CH_2)_n$-O-$(CH_2)_r$-OH, $(CH_2)_n$-O- CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH- $(CH_2)_r$-OH, O-R13, $OCF_3$,
$(CH_2)_n$-$NH_2$, $(CH_2)_n$-NH-$(C_1-C_8)$-Alkyl, $(CH_2)_n$-NH-$(C_3-C_8)$-Cycloalkyl, $(CH_2)_n$-NH-$(CH_2)_n$-CO-[O-$(C_3-C_8)$-Cycloalkyl], $(CH_2)_n$-NH-$(CH_2)_n$-CO-[$(C_1$- $C_8)$-Alkyl], $(CH_2)_n$-NH-$(CH_2)_n$-CO-[$(C_3-C_8)$-Cycloalkyl], $(CH_2)_n$-NH-$(CH_2)_n$- CO-[O-$(CH_2)_v$-Aryl], $(CH_2)_n$-NH-$(CH_2)_n$-CO-[O-$(CH_2)_v$-Heteroaryl], $(CH_2)_n$- NH-$(CH_2)_q$-CO-NH-CN,
$(CH_2)_n$-NH-$(CH_2)_n$-P(O)$(OH)_2$,
$(CH_2)_n$-NH-(CH2)$_n$-$SO_3H$,
$(CH_2)_n$-NH-$(CH_2)_n$-$SO_2$-$NH_2$,
$(CH_2)_n$-NH-$(CH_2)_n$-CR21R22-CO-O[$(C_1-C_6)$-Alkyl], $(CH_2)_n$-NH-$(CH_2)_n$- CR21R22-$CONH_2$, $(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[$(C_1-C_8)$-Alkyl], $(CH_2)_n$-NH- $(CH_2)_n$-$SO_2$-[$(C_3-C_8)$- Cycloalkyl], $(CH_2)_n$-NH-$SO_2$-$(CH_2)_n$-$NH_2$, $(CH_2)_n$-NH-$SO_2$-$(CH_2)_n$-NH-$(C_1-C_8)$- Alkyl, $(CH_2)_n$-NH-$SO_2$-$(CH_2)_n$-NH-$(C_3-C_8)$-Cycloalkyl, $(CH_2)_n$-NH-$SO_2$-$(CH_2)_n$- N[$(C_1-C_8)$-Alkyl]$_2$,
$(CH_2)_n$-NH-CN, $(CH_2)_n$-NH-$SO_2$-R16,
$(CH_2)_n$-NR12-CO-NH-$(C_1-C_8)$-Alkyl, $(CH_2)_n$-NR12-CO-NH-$(C_3-C_8)$- Cycloalkyl, $(CH_2)_n$-NR12-CO-NH2, $(CH_2)_n$-NR12-CO-NH-$SO_2$-$(C_1-C_8)$-Alkyl, $(CH_2)_n$-NR12-CO-NH-$SO_2$-$(C_3-C_8)$-Cycloalkyl, $(CH_2)_n$-NR12-CO-N[$(C_1-C_8)$- Alkyl]$_2$, $(CH_2)_n$-NH-CO-NH-$(CH_2)_n$-CO-[O-$(C_1-C_8)$-Alkyl], $(CH_2)_n$-NH-CO- NH-$(CH_2)_q$-CO-$NH_2$, $(CH_2)_n$-NH-CO-NH-$(CH_2)_q$-COOH, $(CH_2)_n$-NH-C(=NH)- $NH_2$, $(CH_2)_n$-NH-C(=NH)-R16, $(CH_2)$,-NH-C(=NH)-NH[$(C_1-C_8)$-Alkyl], $(CH_2)_n$-NH-C(=N-$SO_2$-$(C_1-C_8)$-Alkyl)-$NH_2$, $(CH_2)_n$-NH-C(=N-$SO_2$-$(C_1-C_8)$- Alkyl)-NH[$(C_1-C_8)$-Alkyl], $(CH_2)_n$-NH-C(=N-$SO_2$-$NH_2$)-$NH_2$, $(CH_2)_n$-NH- C(=N-$SO_2$-$NH_2$)-NH[$(C_1-C_8)$-Alkyl], $(CH_2)_n$-NH-C(=NH)-N[$(C_1-C_8)$-Alkyl]$_2$, $(CH_2)_n$-NH-C(=N-$SO_2$-$(C_1-C_8)$-Alkyl)-N[$(C_1-C_8)$-Alkyl]$_2$, $(CH_2)_n$-NH-$(CH_2)_n$-CO-O-$(CH_2)_r$-$NH_2$, $(CH_2)_n$-NH-$(CH_2)_n$ -CO-NH-[$(C_1-C_8)$-Alkyl], $(CH_2)_n$-NH- $(CH_2)_n$ -CO-NH-$(CH_2)_r$-OH, $(CH_2)_n$-NH-$(CH_2)_n$ -CO-N[$(C_1-C_8)$-Alkyl]$_2$, $(CH_2)_n$- NH-$(CH_2)_n$ -CO-NH-[$(C_3-C_8)$-Cycloalkyl], $(CH_2)_n$-NH-C$(CH_3)_2$-CO-O$(C_3-C_8)$- Cycloalkyl, $(CH_2)_n$-NH-C$(CH_3)_2$-CO-O-$(CH_2)_r$-$NH_2$, $(CH_2)_n$-NH-C$(CH_3)_2$-CO- O-$(CH_2)_n$-Aryl, $(CH_2)_n$-NH-C$(CH_3)_2$-CO-O-$(CH_2)_n$-Heteroaryl, $(CH_2)_n$-NH- C$(CH_3)_2$-CO-NH-[$(C_1-C_8)$-Alkyl], $(CH_2)_n$-NH-C$(CH_3)_2$-CO-NH-$(CH_2)_r$-OH, $(CH_2)_n$-NH-C$(CH_3)_2$-CO-N[$(C_1-C_8)$-Alkyl]$_2$, $(CH_2)_n$-NH-$(CH_3)_2$-CO-NH-[$(C_3$- $C_8)$-Cycloalkyl], $(CH_2)_n$-NH-C$(CH_3)_2$-CO-N[$(C_3-C_8)$-Cycloalkyl]$_2$, $(CH_2)_n$-$S(O)_m$-$(C_1-C_8)$-Alkyl, $(CH_2)_n$-$S(O)_m$-$(C_3-C_8)$-Cycloalkyl, $(CH_2)_n$-$SO_2$- R16, $SO_2$-N=CH-N$(CH_3)_2$,

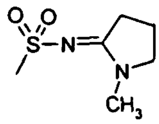,

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)-Alkyl, $(CH_2)_n$-$SO_2$-NH-($C_3$-$C_8$)-Cycloalkyl, $(CH_2)_n$-$SO_2$-N[($C_1$-$C_8$)-Alkyl]$_2$, $SO_2$-NH-$(CH_2)_r$-OH, SO2-NH-$(CH_2)_r$-$NH_2$, $SF_5$,
$(CH_2)_q$-CN,
$(CH_2)_n$-CO-NH-piperidin-1-yl,
$(CH_2)_n$-CO-NH-$SO_2$-NHR12, $(CH_2)_n$-CO-NH-$SO_2$-($C_1$-$C_8$)-Alkyl, $(CH_2)_n$-CO- NH-$SO_2$-($C_3$-$C_8$)-Cycloalkyl,
$(CH_2)_n$-CHO, $(CH_2)_n$-C(=NH)$NH_2$, $(CH_2)_n$-C(=NH)NHOH, $(CH_2)_n$- C(=NH)(R16), $(CH_2)_n$-C(=NR13)NHR12, $(CH_2)_n$-C(=NR12)NR12R13, $(CH_2)_n$- C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, $S(O)_m$-($C_1$-$C_6$)-Alkyl, $SO_2$-$NH_2$, COOH, $CONH_2$, CO-[O($C_1$-$C_6$)-Alkyl], CO-($C_1$-$C_6$)- Alkyl substituiert sein können und wobei die Alkylreste mit Fluoratomen substituiert sein können;

wobei eines der drei Restepaare R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, ($C_1$-$C_8$)-Alkyl oder =O substituiert sein können;

R11 H, ($C_1$-$C_8$)-Alkyl,($C_3$-$C_6$)-Cycloalkyl, $(CH_2)_n$-Aryl, $(CH_2)_n$-CO-[O-($C_1$-$C_6$)- Alkyl], $(CH_2)_n$-CO-[O-($C_3$-$C_6$)-Cycloalkyl], $(CH_2)_n$-CO-[($C_1$-$C_6$)-Alkyl], $(CH_2)_n$- CO-[($C_3$-$C_6$)-Cycloalkyl], $(CH_2)_n$-CO-Aryl, $(CH_2)_n$-CO-Heteroaryl, $(CH_2)_q$-CO- $NH_2$, $(CH_2)_q$-COOH, $(CH_2)_n$-P(O)(OH)[O-($C_1$-$C_4$)-Alkyl], $(CH_2)_n$-P(O)[O-($C_1$- $C_4$)-Alkyl]$_2$, $(CH_2)_n$-P(O)(O-$CH_2$-Aryl)$_2$, $(CH_2)_n$-P(O)$(OH)_2$, $(CH_2)_n$-$SO_3H$, $(CH_2)_n$-$SO_2$-$NH_2$, $(CH_2)_n$-CO-NH-[($C_1$-$C_4$)-Alkyl], $(CH_2)_n$-CO-N[($C_1$-$C_4$)- Alkyl]$_2$, $(CH_2)_n$-CO-NH-[($C_3$-$C_6$)-Cycloalkyl], ($C_2$-$C_6$)-Alkenyl-CO-O[($C_1$-$C_4$)- Alkyl], ($C_2$-$C_6$)-Alkenyl-$CONH_2$, ($C_2$-$C_6$)-Alkenyl-COOH, ($C_2$-$C_6$)-Alkinyl-CO- O[($C_1$-$C_4$)-Alkyl], ($C_2$-$C_6$)-Alkinyl-$CONH_2$, ($C_2$-$C_6$)-Alkinyl-COOH, $(CH_2)_n$- CR21[(CO-O($C_1$-$C_4$)-Alkyl)]$_2$, $(CH_2)_n$-CR21$(CONH_2)_2$, $(CH_2)_n$-CR21$(COOH)_2$, $(CH_2)_n$-CR21R22-CO-O[($C_1$-$C_4$)-Alkyl], $(CH_2)_n$-CR21R22-$CONH_2$, $(CH_2)_n$- CR21R22-CO-NH-(($C_1$-$C_4$)-Alkyl], $(CH_2)_n$-CR21R22-CO-N[($C_1$-$C_4$)-Alkyl]$_2$, $(CH_2)_n$-CR21R22-COOH, $(CH_2)_n$-CO-R16, $(CH_2)_n$-CO-NH-$C(CH_3)_2$-CO-O[($C_1$- $C_8$)-Alkyl], $(CH_2)_n$-CO-NH-$C(CH_3)_2$-$CONH_2$, $(CH_2)_n$-CO-NH-$C(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, ($C_1$-$C_4$)-Alkyl, O-($C_1$-$C_4$)-Alkyl, $S(O)_m$-($C_1$-$C_4$)-Alkyl, $SO_2$-$NH_2$, COOH, $CONH_2$, CO-O($C_1$-$C_4$)-Alkyl substituiert sein kann und wobei die Alkylreste mit Fluoratomen substituiert sein können;

R12 H, ($C_1$-$C_4$)-Alkyl, ($C_3$-$C_6$)-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, ($C_1$-$C_4$)-Alkyl, O-($C_1$-$C_4$)-Alkyl, $SO_2$-$NH_2$, COOH, $CONH_2$, CO-O($C_1$-$C_4$)-Alkyl substituiert sein kann und wobei die Alkylreste mit Fluoratomen substituiert sein können;

R13 H, $SO_2$-[($C_1$-$C_4$)-Alkyl], $SO_2$-[($C_3$-$C_6$)-Cycloalkyl], $SO_2$-$(CH_2)_n$-Aryl, $SO_2$-$(CH_2)_n$-Heteroaryl, wobei die Alkyl- und Cycloalkylreste mit Fluoratomen substituiert sein können und wobei der Aryl- oder Heteroarylrest mit Halogen, CN, $CF_3$, ($C_1$-$C_4$)-Alkyl, O-(($C_1$-$C_4$)-Alkyl], $S(O)_m$-(($C_1$-$C_6$)-Alkyl], $SO_2$-$NH_2$, COOH, $CONH_2$, CO- [O($C_1$-$C_4$)-Alkyl], substituiert sein kann und wobei die Alkylreste mit Fluoratomen substituiert sein können;

R15 ($C_1$-$C_6$)-Alkyl,
wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[($C_1$-$C_6$)- Alkyl]piperazin-1-yl, Thiomorpholin-1,1-Dioxid-4-yl, NH-$(CH_2)_r$-OH, NH- CH$(CH_2OH)_2$, NH-C$(CH_2OH)_3$, N(($C_1$-$C_4$)-Alkyl-OH]$_2$, D-Glucamin-N-yl, N- Methyl-D-Glucamin-N-yl, NH-[($C_1$-$C_4$)-Alkyl]-COOH, NH-[($C_1$-$C_4$)-Alkyl]- $CONH_2$, N[($C_1$-$C_4$)-Al-

kyl][($C_1$-$C_4$)-Alkyl]-COOH, NH-[C(H)(Aryl)]-CO-O($C_1$- $C_4$)-Alkyl, NH-[C(H)(Aryl)]-COOH, NH-[C(H)(Aryl)]-$CONH_2$, NH- [C(H)(Heteroaryl)]-CO-O($C_1$-$C_4$)-Alkyl, NH-[C(H)(Heteroaryl)]-COOH, NH-[C(H)(Heteroaryl)]-$CONH_2$, NH-[($C_3$-$C_6$)-Cycloalkyl]-CO-O($C_1$-$C_4$)-Alkyl, NH- [($C_3$-$C_6$)-Cycloalkyl]-COOH, NH-[($C_3$-$C_6$)-Cycloalkyl]-$CONH_2$, NH-($C_1$-$C_4$)- Alkyl-OH, NH-[($C_1$-$C_4$)-Alkyl]-$SO_2$-($C_1$-$C_4$)-Alkyl, NH-[( $C_1$-$C_4$)-Alkyl]- $SO_3H$, NH-[($C_1$-$C_4$)-Alkyl]-$SO_2$-$NH_2$,
wobei die Alkohol (OH) - Funktionen durch F ersetzt sein können und wobei der Aryl- oder Heteroarylrest mit Halogen, CN, ($C_1$-$C_4$)-Alkyl, O-($C_1$-$C_4$)-Alkyl, OH, $SO_2$-$NH_2$, COOH, $CONH_2$, CO-O($C_1$-$C_4$)-Alkyl substituiert sein kann;

R18 ($CH_2$)$_n$-CR25R26-CO-O($C_1$-$C_4$)-Alkyl, ($CH_2$)$_n$-CR25R26-CO-$NH_2$, ($CH_2$)$_n$- CR25R26-COOH;

R21 H, F, $CF_3$, ($C_1$-$C_4$)-Alkyl, ($C_3$-$C_6$)-Cycloalkyl, OH, O-($C_1$-$C_4$)-Alkyl, O-($C_3$-$C_6$)- Cycloalkyl, O-($CH_2$)$_n$-Aryl, O-(CO)-($C_1$-$C_4$)-Alkyl, O-(CO)-($C_3$-$C_6$)-Cycloalkyl, O-(CO)-O-($C_1$-$C_4$)-Alkyl, O-(CO)-O-($C_3$-$C_6$)-Cycloalkyl, NH-[($C_1$-$C_4$)-Alkyl]- Aryl, $NH_2$, NH-($C_1$-$C_4$)-Alkyl, NH-(CO)-($C_1$-$C_4$)-Alkyl;

R22 H, $CF_3$, ($C_1$-$C_4$)-Alkyl, Aryl, [($C_1$-$C_4$)-Alkyl]-Aryl;

R25, R26 unabhängig voneinander H, F, ($C_1$-$C_4$)-Alkyl, Aryl, [($C_1$-$C_4$)-Alkyl]-Aryl, wobei der Aryl mit Halogen, CN, OH, O-($C_1$-$C_4$)-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, S(O)$_m$, NH, N[($C_1$-$C_4$)-Alkyl] oder CO ersetzt sein kann;

A', D', E', G', L' unabhängig voneinander CH oder N, wobei bei der Bedeutung N der entsprechende Substituent R30, R31 oder R32 entfällt, wenn er an das Stickstoffatom gebunden wäre;

R30, R31, R32 unabhängig voneinander R11, F, Cl, Br, J, CN, $CF_3$, ($CH_2$)$_n$-O-R11, O- R13, $OCF_3$, ($CH_2$)$_n$-NH-R11, ($CH_2$)$_n$-N[($CH_2$)$_q$-CO-O($C_1$-$C_4$)-Alkyl]$_2$, ($CH_2$)$_n$- N[($CH_2$)$_q$-COOH]$_2$, ($CH_2$)$_n$-N[($CH_2$)$_q$-$CONH_2$]$_2$, ($CH_2$)$_n$-NH-R13, ($CH_2$)$_n$- N(R13)$_2$, ($CH_2$)$_n$-NH-$SO_2$-R16, ($CH_2$)$_n$-NH-($CH_2$)$_n$-$SO_2$-R12, ($CH_2$)$_n$-NR12-CO-R16, ($CH_2$)$_n$-NR12-CO-NR12R13, ($CH_2$)$_n$-NR12-CO-N(R12)$_2$, ($CH_2$)$_n$- NR12-CO-NHR11, ($CH_2$)$_n$-NH-C(=NH)-$NH_2$, ($CH_2$)$_n$-NH-C(=NH)-R16, ($CH_2$)$_n$-NH-C(=NH)-NHR12, ($CH_2$)$_n$-NH-($CH_2$)$_n$ -CO-NH-[($C_1$-$C_4$)-Alkyl], ($CH_2$)$_n$-NH-($CH_2$)$_n$ -CO-N[($C_1$-$C_4$)-Alkyl]$_2$, ($CH_2$)$_n$-NH-C($CH_3$)$_2$-CO-O($C_1$-$C_4$)- Alkyl, ($CH_2$)$_n$NH-C($CH_3$)$_2$-CO-O($C_3$-$C_6$)-Cycloalkyl, ($CH_2$)$_n$-NH-C($CH_3$)$_2$-CO- O-($CH_2$)$_r$-$NH_2$, ($CH_2$)$_n$-NH-C($CH_3$)$_2$-CO-O-($CH_2$)$_n$-Aryl, ($CH_2$)$_n$-NH-C($CH_3$)$_2$- CO-O-($CH_2$)$_n$-Heteroaryl, ($CH_2$)$_n$-NH-C($CH_3$)$_2$-CO-$NH_2$, ($CH_2$)$_n$-NH-C($CH_3$)$_2$- CO-NH-[($C_1$-$C_4$)-Alkyl], ($CH_2$)$_n$-NH-C($CH_3$)$_2$-CO-N[($C_1$-$C_4$)-Alkyl]$_2$, ($CH_2$)$_n$- NH-C($CH_3$)$_2$-COOH, S(O)$_m$-R12, $SO_2$-R16, $SO_2$-N=CH-N($CH_3$)$_2$, $SO_2$-NH-CO- R12, $SO_2$-NHR12, $SO_2$-N[($C_1$-$C_4$)-Alkyl]$_2$, $SF_5$, COOH, CO-$NH_2$, ($CH_2$)$_q$-CN, ($CH_2$)$_n$-CO-NH-piperidin-1-yl, ($CH_2$)$_n$-CO-NH-$SO_2$-NHR12, ($CH_2$)$_n$-CO-NH- $SO_2$-R18, ($CH_2$)$_n$-C(=NH)-NHOH, ($CH_2$)$_n$-C(=NR13)NHR12, ($CH_2$)$_n$- C(=NR12)NR12R13, ($CH_2$)$_n$-C(=N$SO_2$-R12)$NH_2$, ($CH_2$)$_n$-P(O)(OH)[O-($C_1$-$C_4$)- Alkyl], ($CH_2$)$_n$-P(O)[O-($C_1$-$C_4$)-Alkyl]$_2$, ($CH_2$)$_n$-P(O)(O-$CH_2$-Aryl)$_2$, ($CH_2$)$_n$ P(O)(OH)$_2$, wobei die Alkyl- und Cycloalkylreste mit Fluoratomen substituiert sein können und wobei die Aryl- oder Heteroarylreste mit Halogen, CN, ($C_1$- $C_4$)-Alkyl, O-($C_1$-$C_4$)-Alkyl, S(O)$_m$-($C_1$-$C_4$)-Alkyl, $SO_2$-$NH_2$, COOH, $CONH_2$, CO-O($C_1$-$C_4$)-Alkyl substituiert sein können und wobei die Alkylreste mit Fluoratomen substituiert sein können;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

Weiter bevorzugt sind Verbindungen der Formel Ia, worin bedeuten

R, R' ($C_1$-$C_4$)-Alkyl;

oder R und R' bilden gemeinsam einen Ring mit drei bis acht Kohlenstoffatomen;

m 0, 1, 2;

n 0, 1, 2;

A, D, E, G, L C;

| | |
|---|---|
| Q | C=O, $SO_2$; |
| R1, R2, R3, R4, R5 | unabhängig voneinander H, F, Cl, Br, CN, $CF_3$, $(C_1-C_8)$-Cycloalkyl, O- Phenyl; |
| R7, R8, R9, R10 | unabhängig voneinander H, F, Cl, Br, $CF_3$, $-OCH_3$; |
| A', E' | unabhängig voneinander CH oder N, wobei bei der Bedeutung N der entsprechende Substituent R30, R31 oder R32 dann entfällt, wenn er an das Stickstoffatom gebunden wäre, |
| R30, R31, R32 | unabhängig voneinander H, F, Cl, Br, $CF_3$, OH, $NO_2$, $NH_2$, $CONH_2$, COOH, $-COO-(C_1-C_8)$-Alkyl, $(CH_2)_n-P(O)(OH)[O-(C_1-C_4)$-Alkyl], $(CH_2)_n-P(O)[O-(C_1-C_4)$-Alkyl$]_2$, $(CH_2)_n-P(O)(OH)_2$, $S(O)_m-(C_1-C_4)$- Alkyl, $S(O)_m-(CH_2)$-COOH, $S(O)_m-(CH_2)-COO-(C_1-C_4)$-Alkyl, $SO_2$-Cl, $SO_2-NH_2$, $S0_3H$; |

sowie deren physiologisch verträgliche Salze.

Weiter bevorzugt sind Verbindungen der Formel Ia, worin bedeuten

| | |
|---|---|
| R, R' | $(C_1-C_4)$-Alkyl; |
| oder R und R' | bilden gemeinsam einen Ring mit drei bis acht Kohlenstoffatomen; |
| m | 0, 1, 2; |
| n | 0, 1, 2; |
| A, D, E, G, L | C; |
| Q | C=O, $SO_2$; |
| R1, R2, R3, R4, R5 | unabhängig voneinander H, F, Cl, Br, CN, $CF_3$, $(C_1-C_8)$-Cycloalkyl, O- Phenyl; |
| R7, R8, R9, R10 | unabhängig voneinander H, F, Cl, Br; |
| A', E' | unabhängig voneinander CH oder N, wobei bei der Bedeutung N der entsprechende Substituent R30, R31 oder R32 dann entfällt, wenn er an das Stickstoffatom gebunden wäre, |
| R30, R31, R32 | unabhängig voneinander H, F, Cl, Br, $CF_3$, OH, $NO_2$, $NH_2$, $CONH_2$, COOH , $-COO-(C_1-C_8)$-Alkyl, $(CH_2)_n-P(O)(OH)[O-(C_1-C_4)$-Alkyl], $(CH_2)_n-P(O)[O-(C_1-C_4)$-Alkyl$]_2$, $(CH_2)_n-P(O)(OH)_2$, $S(O)_m-(C_1-C_4)$- Alkyl, $S(O)_m-(CH_2)$-COOH, $S(O)_m-(CH_2)-COO-(C_1-C_4)$-Alkyl, $SO_2$-Cl, $SO_2-NH_2$, $SO_3H$; |

sowie deren physiologisch verträgliche Salze.

In einer Ausführungsform sind Verbindungen der Formel I bevorzugt, in denen p gleich 1 ist.

In einer Ausführungsform sind Verbindungen der Formel I bevorzugt, in denen T gleich -NH-CO-NH- ist.

In einer Ausführungsform sind Verbindungen der Formel I bevorzugt, in denen T gleich $-NH-SO_2-NH-$ ist.

In einer Ausführungsform sind Verbindungen der Formel I bevorzugt, in denen R und R' gleich Methyl ist.

In einer Ausführungsform sind Verbindungen der Formel I bevorzugt, in denen A, D, E, G und L gleich substituiertes oder unsubstituiertes C (Kohlenstoff) sind.

In einer Ausführungsform sind Verbindungen der Formel I bevorzugt, in denen einer der Reste R1, R2, R3, R4 und R5 ungleich H ist.

In einer Ausführungsform sind Verbindungen der Formel I bevorzugt, in denen zwei der Reste R1, R2, R3, R4 und R5 ungleich H sind.

In einer Ausführungsform sind Verbindungen der Formel I bevorzugt, in denen zwei der Reste R3 gleich CN oder F ist.

In einer Ausführungsform sind Verbindungen der Formel I bevorzugt, in denen R4 gleich $CF_3$ oder Cyclopropyl ist.

In einer Ausführungsform sind Verbindungen der Formel Ia bevorzugt, in denen einer der Reste R30, R31 und 32 ungleich H ist.

Können Reste oder Substituenten (wie z.B. R12) mehrfach in den Verbindungen der Formel I auftreten, so können sie alle unabhängig voneinander die angegebenen Bedeutungen haben und gleich oder verschieden sein.

Gegenstand der Erfindung sind weiterhin sowohl Stereoisomerengemische der Formel I als auch die reinen Stereoisomere der Formel I, sowie Diastereoisomerengemische der Formel I als auch die reinen Diastereoisomere. Die Trennung der Gemische erfolgt z. B. auf chromatographischem Weg.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt z. B. auf chromatographischem Weg.

Die Alkylreste in den Substituenten R1 bis R26 und R und R' können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstofflkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Cycloalkylrest wird ein einen oder mehre Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylreste können ein oder mehrfach mit geeigneten Gruppen wie oben beschrieben substituiert sein.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.
Die Arylreste können ein oder mehrfach mit geeigneten Gruppen wie oben beschrieben substituiert sein.

Unter Heteroarylrest werden aromatische Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heteroarylrest mit Benzolkernen kondensiert ist. Ebenso fallen darunter Systeme, bei welchen eine oder mehrere CH-Gruppe(n) durch C=O oder C=S, vorzugsweise C=O, ersetzt ist (sind).

Geeignete Heteroarylreste sind z.B. Furyl, Imidazolyl, Benzimidazolyl, Benzothiazolyl, Indolyl, Indolinyl, Pyrimidinyl, Pyridyl, Pyrazinyl, Pyrrolyl, Pyrazolyl, Thiazolyl, Oxazolyl, Isoxazolyl, Thienyl, 1,2,3-Triazolyl, 1,2,4-Triazolyl, Tetrazolyl, Isoxazolyl, Pyridazinyl, 1,3,5-Triazinyl, 1,2,4-Triazinyl; das 2H-Pyridazin-3-on-, Dihydropyridazin-3,6-dion-, Imidazolidin-2-on-, 1,3-Dihydro-imidazol-2-on-, Imidazolidin-2,5-dion-, Chinolin-, Isochinolin-, Chinoxalin-, Chinazolin-, Benzo[1,3]dioxol-, 2,3-Dihydro-benzo[1,4]dioxin-, 4H-Benzo[1,3]dioxin- oder 3,4-Dihydro-2H-benzo[b][1,4]dioxepin-System.

Die Verknüpfung mit den Heteroarylresten kann an jedem der dafür in Frage kommenden Atome erfolgen; so kann z. B. Pyridyl sowohl für 2-, 3- als auch 4-Pyridyl stehen; Thienyl sowohl für 2- als auch 3-Thienyl stehen; Furyl sowohl für 2- als auch 3-Furyl stehen.

Umfasst sind weiterhin die entsprechenden N-Oxide dieser Verbindungen, also z.B. 1-Oxy-2-, 3- oder 4-pyridyl.

Die Heteroarylreste können ein- oder mehrfach mit geeigneten Gruppen wie oben beschrieben substituiert sein.

Die Erfindung umfasst auch Solvate oder Hydrate der Verbindungen der Formel I.

Die Verbindungen der Formel I stellen Cannabinoid 1 Rezeptor (CB1R) Modulatoren dar und sind als solche beim Menschen und bei Tieren zur Behandlung oder zur Verhütung von Krankheiten geeignet, die auf einer Störung des Endocannabinoid-systems beruhen.
Zum Beispiel, und nicht einschränkend, sind die Verbindungen der Formel I als psychotrope Medikamente nützlich, insbesondere zur Behandlung psychiatrischer Störungen, darunter Angstzustände, Depressionen, Gemütsstörungen, Schlaflosigkeit, Delirien, Zwangsneurosen, generelle Psychosen, Schizophrenie, Defizit der Aufmerksamkeit und Hyperaktivität (ADHS) bei hyperkinetischen Kindern, sowie zur Behandlung von Störungen in Zusammenhang mit dem Gebrauch psychotroper Substanzen, insbesondere in dem Fall eines Missbrauchs einer Substanz und/oder einer Abhängigkeit von einer solchen Substanz, darunter Alkoholabhängigkeit und Nikotinabhängigkeit aber auch Abhängigkeit von Kokain, Methamphetamin und Heroin (siehe z.B. Behavioural Pharmacology 2005, 16:275-296). Übersichten über CB1R-vermittelte therapeutische Eingriffsmöglichkeiten finden sich z. B. in Ken Mackie: Annu. Rev. Pharmacol. Toxicol. 46, 101-122 (2006), S. C. Black: Curr. Opin. Investig. Drugs 5, 389-394 (2004), V. Di Marzio et al.: Nat. Rev. Drug Discov. 3, 771-784 (2004), B. Le Foll et al.: J. Pharmacol. Exp. Ther. 312, 875-883 (2005) oder L. Walter et al.: Br. J. Pharmacol. 141, 775-785 (2004).
Die erfindungsgemäßen Verbindungen der Formel I können als Medikamente zur Behandlung von Migräne, Stress, Krankheiten psychosomatischen Ursprungs, Panikattackenkrisen, Epilepsie, Bewegungsstörungen, insbesondere Dyskinesien oder Parkinsonsche Krankheit, Zittern und Dystonie verwendet werden.
Die erfindungsgemäßen Verbindungen der Formel I können weiterhin auch als Medikamente zur Behandlung von Gedächtnisstörungen, geistiger Defekte, insbesondere zur Behandlung der Altersdemenzen, der Alzheimer'schen Krankheit sowie zur Behandlung verminderter Aufmerksamkeit oder Wachsamkeit verwendet werden.
Ferner können die Verbindungen der Formel I als Neuroprotektoren, zur Behandlung von Ischämie, Schädelverletzungen und Behandlung neurodegenerativer Krankheiten, darunter Chorea, Chorea Huntington, Tourette-Syndrom, verwendet werden.
Die erfindungsgemäßen Verbindungen der Formel I können ferner als Medikamente bei der Schmerzbehandlung verwendet werden; dazu zählen neuropathische Schmerzen, akute periphere Schmerzen, chronische Schmerzen entzündlicher Herkunft.
Die erfindungsgemäßen Verbindungen der Formel I können weiterhin als Medikamente zur Behandlung von Essstörungen (z. B. zwanghafte Essanfälle (binge eating disorder), Anorexie und Bulimie), zur Behandlung der Sucht nach Süßigkeiten, Kohlenhydraten, Drogen, Alkohol oder anderen suchterzeugenden Substanzen dienen.
Die erfindungsgemäßen Verbindungen der Formel I sind besonders geeignet zur Behandlung der Adipositas oder der Bulimie sowie zur Behandlung von Diabetes Typ II wie auch zur Behandlung von Dyslipidämien und des metabolischen Syndroms. Die erfindungsgemäßen Verbindungen der Formel I sind daher zur Behandlung der Adipositas und der Gefahren in Zusammenhang mit Adipositas, insbesondere der kardiovaskulären Gefahren, nützlich.

Ferner können die erfindungsgemäßen Verbindungen der Formel I als Medikamente zur Behandlung gastrointestinaler Störungen, zur Behandlung von Durchfällen, von Magen-Darmgeschwüren, von Erbrechen, von Blasenleiden und Störungen des Wasserlassens, von Störungen endokrinen Ursprungs, von kardiovaskulären Problemen, von niedrigem Blutdruck, des hämorrhagischen Schocks, des septischen Schocks, chronischer Leberzirrhose, Lebersteatose, der nicht alkoholischen Steatohepatitis, von Asthma, des Raynaudschen Syndroms, des Glaukoms, von Fruchtbarkeitsbeschwerden, Schwangerschaftsunterbrechung, Frühgeburt, Entzündungserscheinungen, Krankheiten des Immunsystems, insbesondere autoimmun- und neuroinflammatorische, wie zum Beispiel rheumatische Gelenkentzündung, reaktive Arthritis, von Krankheiten, die zu Demyelinisation führen, der multiplen Sklerose, von Infektionskrankheiten und viralen Erkrankungen, wie zum Beispiel von Enzephalitis, ischämischem Schlaganfall sowie als Medikamente zur Krebschemotherapie, zur Behandlung des Guillain-Barré-Syndroms und zur Behandlung der Osteoporose verwendet werden.
Die erfindungsgemäßen Verbindungen der Formel I können weiterhin auch als Medikamente zur Behandlung des Syndroms der polycystischen Ovarien (PCOS, polycystic ovary syndrome) Verwendung finden.

Gemäß der vorliegenden Erfindung sind die Verbindungen der Formel I besonders nützlich zur Behandlung psychotischer Beschwerden, insbesondere der Schizophrenie, verminderter Aufmerksamkeit und Hyperaktivität (ADHS) bei hyperkinetischen Kindern, zur Behandlung von Essstörungen und der Adipositas, zur Behandlung des Diabetes Typ II, zur Behandlung von Gedächtnisdefiziten und kognitiven Defiziten, zur Behandlung der Alkoholsucht, der Nikotinsucht, das heißt für die Alkohol- und Tabakentwöhnung.

Ganz besonders nützlich sind die erfindungsgemäßen Verbindungen der Formel I zur Behandlung und Verhütung von Essstörungen Appetitstörungen, metabolischen Störungen, gastrointestinalen Störungen, Entzündungserscheinungen, Erkrankungen des Immunsystems, psychotischen Störungen, der Alkoholsucht und der Nikotinsucht.

Gemäß einem ihrer Aspekte bezieht sich die Erfindung auf den Gebrauch einer Verbindung der Formel I, ihrer pharmazeutisch akzeptablen Salze und deren Solvate oder Hydrate zur Behandlung der oben angegebenen Störungen und Erkrankungen.

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können

beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit

kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:

Alle Antidiabetika, die in der Roten Liste 2007, Kapitel 12 genannt sind; alle Abmagerungsmittel/Appetitzügler, die in der Roten Liste 2007, Kapitel 1 genannt sind; alle Diuretika, die in der Roten Liste 2007, Kapitel 36 genannt sind; alle Lipidsenker, die in der Roten Liste 2007, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen. Die meisten der nachfolgend aufgeführten Wirkstoffe sind in USP Dictionary of USAN and International Drug Names, US Pharmacopeia, Rockville 2006, offenbart.

Antidiabetika umfassen Insulin und Insulinderivate, wie z.B. Lantus® (siehe www.lantus.com) oder HMR 1964 oder Levemir® (insulin detemir), Humalog(R) (Insulin Lispro), Humulin(R), VIAject™, SuliXen(R) oder solche, wie sie in WO2005005477 (Novo Nordisk) beschrieben sind, schnell wirkende Insuline (siehe US 6,221,633), inhalierbare Insuline, wie z. B. Exubera ®, Nasulin™, oder orale Insuline, wie z. B. IN-105 (Nobex) oder Oral-lyn ™ (Generex Biotechnology) oder Technosphere(R) Insulin (MannKind) oder Cobalamin™ orales Insulin oder Insuline, wie sie in WO2007128815, WO2007128817, WO2008034881, WO2008049711 beschrieben sind oder Insuline, die transdermal verabreicht werden können;

GLP-1-Derivate und GLP-1 Agonisten wie z.B. Exenatide oder spezielle Zubereitungen davon, wie sie z.B. in WO2008061355 beschrieben sind, Liraglutide, Taspoglutide (R-1583), Albiglutide, Lixisenatide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), AVE-0010, BIM-51077 (R-1583, ITM-077), PC-DAC:Exendin-4 (ein Exendin-4 Analogon, welches kovalent an rekombinantes menschliches Albumin gebunden ist), CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1 (GLP-1 gebunden an einen Nanocarrier), Agonisten wie sie z.B. bei D. Chen et al., Proc. Natl. Acad. Sci. USA 104 (2007) 943 beschrieben sind, solche wie sie in WO2006124529, WO2007124461, WO2008062457, WO2008082274, WO2008101017, WO2008081418, WO2008112939, WO2008112941, WO2008113601, WO2008116294, WO2008116648, WO2008119238 beschrieben sind, Peptide wie z.B. Obinepitide (TM-30338), Amylinrezeptor Agonisten, wie sie z.B. in WO2007104789 beschrieben sind, Analoga des humanen GLP-1, wie sie in WO2007120899, WO2008022015, WO2008056726 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in WO2006121860 beschrieben sind.

Antidiabetika umfassen auch das Glukose-abhängige insulinotrope Polypeptid (GIP) wie auch analoge Verbindungen wie sie z.B. in WO2008021560 beschrieben sind.

Antidiabetika umfassen auch Analoga und Derivate des Fibroblastenwachstumsfaktors 21 (FGF-21, fibroblast growth factor 21).

Die oral wirksamen hypoglykämischen Wirkstoffe umfassen vorzugsweise Sulfonylharnstoffe, Biguanidine,
Meglitinide,
Oxadiazolidindione,
Thiazolidindione,
PPAR- und RXR-Modulatoren,
Glukosidase-Inhibitoren,
Hemmstoffe der Glykogenphosphorylase,
Glukagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,

Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Modulatoren der natrium-abhängigen Glukosetransporter 1 oder 2 (SGLT1, SGLT2), Hemmstoffe der 11-beta-Hydroxysteroid-Dehydrogenase-1 (11ß-HSD1),
Inhibitoren der Protein-Tyrosin-Phosphatase-1B (PTP-1B),
Nikotinsäurerezeptoragonisten,
Inhibitoren der hormon-sensitiven bzw. endothelialen Lipasen,
Hemmstoffen der Acetyl-CoA Carboxylase (ACC1 und/oder ACC2) oder
Inhibitoren der GSK-3 beta.
Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Farnesoid X Rezeptor (FXR) Modulatoren,
Fibrate,
Cholesterinresreptionsinhibitoren,
CETP-Inhibitoren,
Gallensäureresorptionsinhibitoren,
MTP-Inhibitoren,
Agonisten des Estrogenrezeptors gamma (ERR□ Agonisten),
Sigma-1 Rezeptorantagonisten,
Antagonisten des Somatostatin 5 Rezeptors (SST5 Rezeptor);
Verbindungen, die die Nahrungsmitteleinnahme verringern und
Verbindungen, die die Thermogenese erhöhen.

Bei einer Ausführungsform der Erfindung wird die Verbindung der Formel I in Kombination mit Insulin verabreicht.

Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid, Gliclazide oder Glimepirid, verabreicht.

Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331, WO2008050987, WO2008062273 beschrieben).

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer weiteren Ausführungsform wird die Verbindung der Formel I in Kombination mit antidiabetischen Verbindungen, wie sie in WO2007095462, WO2007101060, WO2007105650 beschrieben sind, verabreicht.

Bei einer weiteren Ausführungsform wird die Verbindung der Formel I in Kombination mit antihypoglykämischen Verbindungen, wie sie in WO2007137008, WO2008020607 beschrieben sind, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, G1 262570, R-483, CS-011 (Rivoglitazon), DRL-17564, DRF-2593 (Balaglitazon), INT-131, T-2384 oder solchen, wie sie in WO2005086904, WO2007060992, WO2007100027, WO2007103252, WO2007122970, WO2007138485, WO2008006319, WO2008006969, WO2008010238, WO2008017398, WO2008028188, WO2008066356, WO2008084303, WO2008089461-WO2008089464, WO2008093639, WO2008096769, WO2008096820, WO2008096829, US2008194617, WO2008099944, WO2008108602, WO2008109334, WO2008126731, WO2008126732 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043, WO2008006043, WO2008006044, WO2008012470, WO2008035359, WO2008087365, WO2008087366, WO2008087367, WO2008117982 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat), MBX-213, KY-201 oder wie in WO 00/64888, WO 00/64876, WO03/020269, WO2004024726, WO2007099553, US2007276041, WO2007085135, WO2007085136, WO2007141423, WO2008016175, WO2008053331, WO2008109697, WO2008109700, WO2008108735 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, WO2007071766, WO2007101864, US2007244094, WO2007119887, WO2007141423, US2008004281, WO2008016175, WO2008066356, WO20080713111, WO2008084962, US2008176861 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505 oder solchen wie sie in WO2008035359 beschrieben sind, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht.

Bei einer Ausführungsform wird die Verbindung der Formel I in Kombination mit einem $\alpha$-Glukosidase-Inhibitor, wie z.B. Miglitol oder Acarbose oder solchen, wie sie z.B. in WO2007114532, WO2007140230, US2007287674, US2008103201, WO2008065796, WO2008082017 beschrieben sind, verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932, WO2008062739, WO2008099000, WO2008113760 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680, WO2006086488, WO2007047177, WO2007106181, WO2007111864, WO2007120270, WO2007120284, WO2007123581, WO2007136577, WO2008042223, WO2008098244 beschrieben, verabreicht.

Bei einer weiteren Ausführungsform wird die Verbindung der Formel I in Kombination mit einer Antisense-Verbindung, z.B. ISIS-325568, verabreicht, welche die Produktion des Glukagonrezeptors inhibiert.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007417649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942, WO2008005914, WO2008005964, WO2008043701, WO2008044777, WO2008047821, US2008096877, WO20008050117, WO2008050101, WO2008059625, US2008146625, WO2008078674, WO2008079787, WO2008084043, , WO2008084044, WO2008084872, WO2008089892, WO2008091770, WO2008075073, WO2008084043, WO2008084044, WO2008084872, WO2008084873, WO2008089892, WO2008091770, JP2008189659, WO2008104994, WO2008111473, WO2008116107, WO2008118718, WO2008120754 beschrieben sind, verabreicht.

Bei einer Ausführungsform wird die Verbindung der Formel I in Kombination mit einem Inhibitor der Glukoneogenese, wie sie z. B. in FR-225654, WO2008053446 beschrieben sind, verabreicht.

Bei einer Ausführungsform wird die Verbindung der Formel I in Kombination mit Inhibitoren der Fructose-1,6-bisphosphatase (FBPase) wie z.B. MB-07729, CS-917 (MB-06322) oder MB-07803 oder solchen wie sie in WO2006023515, WO2006104030, WO2007014619, WO2007137962, WO2008019309, WO2008037628 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200 (Melogliptin), GW-825964X, KRP-104, DP-893, ABT-341, ABT-279 oder ein anderes Salz davon, S-40010, S-40755, PF-00734200, BI-1356, PHX-1149, Alogliptin Benzoat, Linagliptin, Melogliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, US2006890898, US2006803357, US2006303661, WO2007015767 (LY-2463665), WO2007024993, WO2007029086, WO2007063928, WO2007070434, WO2007071738, WO2007071576, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603, WO2007142253, WO2007148185, WO2008017670, US2008051452, WO2008027273, WO2008028662, WO2008029217, JP2008031064, JP2008063256, WO2008033851, WO2008040974, WO2008040995, WO2008060488, WO2008064107, WO2008066070, WO2008077597, JP2008156318, WO2008087560, WO2008089636, WO2008093960, WO2008096841, WO2008141953, WO2008118848, WO2008119005, WO2008119208, WO2008120813, WO2008121506 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas$^{(R)}$ einer festen Kombination von Vildagliptin mit Metformin Hydrochlorid, verabreicht.

Bei einer weiteren Ausführungsform wird die Verbindung der Formel I in Kombination mit einer festen Kombination von Alogliptin Benzoat mit Pioglitazone verabreicht.

Bei einer Ausführungsform wird die Verbindung der Formel I in Kombination mit einer festen Kombination von eines Salzes von Sitagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer festen Kombination von eines Salzes von Sitagliptin mit Metformin Hydrochlorid, verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761, WO2008045484, US2008194617 beschrieben sind, verabreicht.

Bei einer Ausführungsform wird die Verbindung der Formel I in Kombination mit Agonisten des glucose-abhängigen insulinotropischen Rezeptors (GDIR) wie z. B. APD-668 verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin oder Dapagliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116, WO2007143316, WO2007147478, WO2008001864, WO2008002824, WO2008013277, WO2008013280, WO2008013321, WO2008013322, WO2008016132, WO2008020011, JP2008031161, WO2008034859, WO2008042688, WO2008044762, WO2008046497, WO2008049923, WO2008055870, WO2008055940, WO2008069327, WO2008070609, WO2008071288, WO2008072726, WO2008083200, WO2008090209, WO2008090210, WO2008101586, WO2008101939, WO2008116179, WO2008116195, US2008242596 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Hemmstoffen der 11-beta-Hydroxysteroid-Dehydrogenase- (11ß-HSD1), wie z. B. BVT-2733, JNJ-25918646, INCB-13739, INCB-20817, DIO-92

((-)-Ketoconazol) oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810, WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427, WO2007139992, WO2007144394, WO2007145834. WO2007145835, WO2007146761, WO2008000950, WO2008000951, WO2008003611, WO2008005910, WO2008006702, WO2008006703, WO2008011453, WO2008012532, WO2008024497, WO2008024892, WO2008032164, WO2008034032, WO2008043544, WO2008044656, WO2008046758, WO2008052638, WO2008053194, WO2008071169, WO2008074384, WO2008076336, WO2008076862, WO2008078725, WO2008087654, WO2008088540, WO2008099145, WO2008101885, WO2008101886, WO2008101907, WO2008101914, WO2008106128, WO2008110196, WO2008119017, WO2008120655, WO2008127924 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058, US2008004325, WO2008033455, WO2008033931, WO2008033932, WO2008033934, WO2008089581 beschrieben sind, verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A (Laropiprant) oder MK-0524 oder solchen Verbindungen, wie sie in WO2004041274, WO2006045565, WO2006045564, WO2006069242, WO02006085108, WO2006085112, WO2006085113, WO2006124490, WO2006113150, WO2007017261, WO2007017262, WO2007017265, WO2007015744, WO2007027532, WO2007092364, WO2007120575, WO2007134986, WO2007150025, WO2007150026, WO2008016968, WO2008051403, WO2008086949, WO2008091338, WO2008097535, WO2008099448, US2008234277, WO2008127591 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.

Bei einer Ausführungsform der Erfindung wird die Verbindung der Formel I in Kombination mit Nicotinsäure oder einem anderen Nicotinsäurerezeptoragonisten und einem Prostaglandin DP Rezeptorantagonisten, wie z.B. solchen wie sie in WO2008039882 beschrieben sind, verabreicht.

Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform wird die Verbindung der Formel I in Kombination mit Modulatoren des GPR40, wie sie z.B. in WO2007013689, WO2007033002, WO2007106469, US2007265332, WO2007123225, WO2007131619, WO2007131620, WO2007131621, US2007265332, WO2007131622, WO2007136572, WO2008001931, WO2008030520, WO2008030618, WO2008054674, WO2008054675, WO2008066097, US2008176912 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1, PSN-821, PSN-119-2, MBX-2982 oder solchen wie sie z. B. in WO2004065380, WO2005061489 (PSN-632408), WO2006083491, WO2007003960-62 und WO2007003964, WO2007035355, WO2007116229, WO2007116230, WO2008005569, WO2008005576, WO2008008887, WO2008008895, WO2008025798, WO2008025799, WO2008025800, WO2008070692, WO2008076243, WO200807692, WO2008081204, WO2008081205, WO2008081206,

WO2008081207, WO2008081208, WO2008083238, WO2008085316, WO2008109702 beschrieben sind, verabreicht.

Bei einer weiteren Ausführungsform wird die Verbindung der Formel I in Kombination mit Modulatoren des GPR120, wie sie z.B. in EP1688138, WO2008066131, WO2008066131, WO2008103500, WO2008103501 beschrieben sind, verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178, WO2007119837, WO2008122352, WO2008122357 beschrieben, verabreicht.

Bei einer Ausführungsform wird die Verbindung der Formel I in Kombination mit Inhibitoren der endothelialen Lipase, wie z. B. in WO2007110216 beschrieben, verabreicht.

Bei einer Ausführungsform wird die Verbindung der Formel I in Kombination mit einem Phospholipase A2 Inhibitor wie z.B. Darapladib oder A-002 oder solchen, wie sie in WO2008048866, WO20080488867 beschrieben sind, verabreicht.

Bei einer Ausführungsform wird die Verbindung der Formel I in Kombination mit Myricitrin, einem Lipase-Inhibitor (WO2007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110, US2007281949, WO2008002244, WO2008002245, WO2008016123, WO2008023239, WO2008044700, WO2008056266, WO2008057940, WO2008077138, EP1939191, EP1939192, WO2008078196, WO2008094992, WO2008112642, WO2008112651, WO2008113469, WO2008121063, WO2008121064 beschrieben.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Phosphoinositidkinase-3 (PI3K), wie z.B. solchen, wie in WO2008027584, WO2008070150, WO2008125833, WO2008125835, WO2008125839 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, WO2007093264, WO2008009335, WO2008086854 beschrieben, verabreicht.

Bei einer Ausführungsform wird die Verbindung der Formel I in Kombination mit einem Modulator des Glucocorticoidrezeptors, wie z. B. in WO2008057855, WO2008057856, WO2008057857, WO2008057859, WO2008057862, WO2008059867, WO2008059866, WO2008059865, WO2008070507, WO2008124665, WO2008124745 beschrieben, verabreicht.

Bei einer Ausführungsform wird die Verbindung der Formel I in Kombination mit einem Modulator des Mineralocorticoidrezeptors (MR), wie z. B. Drospirenone, oder solchen wie sie in WO2008104306, WO2008119918 beschrieben sind, verabreicht.

Bei einer Ausführungsform wird die Verbindung der Formel I in Kombination mit einem Inhibitor der Protein Kinase C beta (PKC beta), wie z. B. Ruboxistaurin, oder solchen wie sie in WO2008096260, WO2008125945 beschrieben sind, verabreicht.

Bei einer Ausführungsform wird die Verbindung der Formel I in Kombination mit einem Inhibitor der Protein Kinase D, wie z. B. Doxazosin (WO2008088006), verabreicht.

Bei einer weiteren Ausführungsform wird die Verbindung der Formel I in Kombination mit einem Aktivator der AMP-aktivierten Proteinkinase (AMPK), wie sie z. B. in WO2007062568, WO2008006432, WO2008016278, WO2008016730, WO2008083124 beschrieben sind, verabreicht.

Bei einer Ausführungsform wird die Verbindung der Formel I in Kombination mit einem Inhibitor der Ceramidkinase, wie sie z. B. in WO2007112914, WO2007149865 beschrieben sind, verabreicht.

Bei einer weiteren Ausführungsform wird die Verbindung der Formel I in Kombination mit einem Inhibitor der MAPK-interagierenden Kinase 1 oder 2 (MNK1 oder 2), wie sie z.B. in WO2007104053, WO2007115822, WO2008008547, WO2008075741 beschrieben sind, verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022057, WO2004022553, WO2005097129, WO2005113544, US2007244140, WO2008099072, WO2008099073, WO2008099073, WO2008099074, WO2008099075 beschrieben sind, verabreicht.

Bei einer anderen Ausführungsform wird die Verbindung der Formel I in Kombination mit Inhibitoren der NF-kappaB (NFKB) Aktivierung, wie sie z. B. Salsalate verabreicht.

Bei einer weiteren Ausführungsform wird die Verbindung der Formel I in Kombination mit Inhibitoren der ASK-1 (apoptosis signal-regulating kinase 1), wie sie z. B. in WO2008016131 beschrieben sind, verabreicht.

Bei einer Ausführungsform der Erfindung wird die Verbindungen der Formel I in Kombination mit einem HMG-CoA-Reduktase Inhibitor wie Simvastatin, Fluvastatin, Pravastatin, Lovastatin, Atorvastatin, Cerivastatin, Rosuvastatin, Pitavastatin, L-659699, BMS-644950 oder solchen, wie sie in US2007249583, WO2008083551 beschrieben sind, verabreicht.

Bei einer weiteren Ausführungsform der Erfindung wird die Verbindung der Formel I in Kombination mit einem Famesoid X Rezeptor (FXR) Modulatoren, wie z.B. WAY-362450 oder solchen wie in WO2003099821, WO2005056554, WO2007052843, WO2007070796, WO2007092751, JP2007230909, WO2007095174, WO2007140174, WO2007140183, WO2008000643, WO2008002573, WO2008025539, WO2008025540, JP2008214222 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965, WO2008041003, WO2008049047, WO2008065754, WO2008073825, US2008242677 beschrieben, verabreicht.

Bei einer Ausführungsform der Erfindung wird die Verbindung der Formel I in Kombination mit einem Fibrat, wie z.B. Fenofibrat, Clofibrat, Bezafibrat, oder solchen wie sie in WO2008093655 beschrieben sind, verabreicht.

Bei einer Ausführungsform der Erfindung wird die Verbindung der Formel I in Kombination mit Fibraten, wie z.B. dem Cholinsalz von Fenofibrat (SLV-348), verabreicht.

Bei einer Ausführungsform der Erfindung wird die Verbindung der Formel I in Kombination mit Fibraten, wie z.B. dem Cholinsalz von Fenofibrat und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin, Pitavastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358, WO2008033431, WO2008033465, WO2008052658, WO2008057336, WO2008085300 beschrieben, verabreicht.

Bei einer Ausführungsform der Erfindung wird die Verbindung der Formel I in Kombination mit einem NPC1L1-Antagonisten, wie z.B. solchen, wie sie in WO2008033464, WO2008033465 beschrieben sind, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer Ausführungsform der Erfindung ist der weitere Wirkstoff ein Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin, Pitavastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer Ausführungsform der Erfindung wird die Verbindung der Formel I in Kombination mit einem CETP-Inhibitor, wie z.B. Torcetrapib, Anacetrapib oder JTT-705 (Dalcetrapib) oder solchen wie sie in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906, WO2008006257, WO2008009435, WO2008018529, WO2008058961, WO2008058967,

WO2008059513, WO2008070496, WO2008115442, WO2008111604 beschrieben sind, verabreicht.

Bei einer Ausführungsforin der Erfindung wird die Verbindung der Formel I in Kombination mit Gallensäureresorptionsinhibitoren (Inhibitoren des intestinalen Gallensäuretransporters (IBAT)) (siehe z.B. US 6,245,744, US 6,221,897 oder WO00/61568), wie z.B. HMR 1741 oder solchen wie in DE 10 2005 033099.1 und DE 10 2005 033100.9, DE 10 2006 053635, DE 10 2006 053637, WO2007009655-56, WO2008058628, WO2008058629, WO2008058630, WO2008058631 beschrieben, verabreicht.

Bei einer Ausführungsform wird die Verbindung der Formel I in Kombination mit Agonisten des GPBAR1 (G-protein-coupled-bile-acid-receptor-1; TGR5), wie sie z.B. in US20060199795, WO2007110237, WO2007127505, WO2008009407, WO2008067219, WO2008067222, FR2908310, WO2008091540, WO2008097976 beschrieben sind, verabreicht.

Bei einer Ausführungsform wird die Verbindung der Formel I in Kombination mit Inhibitoren des TRPM5 Kanals (TRP-Cation-Channel-M5), wie sie z.B. in WO2008097504 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.

Bei einer Ausführungsform der Erfindung wird die Verbindung der Formel I in Kombination mit Colesevelam Hydrochlorid und Metformin oder einem Sulfonylharnstoff oder Insulin verabreicht.

Bei einer Ausführungsform der Erfindung wird die Verbindung der Formel I in Kombination mit einem Phytosterole enthaltenden Kaugummi (Reductol™) verabreicht.

Bei einer Ausführungsform der Erfindung wird die Verbindung der Formel I in Kombination mit einem Inhibitor des mikrosomalen Triglycerid-Transfer-Proteins (MTP-Inhibitor), wie z.B. Implitapide , BMS-201038, R-103757, AS-1552133, SLx-4090, AEGR-733 oder solchen wie in WO2005085226, WO2005121091, WO2006010423, WO2006113910, WO2007143164, WO2008049806, WO2008049808, WO2008090198, WO2008100423 beschrieben, verabreicht.

Bei einer weiteren Ausführungsform der Erfindung wird die Verbindung der Formel I in Kombination mit einer Kombinbation eines Cholesterolabsorptionsinhibitors, wie z.B. Ezetimibe, und einem Inhibitor des Triglycerid-Transfer-Proteins (MTP-Inhibitor), wie z.B. Implitapide, wie in WO2008030382 oder in WO2008079398 beschrieben, verabreicht.

Bei einer Ausführungsform der Erfindung wird die Verbindung der Formel I in Kombination mit einem antihypertriglyceridämischen Wirkstoff, wie z.B. solchen wie sie in WO2008032980 beschrieben sind, verabreicht.

Bei einer anderen Ausführungsform der Erfindung wird die Verbindung der Formel I in Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382 oder solchen, wie sie in WO2008087029, WO2008087030, WO2008095189 beschrieben sind, verabreicht.

Bei einer weiteren Ausführungsform der Erfindung wird die Verbindung der Formel I in Kombination mit einem Inhibitor der Leber-Carnitin Palmitoyltransferase-1 (L-CPT1), wie sie z.B. in WO2007063012, WO2007096251 (ST-3473), WO2008015081, US2008103182, WO2008074692 beschrieben sind, verabreicht.

Bei einer weiteren Ausführungsform der Erfindung wird die Verbindung der Formel I in Kombination mit einem Modulator der Serin-Palmitoyltransferase (SPT), wie sie z.B. in WO2008031032, WO2008046071, WO2008083280, WO2008084300 beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943, WO2008003424 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem Stimulator des ApoA-1 Gens, wie er z.B. in WO2008092231 beschrieben ist, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738, WO2008020607 beschrieben, verabreicht.

Bei einer anderen Ausführungsform der Erfindung wird die Verbindung der Formel I in Kombination mit einem HDL-Cholesterol-erhöhenden Agens, wie z.B. solchen wie sie in WO2008040651, WO2008099278 beschrieben sind, verabreicht.

Bei einer Ausführungsform der Erfindung wird die Verbindung der Formel I in Kombination mit einem ABCA1 Expressionsverstäker, wie sie z.B. in WO2006072393, WO2008062830 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A1 Rezeptor Agonisten (Adenosin A1 R), wie sie z.B. in EP1258247, EP1375508, WO2008028590, WO2008077050 beschrieben sind, verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923, WO2008070661 beschrieben, verabreicht.

Bei einer weiteren Ausführungsform der Erfindung wird die Verbindung der Formel I in Kombination mit einem Agonisten der Adenosin A1/A2B Rezeptoren, wie z.B. in WO2008064788, WO2008064789 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in US2007270433, WO2008027585, WO2008080461 beschrieben sind, verabreicht.

Bei einer Ausführungsform wird die Verbindung der Formel I in Kombination mit Hemmstoffen der Acetyl-CoA Carboxylase (ACC 1 und/oder ACC2) wie z. B. solchen wie in WO199946262, WO200372197, WO2003072197, WO2005044814, WO2005108370, JP2006131559, WO2007011809, WO2007011811, WO2007013691, WO2007095601-603, WO2007119833, WO2008065508, WO2008069500, WO2008070609, WO2008072850, WO2008079610, WO2008088688, WO2008088689, WO2008088692, US2008171761, WO2008090944, JP2008179621, US2008200461, WO2008102749, WO2008103382, WO2008121592 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in WO2007100833) verabreicht.

Bei einer weiteren Ausführungsform wird die Verbindung der Formel I in Kombination mit Modulatoren der Xanthin-Oxidoreductase (XOR) verabreicht.

Bei einer anderen Ausführungsform wird die Verbindung der Formel I in Kombination mit Inhibitoren der löslichen Epoxidhydrolase (sEH), wie sie z.B. in WO2008051873, WO2008051875, WO2008073623, WO2008094869, WO2008112022 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);

NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A) oder Velneperit;

NPY-5 Rezeptorantagonisten wie L-152804 oder die Verbindung "NPY-5-BY" der Firma Banyu oder wie sie z. B. in WO2006001318, WO2007103295, WO2007125952, WO2008026563, WO2008026564, WO2008052769, WO2008092887, WO2008092888, WO2008092891 beschrieben sind;

NPY-4-Rezeptorantagonisten wie sie z. B. in WO2007038942 beschrieben sind;

NPY-2-Rezeptorantagonisten wie sie z. B. in WO2007038943 beschrieben sind;

Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166, WO2008003947 beschrieben sind;

Derivaten des Peptids Obestatin wie sie WO2006096847 beschrieben sind;

CB1R (Cannabinoid Rezeptor 1) Antagonisten wie z.B. Rimonabant, Surinabant (SR147778), SLV-319 (Ibipinabant), AVE-1625, Taranabant (MK-0364) oder Salze davon, Otenabant (CP-945,598), Rosonabant, V-24343 oder solche Verbindungen wie sie in z. B. EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456,

WO2005063761-62, WO2005061509, WO2005077897, WO2006018662, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007018460, WO2007016460, WO2007020502, Wv2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863, WO2007119001, WO2007120454, WO2007121687, WO2007123949, US2007259934, WO2007131219, WO2007133820, WO2007136571, WO2007136607, WO2007136571, US7297710, WO2007138050, WO2007139464, WO2007140385, WO2007140439, WO2007146761, WO2007148061, WO2007148062, US2007293509, WO2008004698, WO2008017381, US2008021031, WO2008024284, WO2008031734, WO2008032164, WO2008034032, WO2008035356, WO2008036021, WO2008036022, WO2008039023, WO2998043544, WO2008044111, WO2008048648, EP1921072-A1, WO2008053341, WO2008056377, WO2008059207, WO2008059335, WO2008062424, WO2008068423, WO2008068424, WO2008070305, WO2008070306, WO2008074816, WO2008074982, WO2008075012, WO2008075013, WO2008075019, WO2008075118, WO2008076754, WO2008081009, WO2008084057, EP1944295, US2008090809, US2008090810, WO2008092816, WO2008094473, WO2008094476, RWO2008099076, WO2008099139, WO02008101995, US2008207704, WO2008107179, WO2008109027, WO2008112674, WO2008115705, WO2008118414, WO2008119999, WO200812000, WO2008121257, WO2008127585 beschrieben sind;

Cannabinoid Rezeptor 1 / Cannabinoid Rezeptor 2 (CB1/CB2) modulierende Verbindungen wie z.B. delta-9-Tetrahydrocannabivarin oder solchen wie sie z.B. in WO2007001939, WO2007044215, WO2007047737, WO2007095513, WO2007096764, WO2007112399, WO2007112402, WO2008122618 beschrieben sind;

Modulatoren der FAAH (fatty acid amide hydrolase) wie sie z.B. in WO2007140005, WO2008019357, WO2008021625, WO2008023720, WO2008030532 beschrieben sind;

Inhibitoren der Fettsäuresynthase (fatty acid synthase; FAS), wie sie z.B. in WO2008057585, WO2008059214, WO2008075064, WO2008075070, WO2008075077 beschrieben sind;

Inhibitoren der LCE (long chain fatty acid elongase), wie sie z.B. in WO2008120653 beschrieben sind;

Vanilloid-1-Rezeptor Modulatoren (Modulatoren des TRPV1), wie sie z.B. in WO2007091948, WO2007129188, WO2007133637, WO2008007780, WO2008010061, WO2008007211, WO2008010061, WO2008015335, WO2008018827, WO2008024433, WO2008024438, WO2008032204, WO2008050199, WO2008059339, WO2008059370, WO2008066664, WO20080755150, WO2008090382, WO2008090434, WO2008093024, WO2008107543, WO2008107544, WO2008110863 beschrieben sind;

Modulatoren, Antagonisten oder inverse Agonisten der Opioidrezeptoren, wie z.B. GSK-982 oder solche wie sie z.B. in WO2007047397, WO2008021849, WO2008021851, WO2008032156, WO2008059335 beschrieben sind;

Modulatoren des "orphan opioid (ORL-1) receptor" wie sie z.B. in US2008249122, WO2008089201 beschrieben sind;

Agonisten des Prostaglandinrezeptors, wie z.B. Bimatoprost oder solchen Verbindungen wie sie in WO2007111806 beschrieben sind;

MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20044167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763, WO2007141343, WO2008007930, WO2008017852, WO2008039418, WO2008087186, WO2008087187, WO2008087189, WO2008087186-WO2008087190, WO2008090357 beschrieben sind;

Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methylbenzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374, WO2007122591, WO2007126934, WO2007126935, WO2008008517, WO2008008518, WO2008008551, WO2008020405, WO2008026149, WO2008038251, US2008132490, WO2008065626, WO2008078291, WO2008087611, WO2008081399, WO2008108991, WO2008107335, US2008249125 beschrieben sind);

Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884,

WO2005082893, US2005171181 (z.B. PF-00389027), WO2006107661, WO2007003804, WO2007016496, WO2007020213, WO2007049798, WO2007055418, WO2007057329, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111, WO2007137955, US2007281923, WO2007137968, WO2007138431, WO2007146122, WO2008005338, WO2008012010, WO2008015125, WO2008045371, EP1757594, WO2008068173, WO2008068174, US20080171753, WO2008072703, WO2008072724, US2008188484, US2008188486, US2008188487, WO2008109333, WO2008109336 beschrieben sind);

Histamin H1 / Histamin H3 Modulatoren, wie z. B. Betahistin bzw. seinem Dihydrochlorid;

Modulatoren des Histamin H3 Transporters oder der Histamin H3 / Serotonin Transporter wie sie z.B. in WO2008002816, WO2008002817, WO2008002818, WO2008002820 beschrieben sind;

Histamin H4 Modulatoren wie sie z.B. in WO2007117399 beschrieben sind;

CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CRF 1-Antagonisten, wie sie in WO2007105113, WO2007133756, WO2008036541, WO2008036579, WO2008083070 beschrieben sind);

CRF BP-Antagonisten (z.B. Urocortin);

Urocortin-Agonisten;

Modulatoren des beta-3 Adrenoceptors wie z.B. 1-(4-Chloro-3-methanesulfonylmethylphenyl)-2-[2-(2,3-dimethyl-1H-indol-6-yloxy)-ethylamino]-ethanol Hydrochlorid (WO 01/83451) oder Solabegron (GW-427353) oderN-5984 (KRP-204) oder solche, wie sie in JP2006111553, WO2002038543, WO2002038544, WO2007048840-843, WO2008015558, EP1947103 beschrieben sind;

MSH (Melanocyt-stimulierendes Hormon)-Agonisten;

MCH (melanin-konzentrierendes Hormon) Rezeptor Antagonisten (wie z. B. NBI-845, A-761, A-665798, A-798, ATC-0175, T-226296, T-71 (AMG-071, AMG-076), GW-856464, NGD-4715, ATC-0453, ATC-0759, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916, WO2007141200, WO2007142217, US2007299062, WO2007146758, WO2007146759, WO2008001160, WO2008016811, WO2008020799, WO2008022979, WO2008038692, WO2008041090, WO2008044632, WO2008047544, WO2008061109, WO2008065021, WO2008068265, WO2008071646, WO2008076562, JP2008088120, WO2008086404, WO2008086409 beschrieben sind);

CCK-A (CCK-1) Agonisten/Modulatoren (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl} -essigsäure Trifluoressigsäuresalz (WO 99/15525) oder SR-146131 (WO 0244150) oder SSR-125180) oder solchen, wie sie in WO2005116034, WO2007120655, WO2007120688, WO2007120718, WO2008091631 beschrieben sind;

Serotonin-Wiederaufnahme-Inhibitoren (z.B. Dexfenfluramine) oder solchen wie sie in WO2007148341, WO2008034142, WO2008081477, WO2008120761 beschrieben sind;

gemischte Serotonin-/Dopamin-Wiederaufnahme-Inhibitoren (z.B. Bupropion) oder solche wie sie in WO2008063673 beschrieben sind oder feste Kombinationen von Bupropion mit Naltrexon oder Bupropion mit Zonisamid;

gemischte Wiederaufnahmeinhibitoren wie z.B. DOV-21947;

gemischte Sertonin- und noradrenerge Verbindungen (z.B. WO 00/71549);

5-HT-Rezeptor Agonisten z.B. 1-(3-Ethyl-benzofuran-7-yl)-piperazin Oxalsäuresalz (WO 01/09111);

gemischte Dopamin/Norepinephrin/Acetylcholin-Wiederaufnahme-Inhibitoren (z.B. Tesofensine) oder solchen wie sie z.B. in WO2006085118 beschrieben sind;

Dopaminantagonisten wie sie z.B. in WO2008079838, WO2008079839, WO2008079847, WO2008079848 beschrieben sind;

Norepinephrin-Wiederaufnahme-Inhibitoren wie sie z.B. in US2008076724 beschrieben sind;

5-HT2A Rezeptor Antagonisten wie sie z.B. in WO2007138343 beschrieben sind;

5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213, WO2008007661, WO2008007664, WO2008009125, WO2008010073, WO2008108445 beschrieben sind);

5-HT6 Rezeptor Modulatoren, wie z.B. E-6837, BVT-74316 oder PRX-07034 oder solche wie sie z.B. in WO2005058858, WO2007054257, WO2007107373, WO2007108569, WO2007108742-744, WO2008003703, WO2008027073, WO2008034815, WO2008054288, EP1947085, WO2008084491, WO2008084492, WO2008092665, WO2008092666, WO2008101247, WO2008110598, WO2008116831, WO2008116833 beschrieben sind;

Agonisten des Estrogenrezeptors gamma (ERRγ Agonisten), wie sie z.B. in WO2007131005, WO2008052709 beschrieben sind;

Agonisten des Estrogenrezeptors alpha (ERRα / ERR1 Agonisten), wie sie z.B. in WO2008109727 beschrieben sind;

Sigma-1 Rezeptorantagonisten, wie sie z.B. in WO2007098953, WO2007098961, WO2008015266, WO2008055932, WO2008055933 beschrieben sind;

Muscarin 3 Rezeptor (M3R) Antagonisten, wie sie z.B. in WO2007110782, WO2008041184 beschrieben sind;

Bombesin-Rezeptor Agonisten (BRS-3 Agonisten), wie sie z.B. in WO2008051404, WO2008051405, WO2008051406, WO2008073311 beschrieben sind;

Galanin-Rezeptor Antagonisten;

Wachstumshormon (z.B. humanes Wachstumshormon oder AOD-9604);

Wachstumshormon freisetzende Verbindungen (6-Benzyloxy-1-(2-diisopropylaminoethylcarbamoyl)-3,4-dihydro-1H-isochinolin-2-carbonsäuretertiärbutylester (WO 01/85695));

Growth Hormone Secretagogue Receptor Antagonisten (Ghrelin Antagonisten) wie z. B. A-778193 oder solchen, wie sie in WO2005030734, WO2007127457, WO2008008286 beschrieben sind;

Growth Hormone Secretagogue Receptor Modulatoren (Ghrelin-Modulatoren) wie z.B. JMV-2959, JMV-3002, JMV-2810, JMV-2951 oder solchen, wie sie in WO2006012577 (z.B. YIL-781 oder YIL-870), WO2007079239, WO2008092681 beschrieben sind;

TRH-Agonisten (siehe z.B. EP 0 462 884);

entkoppelnde Protein 2- oder 3-Modulatoren;

chemische Entkoppler (z.B. WO2008059023, WO2008059024, WO2008059025, WO2008059026);

Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);

DA-Agonisten (Bromocriptin, Doprexin);

Lipase/Amylase-Inhibitoren (z.B. WO 00/40569, WO2008107184);

Inhibitoren der Diacylglycerol O-Acyltransferasen (DGATs) wie z.B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311, WO2007141502, WO2007141517, WO2007141538, WO2007141545, WO2007144571, WO2008011130, WO2008011131, WO2008039007, WO2008048991, WO2008067257, WO2008099221 beschrieben;

Inhibitoren der Monoacylglycerolacyltransferase (2-Acylglycerol-O-Acyltransferase; MGAT) wie sie z.B. in WO2008038768 beschrieben sind;

Inhibitoren der Fettsäuresynthase (FAS) wie z.B. C75 oder solchen, wie in WO2004005277, WO2008006113 beschrieben;

Inhibitoren der Stearoyl-CoA delta9 Desaturase (SCD1) wie sie z.B. in WO2007009236, WO2007044085, WO2007046867, WO2007046868, WO20070501124, WO2007056846, WO2007071023, WO2007130075, WO2007134457, WO2007136746, WO2007143597, WO2007143823, WO2007143824, WO2008003753, WO2008017161, WO2008024390, WO2008029266, WO2008036715, WO2008043087, WO2008044767, WO2008046226, WO2008056687, WO2008062276, WO2008064474, WO2008074824, WO2008074832, WO2008074833, WO2008074834, WO2008074835, WO2008089580, WO2008096746, WO2008104524, WO2008116898, US2008249100, WO2008120744, WO2008120759, WO2008123469, WO2008127349 beschrieben sind;

Inhibitoren der Fatty-Acid-Desaturase-1 (delta5 Desaturase) wie sie z.B. in WO2008089310 beschrieben sind;

hypoglykämische/hypertriglyceridämische Indolinverbindungen wie sie in WO2008039087 beschrieben sind;

Inhibitoren des "Adipocyte fatty acid-binding protein aP2" wie z.B. BMS-309403;

Aktivatoren der Adiponectinsekretion, wie z.B. in WO2006082978, WO2008105533 beschrieben;

Promotoren der Adiponectinproduktion, wie z.B. in WO2007125946, WO2008038712 beschrieben;

modifizierte Adiponectine wie z.B. in WO2008121009 beschrieben;

Oxyntomodulin oder Analoga davon;

Oleoyl-Estron;

oder Agonisten oder partiellen Agonisten des Schilddrüsenhormonrezeptors (thyroid hormone receptor agonists) wie z. B: KB-2115 (Eprotirome), QRX-431 (Sobetirome) oder DITPA oder solche, wie in WO20058279, WO200172692, WO200194293, WO2003084915, WO2004018421, WO2005092316, WO2007003419, WO2007009913,

WO2007039125, WO2007110225, WO2007110226, WO2007128492, WO2007132475, WO2007134864, WO2008001959, WO2008106213 beschrieben;
oder Agonisten des Schilddrüsenhormonrezeptors beta (TR-beta) wie z. B. MB-07811 1 oder MB-07344, oder solchen wie in WO2008062469 beschrieben, verabreicht.

Bei einer Ausführungsform der Erfindung wird die Verbindung der Formel I in Kombination mit einer Kombination von Eprotirome mit Ezetimibe verabreicht.

Bei einer Ausführungsform der Erfindung wird die Verbindung der Formel I in Kombination mit einem Inhibitor der Site-1 Protease (S1P), wie z.B. PF-429242, verabreicht.

Bei einer weiteren Ausführungsform der Erfindung wird die Verbindung der Formel I in Kombination mit einem Modulator des "Trace-Amine-Associated-Receptor-1" (TAAR1), wie sie z.B. in US2008146523, WO2008092785 beschrieben sind, verabreicht.

Bei einer Ausführungsform der Erfindung wird die Verbindung der Formel I in Kombination mit einem Inhibitor des Growth-Factor-Receptor-Bound-Protein-2 (GRB2), wie z.B. in WO2008067270 beschrieben, verabreicht.

Bei einer weiteren Ausführungsform der Erfindung wird die Verbindung der Formel I in Kombination mit einem RNAi (siRNA) Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel I in Kombination mit Lycopin verabreicht.

Bei einer Ausführungsform der Erfindung wird die Verbindung der Formel I in Kombination mit einem Antioxidans, wie z.B. OPC-14117, AGI-1067 (Succinobucol), Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinide und Metformin (PrandiMet (TM)), Insulin und einem Sulfonylharnstoff, Insulin und Metformin, Insulin und Troglitazon, Insulin und Lovastatin, etc. verabreicht.

Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948 beschrieben, verabreicht.

Bei einer anderen Ausführungsform wird die Verbindung der Formel I in Kombination mit Topiramat oder einem Derivat davon, wie es in WO2008027557 beschrieben ist, verabreicht.

Bei einer weiteren Ausführungsform wird die Verbindung der Formel I in Kombination mit einer festen Kombination von Topiramat mit Phentermin (Qnexa™) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer anderen Ausführungsform wird die Verbindung der Formel I in Kombination mit einem Aldosteronsynthaseinhibitor und einem Antagonisten des Glucocorticoidrezeptors, einem Cortisolsyntheseinhibitor und/oder einem Antagonisten des Corticotropin-freisetzenden Faktors (corticotropin releasing factor), wie z.B. in EP1886695, WO2008119744 beschrieben, verabreicht.

Bei einer Ausführungsform wird die Verbindung der Formel I in Kombination mit einem Agonisten des RUP3 Rezeptors, wie z. B. in WO2007035355, WO2008005576 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in WO2007119463 beschrieben, verabreicht.

Bei einer Ausführungsform wird die Verbindung der Formel I in Kombination mit einem "c-Jun N-terminal kinase" Inhibitor (JNK-Inhibitor), wie z. B. in WO2007125405, WO2008028860, WO2008118626 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in WO2005090336, WO2006071609, WO2006135826, WO2007105766, WO2008120661 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1 und/oder SIRT3 (einer

NAD$^+$-abhängigen Proteindeacetylase); dieser Wirkstoff kann z.B. Resveratrol in geeigneten Formulierungen sein, oder solche Verbindungen wie sie in WO2007019416 (z.B. SRT-1720), WO2008073451 genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2007107587, WO2007111994, WO2008106600, WO2008113796 beschrieben sind, verabreicht.

Bei einer weiteren Ausführungsform wird die Verbindung der Formel I in Kombination mit Inhibitoren des SREBP (sterol regulatory element-binding protein), wie sie z.B. in WO2008097835 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in WO2007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulators"; SARM), wie sie z.B. in WO2007099200, WO2007137874 beschrieben sind, verabreicht.

Bei einer weiteren Ausführungsform wird die Verbindung der Formel I in Kombination mit einem AGE (advanced glycation endproduct) Inhibitor, wie sie z.B. in JP2008024673 beschrieben sind, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere Wirkstoff Metreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin oder solche Derivate wie sie in WO2008034142 beschrieben sind.

Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104) oder Derivate davon (JP2008106008).

Bei einer Ausführungsform ist der weitere Wirkstoff ein nasal verabreichter Calciumkanalblocker wie z.B. Diltiazem oder solche, wie sie in US 7,138,107 beschrieben sind.

Bei einer Ausführungsform ist der weitere Wirkstoff ein Inhibitor des Natrium-Calcium-Ionen-Austausches wie z.B. solche, wie sie in WO2008028958, WO2008085711 beschrieben sind.

Bei einer weiteren Ausführungsform ist der weitere Wirkstoff ein Blocker von Calciumkanälen wie z.B. des CaV3.2 oder CaV2.2 wie sie in WO2008033431, WO2008033447, WO2008033356, WO2008033460, WO2008033464, WO2008033465, WO2008033468, WO2008073461 beschrieben sind.

Bei einer Ausführungsform ist der weitere Wirkstoff ein Modulator eines Calciumkanals wie z.B. solche, wie sie in WO2008073934, WO2008073936 beschrieben sind.

Bei einer Ausführungsform ist der weitere Wirkstoff ein Blocker des "T-type calcium channel" wie sie z.B. in WO2008033431, WO2008110008 beschrieben sind.

Bei einer Ausführungsform ist der weitere Wirkstoff ein Inhibitor des KCNQ-Kaliumkanal-2 bzw. -3 wie z.B. solche, wie sie in US2008027049, US2008027090 beschrieben sind.

Bei einer Ausführungsform ist der weitere Wirkstoff ein Inhibitor des Kalium Kv1.3 Ionenkanals wie z.B. solchen, wie sie in WO2008040057, WO2008040058, WO2008046065 beschrieben sind.

Bei einer anderen Ausführungsform ist der weitere Wirkstoff ein Modulator des MCP-1 Rezeptors (monocyte chemoattractant protein-1 (MCP-1)) wie z.B. solche, wie sie in WO2008014360, WO2008014381 beschrieben sind.

Bei einer Ausführungsform ist der weitere Wirkstoff ein Modulator des Somatostatinrezeptors 5 (SSTR5) wie z.B. solche, wie sie in WO2008019967, US2008064697, US2008249101, WO2008000692 beschrieben sind.

Bei einer Ausführungsform ist der weitere Wirkstoff ein Modulator des Somatostatinrezeptors 2 (SSTR2) wie z.B. solche, wie sie in WO2008051272 beschrieben sind.

Bei einer Ausführungsform ist der weitere Wirkstoff ein Erythropoietin-mimetisches Peptid, welches als Erythropoietin (EPO) Rezeptoragonist agiert. Solche Moleküle sind z.B. in WO2008042800 beschrieben.

Bei einer weiteren Ausführungsform ist der weitere Wirkstoff ein Anorektikum/eine hypoglykämische Verbindung wie z.B. solche, wie sie in WO2008035305, WO2008035306, WO2008035686 beschrieben sind.

Bei einer Ausführungsform ist der weitere Wirkstoff ein Induktor der Liponsäuresynthetase wie z.B. solche, wie

sie in WO2008036966, WO2008036967 beschrieben sind.

Bei einer Ausführungsform ist der weitere Wirkstoff ein Stimulator der endothelialen Nitric-Oxid-Synthase (eNOS) wie z.B. solche, wie sie in WO2008058641, WO2008074413 beschrieben sind.

Bei einer Ausführungsform ist der weitere Wirkstoff ein Modulator des Kohlenhydrat- und/oder Lipidstoffwechsels wie z.B. solche, wie sie in WO2008059023, WO2008059024, WO2008059025, WO2008059026 beschrieben sind.

Bei einer weiteren Ausführungsform ist der weitere Wirkstoff ein Angiotensin II Rezeptorantagonist wie z.B. solche, wie sie in WO2008062905, WO2008067378, WO2008062905 beschrieben sind.

Bei einer Ausführungsform ist der weitere Wirkstoff ein Agonist des Sphingosin-1-Phosphatrezeptors (S1P) wie z.B. solche, wie sie in WO2008064315, WO2008074820. WO2008074821 beschrieben sind.

Bei einer Ausführungsform ist der weitere Wirkstoff ein Mittel, welches die Magenentleerung retardiert wie z.B. 4-Hydroxyisoleucin (WO2008044770).

Bei einer Ausführungsform ist der weitere Wirkstoff eine Muskel-relaxierende Substanz wie sie z.B. in WO2008090200 beschrieben ist.

Bei einer weiteren Ausführungsform ist der weitere Wirkstoff ein Inhibitor der Monoaminoxidase B (MAO-B) wie z.B. solche, wie sie in WO2008092091 beschrieben sind.

Bei einer anderen Ausführungsform ist der weitere Wirkstoff ein Inhibitor der Bindung von Cholesterol und/oder Triglyceriden an das SCP-2 Protein (sterol carrier protein-2) wie z.B. solche, wie sie in US2008194658 beschrieben sind.

Bei einer anderen Ausführungsform ist der weitere Wirkstoff Lisofylline, welcher Autoimmunschäden an insulinproduzierenden Zellen verhindert.

Bei einer Ausführungsform wird die Verbindung der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

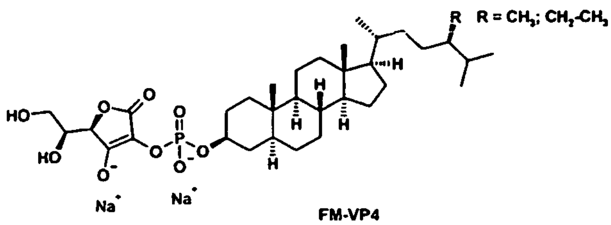

FM-VP4

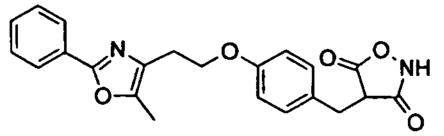

JTT-501

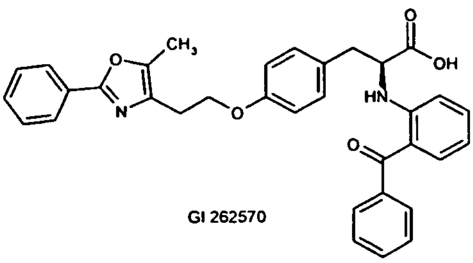

GI 262570

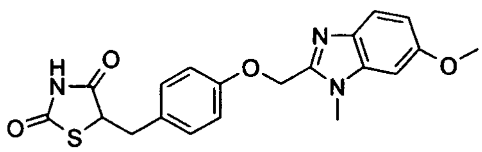

CS-011
Rivoglitazone

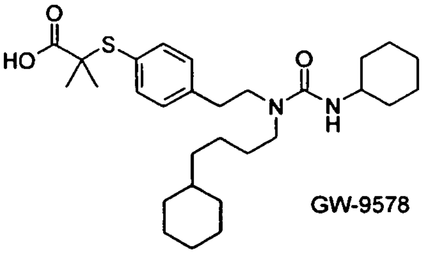

GW-9578

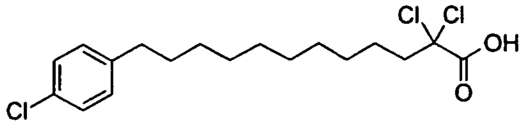

K-111

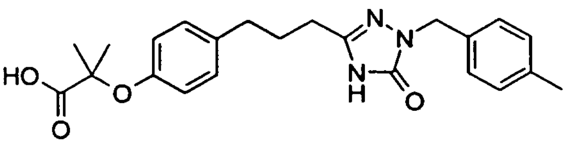

LY-518674

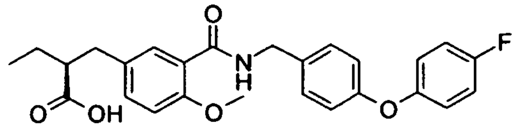

KRP-101

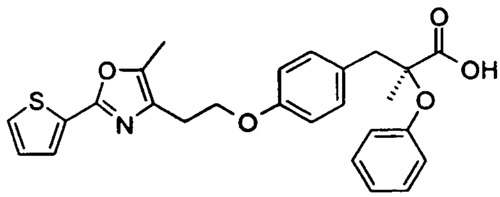

LY-510929

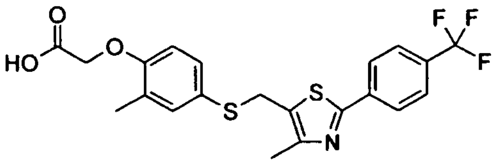

GW-501516

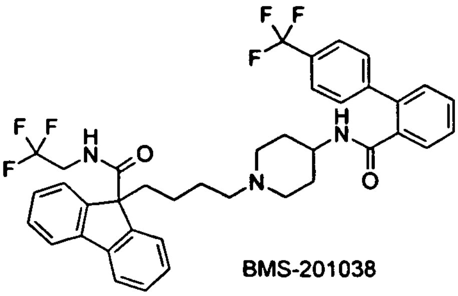

BMS-201038

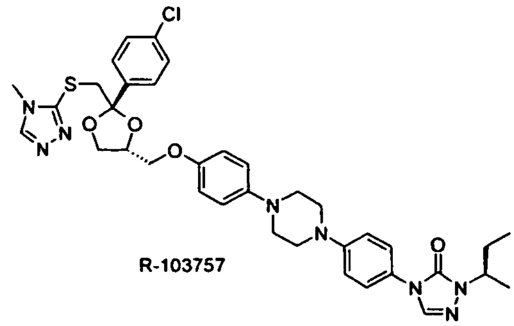

R-103757

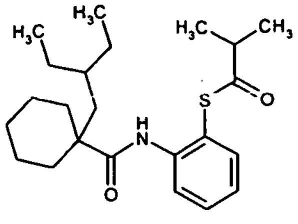

JTT-705

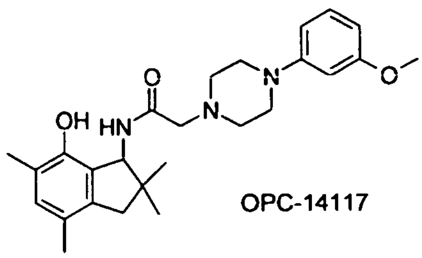

OPC-14117

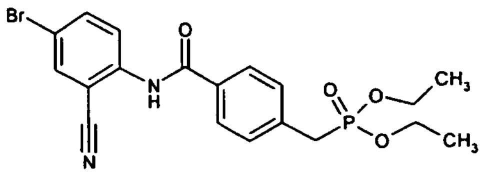

NO-1886

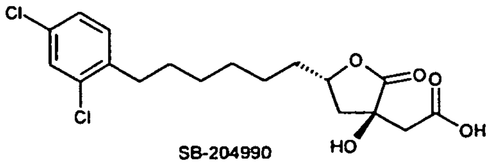

SB-204990

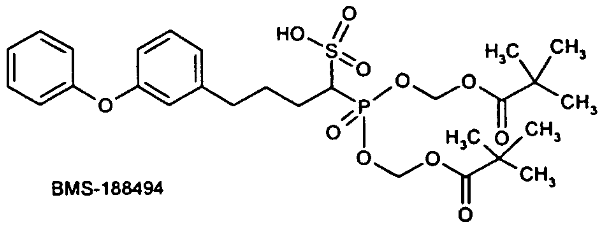

BMS-188494

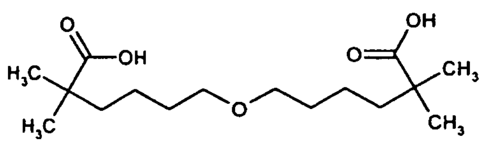

CI-1027

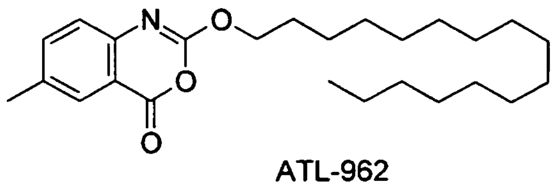

ATL-962

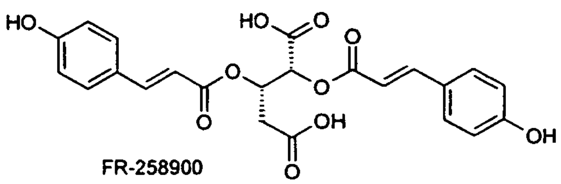

FR-258900

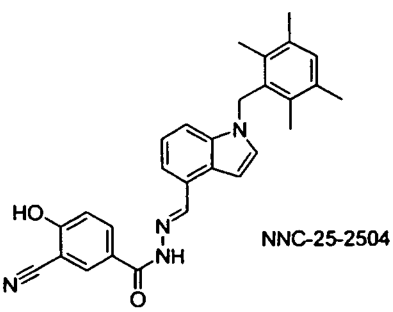

NNC-25-2504

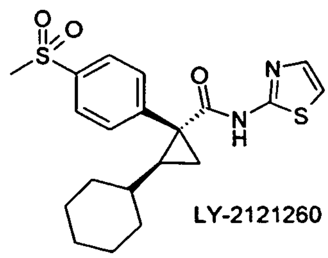

LY-2121260

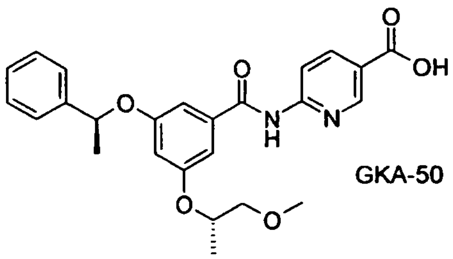

GKA-50

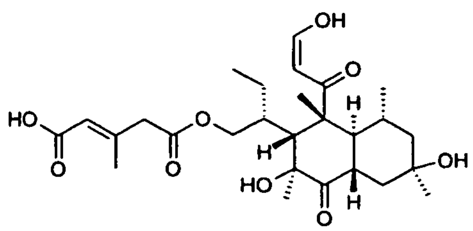

FR-225654

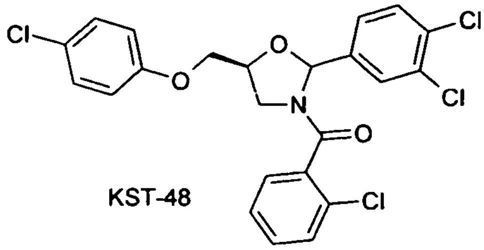

KST-48

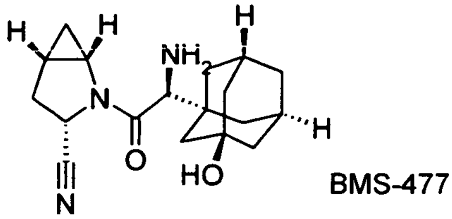

BMS-477118

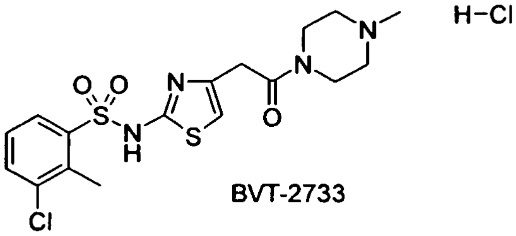

BVT-2733

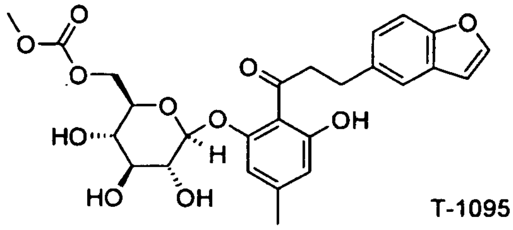

T-1095

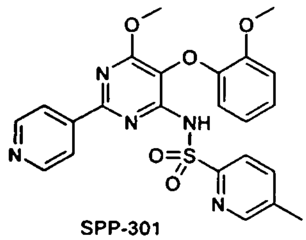

SPP-301

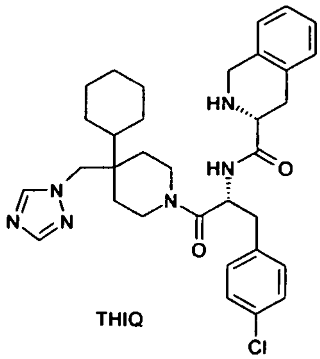

THIQ

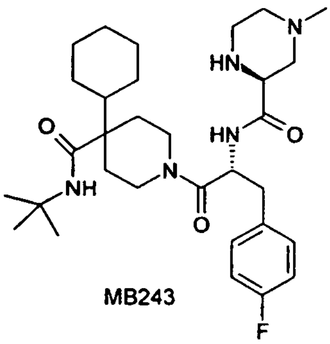

MB243

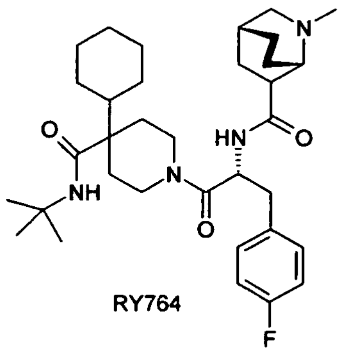

RY764

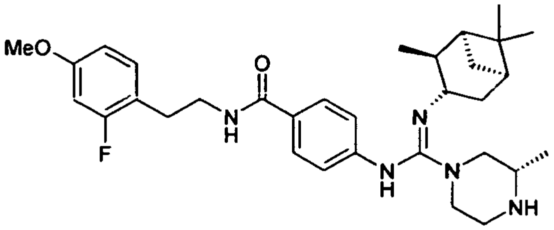

CHIR-785

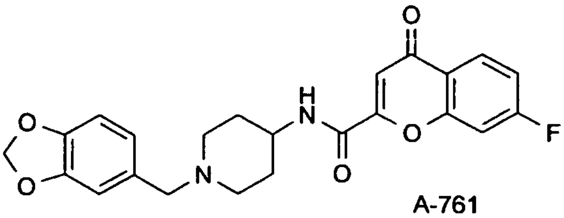

A-761

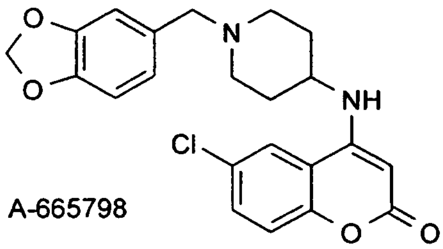

A-665798

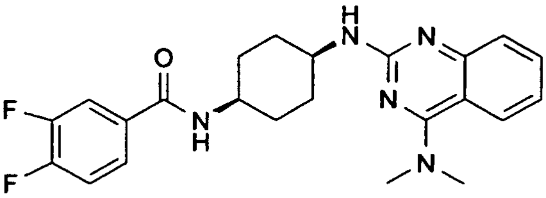

ATC-0175

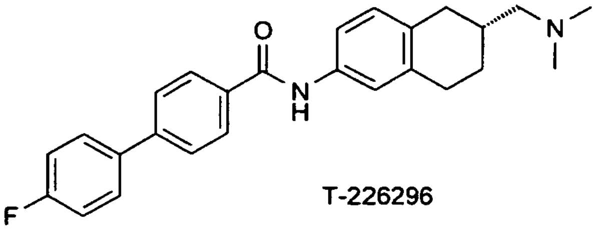

T-226296

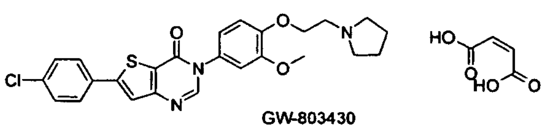

GW-803430

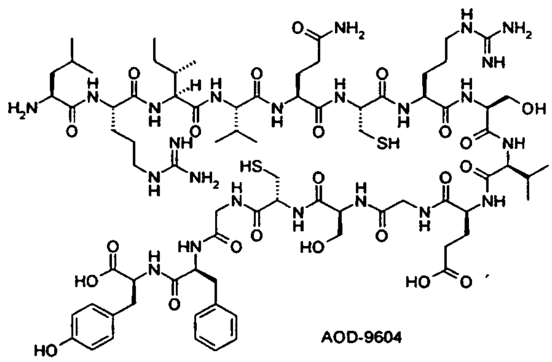

AOD-9604

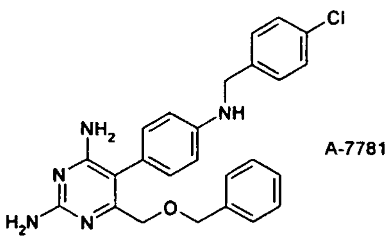

A-778193

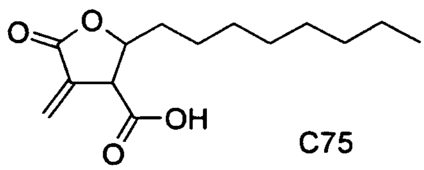

C75

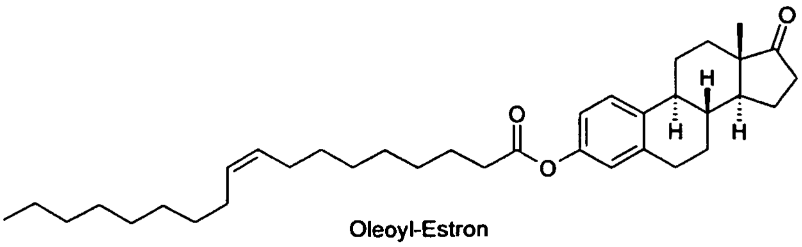

Oleoyl-Estron

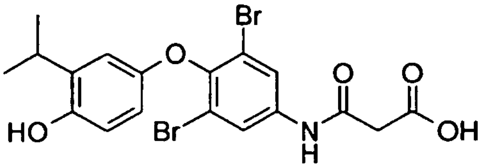

KB-2115

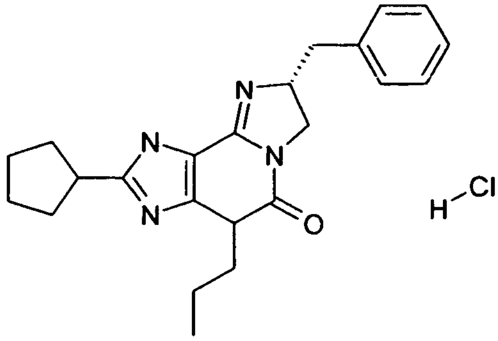

KCP-265

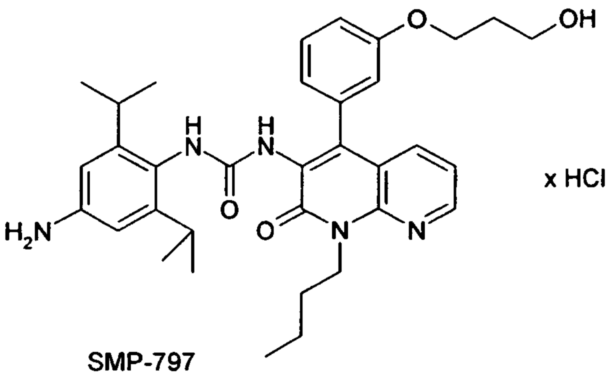

x HCl

SMP-797

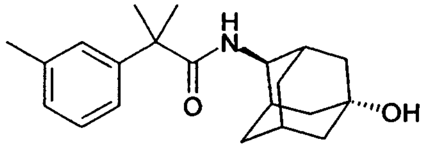

JNJ-25918646

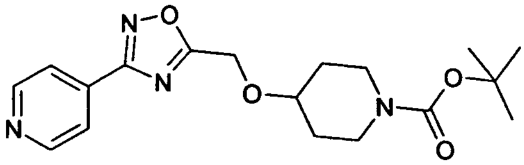

PSN-632408

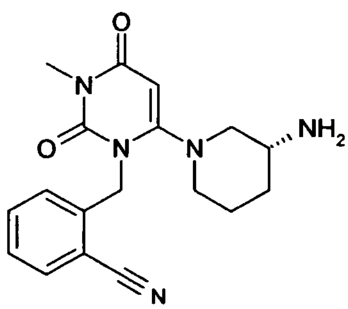

SYR-322

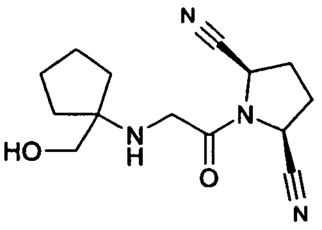

x HCl

DP-893

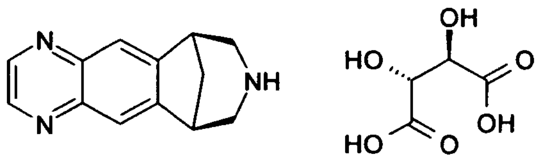

Varenicline Tartrat

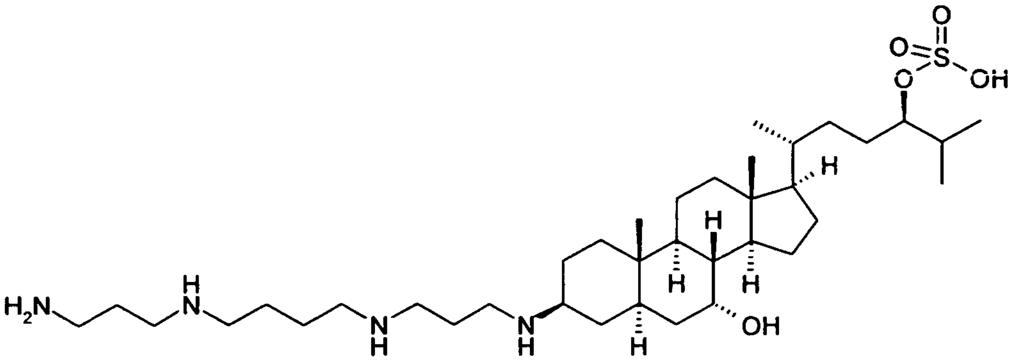

Trodusquemine

x HCl

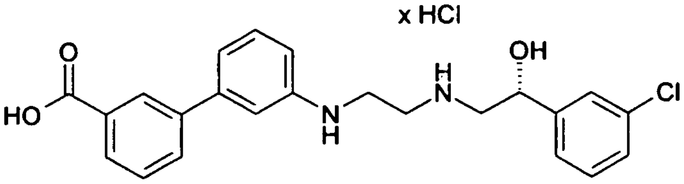

Solabegron

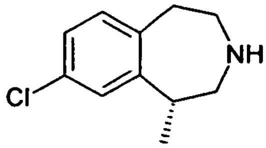

x HCl

Lorcaserin Hydrochlorid

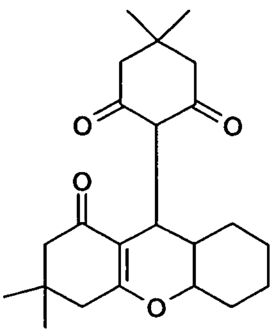

L-152804

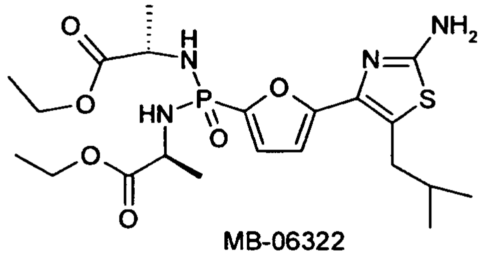

MB-06322
CS-917

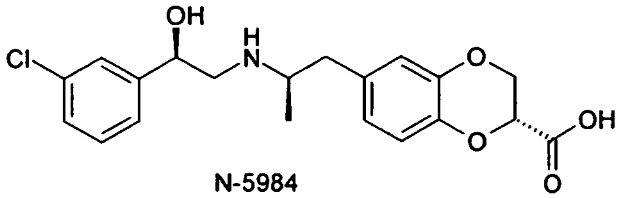

N-5984

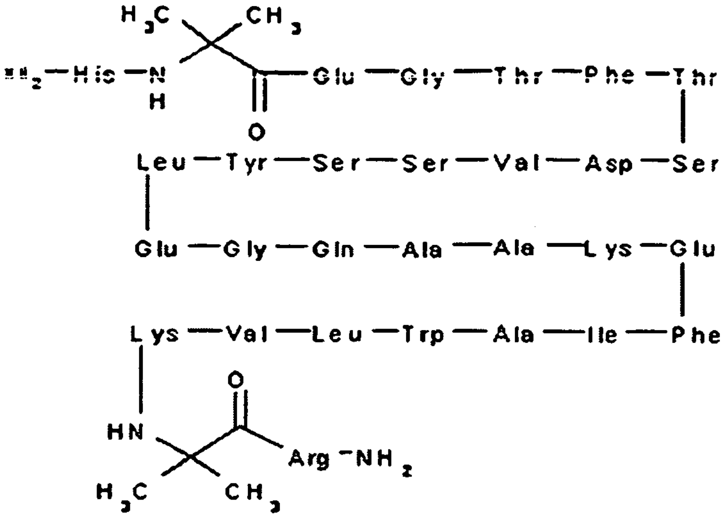

BIM-51077

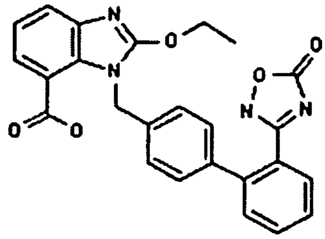

TAK-536

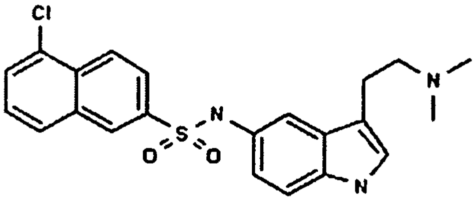

E-6837

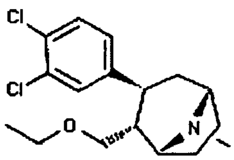

Tesofensine

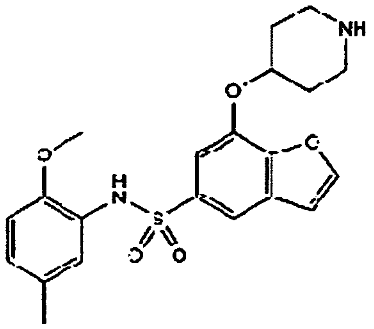

BVT-74316

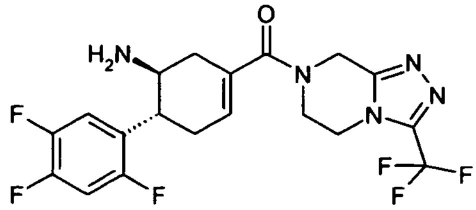

x $CF_3COOH$

ABT-341

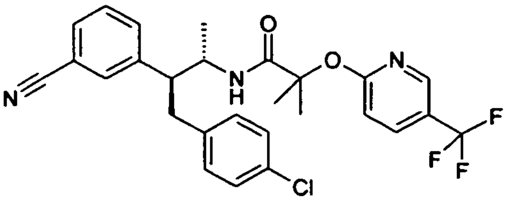

MK-0364

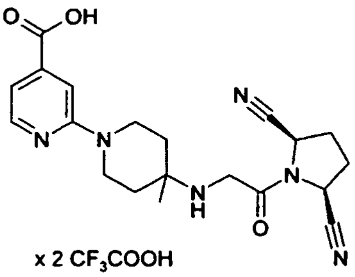

x 2 $CF_3COOH$

ABT-279

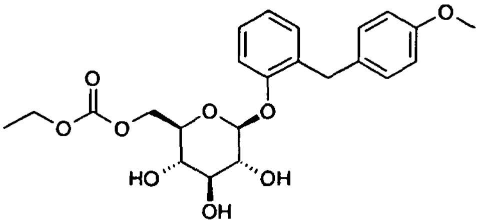

Sergliflozin

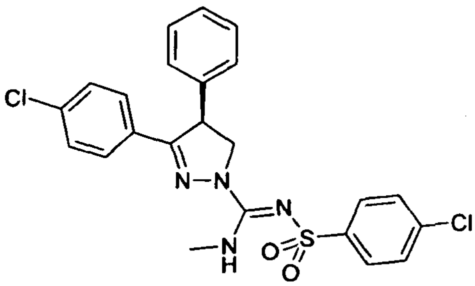

SLV-319

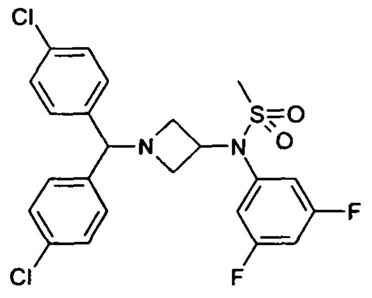

AVE 1625 (proposed INN: drinabant)

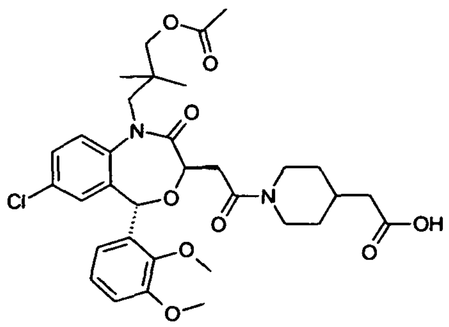

TAK-475 (Lapaquistat Acetat)

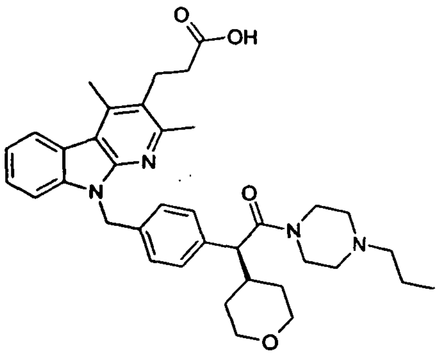

AS-1552133

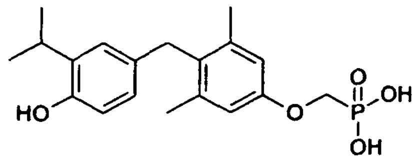

MB-07344

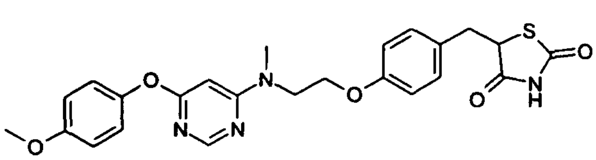

x $H_2SO_4$

CKD-501 (Lobeglitazon Sulfat)

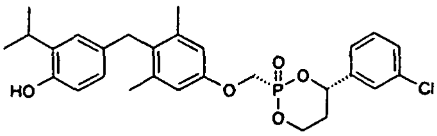

MB-07811

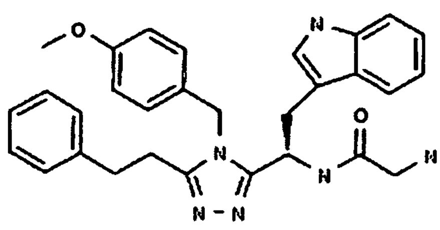

JMV-2959

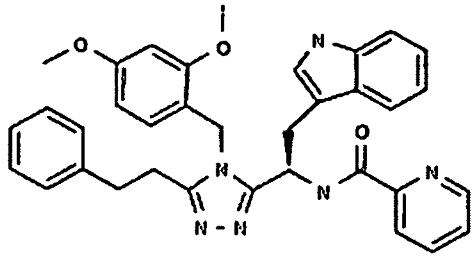

JMV-3002

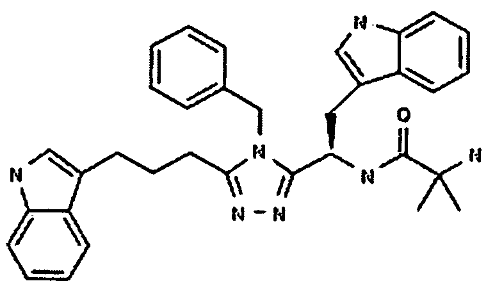

JMV-2810

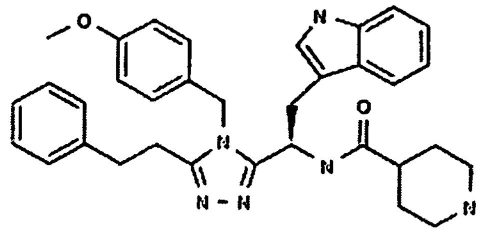

JMV-2951

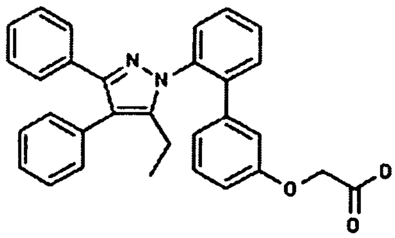

BMS-309403

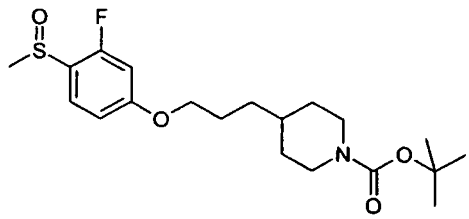

PSN-119-1

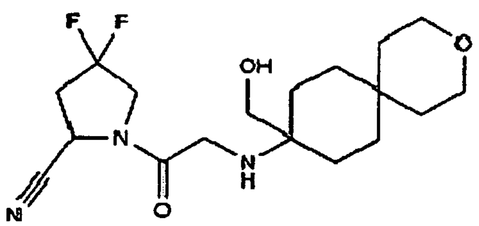

S-40755

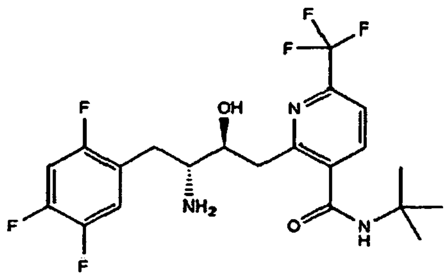

LY-2463665

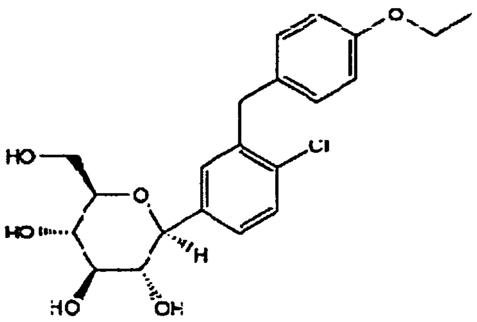

Dapagliflozin, BMS-512148

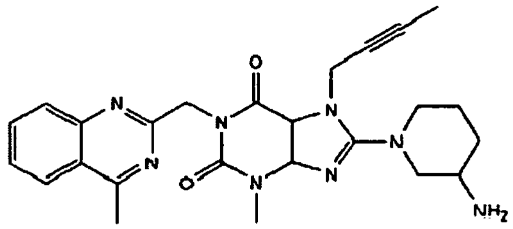

BI-1356

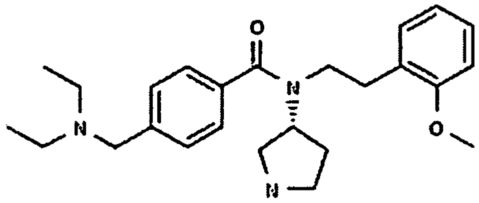

PF-429242

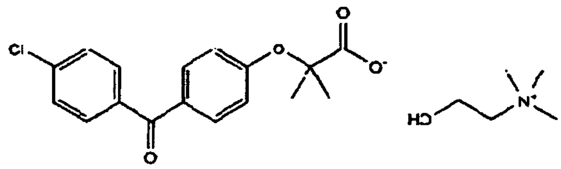

SLV-348

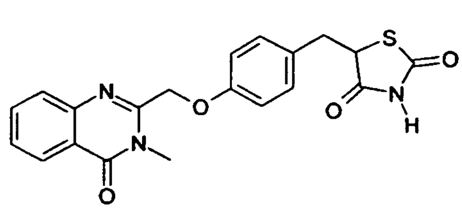

Balaglitazon

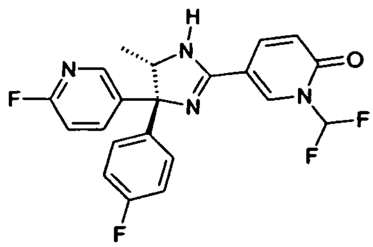

“NPY-5-BY”

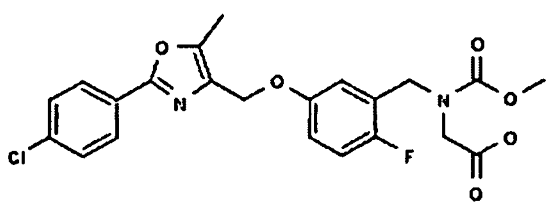

BMS-711939

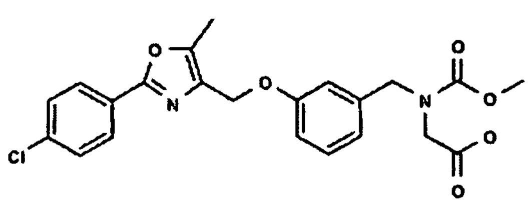

BMS-687453

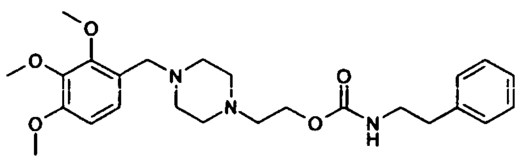

ST-3473

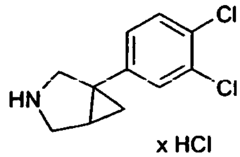

DOV-21947

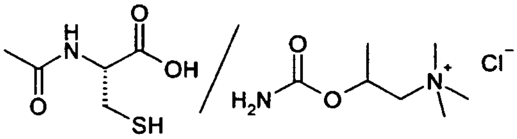

DM-71

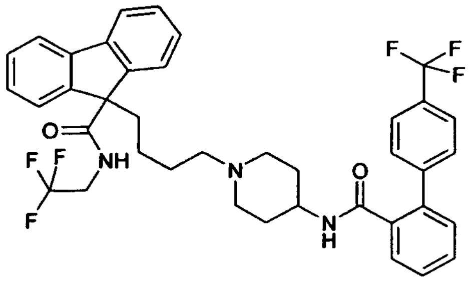

AEGR-733

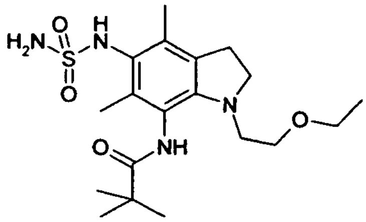

KY-382

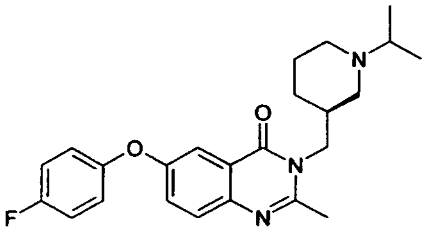

YIL-781

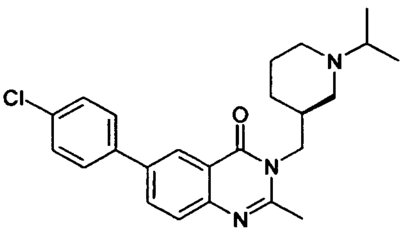

YIL-870

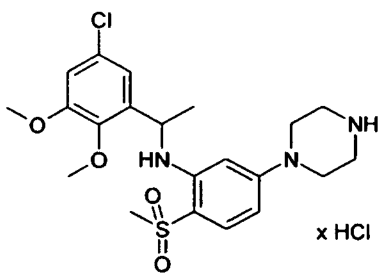

PRX-07034

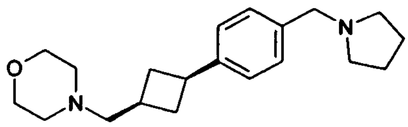

PF-00389027

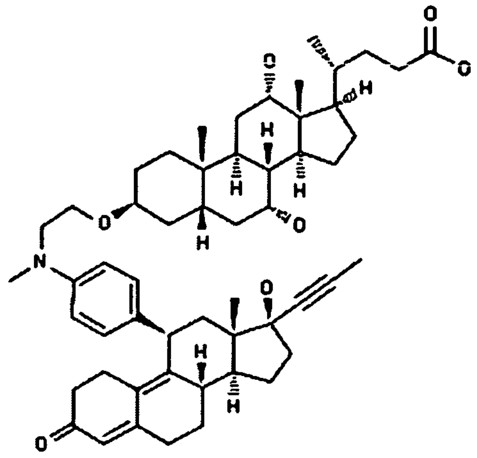

KB-3305

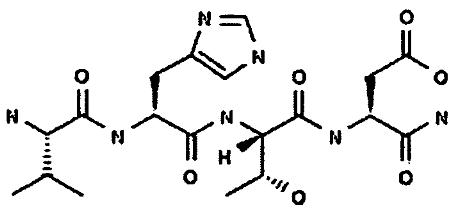

ISF-402

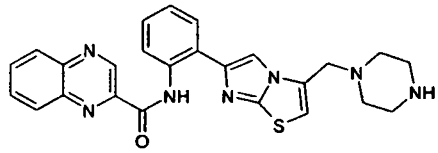

SRT-1720

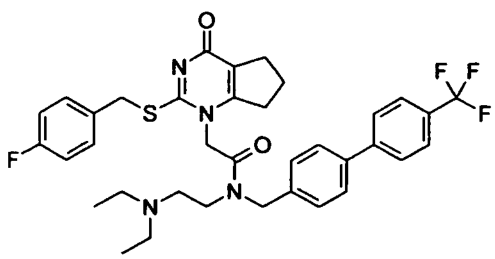

darapladib

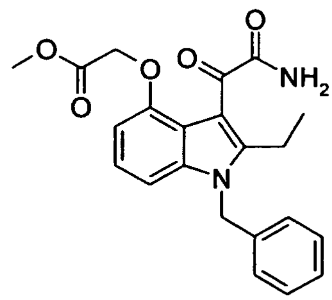

A-002

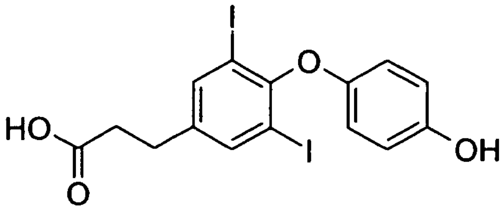

DITPA

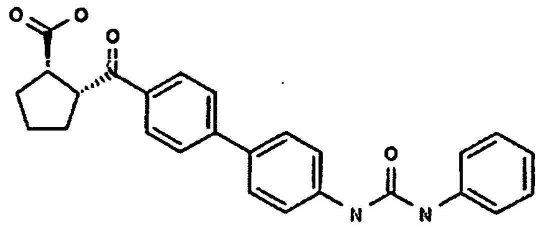

DGAT-1 Inhibitor aus WO2007137103

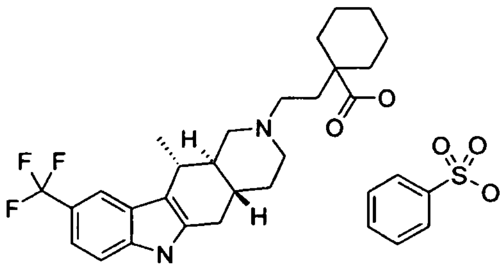

AMG-071

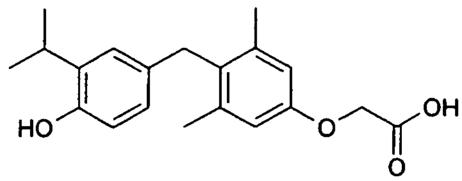

sobetirome

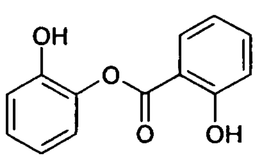

salsalate

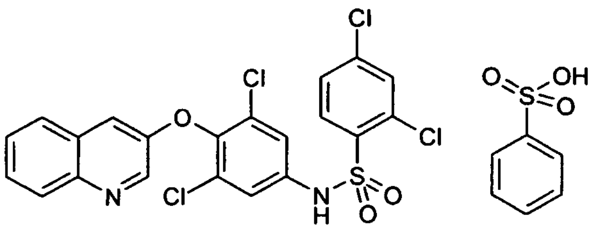

INT-131

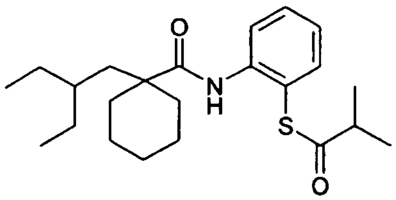

dalcetrapib

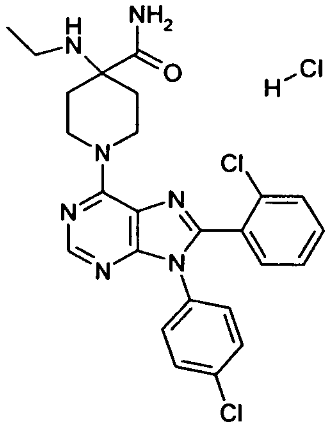

otenabant

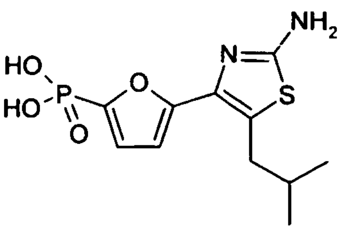

MB-07229

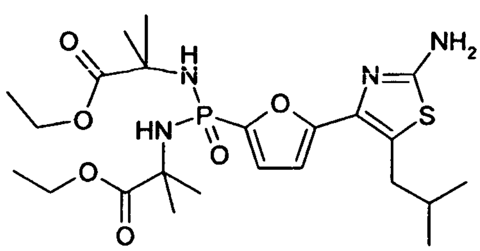

MB-07803

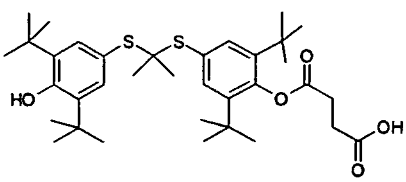

Succinobucol

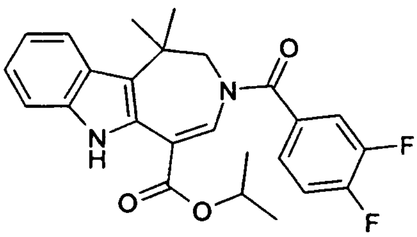

WAY-362450

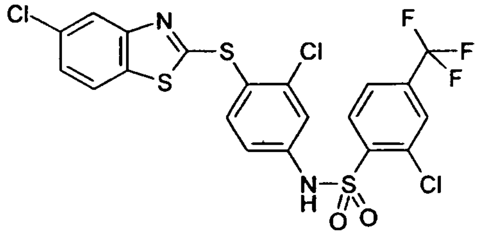

T-2384

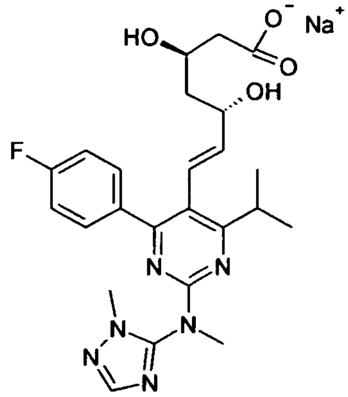

BMS-644950

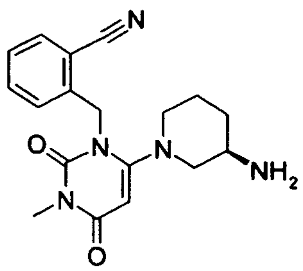

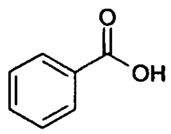

alogliptin benzoate

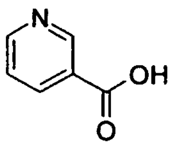

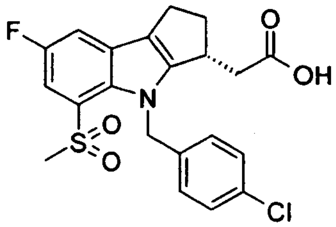

Nicotinsäure / Laropiprant

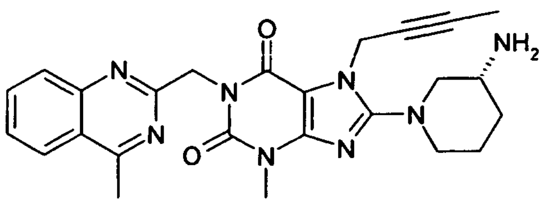

linagliptin

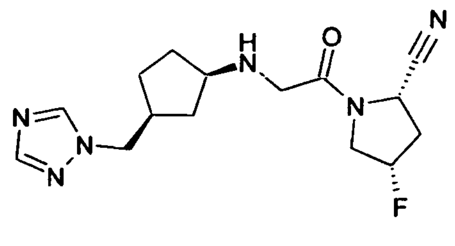

melogliptin

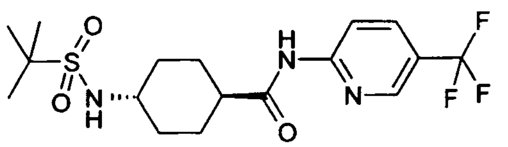

velneperit

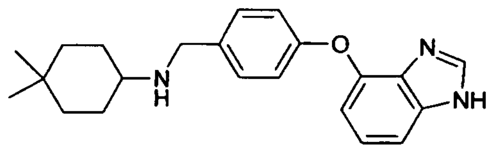

GSK-982

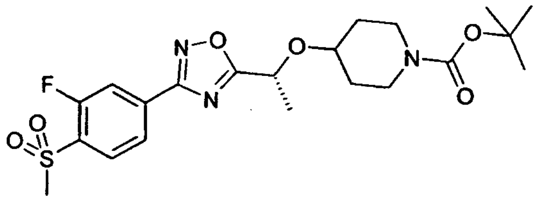

PSN-119-2

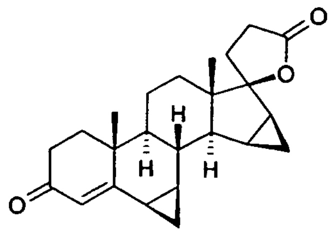

drospirenone

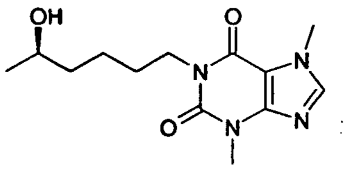

lisofylline

Weiterhin sind folgende Wirkstoffe für Kombinationspräparate geeignet:

Alle Antiepileptika, die in der Roten Liste 2007, Kapitel 15 genannt sind;
alle Antihypertonika, die in der Roten Liste 2007, Kapitel 17 genannt sind;
alle Hypotonika, die in der Roten Liste 2007, Kapitel 19 genannt sind;
alle Antikoagulantia, die in der Roten Liste 2007, Kapitel 20 genannt sind;
alle Arteriosklerosemittel, die in der Roten Liste 2007, Kapitel 25 genannt sind;
alle Betarezeptoren-, Calciumkanalblocker und Hemmstoffe des Renin-Angiotensin-Systems, die in der Roten Liste 2007, Kapitel 27 genannt sind;
alle Diuretika und Durchblutungsfördernde Mittel, die in der Roten Liste 2007, Kapitel 36 und 37 genannt sind;
alle Entwöhnungsmittel/Mittel zur Behandlung von Suchterkrankungen, die in der Roten Liste 2007, Kapitel 39 genannt sind;
alle Koronarmittel und Magen-Darm-Mittel, die in der Roten Liste 2007, Kapitel 55 und 60 genannt sind;
alle Migränemittel, Neuropathiepräparate und Parkinsonmittel, die in der Roten Liste 2007, Kapitel 61, 66 und 70 genannt sind.

Gegenstand der Erfindung sind weiterhin Verfahren zur Herstellung der Verbindungen der allgemeinen Formel I, dadurch gekennzeichnet, daß man die Verbindungen der Formel I so gewinnt, daß analog den folgenden Reaktionsschemata vorgegangen wird.

Verfahren "A":

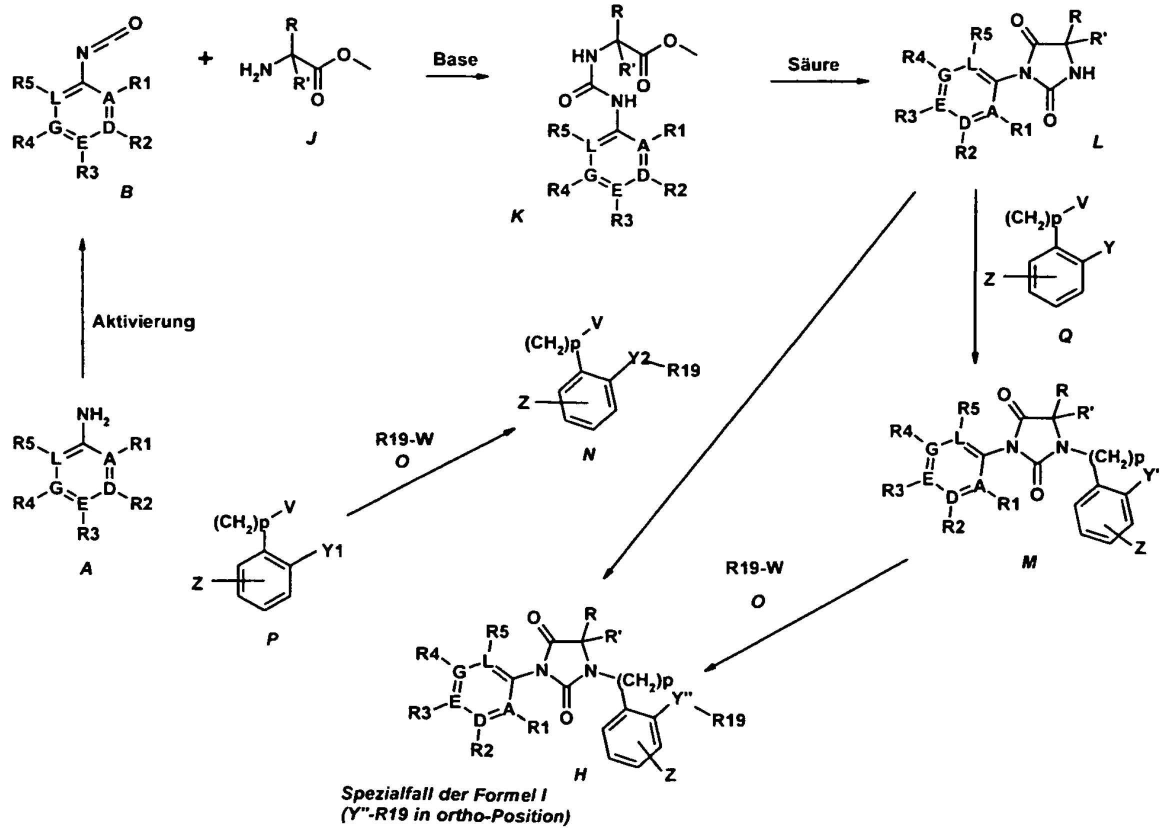


**Verfahren "A"**

In einem ersten Verfahren "A" wird so vorgegangen, dass ein geeignet substituiertes Anilin der Formel ***A,*** in welchem die Reste R1 bis R5 u. U. in geschützter Form vorliegen, in ein Isocyanat der Formel ***B*** umgesetzt wird. Diese Umsetzung kann z. B. mit Phosgen in Toluol oder mit Diphosgen oder Triphosgen durchgeführt werden. Das Isocyanat ***B*** wird anschließend mit dem Methylester oder einem anderen Ester (z.B. tert.-Butyl) der Aminosäure ***J,*** in welcher R und R' die in Formel I genannten Bedeutungen haben, oder einem Salz eines Esters der Aminosäure ***J*** unter Zugabe von Base (z. B. Triethylamin) zu einem Harnstoff der Formel ***K*** umgesetzt. Dieser Harnstoff kann unter basischen oder sauren Bedingungen, vorzugsweise sauren Bedingungen, zum Imidazolidin-2,4-dion der Formel ***L*** ringgeschlossen werden. Die weitere Umsetzung zu einer Verbindung der Formel ***H,*** welches den ortho-substituierten Spezialfall einer Verbindung der Formel I darstellt, kann z. B. so erfolgen, dass mit einer geeignet substituierten Verbindung ***Q,*** wobei Z ein oder mehrere Substituenten wie weiter oben in Formel I beschrieben sein kann, und Y entweder eine Nitro-Funktion ($-NO_2$) oder ein Halogenatom, vorzugsweise ein Bromatom, oder aber eine geeignet geschützte Aminofunktion (z. B. Isoindol-1,3-dion-2-yl oder $N=CH-N(CH_3)_2$), darstellt, und V entweder auch ein Halogenatom, vorzugsweise ein Chlor- oder Bromatom, oder aber zum Beispiel einen $O-SO_2-C_6H_4-4-CH_3$-Rest oder einen $O-SO_2-CH_3$-Rest oder einen $O-SO_2-CF_3$-Rest darstellt, unter Erhalt der Verbindung ***M*** alkyliert wird. Ist Y' in ***M*** Nitro ($NO_2$), so kann es in eine Verbindung ***M*** mit Y' gleich Amino ($NH_2$) durch Reduktion überführt werden. Ist Y' in ***M*** eine geschützte Aminofunktion, so kann sie durch schutzgruppenspezifische Abspaltung in eine freie Aminofunktion umgewandelt werden. Ist Y' in ***M*** ein Halogenantom, bevorzugt ein Bromatom, so kann es unter Buchwald-Hartwig-Bedingungen (z. B.: S.L.Buchwald et al.: Acc. Chem. Res. 1998, 31, 805; J.F.Hartwig et al.: J. Org. Chem. 1999, 64, 5575-5580; J.P.Wolfe et al.: J. Org. Chem. 2000, 65, 144-1157; M.D.Charles et al.: Org. Lett. 2005, 7, 3965-68) z.B. durch Umsatz mit Benzophenonimin und nachfolgender Hydrolyse in eine Verbindung der Formel ***M*** mit Y' gleich Amino ($NH_2$) umgewandelt werden. Die weitere Umsetzung zu Verbindungen der Formel ***H*** kann durch Reaktion mit Isocyanaten der Formel ***O,*** wobei W gleich -N=C=O ist, erfolgen.

Alternativ kann eine Verbindung der Formel ***M,*** worin Y' gleich $NH_2$ ist, mit z.B. Phosgen oder Phosgenäquivalenten in ein Isocyanat, worin Y' -N=C=O bedeutet, überführt werden, welches anschließend mit einer Verbindung der Formel ***O***, worin W $NH_2$ bedeutet, umgesetzt werden kann.

Oder die weitere Umsetzung der Verbindung ***L*** zur Verbindung ***H*** kann so erfolgen, dass ***L*** mit einer Verbindung der Formel ***N,*** wobei V die eben geschilderten Bedeutungen haben kann, und wobei Y2 die Bedeutung NR23-CO-NR23

(für den Fall der Harnstoff-verbrückten Verbindungen) haben kann, alkylierend umgesetzt wird. Die Verbindung ***N*** ihrerseits kann durch Reaktion von ***P,*** worin V die oben beschriebenen Bedeutungen haben kann, und wobei Y1 $NH_2$ bedeutet, mit einer eventuell substituierten R19-W-Verbindung ***O,*** worin W die Bedeutung -N=C=O hat erhalten werden. R19 hat die Bedeutung substituiertes oder unsubstituiertes Aryl, Heteroaryl oder bicyclisches Heteroaryl.
Eventuell vorhandene Schutzgruppen der Verbindung ***H*** können am Ende entfernt werden. Die hier gezeigte Formel ***H*** stellt einen Spezialfall der Formel I dar, worin sich der Rest Y"-R19 in Formel I in der ortho-Position befindet; dieser Rest kann sich sinngemäß auch in meta- oder para-Position befinden.
Das hier beschriebene Verfahren stellt den Spezialfall dar, worin Y2 bzw. Y" eine Harnstoffverbrückung (-NR23-CO-NR24-) beschreibt. Sinngemäß kann das Verfahren auch für andere mit "T" verbrückte Verbindungen angewandt werden. Die Bedeutung von "T" ist weiter oben beschrieben.

Eine Variante des Verfahrens "A" stellt das Verfahren "A'" dar:

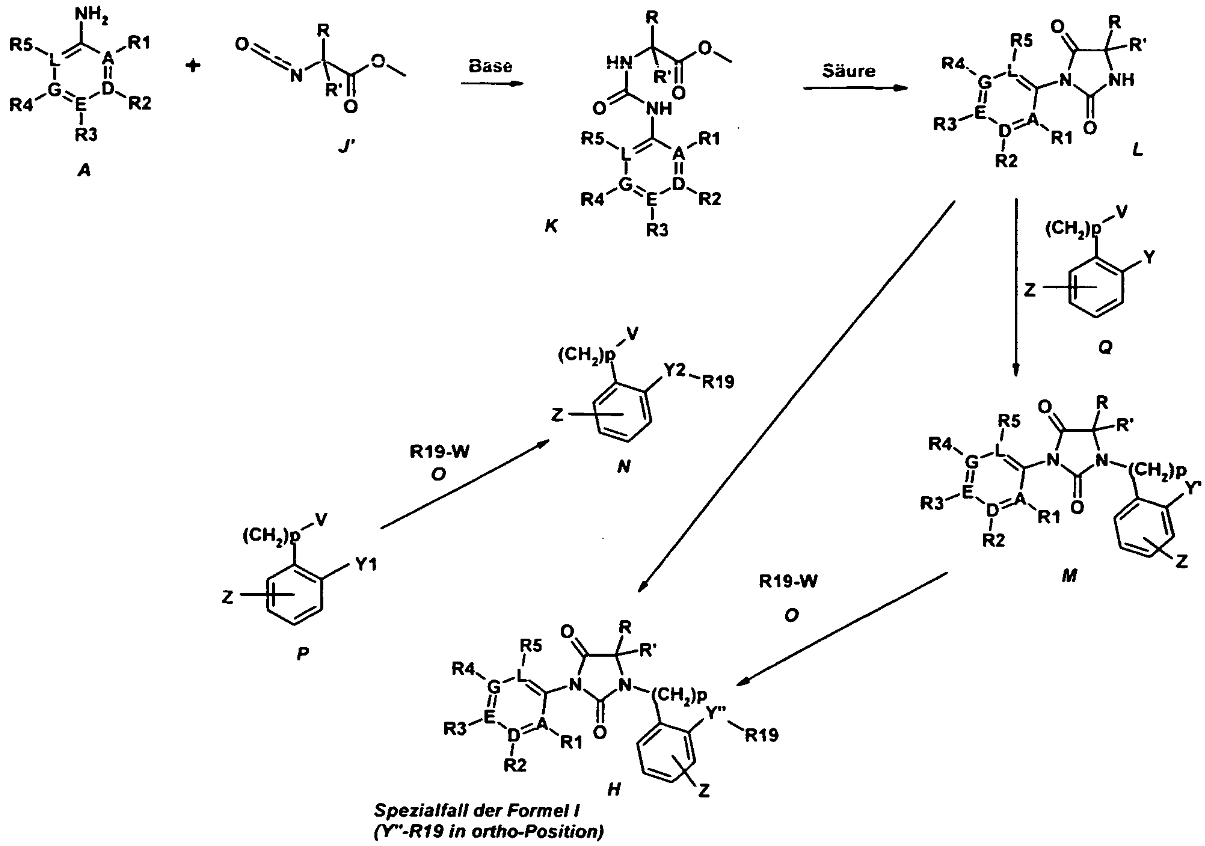


**Verfahren „A'"**

Im Verfahren "A'" wird das Amin ***A*** mit dem Isocyanat des Aminosäureesters ***J'*** unter Bildung der Verbindung ***K*** zur Reaktion gebracht. Die weiteren Schritte können wie bei Verfahren "A" erfolgen.

In einem anderen Verfahren "B"

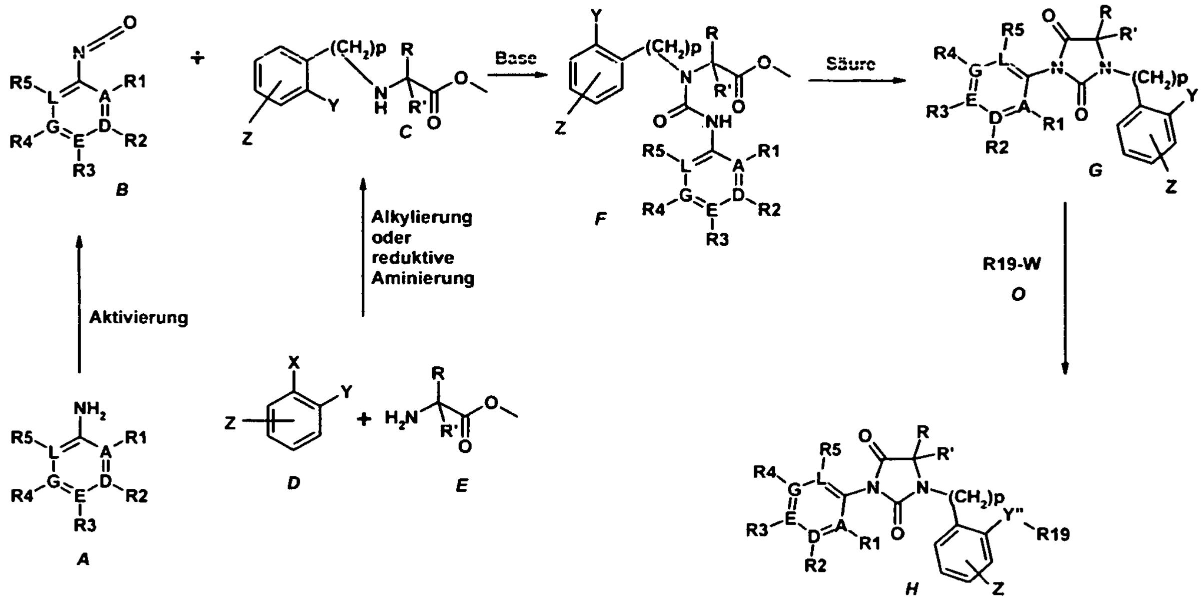


*Spezialfall der Formel I*
*(Y"-R19 in ortho-Position)*

## Verfahren „B“

wird das Isocyanat ***B*** mit einem geeignet substituierten Aminosäureesterderivat ***C,*** worin die jeweiligen Substituenten gegebenenfalls mit Schutzgruppen versehen sind, und wobei der im Schema gezeigte Methylester ein nicht limitierendes Beispiel für einen Ester ist, und wobei Y Brom oder eine geschützte Aminofunktion (z. B. N-CO-$CH_3$ oder N=CH-N$(CH_3)_2$) ist unter Zugabe einer Base (z. B. Triethylamin) zu einem Harnstoff der Formel ***F*** umgesetzt. Das Aminosäureesterderivat ***C*** kann aus der Verbindung ***D,*** worin Z ein oder mehrere Substituenten wie weiter oben in Formel I beschrieben sein kann, und wobei Y Brom oder eine geschützte Aminofunktion und X eine $(CH_2)_p$-U-Gruppierung ist, worin U die Bedeutung Cl, Br, J, O-$SO_2$-$C_6H_4$-4-$CH_3$, O-$SO_2$-$CH_3$ oder O-$SO_2$-$CF_3$ haben kann, mit einem Aminosäureester der Formel ***E,*** worin R und R' die in Formel I genannten Bedeutungen haben, unter alkylierenden Bedingungen hergestellt werden. Alternativ kann die Verbindung der Formel ***C*** durch reduktive Aminierung des Aldehyds ***D*** (Z und Y wie oben beschrieben und X = $(CH_2)_{(p-1)}$-CHO mit dem Aminosäurederivat ***E*** gewonnen werden. Der Harnstoff ***F*** kann unter basischen oder sauren Bedingungen, vorzugsweise sauren Bedingungen, zum Imidazolidin-2,4-dion der Formel ***G*** ringgeschlossen werden. Verbindungen der Formel ***G,*** worin Y Brom bedeutet, können in Verbindungen der Formel ***G***, worin Y $NH_2$ bedeutet, nach der im Verfahren "A" beschriebenen Methode hergestellt werden.
Die Umsetzung von ***G*** nach ***H*** kann anschließend nach den im Verfahren "A" beschriebenen Methoden durchgeführt werden.
Eventuell vorhandene Schutzgruppen der Verbindung ***H*** können am Ende entfernt werden. Die hier gezeigte Formel ***H*** stellt einen Spezialfall der Formel I dar, worin sich der Rest Y"-R19 in Formel I in der ortho-Position befindet; dieser Rest kann sich sinngemäß auch in meta- oder para-Position befinden.
Das hier beschriebene Verfahren stellt den Spezialfall dar, worin Y" eine Harnstoffverbrückung (-NR23-CO-NR24-) beschreibt. Sinngemäß kann das Verfahren auch für andere mit "T" verbrückte Verbindungen angewandt werden. Die Bedeutung von "T" ist weiter oben beschrieben.

In einem weiteren Verfahren (Verfahren "C")

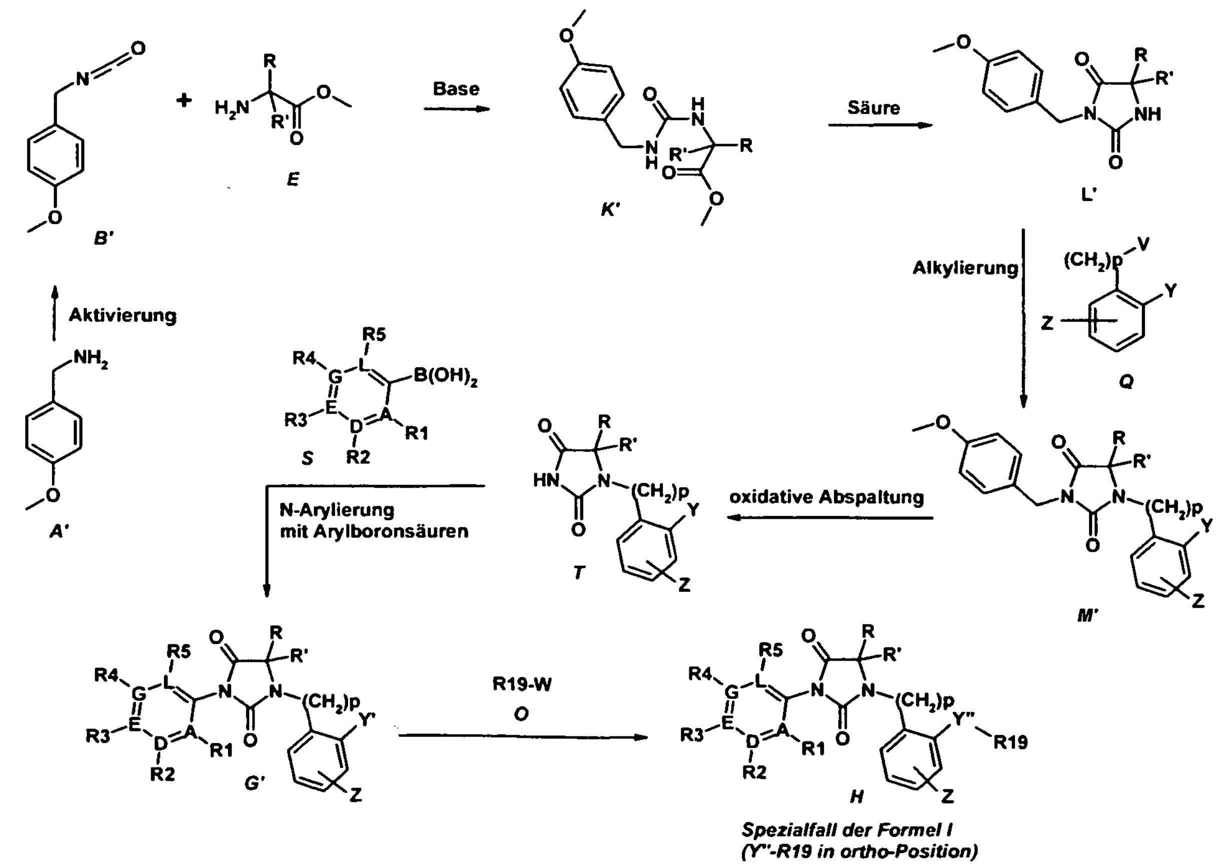


## Verfahren „C“

wird p-Methoxybenzylisocyanat ***B'*** mit einem Aminosäureester wie z. B. ***E***, in welchem R und R' die in Formel I genannten Bedeutungen haben, unter basischen Bedingungen zum Harnstoff ***K'*** umgesetzt. Der Harnstoff ***K'*** kann unter basischen oder sauren Bedingungen, vorzugsweise sauren Bedingungen, zum Imidazolidin-2,4-dion der Formel ***L'*** ringgeschlossen werden. Die Verbindungen ***M'*** werden gewonnen, indem die Verbindungen ***L'*** mit den Verbindungen ***Q*** unter alkylierenden Bedingungen umgesetzt werden. Dabei haben Z, V und Y der Verbindungen ***Q*** die Bedeutungen wie sie im Verfahren "A" genannt sind. Die p-Methoxybenzylgruppe in den Verbindungen ***M'*** kann oxidativ unter Erhalt der Verbindungen ***T*** abgespalten werden. Die N-Arylierung des Imidstickstoffatoms in Verbindungen der Formel ***T*** unter Einsatz von Arylboronsäuren der Formel ***S*** nach Verfahren wie sie z. B. bei J.-B.Lan et al.: SYNLETT 2004, 1095-1097 oder D.M.T.Chan et al.: Tetrahedron Lett. 1998, 39, 2933-2936 beschrieben sind, liefert Verbindungen der Formel ***G'***. Verbindungen der Formel ***G'*** können unter den verschiedenen Bedingungen wie sie in Verfahren "A" für die Umwandlung von ***M*** nach ***H*** genannt sind, in Verbindungen der Formel ***H*** überführt werden.
Eventuell vorhandene Schutzgruppen der Verbindung ***H*** können am Ende entfernt werden.
Die hier gezeigte Formel ***H*** stellt einen Spezialfall der Formel I dar, worin sich der Rest Y"-R19 in Formel I in der ortho-Position befindet; dieser Rest kann sich sinngemäß auch in meta- oder para-Position befinden.
Das hier beschriebene Verfahren stellt den Spezialfall dar, worin Y" eine Harnstoffverbrückung (-NR23-CO-NR24-) beschreibt. Sinngemäß kann das Verfahren auch für andere mit "T" verbrückte Verbindungen angewandt werden. Die Bedeutung von "T" ist weiter oben beschrieben.

Ein weiteres Verfahren "D" findet insbesondere Anwendung in der Synthese von alkyl-, cycloalkyl-, cycloalkenyl, arylalkylen-, heteroarylalkylen-, aryloxy-, heteroaryloxy, alkyloxy-, alkylthio-, cycloalkylthio-, arylthio-, heteroarylthio-, alkylcarbonyl-, cycloalkylcarbonyl-, arylcarbonyl-, heteroarylcarbonyl-, aryl- und heteroaryl-substituierten N3-aryl- oder N3-heteroaryl-substituierten Imidazolidin-2,4-dionen.

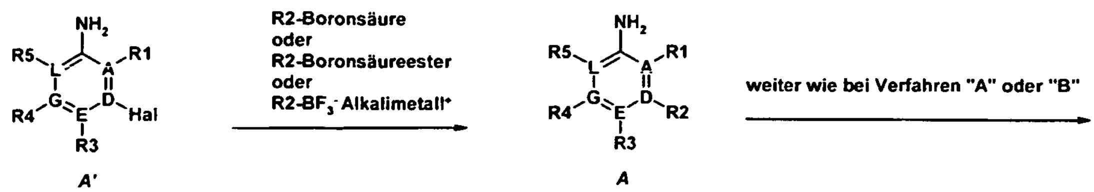


R2 = Halogen

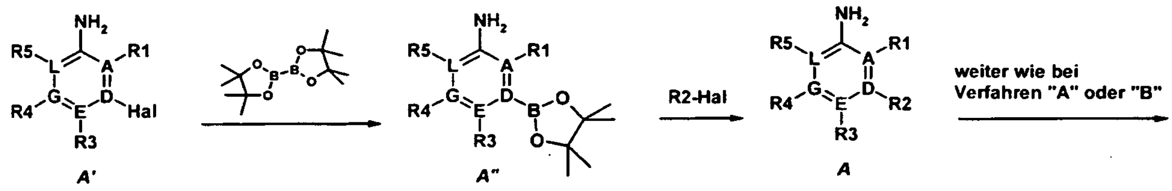


R2 = Halogen

## Verfahren „D"

Zur Herstellung von Verbindungen, worin z. B. R2 = Alkyl, Cycloalkyl, Cycloalkenyl, Aryl oder Heteroaryl oder einen anderen der oben beschriebenen Reste darstellt, kann so vorgegangen werden, dass eine Verbindung der Formel ***A'***, worin die Aminofunktion gegebenenfalls mit einer Schutzgruppe versehen ist und R2 gleich Halogen, bevorzugt Brom oder Chlor, ist, mit einer Alkyl-, Cycloalkyl, Cycloalkenyl-, Aryl- oder Heteroarylboronsäure oder einem Esterderivat davon oder einem R2-Trifluoroborat unter Bedingungen umgesetzt wird wie sie z.B. bei J. Zhou und G. C. Fu, J. Am. Chem. Soc. 126 (2004) 1340-1341; F. Gonzäles-Bobes und G. C. Fu, J. Am. Chem. Soc. 128 (2006) 5360-5361; D. J. Wallace und C.-Y. Chen, Tetrahedron Letters 43 (2002) 6987-6990; T. E. Barder et al., J. Am. Chem. Soc. 127 (2005) 4685-4696; D. W. Old et al., J. Am. Chem. Soc. 120 (1998) 9722; T. E. Barder und St. L. Buchwald, Org. Lett. 6 (2004) 2649-2652 beschrieben sind. Die weitere Umsetzung der so mit R2 substituierten Verbindung A kann so erfolgen wie es für das Verfahren "A" und "B" beschrieben ist.

Bei dem Verfahren "D" kann auch so vorgegangen werden, dass die Verbindung ***A'***, wobei R2 gleich Halogen, bevorzugt Chlor oder Brom, ist, unter Palladium-Katalyse mit einer Dibor-Verbindung, z. B. Bis(pinacolato)dibor, zum Arylboronat der Formel ***A*** " mit R2 gleich

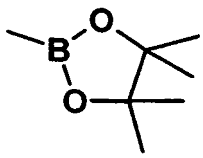

umgesetzt wird. In einem weiteren Schritt kann diese Verbindung mit einer Organohalogenverbindung R2-Hal zu Verbindungen der Formel ***A***, worin R2 z. B. Cycloalkyl oder Aryl bedeutet, umgesetzt werden. Die Folgereakionen zur Gewinnung der Verbindungen der Formel ***H*** können wiederum nach Verfahren "A" oder "B" erfolgen.

Verbindungen der Formel ***A***, worin R2 gleich -O/S-Alkyl, -O/S-Cycloalkyl, -O/S-$CH_2$-Aryl, - O/S-$CH_2$-Heteroaryl, -O/S-Aryl, -O/S-Heteroaryl ist, können aus Verbindungen der Formel ***A***', worin R2 gleich Halogen, bevorzugt Brom oder Chlor, ist, durch Umsatz mit den entsprechenden Alkoholen oder Phenolen bzw. Mercaptanen oder Mercaptoarylen und - heteroarylen und Cäsiumcarbonat unter Palladium- oder Kupfer-Katalyse hergestellt werden (siehe auch R. Frlan und D. Kikelj; Synthesis 14 (2006) 2271-2285; A. V. Vorogushin et al., J. Am. Chem. Soc. 127 (2005) 8146-8149; F. Y. Kwong und St. L. Buchwald, Org. Lett. 4 (2002) 3517-3520).

Verbindungen der Formel ***A***, worin R2 gleich -$CH_2$-Aryl oder -$CH_2$-Heteroaryl ist, können z.B. aus Verbindungen der Formel ***A*** ", durch Umsatz mit Halogenmethylarylen oder Halogenmethylheteroarylen, wobei Halogen bevorzugt Brom oder Chlor ist, unter basischen Bedingungen und Palladium-Katalyse erhalten werden (siehe auch S. M. Nobre

und A. L. Monteiro, Tetrahedron Letters 45 (2004) 8225-8228; S. Langle et al., Tetrahedron Letters 44 (2003) 9255-9258).

Die hier beschriebenen Verfahren sind für den Spezialfall der Harnstoffverbrückung (-NR23-CO-NR24-) ausgeführt. Verbindungen mit anderen beanspruchten Verbrückungen können sinngemäß hergestellt werden.

Die nachfolgenden Beispiele dienen zur näheren Erläuterung der Erfindung, ohne dieselbe auf in den Beispielen beschriebene Produkte und Ausführungsformen einzuschränken.

Allgemeine experimentelle Verfahren:

$^1$H-NMR:

Die $^1$H-NMR Spektren wurden in deuteriertem Dimethylsulfoxid an einem 500 MHz-Gerät (DRX 500, Firma Bruker) oder an einem 400 MHz-Gerät (DRX 400, Firma Bruker) bei 300°K gemessen. Angaben: δ in ppm, Multiplizität (s für Singulett, d für Dublett, t für Triplett, q für Quartett, m für Multiplett, x H (Anzahl der Wasserstoffatome)

HPLC/MS:

Die HPLC-MS-Messungen wurden an einem LCT-Gerät der Firma Waters durchgeführt. Säule: YMC Jshere 33x2 4 μm; Gradient [A]: (Acetonitril+ 0.05% Trifluoressigsäure) : (Wasser + 0.05% Trifluoressigsäure) 5:95 (0 Minuten) nach 95:5 (3 Minuten); Gradient [B]: (Acetonitril+ 0.05% Trifluoressigsäure) : (Wasser + 0.05% Trifluoressigsäure) 5:95 (0 Minuten) nach 95:5 (2.5 Minuten) nach 95:5 (3.0 Minuten); Gradient [C]: (Acetonitril+ 0.05% Trifluoressigsäure) : (Wasser + 0.05% Trifluoressigsäure) 5:95 (0 Minuten) nach 95:5 (3.4 Minuten) nach 95:5 (4.4 Minuten); Gradient [D]: (Acetonitril+ 0.05% Trifluoressigsäure) : (Wasser + 0.05% Trifluoressigsäure) 2:98 (1 Minute) nach 95:5 (5 Minuten) nach 95:5 (6.25 Minuten); Gradient [E]: (Acetonitril+ 0.05% Trifluoressigsäure) : (Wasser + 0.05% Trifluoressigsäure) 5:95 (0 Minuten) nach 5:95 (0.5 Minuten) nach 95:5 (3.5 Minuten) nach 95:5 (4.0 Minuten); Gradient [F]: Säule: YMC Jsphere ODS H80 20x2 mm, 4 μm; (Wasser + 0.05% Trifluoressigsäure) : (Acetonitril + 0.05% Trifluoressigsäure) 96:4 (0 Minuten) nach 5:95 (2 Minuten) nach 96:4 (2.4 Minuten); Detektor: Tecan-LCT.

Chromatographische Reinigungsmethoden:

[RP1]: Fluss: 30 ml/min; Gradient: Acetonitril/Wasser + 0,1% Trifluoressigsäure; 30 min.
Säule: XTerra C18 5 μm 30x100 mm; Detektion: MS (ESI), UV (DAD).

[RP2]: Fluss: 150 ml/min; Gradient: Acetonitril/Wasser + 0,1 % Trifluoressigsäure; 20 min.
Säule: XTerra C 18 10 μm 50x250 mm; Detektion: MS (ESI), UV (DAD).

**Beispiel 1:**

4-(3-{2-[3-(4-Cyano-3-trifluoromethyl-phenyl)-5,5-dimethyl-2,4-dioxo-imidazolidin-1-ylmethyl]-phenyl}-ureido)-benzolsulfonsäureamid

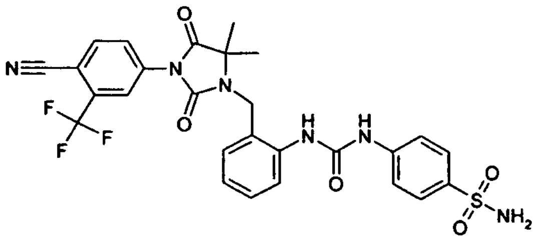

1) Herstellung von 4-(4,4-Dimethyl-2,5-dioxo-imidazolidin-1-yl)-2-trifluoromethyl-benzonitril (**1.1**):

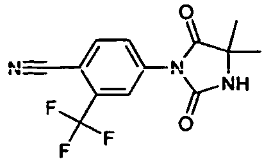

Die Verbindung **1.1** kann nach Verfahren "A" dargestellt werden. Dazu wurden 14,74 g (79,21 mMol) 4-Amino-2-trifluoromethyl-benzonitril in 200 ml trockenem Acetonitril gelöst. Diese Lösung wurde unter Rühren zu einer auf 70°C erwärmten 20%igen Lösung von Phosgen in Toluol zugetropft und anschließend 1 h gerührt. Die abgekühlte Reaktionslösung wurde im Vakuum eingeengt, der Rückstand mit Toluol aufgenommen und wieder im Vakuum eingeengt. Schließlich wurde der Rückstand in 150 ml trockenem Acetonitril gelöst und die Lösung unter Rühren mit 15,5 g (79,21 mMol) 2-Amino-2-methyl-propionsäure-tert.-butylester Hydrochlorid versetzt. Zu die Reaktionsmischung wurden langsam 12,02 g (118,8 mMol) Triethylamin zugetropft und anschließend 45 min. bei Raumtemperatur gerührt. Danach wurde die Mischung vorsichtig mit 50 ml konzentrierter Salzsäure versetzt und 1 h bei 70°C gerührt. Die abgekühlte Reaktionsmischung wurde im Vakuum eingeengt und der Rückstand mit Essigsäureethylester und Wasser versetzt. Die organische Phase wurde abgetrennt, mit gesättigter Natriumhydrogen-carbonatlösung und anschließend mit Wasser gewaschen, über Natriumsulfat getrocknet, filtriert und im Vakuum eingeengt. Der Rückstand wurde chromatographisch über Kieselgel mit Heptan / Essigsäureethylester 2:1 gereinigt. Man erhielt 21,2 g (90% Ausbeute) 4-(4,4-Dimethyl-2,5-dioxo-imidazolidin-1-yl)-2-trifluoromethyl-benzonitril **1.1** mit dem Schmelzpunkt 208 - 211 °C.

2) Herstellung von 4-[3-(2-Brombenzyl)-4,4-dimethyl-2,5-dioxo-imidazolidin-1-yl]-2-trifluoromethyl-benzonitril (**1.2**):

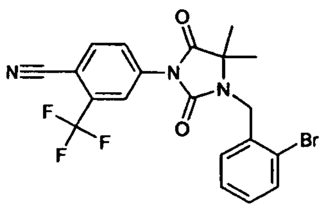

Die Verbindung **1.2** kann nach Verfahren "A" dargestellt werden. Dazu wurden 21,2 g (71,32 mmol) der Verbindung **1.1** und 17,83 g (71,32 mMol) 2-Brombenzylbromid in 200 ml trockenem Acetonitril gelöst, mit 12,32 g Kaliumcarbonat versetzt und 5 h bei Raumtemperatur gerührt. Zur Aufarbeitung wurde die Reaktionsmischung mit Wasser versetzt, die Mischung mit Essigsäureethylester ausgeschüttelt, die organische Phase über Natriumsulfat getrocknet, filtriert und im Vakuum eingeengt. Der Rückstand wurde chromatographisch über Kieselgel mit Heptan / Essigsäureethylester 3:1 gereinigt. Man erhielt 28,5 g (86% Ausbeute) 4-[3-(2-Brom-benzyl)-4,4-dimethyl-2,5-dioxo-imidazolidin-1-yl]-2-trifluoromethyl-benzonitril (**1.2**) mit dem Schmelzpunkt 56 - 58°C.

3) Herstellung von 4-[3-(2-Amino-benzyl)-4,4-dimethyl-2,5-dioxo-imidazolidin-1-yl]-2-trifluoromethyl-benzonitril **1.3:**

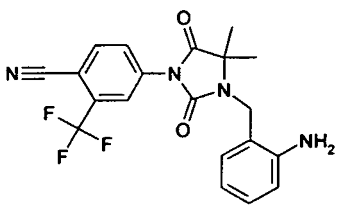

Die Verbindung **1.3** kann nach Verfahren "A" hergestellt werden. 370 mg (0,794 mmol) der Verbindung des

Beispiels **1.2** wurden mit 216 mg Benzophenonimin, 776 mg Cäsiumcarbonat, 9 mg Palladium-II-acetat und 46 mg 9,9-Dimethyl-4,5-bis(diphenyl-phosphino)-xanthen unter einer Argonatmosphäre mit 2,8 ml trockenem Dioxan versetzt. Die Mischung wurde 6 h bei 95° C gerührt; zur abgekühlten Reaktionsmischung wurden 7,5 ml 1 N Salzsäure zugegeben. Die Mischung wurde 10 min. bei Raumtemperatur gerührt und mit wässriger Natronlauge neutralisiert. Zur Aufarbeitung wurde die Reaktionsmischung 3 x mit Dichlormethan ausgeschüttelt, die organische Phase wurde über Magnesiumsulfat getrocknet, filtriert und im Vakuum eingeengt. Der Rückstand wurde chromatographisch gereinigt (Methode [RP2]). Man erhielt 4-[3-(2-Amino-benzyl)-4,4-dimethyl-2,5-dioxo-imidazolidin-1-yl]-2-trifluoromethyl-benzonitril **1.3** in 78% Ausbeute. Molekulargewicht 402,13 ($C_{20}H_{17}F_3N_4O_2$); Retentionszeit $R_1$ = 1.61 min. [B]; MS (ESI): 403,06 ($MH^+$).

4) Herstellung von 4-(3-{2-[3-(4-Cyano-3-trifluoromethyl-phenyl)-5,5-dimethyl-2,4-dioxo-imidazolidin-1-ylmethyl]-phenyl}-ureido)-benzolsulfonsäurechlorid **1.4:**

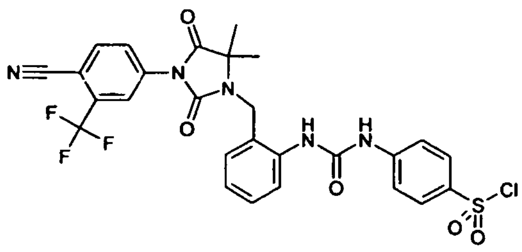

Die Verbindung **1.4** kann nach Verfahren "A" hergestellt werden. Dazu wurden 0,2 g der Verbindung **1.3** wurden bei Raumtemperatur in 5 ml trockenem Tetrahydrofuran gelöst, mit 0,11 g 4-(Chlorsulfonyl)-phenylisocyanat versetzt, 4 h bei Raumtemperatur gerührt und über Nacht stehen gelassen. Zur vollständigen Umsetzung wurden weitere 55 mg des Isocyanat zugegeben und weitere 6 h bei Raumtemperatur gerührt. Zur Aufarbeitung wurde die Reaktionsmischung filtriert und das Filtrat im Vakuum eingeengt. Die chromatographische Reinigung (Kieselgel; n-Heptan / Essigsäureethylester 50 / 50 nach n-Heptan / Essigsäureethylester 33 / 67 in 25 min,) lieferte 4-(3-{2-[3-(4-Cyano-3-trifluoromethyl-phenyl)-5,5-dimethyl-2,4-dioxo-imidazolidin-1-ylmethyl]-phenyl}-ureido)-benzolsulfonylchlorid **1.4.** Molekulargewicht 619,09 ($C_{27}H_{21}ClF_3N_5O_5S$); Retentionszeit $R_t$ = 2.52 min. [B]; MS ($ES^-$): 618,21 ($M-H^+$).

5) Herstellung von 4-(3-{2-[3-(4-Cyano-3-trifluoromethyl-phenyl)-5,5-dimethyl-2,4-dioxo-imidazolidin-1-ylmethyl]-phenyl}-ureido)-benzolsulfonsäureamid **1:**

0,12 g der Verbindung 1.4 wurden bei Raumtemperatur mit 3 ml einer Lösung von Ammoniak in Methanol (7 M) versetzt und 3 Tage bei Raumtemperatur stehen gelassen. Zur Aufarbeitung wurde die Reaktionsmischung im Vakuum eingeengt und chromatographisch (Methode [RP1]) gereinigt. Die Eluate wurden im Vakuum eingeengt, mit einer Lösung von Ammoniak in Wasser neutralisiert und gefriergetrocknet. Man erhielt 4-(3-{2-[3-(4-Cyano-3-trifluoromethyl- phenyl)-5,5-dimethyl-2,4-dioxo-imidazolidin-1-ylmethyl]-phenyl}-ureido)-benzolsulfonsäureamid **1.** Molekulargewicht 600,14 ($C_{27}H_{23}F_3N_6O_5S$); Retentionszeit $R_t$ = 1.73 min. [B]; MS (ESI): 601,12 ($MH^+$).

Die Verbindung **1.3** (4-[3-(2-Amino-benzyl)-4,4-dimethyl-2,5-dioxo-imidazolidin-1-yl]-2-trifluoromethyl-benzonitril) kann alternativ auch auf folgendem Wege hergestellt werden:

1) Herstellung von (2-Iodmethyl-phenyl)-carbaminsäure-tert-butyl ester **1.3.2:**

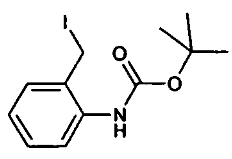

0,7 g Imidazol wurden bei Raumtemperatur in 20 ml trockenem Dichlormethan gelöst und mit 3,4 g polymergebundenem Triphenylphosphin (ca. 3 mmol/g Harz) versetzt. Zu dieser Mischung wurde unter Rühren langsam eine Lösung von 2,5 g Iod in 80 ml trockenem Dichlormethan zugetropft und bis zur Entfärbung der Lösung weitergerührt. Diese Mischung wurde anschließend langsam mit einer Lösung von 1,0 g (2-Hydroxymethyl-phenyl)-carbaminsäure-tert-butyl ester **1.3.1** in 20 ml trockenem Dichlormethan versetzt und 4 h bei Raumtemperatur gerührt. Zur Aufarbeitung wurde die Reaktionsmischung filtriert; das Filtrat wurde nacheinander mit gesättigter Ammoniumchloridlösung, Wasser und gesättigter Kochsalzlösung gewaschen. Die organische Phase wurde über Magnesiumsulfat getrocknet, flitriert und im Vakuum eingeengt. Das Produkt ((2-Iodmethyl-phenyl)-carbaminsäure-tert-butyl ester) wurde ohne weitere Reinigung in die nächste Stufe eingesetzt.

2) Herstellung von {2-[3-(4-Cyano-3-trifluoromethyl-phenyl)-5,5-dimethyl-2,4-dioxo-imidazolidin-1-ylmethyl]-phenyl}-carbaminsäure-tert-butyl ester **1.3.3**:

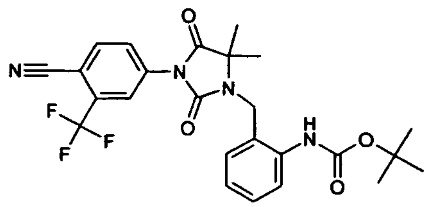

0,86 g der Verbindung 1.1 wurden bei Raumtemperatur in 20 ml trockenem Acetonitril gelöst, mit 1,06 g der Verbindung 1.3.2 und 1,04 g Cäsiumcarbonat versetzt, 4 h bei Raumtemperatur gerührt und anschließend über Nacht stehen gelassen. Zur Aufarbeitung wurde die Reaktionsmischung im Vakuum eingeengt, der Rückstand mit Wasser aufgenommen, die wässrige Phase mit Dichlormethan extrahiert, die organische Phase über Magnesiumsulfat getrocknet, filtriert und im Vakuum eingeengt. Die chromatographische Reinigung (Kieselgel; n-Heptan / Essigsäureethylester 67 / 33 EE nach n-Heptan / Essigsäureethylester 50 / 50 in 35 min.) lieferte {2-[3-(4-Cyano-3-trifluoromethyl-phenyl)-5,5-dimethyl-2,4-dioxo-imidazolidin-1-ylmethyl]-phenyl}-carbaminsäure-tert-butyl ester **1.3.3.** $^1$H NMR: 8.82, s, 1H; 8.35, d, 1H; 8.27, s, 1H; 8.1, d, 1H; 7.44, d, 1H; 7.33, d, 1H; 7.26, t, 1H; 7.13, t, 1H; 4.58, s, 2H; 1.48, s, 9H; 13 5, s, 6H.

3) Herstellung von 4-[3-(2-Amino-benzyl)-4,4-dimethyl-2,5-dioxo-imidazolidin-1-yl]-2-trifluoromethyl-benzonitril **1.3:**

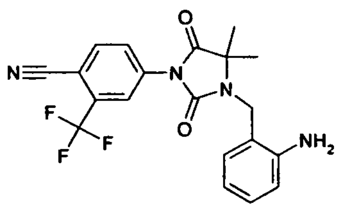

1,15 g der Verbindung 1.3.3 wurden bei Raumtemperatur in 20 ml trockenem Dichlormethan gelöst, mit 3,5 ml Trifluoroessigsäure und 0,35 ml Wasser versetzt, 4 h bei Raumtemperatur gerührt und danach über Nacht stehen gelassen. Die Reaktionsmischung wurde im Vakuum eingeengt und der Rückstand in Toluol gelöst und erneut im Vakuum eingeengt. Diser Rückstand wurde in Dichlormethan gelöst, mit gesättigter Natriumhydrogen-carbonatlösung gewaschen, die organische Phase abgetrennt, über Magnesiumsulfat getrocknet, filtriert und im Vakuum eingeengt. Man erhielt 4-[3-(2-Amino-benzyl)-4,4-dimethyl-2,5-dioxo-imidazolidin-1-yl]-2-trifluoromethyl-benzonitril **1.3.** $^1$H NMR: 8.34, d, 1H; 8.25, s, 1H; 8.09, d, 1H; 7.2, d, 1H; 7.01, t, 1H; 6.69, d, 1H; 6.57, t, 1H; 5.7, s, breit, 2H; 4.48, s, 2H; 1.39, s, 6H.

**Beispiel 2:** 4-(3-{2-[3-(4-Cyano-3-trifluoromethyl-phenyl)-5,5-dimethyl-2,4-dioxo-imidazolidin-1-ylmethyl]-phenyl}-ureido)-benzolsulfonsäure

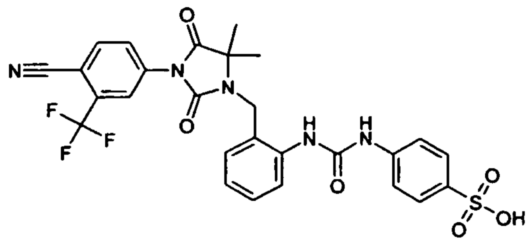

0,12 g des Sulfonsäurechlorids **1.4** wurden bei Raumtemperatur in 2 ml trockenem Acetonitril gelöst und mit 1,9 ml 1 N Natronlauge versetzt und 2 Tage bei Raumtemperatur gerührt. Zur Aufarbeitung wurde die Reaktionsmischung mit 1 N Salzsäure neutralisiert und im Vakuum eingeengt. Die chromatographische Reinigung (Methode [RP1]) lieferte 4-(3-{2-[3-(4-Cyano-3-trifluoromethyl-phenyl)-5,5-dimethyl-2,4-dioxo-imidazolidin-1-ylmethyl]-phenyl}-ureido)-benzolsulfonsäure **2.** Molekulargewicht 601,12 ($C_{27}H_{22}F_3N_6O_6S$); Retentionszeit $R_t$ = 1.51 min. [B]; MS (ESI): 602,11 ($MH^+$).

Die Verbindung des Beispiels **3,** 4-(3-{2-[3-(4-Cyano-3-trifluoromethyl-phenyl)-5,5-dimethyl-2,4-dioxo-imidazolidin-1-ylmethyl]-phenyl}-ureido)-benzoesäuremethylester (Molekulargewicht 579,17 ($C_{29}H_{24}F_3N_5O_5$); Retentionszeit $R_t$ = 1.99 min. [B]; MS (ESI): 580,15 ($MH^+$)),
des Beispiels **5,** 1-{2-[3-(4-Cyano-3-trifluoromethyl-phenyl)-5,5-dimethyl-2,4-dioxo-imidazolidin-1-ylmethyl]-phenyl)-3-(3,4-dimethoxy-phenyl)-harnstoff (Molekulargewicht 581,18 ($C_{29}H_{26}F_3N_5O_5$); Retentionszeit $R_t$ = 2.34 min. [C]; MS (ESI): 582,28 ($MH^+$)),
des Beispiels **6,** 1-(4-Cyano-phenyl)-3-{2-[3-(4-cyano-3-trifluoromethyl-phenyl)-5,5-dimethyl-2,4-dioxo-imidazolidin-1-ylmethyl]-phenyl}-harnstoff (Molekulargewicht 546,16 ($C_{28}H_{21}F_3N_6O_3$); Retentionszeit $R_t$ = 1.96 min. [B]; MS (ESI): 547,13 ($MH^+$)),
des Beispiels **7,** 1-{2-[3-(4-Cyano-3-trifluoromethyl-phenyl)-5,5-dimethyl-2,4-dioxo-imidazolidin-1-ylmethyl]-phenyl}-3-(2,4-difluoro-phenyl)-harnstoff (Molekulargewicht 557,14 ($C_{27}H_{20}F_5N_5O_3$); Retentionszeit $R_t$ = 2.06 min. [B]; MS (ESI): 558,18 ($MH^+$)),
des Beispiels **8,** 1-(3-Cyano-phenyl)-3-{2-[3-(4-cyano-3-trifluoromethyl-phenyl)-5,5-dimethyl-2,4-dioxo-imidazolidin-1-ylmethyl]-phenyl)-harnstoff (Molekulargewicht 546,16 ($C_{28}H_{21}F_3N_6O_3$); Retentionszeit $R_t$ = 1.96 min. [B]; MS (ESI): 547,15 ($MH^+$)),
des Beispiels **9,** 1-{2-[3-(4-Cyano-3-trifluoromethyl-phenyl)-5,5-dimethyl-2,4-dioxo-imidazolidin-1-ylmethyl]-phenyl}-3-phenyl-harnstoff (Molekulargewicht 521,16 ($C_{27}H_{22}F_3N_5O_3$); Retentionszeit $R_t$ = 1.99 min. [B]; MS (ESI): 522,15 ($MH^+$)),
des Beispiels **10,** 1-{2-[3-(4-Cyano-3-trifluoromethyl-phenyl)-5,5-dimethyl-2,4-dioxo-imidazolidin-1-ylmethyl]-phenyl}-3-(4-ethoxy-phenyl)-harnstoff (Molekulargewicht 565,19 ($C_{29}H_{26}F_3N_5O_4$); Retentionszeit $R_t$ = 2.01 min. [B]; MS (ESI): 566,16 ($MH^+$)),
des Beispiels **11,** 3-(3-{2-[3-(4-Cyano-3-trifluoromethyl-phenyl)-5,5-dimethyl-2,4-dioxo-imidazolidin-1-ylmethyl]-phenyl}-ureido)-benzoesäuremethylester (Molekulargewicht 579,17 ($C_{29}H_{24}F_3N_5O_5$); Retentionszeit $R_t$ = 1.98 min. [B]; MS (ESI): 580,15 ($MH^+$)),
des Beispiels **35,** 1-{2-[3-(4-Cyano-3-trifluoromethyl-phenyl)-5,5-dimethyl-2,4-dioxo-imidazolidin-1-ylmethyl]-phenyl}-3-(4-methylsulfanyl-phenyl)-harnstoff (Molekulargewicht 567,15 ($C_{28}H_{24}F_3N_5O_3S$); Retentionszeit $R_t$ = 2.44 min. [B]; MS (ESI): 568,33 ($MH^+$)), wurden wie die Verbindung **1.4** durch Umsatz der Verbindung **1.3** mit 4-Isocyanato-benzoesäuremethylester (für **3**),
4-Isocyanato-1,2-dimethoxy-benzol (für **5**),
4-Isocyanato-benzonitril (für **6**),
2,4-Difluoro-1-isocyanato-benzol (für **7**),
3-Isocyanato-benzonitril (für **8**),
Isocyanato-benzol (für **9**),
1-Ethoxy-4-isocyanato-benzol (für **10**),
3-Isocyanato-benzoesäuremethylester (für **11**),
1-Isocyanato-4-methylsulfanyl-benzol (für **35**)
erhalten.

**Beispiel 4:**
4-(3-{2-[3-(4-Cyano-3-trifluoromethyl-phenyl)-5,5-dimethyl-2,4-dioxo-imidazolidin-1-ylmethyl]-phenyl }-ureido)-benzoe säure

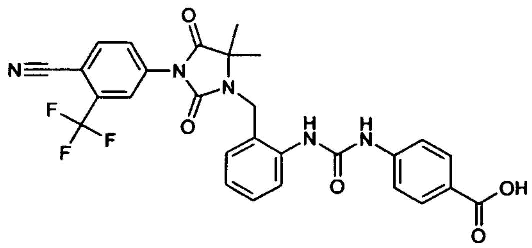

0,23 g der Verbindung des Beispiels **3** wurden bei Raumtemperatur in 4 ml trockenem Dioxan gelöst, mit 0,33 ml konzentrierter Salzsäure versetzt und 2 h bei 80°C gerührt. Zur vollständigen Umsetzung wurden nochmals 0,67 ml konzentrierte Salzsäure zugesetzt und weitere 8 h bei 80°C gerührt. Zur Aufarbeitung wurde die abgekühlte Reaktionsmischung im Vakuum eingeengt und der Rückstand chromatographisch gereinigt (Methode [RP1]). Die Eluate, die Produkt enthielten, wurden zusammengegeben und gefriergetrocknet. Man erhielt 4-(3-{2-[3-(4-Cyano-3-trifluoromethyl-phenyl)-5,5-dimethyl-2,4-dioxo-imidazolidin-1-ylmethyl]-phenyl}-ureido)-benzoesäure 4. Molekulargewicht 565,15 ($C_{28}H_{22}F_3N_5O_5$); Retentionszeit $R_t$ = 1.76 min. [B]; MS (ESI): 566,12 ($MH^+$).

**Beispiel 12:**
1-{2-[3-(4-Cyano-3-trifluoromethyl-phenyl)-5,5-dimethyl-2,4-dioxo-imidazolidin-1-ylmethyl]-phenyl)-3-(4-methansulfinyl -phenyl)-harnstoff

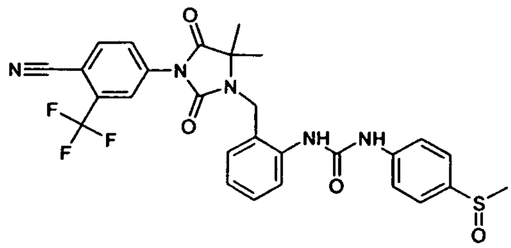

0,17 g Natriumperjodat wurden in 2,5 ml Wasser gelöst. Unter Eisbadkühlung wurde eine Lösung von 0,3 g der Verbindung des Beispiels **35** in 5 ml trockenem Tetrahydrofuran langsam zugegeben und 4 h bei Raumtemperatur gerührt. Die Reaktionsmischung stand über Nacht bei Raumtemperatur. Zur Aufarbeitung wurde das Reaktionsgemisch mit Wasser versetzt und anschließend mit Essigsäureethylester extrahiert. Die organische Phase wurde über Magnesiumsulfat getrocknet, filtriert und im Vakuum eingeengt. Das Rohprodukt wurde chromatographisch (Methode [RP1]) gereinigt. Die Gefriertrocknung der Produkt enthaltenden Fraktionen lieferte 1-{2-[3-(4-Cyano-3-trifluoromethyl-phenyl)-5,5-dimethyl-2,4-dioxo-imidazolidin-1-ylmethyl]-phenyl}-3-(4-methansulfinyl-phenyl)-harnstoff 12. Molekulargewicht 583,15 ($C_{28}H_{24}F_3N_5O_4S$); Retentionszeit $R_t$ = 1.98 min. [C]; MS (ESI): 584,29 ($MH^+$).

**Beispiel 13:** 1-{2-[3-(4-Cyano-3-trifluoromethyl-phenyl)-5,5-dimethyl-2,4-dioxo-imidazolidin-1-ylmethyl]- phenyl}-3-(4-methansulfonyl-phenyl)-harnstoff

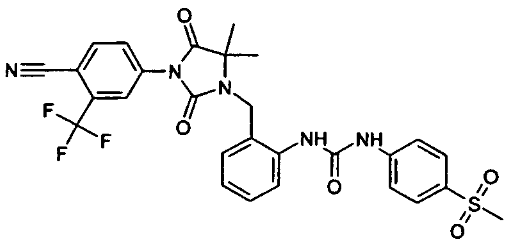

0,3 g der Verbindung des Beispiels **35** wurden bei Raumtemperatur in 10 ml trockenem Dichlormethan gelöst, portionsweise mit insgesamt 0,27 g m-Chlorperbenzoesäure (85%ig) versetzt und 20 h bei Raumtemperatur gerührt. Zur Aufarbeitung wurde die Reaktionslösung mit gesättigter Natriumsulfitlösung versetzt und kurz gerührt. Sodann wird die wässrige Phase mit Dichlormethan extrahiert; die organische Phase wird mit gesättigter Natriumcarbonatlösung gewaschen, über Magnesiumsulfat getrocknet, filtriert und im Vakuum eingeengt. Das Rohprodukt wurde chromatographisch (Methode [RP1]) gereinigt. Die Gefriertrocknung der Produkt enthaltenden Fraktionen lieferte 1-{2-[3-(4- Cyano-3-trifluoromethyl-phenyl)-5,5-dimethyl-2,4-dioxo-imidazolidin-1-ylmethyl]-phenyl}-3-(4-methansulfonyl-phenyl)-harnstoff **13.** Molekulargewicht 599,14 ($C_{28}H_{24}F_3N_5O_5S$); Retentionszeit $R_t$ = 2.25 min. [C]; MS (ESI): 600,31 ($MH^+$).

**Beispiel 16:** 4-(3-{2-[3-(4-Cyano-3-trifluoromethyl-phenyl)-5,5-dimethyl-2,4-dioxo-imidazolidin-1-ylmethyl]-phenyl} -ureido)-benzamid

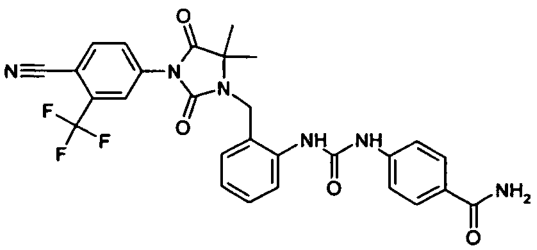

0,3 g des Methylesters des Beispiels **3** wurden bei Raumtemperatur mit 7,4 ml einer 7 M Lösung von Ammoniak in Methanol versetzt und 6 Tage bei Raumtemperatur stehen gelassen. Danach wurden weitere 5 ml der methanolischen Ammoniaklösung zugegeben und die Mischung 10 Wochen sich selbst überlassen. Zur Aufarbeitung wurde die Reaktionsmischung im Vakuum eingeengt und das Rohprodukt chromatographisch (Methode [RP1]) gereinigt. Die Gefriertrocknung der Produkt enthaltenden Fraktionen lieferte 4-(3-{2-[3-(4-Cyano-3-trifluoromethyl-phenyl)-5,5-dimethyl-2,4-dioxo-imidazolidin-1-ylmethyl]-phenyl}-ureido)-benzamid **16.** Molekulargewicht 564,17 ($C_{28}H_{23}F_3N_6O_4$); Retentionszeit $R_t$ = 1.68 min. [B]; MS (ESI): 565,26 ($MH^+$).

**Beispiel 14:** N-{2-[3-(4-Cyano-3-trifluoromethyl-phenyl)-5,5-dimethyl-2,4-dioxo-imidazolidin-1-ylmethyl]-phenyl}-C-(4-trifluoromethyl-phenyl)-methansulfonamid

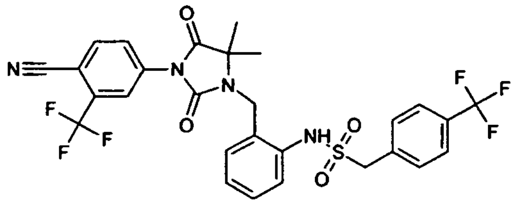

0,2 g der Verbindung **1.3** wurden in 0 ml trockenem Dichlormethan gelöst, mit 60 µl Pyridin und 0,19 g (4-Trifluoromethyl-phenyl)-methansulfonylchlorid versetzt, 4 h bei Raumtemperatur gerührt und über Nacht stehen gelassen. Zur Aufarbeitung wurde die Reaktions-mischung im Vakuum eingeengt und der Rückstand chromatographisch (Methode [RP1]) gereinigt. Die Gefriertrocknung der Produkt enthaltenden Fraktionen lieferte N-{2-[3-(4-Cyano-3-trifluoromethyl-phenyl)-5,5-dimethyl- 2,4-dioxo-imidazolidin-1-ylmethyl]-phenyl}-C-(4-trifluoromethyl-phenyl)-methansulfonamid **14.** Molekulargewicht 624,12 ($C_{28}H_{22}F_6N_4O_4S$); Retentionszeit $R_t$ = 2.55 min. [B]; MS ($ES^-$): 623,06 ($M-H^+$).

**Beispiel 15:**

N-{2-[3-(4-Cyano-3-trifluoromethyl-phenyl)-5,5-dimethyl-2,4-dioxo-imidazolidin-1-ylmethyl]-phenyl}-C-(2,4-difluoro-phe nyl)-methansulfonamid

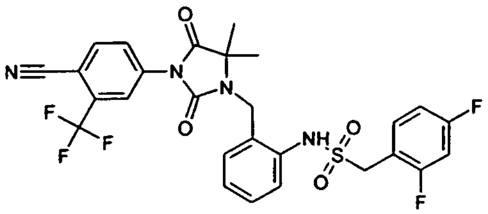

Die Verbindung des Beispiels **15** wurde in analoger Weise durch Reaktion von **1.3** mit (2,4-Difluoro-phenyl)-methansulfonylchlorid hergestellt. Man erhielt N-{2-[3-(4-Cyano-3-trifluoromethyl-phenyl)-5,5-dimethyl- 2,4-dioxo-imidazolidin-1-ylmethyl]-phenyl}-C-(2,4-difluoro-phenyl)-methansulfonamid **15.** Molekulargewicht 592,12 ($C_{27}H_{21}F_5N_4O_4S$); Retentionszeit $R_t$ = 2.06 min. [B]; MS (ESI): 593,15 ($MH^+$).

**Beispiel 17:**

4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzoesäuremethyl-ester

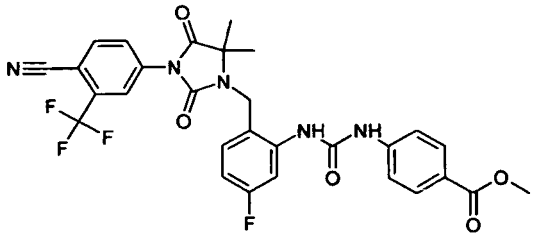

1) Herstellung von 4-[3-(2-Brom-4-fluoro-benzyl)-4,4-dimethyl-2,5-dioxo-imidazolidin-1-yl]-2-trifluoromethyl-benzonitril (**17.2**):

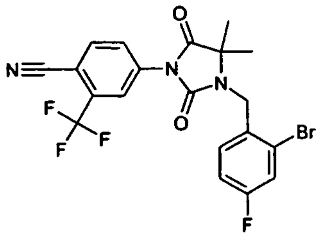

Die Verbindung **17.2** wurde wie für Beispiel **1.2** beschrieben hergestellt, indem die Verbindung **1.1** anstatt mit 2-Brombenzylbromid mit 2-Brom-1-brommethyl-4-fluoro-benzol (hergestellt durch radikalische Bromierung aus 2-Brom-4-fluoro-1-methylbenzol mit N-Bromsuccinimid in Tetrachlormethan; $^1H$ NMR: 7.7, m, 1H; 7.65, d, 1H; 7.3, m, 1H; 4.75, s, 2H) umgesetzt wurde. Molekulargewicht 483,02 ($C_{20}H_{14}BrF_4N_3O_3$); $^1H$ NMR: 8.37, d, 1H; 8.25, s, 1H; 8.1, d, 1H; 7.65, m, 2H; 7.27, m, 1H; 4.62, s, 2H; 1.41, s, 6H.

2) Herstellung von 4-[3-(2-Amino-4-fluoro-benzyl)-4,4-dimethyl-2,5-dioxo-imidazolidin-1-yl]-2-trifluoromethyl-benzonitril (**17.3**):

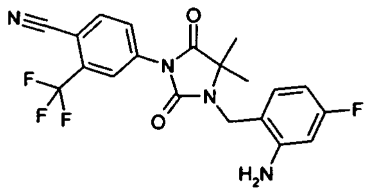

Analog der Vorgehensweise wie sie für die Herstellung von **1.3** beschrieben ist, wurde **17.2** mit Benzophenonimin zu **17.3** umgesetzt. Molekulargewicht 420,12 ($C_{20}H_{16}F_4N_4O_2$); Retentionszeit $R_t$ = 2.71 min. [E]; MS (ESI): 421,08 ($MH^+$).

3) Herstellung von 4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzoesäure-methylester **17:**

Die Verbindung **17** wurde analog der Vorgehensweise bei der Herstellung der Verbindung des Beispiels **3** hergestellt, indem das Anilin **17.3** mit 4-Isocyanato-benzoesäuremethylester umgesetzt wurde. Man erhielt 4-(3-{2-[3-(4-Cyano-3-trifluoromethyl-phenyl)-5,5-dimethyl-2,4-dioxo-imidazolidin-1-ylmethyl]-5-fluorophenyl}-ureido)-benzoesäuremethylester. Molekulargewicht 597,16 ($C_{29}H_{23}F_4N_5O_5$); Retentionszeit $R_t$ = 2.05 min. [B]; MS (ESI): 598,20 ($MH^+$).

Die Verbindung des Beispiels **36,** 1-{2-[3-(4-Cyano-3-trifluoromethyl-phenyl)-5,5-dimethyl-2,4-dioxo-imidazolidin-1-ylmethyl]-5-fluoro-phenyl}-3-(4-methylsulfanyl-phenyl)-harnstoff (Molekulargewicht 585,14 ($C_{28}H_{23}F_4N_5O_3S$); Retentionszeit $R_t$ = 2.15 min. [B]; MS (ESI): 586,18 ($MH^+$)),
des Beispiels **20,** 1-(6-Chlor-pyridin-3-yl)-3-{2-[3-(4-cyano-3-trifluoromethyl-phenyl)-5,5-dimethyl-2,4-dioxo-imidazolidin-1-ylmethyl]-5-fluoro-phenyl}-harnstoff (isoliert als Hydrochlorid, Molekulargewicht (freie Base) 574,11 ($C_{26}H_{19}ClF_4N_6O_3$); Retentionszeit $R_t$= 2.46 min. [C]; MS (ESI): 575,38 ($MH^+$)),
des Beispiels **21,** 1-{2-[3-(4-Cyano-3-trifluoromethyl-phenyl)-5,5-dimethyl-2,4-dioxo-imidazolidin-1-ylmethyl]-5-fluoro-phenyl}-3-(2,6-dichlor-pyridin-4-yl)-harnstoff (isoliert als Hydrochlorid, Molekulargewicht (freie Base) 608,07 ($C_{26}H_{18}Cl_2F_4N_6O_3$); Retentionszeit $R_t$ = 2.72 min. [C]; MS (ESI): 609,36 ($MH^+$)),
wurden wie die Verbindung **1.4** durch Umsatz der Verbindung **17.3** mit
1-Isocyanato-4-methylsulfanyl-benzol (für **36**),
2-Chlor-5-isocyanato-pyridin (für **20**),

2,6-Dichlor-4-isocyanato-pyridin (für **21**)
erhalten.

**Beispiel 18:** 4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzoesäure

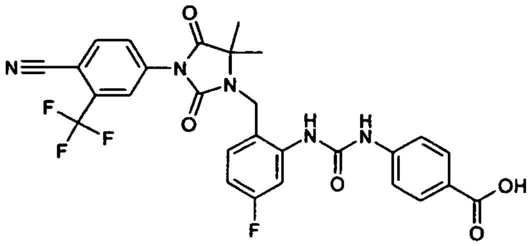

0,24 g des Esters des Beispiels **17** wurden bei Raumtemperatur in 10 ml trockenem Tetrahydrofuran gelöst, mit 0,51 g Kalium-trimetylsilanolat versetzt, 4 h bei Raumtemperatur gerührt und danach über Nacht bei Raumtemperatur stehen gelassen. Zur Aufarbeitung wurde die Reaktionsmischung im Vakuum eingeengt und der Rückstand chromatographisch (Methode [RP1]) gereinigt. Man erhielt 4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzoesäure **18.** Molekulargewicht 583,14 ($C_{28}H_{21}F_4N_5O_5$); Retentionszeit $R_t$ = 2.39 min. [C]; MS (ESI): 584,28 ($MH^+$).

**Beispiel 19:** 1-{2-[3-(4-Cyano-3-trifluoromethyl-phenyl)-5,5-dimethyl-2,4-dioxo-imidazolidin-1-ylmethyl]-5-fluoro-phenyl}-3-(4-methansulfonyl-phenyl)-harnstoff

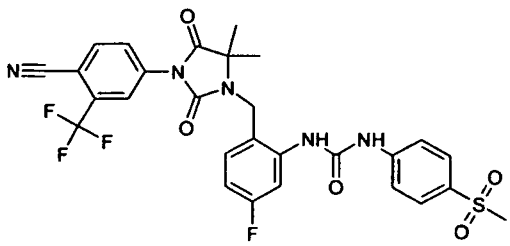

Analog der Vorgehensweise für die Herstellung der Verbindung des Beispiels **13** wurde für die Herstellung der Verbindung des Beispiels **18** die Verbindung **36** mit m-Chlorperbenzoesäure oxidiert. Molekulargewicht 617,13 ($C_{28}H_{23}F_4N_5O_5S$); Retentionszeit $R_t$ = 2.28 min. [C]; MS (ESI): 618,27 ($MH^+$).

**Beispiel 22:** 4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzolsulfonamid

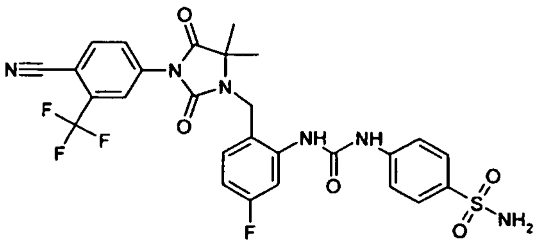

1) Herstellung von 4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzolsulfonyl-chlorid (**22.1**):

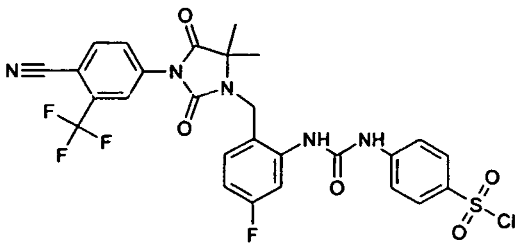

Analog der Vorgehensweise wie sie bei der Herstellung der Verbindung **1.4** beschrieben ist, wurde das Anilin **17.3** mit 4-(Chlorsulfonyl)-phenylisocyanat umgesetzt und lieferte 4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzolsulfonyl-chlorid (**22.1**), welches ohne weitere Reinigung in die nächste Stufe eingesetzt wurde.

2) Herstellung von 4-(3-{2-[3-(4-Cyano-3-trifluoromethyl-phenyl)-5,5-dimethyl-2,4-dioxo-imidazolidin-1-ylmethyl]-5-fluoro-phenyl }-ureido)-benzolsulfonamid:

Die Umsetzung des Sulfonsäurechlorids **22.1** mit einer Lösung von Ammoniak in Methanol (siehe Herstellung der Verbindung des Beispiels **1**) lieferte 4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-benzolsulfonamid **(22)**. Molekulargewicht 618,13 ($C_{27}H_{22}F_4N_6O_5S$); Retentionszeit $P_t$ = 2.16 min. [C]; MS (ESI): 619,45 ($MH^+$).

**Beispiel 23:** 1-(4-Amino-phenyl)-3-{5-fluoro-2-[3-(4-fluoro-3-trifluoromethyl-phenyl)-5,5-dimethyl-2,4-dioxo-imidazolidin-1-ylmethyl]-phenyl}-harnstoff Hydrochlorid

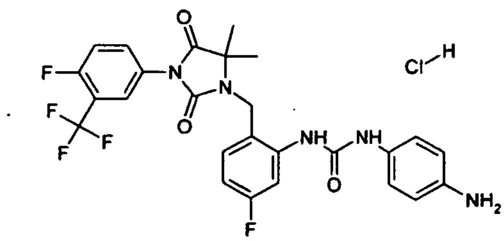

1) Herstellung von 3-(4-Fluoro-3-trifluoromethyl-phenyl)-5,5-dimethyl-imidazolidin-2,4-dion **(23.1):**

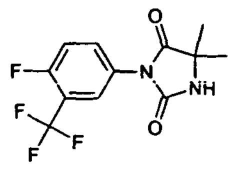

Die Verbindung **23.1** kann nach Verfahren "A" dargestellt werden. Dazu wurden 1,5 g (9,76 mMol) 2-Amino-2-methyl-propionsäuremethylester Hydrochlorid in 20 ml trockenem Tetrahydrofuran suspendiert, mit 1,38 ml (9,76 mMol) Triethylamin und 2 g (9,76 mMol) 1-Fluoro-4-isocyanato-2-trifluoromethyl-benzol versetzt. Die Mischung wurde 1 h bei 70°C gerührt; danach ließ man etwas abkühlen, fügte 10 ml konzentrierte Salzsäure zu und rührte für 2 h bei 70°C. Die abgekühlte Reaktionsmischung wurde mit Essigsäureethylester und Wasser versetzt; die organische Phase wurde abgetrennt, über Natriumsulfat getrocknet, filtriert und im Vakuum eingeengt. Der Rückstand wurde chromatographisch gereinigt (Methode [RP2]) und wurde nach Lösen in Essigsäureethylester, Trocknen der Lösung, Einengen im Vakuum und erneutem Lösen in Dichlormethan mit n-Heptan zur Kristallisation gebracht. Man erhielt 2,8 g 3-(4-Fluoro-3-trifluoromethyl-phenyl)-5,5-dimethyl-imidazolidine-2,4-dion (**23.1**) mit dem Schmelzpunkt 111 - 114°C. Molekulargewicht 290,06 ($C_{12}H_{10}F_4N_2O_2$); Retentionszeit $R_t$ = 1.55 min. [B]; MS (ESI): 291,27 ($MH^+$).

2) Herstellung von 1-(2-Brom-4-fluoro-benzyl)-3-(4-fluoro-3-trifluoromethyl-phenyl)-5,5-dimethyl-imidazolidin-2,4-dion **23.2:**

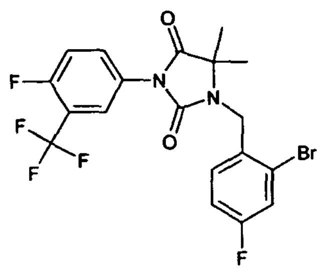

Die Titelverbindung wurde hergestellt, indem **23.1** mit 2-Brom-1-brommethyl-4-fluoro-benzol unter Bedingungen wie sie weiter oben für **1.2** beschrieben sind, zur Reaktion gebracht wurden. $^1H$ NMR: 7.98, m, 1H; 7.9, m, 1H; 7.7 - 7.6, m, 3H; 7.26, m, 1H; 4.6, s, 2H; 1.4, s, 6H.

3) Herstellung von 1-(2-Amino-4-fluoro-benzyl)-3-(4-fluoro-3-trifluoromethyl-phenyl)-5,5-dimethyl-imidazolidin-2,4-dion 23.3 :

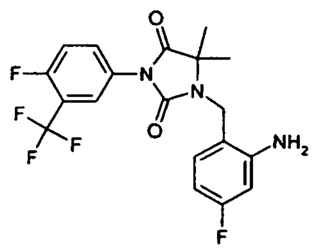

Analog der Vorgehensweise wie sie für die Herstellung von **1.3** beschrieben ist, wurde **23.2** mit Benzophenonimin zu **23.3** umgesetzt. Die Verbindung wurde als Salz mit Trifluoroessigsäure isoliert. Molekulargewicht (freie Base) 413,11 ($C_{19}H_{16}F_5N_3O_2$); Retentionszeit $R_t$ = 2.40 min. [C]; MS (ESI): 414,21 ($MH^+$).

4) Herstellung von 1-{5-Fluoro-2-[3-(4-fluoro-3-trifluoromethyl-phenyl)-5,5-dimethyl-2,4-dioxo-imidazolidin-1-ylmethyl]-phenyl}-3-(4-nitro-phenyl)-harnstoff **23.4:**

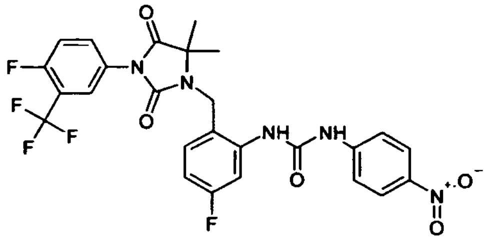

Analog der Vorgehensweise wie sie bei der Herstellung der Verbindung **1.4** beschrieben ist, wurde das Anilin **23.3** mit 1-Isocyanato-4-nitro-benzol umgesetzt und lieferte 1-{5-Fluoro-2-[3-(4-fluoro-3-trifluoromethyl-phenyl)-5,5-dimethyl-2,4-dioxo-imidazolidin-1-ylmethyl]-phenyl}-3-(4-nitro-phenyl)-harnstoff (**23.4**). Molekulargewicht 577,13 ($C_{26}H_{20}F_5N_5O_5$); Retentionszeit $R_t$ = 2.71 min. [C]; MS (ESI): 578,13 ($MH^+$).

5) Herstellung von 1-(4-Amino-phenyl)-3-{5-fluoro-2-[3-(4-fluoro-3-trifluoromethyl-phenyl)-5,5-dimethyl-2,4-dioxo-imidazolidin-1-ylmethyl]-phenyl}-harnstoff Hydrochlorid:

0,22 g der Verbindung des Beispiels **23.4** wurden bei Raumtemperatur in 3 ml trockenem Methanol gelöst, mit 1,5 ml Ameisensäure verdünnt, unter Argon mit 45 mg Raney-Nickel versetzt und 6 h bei Raumtemperatur gerührt. Die Reaktionsmischung blieb 2 Tage bei Raumtemperatur stehen. Zur Aufarbeitung wurde über ein Tiefenfilter filtriert und das Filtrat im Vakuum eingeengt. Die chromatographische Reinigung (Methode [RP1]) lieferte das Trifluoroessigsäure-Salz der Verbindung gelöst in einer Mischung von Wasser mit Acetonitril. Das Lösemittelgemisch wurde im Vakuum entfernt, der Rückstand mit einer Lösung von Salzsäure in Dioxan versetzt und anschließend gefriergetrocknet. Man erhielt 1-(4-Amino-phenyl)-3-{5-fluoro-2-[3-(4-fluoro-3-trifluoromethyl-phenyl)-5,5-dimethyl-2,4-dioxo-imidazolidin-1-ylmethyl]-phenyl}-harnstoff Hydrochlorid (**23**). Molekulargewicht (freie Base) 547,16 ($C_{26}H_{22}F_5N_5O_3$); Retentionszeit $P_t$ = 1.50 min. [B]; MS (ESI): 548,11 ($MH^+$).

Alternative Herstellung von 1-(2-Amino-4-fluoro-benzyl)-3-(4-fluoro-3-trifluoromethyl-phenyl)-5,5-dimethyl-imidazolidin-2,4-dion **23.3:**

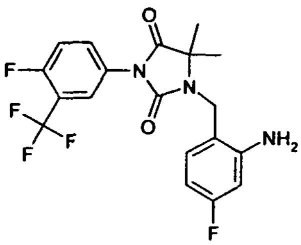

1) Herstellung von 1-Brommethyl-4-fluoro-2-nitro-benzol **23.10:**

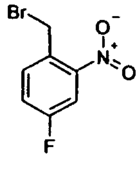

1,7 g 4-Fluoro-nitrobenzylalkohol wurden bei Raumtemperatur in 30 ml trockenem Dichlormethan gelöst und bei Raumtemperatur tropfenweise mit einer Lösung von 0,38 ml Phosphortribromid in 10 ml trockenem Dichlormethan

versetzt. Nach beendeter Zugabe wurde die Reaktionsmischung über Nacht bei Raumtemperatur gerührt. Zur Aufarbeitung wurde die Mischung mit gesättigter Natriumhydrogencarbonatlösung versetzt und mit Dichlormethan extrahiert. Die organische Phase wurde mit gesättigter Kochsalzlösung gewaschen, über Magnesiumsulfat getrocknet, filtriert und im Vakuum eingeengt. Man erhielt 1-Brommethyl-4-fluoro-2-nitro-benzol. $^1$H NMR: 8.02, d, 1H; 7.85, m, 1H; 7.69, t, 1H; 4.91, s, 2H.

2) Herstellung von 1-(4-Fluoro-2-nitro-benzyl)-3-(4-fluoro-3-trifluoromethyl-phenyl)-5,5-dimethyl-imidazolidin-2,4-dion **23.11:**

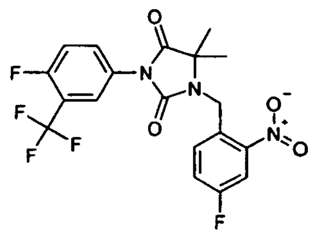

Der Umsatz von **23.1** mit **23.10** in Acetonitril mit Cäsiumcarbonat lieferte 1-(4-Fluoro-2-nitro-benzyl)-3-(4-fluoro-3-trifluoromethyl-phenyl)-5,5-dimethyl-imidazolidin-2,4-dion **23.11.** Molekulargewicht 443,09 ($C_{19}H_{14}F_5N_3O_4$); Retentionszeit $R_t$ = 2.82 min. [E]; MS (ESI): 444,12 ($MH^+$).

3) Herstellung von 1-(2-Amino-4-fluoro-benzyl)-3-(4-fluoro-3-trifluoromethyl-phenyl)-5,5-dimethyl-imidazolidin-2,4-dion **23.3:**

2,33 g der Verbindung **23.11** wurden bei Raumtemperatur in 50 ml trockenem Methanol gelöst und unter Argon mit 37 mg Palladiumhydroxid auf Kohle versetzt. Danach wurden 0,46 g Trimethylamin-Boran zugegeben und 4 h unter Rückfluss gerührt. Zur Aufarbeitung wurde die Reaktionsmischung filtriert, das Filtrat im Vakuum eingeengt und der Rückstand mit Diisopropylether aufgenommen. Die Suspension wurde filriert, das Filtrat im Vakuum eingeengt und der Rückstand gefriergetrocknet. Man erhielt 1-(2-Amino-4-fluoro-benzyl)-3-(4-fluoro-3-trifluoromethyl-phenyl)-5,5-dimethyl-imidazolidin-2,4-dion **(23.3).** $^1$H NMR: 7.98, d, 1H; 7.88, m, 1H; 7.7, t, 1H; 7.21, m, 1H; 6.44, m, 1H; 6.31, m, 1H; 5.45, s, breit, 2H; 4.42, s, 2H; 1.38, s, 6H.

**Beispiel 24:** 4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzolsulfonamid

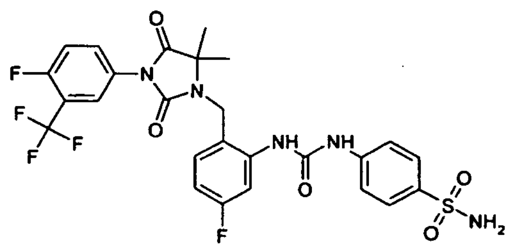

1) Herstellung von 4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzolsulfonylchlorid (**24.1**):

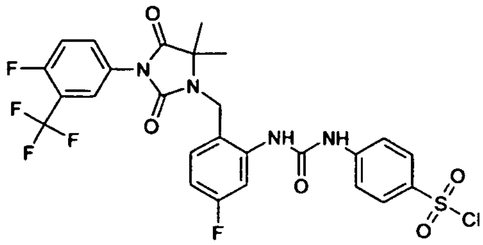

Analog der Vorgehensweise wie sie bei der Herstellung der Verbindung **1.4** beschrieben ist, wurde das Anilin **23.3** mit 4-(Chlorsulfonyl)-phenylisocyanat umgesetzt und lieferte 4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzolsulfonylchlorid (**24.1**), welches ohne weitere Reinigung in die nächste Stufe eingesetzt wurde.

2) Herstellung von 4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzolsulfonamid:

Die Umsetzung des Sulfonsäurechlorids **24.1** mit einer Lösung von Ammoniak in Methanol (siehe Herstellung der Verbindung des Beispiels **1**) lieferte 4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzolsulfonamid (**24**). Molekulargewicht 611,12 ($C_{26}H_{22}F_5N_5O_5S$); Retentionszeit $R_t$ = 1.83 min. [B]; MS (ESI): 612,30 ($MH^+$).

**Beispiel 25:** 4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzolsulfonsäure

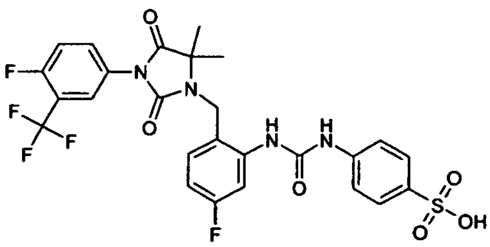

0,4 g des Sulfonsäurechlorids **24.1** wurden bei Raumtemperatur in 3 ml Wasser suspendiert, mit 0,65 ml Pyridin versetzt und 4 h bei 80°C gerührt. Die Reaktionsmischung wurde im Vakuum eingeengt und chromatographisch (Methode [RP1]) gereinigt. Die Produkt enthaltenden Fraktionen wurden gefriergetrocknet und lieferten 4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzolsulfonsäure **25.** Molekulargewicht 612,11 ($C_{26}H_{21}F_5N_4O_6S$); Retentionszeit $R_t$ = 1.57 min. [B]; MS (ESI): 613,28 ($MH^+$).

**Beispiel 26:** Natriumsalz der 4-(3-{5-Fluoro-2-[3-(4-fluoro-3-trifluoromethyl-phenyl)-5,5-dimethyl-2,4-dioxo-imidazolidin-1-ylmethyl]-phenyl }-ureido)-benzolsulfonsäure

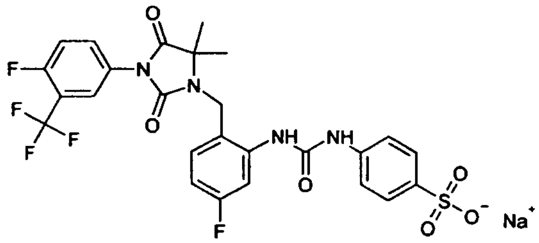

0,32 g der Sulfonsäure des Beispiels **25** wurden bei Raumtemperatur in 20 ml Wasser gelöst und solange mit Natriumhydrogencarbonat versetzt bis ein pH-Wert von ca. 7 erreicht war. Die Lösung wurde gefriergetrocknet und lieferte das Natriumsalz **26.** $^{1}$H NMR: 9.2, s, 1H; 8.45, s, 1H; 8.0, m, 1H; 7.88, m, 1H; 7.65, m, 2H; 7.5, d, 2H; 7.44, m, 3H; 6.9, t, 1H; 4.58, s, 2H; 1.4, s, 6H.

**Beispiel 27:** 1-{5-Fluoro-2-[3-(4-fluoro-3-trifluoromethyl-phenyl)-5,5-dimethyl-2,4-dioxo imidazolidin-1-ylmethyl]-phenyl}-3-(4-methansulfonyl-phenyl)-harnstoff

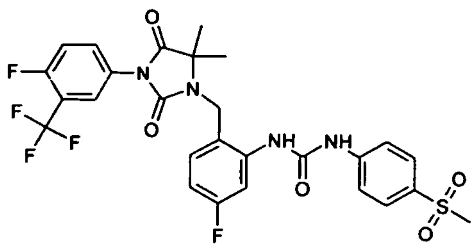

1) Herstellung von 1-{5-Fluoro-2-[3-(4-fluoro-3-trifluoromethyl-phenyl)-5,5-dimethyl-2,4-dioxo-imidazolidin-1-ylmethyl]-phenyl}-3-(4-methylsulfanyl-phenyl)-harnstoff (Beispiel **37**):

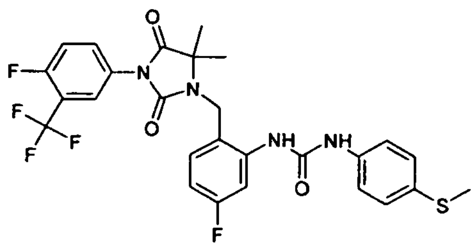

Die Verbindung des Beispiels **37,** 1-{5-Fluoro-2-[3-(4-fluoro-3-trifluoromethyl-phenyl)-5,5-dimethyl-2,4-dioxo-imidazolidin-1-ylmethyl]-phenyl}-3-(4-methylsulfanyl-phenyl)-harnstoff (Molekulargewicht 578,14 ($C_{27}H_{23}F_5N_4O_3S$); Retentionszeit $R_t$ = 2.22 min. [B]; MS (ESI): 579,29 ($MH^+$)), wurde wie die Verbindung **1.4** durch Umsatz der Verbindung **23.3** mit 1-Isocyanato-4-methylsulfanyl-benzol erhalten.

2) Herstellung von 1-{5-Fluoro-2-[3-(4-fluoro-3-trifluoromethyl-phenyl)-5,5-dimethyl-2,4-dioxo-imidazolidin-1-ylmethyl]-phenyl}-3-(4-methansulfonyl-phenyl)-harnstoff:

Analog der Vorgehensweise für die Herstellung der Verbindung des Beispiels **13** wurde für die Herstellung der Verbindung des Beispiels **27** die Verbindung **37** mit m-Chlorperbenzoesäure oxidiert. Molekulargewicht 610,13 ($C_{27}H_{23}F_5N_4O_5S$); Retentionszeit $R_t$ = 1.91 min. [B]; MS (ESI): 611,30 ($MH^+$).

**Beispiel 28:** 4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonsäure-amid

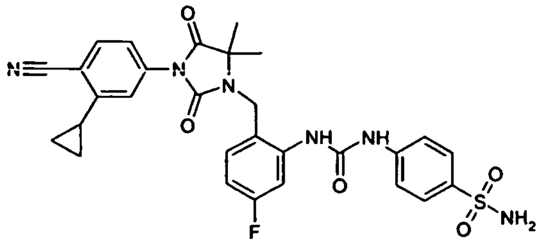

1) Herstellung von 4-Amino-2-cyclopropyl-benzonitril (**28.5**):

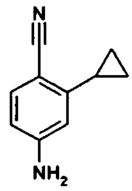

Zur Herstellung der Verbindung des Beispiels **28** findet Verfahren "D" Anwendung: Zu einer Suspension von 916 mg 2-Chlor-4-aminobenzonotril, 773 mg Cyclopropylboronsäure, 5,094 g Kaliumphosphat und 673 mg Tricyclohexylphosphin in einer Mischung aus 10,5 ml Toluol und 1,74 ml Wasser wurden unter einer Argonatmosphäre 269 mg Palladiumacetat zugefügt. Die Mischung wurde über Nacht bei 80°C gerührt. Die abgekühlte Reaktionsmischung wurde mit Wasser und Essigsäureethylester versetzt, filtriert und das Filtrat dreimal mit einer Mischung von Essigsäureethylester mit Toluol extrahiert. Die organischen Phasen wurden über Magnesiumsulfat getrocknet, filtriert und im Vakuum eingeengt. Der Rückstand wurde chromatographisch gereinigt (Methode [PR1]) und lieferte 4-Amino-2-cyclopropyl-benzonitril. $^1$HNMR: 7.3, d, 1H; 6.4, d, 1H; 6.1, d, 1H; 4.0, s (breit), 2H; 2.0, m, 1H; 1.0, m, 2H; 0.65, m, 2H.

2) Herstellung von 2-Cyclopropyl-4-(4,4-dimethyl-2,5-dioxo-imidazolidin-1-yl)-benzonitril **(28.1):**

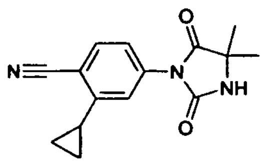

Die Verbindung **28.1** wurde nach dem Verfahren wie für **1.1** beschrieben durch Umsatz von **28.5** mit 2-Amino-2-methyl-propionsäure-tert.-butylester Hydrochlorid und Phosgen (Lösung in Toluol) hergestellt. Molekulargewicht 269,11 ($C_{15}H_{15}N_3O_2$); Retentionszeit $R_t$ = 1.43 min. [B]; MS (ESI): 270,18 ($MH^+$).

3) Herstellung von 4-[3-(2-Brom-4-fluoro-benzyl)-4,4-dimethyl-2,5-dioxo-imidazolidin-1-yl]-2-cyclopropyl-benzonitril (**28.2**):

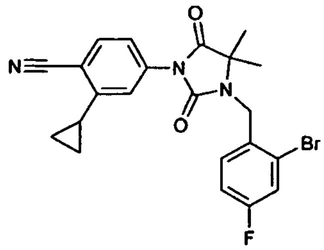

Unter Bedingungen wie sie für die Herstellung von **1.2** beschrieben sind wurde die Verbindung **28.1** mit 2-Brom-1-brommethyl-4-fluoro-benzol zur Reaktion gebracht und lieferte **28.2.** Molekulargewicht 455,06 ($C_{22}H_{19}BrFN_3O_2$); Retentionszeit $R_t$ = 2.15 min. [B]; MS (ESI): 456,02 ($MH^+$).

4) Herstellung von 4-[3-(2-Amino-4-fluoro-benzyl)-4,4-dimethyl-2,5-dioxo-imidazolidin-1-yl]-2-cyclopropyl-benzonitril **28.3:**

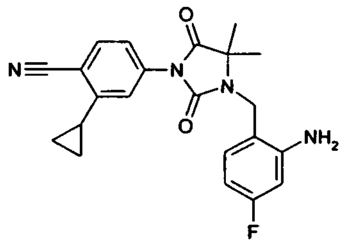

Analog der Vorgehensweise wie sie für die Herstellung von 1.3 beschrieben ist, wurde **28.2** mit Benzophenonimin zu **28.3** umgesetzt. Die Verbindung wurde als Salz mit Trifluoroessigsäure isoliert. Molekulargewicht (freie Base) 392,16 ($C_{22}H_{21}FN_4O_2$); Retentionszeit $R_t$ = 1.87 min. [B]; MS (ESI): 393,25 ($MH^+$).

5) Herstellung von 4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonylchlorid **28.4:**

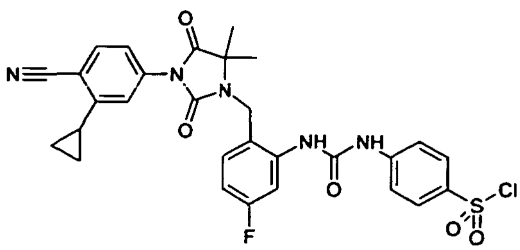

Analog der Vorgehensweise wie sie bei der Herstellung der Verbindung **1.4** beschrieben ist, wurde das Anilin **28.3** mit 4-(Chlorsulfonyl)-phenylisocyanat umgesetzt und lieferte 4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonylchlorid (**28.4**), welches ohne weitere Reinigung in die nächste Stufe eingesetzt wurde.

6) Herstellung von 4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonsäure-amid **28:**

Die Umsetzung des Sulfonsäurechlorids **28.4** mit einer Lösung von Ammoniak in Methanol (siehe Herstellung

der Verbindung des Beispiels **1**) lieferte 4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonsäure-amid **(28).** Molekulargewicht 590,17 ($C_{29}H_{27}FN_6O_5S$); Retentionszeit $R_t$ = 1.76 min. [B]; MS (ESI): 591,32 ($MH^+$).

**Beispiel 29:** 4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonsäure

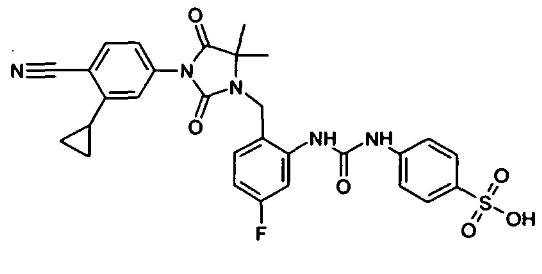

Analog der Vorgehensweise zur Herstellung der Verbindung des Beispiels **25** wurde das Sulfonsäurechlorid **28.4** mit Pyridin umgesetzt und lieferte nach chromatographischer Reinigung 4-(3-{2-[3-(4-Cyano-3-cyclopropyl-phenyl)-5,5-dimethyl-2,4-dioxo-imidazolidin-1-ylmethyl]-5-fluoro-phenyl- ureido)-benzolsulfonsäure (**29**). Molekulargewicht 591,15 ($C_{29}H_{26}FN_5O_6S$); Retentionszeit $R_t$ = 1.51 min. [B]; MS (ESI): 592,30 ($MH^+$).

**Beispiel 30:** 1-{2-[3-(4-Cyano-3-cyclopropyl-phenyl)-5,5-dimethyl-2,4-dioxo-imidazolidin-1-ylmethyl]-5-fluoro-phenyl} -3-(4-methansulfonyl-phenyl)-harnstoff

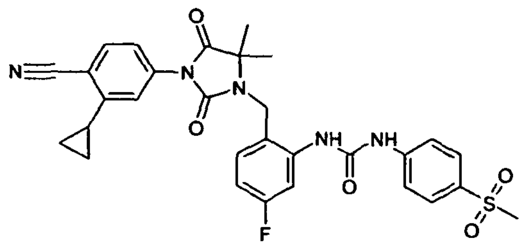

1) Herstellung von 1-{2-[3-(4-Cyano-3-cyclopropyl-phenyl)-5,5-dimethyl-2,4-dioxo-imidazolidin-1-ylmethyl]-5-fluoro-phenyl }-3-(4-methyls ulfanyl-phenyl)-harnstoff **(30.1):**

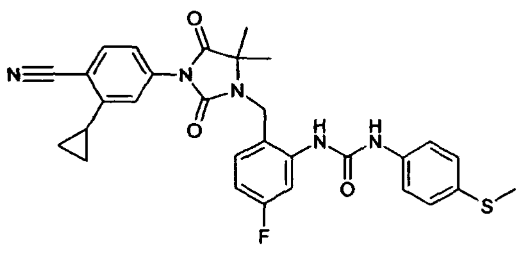

Die Verbindung **30.1,** 1-{2-[3-(4-Cyano-3-cyclopropyl-phenyl)-5,5-dimethyl-2,4-dioxo-imidazolidin-1-ylmethyl]-5-fluoro-phenyl}-3-(4-methylsulfanyl-phenyl)-harnstoff, wurde wie für die Verbindung **35** beschrieben durch

Umsatz der Verbindung **28.3** mit 1-Isocyanato-4-methylsulfanyl-benzol erhalten. Molekulargewicht 557,18 ($C_{30}H_{28}FN_5O_3s$); Retentionszeit $R_t$ = 3.70 min. [E]; MS (ESI): 558,14 ($MH^+$).

2) Herstellung von 1-{2-[3-(4-Cyano-3-cyclopropyl-phenyl)-5,5-dimethyl-2,4-dioxo-imidazolidin-1-ylmethyl]-5-fluoro-phenyl}-3-(4-methansulfonyl-phenyl)-harnstoff:

Analog der Vorgehensweise für die Herstellung der Verbindung des Beispiels **13** wurde für die Herstellung der Verbindung des Beispiels **30** die Verbindung **30.1** mit m-Chlorperbenzoesäure oxidiert. Molekulargewicht 589,17 ($C_{30}H_{28}FN_5O_5S$); Retentionszeit $R_t$ = 1.86 min. [B]; MS (ESI): 590,31 ($MH^+$).

**Beispiels 31 :** N-[4-(3-{5-Fluoro-2-[3-(4-fluoro-3-trifluoromethyl-phenyl)-5,5-dimethyl-2,4-dioxo-imidazolidin-1-ylmethyl]-phenyl }-ureido)-phenyl]-methansulfonamid

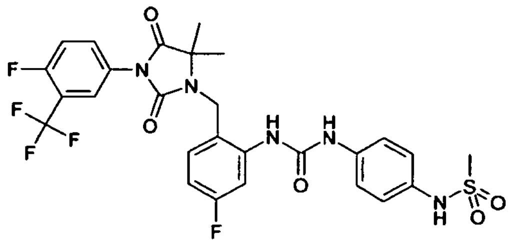

0,21 g der Verbindung des Beispiels **23** wurden in 5 ml trockenem Dichlormethan gelöst, mit 46 µl Pyridin und 44 µl Methansulfonylchlorid versetzt und 4 h bei Raumtemperatur gerührt. Zur Aufarbeitung wurde die Reaktionsmischung im Vakuum eingeengt und der Rückstand chromatographisch (Methode [RP1]) gereinigt. Die Gefriertrocknung der Produkt enthaltenden Eluate ergab N-[4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-phenyl]-methan-sulfonamid **31.** Molekulargewicht 625,14 ($C_{27}H_{24}F_5N_5O_5S$); Retentionszeit $R_t$ = 1.88 min. [B]; MS (ESI): 626,42 ($MH^+$).

**Beispiel 32:** [4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-phenyl]-phosphonsäurediethylester

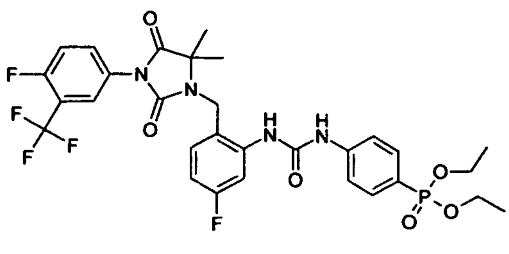

Zu 0,74 ml einer Lösung von Phosgen in Toluol (20%) wurden unter Argon bei 75°C 0,29 g der Verbindung **23.3,** gelöst in 10 ml trockenem Acetonitril, langsam zugetropft. Nach beendeter Zugabe wurde die Mischung 2 h bei 80°C gerührt. Danach wurde das Reaktionsgemisch im Vakuum eingeengt, der Rückstand mit Toluol versetzt und erneut im Vakuum eingeengt. Der Rückstand wurde in 10 ml trockenem Tetrahydrofuran gelöst und mit 0,16 g 4-Aminophenylphosphon-säurediethylester versetzt. Zu dieser Mischung wurden langsam 0,15 ml Triethylamin zugetropft. Anschließend wurde 4 h bei Raumtemperatur gerührt und dann der Ansatz über Nacht bei Raumtemperatur stehen gelassen. Die Reaktionsmischung wurde im Vakuum eingeengt und der Rückstand chromatographisch (Methode [RP1]) gereinigt. Man erhielt [4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-phenyl]-phosphonsäurediethylester. Molekulargewicht 668,18 ($C_{30}H_{30}F_5N_44_6P$); Retentionszeit $R_t$ = 2.70 min. [E]; MS (ESI): 669,34 ($MH^+$).

**Beispiel 33:** [4-(3-{5-Fluoro-2-[3-(4-fluoro-3-trifluoromethyl-phenyl)-5,5-dimethyl-2,4-dioxo-imidazolidin-1-ylmethyl]-phenyl }-ureido)-phenyl]-phosphonsäuremonoethylester

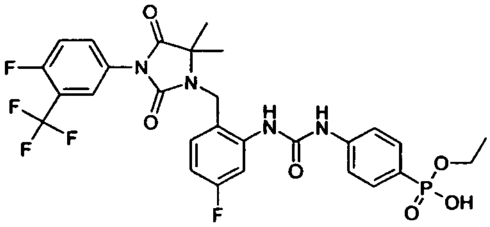

Wurde die Verbindung des Beispiels **32** mit Kaliumtrimethylsilanolat in Tetrahydrofuran behandelt, so konnte der Monoethylester **33** isoliert werden. Molekulargewicht 640,15 ($C_{28}H_{26}FsN_4O_6P$); Retentionszeit $R_t$ = 2.40 min. [E]; MS (ESI): 641,23 ($MH^+$).

**Beispiel 34:** [4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-phenyl]-phosphonsäure

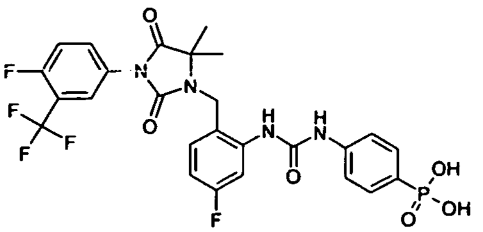

Eine Lösung der Verbindung des Beispiels **33** in Dioxan wurde mit konzentrierter Salzsäure versetzt und 8h bei 80° C gerührt. Man erhielt [4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-phenyl]-phosphonsäure **34.** Molekulargewicht 612,11 ($C_{26}H_{22}F_5N_4O_6P$); Retentionszeit $R_t$ = 2.26 min. [E]; MS (ESI): 613,18 ($MH^+$).

**Beispiel 38:** 1-{4-[5,5-Dimethyl-2,4-dioxo-3-(2-phenoxy-phenyl)-imidazolidin-1-ylmethyl]-phenyl} -3-phenyl-harnstoff

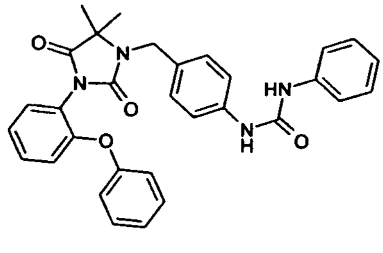

1) Herstellung von 5,5-Dimethyl-3-(2-phenoxy-phenyl)-imidazolidin-2,4-dion **(38.1):**

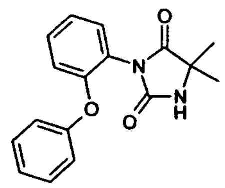

Die Verbindung **38.1**, 5,5-Dimethyl-3-(2-phenoxy-phenyl)-imidazolidin-2,4-dion, wurde analog wie für die Verbindung **23.1** beschrieben, durch Umsatz von 2-Amino-2-methyl-propionsäure-tert.-butylester Hydrochlorid mit Triethylamin und 1-Isocyanato-2-phenoxy-benzol gewonnen. $^1$H NMR: 8.4, s, 1H; 7.5 - 7.25, m, 5H; 7.18, m, 2H; 6.9, d, 2H; 1.35, s, 3H; 1.1, s, 3H.

2) Herstellung von 1-{4-[5,5-Dimethyl-2,4-dioxo-3-(2-phenoxy-phenyl)-imidazolidin-1-ylmethyl]-phenyl}-3-phenyl-harnstoff:

Der alkylierende Umsatz von **38.1** mit 1-(4-Chlormethyl-phenyl)-3-phenylharnstoff mit Cäsiumcarbonat in Acetonitril lieferte die Titelverbindung des Beispiels **38.** Molekulargewicht 520,21 ($C_{31}H_{28}N_4O_4$); Retentionszeit $R_t$ = 2.41 min. [C]; MS (ESI): 521,19 ($MH^+$).

In entsprechender Weise wurde die Verbindung des Beispiels **39**

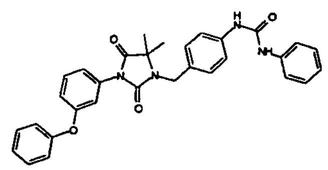

(1-{4-[5,5-Dimethyl-2,4-dioxo-3-(3-phenoxy-phenyl)-imidazolidin-1-ylmethyl]-phenyl]-3-phenyl-harnstoff; Molekulargewicht 520,21 ($C_{31}H_{28}N_4O_4$); Retentionszeit $R_t$ = 2.06 min. [B]; MS (ESI): 521,24 ($MH^+$)) durch Alkylierung von **39.1** (5,5-Dimethyl-3-(3-phenoxy-phenyl)-imidazolidin-2,4-dion; hergestellt durch Reaktion von 2-Amino-2-methyl-propionsäure-tert.-butylester mit 1-Isocyanato-3-phenoxy-benzol; $^1$H NMR: 8.55, s, 1H; 7.5 - 7.38, m, 3H; 7.16, m, 2H; 7.1-7.0, m, 4H; 1.39, s, 6H) mit 1-(4-Chlormethyl-phenyl)-3-phenyl-harnstoff gewonnen.

In Analogie dazu wurde auch die Verbindung des Beispiels **40**

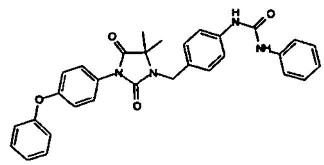

(1-{4-[5,5-Dimethyl-2,4-dioxo-3-(4-phenoxy-phenyl)-imidazolidin-1-ylmethyl]-phenyl}-3-phenyl-harnstoff; Molekulargewicht 520,21 ($C_{31}H_{28}N_4O_4$); Retentionszeit $R_t$ = 2.04 min. [B]; MS (ESI): 521,52 ($MH^+$)) durch Alkylierung von **40.1** (5,5-Dimethyl-3-(4-phenoxy-phenyl)-imidazolidin-2,4-dion; hergestellt durch Reaktion von 2-Amino-2-methyl-propionsäure-tert.-butylester mit 1-Isocyanato-4-phenoxy-benzol; $^1$HMR: 8.52, s, 1H; 7.47 - 7.35, m, 4H; 7.20, t, 1H; 7.08, m, 4H; 1.40, s, 6H) mit 1-(4-Chlormethyl-phenyl)-3-phenyl-harnstoff gewonnen.

**Beispiel 41:** 1-{2-[3-(4-Cyano-3-trifluoromethyl-phenyl)-5,5-dimethyl-2,4-dioxo-imidazolidin-1-ylmethyl]-5-fluoro-phenyl}-3-(4-hydroxy-phenyl)-harnstoff

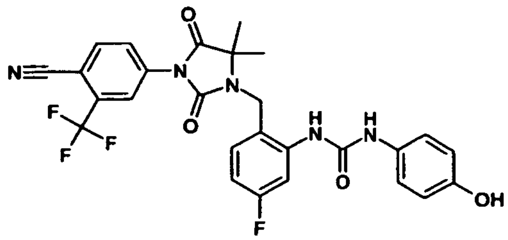

Zu 0,38 ml einer Lösung von Phosgen in Toluol (20%) wurden unter Argon bei 75°C 0,15 g der Verbindung **17.3,** gelöst in 10 ml trockenem Acetonitril, langsam zugetropft. Nach beendeter Zugabe wurde die Mischung 2 h bei 80°C gerührt. Danach wurde das Reaktionsgemisch im Vakuum eingeengt, der Rückstand mit Toluol versetzt und erneut im Vakuum eingeengt. Der Rückstand wurde in 5 ml trockenem Pyridin gelöst und mit 59 mg p-Aminophenol versetzt. Anschließend wurde 4 h bei Raumtemperatur gerührt und dann der Ansatz über Nacht bei Raumtemperatur stehen gelassen. Die Reaktionsmischung wurde im Vakuum eingeengt und der Rückstand chromatographisch (Methode [RP1]) gereinigt. Nach Gefriertrocknung erhielt man 1-{2-[3-{4-Cyano-3-trifluoromethyl-phenyl)-5,5-dimethyl-2,4-dioxo-imidazolidin-1-ylmethyl]-5-fluoro-phenyl}-3-(4-hydroxy-phenyl)-harnstoff. Molekulargewicht 555,15 ($C_{27}H_{21}F_4N_5O_4$); Retentionszeit $R_t$ = 3.22 min. [E]; MS (ESI): 556,12 ($MH^+$).

Die Verbindung des Beispiels **42** (isoliert als Hydrochlorid),

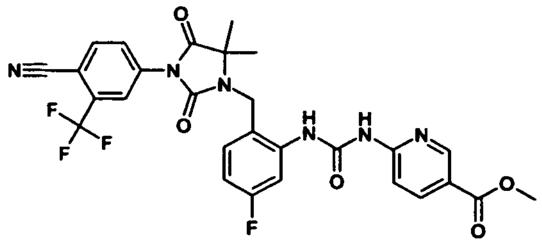

,

6-(3-{2-[3-(4-Cyano-3-trifluoromethyl-phenyl)-5,5-dimethyl-2,4-dioxo-imidazolidin-1-ylmethylJ-5-fluoro-phenyl}-ureido)-nicotinsäuremethylester (Molekulargewicht (freie Base) 598,15 ($C_{28}H_{22}F_4N_6O_5$); Retentionszeit $R_t$ = 3.45 min. [E]; MS (ESI): 599,07 ($MH^+$)),

des Beispiels **43,**

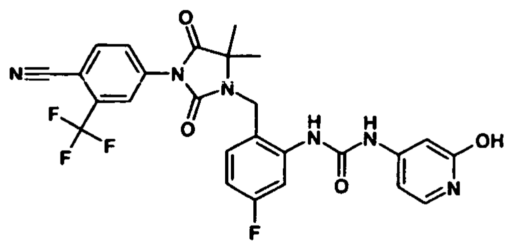

,

1-{2-[3-(4-Cyano-3-trifluoromethyl-phenyl)-5,5-dimethyl-2,4-dioxo-imidazolidin-1-ylmethyl]-5-fluoro- phenyl}-3-(2-hydroxy-pyridin-4-yl)-harnstoff (isoliert als Hydrochlorid), Molekulargewicht (freie Base) 556,14 $O_{26}H_{20}F_4N_6O_4$); Retentionszeit $R_t$ = 2.88 min. [E]; MS (ESI): 557,09 ($MH^+$)),

wurden wie die Verbindung **41** durch Umsatz der Verbindung **17.3** mit

6-Amino-nicotinsäuremethylester (für **42**),

3-Deazacytosin (für **43**)

erhalten.

**Beispiel 44:** 1-{5-Fluoro-2-[3-(4-fluoro-3-trifluoromethyl-phenyl)-5,5-dimethyl-2,4-dioxo-imidazolidin-1-ylmethyl]-phenyl}-3-(4-methansulfonyl-phenyl)-sulfamid

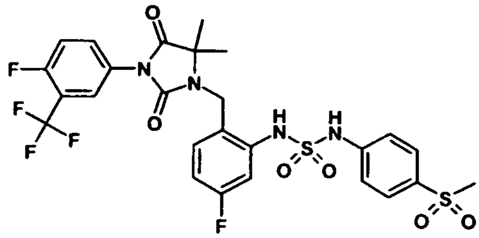

1) Herstellung von 1-tert-Butoxycarbonyl-3-{5-fluoro-2-[3-(4-fluoro-3-trifluoromethyl-phenyl)-5,5-dimethyl-2,4-dioxo-imidazolidin-1-ylmethyl]-phenyl}-sulfamid (**44.2**):

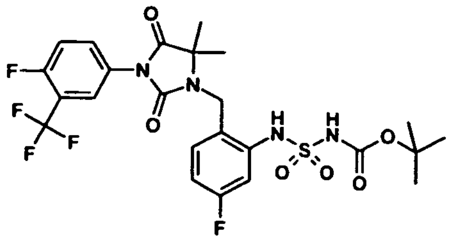

0,35 g der Verbindung **23.3** wurden bei Raumtemperatur in 15 ml trockenem Dichlormethan gelöst, mit 0,28 g N-(tert-butoxycarbonyl)sulfamoylchlorid (*in situ* hergestellt wie bei G. Dewynter et al., C. R. Acad. Sci. Paris, Ser. II, 1992, 315, 1675-1682 beschrieben) und 0,18 ml Triethylamin versetzt und 6 h bei Raumtemperatur gerührt und danach über Nacht stehen gelassen. Zur Aufarbeitung wurde die Reaktionsmischung im Vakuum eingeengt; der Rückstand wurde chromatographisch (Methode [RP2]) gereinigt. Nach Gefriertrocknung erhielt man 1-tert-Butoxycarbonyl-3 -{5-fluoro-2-[3-(4-fluoro-3-trifluoromethyl-phenyl)-5,5-dimethyl-2,4-dioxo-imidazolidin-1-ylmethyl]-phenyl}-sulfamid (**44.2**). Molekulargewicht 592,14 ($C_{24}H_{25}F_5N_4O_6S$); Retentionszeit $R_t$ = 3.68 min. [E]; MS (ESI): 537,04 ($MH^+$- $C_4H_8$).

2) Herstellung von {5-Fluoro-2-[3-(4-fluoro-3-trifluoromethyl-phenyl)-5,5-dimethyl-2,4-dioxo-imidazolidin-1-ylmethyl]-phenyl}-sulfamid (**44.1**):

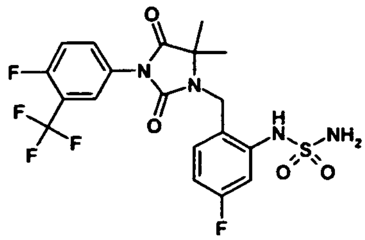

;

0,41 g der Verbindung **44.2** wurden bei Raumtemperatur in 10 ml trockenem Dichlormethan gelöst, mit 1,06 ml Trifluoroessigsäure und 0,11 ml Wasser versetzt und 4 h bei RT gerührt und anschließend über Nacht stehen gelassen, Zur Aufarbeitung wurde die Reaktionsmischung im Vakuum eingeengt, der Rückstand in Toluol gelöst und erneut eingeengt. Schließlich wurde der Rückstand in Dichlormethan gelöst und die organische Phase mit Natriumhydrogencarbonatlösung ausgeschüttelt. Die organische Phase wurde über Magnesiumsulfat getrocknet, filtriert und das Filtrat im Vakuum eingeengt. Man erhielt {5-Fluoro-2-[3-(4-fluoro-3-trifluoromethyl-phenyl)-S,5-dimethyl-2,4-dioxo-imidazolidin-1-ylmethyl]-phenyl}-sulfamid (**44.1**). Molekulargewicht 492,08 ($C_{19}H_{17}F_5N_4O_4S$); Retentionszeit $R_t$ = 3.53 min. [D]; MS (ESI): 493,14 ($MH^+$).

3) 1-{5-Fluoro-2-[3-(4-fluoro-3-trifluoromethyl-phenyl)-5,5-dimethyl-2,4-dioxo-imidazolidin-1-ylmethyl]-phenyl}-3-(4-methansulfonyl-phenyl)-sulfamid **44:**

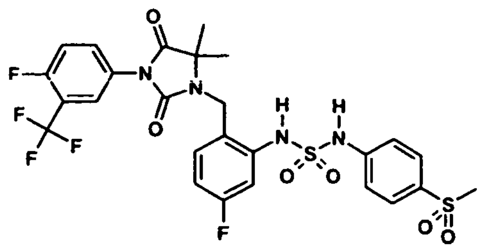

99 mg der Verbindung **44.1** und 0,14 g p-Brom-phenyl-methylsulfon wurden bei Raumtemperatur mit 12 mg Tris(dibenzylidenaceton)dipalladium(0), 10 µl Tri-(tert.)-butylphosphin und 65 mg Cäsiumcarbonat versetzt; unter einer Argonatmosphäre wurden 10 ml trockenes Dioxan zugegeben und die Reaktionsmischung wurde 6 h bei 80° C gerührt. Zur Aufarbeitung wurde die abgekühlte Reaktionsmischung filtriert und das Filtrat im Vakuum eingeengt. Der Rückstand wurde chromatographisch (Methode [RP1]) gereinigt und lieferte 1-{5-Fluoro-2-[3-(4-fluoro-3-trifluoromethyl-phenyl)-5,5-dimethyl-2,4-dioxo-imidazolidin-1-ylmethyl]-phenyl}-3-(4-methansulfonyl-phenyl)-sulfamid **44.** Molekulargewicht 646,09 ($C_{26}H_{23}F_5N_4O_6S_2$); Retentionszeit $R_t$ = 3.39 min. [E]; MS (ESI): 647,11 ($MH^+ - C_4H_8$).

**Beispiel 45:** 1-{2-[3-(4-Cyano-3-cyclopropyl-phenyl)-5,5-dimethyl-2,4-dioxo-imidazolidin-1-ylmethyl]-5-fluoro-phenyl}-3-(4-ethansulfonyl-phenyl)-harnstoff

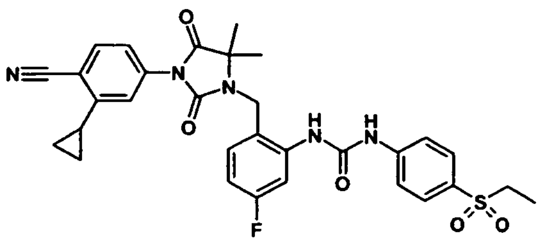

0,32 ml einer Lösung von Phosgen in Toluol (20%ig) wurden unter einer Argonatmosphäre bei 75°C tropfenweise unter Rühren mit einer Lösung von 0,12 g der Verbindung **28.3** in 10 ml trockenem Acetonitril versetzt. Nach beendeter Zugabe wurde die Mischung noch 2 h bei 80°C gerührt. Zur weiteren Verarbeitung wurde das Gemisch im Vakuum eingeengt, der Rückstand mit Toluol versetzt und erneut im Vakuum eingeengt. Der Rückstand wurde in 5 ml trockenem Tetrahydrofuran gelöst, mit 83 mg 4-Ethylsulfonylanilin und 0,21 ml Triethylamin versetzt und 4 h bei Raumtemperatur gerührt und dann über Nacht stehengelassen. Zur Aufarbeitung wurde das Reaktionsgemisch im Vakuum eingeengt und der Rückstand chromatographisch (Methode [RP1]) gereinigt Man erhielt 1-{2-[3-(4-Cyano-3-cyclopropyl-phenyl)-5,5-dimethyl-2,4-dioxo-imidazolidin-1-ylmethyl]-5-fluoro-phenyl}-3-(4-ethansulfonyl-phenyl)-harnstoff **45.** Molekulargewicht 603,19 ($C_{31}H_{30}FN_5O_5S$); Retentionszeit $R_t$ = 3.36 min. [E]; MS (ESI): 604,24 ($MH^+$).

Auf dieselbe Art und Weise, jedoch unter Einsatz von (4-Amino-phenylsulfanyl)-essigsäuremethylester (**49.1**; hergestellt durch Veresterung (Thionylchlorid / Methanol) der entsprechenden Säure; $^1H$ NMR: 7.1, d, 2H; 6.5, d, 2H; 5.3, s, 2H; 3.6, s, 3H; 3.5, s, 2H) anstelle von Ethylsulfonylanilin und von **17.3** anstelle von **28.3** wurde die Verbindung **49** ([4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-phenylsulfanyl]-essigsäuremethylester,

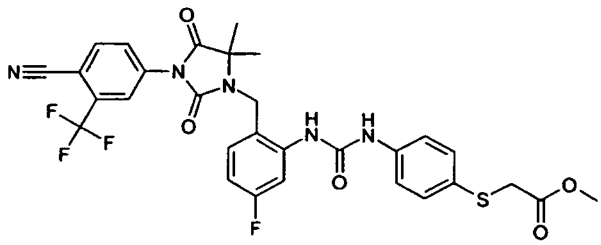

,

gewonnen. Molekulargewicht 643,15 ($C_{30}H_{25}F_4N_5O_5S$); Retentionszeit $R_t$ = 3.61 min. [E]; MS (ESI): 644,10 ($MH^+$).

**Beispiel 46:** [4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonyl]-essigsäuremethylester

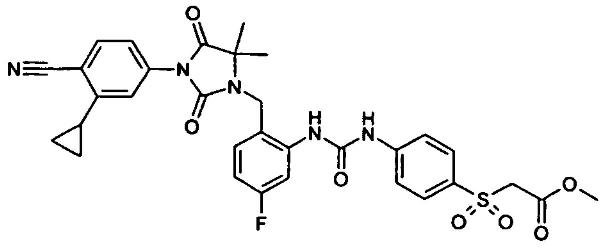

Die oxidative Umsetzung von **46.1** ([4-(3-{2-[3-(4-Cyano-3-cyclopropyl-phenyl)-5,5-dimethyl-2,4-dioxo-imidazolidin-1-ylmethyl]-5-fluoro-phenyl}-ureido)-phenylsulfanyl]-essigsäuremethylester, gewonnen durch Umsatz von **28.3** mit Phosgen in Toluol und **49.1;** Molekulargewicht 615,19 ($C_{32}H_{30}FN_5O_5S$); Retentionszeit $R_t$ = 3.58 min. [E]; MS (ESI): 616,19 ($MH^+$)) mit m-Chlorperbenzoesäure lieferte [4-(3-{2-[3-(4-Cyano-3-cyclopropyl-phenyl)-5,5-dimethyl-2,4-dioxo-imidazolidin-1-ylmethyl]-5-fluorophenyl}-ureido)-benzolsulfonyl]-essigsäuremethylester **46.** Molekulargewicht 647,18 ($C_{32}H_{30}FN_5O_7S$); Retentionszeit $R_t$ = 3.36 min. [E]; MS (ESI): 648,23 ($MH^+$).

**Beispiel 47:** [4-(3-{2-[3-(4-Cyano-3-cyclopropyl-phenyl)-5,5-dimethyl-2,4-dioxo-imidazolidin-1-ylmethyl]-5-fluoro-phenyl}-ureido)-benzolsulfonyl]-essigsäure

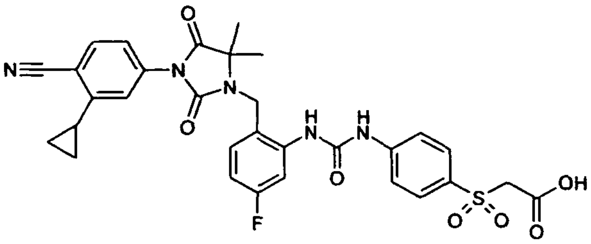

Die Umsetzung der Verbindung des Beispiels **46** mit Kaliumtrimethylsilanolat in Tetrahydrofuran lieferte die Säure des Beispiels **47** ([4-(3-{2-[3-(4-Cyano-3-cyclopropyl-phenyl)-5,5-dimethyl-2,4-dioxo-imidazolidin-1-ylmethyl]-5-fluorophenyl}-ureido)-benzolsulfonyl]-essigsäure). Molekulargewicht 633,16 ($C_{31}H_{28}FN_5O_7S$); Retentionszeit $R_t$ = 3.13 min. [E]; MS (ESI): 634,24 ($MH^+$).

**Beispiel 48:** 1-{2-[3-(4-Cyano-3-trifluoromethyl-phenyl)-5,5-dimethyl-2,4-dioxo-imidazolidin-1-ylmethyl]-5-fluoro-phenyl}-3-(4-methoxycarbonyl-phenyl)-sulfamid

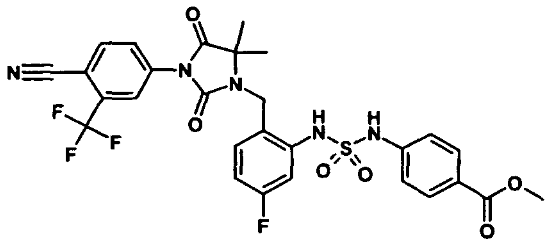

1) Herstellung von 4-Sulfoamino-benzoesäuremethylester Natriumsalz (**48.2**):

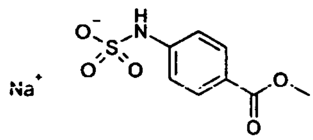

0,45 g 4-Aminobenzoesäuremetylester wurden in 15 ml trockenem Dichlormethan gelöst und die Lösung auf 0°C abgekühlt. Es wurden 2,1 ml Triethylamin zugegeben und danach tropfenweise mit 0,22 ml Chlorsulfonsäure versetzt. Man ließ die Reaktionsmischung auf Raumtemperatur erwärmen und rührte über Nacht. Zur Aufarbeitung wurde die Mischung im Vakuum eingeengt, der Rückstand mit 9 ml 1 N Natronlauge versetzt und erneut im Vakuum eingeengt. Das Rohprodukt wurde in heißem Ethanol aufgenommen, filtriert und das Filtrat im Vakuum eingeengt. Man erhielt das Natriumsalz des 4-Sulfoamino-benzoesäuremethylester (**48.2**). Molekulargewicht 230,01 ($C_8H_8NO_5$), Retentionszeit $R_t$ = 2.01 min. [D]; MS ($ES^-$): 229,89 ($M-H^+$).

2) Herstellung von 4-Chlorsulfonylamino-benzoesäuremethylester (**48.1**):

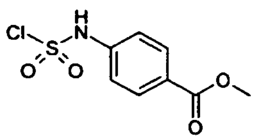

0,1 g der Verbindung des Beispiels **48.2** wurden in 5 ml trockenem Dichlormethan langsam mit 0,17 g Phosphorpentachlorid versetzt. Danach wurde die Mischung 4 h bei Raumtemperatur gerührt. Zur Aufarbeitung wurde die Mischung filtriert und das Filtrat im Vakuum eingeengt. Der Rückstand wurde ohne weitere Reinigung in die nächste Stufe eingesetzt.

3) 1-{2-[3-(4-Cyano-3-trifluoromethyl-phenyl)-5,5-dimethyl-2,4-dioxo-imidazolidin-1-ylmethyl]-5-fluoro-phenyl}-3-(4-methoxycarbonyl-phenyl)-sulfamid:

67 mg der Verbindung des Beispiels **17.3** wurden bei Raumtemperatur in 5 ml trockenem Dichlormethan gelöst, mit 80 mg **48.1** und 45 µl Triethylamin versetzt und 12h bei Raumtemperatur gerührt. Zur Aufarbeitung wurde die Reaktionsmischung im Vakuum eingeengt und der Rückstand chromatographisch (Methode [RP1]) gereinigt. Man erhielt

1-{2-[3-(4-Cyano-3-trifluoromethyl-phenyl)-5,5-dimethyl-2,4-dioxo-imidazolidin-1-ylmethyl]-5-fluoro-phenyl}-3-(4-metho xycarbonyl-phenyl)-sulfamid **48.** Molekulargewicht 633,13 ($C_{28}H_{23}F_4N_5O_6S$), Retentionszeit $R_t$ = 4.91 min. [D]; MS ($ES^-$): 631,94 ($M-H^+$).

**Beispiel 50:** [4-(3-{2-[3-(4-Cyano-3-trifluoromethyl-phenyl)-5,5-dimethyl-2,4-dioxo-imidazolidin-1-ylmethyl]-5-fluoro-phenyl}-ureido)-phenylsulfanyl]-essigsäure

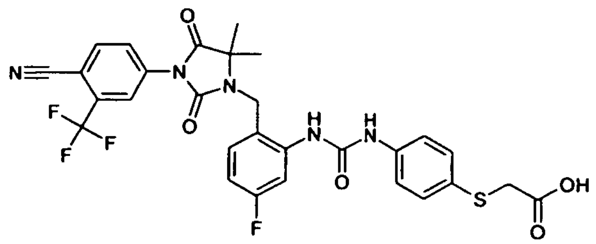

Analog der Vorgehensweise für die Herstellung der Verbindung des Beispiels **18** wurde der Ester **49** mit Kaliumtrimethylsilanolat unter Erhalt von **50** umgesetzt. Molekulargewicht 629,13 ($C_{29}H_{23}F_4N_5O_5S$); Retentionszeit $R_t$ = 3.69 min. [D]; MS (ESI): 630,14 ($MH^+$).

**Beispiel 54:** 4-(3-{2-[3-(2-Chloro-pyridin-4-yl)-5,5-dimethyl-2,4-dioxo-imidazolidin-1-ylmethyl]-5-fluoro-phenyl}-ureido)-benzoesäuresäuremethylester

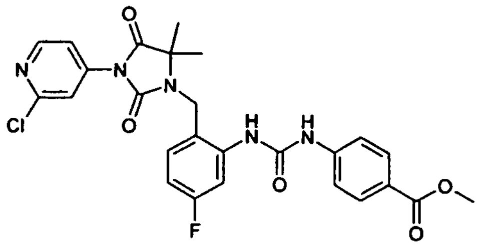

1) Herstellung von 3-(2-Chloro-pyridin-4-yl)-5,5-dimethyl-imidazolidin-2,4-dion (**54.3**):

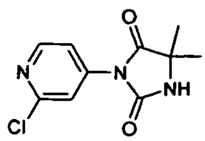

1,35 g 2-Chlor-4-Aminopyridin wurden bei Raumtemperatur in 10 ml trockenem Pyridin gelöst, mit 1,65 g 2-Isocyanoto-2-methyl-propionsäuremethylester versetzt, 20 h an mehreren Tagen bei 80°C gerührt und jeweils über Nacht bei Raumtemperatur stehengelassen. Das Reaktionsgemisch wurde im Vakuum eingeengt und der Rückstand chromatographisch (Methode [RP1]) gereinigt. Man erhielt 3-(2-Chloro-pyridin-4-yl)-5,5-dimethyl-imidazolidin-2,4-dion (**54.3**). Molekulargewicht 239,04 ($C_{10}H_{10}N_3O_2$), Retentionszeit $R_t$ = 2.75 min. [E]; MS (ESI): 240,06 ($MH^+$).

2) Herstellung von 3-(2-Chloro-pyridin-4-yl)-1-(4-fluoro-2-nitro-benzyl)-5,5-dimethyl-imidazolidin-2,4-dion (**54.2**):

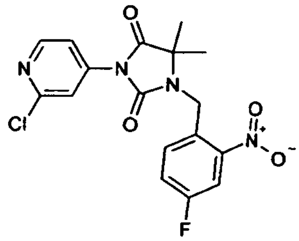

Die Verbindung **54.2** wurde analog der Herstellung der Verbindung **23.11** durch Umsatz von **54.3** mit **23.10** gewonnen. Molekulargewicht 392,06 ($C_{17}H_{14}ClFN_4O_4$), Retentionszeit $R_t$ = 3.33 min. [E]; MS (ESI): 393,10 ($MH^+$).

3) Herstellung von 1-(2-Amino-4-fluoro-benzyl)-3-(2-chloro-pyridin-4-yl)-5,5-dimethyl-imidazolidin-2,4-dion **(54.1):**

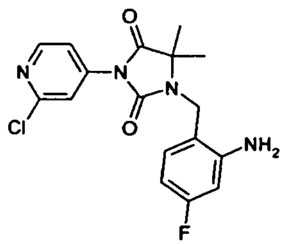

Die Verbindung **54.1** wurde analog der Vorgehensweise bei der Herstellung der Verbindung **23.3** durch Umsatz von **54.2** mit Palladiumhydroxid auf Kohle und Trimethylamin-Boran gewonnen. Molekulargewicht 362,09 ($C_{17}H_{16}ClFN_4O_2$), Retentionszeit $R_t$ = 3.15 min. [E]; MS (ESI): 363,08 ($MH^+$).

4) Herstellung von 4-(3-{2-[3-(2-Chloro-pyridin-4-yl)-5,5-dimethyl-2,4-dioxo-imidazolidin-1-ylmethyl]-5-fluoro-phenyl}-ureido)-benzoesäuresäuremethylester (**54**):

Wie für die Herstellung der Verbindung **1.4** beschrieben, wurde die Verbindung des Beispiels **54** durch Umsatz von **54.1** mit 4-Isocyanatobenzoesäuremethylester gewonnen. Molekulargewicht 539,13 ($C_{26}H_{23}ClFN_5O_5$), Retentionszeit $R_t$ = 3.41 min. [E]; MS (ESI): 540,17 ($MH^+$).

Die Verbindung des Beispiels **59**,

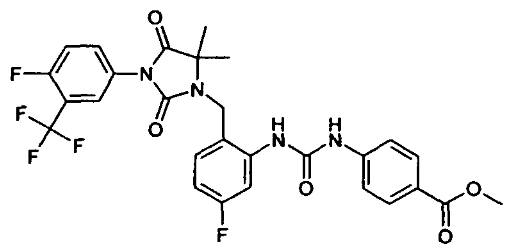

,

4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzoesäuremethylester (Molekulargewicht 590,15 ($C_{28}H_{23}F_5N_4O_5$); Retentionszeit $R_t$ = 3,68 min. [E]; MS (ESI): 591,16 ($MH^+$)), wurde wie die Verbindung **1.4** durch Umsatz der Verbindung **23.3** mit 4-Isocyanato-benzoesäuremethylester erhalten.

Die Verbindungen der Beispiele **57**,

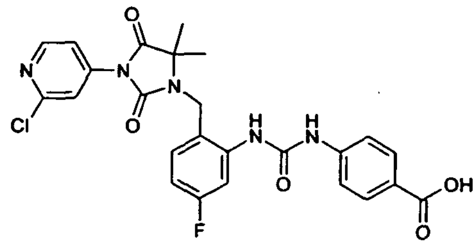

,

4-(3-{2-[3-(2-Chloro-pyridin-4-yl)-5,5-dimethyl-2,4-dioxo-imidazolidin-1-ylmethyl]-5-fluoro-phenyl}-ureido)-benzoesäure (Molekulargewicht 525,12 ($C_{25}H_{21}F_5N_5O_5$); Retentionszeit $R_t$ = 3,03 min. [E]; MS (ESI): 526,09 ($MH^+$)), und 60,

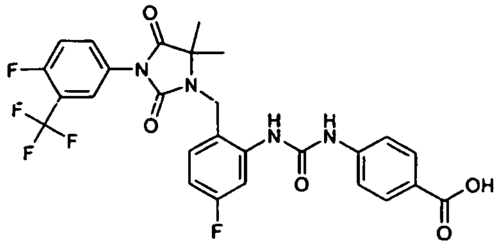

,

4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzoesäure (Molekulargewicht 576,14 ($C_{27}H_{21}F_5N_4O_5$); Retentionszeit $R_t$ = 3,33 min. [E]; MS (ESI): 577,11 ($MH^+$)), wurden, wie für das Beispiel **18** beschrieben, aus den Methylestern **54** und **59** durch Umsatz mit Kalium-trimethylsilanolat gewonnen.

**Beispiel 61**: N-[4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzoyl]-methansulfonamid

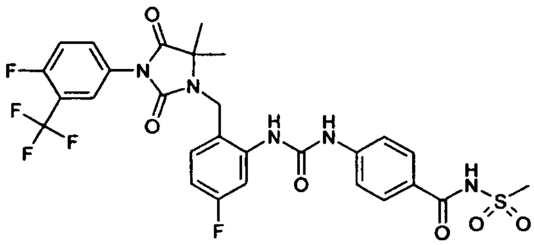

Zur Herstellung der Verbindung des Beispiels **61** wurden 0,17 g der Verbindung **60** bei Raumtemperatur in 10 ml trockenem Dichlormethan gelöst und unter Rühren mit 0,14 g N-(3-Dimethylaminopropyl)-N'-ethylcarbodiimid (EDC) und 0,11 g Dimethylaminopyridin (DMAP) versetzt. Zu dieser Mischung wurden 29 mg Methansulfonsäureamid zugegeben und anschließend 4 h bei Raumtemperatur gerührt. Die Reaktionsmischung wurde im Vakuum eingeengt; der Rückstand wurde in wenig Wasser aufgenommen und mit 1 N Salzsäure angesäuert. Das ausgefallene Produkt wurde abgesaugt, mit Wasser gewaschen und getrocknet. Die chromatographische Reinigung (Methode [RP1]) lieferte N-[4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzoyl]-methansulfonamid **61**. Molekulargewicht 653,13 ($C_{28}H_{24}F_5N_5O_6S$); Retentionszeit $R_t$ = 3,33 min. [E]; MS (ESI): 654,11 ($MH^+$).

Die Verbindungen des Beispiels **62**,

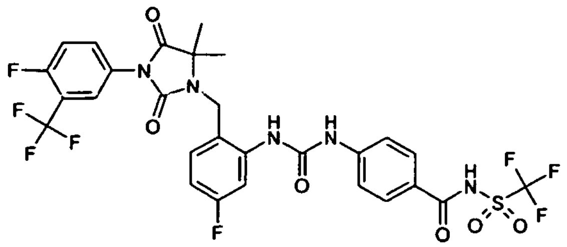

,

C,C,C-Trifluoro-N-[4-(3-{5-fluoro-2-[3-(4-fluoro-3-trifluoromethyl-phenyl)-5,5-dimethyl-2,4-dioxo-imidazolidin-1-ylmethyl]-phenyl}-ureido)-benzoyl]-methanesulfonsäureamid, (Molekulargewicht 707,10 ($C_{28}H_{21}F_8N_5O_6S$); Retentionszeit $R_t$ = 3,33 min. [E]; MS (ESI): 708,15 ($MH^+$)); des Beispiels **63**,

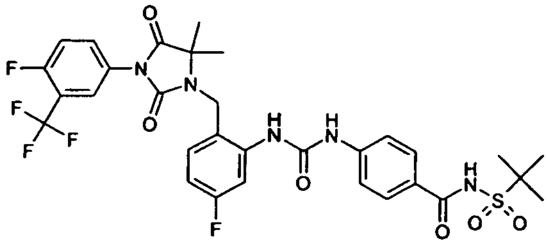

,

2-Methyl-propan-2-sulfonsäure- **4-**(3-{5-fluoro-2-[3-(4-fluoro-3-trifluoromethyl-phenyl)-5,5-dimethyl-2,4-dioxo-imidazolidin-1-ylmethyl]-phenyl}-ureido)-benzoylamid, (Molekulargewicht 695,18 ($C_{31}H_{30}F_5N_5O_6S$); Retentionszeit $R_t$ = 3,48 min. [E]; MS (ESI): 696,18 ($MH^+$)); des Beispiels **64**,

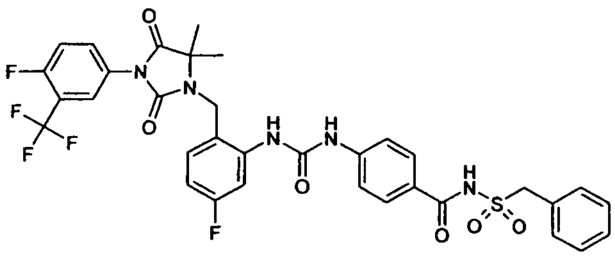

,

N-[4-(3-{5-Fluoro-2-[3-(4-fluoro-3-trifluoromethyl-phenyl)-5,5-dimethyl-2,4-dioxo-imidazolidin-1-ylmethyl]-phenyl}-ureido)-benzoyl]-C-phenyl-methansulfonsäureamid, (Molekulargewicht 729,16 ($C_{34}H_{28}F_5N_5O_6S$); Retentionszeit $R_t$ = 3,58 min. [E]; MS (ESI): 730,19 ($MH^+$)); und des Beispiels **65**,

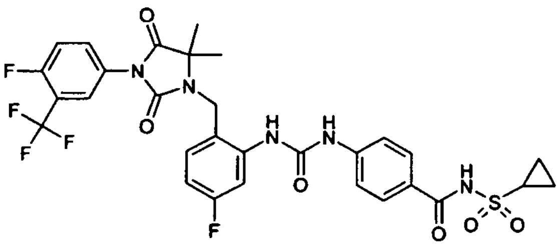

,

Cyclopropansulfonsäure-4-(3-{5-fluoro-2-[3-(4-fluoro-3-trifluoromethyl-phenyl)-5,5-dimethyl-2,4-dioxo-imidazolidin-1-ylmethyl]-phenyl}-ureido)-benzoylamid, (Molekulargewicht 679,15 ($C_{30}H_{26}F_5N_5O_6S$); Retentionszeit $R_t$ = 3,41 min. [E]; MS (ESI): 680,17 ($MH^+$));
wurden analog der Vorgehensweise für die Verbindung **61** hergestellt: für **62** wurde Trifluormethansulfonsäureamid, für

**63** tert-Butylsulfonsäureamid, für **64** Benzylsulfonsäureamid und für **65** Cyclopropansulfonsäureamid eingesetzt.

**Beispiel 66**: 1-{2-[3-(4-Cyano-3-cyclopropyl-phenyl)-5,5-dimethyl-2,4-dioxo-imidazolidin-1-ylmethyl]-4-trifluoromethyl-phenyl}-3-(4-methansulfonyl-phenyl)-harnstoff

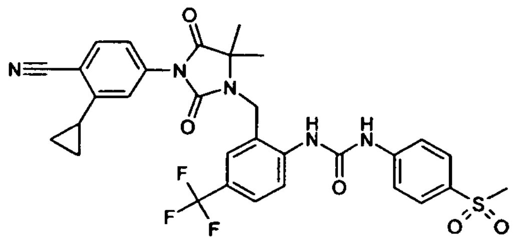

1) Herstellung von 4-[3-(2-Brom-5-trifluoromethyl-benzyl)-4,4-dimethyl-2,5-dioxo-imidazolidin-1-yl]-2-cyclopropyl-benzonitril (**66.3**):

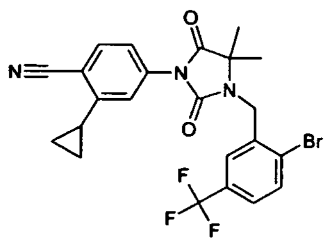

Unter Bedingungen wie sie für die Herstellung von **1.2** beschrieben sind, wurde die Verbindung **28.1** mit 2-Brom-1-brommethyl-5-trifluoromethyl-benzol zur Reaktion gebracht und lieferte **66.3**. Molekulargewicht 505,06 ($C_{23}H_{19}BrF_3N_3O_2$); Retentionszeit $R_t$ = 3,85 min. [E]; MS (ESI): 506,09 ($MH^+$).

2) Herstellung von 4-[3-(2-Amino-5-trifluoromethyl-benzyl)-4,4-dimethyl-2,5-dioxo-imidazolidin-1-yl]-2-cyclopropyl-benzonitril Trifluororessigsäuresalz (**66.2**):

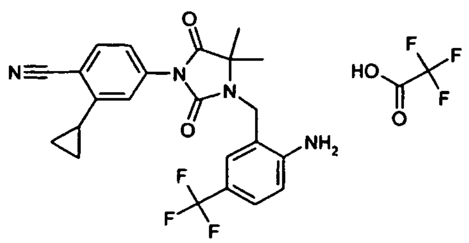

Analog der Vorgehensweise wie sie für die Herstellung von **1.3** beschrieben ist, wurde **66.3** mit Benzophenonimin zu **66.2** umgesetzt. Die Verbindung wurde als Salz mit Trifluoroessigsäure isoliert. Molekulargewicht (freie Base) 442,16 ($C_{23}H_{21}F_3N_4O_2$); Retentionszeit $R_t$ = 3.13 min. [E]; MS (ESI): 443,17 ($MH^+$).

3) Herstellung von 1-{2-[3-(4-Cyano-3-cyclopropyl-phenyl)-5,5-dimethyl-2,4-dioxo-imidazolidin-1-ylmethyl]-4-trifluoromethyl-phenyl}-3-(4-methylsulfanyl-phenyl)-harnstoff (**66.1**):

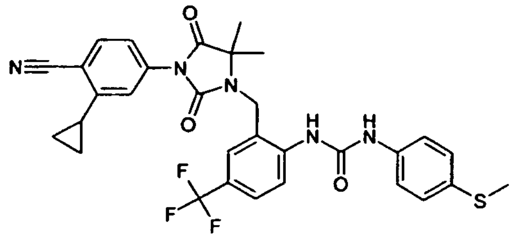

Die Verbindung **66.1** wurde wie für die Verbindung **35** beschrieben durch Umsatz der Verbindung **66.2** mit 1-Isocyanato-4-methylsulfanyl-benzol erhalten. Molekulargewicht 607,28 ($C_{31}H_{28}F_3N_5O_3S$); Retentionszeit $R_t$ = 2.75 min. [E]; MS (ESI): 608,23 ($MH^+$).

4) Herstellung von 1-{2-[3-(4-Cyano-3-cyclopropyl-phenyl)-5,5-dimethyl-2,4-dioxo-imidazolidin-1-ylmethyl]-4-trifluoromethyl-phenyl}-3-(4-methansulfonyl-phenyl)-harnstoff:

Analog der Vorgehensweise für die Herstellung der Verbindung des Beispiels **13** wurde für die Herstellung der Verbindung des Beispiels **66** die Verbindung **66.1** mit m-Chlorperbenzoesäure oxidiert. Molekulargewicht 639,17 ($C_{31}H_{28}F_3N_5O_5S$); Retentionszeit $R_t$ = 2.49 min. [E]; MS (ESI): 640,19 ($MH^+$).

In analoger Weise wurde die Verbindung des Beispiels **67**,

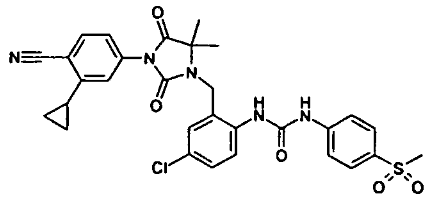

, 1-{4-Chloro-2-[3-(4-cyano-3-cyclopropyl-phenyl)-5,5-dimethyl-2,4-dioxo-imidazolidin-1-ylmethyl]-phenyl}-3-(4-methansul fonyl-phenyl)-harnstoff (Molekulargewicht 605,15 ($C_{30}H_{28}ClN_5O_5S$); Retentionszeit $R_t$ = 2.40 min. [E]; MS (ESI): 606,17 ($MH^+$)) über die Reaktionssequenz 5-Chlor-2-nitro-benzylalkohol → 1-Brommethyl-5-chlor-2-nitro-benzol (Bromierung mit Phosphortribromid in Dichlormethan; $^1H$ NMR: 8.12, d, 1H; 7.9, s, 1H; 7.72, d, 1H; 4.9, s, 2H) → 4-[3-(5-Chlor-2-nitro-benzyl)-4,4-dimethyl-2,5-dioxo-imidazolidin-1-yl]-2-cyclopropyl-benzonitril (**67.3**; Molekulargewicht 438,10 ($C_{22}H_{19}ClN_4O_4$); Retentionszeit $R_t$ = 3.10 min. [E]; MS (ESI): 439,16 ($MH^+$)) → 4-[3-(2-Amino-5-chlor-benzyl)-4,4-dimethyl-2,5-dioxo-imidazolidin-1-yl]-2-cyclopropyl-benzonitril Salz mit Trifluoressigsäure (**67**.2; freie Base: Molekulargewicht 408,13 ($C_{22}H_{21}ClN_4O_2$); Retentionszeit $R_t$ = 2.47 min. [E]; MS (ESI): 409,14 ($MH^+$)) → 1-{4-Chlor-2-[3- (4-cyano-3-cyclopropyl-phenyl)-5,5-dimethyl-2,4-dioxo-imidazolidin-1-ylmethyl]-phenyl}-3-(4-methylsulfanyl-phenyl)-harnstoff (**67.1**; Molekulargewicht 573,16 ($C_{31}H_{28}ClN_5O_3S$); Retentionszeit $R_t$ = 4.85 min. [D]; MS (ESI): 574,37 ($MH^+$)) → 1-{4-Chloro-2-[3-(4-cyano-3-cyclopropyl-phenyl)-5,5-dimethyl-2,4-dioxo-imidazolidin-1-ylmethyl]-phenyl}-3-(4-methans ulfonyl-phenyl)-harnstoff (**67**).

Die Verbindung des Beispiels **68**,

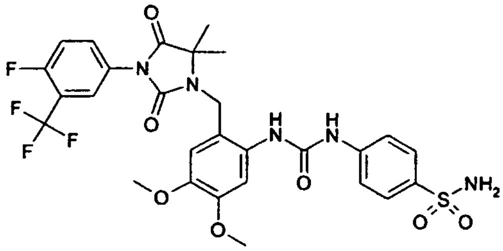

,

4-(3-{2-[3-(4-Fluoro-3-trifluoromethyl-phenyl)-5,5-dimethyl-2,4-dioxo-imidazolidin-1-ylmethyl]-4,5-dimethoxyphenyl}-ureido)-benzolsulfonsäureamid (Molekulargewicht 653,17 ($C_{28}H_{27}F_4N_5O_7S$); Retentionszeit $R_t$ = 2.51 min. [E]; MS (ESI): 654,17 ($MH^+$)) wurde über die Reaktionssequenz 3-(4-Fluoro-3-trifluoromethyl-phenyl)-5,5-dimethyl-imidazolidin-2,4-dion (**23.1**) → 1-(4,5-Dimethoxy-2-nitro-benzyl)-3-(4-fluoro-3-trifluoromethyl-phenyl)-5,5-dimethyl-imidazolidin-2,4-dion (**68.3**, Alkylierung mit 4,5-Dimethoxy-2-nitro-benzylbromid mit Cäsiumcarbonat in Acetonitril; Molekulargewicht 485,12 ($C_{21}H_{19}F_4N_3O_6$); Retentionszeit $R_t$ = 4.73 min. [D]; MS (ESI): 486,09 ($MH^+$)) → 1-(2-Amino-4,5-dimethoxy-benzyl)-3-(4-fluoro-3-trifluoromethyl-phenyl)-5,5-dimethyl-imidazolidin-2,4-dion (**68.2**, Reduktion von **68.3** mit Palladiumhydroxid auf Kohle und Trimethylamin Boran in Methanol; Molekulargewicht 455,14 ($C_2(H_{21}F_4N_3O_{4)}$; Retentionszeit $R_t$ = 2.12 min. [E]; MS (ESI): 456,12 ($MH^+$)) → 4-(3-{2-[3-(4-Fluoro-3-trifluoromethyl-phenyl)-5,5-dimethyl-2,4- dioxo-imidazolidin-1-ylmethyl]-4,5-dimethoxy-phenyl}-ureido)-benzolsulfonsäurechlorid (**68.1**, gewonnen analog der Vorgehensweise wie bei der Herstellung von **1.4** beschrieben durch Umsatz mit 4-Isocyanato-benzolsulfonsäurechlorid; die Verbindung wurde ohne weitere Reinigung in die nächste Stufe eingesetzt) → 4-(3-{2-[3-(4-Fluoro-3-trifluoromethyl-phenyl)-5,5-dimethyl-2,4-dioxo-imidazolidin-1-ylmethyl]-4,5-dimethoxy-phenyl}-ureido)-benzolsulfonsäureamid, **68** (gewonnen aus dem Sulfonsäurechlorid **68.1** durch Umsatz mit Ammoniak in Methanol analog der Vorgenhsweise wie bei 1.5 beschrieben).

In ähnlicher Weise wurde die Verbindung des Beispiels **69**,

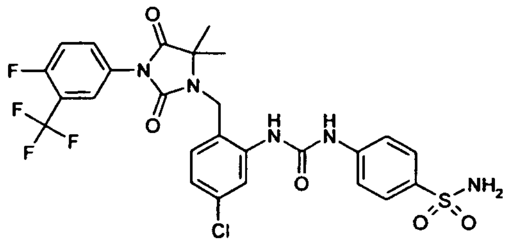

,

4-(3-{5-Chlor-2-[3-(4-fluoro-3-trifluoromethyl-phenyl)-5,5-dimethyl-2,4-dioxo-imidazolidin-1-ylmethyl]-phenyl}-ureido)-benzolsulfonsäureamid (Molekulargewicht 627,09 ($C_{26}H_{22}ClF_4N_5O_5S$); Retentionszeit $R_t$ = 2.52 min. [E]; MS (ESI): 628,11 ($MH^+$)) hergestellt: 3-(4-Fluoro-3-trifluoromethyl-phenyl)-5,5-dimethyl-imidazolidin-2,4-dion (**23.1**) → 1-(2-Brom-4-chlor-benzyl)-3-(4-fluoro-3-trifluoromethyl-phenyl)-5,5-dimethyl-imidazolidin-2,4-dion (**69.3**, durch Umsatz von **23.1** mit 2-Brom-4-chlor-benzylbromid; $^1H$ NMR: 8.0, m, 1H; 7.9, m, 1H; 7.8, s, 1H; 7.7, t, 1H; 7.6, d, 1H; 7.48, d, 1H; 4.6, s, 2H; 1.4, s, 6H) → 1-(2-Amino-4-chloro-benzyl)-3-(4-fluoro-3-trifluoromethyl-phenyl)-5,5-dimethyl-imidazolidin-2,4-dion (**69.2,** durch Umsatz mit Benzophenonimin analog der Vorgehensweise bei der Herstellung von **1.3**; Molekulargewicht 429,08 ($C_{19}H_{16}ClF_4N_3O_2$); Retentionszeit $R_t$ = 2.65 min. [E]; MS (ESI): 430,08 ($MH^+$)) → 4-(3-{5-Chlor-2-[3-(4-fluoro-3-trifluoromethyl-phenyl)-5,5-dimethyl-2,4-dioxo-imidazolidin-1-ylmethyl]-phenyl}-ureido)-benzolsulfonsäurechlorid (**69.1**, durch Umsatz mit 4-Isocyanato-benzolsulfonsäurechlorid; die Verbindung wurde ohne weitere Reinigung in die nächste Stufe eingesetzt) → 4-(3-{5-Chlor-2-[3-(4-fluoro-3-trifluoromethyl-phenyl)-5,5-dimethyl-2,4-dioxo-imidazolidin-1-ylmethyl]-phenyl}-ureido)-benzolsulfonsäureamid **69** (gewonnen aus dem Sulfonsäurechlorid **69.1** durch Umsatz mit Ammoniak in Methanol analog der Vorgenhsweise wie bei **1.5** beschrieben).

Auch die Verbindung des Beispiels **70**,

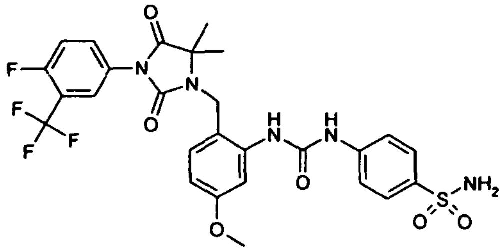

,

4-(3-{2-[3-(4-Fluoro-3-trifluoromethyl-phenyl)-5,5-dimethyl-2,4-dioxo-imidazolidin-1-ylmethyl]-5-methoxyphenyl}-ureido)-benzolsulfonsäureamid (Molekulargewicht 623,14 ($C_{27}H_{25}F_4N_5O_6S$); Retentionszeit $R_t$ = 2.31 min. [C]; MS (ESI): 624,12 ($MH^+$)) wurde über eine ähnliche Reaktionssequenz gewonnen: 3-(4-Fluoro-3-trifluoromethyl-phenyl)-5,5-dimethyl-imidazolidin-2,4-dion (**23.1**) → 3-(4-Fluoro-3-trifluoromethyl-phenyl)-1-(4-methoxy-2-nitrobenzyl)-5,5-dimethyl-imidazolidin-2,4-ion (**70.3**, durch Umsatz von **23.1** mit 1-Brommethyl-4-methoxy-2-nitro-benzol; Molekulargewicht 455,11 ($C_{20}H_{17}F_4N_3O_5$); Retentionszeit $R_t$ = 3.95 min. [D]; MS (ESI): 456,03 ($MH^+$)) → 1-(2-Amino-4-methoxy-benzyl)-3-(4-fluoro-3-trifluoromethyl-phenyl)-5,5-dimethyl-imidazolidin-2,4-dion (**70.2**, Reduktion von **70.3** mit Palladiumhydroxid auf Kohle und Trimethylamin Boran in Methanol; Molekulargewicht 425,13 ($C_{20}H_{19}F_4N_3O_3$); Retentionszeit $R_t$ = 2.34 min. [C]; MS (ESI): 426,06 ($MH^+$)) → 4-(3-{2-[3-(4-Fluoro-3-trifluoromethyl-phenyl)-5,5-dimethyl-2,4-dioxo-imidazolidin-1-ylmethyl]-5-methoxy-phenyl}-ureido)-benzolsulfonsäurechlorid (**70.1**, durch Umsatz mit 4-Isocyanato-benzolsulfonsäurechlorid; die Verbindung wurde ohne weitere Reinigung in die nächste Stufe eingesetzt) → 4-(3-{2-[3-(4-Fluoro-3-trifluoromethyl-phenyl)-5,5-dimethyl-2,4-dioxo-imidazolidin-1-ylmethyl]-5-methoxy-phenyl}-ureido)-benz olsulfonsäureamid **70** (gewonnen aus dem Sulfonsäurechlorid **70.1** durch Umsatz mit Ammoniak in Methanol analog der Vorgenhsweise wie bei **1.5** beschrieben).

Pharmakologische Prüfung:

*In vitro* Prüfungen:

*In vitro* funktionale Assays mit rekombinanten Zellen:

Funktionsüberprüfende Assays wurden mittels der FLIPR-Technik ("Fluorometric Imaging Plate Reader", Molecular Devices Corp.) durchgeführt.

Hierzu wurden ligand-induzierte Änderungen der intrazellulären Konzentration von $Ca^{2+}$ in rekombinanten HEK293 Zellen bestimmt, die sowohl einen Cannabinoidrezeptor (CB1 oder CB2) als auch G-Protein Galpha16 exprimierten. Für die Untersuchungen wurden Zellen in 96-well-Mikrotiterplatten (60000 Zellen/Vertiefung) ausgesät und übernacht wachsengelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz in Puffer gelöst zugegeben, 20 Minuten inkubiert, ein bekannter Cannabinoid-Rezeptor-Agonist als Referenzagonist in Puffer zugegeben und zum Schluss die Änderungen der intrazellulären $Ca^{2+}$-Konzentration im FLIPR-Gerät gemessen.

Ergebnisse wurden als prozentuale Änderung relativ zur Kontrolle dargestellt (0%: analoges Experiment ohne Testsubsstanz und ohne Referenzagonist, d.h. nur mit Puffer; 100%: analoges Experiment ohne Testsubstanz, aber mit Referenzagonist im Überschuss), zur Berechnung von Dosis/Wirkungskurven verwendet und $IC_{50}$-Werte bestimmt.

Ergebnisse:

Der folgenden **Tabelle 1** sind die Werte des funktionellen Assays gegenüber dem Cannabinoid 1 Rezeptor einschließlich beispielhafter Selektivitäten gegenüber dem Cannabinoid 2 Rezeptor zu entnehmen.

**Tabelle 1**:

| Beispiel Nr. | hCB1R: FLIPR; $IC_{50}$ [nM] | hCB2R: FLIPR; $IC_{50}$ [nM] |
|---|---|---|
| **1** | 24 | > 10000 |
| **2** | 220 | > 10000 |
| **3** | 19 | > 10000 |
| **4** | 109 | > 10000 |

(fortgesetzt)

| Beispiel Nr. | hCB1R: FLIPR; $IC_{50}$ [nM] | hCB2R: FLIPR; $IC_{50}$ [nM] |
|---|---|---|
| **6** | 11 | > 10000 |
| **8** | 35 | > 10000 |
| **10** | 53 | > 10000 |
| **16** | 12 | |
| **17** | 10 | |
| **19** | 9 | |
| **20** | 16 | |
| **22** | 9 | |
| **25** | 58 | |
| **26** | 66 | |
| **27** | 13 | |
| **28** | 9 | |
| **29** | 69 | |
| **30** | 11 | |
| **30.1** | 12 | |
| **31** | 32 | |
| **41** | 4 | |
| **43** | 27 | |
| **47** | 25 | |
| **49** | 2 | |
| **50** | 13 | |
| **60** | 53 | |
| **61** | 16 | |
| **62** | 46 | |
| **64** | 39 | |
| **65** | 42 | |
| **66** | 10 | |
| **67** | 6 | |
| **68** | 39 | |
| **69** | 11 | |
| **70** | 14 | |

Bindung an den CB 1 Rezeptor:

Testverbindungen: Die Verbindungen (3 µl, 10 mM, 100% DMSO), einpipettiert in 96-well PP Mikrotiterplatten, wurden mit 27 µl 100 % DMSO (Dimethylsulfoxid) verdünnt. Ausgehend von dieser Lösung wurden weitere 3-fach Verdünnungsschritte vorgenommen, indem jeweils 10 µl auf einer neue PP Mikrotiterplatte überführt und weitere 20 µl 100 % DMSO zugefügt wurden. Jeweils 6 µl dieser Lösungen wurden in neue 96-well PP Mikrotiterplatten transferiert und mit 144 µl Assaypuffer aufgefüllt. Die Endkonzentrationen reichten von 10 µM bis 0.005 µM.
Negativkontrolle: AM 251, gelöst in Assaypuffer mit 1 % DMSO, wurde zu den Verdünnungsreihen in den Mikrotiterplatten als Kontrolle mitgeführt. Die Endkonzentration betrug 1 µM.

Leerkontrolle: Assaypuffer mit 1 % DMSO wurde in den Verdünnungsreihen der Mikrotiterplatten als Leerkontrolle mitgeführt.

Zusammenfassung der Assayparameter:

| | | |
|---|---|---|
| Assayvolumen | | 200 µl |
| Rezeptor | CHO-K1/ cannabinoid CB1 Protein | 2 µg / well |
| Ligand | [$^3$H]-SR141716A | 0.5 nM<br>0.0195 µCi / well |
| Ionen | Tris-HCl | 50 mM, pH 7.4 |
| | $MgCl_2$ | 5 mM |
| | EDTA | 2.5 mM |
| | BSA (fettsäurefrei) | 0.2% |
| Unspezifische Bindung | AM 251 | 1 µM |
| Verbindung | in 1% DMSO | 10 µM bis 0.0050 µM |

Analyse der Daten:

Hohe Kontrolle: $^3$H Bindung ohne Zugabe der Verbindung
Niedrige Kontrolle: $^3$H Bindung in Gegenwart von 1 µM AM 251

Die Werte wurden über die korrigierten Rohdaten berechnet.

$$\text{Inhibierung der Ligandenbindung (\%)} = 100 * \left(1 - \frac{(sample - lowcontrol)}{(highcontrol - lowcontrol)}\right)$$

Die aufgeführten Werte wurden als Durchschnittswerte einer Doppelbestimmung gewonnen. Die $IC_{50}$ Werte wurden aus den Messwerten mit dem Programm Xlfit, Formel 205, berechnet. Ki-Werte wurden aus den $IC_{50}$- and Kd-Werten unter Benutzung der Cheng-Prusoff Gleichung erhalten:

$$Ki = \frac{IC50}{1 + \frac{C}{Kd}}$$

(C= Konzentration des Radioliganden)

Literatur: Cheng, Y.-C., und Prusoff, W.H. (1973) Biochem. Pharmacol 22, 3099-3108
Ergebnisse: $K_i$-Werte von Beispielverbindungen; **Tabelle 2:**

| Beispiel Nr. | hCB1R; Bindung $K_i$ [nM] |
|---|---|
| **1** | 20 |
| **3** | 11 |
| **7** | 73 |
| **9** | 32 |

(fortgesetzt)

| Beispiel Nr. | hCB1R; Bindung $K_i$ [nM] |
|---|---|
| **12** | 11 |
| **13** | 7 |
| **15** | 58 |
| **18** | 68 |
| **21** | 34 |
| **23** | 17 |
| **24** | 9 |
| **32** | 40 |
| **42** | 35 |
| **43** | 17 |
| **45** | 6 |
| **46** | 7 |
| **49** | 11 |
| **59** | 29 |

Aus dem Messdaten ist abzulesen, dass die erfindungsgemäßen Verbindungen der Formel I als CB1R Antagonisten wirken und daher gut zur Behandlung des metabolischen Syndroms, des Diabetes Typ II und der Adipositas geeignet sind.

*In vivo* Prüfungen:

"Milchkonsum bei Mäusen"

Der Test wird zur Untersuchung der anorexigenen Potenz der Testsubstanzen verwendet. Es werden weibliche NMRI-Mäuse, 25-35 g schwer, verwendet. Die Mäuse werden mindestens eine Woche an die Haltungsbedingungen und 2 Tage an die angebotene Kondensmilch gewöhnt.
Die Mäuse werden für 24 Stunden nüchtern gesetzt, haben aber beständig Zugang zu Wasser. Am Tage des Versuchs werden die Tiere einzeln aufgestallt, die Käfigdeckel können die mit Milch gefüllten Pipetten aufnehmen. Die Prüfsubstanzen werden oral, intraperitoneal oder subkutan verabreicht. Nach der Applikation werden die Mäuse in ihre Käfige gesetzt und erhalten 30 min. später Zugang zur Milch. Der Milchverbrauch wird alle 30 min. über 7 Stunden abgelesen, gleichzeitig werden offensichtliche Verhaltensänderungen der Tiere notiert.

"Antagonisierung CB1-vermittelter Hypothermie"

Der Test wird zur Messen der Potenz von cannabinoiden CB1-Rezeptor (CB1)-Antagonisten verwendet. Dabei wird gemessen, inwieweit die zu prüfenden CB1-Antagonisten in der Lage sind, die durch einen CB1-Agonisten induzierte Hypothermie zu verhindern, bzw. zu antagonisieren.
Es werden weibliche NMRI-Mäuse, 25-35 g schwer, verwendet. Die Mäuse werden mindestens eine Woche an die Haltungsbedingungen gewöhnt.
Zum Zeitpunkt 0 min. werden die Tiere mit dem zu prüfenden CB1-Antagonisten oral, intravenös oder intraperitoneal behandelt. 30 min. später wird den Mäusen der CB1-Agonist CP55.940, 1,25 mg/kg intraperitoneal verabreicht. Dies bewirkt ein Absinken der Körpertemperatur um 5-6 °C innerhalb von 30 min. Die Körpertemperatur wird zum ersten Mal 30 min. vor der Prüfsubstanz-Applikation und dann alle 30 min. nach dieser Applikation, ggf. unmittelbar vor einer Substanzapplikation über 4 Stunden rektal gemessen.
Die Potenz der Prüfsubstanzen wird als prozentuale Verringerung der Fläche unter der Temperatur-Zeit-Kurve angegeben, die zum einen von der durchschnittlichen Basaltemperatur, zum anderen von der Temperatur-Zeit-Kurve, der ausschließlich mit dem CB1-Antagonisten behandelten Tiere gebildet wird.

"Intestinale Motilität bei der Maus"

Die Methode dient zum einen der Untersuchung des Einflusses von Testsubstanzen selbst auf die Dünndarm-Motilität, zum anderen der Untersuchung, inwieweit spezifisch induzierte Effekte auf die Dünndarm-Motilität verhindert bzw. antagonisiert werden können, z.B. die Verzögerung der intestinalen Passage durch den cannabinoiden CB1-Agonist CP55.940.
Es werden weibliche NMRI-Mäuse mit einem gewicht von 25-35 g verwendet. Die Mäuse werden mindestens eine Woche an die Haltungsbedingungen gewöhnt.
Die Mäuse werden für 24 Stunden nüchtern gesetzt, haben aber beständig Zugang zu Wasser. Die Prüfsubstanzen werden oral, intravenös, subkutan aber nicht intraperitoneal verabreicht. Soll ein spezifischer Effekt antagonisiert werden, so wird die Prüfsubstanz 30-120 min. vor dem spezifischen Effektor verabreicht. 30 min. nach dieser Applikation wird eine definierte Menge eines gefärbten, nicht-kalorischen Füllstoffes per Gavage in den Magen eingebracht. Nach weiteren 30 min, der gefärbte Füllstoff hat zu diesem Zeitpunkt in etwa 80% des Dünndarms gefüllt, werden die Tiere getötet und der Dünndarm präpariert. Die intestinale Motilität wird als Passage des gefärbten Füllstoffes im Vergleich zur Geamtlänge des Dünndarms in Prozent angegeben. Ein Behandlungseffekt wird als Unterschied dieser Passage zur Vehikel-Kontrolle ebenfalls in Prozent angegeben.

## Patentansprüche

1. Verbindungen der Formel I,

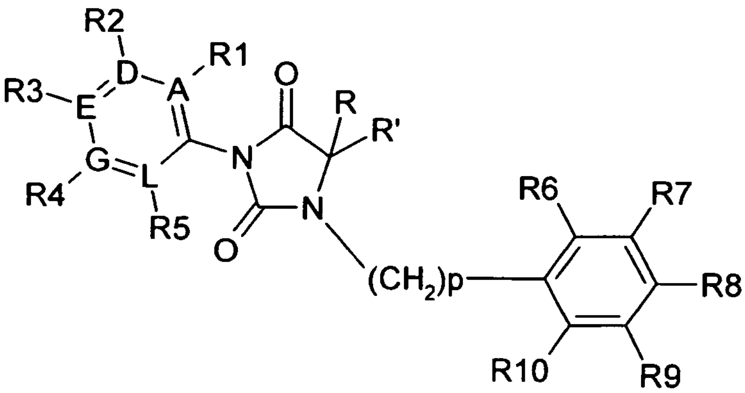

I

worin bedeuten

R, R' unabhängig voneinander H, $(CH_2)_n$-Aryl, $(C_1-C_6)$-Alkyl, wobei $(C_1-C_6)$-Alkyl oder der Arylrest substituiert sein kann mit Halogen, O-R14, $S(O)_m$-R12 oder NR13R15;
oder R und R' bilden gemeinsam einen Ring mit drei bis acht Kohlenstoffatomen, wobei ein Kohlenstoffatom durch O, $S(O)_m$, NR13 oder NR15 ersetzt sein kann;
m 0, 1, 2;
n 0, 1, 2, 3, 4;
p 1, 2, 3, 4, 5;
q 1, 2, 3, 4;
r 2, 3, 4, 5, 6;
v 0, 1, 2, 3, 4;
A, D, E, G, L unabhängig voneinander C oder N, wobei bei der Bedeutung N der entsprechende Substituent R1, R2, R3, R4, R5 entfällt,
oder R2-D=E-R3 oder R4-G=L-R5 haben die Bedeutung S oder O und wobei der Fünf- oder Sechsring mit $-(CH_2)_3-$ oder $-(CH_2)_4-$ oder -CH=CH-CH=CH- zu einem Bicyclus anelliert sein kann;
R1, R2, R3, R4, R5 unabhängig voneinander H, F, Cl, Br, J, CN, $N_3$, NC, $NO_2$, $CF_3$, $(C_1-C_8)$- Alkyl, $(C_3-C_8)$-Cycloalkyl, $(CH_2)_q$-[$(C_3-C_8)$-Cycloalkyl], $(CH_2)_n$-[$(C_3-C_8)$- Cycloalkenyl], $(CH_2)_n$-[$(C_7-C_{12})$-Bicycloalkyl],

$(CH_2)_n$-[($C_7$-$C_{12}$)-Tricycloalkyl], Adamantan-1-yl, Adamantan-2-yl, $(CH_2)_n$-Aryl, $(CH_2)_n$-Heteroaryl, $OCF_3$, O-R11, NR13R15, NH-CN, $S(O)_m$-R12, $SO_2$-$NH_2$, $SO_2$-N=CH-N$(CH_3)_2$, $SO_2$-NH- [($C_1$-$C_8$)-Alkyl], $SO_2$-NH-[($C_3$-$C_8$)-Cycloalkyl], $SO_2$-NH-$(CH_2)_n$-Aryl, $SO_2$-NH- $(CH_2)_n$-Heteroaryl, $SO_2$-N[($C_1$-$C_8$)-Alkyl]$_2$, $SO_2$-R16, $SF_5$, CO-O[($C_1$-$C_8$)- Alkyl], CO-O[($C_3$-$C_8$)-Cycloalkyl], CO-O-$(CH_2)_n$-Aryl, CO-O-$(CH_2)_n$-Heteroaryl, CO- $NH_2$, CO-NH-CN, CO-NH-[($C_1$-$C_8$)-Alkyl], CO-N[($C_1$-$C_8$)-Alkyl]$_2$, CO-NH- [($C_3$-$C_8$)-Cycloalkyl], CO-N[($C_3$-$C_8$)-Cycloalkyl]$_2$, C(=NH)-O-[($C_1$-$C_6$-Alkyl)], C(=NH)-$NH_2$, C(=NH)-R16, C(=NR13)-NR12R13, $(CH_2)_n$-C(=$NSO_2$-R12)$NH_2$, CO-R16, COOH, CO-($C_1$-$C_8$)-Alkyl, CO-($C_3$-$C_8$)-Cycloalkyl, CO-Aryl, CO- Heteroaryl, CH(OH)-Aryl, CH(OH)-Heteroaryl, CH[O-($C_1$-$C_6$)-Alkyl]-Aryl, CH[O-($C_1$-$C_6$)-Alkyl]-Heteroaryl, CHF-Aryl, CHF-Heteroaryl, $CF_2$-Aryl, $CF_2$- Heteroaryl, CHO, $CH_2$-OH, $CH_2$-CN, $CH_2$-O-R12, $CH_2$-O-$(CH_2)_n$-CO-O[($C_1$- $C_8$)-Alkyl], $CH_2$-O-$(CH_2)_n$-CO-$NH_2$, $CH_2$-O-$(CH_2)_q$-COOH,
wobei die Alkyl-, Cycloalkyl-, Cycloalkenyl-, Bicycloalkyl- und Tricycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, $OCF_3$, OH, O-$(CH_2)_n$-Aryl, $(CH_2)_n$-Aryl, $S(O)_m$-($C_1$-$C_6$)-Alkyl, $SO_2$-$NH_2$, SH, NR12R13, NH-CO-[($C_1$-$C_6$)-Alkyl], NH-CO-$(CH_2)_n$-Aryl, $(CH_2)_n$-COOH, $(CH_2)_n$-$CONH_2$, $(CH_2)_n$-CO-0($C_1$-$C_6$)-Alkyl, $(CH_2)_n$-CO-($C_1$- $C_6$)-Alkyl substituiert sein können und wobei die Alkylreste mit Fluoratomen substituiert sein können;
R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder 6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -($C_1$-$C_6$)-Alkyl substituiert sein kann und wobei der bicyclische Heterocyclus 9 bis 12 Ringglieder enthalten kann und bis zu fünf CH- bzw. $CH_2$- Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit

R11, F, Cl, Br, J, CN, $N_3$, NC, $NO_2$, $CF_3$, $(CH_2)_n$-O-R11, $(CH_2)_n$-O- $(CH_2)_r$-OH, $(CH_2)_n$-O-CH$(CH_2OH)_2$, $(CH_2)_n$-O-$(CH_2)_n$-CO-O-$(CH_2)_r$- $NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O($C_1$-$C_6$)-Alkyl]$_2$, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N(R13)$_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, $(CH_2)_n$-NR12-CO-N(R12)$_2$, $(CH_2)_n$-NR12-CO-NHR11, $(CH_2)_n$-NH- C(=NH)-$NH_2$, $(CH_2)_n$-NH-C(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$ -CO-NH- [($C_1$-$C_8$)-Alkyl], $(CH_2)_n$-NH-$(CH_2)_n$ -CO-NH-$(CH_2)_r$-OH, $(CH_2)_n$-NH- $(CH_2)_n$ -CO-N[(C1-C8)-Alkyl]$_2$, $(CH_2)_n$-NH-$(CH_2)_n$ -CO-NH-[($C_3$-$C_8$)- Cycloalkyl], $(CH_2)_n$-NH-$(CH_2)_n$-CO-N[($C_3$-$C_8$)-Cycloalkyl]$_2$, $(CH_2)_n$- NH-C$(CH_3)_2$-CO-O($C_1$-$C_8$)-Alkyl, $(CH_2)_n$-NH-C$(CH_3)_2$-CO-O($C_3$-$C_8$)-Cycloalkyl, $(CH_2)_n$-NH-C$(CH_3)_2$-CO-O-$(CH_2)_r$-$NH_2$, $(CH_2)_n$-NH- C$(CH_3)_2$-CO-O-$(CH_2)_n$-Aryl, $(CH_2)_n$-NH-C$(CH_3)_2$-CO-O-$(CH_2)_n$- Heteroaryl, $(CH_2)_n$-NH-C$(CH_3)_2$-CO-$NH_2$, $(CH_2)_n$-NH-C$(CH_3)_2$-CO-NH- [($C_1$-$C_8$)-Alkyl], $(CH_2)_n$-NH-C$(CH_3)_2$-CO-NH-$(CH_2)_r$-OH, $(CH_2)_n$-NH- C$(CH_3)_2$-CO-N[($C_1$-$C_8$)-Alkyl]$_2$, $(CH_2)_n$-NH-$(CH_3)_2$-CO-NH-[($C_3$-$C_8$)- Cycloalkyl], $(CH_2)_n$-NH-C$(CH_3)_2$-CO-N[($C_3$-$C_8$)-Cycloalkyl]$_2$, $(CH_2)_n$-NH-C$(CH_3)_2$-COOH, $S(O)_m$-R12, $SO_2$-R16, $SO_2$-N=CH-N$(CH_3)_2$,

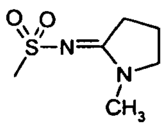
,

$SO_2$-NH-CO-R12, $SO_2$-NHR12, $SO_2$-NH-$(CH_2)_r$-OH, $SO_2$- N[($C_1$-$C_8$)-Alkyl]$_2$, $SO_2$-NH-$(CH_2)_r$-$NH_2$, $SF_5$, COOH, CO-$NH_2$, $(CH_2)_q$- CN, $(CH_2)_n$-CO-NH-CN, $(CH_2)_n$-CO-NH-piperidin-1-yl, $(CH_2)_n$-CO-NH- $SO_2$-NHR12, $(CH_2)_n$-CO-NH-$SO_2$-R18, $(CH_2)_n$-CHO, $(CH_2)_n$- C(=NH)$NH_2$, $(CH_2)_n$-C(=NH)-NHOH, $(CH_2)_n$-C(=NH)-[NH-O-($C_1$-$C_6$)- Alkyl], $(CH_2)_n$-C(=NH)(R16), $(CH_2)_n$-C(=NR13)NHR12, $(CH_2)_n$- C(=NR12) NR12R13, $(CH_2)_n$-C(=$NSO_2$-R12)$NH_2$, $(CH_2)_n$- C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O- ($C_1$-$C_6$)-Alkyl, $S(O)_m$-($C_1$-$C_6$)-Alkyl, $SO_2$-$NH_2$, COOH, $CONH_2$, CO-O($C_1$-$C_6$)-Alkyl, CO-($C_1$-$C_6$)-Alkyl substituiert sein können und wobei die Alkylreste mit Fluoratomen substituiert sein können, und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff

verschiedenen Substituenten versehen ist;

H, F, Cl, Br, J, CN, $N_3$, NC, $NO_2$, $CF_3$,
$(C_1-C_8)$-Alkyl, $(C_2-C_{10})$-Alkenyl, $(C_2-C_{10})$-Alkinyl, $(C_3-C_8)$-Cycloalkyl, Aryl, Heteroaryl,
$(CH_2)_n$-CO-[O-$(C_1-C_8)$-Alkyl], $(CH_2)_n$-CO-[O-$(C_3-C_8)$-Cycloalkyl], $(CH_2)_n$-CO- [$(C_1-C_8)$-Alkyl], $(CH_2)_n$-CO-[$(C_3-C_8)$-Cycloalkyl),
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-$(C_1-C_6)$-Alkyl], $(CH_2)_n$-P(O)[O-$(C_1-C_6)$-Alkyl]$_2$, $(CH_2)_n$- P(O)(OH)(O-$CH_2$-Aryl), $(CH_2)_n$-P(O)(O-$CH_2$-Aryl)$_2$, $(CH_2)_n$-P(O)$(OH)_2$, $(CH_2)_n$-$SO_3H$, $(CH_2)_n$-$SO_2$-$NH_2$,
$(CH_2)_n$-CO-NH-[$(C_1-C_8)$-Alkyl], $(CH_2)_n$-CO-N($(C_1-C_8)$-Alkyl]$_2$, $(CH_2)_n$-CO-NH- [$(C_3-C_8)$-Cycloalkyl],
$(C_2-C_{10})$-Alkenyl-CO-O[$(C_1-C_6)$-Alkyl], $(C_2-C_{10})$-Alkenyl-CONH$_2$, $(C_2-C_{10})$- Alkenyl-COOH, $(C_2-C_{10})$-Alkinyl-CO-O[$(C_1-C_6)$-Alkyl], $(C_2-C_{10})$-Alkinyl- CONH$_2$, $(C_2-C_{10})$-Alkinyl-COOH,
$(CH_2)_n$-CO-R16,
$(CH_2)_n$-OH, $(CH_2)_n$-O-$(C_1-C_8)$-Alkyl, $(CH_2)_n$-O-$(C_2-C_{10})$-Alkenyl, $(CH_2)_n$-O-$(C_2$- $C_{10})$-Alkinyl, $(CH_2)_n$-O-$(C_3-C_8)$-Cycloalkyl, $(CH_2)_n$-O-$(CH_2)_q$-[$(C_3-C_8)$- Cycloalkyl], $(CH_2)_n$-O-$(CH_2)_n$-CO-[O-$(C_1-C_8)$-Alkyl], $(CH_2)_n$-O-$(CH_2)_n$-CO-[O- $(C_3-C_8)$-Cycloalkyl], $(CH_2)_n$-O-$(CH_2)_n$-CO-[$(C_1-C_8)$-Alkyl], $(CH_2)$n-O-$(CH_2)_n$- CO-[$(C_3-C_8)$-Cycloalkyl], $(CH_2)_n$-O-$(CH_2)_n$-CO-[O-$(CH_2)_v$-Aryl], $(CH_2)_n$-O- $(CH_2)_n$-CO-[O-$(CH_2)_v$-Heteroaryl], $(CH_2)_n$-O-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O-$(CH_2)_n$-P(O)(OH)[O- $(C_1-C_6)$-Alkyl], $(CH_2)_n$-O-$(CH_2)_n$-P(O)[O-$(C_1-C_6)$-Alkyl]$_2$, $(CH_2)_n$-O-$(CH_2)_n$- P(O)(OH)(O-$CH_2$-Aryl), $(CH_2)_n$-O-$(CH_2)_n$-P(O)(O-$CH_2$-Aryl)$_2$, $(CH_2)_n$-O- $(CH_2)_n$-P(O)$(OH)_2$, $(CH_2)_n$-O-$(CH_2)_n$-S3H, $(CH_2)_n$-O-$(CH_2)_n$-$SO_2$-$NH_2$, $(CH_2)_n$- O-$(CH_2)_n$-CO-NH-[$(C_1-C_8)$-Alkyl], $(CH_2)_n$-O-$(CH_2)_n$-CO-N[$(C_1-C_8)$-Alkyl]$_2$, $(CH_2)_n$-O-$(CH_2)_n$-CO-NH-[$(C_3-C_8)$-Cycloalkyl], $(CH_2)_n$-O-$(CH_2)_n$-CR21R22- CO-O[$(C_1-C_6)$-Alkyl], $(CH_2)_n$-O-$(CH_2)_n$-CR21R22-CONH$_2$, $(CH_2)_n$-O-$(CH_2)_n$- CR21R22-COOH, $(CH_2)_n$-O-$(CH_2)_n$-CO-R16, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH- $(CH_2)_r$-OH, O-R13, $OCF_3$,
$(CH_2)_n$-$NH_2$, $(CH_2)_n$-NH-$(C_1-C_8)$-Alkyl, $(CH_2)_n$-NH-$(C_3-C_8)$-Cycloalkyl, $(CH_2)_n$-NH-$(CH_2)_n$-CO-[O-$(C_3-C_8)$-Cycloalkyl], $(CH_2)_n$-NH-$(CH_2)_n$-CO-[$(C_1$- $C_8)$-Alkyl], $(CH_2)_n$-NH-$(CH_2)_n$-CO-[$(C_3-C_8)$-Cycloalkyl], $(CH_2)_n$-NH-$(CH_2)_n$- CO-[O-$(CH_2)_v$-Aryl], $(CH_2)_n$-NH-$(CH_2)_n$-CO-[O-$(CH_2)_v$-Heteroaryl], $(CH_2)_n$- NH-$(CH_2)_q$- CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[$(C_1-C_6)$-Alkyl], $(CH_2)_n$-NH-$(CH_2)_n$- CR21R22-CONH$_2$, $(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[$(C_1-C_8)$-Alkyl], $(CH_2)_n$-NH-$(CH_2)_n$-$SO_2$-$(C_3-C_8)$- Cycloalkyl], $(CH_2)_n$-NH-$SO_2$-$(CH_2)_n$-$NH_2$, $(CH_2)_n$-NH-$SO_2$-$(CH_2)_n$-NH-$(C_1-C_8)$- Alkyl, $(CH_2)_n$-NH-$SO_2$-$(CH_2)_n$-NH-$(C_3-C_8)$-Cycloalkyl, $(CH_2)_n$-NH-$SO_2$-$(CH_2)_n$- N[$(C_1-C_8)$-Alkyl]$_2$,
$(CH_2)_n$-NH-CN, $(CH_2)_n$-NH-$SO_2$-R16,
$(CH_2)_n$-NR12-CO-NH-$(C_1-C_8)$-Alkyl, $(CH_2)_n$-NR12-CO-NH-$(C_3-C_8)$- Cycloalkyl, $(CH_2)_n$-NR12-CO-$NH_2$, $(CH_2)_n$-NR12-CO-NH-$SO_2$-$(C_1-C_8)$-Alkyl, $(CH_2)_n$-NR12-CO-NH-$SO_2$-$(C_3-C_8)$-Cycloalkyl, $(CH_2)_n$-NR12-CO-N[$(C_1-C_8)$-Alkyl]$_2$, $(CH_2)_n$-NH-CO-NH-$(CH_2)_n$-CO-[O-$(C_1-C_8)$-Alkyl], $(CH_2)_n$-NH-CO- NH-$(CH_2)_q$-CO-$NH_2$, $(CH_2)_n$-NH-CO-NH-$(CH_2)_q$-COOH, $(CH_2)_n$-NH-C(=NH)- $NH_2$, $(CH_2)_n$-NH-C(=NH)-R16, $(CH_2)_n$-NH-C(=NH)-NH[$(C_1-C_8)$-Alkyl], $(CH_2)_n$-NH-C(=N-$SO_2$-$(C_1-C_8)$-Alkyl)-$NH_2$, $(CH_2)_n$-NH-C(=N-$SO_2$-$(C_1-C_8)$- Alkyl)-NH[$(C_1-C_8)$-Alkyl], $(CH_2)_n$-NH-C(=N-$SO_2$-$NH_2$)-$NH_2$, $(CH_2)_n$-NH- C(=N-$SO_2$-$NH_2$)-NH[$(C_1-C_8)$-Alkyl], $(CH_2)_n$-NH-C(=NH)- N[$(C_1-C_8)$-Alkyl]$_2$, $(CH_2)_n$-NH-C(=N-$SO_2$-$(C_1-C_8)$-Alkyl)-N[$(C_1-C_8)$-Alkyl]$_2$, $(CH_2)_n$-NH-$(CH_2)_n$- CO-O- $(CH_2)_r$-$NH_2$, $(CH_2)_n$-NH-$(CH_2)_n$ -CO-NH-[$(C_1-C_8)$-Alkyl], $(CH_2)_n$-NH- $(CH_2)_n$ -CO-NH-$(CH_2)_r$-OH, $(CH_2)_n$- NH-$(CH_2)_n$ -CO-N[$(C_1-C_8)$-Alkyl]$_2$, $(CH_2)_n$- NH-$(CH_2)_n$ -CO-NH-[$(C_3-C_8)$-Cycloalkyl], $(CH_2)_n$-NH-C$(CH_3)_2$- CO-O$(C_3-C_8)$-Cycloalkyl, $(CH_2)_n$-NH-C$(CH_3)_2$-CO-O-$(CH_2)_r$-$NH_2$, $(CH_2)_n$-NH-C$(CH_3)_2$-CO- O-$(CH_2)_n$-Aryl, $(CH_2)_n$-NH-C$(CH_3)_2$-CO-O-$(CH_2)_n$-Heteroaryl, $(CH_2)_n$-NH- C$(CH_3)_2$-CO-NH-[$(C_1-C_8)$-Alkyl], $(CH_2)_n$-NH- C$(CH_3)_2$-CO-NH-$(CH_2)_r$-OH, $(CH_2)_n$-NH-C$(CH_3)_2$-CO-N[$(C_1-C_8)$-Alkyl]$_2$, $(CH_2)_n$-NH-$(CH_3)_2$-CO-NH-[$(C_3$- $C_8)$-Cycloalkyl], $(CH_2)_n$-NH-C$(CH_3)_2$-CO-N[$(C_3-C_8)$-Cycloalkyl]$_2$, $(CH_2)_n$-S$(O)_m$-$(C_1$-C8)-Alkyl, $(CH_2)_n$- S$(O)_m$-$(C_3-C_8)$-Cycloalkyl, $(CH_2)_n$-$SO_2$- R16, $SO_2$-N=CH-N$(CH_3)_2$,

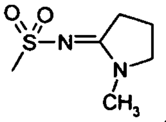

,

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)-Alkyl, $(CH_2)_n$-$SO_2$-NH-($C_3$-$C_8$)-Cycloalkyl, $(CH_2)_n$-$SO_2$-N[($C_1$-$C_8$)-Alkyl]$_2$, $SO_2$-NH- $(CH_2)_r$-OH, $SO_2$-NH-$(CH_2)_r$-$NH_2$, $SF_5$,
$(CH_2)_q$-CN,
$(CH_2)_n$-CO-NH-pipendin-1-yl,
$(CH_2)_n$-CO-NH-$SO_2$-NHR12, $(CH_2)_n$-CO-NH-$SO_2$-($C_1$-$C_8$)-Alkyl, $(CH_2)_n$-CO- NH-$SO_2$-($C_3$-$C_8$)-Cycloalkyl, $(CH_2)_n$-CHO, $(CH_2)_n$-C(=NH)$NH_2$, $(CH_2)_n$-C(=NH)NHOH, $(CH_2)_n$- C(=NH)(R16), $(CH_2)_n$-C(=NR13)NHR12, $(CH_2)_n$-C(=NR12)NR12R13, $(CH_2)_n$- C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, $S(O)_m$- ($C_1$-$C_6$)-Alkyl, $SO_2$-$NH_2$, COOH, $CONH_2$, CO-[O($C_1$-$C_6$)-Alkyl], CO-($C_1$-$C_6$)- Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und
wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, ($C_1$-$C_8$)-Alkyl oder =O substituiert sein können;

T NR23-CO-NR24, NR23-$SO_2$-NR24, $SO_2$-NR23-$SO_2$ CO-NR23-CO, NR23-. C(=NR 13)-NR24, NR23-C(=NR22)-NR24, CO-NR23-CR22R23, CO- CR22R23-CO, CR22R23-CO-CR22R24, NR23-CO-CR22R24, NR23-$SO_2$- CR22R24, CR22R24-CO-NR23, CR22R24-$SO_2$-NR23, CR22R23-NR23-$SO_2$, $SO_2$-CR22R23-NR23, $SO_2$-NR23-CR22R23-, NP,23-CR22R23-$SO_2$, CO-NR23- $SO_2$, $SO_2$-NR23-CO, CO-CR22R23-$SO_2$, $SO_2$-CR22R23-CO, CR23R24- CR23R24-CR23R24, CR23R24-NR23-CR23R24;
R11 H, ($C_1$-$C_8$)-Alkyl, ($C_2$-$C_{10}$)-Alkenyl, ($C_2$-$C_{10}$)-Alkinyl, ($C_3$-$C_8$)-Cycloalkyl, $(CH_2)_q$-[($C_3$-$C_8$)-Cycloalkyl], $(CH_2)_n$-Aryl, $(CH_2)_n$-CO-[O-($C_1$-$C_8$)-Alkyl], $(CH_2)_n$-CO-[O-($C_3$-$C_8$)-Cycloalkyl], $(CH_2)_n$-CO-[($C_1$-$C_8$)-Alkyl], $(CH_2)_n$-CO- [($C_3$-$C_8$)-Cycloalkyl], $(CH_2)_n$-CO-Aryl, $(CH_2)_n$-CO-Heteroaryl, $(CH_2)_q$-CO-$NH_2$, $(CH_2)_q$-COOH, $(CH_2)_n$-P(O)(OH)[O-($C_1$-$C_6$)-Alkyl], $(CH_2)_n$-P(O)[O-($C_1$-$C_6$)- Alkyl]$_2$, $(CH_2)_n$-P(O)(OH)(O-$CH_2$-Aryl), $(CH_2)_n$-P(O)(O-$CH_2$-Aryl)$_2$, $(CH_2)_n$- $P(O)(OH)_2$, $(CH_2)_n$-$SO_3H$, $(CH_2)_n$-$SO_2$-$NH_2$, $(CH_2)_n$-CO-NH-[($C_1$-$C_8$)-Alkyl], $(CH_2)_n$-CO-N[($C_1$-$C_8$)-Alkyl]$_2$, $(CH_2)_n$-CO-NH-[($C_3$-$C_8$)-Cycloalkyl], ($C_2$-$C_{10}$)- Alkenyl-CO-O[($C_1$-$C_6$)-Alkyl], ($C_2$-$C_{10}$)-Alkenyl-$CONH_2$, ($C_2$-$C_{10}$)-Alkenyl- COOH, ($C_2$-$C_{10}$)-Alkinyl-CO-O[($C_1$-$C_6$)-Alkyl], ($C_2$-$C_{10}$)-Alkinyl-$CONH_2$, ($C_2$- $C_{10}$)-Alkinyl-COOH, $(CH_2)_n$-CR21[(CO-O($C_1$-$C_6$)-Alkyl)]$_2$, $(CH_2)_n$- CR21$(CONH_2)_2$, $(CH_2)_n$-CR21$(COOH)_2$, $(CH_2)_n$-CR21R22-CO-O[($C_1$-$C_6$)- Alkyl], $(CH_2)_n$-CR21R22-$CONH_2$, $(CH_2)_n$-CR21R22-CO -NH-[($C_1$-$C_8$)-Alkyl], $(CH_2)_n$-CR21R22-CO-N[($C_1$-$C_8$)-Alkyl]$_2$, $(CH_2)_n$-CR21R22-COOH, $(CH_2)_n$-CO-R16, $(CH_2)_n$-CO-NH-C$(CH_3)_2$-CO-O[($C_1$-$C_8$)-Alkyl], $(CH_2)_n$-CO- NH-C$(CH_3)_2$-$CONH_2$, $(CH_2)_n$-CO-NH-C$(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, ($C_1$-$C_6$)-Alkyl, ($C_3$- $C_6$)-Cycloalkyl, O-($C_1$-$C_6$)-Alkyl, $S(O)_m$-($C_1$-$C_6$)-Alkyl, $SO_2$-$NH_2$, COOH, $CONH_2$, CO-O($C_1$-$C_6$)-Alkyl, CO-($C_1$-$C_6$)-Alkyl substituiert sein kann und wobei die Alkylreste mit Fluoratomen substituiert sein können;
R12 H, ($C_1$-$C_8$)-Alkyl, ($C_3$-$C_8$)-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, ($C_1$-$C_6$)-Alkyl, O-($C_1$-$C_6$)-Alkyl, $SO_2$-$NH_2$, COOH, $CONH_2$, CO-O($C_1$-$C_6$)-Alkyl, CO-($C_1$-$C_6$)- Alkyl substituiert sein kann und wobei die Alkylreste mit Fluoratomen substituiert sein können;
R13 H, $SO_2$-[($C_1$-$C_8$)-Alkyl), $SO_2$-[($C_3$-$C_8$)-Cycloalkyl], $SO_2$-$(CH_2)_n$-Aryl, $SO_2$-$(CH_2)_n$-Heteroaryl,
wobei die Alkyl- und Cycloalkylreste mit Fluoratomen substituiert sein können und wobei der Aryl- oder Heteroarylrest mit Halogen, CN, $CF_3$, ($C_1$-$C_6$)-Alkyl, ($C_3$-$C_6$)-Cycloalkyl, O-[($C_1$-$C_6$)-Alkyl], $S(O)_m$-[($C_1$-$C_6$)-Alkyl], $SO_2$-$NH_2$, COOH, $CONH_2$, CO-[O($C_1$-$C_6$)-Alkyl], CO-($C_1$-$C_6$)-Alkyl substituiert sein kann und wobei die Alkylreste mit Fluoratomen substituiert sein können;
R14 H, ($C_1$-$C_8$)-Alkyl, ($C_3$-$C_8$)-Cycloalkyl, $(CH_2)_n$-Aryl, $(CH_2)_n$-Heteroaryl, $(CH_2)_n$- CO-[O-($C_1$-$C_8$)-Alkyl], $(CH_2)_n$-CO-[O-$(CH_2)_n$-Aryl], $(CH_2)_n$-CO-[($C_1$-$C_8$)-Alkyl], $(CH_2)_n$-CO-[($C_3$-$C_8$)-Cycloalkyl], $(CH_2)_n$-CO-Aryl, $(CH_2)_n$-CO-Heteroaryl, $(CH_2)_q$-COOH,
wobei die Alkyl- und Cycloalkylreste mit Fluoratomen substituiert sein können und wobei der Aryl- oder Hete-

roarylrest mit Halogen, CN, $(C_1-C_6)$-Alkyl, $(C_3-C_6)$-Cycloalkyl, O-$(C_1-C_6)$-Alkyl, $S(O)_m$-$(C_1-C_6)$-Alkyl, $SO_2$-$NH_2$, COOH, $CONH_2$, CO-O$(C_1-C_6)$-Alkyl, CO-$(C_1-C_6)$-Alkyl substituiert sein kann und wobei die Alkylreste mit Fluoratomen substituiert sein können;

R15 H, $(C_1-C_8)$-Alkyl, $(C_3-C_8)$-Cycloalkyl, $(CH_2)_n$-Aryl, $(CH_2)_n$-Heteroaryl, $(CH_2)_n$-CO-[O-$(C_1-C_8)$-Alkyl], CO-[$(C_1-C_8)$-Alkyl], CO-[(C3-C8)-Cycloalkyl], CO-Aryl, CO-Heteroaryl, $(CH_2)_n$-CO-$NH_2$, $(CH_2)_q$-COOH, $(CH_2)_n$-$SO_2$-$NH_2$, $(CH_2)_n$-C$(CH_3)_2$-COOH,

wobei die Alkyl- und Cycloalkylreste mit Fluoratomen substituiert sein können und wobei der Aryl- oder Heteroarylrest mit Halogen, CN, $(C_1-C_6)$-Alkyl, O- $(C_1-C_6)$-Alkyl, $SO_2$-$NH_2$, COOH, $CONH_2$, CO-O$(C_1-C_6)$-Alkyl, CO-$(C_1-C_6)$- Alkyl substituiert sein kann

und wobei die Alkylreste mit Fluoratomen substituiert sein können;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[$(C_1-C_6)$- Alkyl]piperazin-1-yl, Thiomorpholin-4-yl, Thiomorpholin-1,1-Dioxid-4-yl, NH- $(CH_2)_r$-OH, NH-CH$(CH_2OH)_2$, NH-C$(CH_2OH)_3$, N[$(C_1-C_6)$-Alkyl-OH]$_2$, N[$(C_1-C_6)$-Alkyl][$(C_1-C_6)$-Alkyl-OH], D-Glucamin-N-yl, N-Methyl-D-Glucamin-N-yl, NH-[$(C_1-C_8)$-Alkyl]-CO-O$(C_1-C_6)$-Alkyl, NH-[$(C_1-C_8)$-Alkyl]-COOH, NH-[$(C_1-C_8)$-Alkyl]-$CONH_2$, N[$(C_1-C_6)$-Alkyl][$(C_1-C_8)$-Alkyl]-CO-O$(C_1-C_6)$-Alkyl, N[$(C_1-C_6)$- Alkyl][$(C_1-C_8)$-Alkyl]-COOH, N[$(C_1-C_6)$-Alkyl]$(C_1-C_8)$-Alkyl]- $CONH_2$, NH-[C(H)(Aryl)]-CO-O$(C_1-C_6)$-Alkyl, NH-[C(H)(Aryl)]-COOH, NH- [C(H)(Aryl)]-$CONH_2$, N[$(C_1-C_6)$-Alkyl][C(H)(Aryl)]-CO-O$(C_1-C_6)$-Alkyl, N[$(C_1-C_6)$-Alkyl][C(H)(Aryl)]-COOH, N[$(C_1-C_6)$-Alkyl][C(H)(Aryl)]-$CONH_2$, NH-[C(H)(Heteroaryl)]-CO-O$(C_1-C_6)$-Alkyl, NH-[C(H)(Heteroaryl)]-COOH, NH-[C(H)(Heteroaryl)]-$CONH_2$, N[$(C_1-C_6)$-Alkyl][C(H)(Heteroaryl)]-CO-O$(C_1-C_6)$-Alkyl, N[$(C_1-C_6)$-Alkyl][C(H)(Heteroaryl)]-COOH, N[$(C_1-C_6)$- Alkyl][C(H)(Heteroaryl)]-$CONH_2$, N[$(C_1-C_6)$-Alkyl][$(C_3-C_8)$-Cycloalkyl]-CO- O$(C_1-C_6)$-Alkyl, N[$(C_1-C_6)$-Alkyl][$(C_3-C_8)$-Cycloalkyl]-COOH, N[$(C_1-C_6)$- Alkyl][$(C_3-C_8)$-Cycloalkyl]-$CONH_2$, NH-[$(C_3-C_8)$-Cycloalkyl]-CO-O$(C_1-C_6)$- Alkyl, NH-[$(C_3-C_8)$-Cycloalkyl]-COOH, NH-[$(C_3-C_8)$-Cycloalkyl]-$CONH_2$, NH-$(C_1-C_8)$-Alkyl-OH, NH-[$(C_1-C_6)$-Alkyl]-$SO_2$-$(C_1-C_6)$-Alkyl, NH-[$(C_1-C_6)$- Alkyl]-$SO_3H$, NH-[$(C_1-C_6)$-Alkyl]-$SO_2$-$NH_2$, N[$(C_1-C_6)$-Alkyl]{[$(C_1-C_6)$-Alkyl]-$SO_3H$},

wobei die Alkohol (OH) - Funktionen durch F ersetzt sein können und wobei der Aryl- oder Heteroarylrest mit Halogen, CN, $(C_1-C_6)$-Alkyl, O-$(C_1-C_6)$-Alkyl, OH, $SO_2$-$NH_2$, COOH, $CONH_2$, CO-O$(C_1-C_6)$-Alkyl, CO-$(C_1C_6)$-Alkyl substituiert sein kann;

R18 $(CH_2)_n$-CR25R26-CO-O$(C_1-C_6)$-Alkyl, $(CH_2)_n$-CR25R26-CO-$NH_2$, $(CH_2)_n$- CR25R26-COOH;

R20 H, $(C_1-C_6)$-Alkyl, $(C_3-C_8)$-Cycloalkyl, Aryl, [$(C_1-C_6)$-Alkyl]-Aryl;

R21 H, F, $CF_3$, $(C_1-C_6)$-Alkyl, $(C_3-C_8)$-Cycloalkyl, OH, O-$(C_1-C_6)$-Alkyl, O-$(C_3-C_8)$- Cycloalkyl, O-$(CH_2)_n$-Aryl, O-(CO)-$(C_1-C_6)$-Alkyl, O-(CO)-$(C_3-C_8)$-Cycloalkyl, O-(CO)-O-$(C_1-C_6)$-Alkyl, O-(CO)-O-$(C_3-C_8)$-Cycloalkyl, NH[$(C_1-C_6)$-Alkyl]- Aryl, $NH_2$, NH-$(C_1-C_6)$-Alkyl, NH-(CO)-$(C_1-C_6)$-Alkyl;

R22 H, $CF_3$, $(C_1-C_6)$-Alkyl, Aryl, [$(C_1-C_6)$-Alkyl]-Aryl;

R23, R24 unabhängig voneinander H, $(C_1-C_6)$-Alkyl, $(C_3-C_8)$-Cycloalkyl, [$(C_1-C_6)$-Alkyl]- [$(C_3-C_8)$-Cycloalkyl], Aryl, [$(C_1-C_6)$-Alkyl]-Aryl

oder R23 und R24 bilden zusammen eine -CH=CH-, -$CH_2$-$CH_2$-, -$CH_2$-$CH_2$- $CH_2$-, oder -$CH_2$-$CH_2$-$CH_2$-$CH_2$-Einheit, worin eine $CH_2$-Gruppierung durch C=O, CHF oder $CF_2$ ersetzt sein kann, und worin bis zu vier Wasserstoffatome durch einen $(C_1-C_6)$-Alkylrest ersetzt sein können;

R25, R26 unabhängig voneinander H, F, $(C_1-C_6)$-Alkyl, Aryl, [$(C_1-C_6)$-Alkyl]-Aryl, wobei der Aryl mit Halogen, CN, OH, O-(C)-$C_6$)-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[$(C_1-C_6)$-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

2. Verbindungen der Formel I, gemäß Anspruch 1, **dadurch gekennzeichnet, dass** darin bedeuten

R, R' unabhängig voneinander H, $(CH_2)_n$-Aryl, $(C_1-C_{,6})$-Alkyl, wobei $(C_1-C_6)$-Alkyl oder der Arylrest substituiert sein kann mit Halogen;

oder R und R' bilden gemeinsam einen Ring mit drei bis acht Kohlenstoffatomen, wobei ein Kohlenstoffatom durch O, $S(O)_m$, NR13 oder NR15 ersetzt sein kann;

m 0, 1, 2;

n 0, 1, 2, 3;

p 1, 2, 3, 4;

q 1, 2, 3;

r 2, 3, 4, 5;

v 0, 1, 2, 3;

A, D, E, G, L unabhängig voneinander C oder N, wobei bei der Bedeutung N der entsprechende Substituent R1, R2, R3, R4., R5 entfällt,

oder R2-D=E-R3 Sechsring mit kann; oder R4-G=L-R5 haben die Bedeutung S oder O und wobei der Fünf- oder $-(CH_2)_3-$ oder $-(CH_2)_4-$ oder -CH=CH-CH=CH- zu einem Bicyclus anelliert sein

R1, R2, R3, R4, R5 unabhängig voneinander H, F, Cl, Br, J, CN, $CF_3$, $(C_1-C_8)$-Alkyl, $(C_3- C_8)$-Cycloalkyl, $(CH_2)_q-[(C_3-C_8)$-Cycloalkyl], $(CH_2)_n-[(C_7-C_{12})$-Bicycloalkyl], $(CH_2)_n-[(C_7-C_{12})$-Tricycloalkyl], Adamantan-1-yl, Adamantan-2-yl, *$(CH_2)_n$- Aryl, $(CH_2)_n$-Heteroaryl, $OCF_3$, O-R11, NR13R15, NH-CN, $S(O)_m$-R12, $SO_2$- $NH_2$, $SO_2-N=CH-N(CH_3)_2$, $SO_2-NH-[(C_1-C_8)$-Alkyl], $SO_2-NH-[(C_3-C_8)$- Cycloalkyl], $SO_2-NH-(CH_2)_n$-Aryl, $SO_2-NH-(CH_2)_n$-Heteroaryl, $SO_2-N[(C_1-C_8)$- Alkyl]$_2$, $SO_2$-R16, $SF_5$, CO-O[$(C_1-C_8)$-Alkyl], CO-O[$(C_3-C_8)$-Cycloalkyl], CO- O-$(CH_2)_n$-Aryl, CO-O-$(CH_2)_n$-Heteroaryl, CO-$NH_2$, CO-NH-CN, CO-NH-[$(C_1- C_8)$-Alkyl], CO-N[$(C_1-C_8)$-Alkyl]$_2$, CO-NH-[$(C_3-C_8)$-Cycloalkyl], C(=NH)-O- [$(C_1-C_6$-Alkyl)], C(=NH)-$NH_2$, C(=NH)-R16, C(=NR13)-NR12R13, $(CH_2)_n$- C(=$NSO_2$-R12)$NH_2$, CO-R16, COOH, CO-$(C_1-C_8)$-Alkyl, CO-$(C_3-C_8)$- Cycloalkyl, CO-Aryl, CO-Heteroaryl, CH(OH)-Aryl, CH(OH)-Heteroaryl, CH[O-$(C_1-C_6)$-Alkyl]-Aryl, CH[O-$(C_1-C_6)$-Alkyl]-Heteroaryl, CHF-Aryl, CHF- Heteroaryl, $CF_2$-Aryl, $CF_2$-Heteroaryl, CHO, $CH_2$-OH, $CH_2$-CN, $CH_2$-O-R12, $CH_2$-O-$(CH_2)_q$-COOH,

wobei die Alkyl-, Cycloalkyl-, Cycloalkenyl-, Bicycloalkyl- und Tricycloalkylreste mit Fluoratomen substituiert sein können und wobei die Aryl- oder Heteroarylreste mit Halogen, CN, $(C_1-C_6)$-Alkyl, $(C_3-C_6)$-Cycloalkyl, O-$(C_1C_6)$-Alkyl, $OCF_3$, OH, O-$(CH_2)_n$-Aryl, $(CH_2)_n$-Aryl, $S(O)_m$-$(C_1-C_6)$-Alkyl, $SO_2$-$NH_2$,SH,NR12R13,NH-CO-[$(C_1-C_6)$-Alkyl],NH-CO-$(CH_2)$,-Aryl, $(CH_2)_n$-COOH, $(CH_2)_n$-$CONH_2$, $(CH_2)_n$-CO-O$(C_1-C_6)$-Alkyl, $(CH_1)_n$-CO-$(C_1- C_6)$-Alkyl substituiert sein können und wobei die Alkylreste mit Fluoratomen substituiert sein können;

R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder 6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -$(C_1-C_6)$-Alkyl substituiert sein kann und wobei der bicyclische Heterocyclus 9 bis 12 Ringglieder enthalten kann und bis zu fünf CH- bzw. $CH_2$- Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit

R11, F, Cl, Br, J, CN, $N_3$, NC, $NO_2$, $CF_3$, $(CH_2)_n$-O-R1 1, $(CH_2)_n$-O- $(CH_2)_r$-OH, $(CH_2)_n$-O-CH$(CH_2OH)_2$, $(CH_2)_n$-O-$(CH_2)_n$-CO-O-$(CH_2)_r$ $NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)$,-N[$(CH_2)_q$-CO-O$(C_1-C_6)$-Alkyl]$_2$, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N$(R13)_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, $(CH_2)_n$-NR12-CO-N$(R12)_2$, $(CH_2)_n$-NR12-CO-NHR11, $(CH_2)_n$-NH- C(=NH)-$NH_2$, $(CH_2)_n$-NH-C(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$ -CO-NH- [$(C_1-C_8)$-Alkyl], $(CH_2)_n$-NH-$(CH_2)_n$ -CO-NH-$(CH_2)_r$-OH, $(CH_2)_n$-NH- $(CH_2)_n$ -CO-N[$(C_1-C_8)$-Alkyl]$_2$, $(CH_2)_n$-NH-$(CH_2)_n$ -CO-NH-[$(C_3-C_8)$- Cycloalkyl], $(CH_2)_n$-NH-$(CH_2)_n$ -CO-N[$(C_3-C_8)$-Cycloalkyl]$_2$, $(CH_2)_n$- NH-C$(CH_3)_2$-CO-O$(C_1-C_8)$-Alkyl, $(CH_2)_n$-NH-C$(CH_3)_2$-CO-O$(C_3-C_8)$-Cycloalkyl, $(CH_2)_n$-NH-C$(CH_3)_2$-CO-O-$(CH_2)_r$-$NH_2$, $(CH_2)_n$-NH- C$(CH_3)_2$-CO-O-$(CH_2)_n$-Aryl, $(CH_2)_n$-NH-C$(CH_3)_2$-CO-O-$(CH_2)_n$- Heteroaryl, $(CH_2)_n$-NH-C$(CH_3)_2$-CO-$NH_2$, $(CH_2)_n$-NH-C$(CH_3)_2$-CO-NH- [$(C_1-C_8)$-Alkyl], $(CH_2)_n$-NH-C$(CH_3)_2$-CO-NH-$(CH_2)_r$-OH, $(CH_2)_n$-NH- C$(CH_3)_2$-CO-N[$(C_1-C_8)$-Alkyl]$_2$, $(CH_2)_n$-NH-$(CH_3)_2$-CO-NH-[$(C_3-C_8)$- Cycloalkyl], $(CH_2)_n$-NH-C$(CH_3)_2$-CO-N[$(C_3-C_8)$-Cycloalkyl]$_2$, $(CH_2)_n$- NH-C$(CH_3)_2$-COOH, $S(O)_m$-R12, $SO_2$-R16, $SO_2$-N=CH-N$(CH_3)_2$,

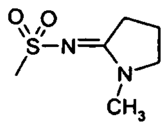,

$SO_2$-NH-CO-R12, $SO_2$-NHR12, $SO_2$-NH-$(CH_2)_r$-OH, $SO_2$- N[$(C_1-C_8)$-Alkyl]$_2$, $SO_2$-NH-$(CH_2)_r$-$NH_2$, $SF_5$, COOH, CO-$NH_2$, $(CH_2)_q$- CN, $(CH_2)_n$-CO-NH-CN, $(CH_2)_n$-CO-NH-piperidin-1-yl, $(CH_2)_n$-CO-NH- $SO_2$-NHR12, $(CH_2)_n$-CO-NH-$SO_2$-R18, $(CH_2)_n$-CHO, $(CH_2)_n$- C(=NH)$NH_2$, $(CH_2)_n$-C(=NH)-NHOH, $(CH_2)_n$-C(=NH)-[NH-O-$(C_1-C_6)$- Alkyl], $(CH_2)_n$-C(=NH)(R16), $(CH_2)_n$-C(=NR13)NHR12, $(CH_2)_n$- C(=NR12) NR12R13, $(CH_2)_n$-C(=$NSO_2$-R12)$NH_2$, $(CH_2)_n$- C(=NH)O[$(C_1-C_6)$-Alkyl], wobei die Alkyl- und Cycloalkylreste mit Fluoratomen substituiert sein können und wobei die Aryl- oder Heteroarylreste mit Halogen, CN,

$(C_1-C_6)$-Alkyl, $(C_3-C_6)$-Cycloalkyl, O- $(C_1-C_6)$-Alkyl, $S(O)_m-(C_1-C_6)$-Alkyl, $SO_2-NH_2$, COOH, $CONH_2$, CO-$O(C_1-C_6)$-Alkyl, CO-$(C_1-C_6)$-Alkyl substituiert sein können und wobei die Alkylreste mit Fluoratomen substituiert sein können, und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff verschiedenen Substituenten versehen ist;

H, F, Cl, Br, J, CN, $N_3$, NC, $NO_2$, $CF_3$,
$(C_1-C_8)$-Alkyl, $(C_2-C_{10})$-Alkenyl, $(C_2-C_{10})$-Alkinyl, $(C_3-C_8)$-Cycloalkyl, Aryl, Heteroaryl,
$(CH_2)_n$-CO-[O-$(C_1-C_8)$-Alkyl], $(CH_2)_n$-CO- [O-$(C_3-C_8)$-Cycloalkyl], $(CH_2)_n$-CO- [$(C_1-C_8)$-Alkyl], $(CH_2)_n$-CO-[$(C_3-C_8)$-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-$(C_1-C_6)$-Alkyl], $(CH_2)_n$-P(O)[O-$(C_1-C_6)$-Alkyl$]_2$, $(CH_2)_n$- P(O)(OH)(O-$CH_2$-Aryl), $(CH_2)_n$-P(O)(O-$CH_2$-Aryl$)_2$, $(CH_2)_n$-P(O)$(OH)_2$, $(CH_2)_n$-$SO_3H$, $(CH_2)_n$-$SO_2$-$NH_2$,
$(CH_2)_n$-CO-NH -[$(C_1-C_8)$-Alkyl], $(CH_2)_n$-CO-N[$(C_1-C_8)$-Alkyl$]_2$, $(CH_2)_n$-CO-NH- [$(C_3-C_8)$-Cycloalkyl],
$(C_2-C_{10})$-Alkenyl-CO-O[$(C_1-C_6)$-Alkyl], $(C_2-C_{10})$-Alkenyl-$CONH_2$, $(C_2-C_{10})$- Alkenyl-COOH, $(C_2-C_{10})$-Alkinyl-CO-O[$(C_1-C_6)$-Alkyl], $(C_2-C_{10})$-Alkinyl- $CONH_2$, $(C_2-C_{10})$-Alkinyl-COOH,
$(CH_2)_n$-CO-R16,
$(CH_2)_n$-OH, $(CH_2)_n$-O-$(C_1-C_8)$-Alkyl, $(CH_2)_n$-O-$(C_2-C_{10})$-Alkenyl, $(CH_2)_n$-O-$(C_2$- $C_{10})$-Alkinyl, $(CH_2)_n$-O-$(C_3-C_8)$-Cycloalkyl, $(CH_2)_n$-O-$(CH_2)_q$-[$(C_3-C_8)$- Cycloalkyl], $(CH_2)_n$-O-$(CH_2)_n$-CO-[O-$(C_1-C_8)$-Alkyl], $(CH_2)_n$-O-$(CH_2)_n$-CO-[O- $(C_3-C_8)$-Cycloalkyl], $(CH_2)_n$-O-$(CH_2)_n$-CO-[$(C_1-C_8)$-Alkyl], $(CH_2)_n$-O-$(CH_2)_n$- CO-[$(C_3-C_8)$-Cycloalkyl], $(CH_2)_n$-O-$(CH_2)_n$-CO-[O-$(CH_2)_v$-Aryl], $(CH_2)_n$-O- $(CH_2)_n$-CO-[O-$(CH_2)_v$-Heteroaryl], $(CH_2)_n$-O)-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O-$(CH_2)_n$-P(O)(OH)[O- $(C_1-C_6)$-Alkyl], $(CH_2)_n$-O-$(CH_2)_n$-P(O)[O-$(C_1-C_6)$-Alkyl$]_2$, $(CH_2)_n$-O-$(CH_2)_n$- P(O)(OH)(O-$CH_2$-Aryl), $(CH_2)_n$-O-$(CH_2)_n$-P(O)(O-$CH_2$-Aryl$)_2$, $(CH_2)_n$-O- $(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$- O-$(CH_2)_n$-CO-NH-[$(C_1-C_8)$-Alkyl], $(CH_2)_n$-O-$(CH_2)_n$-CO-N[$(C_1-C_8)$-Alkyl$]_2$, $(CH_2)_n$-O-$(CH_2)_n$-CO-NH-[$(C_3-C_8)$-Cycloalkyl], $(CH_2)_n$-O-$(CH_2)_n$-CR21R22- CO-O[$(C_1-C_6)$-Alkyl], $(CH_2)_n$-O-$(CH_2)_n$-CR21R22-$CONH_2$, $(CH_2)_n$-O-$(CH_2)_n$- CR21 R22-COOH, $(CH_2)_n$-O-$(CH_2)_n$-CO-R16, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH- $(CH_2)_r$-OH, O-R13, $OCF_3$,
$(CH_2)_n$-$NH_2$, $(CH_2)_n$-NH-$(C_1-C_8)$-Alkyl, $(CH_2)_n$-NH-$(C_3-C_8)$-Cycloalkyl, $(CH_2)_n$-NH-$(CH_2)_n$-CO-[O-$(C_3-C_8)$-Cycloalkyl], $(CH_2)_n$-NH-$(CH_2)_n$-CO-[$(C_1$- $C_8)$-Alkyl], $(CH_2)_n$-NH-$(CH_2)_n$-CO-[$(C_3-C_8)$-Cycloalkyl], $(CH_2)_n$-NH-$(CH_2)_n$- CO-[O-$(CH_2)_v$-Aryl], $(CH_2)_n$-NH-$(CH_2)_n$-CO-[O-$(CH_2)_v$-Heteroaryl], $(CH_2)_n$- NH-$(CH_2)_q$-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[$(C_1-C_6)$-Alkyl], $(CH_2)_n$-NH-$(CH_2)_n$- CR21R22-$CONH_2$, $(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[$(C_1-C_8)$-Alkyl], $(CH_2)_n$-NH- $(CH_2)_n$-$SO_2$-[$(C_3-C_8)$- Cycloalkyl], $(CH_2)_n$-NH-$SO_2$-$(CH_2)_n$-$NH_2$, $(CH_2)_n$-NH-$SO_2$-$(CH_2)_n$-NH-$(C_1-C_8)$- Alkyl, $(CH_2)_n$-NH-$SO_2$-$(CH_2)_n$-NH-$(C_3-C_8)$-Cycloalkyl, $(CH_2)_n$-NH-$SO_2$-$(CH_2)_n$- N[$(C_1-C_8)$-Alkyl$]_2$,
$(CH_2)_n$-NH-CN, $(CH_2)_n$-NH-$SO_2$-R16,
$(CH_2)_n$-NR12-CO-NH-$(C_1-C_8)$-Alkyl, $(CH_2)_n$-NR12-CO-NH-$(C_3-C_8)$- Cycloalkyl, $(CH_2)_n$-NR12-CO-$NH_2$, $(CH_2)_n$-NR12-CO-NH-$SO_2$-$(C_1-C_8)$-Alkyl, $(CH_2)_n$-NR12-CO-NH-$SO_2$-$(C_3-C_8)$-Cycloalkyl, $(CH_2)_n$-NR12-CO-N[$(C_1-C_8)$-Alkyl$]_2$, $(CH_2)$n-NH-CO-NH-$(CH_2)_n$-CO-[O-$(C_1-C_8)$-Alkyl], $(CH_2)_n$-NH-CO- NH-$(CH_2)_q$-CO-$NH_2$, $(CH_2)_n$-NH-CO-NH-$(CH_2)_q$-COOH, $(CH_2)_n$-NH-C(=NH)- $NH_2$, $(CH_2)_n$-NH-C(=NH)-R16, $(CH_2)_n$-NH-C(=NH)-NH[$(C_1-C_8)$-Alkyl], $(CH_2)_n$-NH-C(=N-$SO_2$-$(C_1-C_8)$-Alkyl)-$NH_2$, $(CH_2)_n$-NH-C(=N-$SO_2$-$(C_1-C_8)$- Alkyl)-NH[$(C_1-C_8)$-Alkyl], $(CH_2)_n$-NH-C(=N-$SO_2$-$NH_2$)-$NH_2$, $(CH_2)_n$-NH- C(=N-$SO_2$-$NH_2$)-NH[$(C_1-C_8)$-Alkyl], $(CH_2)_n$-NH-C(=NH)-N[(C1-$C_8)$-Alkyl$]_2$, $(CH_2)_n$-NH-C(=N-$SO_2$-$(C_1-C_8)$-Alkyl)-N[$(C_1-C_8)$-Alkyl$]_2$, $(CH_2)_n$-NH-$(CH_2)_n$- CO-O-$(CH_2)_r$-$NH_2$, $(CH_2)_n$-NH-$(CH_2)_n$ -CO-NH-[$(C_1-C_8)$-Alkyl], $(CH_2)_n$-NH- $(CH_2)_n$ -CO-NH-$(CH_2)_r$OH, $(CH_2)_n$-NH-$(CH_2)_n$ -CO-N[$(C_1-C_8)$-Alkyl$]_2$, $(CH_2)_n$- NH-$(CH_2)_n$ -CO-NH-[$(C_3-C_8)$-Cycloalkyl], $(CH_2)_n$-NH-C$(CH_3)_2$-CO-O$(C_3-C_8)$-Cycloalkyl, $(CH_2)_n$-NH-C$(CH_3)_2$-CO-O-$(CH_2)_n$-$NH_2$, $(CH_2)_n$-NH-C$(CH_3)_2$-CO- O-$(CH_2)_n$-Aryl, $(CH_2)_n$-NH-C$(CH_3)_2$-CO-O-$(CH_2)_n$-Heteroaryl, $(CH_2)_n$-NH- C$(CH_3)_2$-CO-NH-[$(C_1-C_8)$-Alkyl], $(CH_2)_n$-NH-C$(CH_3)_2$-CO-NH-$(CH_2)_r$-OH, $(CH_2)_n$-NH-C$(CH_3)_2$-CO-N[$(C_1-C_8)$-Alkyl$]_2$, $(CH_2)_n$-NH-$(CH_3)_2$-CO-NH-[$(C_3$- $C_8)$-Cycloalkyl], $(CH_2)_n$-NH-C$(CH_3)_2$-CO-N[$(C_3-C_8)$-Cycloalkyl$]_2$, $(CH_2)_n$-$S(O)_n$-$(C_1-C_8)$-Alkyl, $(CH_2)_n$-$S(O)_m$-$(C_3-C_8)$-Cycloalkyl, $(CH_2)_n$-$SO_2$- R16, $SO_2$-N=CH-N$(CH_3)_2$,

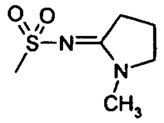

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)- Alkyl, $(CH_2)_n$-$SO_2$-NH-($C_3$-$C_8$)-Cycloalkyl, $(CH_2)_n$-$SO_2$-N[($C_1$-$C_8$)-Alkyl]$_2$, $SO_2$-NH- $(CH_2)_r$-OH, $SO_2$-NH-$(CH_2)_r$-$NH_2$, $SF_5$,
$(CH_2)_q$-CN,
$(CH_2)_n$-CO-NH-piperidin-1-yl,
$(CH_2)_n$-CO-NH-$SO_2$-NHR12, $(CH_2)_n$-CO-NH-$SO_2$-($C_1$-$C_8$)-Alkyl, $(CH_2)_n$-CO- NH-$SO_2$-($C_3$-$C_8$)-Cycloalkyl, $(CH_2)_n$-CHO, $(CH_2)_n$-C(=NH)$NH_2$, $(CH_2)_n$-C(=NH)NHOH, $(CH_2)_n$- C(=NH)(R16), $(CH_2)_n$-C(=NR13)NHR12, $(CH_2)_n$-C(=NR12)NR12R13, $(CH_2)_n$- C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, $S(O)_m$- ($C_1$-$C_6$)-Alkyl, $SO_2$-$NH_2$, COOH, $CONH_2$, CO-[O($C_1$-$C_6$)-Alkyl], CO-($C_1$-$C_6$)- Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und
wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, ($C_1$-$C_8$)-Alkyl oder =O substituiert sein können;

T NR23-CO-NR24, NR23-$SO_2$-NR24, $SO_2$-NR23-$SO_2$, CO-NR23-CO, NR23- C(=NR13)-NR24, NR23-C(=NR22)-NR24, CO-NR23-CR22R23, CO- CR22R23-CO, CR22R23-CO-CR22R24, NR23-CO-CR22R24, NR23-$SO_2$- CR22R24, CR22R24-CO-NR23, CR22R24-$SO_2$-NR23, CR22R23-NR23-$SO_2$, $SO_2$-CR22R23-NR23, $SO_2$-NR23-CR22R23-, NR23-CR22R23-$SO_2$, CO-NR23- $SO_2$, $SO_2$-NR23-CO, CO-CR22R23-$SO_2$, $SO_2$-CR22R23-CO, CR23R24- CR23R24-CR23R24, CR23R24-NR23-CR23R24;

R11 H, ($C_1$-$C_8$)-Alkyl, ($C_2$-$C_{10}$)-Alkenyl, ($C_2$-$C_{10}$)-Alkinyl, ($C_3$-$C_8$)-Cycloalkyl, $(CH_2)_q$-[($C_3$-$C_8$)-Cycloalkyl], $(CH_2)_n$-Aryl, $(CH_2)_n$-CO-[O-($C_1$-$C_8$)-Alkyl], $(CH_2)_n$CO-[O-($C_3$-$C_8$)-Cycloalkyl], $(CH_2)_n$-CO-[($C_1$-$C_8$)-Alkyl], $(CH_2)_n$-CO- [($C_3$-$C_8$)-Cycloalkyl], $(CH_2)_n$-CO-Aryl, $(CH_2)_n$-CO-Heteroaryl, $(CH_2)_q$-CO-$NH_2$, $(CH_2)_q$-COOH, $(CH_2)_n$-P(O)(OH)[O-($C_1$-$C_6$)-Alkyl], $(CH_2)_n$-P(O)[O-($C_1$-$C_6$)- Alkyl]$_2$, $(CH_2)_n$-P(O)(OH)(O-$CH_2$-Aryl), $(CH_2)_n$-P(O)(O-$CH_2$-Aryl)$_2$, $(CH_2)_n$- P(O)$(OH)_2$, $(CH_2)_n$-$SO_3H$, $(CH_2)_n$-$SO_2$-$NH_2$, $(CH_2)_n$-CO-NH-[($C_1$-$C_8$)-Alkyl], $(CH_2)_n$-CO-N[($C_1$-$C_8$)-Alkyl]$_2$, $(CH_2)_n$-CO-NH-[($C_3$-$C_8$)-Cycloalkyl], ($C_2$-$C_{10}$)- Alkenyl-CO-O[($C_1$-$C_6$)-Alkyl], ($C_2$-$C_{10}$)-Alkenyl-$CONH_2$, ($C_2$-$C_{10}$)-Alkenyl- COOH, ($C_2$-$C_{10}$)-Alkinyl-CO-O[($C_1$-$C_6$)-Alkyl], ($C_2$-$C_{10}$)-Alkinyl-$CONH_2$, ($C_2$- $C_{10}$)-Alkinyl-COOH, $(CH_2)_n$-CR21[(CO-O($C_1$-$C_6$)-Alkyl)]$_2$, $(CH_2)_n$- CR21$(CONH_2)_2$, $(CH_2)_n$-CR21$(COOH)_2$, $(CH_2)_n$-CR21 R22-CO-O[($C_1$-$C_6$)- Alkyl], $(CH_2)_n$-CR21R22-$CONH_2$, $(CH_2)_n$-CR21R22-CO-NH-[($C_1$-$C_8$)-Alkyl], $(CH_2)_n$-CR21R22-CO-N[($C_1$-$C_8$)-Alkyl]$_2$, $(CH_2)_n$-CR21R22-COOH, $(CH_2)_n$-CO-R16, $(CH_2)_n$-CO-NH-C(CH3)2-CO-O[($C_1$-$C_8$)-Alkyl], $(CH_2)_n$-CO- NH-C$(CH_3)_2$-$CONH_2$, $(CH_2)_n$-CO-NH-C$(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, ($C_1$-$C_6$)-Alkyl, ($C_3$- $C_6$)-Cycloalkyl, O-($C_1$-$C_6$)-Alkyl, $S(O)_m$-($C_1$-$C_6$)-Alkyl, $SO_2$-$NH_2$, COOH, $CONH_2$, CO-O($C_1$-$C_6$)-Alkyl, CO-($C_1$-$C_6$)-Alkyl substituiert sein kann und wobei die Alkylreste mit Fluoratomen substituiert sein können;

R12 H, ($C_1$-$C_8$)-Alkyl, ($C_3$-$C_8$)-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, ($C_1$-$C_6$)-Alkyl, O-($C_1$-$C_6$)-Alkyl, $SO_2$-$NH_2$, COOH, $CONH_2$, CO-O($C_1$-$C_6$)-Alkyl, CO-($C_1$-$C_6$)- Alkyl substituiert sein kann und wobei die Alkylreste mit Fluoratomen substituiert sein können;

R13 H, $SO_2$-[($C_1$-$C_8$)-Alkyl], $SO_2$-[($C_3$-$C_8$)-Cycloalkyl], $SO_2$-$(CH_2)_n$-Aryl, $SO_2$-$(CH_2)_n$-Heteroaryl,
wobei die Alkyl- und Cycloalkylreste mit Fluoratomen substituiert sein können und wobei der Aryl- oder Heteroarylrest mit Halogen, CN, $CF_3$, ($C_1$-$C_6$)-Alkyl, ($C_3$-$C_6$)-Cycloalkyl, O-[($C_1$-$C_6$)-Alkyl], $S(O)_m$-[($C_1$-$C_6$)-Alkyl], $SO_2$-$NH_2$, COOH, $CONH_2$, CO-[O($C_1$-$C_6$)-Alkyl], CO-($C_1$-$C_6$)-Alkyl substituiert sein kann und wobei die Alkylreste mit Fluoratomen substituiert sein können;

R15 ($C_1$-$C_8$)-Alkyl,
wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[($C_1$-$C_6$)- Alkyl]piperazin-1-yl, Thiomorpholin-4-yl, Thiomorpholin-1,1-Dioxid-4-yl,

NH- $(CH_2)_r$-OH, NH-CH$(CH_2OH)_2$, NH-C$(CH_2OH)_3$, N[$(C_1$-$C_6)$-Alkyl-OH]$_2$, D- Glucamin-N-yl, N-Methyl-D-Glucamin-N-yl, NH-[$(C_1$-$C_8)$-Alkyl]-CO-O($C_1$- $C_6$)-Alkyl, NH-[$(C_1$-$C_8)$-Alkyl]-COOH, NH-[$(C_1$-$C_8)$-Alkyl]-$CONH_2$, N[($C_1$- $C_6$)-Alkyl][$(C_1$-$C_8)$-Alkyl]-COOH, NH-[C(H)(Aryl)]-CO-O$(C_1$-$C_6)$-Alkyl, NH- [C(H)(Aryl)]-COOH, NH-[C(H)(Aryl)]-$CONH_2$, NH-[C(H)(Heteroaryl)]-CO- O$(C_1$-$C_6)$-Alkyl, NH-[C(H)(Heteroaryl)]-COOH, NH-[C(H)(Heteroaryl)]- $CONH_2$,NH-[$(C_3$-$C_8)$-Cycloalkyl]-CO-O$(C_1$-$C_6)$-Alkyl, NH-[$(C_3$-$C_8)$- Cycloalkyl]-COOH, NH-[$(C_3$-$C_8)$-Cycloalkyl]-$CONH_2$, NH-$(C_1$-$C_6)$-Alkyl-OH, NH-[$(C_1$-$C_6)$-Alkyl]-$SO_2$-$(C_1$-$C_6)$-Alkyl, NH-[$(C_1$-$C_6)$-Alkyl]-$SO_3H$, NH-[( $C_1$- $C_6$)-Alkyl]-$SO_2$-$NH_2$,
wobei die Alkohol (OH) - Funktionen durch F ersetzt sein können und wobei der Aryl- oder Heteroarylrest mit Halogen, CN, $(C_1$-$C_6)$-Alkyl, O-$(C_1$-$C_6)$-Alkyl, OH, $SO_2$-$NH_2$, COOH, $CONH_2$, CO-O$(C_1$-$C_6)$-Alkyl, CO-$(C_1$-$C_6)$-Alkyl substituiert sein kann;
R18 $(CH_2)_n$-CR25R26-CO-O$(C_1$-$C_4)$-Alkyl, $(CH_2)_n$-CR25R26-CO-$NH_2$, $(CH_2)_n$- CR25R26-COOH;
R20 H, $(C_1$-$C_6)$-Alkyl, $(C_3$-$C_8)$-Cycloalkyl, Aryl, [$(C_1$-$C_6)$-Alkyl]-Aryl;
R21 H, F, $CF_3$, $(C_1$-$C_6)$-Alkyl, $(C_3$-$C_8)$-Cycloalkyl, OH, O-$(C_1$-$C_6)$-Alkyl, O-$(C_3$-$C_8)$- Cycloalkyl, O-$(CH_2)_n$-Aryl, O-(CO)-$(C_1$-$C_6)$-Alkyl, O-(CO)-$(C_3$-$C_8)$-Cycloalkyl, O-(CO)-O-$(C_1$-$C_6)$-Alkyl, O-(CO)-O-$(C_3$-$C_8)$-Cycloalkyl, NH-[$(C_1$-$C_6)$-Alkyl]- Aryl, $NH_2$, NH-$(C_1C_6)$-Alkyl, NH-(CO)-$(C_1$-$C_6)$-Alkyl;
R22 H, $CF_3$, $(C_1$-$C_6)$-Alkyl, Aryl, [$(C_1$-$C_6)$-Alkyl]-Aryl;
R23, R24 unabhängig voneinander H, $(C_1$-$C_6)$-Alkyl, $(C_3$-$C_6)$-Cycloalkyl, [$(C_1$-$C_4)$-Alkyl]- [$(C_3$-$C_6)$-Cycloalkyl], Aryl, [$(C_1$-$C_4)$-Alkyl]-Aryl
oder R23 und R24 bilden zusammen eine -CH=CH-, -$CH_2$-$CH_2$-, -$CH_2$-$CH_2$- $CH_2$-,oder -$CH_2$-$CH_2$-$CH_2$-$CH_2$-Einheit, worin eine $CH_2$-Gruppierung durch C=O, CHF oder $CF_2$ ersetzt sein kann, und worin bis zu vier Wasserstoffatome durch einen $(C_1$-$C_4)$-Alkylrest ersetzt sein können;
R25, R26 unabhängig voneinander H, F, $(C_1$-$C_4)$-Alkyl, Aryl, [$(C_1$-$C_4)$-Alkyl]-Aryl, wobei der Aryl mit Halogen, CN, OH, O-$(C_1$-$C_4)$-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[$(C_1$-$C_4)$-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

**3.** Verbindungen der Formel I, gemäß Anspruch 1 oder 2, **dadurch gekennzeichnet, dass** darin bedeuten

R, R' unabhängig voneinander H, $(CH_2)_n$-Aryl, $(C_1$-$C_6)$-Alkyl, wobei $(C_1$-$C_6)$-Alkyl oder der Arylrest substituiert sein kann mit Halogen;
oder R und R' bilden gemeinsam einen Ring mit drei bis acht Kohlenstoffatomen, wobei ein Kohlenstoffatom durch O, $S(O)_m$, NR13 oder NR15 ersetzt sein kann;
m 0,1,2;
n 0,1,2;
p 1,2,3;
q 1, 2;
r 2, 3, 4;
v 0,1,2;
A, D, E, G, L unabhängig voneinander C oder N, wobei bei der Bedeutung N der entsprechende Substituent R1, R2, R3, R4, R5 entfällt, oder R2-D=E-R3 oder R4-G=L-R5 haben die Bedeutung S oder O und wobei der Fünf- oder Sechsring mit -$(CH_2)_3$- oder -CH=CH-CH=CH- oder zu einem Bicyclus anelliert sein kann;
R1, R2, R3, R4, unabhängig voneinander H, F, Cl, Br, J, CN, $CF_3$, $(C_1$-$C_8)$-Alkyl, ($C_3$- $C_8$)-Cycloalkyl, Adamantan-1-yl, Adamantan-2-yl, $(CH_2)_n$-Aryl, $(CH_2)_n$- Heteroaryl, $OCF_3$, O-R11, NR13R15, $S(O)_m$-R12, $SO_2$-$NH_2$ $SO_2$N=CH- $N(CH_3)_2$, $S0_2$-NH-[$(C_1$-$C_8)$-Alkyl], $SO_2$-NH-[$(C_3$-$C_8)$-Cycloalkyl], $SO_2$-NH- $(CH_2)_n$-Aryl, $SO_2$NH-$(CH_2)_n$-Heteroaryl, $SO_2$-N[$(C_1$-$C_8)$-Alkyl]$_2$, $SO_2$-R16, $SF_5$, CO-O[$(C_1$-$C_8)$-Alkyl],
R5 CO-O[$(C_3$-$C_6)$-Cycloalkyl], CO-$NH_2$, CO-NH-[$(C_1$-$C_4)$-Alkyl], CO-N[$(C_1$-$C_4)$- Alkyl]$_2$, CO-NH-[$(C_3$-$C_6)$-Cycloalkyl], C(=NH)-O-[(C,-$C_6$-Alkyl)], C(=NH)- $NH_2$, C(=NH)-R16, C(=NR13)-NR12R13, $(CH_2)_n$-C(=$NSO_2$-R12)$NH_2$, CO-R16, COOH, CO-$(C_1$-$C_4)$-Alkyl, CO-$(C_3$-$C_6)$-Cycloalkyl, CO-Aryl, CO-Heteroaryl, CH(OH)-Aryl, CH(OH)-Heteroaryl, CHF-Aryl, CHF-Heteroaryl, $CF_2$-Aryl, $CF_2$-Heteroaryl, $CH_2$-OH, $CH_2$-CN, $CH_2$-O-R12, $CH_2$-O-$(CH_2)_q$-COOH, wobei die Alkyl- und Cycloalkylreste mit Fluoratomen substituiert sein können und wobei die Aryl- oder Heteroarylreste mit Halogen, CN, $(C_1$-$C_4)$-Alkyl, ($C_3$- $C_6$)-Cycloalkyl, O-$(C_1$-$C_4)$-Alkyl, $OCF_3$, OH, O-$(CH_2)_n$-Aryl, $(CH_2)_n$-Aryl, $S(O)_m$-$(C_1$-$C_4)$-Alkyl, $SO_2$-$NH_2$, SH, NR12R13, NH-CO-[$(C_1C_4)$-Alkyl], NH-CO-$(CH_2)_n$-Aryl, $(CH_2)_n$-COOH, $(CH_2)_n$-$CONH_2$, $(CH_2)_n$-CO-O$(C_1$-$C_4)$-Alkyl, $(CH_2)_n$-CO-$(C_1$-$C_4)$-Alkyl substituiert sein können und wobei die Alkylreste mit Fluoratomen substituiert sein können;

R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder 6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -($C_1$-$C_6$)-Alkyl substituiert sein kann und wobei der bicyclische Heterocyclus 9 bis 12 Ringglieder enthalten kann und bis zu fünf CH- bzw. $CH_2$- Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit

R11, F, Cl, Br, J, CN, $N_3$, NC, $NO_2$, $CF_3$, $(CH_2)_n$-O-R11, $(CH_2)_n$-O- $(CH_2)_r$-OH, $(CH_2)_n$-O-$CH(CH_2OH)_2$, $(CH_2)_n$-O-$(CH_2)_n$-CO-O-$(CH_2)_r$ $NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O($C_1$-$C_6$)-Alkyl]$_2$, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N(R13)$_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, $(CH_2)_n$-NR12-CO-N(R12)$_2$, $(CH_2)_n$-NR12-CO-NHR11, $(CH_2)_n$-NH- C(=NH)-$NH_2$, $(CH_2)_n$-NH-C(=NH)-R16, $(CH_2)_n$-NH- C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$ -CO-NH- [($C_1$-$C_8$)-Alkyl], $(CH_2)_n$-NH-$(CH_2)_n$ -CO-NH-$(CH_2)_r$-OH, $(CH_2)_n$-NH- $(CH_2)_n$ -CO-N[($C_1$-$C_8$)-Alkyl]$_2$, $(CH_2)_n$-NH-$(CH_2)_n$ -CO-NH-[($C_3$-$C_8$)- Cycloalkyl], $(CH_2)_n$-NH-$(CH_2)_n$ -CO-N[($C_3$-$C_8$)-Cycloalkyl]$_2$, $(CH_2)_n$- NH-$C(CH_3)_2$-CO-O($C_1$-$C_8$)-Alkyl, $(CH_2)_n$-NH-$C(CH_3)_2$-CO-O($C_3$-$C_8$)-Cycloalkyl, $(CH_2)_n$-NH-$C(CH_3)_2$-CO-O-$(CH_2)_r$-$NH_2$, $(CH_2)_n$-NH- $C(CH_3)_2$-CO-O-$(CH_2)_n$-Aryl, $(CH_2)_n$-NH-$C(CH_3)_2$-CO-O-$(CH_2)_n$- Heteroaryl, $(CH_2)_n$-NH-$C(CH_3)_2$-CO-$NH_2$, $(CH_2)_n$-NH-$C(CH_3)_2$-CO-NH-[($C_1$-$C_8$)-Alkyl], $(CH_2)_n$-NH-$C(CH_3)_2$-CO-NH-$(CH_2)_r$-OH, $(CH_2)_n$-NH- $C(CH_3)_2$-CO-N[($C_1$-$C_8$)-Alkyl]$_2$, $(CH_2)_n$-NH-$(CH_3)_2$-CO-NH-[($C_3$-$C_8$)- Cycloalkyl], $(CH_2)_n$-NH-$C(CH_3)_2$-CO-N[($C_3$-$C_8$)-Cycloalkyl]$_2$, $(CH_2)_n$-NH-$C(CH_3)_2$-COOH, $S(O)_m$-R12, $SO_2$-R16, $SO_2$-N=CH-$N(CH_3)_2$,

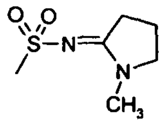,

$SO_2$-NH-CO-R12, $SO_2$-NHR12, $SO_2$-NH-$(CH_2)_r$-OH, $SO_2$- N[($C_1$-$C_8$)-Alkyl]$_2$, $SO_2$-NH-$(CH_2)_r$-$NH_2$, $SF_5$, COOH, CO-$NH_2$, $(CH_2)_q$- CN, $(CH_2)_n$-CO-NH-CN, $(CH_2)_n$-CO-NH-piperidin-1-yl, $(CH_2)_n$-CO-NH- $SO_2$-NHR12, $(CH_2)_n$-CO-NH-$SO_2$-R18, $(CH_2)_n$-CHO, $(CH_2)_n$- C(=NH)$NH_2$, $(CH_2)_n$-C(=NH)-NHOH, $(CH_2)_n$-C(=NH)-[NH-O-($C_1$-$C_6$)- Alkyl], $(CH_2)_n$-C(=NH)(R16), $(CH_2)_n$-C(=NR13)NHR12, $(CH_2)_n$- C(=NR12)NR12 R13, $(CH_2)_n$-C(=$NSO_2$R12)$NH_2$, (CH2)n- C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O- ($C_1$-$C_6$)-Alkyl, $S(O)_m$-($C_1$-$C_6$)-Alkyl, $SO_2$-$NH_2$, COOH, $CONH_2$, CO- O($C_1$-$C_6$)-Alkyl, CO-($C_1$-$C_6$)-Alkyl substituiert sein können und wobei die Alkylreste mit Fluoratomen substituiert sein können, und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff verschiedenen Substituenten versehen ist;

H, F, Cl, Br, J, CN, $N_3$, NC, $NO_2$, $CF_3$,
($C_1$-$C_8$)-Alkyl, ($C_2$-$C_{10}$)-Alkenyl, ($C_2$-$C_{10}$)-Alkinyl, ($C_3$-$C_8$)-Cycloalkyl, Aryl, Heteroaryl,
$(CH_2)_n$-CO-[O-($C_1$-$C_8$)-Alkyl], $(CH_2)_n$-CO-[O-($C_3$-$C_8$)-Cycloalkyl], $(CH_2)_n$-CO- [($C_1$-$C_8$)-Alkyl], $(CH_2)_n$-CO-[($C_3$-$C_8$)-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-($C_1$-$C_6$)-Alkyl], $(CH_2)_n$-P(O)[O-($C_1$-$C_6$)-Alkyl]$_2$, $(CH_2)_n$- P(O)(OH)(O-$CH_2$-Aryl), $(CH_2)_n$-P(O)(O-$CH_2$-Aryl)$_2$, $(CH_2)_n$-P(O)$(OH)_2$, $(CH_2)_n$-$SO_3H$, $(CH_2)_n$-$SO_2$-$NH_2$,
$(CH_2)_n$-CO-NH-[($C_1$-$C_8$)-Alkyl], $(CH_2)_n$-CO-N[($C_1$-$C_8$)-Alkyl]$_2$, $(CH_2)_n$-CO-NH- [($C_3$-$C_8$)-Cycloalkyl],
($C_2$-$C_{10}$)-Alkenyl-CO-O[($C_1$-$C_6$)-Alkyl], ($C_2$-$C_{10}$)-Alkenyl-$CONH_2$, ($C_2$-$C_{10}$)- Alkenyl-COOH, ($C_2$-$C_{10}$-Alkinyl-CO-O[($C_1$-$C_6$)-Alkyl], ($C_2$-$C_{10}$)-Alkinyl- $CONH_2$, ($C_2$-$C_{10}$)-Alkinyl-COOH,
$(CH_2)_n$-CO-R16,
$(CH_2)_n$-OH, $(CH_2)_n$-O-($C_1$-$C_8$)-Alkyl, $(CH_2)_n$-O-($C_2$-$C_{10}$)-Alkenyl, $(CH_2)_n$-O-($C_2$- $C_{10}$)-Alkinyl, $(CH_2)_n$-O-($C_3$-$C_8$)-Cycloalkyl, $(CH_2)_n$-O-$(CH_2)_q$-[($C_3$-$C_8$)- Cycloalkyl], $(CH_2)_n$-O-$(CH_2)_n$-CO-[O-($C_1$-$C_8$)-Alkyl], $(CH_2)_n$-O-$(CH_2)_n$-CO-[O- ($C_3$-$C_8$)-Cycloalkyl], $(CH_2)_n$-O-$(CH_2)_n$-CO-[($C_1$-$C_8$)-Alkyl], $(CH_2)_n$-O-$(CH_2)_n$- CO-[($C_3$-$C_8$)-Cycloalkyl],

$(CH_2)_n$-O-$(CH_2)_n$-CO-[O-$(CH_2)_v$-Aryl], $(CH_2)_n$-O- $(CH_2)_n$-CO-[O-$(CH_2)_v$-Heteroaryl], $(CH_2)_n$-O-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O-$(CH_2)_n$-P(O)(OH)[O- $(C_1$-$C_6)$-Alkyl], $(CH_2)_n$-O-$(CH_2)_n$-P(O)[O-$(C_1$-$C_6)$-Alkyl]$_2$, $(CH_2)_n$-O-$(CH_2)_n$- P(O)(OH)(O-$CH_2$-Aryl), $(CH_2)_n$-O-$(CH_2)_n$-P(O)(O-$CH_2$-Aryl)$_2$, $(CH_2)_n$-O- $(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$- O-$(CH_2)_n$-CO-NH-[$(C_1$-$C_8)$-Alkyl], $(CH_2)_n$-O-$(CH_2)_n$-CO-N[$(C_1$-$C_8)$-Alkyl]$_2$, $(CH_2)_n$-O-$(CH_2)_n$-CO-NH-[$(C_3$-$C_8)$-Cycloalkyl], $(CH_2)_n$-O-$(CH_2)_n$-CR21R22- CO-O[$(C_1$-$C_6)$-Alkyl], $(CH_2)_n$-O-$(CH_2)_n$-CR21R22-$CONH_2$, $(CH_2)_n$-O-$(CH_2)_n$-CR21R22-COOH, $(CH_2)_n$-O-$(CH_2)_n$-CO-R16, $(CH_2)_n$-O-$(CH_2)_r$-OH, $(CH_2)_n$-O- $CH(CH_2OH)_2$, $(CH_2)_n$-O-$(CH_2)_n$-CO-O-$(CH_2)_r$-$NH_2$, $(CH_2)_n$-O-$(CH_2)_n$-CO-NH- $(CH_2)_r$-OH, O-R13, $OCF_3$,

$(CH_2)_n$-$NH_2$, $(CH_2)_n$-NH-$(C_1$-$C_8)$-Alkyl, $(CH_2)_n$-NH-$(C_3$-$C_8)$-Cycloalkyl, $(CH_2)_n$-NH-$(CH_2)_n$-CO-[O-$(C_3$-$C_8)$-Cycloalkyl], $(CH_2)_n$-NH-$(CH_2)_n$-CO-[$(C_1$- $C_8)$-Alkyl], $(CH_2)_n$-NH-$(CH_2)_n$-CO-[$(C_3$-$C_8)$-Cycloalkyl], $(CH_2)_n$-NH-$(CH_2)_n$- CO-[O-$(CH_2)_v$-Aryl], $(CH_2)_n$-NH-$(CH_2)_n$-CO-[O-$(CH_2)_v$-Heteroaryl], $(CH_2)_n$- NH-$(CH_2)_q$-CO-NH-CN,

$(CH_2)_n$-NH-$(CH_2)_n$-P(O)$(OH)_2$,

$(CH_2)_n$-NH-$(CH_2)_n$-$SO_3H$,

$(CH_2)_n$-NH-$(CH_2)_n$-$SO_2$-$NH_2$,

$(CH_2)_n$-NH-$(CH_2)_n$-CR21 R22-CO-O[$(C_1$-$C_6)$-Alkyl], $(CH_2)_n$-NH-$(CH_2)_n$- CR21R22-$CONH_2$, $(CH_2)_n$-NH-$(CH_2)_n$-CR21 R22-COOH,

$(CH_2)_n$-NH-$(CH_2)_n$-CO-R16,

$(CH_2)_n$-NH-$(CH_2)_n$-$SO_r$[$(C_1$-$C_8)$-Alkyl], $(CH_2)_n$-NH - $(CH_2)_n$-$SO_2$-[$(C_3$-$C_8)$- Cycloalkyl], $(CH_2)_n$-NH-$SO_2$-$(CH_2)_n$-$NH_2$, $(CH_2)_n$-NH-$SO_2$-$(CH_2)_n$-NH-$(C_1$-$C_8)$- Alkyl, $(CH_2)_n$-NH-$SO_2$-$(CH_2)_n$-NH-$(C_3$-$C_8)$-Cycloalkyl, $(CH_2)_n$-NH-$SO_2$-$CH_2)_n$- N[$(C_1$-$C_8)$-Alkyl]$_2$,

$(CH_2)_n$-NH-CN, $(CH_2)_n$-NH-$SO_2$-R16,

$(CH_2)_n$-NR12-CO-NH-$(C_1$-$C_8)$-Alkyl, $(CH_2)_n$-NR12-CO-NH-$(C_3$-$C_8)$- Cycloalkyl, $(CH_2)_n$-NR12-CO-$NH_2$, $(CH_2)_n$-NR12-CO-NH-$SO_2$-$(C_1$-$C_8)$-Alkyl, $(CH_2)_n$-NR12-CO-NH-$SO_2$-$(C_3$-$C_8)$-Cycloalkyl, $(CH_2)_n$-NR12-CO-N[$(C_1$-$C_8)$-Alkyl]$_2$, $(CH_2)$n-NH-CO-NH-$(CH_2)_n$-CO-[O-$(C_1$-$C_8)$-Alkyl], $(CH_2)$n-NH-CO- NH-$(CH_2)_q$-CO-$NH_2$, $(CH_2)_n$-NH-CO-NH-$(CH_2)_q$-COOH, $(CH_2)_n$-NH-C(=NH)- $NH_2$, $(CH_2)_n$-NH-C(=NH)-R16, $(CH_2)_n$-NH-C(=NH)-NH[$(C_1$-$C_8)$-Alkyl], $(CH_2)_n$-NH-C(=N-$SO_2$-$(C_1$-$C_8)$-Alkyl)-$NH_2$, $(CH_2)_n$-NH-C(=N-$SO_2$-$(C_1$-$C_8)$- Alkyl)-NH[$(C_1$-$C_8)$-Alkyl], $(CH_2)_n$-NH-C(=N-$SO_2$-$NH_2$)-$NH_2$, $(CH_2)_n$-NH- C(=N-$SO_2$-$NH_2$)-NH[$(C_1$-$C_8)$-Alkyl], $(CH_2)_n$-NH-C(=NH)-N[$(C_1$-$C_8)$-Alkyl]$_2$, $(CH_2)_n$-NH-C(=N-$SO_2$-$(C_1$-$C_8)$-Alkyl)-N[$(C_1$-$C_8)$-Alkyl]$_2$, $(CH_2)_n$-NH-$(CH_2)_n$- CO-O-$(CH_2)_r$-$NH_2$, $(CH_2)_n$-NH-$(CH_2)_n$ -CO-NH-[$(C_1$-$C_8)$-Alkyl], $(CH_2)_n$-NH- $(CH_2)_n$ -CO-NH-$(CH_2)_r$-OH, $(CH_2)_n$-NH-$(CH_2)_n$ -CO-N[$(C_1$-$C_8)$-Alkyl]$_2$, $(CH_2)_n$- NH-$(CH_2)_n$ -CO-NH-[$(C_3$-$C_8)$-Cycloalkyl], $(CH_2)_n$-NH-$C(CH_3)_2$-CO-O$(C_3$-$C_8)$-Cycloalkyl, $(CH_2)_n$-NH-$C(CH_3)_2$-CO-O-$(CH_2)_r$-$NH_2$, $(CH_2)_n$-NH-$C(CH_3)_2$-CO- O-$(CH_2)_n$-Aryl, $(CH_2)_n$-NH-$C(CH_3)_2$-CO-O-$(CH_2)_n$-Heteroaryl, $(CH_2)_n$-NH- $C(CH_3)_2$-CO-NH-[$(C_1$-$C_8)$-Alkyl], $(CH_2)_n$-NH-$C(CH_3)_2$-CO-NH-$(CH_2)_r$-OH, $(CH_2)_n$-NH-$C(CH_3)_2$-CO-N[$(C_1$-$C_8)$-Alkyl]$_2$, $(CH_2)_n$-NH-$(CH_3)_2$-CO-NH-[$(C_3$- $C_8)$-Cycloalkyl], $(CH_2)_n$-NH-$C(CH_3)_2$-CO-N[$(C_3$-$C_8)$-Cycloalkyl]$_2$, $(CH_2)_n$-S$(O)_m$-$(C_1$-$C_8)$-Alkyl, $(CH_2)_n$-S(O )$_m$-$(C_3$-$C_8)$- Cycloalkyl, $(CH_2)_n$-$SO_2$- R16, $SO_2$-N=CH-N$(CH_3)_2$,

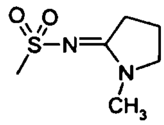

,

$(CH_2)_n$-$SO_2$-NH-CO-$(C_1$-$C_8)$-Alkyl, $(CH_2)_n$-$SO_2$-NH-CO-$(C_3$-$C_8)$-Cycloalkyl, $(CH_2)_n$-$SO_2$-NH-$(C_1$-$C_8)$-Alkyl, $(CH_2)_n$-$SO_2$-NH-$(C_3$-$C_8)$-Cycloalkyl, $(CH_2)_n$-$SO_2$-N[$(C_1$-$C_8)$-Alkyl]$_2$, $SO_2$-NH- $(CH_2)_r$-OH, $SO_2$-NH-$(CH_2)_r$-$NH_2$, $SF_5$. $(CH_2)_q$-CN,

$(CH_2)_n$-CO-NH-piperidin-1-yl,

$(CH_2)_n$-CO-NH-$SO_2$-NHR12, $(CH_2)_n$-CO-NH-$SO_2$-$(C_1$-$C_8)$-Alkyl, $(CH_2)_n$-CO- NH-$SO_2$-$(C_3$-$C_8)$-Cycloalkyl,

$(CH_2)_n$-CHO, $(CH_2)_n$-C(=NH)$NH_2$, $(CH_2)_n$-C(=NH)NHOH, $(CH_2)_n$- C(=NH)(R16), $(CH_2)_n$-C(=NR13)NHR12, $(CH_2)_n$-C(=NR12)NR12R13, $(CH_2)_n$- C(=NH)O[$(C_1$-$C_6)$-Alkyl], wobei die Alkyl- und Cycloalkylreste mit Fluoratomen substituiert sein können und wobei die Aryl- oder Heteroarylreste mit Halogen, CN, $(C_1$-$C_6)$-Alkyl, $(C_3$-$C_6)$-Cycloalkyl, O-$(C_1$-$C_6)$-Alkyl, S$(O)_m$- $(C_1$-$C_6)$-Alkyl, $SO_2$-$NH_2$, COOH, $CONH_2$, CO-[O$(C_1$-$C_6)$-Alkyl], CO-$(C_1$-$C_6)$- Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und

wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, $(C_1$-$C_8)$-Alkyl oder

=O substituiert sein können;

T NR23-CO-NR24, NR23-$SO_2$-NR24, $SO_2$-NR23-$SO_2$, CO-NR23-CO, NR23- C(=NR13)-NR24, NR23-C(=NR22)-NR24, CO-NR23-CR22R23, NR23-CO- CR22R24, NR23-$SO_2$-CR22R24, CR22R24-CO-NR23, CR22R24-$SO_2$-NR23, CR22R23-NR23-$SO_2$, $SO_2$-CR22R23-NR23, $SO_2$-NR23-CR22R23-, NR23- CR22R23-$SO_2$, CR23R24-CR23R24-CR23R24, CR23R24-NR23-CR23R24;

R11 H, ($C_1$-$C_8$)-Alkyl, ($C_2$-$C_{10}$)-Alkinyl, ($C_3$-$C_6$)-Cycloalkyl, $(CH_2)_n$-Aryl, $(CH_2)_n$- CO-[O-($C_1$-$C_6$)-Alkyl], $(CH_2)_n$-CO-[O-($C_3$-$C_6$)-Cycloalkyl], $(CH_2)_n$-CO-[($C_1$- $C_6$)-Alkyl], $(CH_2)_n$-CO-[($C_3$-$C_6$)-Cycloalkyl], $(CH_2)_n$-CO-Aryl, $(CH_2)_n$-CO- Heteroaryl, $(CH_2)_q$-CO-$NH_2$, $(CH_2)_q$-COOH, $(CH_2)_n$-P(O)(OH)[O-($C_1$-$C_6$)- Alkyl], $(CH_2)_n$-P(O)[O-($C_1$-$C_4$)-Alkyl]$_2$, $(CH_2)_n$-P(O)(O-$CH_2$-Aryl)$_2$, $(CH_2)_n$- P(O)(OH)$_2$, $(CH_2)_n$-$SO_3H$, $(CH_2)_n$-$SO_2$-NH2, $(CH_2)_n$-CO-NH-[($C_1C_4$)-Alkyl], $(CH_2)_n$-CO-N[($C_1$-$C_4$)-Alkyl]$_2$, $(CH_2)_n$-CO-NH-[($C_3$-$C_6$)-Cycloalkyl], ($C_2$-$C_6$)- Alkenyl-CO-O[($C_1$-$C_4$)-Alkyl], ($C_2$-$C_6$)-Alkenyl-$CONH_2$, ($C_2$-$C_6$)-Alkenyl- COOH, ($C_2$-$C_6$)-Alkinyl-CO-O[($C_1$-$C_4$)-Alkyl], ($C_2$-$C_6$)-Alkinyl-$CONH_2$, ($C_2$- $C_6$)-Alkinyl-COOH, $(CH_2)_n$-CR21 [(CO-O($C_1$-$C_4$)-Alkyl)]$_2$, $(CH_2)_n$- CR21($CONH_2$)$_2$, $(CH_2)_n$-CR21(COOH)$_2$, $(CH_2)_n$-CR21R22-CO-O[($C_1$-$C_4$)- Alkyl], $(CH_2)_n$-CR21R22-$CONH_2$, $(CH_2)_n$-CR21R22-CO-NH-[($C_1$-$C_4$)-Alkyl], $(CH_2)_n$-CR21R22-CO-N[($C_1$-$C_4$)-Alkyl]$_2$, $(CH_2)_n$-CR21R22-COOH, (CH2)$_n$-CO-R16, $(CH_2)_n$-CO-NH-C$(CH_3)_2$-CO-O[($C_1$-$C_8$)-Atkyl], $(CH_2)_n$-CO- NH-C$(CH_3)_2$-$CONH_2$, $(CH_2)_n$-CO-NH-C$(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, ($C_1$-$C_4$)-Alkyl, O- ($C_1$-$C_4$)-Alkyl, $S(O)_m$-($C_1$-$C_4$)-Alkyl, $SO_2$-$NH_2$, COOH, $CONH_2$, CO-O($C_1$-$C_4$)- Alkyl substituiert sein kann und wobei die Alkylreste mit Fluoratomen substituiert sein können;

R12 H, ($C_1$-$C_8$)-Alkyl, ($C_3$-$C_8$)-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, ($C_1$-$C_4$)-Alkyl, O-($C_1$-$C_4$)-Alkyl, $SO_2$-$NH_2$, COOH, $CONH_2$, CO-O($C_1$-$C_4$)-Alkyl substituiert sein kann und wobei die Alkylreste mit Fluoratomen substituiert sein können;

R13 H, $SO_2$-[($C_1$-$C_8$)-Alkyl], $SO_2$-[($C_3$-$C_8$)-Cycloalkyl], $SO_2$-$(CH_2)_n$-Aryl, $SO_2$-$(CH_2)_n$-Heteroaryl,
wobei die Alkyl- und Cycloalkylreste mit Fluoratomen substituiert sein können und wobei der Aryl- oder Heteroarylrest mit Halogen, CN, $CF_3$, ($C_1$-$C_4$)-Alkyl, O-[($C_1$-$C_4$)-Alkyl], $S(O)_m$-[($C_1$-$C_6$)-Alkyl], $SO_2$-$NH_2$, COOH, $CONH_2$, CO- [O($C_1$-$C_4$)-Alkyl] substituiert sein kann und wobei die Alkylreste mit Fluoratomen substituiert sein können;

R15 ($C_1$-$C_6$)-Alkyl,
wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[($C_1$-$C_6$)- Alkyl]piperazin-1-yl, Thiomorpholin-1,1-Dioxid-4-yl, NH-$(CH_2)_r$-OH, NH-CH$(CH_2OH)_2$, NH-C$(CH_2OH)_3$, N[($C_1$-$C_4$)-Alkyl-OH]$_2$, D-Glucamin-N-yl, N- Methyl-D-Glucamin-N-yl, NH-[($C_1$-$C_4$)-Alkyl]-COOH, NH-[($C_1$-$C_4$)-Alkyl]- $CONH_2$, N[($C_1$-$C_4$)-Alkyl][($C_1$-$C_4$)-Alkyl]-COOH, NH-[C(H)(Aryl)]-CO-O($C_1$- $C_4$)-Alkyl, NH-[C(H)(Aryl)]-COOH, NH-[C(H)(Aryl)]-$CONH_2$, NH- [C(H)(Heteroaryl)]-CO-O($C_1$-$C_4$)-Alkyl, NH-[C(H)(Heteroaryl)]-COOH, NH- [C(H)(Heteroaryl)]-$CONH_2$, NH-[($C_3$-$C_6$)-Cyloalkyl]-CO-O($C_1$-$C_4$)-Alkyl, NH- [($C_3$-$C_6$)-Cycloalkyl]-COOH, NH-[($C_3$-$C_6$)-Cycloalkyl]-$CONH_2$, NH-($C_1$-$C_4$)- Alkyl-OH, NH-[($C_1$-$C_4$)-Alkyl]-$SO_2$-($C_1$- $C_4$)-Alkyl, NH-[($C_1$-$C_4$)-Alkyl]- $SO_3H$, NH-[($C_1$-$C_4$)-Alkyl]-$SO_2$-$NH_2$,
wobei die Alkohol (OH) - Funktionen durch F ersetzt sein können und wobei der Aryl- oder Heteroarylrest mit Halogen, CN, ($C_1$-$C_4$)-Alkyl, O-($C_1$-$C_4$)-Alkyl, OH, $SO_2$-$NH_2$, COOH, $CONH_2$, CO-O($C_1$-$C_4$)-Alkyl substituiert sein kann;

R18 $(CH_2)$,-CR25R26-CO-O($C_1$-$C_4$)-Alkyl, $(CH_2)_n$-CR25R26-CO-$NH_2$, $(CH_2)_n$- CR25R26-COOH;

R20 H, ($C_1$-$C_4$)-Alkyl, ($C_3$-$C_6$)-Cycloalkyl, Aryl, [($C_1$-$C_4$)-Alkyl]-Aryl;

R21 H, F, $CF_3$, ($C_1$-$C_4$)-Alkyl, ($C_3$-$C_6$)-Cycloalkyl, OH, O-($C_1$-$C_4$)-Alkyl, O-($C_3$-$C_6$)- Cycloalkyl, O-$(CH_2)_n$-Aryl, O-(CO)-($C_1$-$C_4$)-Alkyl, O-(CO)-($C_3$-$C_6$)-Cycloalkyl, O-(CO)-O-($C_1$-$C_4$)-Alkyl, O-(CO)-O-($C_3$-$C_6$)-Cycloalkyl, NH-[($C_1$-$C_4$)-Alkyl]- Aryl, $NH_2$, NH-($C_1$-$C_4$)-Alkyl, NH-(CO)-($C_1$-$C_4$)-Alkyl;

R22 H, $CF_3$, ($C_1$-$C_4$)-Alkyl, Aryl, [($C_1$-$C_4$)-Alkyl]-Aryl;

R23, R24 unabhängig voneinander H, ($C_1$-$C_4$)-Alkyl, ($C_3$-$C_6$)-Cycloalkyl, [($C_1$-$C_4$)-Alkyl]- [($C_3$-$C_6$)-Cycloalkyl], Aryl, [($C_1$-$C_4$)-Alkyl]-Aryl
oder R23 und R24 bilden zusammen eine -CH=CH-, -$CH_2$-$CH_2$-, -$CH_2$-$CH_2$- $CH_2$-, oder -$CH_2$-$CH_2$-$CH_2$-$CH_2$-Einheit, worin eine $CH_2$-Gruppierung durch C=O, CHF oder $CF_2$ ersetzt sein kann, und worin bis zu vier Wasserstoffatome durch einen ($C_1$-$C_4$)-Alkylrest ersetzt sein können;

R25, R26 unabhängig voneinander H, F, ($C_1$-$C_4$)-Alkyl, Aryl, [($C_1$-$C_4$)-Alkyl]-Aryl, wobei der Aryl mit Halogen, CN, OH, O-($C_1$-$C_4$)-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[($C_1$-$C_4$)-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

**4.** Verbindungen der Formel I, gemäß einem oder mehreren der Ansprüche 1 bis 3, **dadurch gekennzeichnet, dass** darin bedeuten

R, R' unabhängig voneinander H, Aryl, $(C_1-C_4)$-Alkyl, wobei $(C_1-C_4)$-Alkyl oder der Arylrest substituiert sein kann mit Halogen;
oder R und R' bilden gemeinsam einen Ring mit drei bis acht Kohlenstoffatomen, wobei ein Kohlenstoffatom durch O, $S(O)_m$, NR13 oder NR15 ersetzt sein kann;
m 0, 1, 2;
n 0, 1, 2;
p 1, 2, 3;
q 1, 2;
r 2, 3;
v 0, 1, 2;
A, D, E, G, L unabhängig voneinander C oder N, wobei bei der Bedeutung N der entsprechende Substituent R1, R2, R3, R4, R5 entfällt, oder R2-D=E-R3 oder R4-G=L-R5 haben die Bedeutung S oder O und wobei der Fünf- oder Sechsring mit $-(CH_2)_3-$ oder $-(CH_2)_4-$ oder -CH=CH-CH=CH- zu einem Bicyclus anelliert sein kann;
R1, R2, R3, R4, R5 unabhängig voneinander H, F, Cl, Br, J, CN, $CF_3$, $(C_1-C_8)$-Alkyl, $(C_3-C_8)$-Cycloalkyl, $(CH_2)_n$-Aryl, $(CH_2)_n$-Heteroaryl, $OCF_3$, O-R11, NR13R15, $S(O)_{rm}$-R12, $SO_2-NH_2$, $SO_2$-NH-[$(C_1-C_8)$-Alkyl], $SO_2$-NH-[$(C_3-C_8)$-Cycloalkyl], $SO_2$-NH-$(CH_2)_n$-Aryl, $SO_2$-NH-$(CH_2)_n$-Heteroaryl, $SO_2$-N[$(C_1-C_8)$-Alkyl]$_2$, $SO_2$-R16, $SF_5$, CO-O[$(C_1-C_8)$-Alkyl],
CO-O[$(C_3-C_6)$-Cycloalkyl], CO-$NH_2$, CO-NH-[$(C_1-C_4)$-Alkyl], CO-N[$(C_1-C_4)$- Alkyl]$_2$, C(=NH)-$NH_2$, C(=NH)-R16, $(CH_2)_n$-C(=NSO2-R12)$NH_2$, CO-R16, COOH, CO-$(C_1-C_4)$-Alkyl, CO-$(C_3-C_6)$-Cycloalkyl, CO-Aryl, CO-Heteroaryl, CH(OH)-Aryl, CH(OH)-Heteroaryl, CHF-Aryl, CHF-Heteroaryl, $CF_2$-Aryl, $CF_2$-Heteroaryl, $CH_2$-OH, $CH_2$-CN, $CH_2$-O-R12, $CH_2$-O-$(CH_2)_q$-COOH, wobei die Alkyl- und Cycloalkylreste mit Fluoratomen substituiert sein können und wobei die Aryl- oder Heteroarylreste mit Halogen, CN, $(C_1$-C4)-Alkyl, O- $(C_1-C_4)$-Alkyl, $OCF_3$, OH, O-$(CH_2)_n$-Aryl, $(CH_2)_n$-Aryl, $S(O)_m$-$(C_1-C_4)$-Alkyl, $SO_2-NH_2$, SH, NR12R13, NH-CO-[$(C_1-C_4)$-Alkyl], NH-CO-$(CH_2)_n$-Aryl, $(CH_2)_n$-COOH, $(CH_2)_n$-$CONH_2$, $(CH_2)_n$-CO-O$(C_1$-C4)-Alkyl, $(CH_2)_n$-CO-$(C_1-C_4)$-Alkyl substituiert sein können und wobei die Alkylreste mit Fluoratomen substituiert sein können;
R6, R7, R8, R9, R10 unabhängig voneinander T-bicyclischer Heterocyclus, T-Aryl oder T- Heteroaryl, wobei der bicyclische Heterocyclus oder der Aryl- oder Heteroarylrest anelliert sein kann mit einem 5- oder 6-gliedrigen aromatischen oder nicht aromatischen Kohlenstoffring, bei welchen eine oder mehrere CH- bzw. $CH_2$-Gruppen durch Sauerstoffatome ersetzt sein können und wobei der 5- oder 6-gliedrige aromatische oder nicht aromatische Kohlensstoffring mit F, =O oder -$(C_1-C_6)$-Alkyl substituiert sein kann und wobei der bicyclische Heterocyclus 9 bis 12 Ringglieder enthalten kann und bis zu fünf CH- bzw. $CH_2$- Gruppen unabhängig voneinander durch N, NR20, O, $S(O)_m$ oder C=O ersetzt sein können und wobei der Aryl oder Heteroarylrest oder bicyclische Heterocyclus unsubstituiert sein kann oder einfach oder mehrfach substituiert sein kann mit R10

R11, F, Cl, Br, J, CN, $N_3$, NC, $NO_2$, $CF_3$, $(CH_2)_n$-O-R11, $(CH_2)_n$-O- $(CH_2)_r$-OH, $(CH_2)_n$-O-CH$(CH_2OH)_2$, $(CH_2)_n$-O-$(CH_2)_n$-CO-O-$(CH_2)_r$ $NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O$(C_1-C_6)$-Alkyl]$_2$, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N$(R13)_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-CO-R16, $(CH_2)_n$-NR12-CO-NR12R13, $(CH_2)_n$-NR12-CO-N$(R12)_2$, $(CH_2)_n$-NR12-CO-NHR11, $(CH_2)_n$-NH- C(=NH)-$NH_2$, $(CH_2)_n$-NH-C(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$ -CO-NH- [$(C_1-C_8)$-Alkyl], $(CH_2)_n$-NH-$(CH_2)_n$ -CO-NH-$(CH_2)_r$-OH, $(CH_2)_n$-NH- $(CH_2)_n$ -CO-N[(C1-C8)-Alkyl]$_2$, $(CH_2)$n-NH-$(CH_2)_n$ -CO-NH-[$(C_3-C_8)$- Cycloalkyl], $(CH_2)_n$-NH-$(CH_2)_n$ -CO-N[$(C_3-C_8)$-Cycloalkyl]$_2$, $(CH_2)_n$- NH-C$(CH_3)_2$-CO-O$(C_1-C_8)$-Alkyl, $(CH_2)_n$-NH-C$(CH_3)_2$-CO-O$(C_3-C_8)$- Cycloalkyl, $(CH_2)_n$-NH-C$(CH_3)_2$-CO-O-$(CH_2)_r$$NH_2$, $(CH_2)_n$-NH- C$(CH_3)_2$-CO-O-$(CH_2)_n$-Aryl, $(CH_2)_n$-NH-C$(CH_3)_2$-CO-O-$(CH_2)_n$- Heteroaryl, $(CH_2)_n$-NH-C$(CH_3)_2$-CO-$NH_2$, $(CH_2)_n$-NH-C$(CH_3)_2$-CO-NH- [$(C_1-C_8)$-Alkyl], $(CH_2)_n$-NH-C$(CH_3)_2$-CO-NH-$(CH_2)_r$-OH, $(CH_2)_n$-NH- C$(CH_3)_2$-CO-N[$(C_1-C_8)$-Alkyl]$_2$, $(CH_2)_n$-NH-$(CH_3)_2$-CO-NH-[$(C_3-C_8)$- Cycloalkyl], $(CH_2)_n$-NH-C$(CH_3)_2$-CO-N[$(C_3-C_8)$-Cycloalkyl]$_2$, $(CH_2)_n$- NH-C$(CH_3)_2$-COOH, $S(O)_m$-R12, $SO_2$-R16, $SO_2$-N=CH-N$(CH_3)_2$,

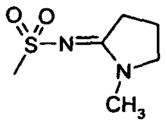
,

$SO_2$-NH-CO-R12, $SO_2$-NHR12, $So_2$-NH-$(CH_2)_r$-OH, $SO_2$- N[($C_1$-$C_8$)-Alkyl]$_2$, $SO_2$-NH-$(CH_2)_r$-$NH_2$, $SF_5$, COOH, CO-$NH_2$, $(CH_2)_q$- CN, $(CH_2)_n$-CO-NH-CN, $(CH_2)_n$-CO-NH-piperidin-1-yl, $(CH_2)_n$-CO-NH-$SO_2$-NHR12, $(CH_2)_n$-CO-NH-$SO_2$-R18, $(CH_2)_n$-CHO, $(CH_2)_n$- C(=NH)$NH_2$, $(CH_2)_n$-C(=NH)-NHOH, $(CH_2)_n$-C(=NH)-[NH-O-($C_1$-$C_6$)- Alkyl], $(CH_2)_n$-C(=NH)(R16), $(CH_2)_n$-C(=NR13)$_r$NHR12, $(CH_2)_n$- C(=NR12)NR12-R13, $(CH_2)_n$-C(=$NSO_2$-R12)$NH_2$, $(CH_2)_n$- C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O- ($C_1$-$C_6$)-Alkyl, S(O)$_m$-($C_1$-$C_6$)-Alkyl, $SO_2$-$NH_2$, COOH, $CONH_2$, CO- O($C_1$-$C_6$)-Alkyl, CO-($C_1$-$C_6$)-Alkyl substituiert sein können und wobei die Alkylreste mit Fluoratomen substituiert sein können, und wobei, wenn R3 gleich CN, $NO_2$ oder Halogen und R4 gleich $CF_3$ oder Halogen und R gleich R' gleich Methyl ist, der X-Arylrest mit mindestens einem der oben genannten von Wasserstoff verschiedenen Substituenten versehen ist;

H, F, Cl, Br, J, CN, $N_3$, NC, $NO_2$ $CF_3$,
($C_1$-$C_8$)-Alkyl, ($C_2$-$C_{10}$)-Alkenyl, ($C_2$-$C_{10}$)-Alkinyl, ($C_3$-$C_8$)-Cycloalkyl, Aryl, Heteroaryl,
$(CH_2)_n$-CO-[O-($C_1$-$C_8$)-Alkyl], $(CH_2)_n$-CO-[O-($C_3$-$C_8$)-Cycloalkyl], $(CH_2)_n$-CO- [($C_1$-$C_8$)-Alkyl], $(CH_2)_n$-CO-[($C_3$-$C_8$)-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-($C_1$-$C_6$)-Alkyl], $(CH_2)_n$-P(O)[O-($C_1$-$C_6$)-Alkyl]$_2$, $(CH_2)_n$- P(O)(OH)(O-$CH_2$-Aryl), $(CH_2)_n$-P(O)(O-$CH_2$-Aryl)$_2$, $(CH_2)_n$-P(O)(OH)$_2$,
$(CH_2)$n-$SO_3H$, $(CH_2)_n$-$SO_2$-$NH_2$,
$(CH_2)_n$-CO-NH-[($C_1$-$C_8$)-Alkyl], $(CH_2)_n$-CO-N[($C_1$-$C_8$)-Alkylh, $(CH_2)_n$-CO-NH- [($C_3$-$C_8$)-Cycloalkyl],
($C_2$-$C_{10}$)-Alkenyl-CO-O[($C_1$-$C_6$)-Alkyl], ($C_2$-$C_{10}$)-Alkenyl-$CONH_2$, ($C_2$-$C_{10}$)- Alkenyl-COOH, ($C_2$-$C_{10}$)-Alkinyl-CO-O[($C_1$-$C_6$)-Alkyl], ($C_2$-$C_{10}$)-Alkinyl- $CONH_2$, ($C_2$-$C_{10}$)-Alkinyl-COOH,
$(CH_2)_n$-CO-R16,
$(CH_2)_n$-OH, $(CH_2)_n$-O-($C_1$-$C_8$)-Alkyl, $(CH_2)_n$-O-($C_2$-$C_{10}$)-Alkenyl, $(CH_2)_n$-O-($C_2$- $C_{10}$)-Alkinyl, $(CH_2)_n$-O-($C_3$-$C_8$)-Cycloalkyl, $(CH_2)_n$-O-$(CH_2)_q$-[($C_3$-$C_8$)- Cycloalkyl], $(CH_2)_n$-O-$(CH_2)_n$-CO-[O-($C_1$-$C_8$)-Alkyl], $(CH_2)_n$-O-$(CH_2)_n$-CO-[O- ($C_3$-$C_8$)-Cycloalkyll, $(CH_2)_n$-O-$(CH_2)_n$-CO-[($C_1$-$C_8$)-Alkyl], $(CH_2)_n$-O-$(CH_2)_n$- CO-[($C_3$-$C_8$)-Cycloalkyl], $(CH_2)_n$-O-$(CH_2)_n$-CO-[O-$(CH_2)_v$-Aryl], $(CH_2)_n$-O- $(CH_2)_n$-CO-[O-$(CH_2)_v$-Heteroaryl], $(CH_2)_n$-O-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O-$(CH_2)_n$-P(O)(OH)[O- ($C_1$-$C_6$)-Alkyl], $(CH_2)_n$-O-$(CH_2)_n$P(O)[O-($C_1$-$C_6$)-Alkyl]$_2$, $(CH_2)_n$-O-$(CH_2)_n$- P(O)(OH)(O-$CH_2$-Aryl), $(CH_2)_n$-O-$(CH_2)_n$-P(O)(O-$CH_2$-Aryl)$_2$, $(CH_2)_n$-O- $(CH_2)_n$-P(O)(OH)$_2$, $(CH_2)_n$-O-$(CH_2)_n$-$SO_3H$, $(CH_2)_n$-O-$(CH_2)_n$-$SO_2$-$NH_2$, $(CH_2)_n$- O-$(CH_2)_n$-CO-NH-[($C_1$-$C_8$)-Alkyl], $(CH_2)_n$-O-$(CH_2)_n$-CO-N[($C_1$-$C_8$)-Alkyl]$_2$, $(CH_2)_n$-O-$(CH_2)_n$-CO-NH-[($C_3$-$C_8$)-Cycloalkyl], $(CH_2)_n$-O-$(CH_2)_n$-CR21 R22- CO-O[($C_1$-$C_6$)-Alkyl], $(CH_2)_n$-O-$(CH_2)_n$-CR21R22-$CONH_2$, $(CH_2)_n$-O-$(CH_2)_n$- CR21R22-COOH, $(CH_2)_n$-O-$(CH_2)_n$-CO-R16, $(CH_2)_n$-O-$(CH_2)_r$-OH, $(CH_2)_n$-O- CH($CH_2OH$)$_2$, $(CH_2)_n$-O-$(CH_2)_n$-CO-O-$(CH_2)_r$-$NH_2$, $(CH_2)_n$-O-$(CH_2)_n$-CO-NH- $(CH_2)_r$-OH, O-R13, $OCF_3$,
$(CH_2)_n$-$NH_2$, $(CH_2)_n$-NH-($C_1$-$C_8$)-Alkyl, $(CH_2)_n$-NH-($C_3$-$C_8$)-Cycloalkyl,
$(CH_2)_n$-NH-$(CH_2)_n$-CO-[O-($C_3$-$C_8$)-Cycloalkyl], $(CH_2)_n$-NH-$(CH_2)_n$-CO-[($C_1$- $C_8$)-Alkyl], $(CH_2)_n$-NH-$(CH_2)_n$-CO-[($C_3$-$C_8$)-Cycloalkyl], $(CH_2)_n$-NH-$(CH_2)_n$- CO-[O-$(CH_2)_v$-Aryl], $(CH_2)_n$-NH-$(CH_2)_n$-CO-[O-$(CH_2)_v$-Heteroaryl], $(CH_2)_n$- NH-$(CH_2)_q$-CO-NH-CN,
$(CH_2)_n$-NH-$(CH_2)_n$-P(O)(OH)$_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[($C_1$-$C_6$)-Alkyl], $(CH_2)_n$-NH-$(CH_2)_n$- CR21 R22-$CONH_2$, $(CH_2)_n$-NH-$(CH_2)_n$-CR21 R22-COOH,
$(CH_2)_n$-NH-$(CH_2)_n$-CO-R16,
$(CH_2)_n$-NH-$(CH_2)_n$-$SO_2$-[($C_1$-$C_8$)-Alkyl], $(CH_2)_n$-NH- $(CH_2)_n$-$SO_2$-[($C_3$-$C_8$)- Cycloalkyl], $(CH_2)_n$-NH-$SO_2$- $(CH_2)_n$-$NH_2$, $(CH_2)_n$-NH-$SO_2$-$(CH_2)_n$-NH-($C_1$-$C_8$)- Alkyl, $(CH_2)_n$-NH-$SO_2$-$(CH_2)_n$-NH-($C_3$-$C_8$)-Cycloalkyl, $(CH_2)_n$-NH-$SO_2$-$(CH_2)_n$- N[($C_1$-$C_8$)-Alkyl]$_2$,
$(CH_2)_n$-NH-CN, $(CH_2)_n$-NH-$SO_2$-R16,
$(CH_2)_n$-NR12-CO-NH-($C_1$-$C_8$)-Alkyl, $(CH_2)_n$-NR12-CO-NH-($C_3$-$C_8$)- Cycloalkyl, $(CH_2)_n$-NR12-CO-$NH_2$, $(CH_2)_n$-NR12-CO-NH-$SO_2$-($C_1$-$C_8$)-Alkyl, $(CH_2)_n$-NR12-CO-NH-$SO_2$-($C_3$-$C_8$)-Cycloalkyl, $(CH_2)_n$-NR12-CO-N[($C_1$-$C_8$)-

Alkyl]$_2$, $(CH_2)_n$-NH-CO-NH-$(CH_2)_n$-CO-[O-($C_1$-$C_8$)-Alkyl], $(CH_2)_n$-NH-CO- NH-$(CH_2)_q$-CO-$NH_2$, $(CH_2)_n$-NH-CO-NH-$(CH_2)_q$-COOH, $(CH_2)_n$-NH-C(=NH)- $NH_2$, $(CH_2)_n$-NH-C(=NH)-R16, $(CH_2)_n$-NH-C(=NH)-NH[($C_1$-$C_8$)-Alkyl], $(CH_2)_n$-NH-C(=N-$SO_2$-($C_1$-$C_8$)-Alkyl)-$NH_2$, $(CH_2)_n$-NH-C(=N-$SO_2$-($C_1$-$C_8$)- Alkyl)-NH[($C_1$-$C_8$)-Alkyl], $(CH_2)_n$-NH-C(=N-$SO_2$-$NH_2$)-$NH_2$, $(CH_2)_n$-NH- C(=N-$SO_2$-$NH_2$)-NH[($C_1$-$C_8$)-Alkyl], $(CH_2)_n$-NH-C(=NH)-N[($C_1$-$C_8$)-Alkyl]$_2$, $(CH_2)_n$-NH-C(=N-$SO_2$-($C_1$-$C_8$)-Alkyl)-N[($C_1$-$C_8$)-Alkyl]$_2$, $(CH_2)_n$-NH-$(CH_2)_n$- CO-O-$(CH_2)_r$-$NH_2$, $(CH_2)_n$-NH-$(CH_2)_n$ -CO-NH-[($C_1$-$C_8$)-Alkyl], $(CH_2)_n$-NH- $(CH_2)_n$ -CO-NH-$(CH_2)_r$-OH, $(CH_2)_n$-NH-$(CH_2)_n$ -CO-N[($C_1$-$C_8$)-Alkyl]$_2$, $(CH_2)_n$- NH-$(CH_2)_n$ -CO-NH-[($C_3$-$C_8$)-Cycloalkyl], $(CH_2)_n$-NH-C$(CH_3)_2$-CO-O($C_3$-$C_8$)- Cycloalkyl, $(CH_2)_n$-NH-C$(CH_3)_2$-CO-O-$(CH_2)_r$-$NH_2$, $(CH_2)_n$-NH-C$(CH_3)_2$-CO- O-$(CH_2)_n$-Aryl, $(CH_2)_n$-NH-C$(CH_3)_2$-CO-O-$(CH_2)_n$-Heteroaryl, $(CH_2)_n$-NH- C$(CH_3)_2$-CO-NH-[($C_1$-$C_8$)-Alkyl], $(CH_2)_n$-NH-C$(CH_3)_2$-CO-NH-$(CH_2)_r$-OH, $(CH_2)_n$-NH-C$(CH_3)_2$-CO-N[($C_1$-$C_8$)-Alkyl]$_2$, $(CH_2)_n$-NH-$(CH_3)_2$-CO-NH-[($C_3$- $C_8$)-Cycloalkyl], $(CH_2)_n$-NH-C$(CH_3)_2$-CO-N[($C_3$-$C_8$)-Cycloalkyl]$_2$, $(CH_2)_n$-S$(O)_m$-($C_1$-$C_8$)-Alkyl, $(CH_2)_n$-S$(O)_m$-($C_3$-$C_8$)-Cycloalkyl, $(CH_2)_n$-$SO_2$- R16, $SO_2$-N=CH-N$(CH_3)_2$,

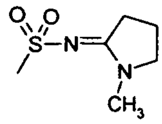,

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)-Alkyl, $(CH_2)_n$-$SO_2$-NH-($C_3$-$C_8$)-Cycloalkyl, $(CH_2)_n$-$SO_2$-N[($C_1$-$C_8$)-Alkylh, $SO_2$-NH- $(CH_2)_r$-OH, $SO_2$-NH-$(CH_2)_r$-$NH_2$, $SF_5$,

$(CH_2)_q$-CN,

$(CH_2)_n$-CO-NH-pipefidin-1-yl,

$(CH_2)_n$-CO-NH-$SO_2$-NHR12, $(CH_2)_n$-CO-NH-$SO_2$($C_1$-$C_8$)-Alkyl, $(CH_2)_n$-CO- NH-$SO_2$-($C_3$-$C_8$)-Cycloalkyl, $(CH_2)_n$-CHO, $(CH_2)_n$-C(=NH)$NH_2$, $(CH_2)_n$-C(=NH)NHOH, $(CH_2)_n$- C(=NH)(R16), $(CH_2)_n$-C(=NR13)NHR12, $(CH_2)_n$-C(=NR12)NR12R13, $(CH_2)_n$- C(=NH)O[($C_1$-$C_6$)-Alkyl], wobei die Alkyl- und Cycloalkylreste mit Fluoratomen substituiert sein können und wobei die Aryl- oder Heteroarylreste mit Halogen, CN, ($C_1$-$C_6$)-Alkyl, ($C_3$-$C_6$)-Cycloalkyl, O-($C_1$-$C_6$)-Alkyl, S$(O)_m$- ($C_1$-$C_6$)-Alkyl, $SO_2$-$NH_2$, COOH, $CONH_2$, CO-[O($C_1$-$C_6$)-Alkyl], CO-($C_1$-$C_6$)- Alkyl substituiert sein können und wobei die Alkylreste mit Fluoratomen substituiert sein können;

wobei immer mindestens einer der Reste R6, R7, R8, R9 und R10 die Bedeutung T-bicyclischer Heterocyclus, T-Aryl oder T-Heteroaryl besitzt und
wobei eines der vier Restepaare R6 und R7, oder R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, ($C_1$-$C_8$)-Alkyl oder =O substituiert sein können;

T NR23-CO-NR24, NR23-$SO_2$-NR24, $SO_2$-NR23-$SO_2$, CO-NR23-CO, NR23- C(=NR13)-NR24, NR23-C(=NR22)-NR24, CO-NR23-CR22R23, NR23-$SO_2$ CR22R24, CR22R24-$SO_2$-NR23, CR22R23-NR23-$SO_2$, $SO_2$-CR22R23-NR23, $SO_2$-NR23-CR22R23-, NR23-CR22R23-$SO_2$, CR23R24-CR23R24-CR23R24;

R11 H, ($C_1$-$C_8$)-Alkyl,($C_3$-$C_6$)-Cycloalkyl, $(CH_2)_n$-Aryl, $(CH_2)_n$-CO-[O-($C_1$-$C_6$)- Alkyl], $(CH_2)_n$-CO-[O-($C_3$-$C_6$)-Cycloalkyl], $(CH_2)_n$-CO-[($C_1$-C6)-Alkyl], $(CH_2)_n$- CO-[($C_3$-$C_6$)-Cycloalkyl], $(CH_2)_n$-CO-Aryl, $(CH_2)_n$-CO-Heteroaryl, $(CH_2)_q$-CO- $NH_2$, $(CH_2)_q$-COOH, $(CH_2)_n$-P(O)(OH)[O-($C_1$-$C_4$)-Alkyl], $(CH_2)_n$-P(O)[O-($C_1$- $C_4$)-Alkyl]$_2$, $(CH_2)_n$-P(O)(O-$CH_2$-Aryl)$_2$, $(CH_2)_n$-P(O)$(OH)_2$, $(CH_2)_n$-$SO_3$H, $(CH_2)_n$-$SO_2$-$NH_2$, $(CH_2)_n$-CO-NH-[($C_1$-$C_4$)-Alkyl], $(CH_2)_n$-CO-N[($C_1$-$C_4$)- Alkyl]$_2$, $(CH_2)_n$-CO-NH-[($C_3$-$C_6$)-Cycloalkyl], ($C_2$-$C_6$)-Alkenyl-CO-O[($C_1$-$C_4$)- Alkyl], ($C_2$-$C_6$)-Alkenyl-$CONH_2$, ($C_2$-$C_6$)-Alkenyl-COOH, ($C_2$-$C_6$)-Alkinyl-CO- O[($C_1$-$C_4$)-Alkyl], ($C_2$-$C_6$)-Alkinyl-$CONH_2$, ($C_2$-$C_6$)-Alkinyl-COOH, $(CH_2)_n$- CR21[(CO-O($C_1$-$C_4$)-Alkyl)]$_2$, $(CH_2)_n$-CR21$(CONH_2)_2$, $(CH_2)_n$-CR21 $(COOH)_2$, $(CH_2)_n$-CR21R22-CO-O[($C_1$-$C_4$)-Alkyl], $(CH_2)_n$-CR21R22-$CONH_2$, $(CH_2)_n$- CR21R22-CO-NH-[($C_1$-$C_4$)-Alkyl], $(CH_2)_n$-CR21R22-CO-N[($C_1$-$C_4$)-Alkyl)$_2$, $(CH_2)_n$-CR21R22-COOH, $(CH_2)_n$-CO-R16, $(CH_2)_n$- CO-NH-C$(CH_3)_2$-CO-O[($C_1$- $C_8$)-Alkyl], $(CH_2)_n$-CO-NH-C$(CH_3)_2$-$CONH_2$, $(CH_2)_n$-CO-NH-C$(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, ($C_1$-$C_4$)-Alkyl, O-($C_1$-$C_4$)-Alkyl, S$(O)_m$-($C_1$-$C_4$)-Alkyl, $SO_2$-$NH_2$, COOH, $CONH_2$, CO-O($C_1$-$C_4$)-Alkyl substituiert sein kann und wobei die Alkylreste mit Fluoratomen substituiert sein können;

R12 H, ($C_1$-$C_4$)-Alkyl, ($C_3$-$C_6$)-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, ($C_1$-$C_4$)-Alkyl, O-($C_1$-$C_4$)-Alkyl, $SO_2$-$NH_2$, COOH, $CONH_2$, CO-O($C_1$-$C_4$)-Alkyl substituiert sein kann und wobei die Alkylreste mit Fluoratomen substituiert sein können;

R13 H, $SO_2$-[($C_1$-$C_4$)-Alkyl], $SO_2$-[($C_3$-$C_6$)-Cycloalkyl], $SO_2$-$(CH_2)_n$-Aryl, $SO_2$-$(CH_2)_n$-Heteroaryl, wobei die Alkyl- und Cycloalkylreste mit Fluoratomen substituiert sein können und wobei der Aryl- oder Heteroarylrest mit Halogen, CN, $CF_3$, ($C_1$-$C_4$)-Alkyl, O-[($C_1$-$C_4$)-Alkyl], $S(O)_m$-[($C_1$-$C_6$)-Alkyl], $SO_2$-$NH_2$, COOH, $CONH_2$, CO- [O($C_1$-$C_4$)-Alkyl], substituiert sein kann und wobei die Alkylreste mit Fluoratomen substituiert sein können;

R15 ($C_1$-$C_6$)-Alkyl, wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[($C_1$-$C_6$)- Alkyl]piperazin-1-yl, Thiomorpholin-1,1-Dioxid-4-yl, NH-$(CH_2)_r$-OH, NH-$CH(CH_2OH)_2$, NH-$C(CH_2OH)_3$, N[($C_1$-$C_4$)-Alkyl-OH$]_2$, D-Glucamin-N-yl, N- Methyl-D-Glucamin-N-yl, NH-[($C_1$-$C_4$)-Alkyl]-COOH, NH-[($C_1$-$C_4$)-Alkyl]- $CONH_2$, N[( $C_1$-$C_4$)-Alkyl][($C_1$-$C_4$)-Alkyl]-COOH, NH-[C(H)(Aryl)]-CO-O($C_1$- $C_4$)-Alkyl, NH-[C(H)(Aryl)]-COOH, NH-[C(H)(Aryl)]-$CONH_2$, NH- [C(H)(Heteroaryl)]-CO-O($C_1$-$C_4$)-Alkyl, NH-[C(H)(Heteroaryl)]-COOH, NH- [C(H)(Heteroaryl)]-$CONH_2$, NH-[($C_3$-$C_6$)-Cycloalkyl]-CO-O($C_1$-$C_4$)-Alkyl, NH- [($C_3$-$C_6$)-Cycloalkyl]-COOH, NH-[($C_3$-$C_6$)-Cycloalkyl]-$CONH_2$, NH-($C_1$-$C_4$)-Alkyl-OH, NH-[( $C_1$-$C_4$)-Alkyl]-$SO_2$-($C_1$- $C_4$)-Alkyl, NH-[( $C_1$-$C_4$)-Alkyl]- $SO_3H$, NH-[( $C_1$-$C_4$)-Alkyl]-$SO_2$-$NH_2$, wobei die Alkohol (OH) - Funktionen durch F ersetzt sein können und wobei der Aryl- oder Heteroarylrest mit Halogen, CN, ($C_1$-$C_4$)-Alkyl, O-($C_1$-$C_4$)-Alkyl, OH, $SO_2$-$NH_2$, COOH, $CONH_2$, CO-O($C_1$-$C_4$)-Alkyl substituiert sein kann;

R18 $(CH_2)_n$-CR25R26-CO-O($C_1$-$C_4$)-Alkyl, $(CH_2)_n$-CR25R26-CO-$NH_2$, $(CH_2)_n$- CR25R26-COOH;

R20 H, ($C_1$-$C_4$)-Alkyl, ($C_3$-$C_6$)-Cycloalkyl, Aryl, [($C_1$-$C_4$)-Alkyl]-Aryl;

R21 H, F, $CF_3$, ($C_1$-$C_4$)-Alkyl, ($C_3$-$C_6$)-Cycloalkyl, OH, O-($C_1$-$C_4$)-Alkyl, O-($C_3$-$C_6$)- Cycloalkyl, O-$(CH_2)_n$Aryl, O-(CO)-($C_1$-$C_4$)-Alkyl, O-(CO)-($C_3$-$C_6$)-Cycloalkyl, O-(CO)-O-($C_1$-$C_4$)-Alkyl, O-(CO)-O-($C_3$-$C_6$)-Cycloalkyl, NH-[($C_1$-$C_4$)-Alkyl]- Aryl, $NH_2$, NH-($C_1$-$C_4$)-Alkyl, NH-(CO)-($C_1$-$C_4$)-Alkyl;

R22 H, $CF_3$, ($C_1$-$C_4$)-Alkyl, Aryl, [($C_1$-$C_4$)-Alkyl]-Aryl;

R23, R24 unabhängig voneinander H, ($C_1$-$C_4$)-Alkyl, ($C_3$-$C_6$)-Cycloalkyl, [($C_1$-$C_4$)-Alkyl]- [($C_3$-$C_6$)-Cycloalkyl], Aryl, [($C_1$,-$C_4$)-Alkyl]-Aryl

oder R23 und R24 bilden zusammen eine -CH=CH-, -$CH_2$-$CH_2$-, -$CH_2$-$CH_2$- $CH_2$-, oder -$CH_2$-$CH_2$-$CH_2$-$CH_2$-Einheit, worin eine $CH_2$-Gruppierung durch C=O, CHF oder $CF_2$ ersetzt sein kann, und worin bis zu vier Wasserstoffatome durch einen ($C_1$-$C_4$)-Alkylrest ersetzt sein können;

R25, R26 unabhängig voneinander H, F, ($C_1$-$C_4$)-Alkyl, Aryl, [($C_1$-$C_4$)-Alkyl]-Aryl, wobei der Aryl mit Halogen, CN, OH, O-($C_1$-$C_4$)-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[($C_1$-$C_4$)-Alkyl] oder CO ersetzt sein kann;

wobei die Verbindung N-[3-t-butyl-1-(4-methylphenyl)-1H-pyrazol-5-yl]-N'-(4-{[3-(2-methylphenyl)-2,4-dioxo-1 -imidazolidinyl]methyl]}phenyl)harnstoff ausgenommen ist,
sowie deren physiologisch verträgliche Salze.

**5.** Verbindungen der Formel Ia gemäss Ansprüch 1

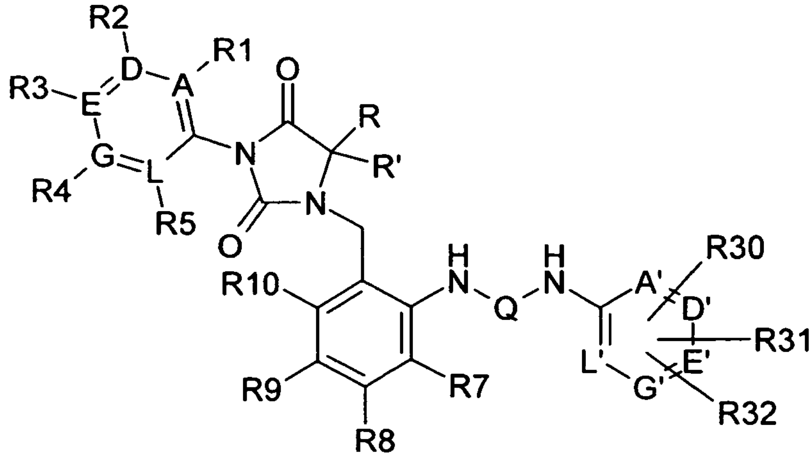

Ia

worin bedeuten

R, R' unabhängig voneinander H, Aryl, $(C_1-C_4)$-Alkyl, wobei $(C_1-C_4)$-Alkyl oder der Arylrest substituiert sein kann mit Halogen;

oder R und R' bilden gemeinsam einen Ring mit drei bis acht Kohlenstoffatomen, wobei ein Kohlenstoffatom durch O, $S(O)_m$, NR13 oder NR15 ersetzt sein kann;

m 0, 1, 2;

n 0, 1, 2;

q 1, 2;

r 2, 3;

v 0, 1, 2;

A, D, E, G, L unabhängig voneinander C oder N, wobei bei der Bedeutung N der entsprechende Substituent R1, R2, R3, R4, R5 entfällt,

oder R2-D=E-R3 oder R4-G=L-R5 haben die Bedeutung S oder O und wobei der Fünf- oder Sechsring mit $-(CH_2)_3-$ oder $-(CH_2)_4-$ oder -CH=CH-CH=CH- zu einem Bicyclus anelliert sein kann;

Q C=O, $SO_2$;

R1, R2, R3, R4, R5 unabhängig voneinander H, F, Cl, Br, J, CN, $CF_3$, $(C_1-C_8)$-Alkyl, $(C_3-C_8)$-Cycloalkyl, $(CH_2)_n$-Aryl, $(CH_2)_n$-Heteroaryl, $OCF_3$, O-R11, NR13R15, $S(O)_m$-R12, $SO_2-NH_2$, $SO_2$-NH-[$(C_1-C_8)$-Alkyl], $SO_2$-NH-[$(C_3-C_8)$-Cycloalkyl], $SO_2$-NH-$(CH_2)_n$-Aryl, $SO_2$-NH-$(CH_2)_n$-Heteroaryl, $SO_2$-N[$(C_1-C_8)$-Alkyl]$_2$, $SO_2$-R16, $SF_5$, CO-O[$(C_1-C_8)$-Alkyl],
CO-O[$(C_3-C_6)$-Cycloalkyl], $CO-NH_2$, CO-NH-[$(C_1-C_4)$-Alkyl], CO-N[$(C_1-C_4)$- Alkyl]$_2$, C(=NH)-$NH_2$, C(=NH)-R16, $(CH_2)_n$-C(=$NSO_2$-R12)$NH_2$, CO-R16, COOH, CO-$(C_1-C_4)$-Alkyl, CO-$(C_3-C_6)$-Cycloalkyl, CO-Aryl, CO-Heteroaryl, CH(OH)-Aryl, CH(OH)-Heteroaryl, CHF-Aryl, CHF-Heteroaryl, $CF_2$-Aryl, $CF_2$-Heteroaryl, $CH_2$-OH, $CH_2$-CN, $CH_2$-O-R12, $CH_2$-O-$(CH_2)_q$-COOH,

wobei die Alkyl- und Cycloalkylreste mit Fluoratomen substituiert sein können und wobei die Aryl- oder Heteroarylreste mit Halogen, CN, $(C_1-C_4)$-Alkyl, O- $(C_1-C_4)$-Alkyl, $OCF_3$, OH, O-$(CH_2)_n$-Aryl, $(CH_2)_n$-Aryl, $S(O)_m$-$(C_1-C_4)$-Alkyl, $SO_2-NH_2$, SH, NR12R13, NH-CO-[$(C_1-C_4)$-Alkyl], NH-CO-$(CH_2)_n$-Aryl, $(CH_2)_n$-COOH, $(CH_2)_n$-$CONH_2$, $(CH_2)_n$-CO-O$(C_1-C_4)$-Alkyl, $(CH_2)_n$-CO-$(C_1-C_4)$-Alkyl substituiert sein können und wobei die Alkylreste mit Fluoratomen substituiert sein können;

R7, R8, R9, R10 unabhängig voneinander H, F, C1, Br, J, CN, $N_3$, NC, $CO_2$, $CF_3$, $(C_1-C_8)$-Alkyl, $(C_2-C_{10})$-Alkenyl, $(C_2-C_{10})$-Alkinyl, $(C_3-C_8)$-Cycloalkyl, Aryl, Heteroaryl,
$(CH_2)_n$-CO-[O-$(C_1-C_8)$-Alkyl], $(CH_2)_n$-CO-[O-$(C_3-C_8)$-Cycloalkyl], $(CH_2)_n$-CO- (($C_1-C_8$)-Alkyl], $(CH_2)_n$-CO-[$(C_3-C_8)$-Cycloalkyl],
$(CH_2)_n$-CO-[O-$(CH_2)_v$-Aryl],
$(CH_2)_n$-CO-$NH_2$, $(CH_2)_n$-COOH, $(CH_2)_n$-CO-NH-CN,
$(CH_2)_n$-P(O)(OH)[O-$(C_1-C_6)$-Alkyl], $(CH_2)_n$-P(O)[O-$(C_1-C_6)$-Alkyl]$_2$, $(CH_2)_n$- P(O)(OH)(O-$CH_2$-Aryl), $(CH_2)_n$-P(O)(O-$CH_2$-Aryl)$_2$, $(CH_2)_n$-P(O)$(OH)_2$,
$(CH_2)_n$-$SO_3H$, $(CH_2)_n$-$SO_2$-$NH_2$,

$(CH_2)_n$-CO-NH-[($C_1$-$C_8$)-Alkyl], $(CH_2)_n$-CO-N[($C_1$-$C_8$)-Alkyl], $(CH_2)_n$-CO-NH- [($C_3$-$C_8$)-Cycloalkyl], ($C_2$-$C_{10}$)-Alkenyl-CO-O[($C_1$-$C_6$)-Alkyl], ($C_2$-$C_{10}$)-Alkenyl-CONH12, ($C_2$-$C_{10}$)- Alkenyl-COOH, ($C_2$-$C_{10}$)-Alkinyl-CO-O[($C_1$-$C_6$)-Alkyl], ($C_2$-$C_{10}$)-Alkinyl- $CONH_2$, ($C_2$-$C_{10}$)-Alkinyl-COOH,

$(CH_2)_n$-CO-R16,

$(CH_2)_n$-OH, $(CH_2)_n$-O-($C_1$-$C_8$)-Alkyl, $(CH_2)_n$-O-($C_2$-$C_{10}$)-Alkenyl, $(CH_2)_n$-O-($C_2$- $C_{10}$)-Alkinyl, $(CH_2)_n$-O-($C_3$-$C_8$)-Cycloalkyl, $(CH_2)_n$-O-$(CH_2)$q.[($C_3$-$C_8$)- Cycloalkyl], $(CH_2)_n$-O-$(CH_2)_n$-CO-[O-($C_1$-$C_8$)-Alkyl], $(CH_2)_n$-O-$(CH_2)_n$-CO-[O- ($C_3$-$C_8$)-Cycloalkyl], $(CH_2)_n$-O-$(CH_2)_n$-CO-[($C_1$-$C_8$)-Alkyl], $(CH_2)_n$-O-$(CH_2)_n$- CO-[($C_3$-$C_8$)-Cycloalkyl], $(CH_2)_n$-O-$(CH_2)_n$-CO-[O-$(CH_2)_v$-Aryl], $(CH_2)_n$-O- $(CH_2)_n$-CO-[O-$(CH_2)_v$-Heteroaryl], $(CH_2)_n$-O-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O-$(CH_2)_n$-P(O)(OH)[O- ($C_1$-$C_6$)-Alkyl], $(CH_2)_n$-O-$(CH_2)_n$-P(O)[O-($C_1$-$C_6$)-Alkyl]$_2$, $(CH_2)$n-O-$(CH_2)_n$- P(O)(OH)(O-$CH_2$-Aryl), $(CH_2)_n$-O-$(CH_2)_n$-P(O)(O-$CH_2$-Aryl)$_2$, $(CH_2)_n$-O- $(CH_2)_n$-P(O)$(OH)_2$, $(CH_2)_n$-O-$(CH_2)_n$-$SO_3H$, $(CH_2)_n$-O-$(CH_2)_{n}$-$SO_2$-$NH_2$, $(CH_2)_n$- O-$(CH_2)_n$-CO-NH-[($C_1$-$C_8$)-Alkyl], $(CH_2)_n$-O-$(CH_2)_n$-CO-N[($C_1$-$C_8$)-Alkyl]$_2$, $(CH_2)_n$-O-$(CH_2)_n$-CO-NH-[($C_3$-$C_8$)-Cycloalkyl], $(CH_2)_n$-O-$(CH_2)_n$-CR21R22- CO-O[($C_1$-$C_6$)-Alkyl], $(CH_2)_n$-O-$(CH_2)_n$-CR21R22-$CONH_2$, $(CH_2)_n$-O-$(CH_2)_n$- CR21R22-COOH, $(CHz)_n$-O-$(CH_2)_n$-CO-R16, $(CH_2)_n$-O-$(CH_2)_r$-OH, $(CH_2)_n$-O- $CH(CH_2OH)_2$, $(CH_2)_n$-O-$(CH_2)_n$-CO-O-$(CH_2)_r$-$NH_2$, $(CH_2)_n$-O-$(CH_2)_n$-CO-NH- $(CH_2)_r$-OH, O-R13, $OCF_3$,

$(CH_2)_n$-$NH_2$, $(CH_2)_n$-NH-($C_1$-$C_8$)-Alkyl, $(CH_2)_n$-NH-($C_3$-$C_8$)-Cycloalkyl,

$(CH_2)_n$-NH-$(CH_2)_n$-CO-[O-($C_3$-$C_8$)-Cycloalkyl], $(CH_2)_n$-NH-$(CH_2)_n$-CO-[($C_1$- $C_8$)-Alkyl], $(CH_2)_n$-NH-$(CH_2)_n$-CO-[($C_3$-$C_8$)-Cycloalkyl], $(CH_2)_n$-NH-$(CH_2)_n$- CO-[O-$(CH_2)_v$-Aryl], $(CH_2)_n$-NH-$(CH_2)_n$-CO-[O-$(CH_2)_v$-Heteroaryl], $(CH_2)_n$- NH-$(CH_2)_q$-CO-NH-CN,

$(CH_2)_n$-NH-$(CH_2)_n$-P(O)$(OH)_2$,

$(CH_2)_n$-NH-$(CH_2)_n$-$SO_3H$,

$(CH_2)_n$-NH-$(CH_2)_n$-$SO_2$-$NH_2$,

$(CH_2)_n$-NH-$(CH_2)_n$-CR21R22-CO-O[($C_1$-$C_6$)-Alkyl], $(CH_2)_n$-NH-$(CH_2)_n$- CR21R22-$CONH_2$, $(CH_2)_n$-NH-$(CH_2)_n$-CR21R22-COOH,

$(CH_2)_n$-NH-$(CH_2)_n$-CO-R16,

$(CH_2)_n$-NH-$(CH_2)_n$-$SO_2$-[($C_1$-$C_8$)-Alkyl], $(CH_2)_n$-NH- $(CH_2)_n$-$SO_2$-[($C_3$-$C_8$)- Cycloalkyl], $(CH_2)_n$-NH-$SO_2$-$(CH_2)_n$-$NH_2$, $(CH_2)_n$-NH-$SO_2$$(CH_2)_n$-NH-($C_1$-$C_8$)- Alkyl, $(CH_2)_n$-NH-$SO_2$-$(CH_2)_n$-NH-($C_3$-$C_8$)-Cycloalkyl, $(CH_2)_n$-NH-$SO_2$-$(CH_2)_n$- N[($C_1$-$C_8$)-Alkyl]$_2$,

$(CH_2)_n$-NH-CN, $(CH_2)_n$-NH-$SO_2$-R16,

$(CH_2)_n$-NR12-CO-NH-($C_1$-$C_8$)-Alkyl, $(CH_2)_n$-NR12-CO-NH-($C_3$-$C_8$)- Cycloalkyl, $(CH_2)_n$-NR12-CO-$NH_2$, $(CH_2)_n$-NR12-CO-NH-$SO_2$-($C_1$-$C_8$)-Alkyl, $(CH_2)_n$-NR12-CO-NH-$SO_2$-($C_3$-$C_8$)-Cycloalkyl, $(CH_2)_n$-NR12-CO-N[($C_1$-$C_8$)-Alkyl]$_2$, $(CH_2)$n-NH-CO-NH-$(CH_2)_n$-CO-[O-($C_1$-$C_8$)-Alkyl], $(CH_2)_n$-NH-CO- NH-$(CH_2)_q$-CO-$NH_2$, $(CH_2)_n$-NH-CO-NH-$(CH_2)_q$-COOH, $(CH_2)_n$-NH-C(=NH)- $NH_2$, $(CH_2)_n$-NH-C(=NH)-R16, $(CH_2)_n$-NH-C(=NH)-NH[($C_1$$C_8$)-Alkyl], $(CH_2)_n$-NH-C(=N-$SO_2$-($C_1$-$C_8$)-Alkyl)-$NH_2$, $(CH_2)_n$-NH-C(=N-$SO_2$-($C_1$-$C_8$)- Alkyl)-NH[($C_1$-$C_8$)-Alkyl], $(CH_2)_n$-NH-C(=N-$SO_2$-$NH_2$)-$NH_2$, $(CH_2)_n$-NH- C(=N-$SO_2$-$NH_2$)-NH[($C_1$-$C_8$)-Alkyl], $(CH_2)_n$-NH-C(=NH)-N[($C_1$-$C_8$)-Alkyl]$_2$, $(CH_2)_n$-NH-C(=N-$SO_2$-($C_1$-$C_8$)-Alkyl)-N[($C_1$-$C_8$)-Alkyl]$_2$, $(CH_2)_n$-NH-$(CH_2)_n$- CO-O-$(CH_2)_r$-$NH_2$, $(CH_2)_n$-NH-$(CH_2)_n$ -CO-NH-[($C_1$-$C_8$)-Alkyl], $(CH_2)_n$-NH- $(CH_2)_n$ -CO-NH-$(CH_2)_r$-OH, $(CH_2)_n$-NH-$(CH_2)_n$ -CO-N[($C_1$-$C_8$)-Alkyl]$_2$, $(CH_2)_n$- NH-$(CH_2)_n$ -CO-NH-[($C_3$-$C_8$)-Cycloalkyl], $(CH_2)_n$-NH-$C(CH_3)_2$-CO-O($C_3$-$C_8$)-Cycloalkyl, $(CH_2)_n$-NH-$C(CH_3)_2$-CO-O-$(CH_2)_r$-$NH_2$, $(CH_2)_n$-NH-$C(CH_3)_2$-CO- O-$(CH_2)_n$-Aryl, $(CH_2)_n$-NH-$C(CH_3)_2$-CO-O-$(CH_2)_n$-Heteroaryl, $(CH_2)_n$-NH- $C(CH_3)_2$-CO-NH-[($C_1$-$C_8$)-Alkyl], $(CH_2)_n$-NH-$C(CH_3)_2$-CO-NH-$(CH_3)_2$-OH, $(CH_2)_n$-NH-$C(CH_3)_2$-CO-N[($C_1$-$C_8$)-Alkyl]$_2$, $(CH_2)_n$-NH-$(CH_3)_2$-CO-NH-[($C_3$- $C_8$)-Cycloalkyl], $(CH_2)_n$-NH-$C(CH_3)_2$-CO-N[($C_3$-$C_8$)-Cycloalkyl]$_2$, $(CH_2)_n$-$S(O)_m$ ($C_1$-$C_8$)-Alkyl, $(CH_2)_n$-$S(O)_m$-($C_3$-$C_8$)-Cycloalkyl, $(CH_2)_n$-$SO_2$- R16, $SO_2$-N=CH-$N(CH_3)_2$,

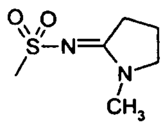
,

$(CH_2)_n$-$SO_2$-NH-CO-($C_1$-$C_8$)-Alkyl, $(CH_2)_n$-$SO_2$-NH-CO-($C_3$-$C_8$)-Cycloalkyl, $(CH_2)_n$-$SO_2$-NH-($C_1$-$C_8$)-Alkyl, $(CH_2)_n$-$SO_2$-NH-($C_3$-$C_8$)-Cycloalkyl, $(CH_2)_n$-$SO_2$-N[($C_1$-$C_8$)-Alkyl]$_2$, $SO_2$-NH- $(CH_2)_r$-OH, $SO_2$-NH-$(CH_2)_r$-$NH_2$, $SF_5$,

$(CH_2)_q$-CN,

$(CH_2)_n$-CO-NH-piperidin-1-yl,

$(CH_2)_n$-CO-NH-$SO_2$-NHR12, $(CH_2)_n$-CO-NH-$SO_2$-($C_1$-$C_8$)-Alkyl, $(CH_2)_n$-CO- NH-$SO_2$-($C_3$-$C_8$)-Cycloalkyl,

$(CH_2)_n$-CHO, $(CH_2)_n$-C(=NH)$NH_2$, $(CH_2)_n$-C(=NH)NHOH, $(CH_2)$n- C(=NH)(R16), $(CH_2)_n$-C(=NR13)NHR12,

$(CH_2)_n$-C(=NR12)NR12R13, $(CH_2)_n$- C(=NH)O[$(C_1-C_6)$-Alkyl], wobei die Alkyl- und Cycloalkylreste mit Fluoratomen substituiert sein können und wobei die Aryl- oder Heteroarylreste mit Halogen, CN, $(C_1-C_6)$-Alkyl, $(C_3-C_6)$-Cycloalkyl, O-$(C_1-C_6)$-Alkyl, $S(O)_m$ $(C_1-C_6)$-Alkyl, $SO_2$-$NH_2$, COOH, $CONH_2$, CO-[O$(C_1-C_6)$-Alkyl], CO-$(C_1-C_6)$- Alkyl substituiert sein können und wobei die Alkylreste mit Fluoratomen substituiert sein können;

wobei eines der drei Restepaare R7 und R8, oder R8 und R9, oder R9 und R10 jeweils gemeinsam die Gruppen -$CH_2$-$CH_2$-$CH_2$- oder -$CH_2$-$CH_2$-$CH_2$-$CH_2$- bilden kann, worin bis zu zwei -$CH_2$-Gruppen durch -O- ersetzt sein können und wobei die Gruppen -$CH_2$-$CH_2$-$CH_2$- oder -$CH_2$-$CH_2$-$CH_2$-$CH_2$- mit F, $(C_1-C_8)$-Alkyl oder =O substituiert sein können;

R11 H, $(C_1-C_8)$-Alkyl,$(C_3-C_6)$-Cycloalkyl, $(CH_2)_n$-Aryl, $(CH_2)_n$-CO-[O-$(C_1-C_6)$- Alkyl], $(CH_2)_n$-CO-[O-$(C_3-C_6)$-Cycloalkyl], $(CH_2)_n$-CO-[$(C_1-C_6)$-Alkyl], $(CH_2)_n$- CO-[$(C_3-C_6)$-Cycloalkyl], $(CH_2)_n$-CO-Aryl, $(CH_2)_n$-CO-Heteroaryl, $(CH_2)_q$-CO- $NH_2$, $(CH_2)_q$-COOH, $(CH_2)_n$-P(O)(OH)[O-$(C_1-C_4)$-Alkyl], $(CH_2)_n$-P(O)[O-$(C_1-C_4)$-Alkyl$]_2$, $(CH_2)_n$-P(O)(O-$CH_2$-Aryl$)_2$, $(CH_2)_n$-P(O)$(OH)_2$, $(CH_2)_n$-$SO_3H$, $(CH_2)_n$-SOrNH$_2$, $(CH_2)_n$-CO-NH-[$(C_1-C_4)$-Alkyl], $(CH_2)_n$-CO-N[$(C_1-C_4)$- Alkyl$]_2$, $(CH_2)_n$-CO-NH-[$(C_3-C_6)$-Cycloalkyl], $(C_2-C_6)$-Alkenyl-CO-O[$(C_1-C_4)$- Alkyl], $(C_2-C_6)$-Alkenyl-$CONH_2$, $(C_2-C_6)$-Alkenyl-COOH, $(C_2-C_6)$-Alkinyl-CO- O[$(C_1-C_4)$-Alkyl], $(C_2-C_6)$-Alkinyl-$CONH_2$, $(C_2-C_6)$-Alkinyl-COOH, $(CH_2)_n$- CR21 [(CO-O$(C_1-C_4)$-Alkyl)$]_2$, $(CH_2)_n$-CR21 $(CONH_2)_2$, $(CH_2)_n$-CR21 $(COOH)_2$, $(CH_2)_n$-CR21R22-CO-O[$(C_1-C_4)$-Alkyl], $(CH_2)_n$-CR21R22-$CONH_2$, $(CH_2)_n$- CR21R22-CO-NH-[$(C_1-C_4)$-Alkyl], $(CH_2)_n$-CR21R22-CO-N[$(C_1-C_4)$-Alkyl$]_2$, $(CH_2)_n$-CR21R22-COOH, $(CH_2)$-CO-R16, $(CH_2)_n$-CO-NH-C$(CH_3)_2$-CO-O[$(C_1-C_8)$-Alkyl], $(CH_2)_n$-CO-NH-C$(CH_3)_2$-$CONH_2$, $(CH_2)_n$-CO-NH-C$(CH_3)_2$-COOH, wobei die Alkyl-, Alkenyl-, Alkinyl- und Cycloalkylreste mit Fluoratomen substituiert sein können und wobei der Aryl- oder Heteroarylrest mit Halogen, CN, $(C_1-C_4)$-Alkyl, O-$(C_1-C_4)$-Alkyl, $S(O)_m$-$(C_1-C_4)$-Alkyl, $SO_2$-$NH_2$, COOH, $CONH_2$, CO-O$(C_1-C_4)$-Alkyl substituiert sein kann und wobei die Alkylreste mit Fluoratomen substituiert sein können;

R12 H, $(C_1-C_4)$-Alkyl, $(C_3-C_6)$-Cycloalkyl, $(CH_2)_n$-Aryl, $(CH_2)_n$-Heteroaryl, wobei die Alkyl- oder Cycloalkylreste mit Fluoratomen substituiert sein können, und wobei der Aryl- oder Heteroarylrest mit Halogen, CN, $(C_1-C_4)$-Alkyl, O-$(C_1-C_4)$-Alkyl, $SO_2$-$NH_2$, COOH, $CONH_2$, CO-O$(C_1-C_4)$-Alkyl substituiert sein kann und wobei die Alkylreste mit Fluoratomen substituiert sein können;

R13 H, $SO_2$-[$(C_1-C_4)$-Alkyl], $SO_2$-[$(C_3-C_6)$-Cycloalkyl], $SO_2$-$(CH_2)_n$-Aryl, $SO_2$-$(CH_2)_n$-Heteroaryl, wobei die Alkyl- und Cycloalkylreste mit Fluoratomen substituiert sein können und wobei der Aryl- oder Heteroarylrest mit Halogen, CN, $CF_3$, $(C_1-C_4)$-Alkyl, O-[$(C_1-C_4)$-Alkyl], $S(O)_m$-[$(C_1-C_6)$-Alkyl], $SO_2$-$NH_2$, COOH, $CONH_2$, CO- [O$(C_1-C_4)$-Alkyl], substituiert sein kann und wobei die Alkylreste mit Fluoratomen substituiert sein können;

R15 $(C_1-C_6)$-Alkyl, wobei der Alkylrest mit Fluoratomen substituiert sein kann;

R16 Aziridin-1-yl, Azetidin-1-yl, 3-Hydroxy-azetidin-1-yl, Piperidin-1-yl, Pyrrolidin-1-yl, 3-Pyrrolidinol-1-yl, Morpholin-N-yl, Piperazin-1-yl, 4-[$(C_1-C_6)$- Alkyl]piperazin-1-yl, Thiomorpholin-1,1-Dioxid-4-yl, NH-$(CH_2)_r$-OH, NH-CH$(CH_2OH)_2$, NH-C$(CH_2OH)_3$, N[$(C_1-C_4)$-Alkyl-OH$]_2$, D-Glucamin-N-yl, N- Methyl-D-Glucamin-N-yl, NH-[$(C_1-C_4)$-Alkyl]-COOH, NH-[$(C_1-C_4)$-Alkyl]- $CONH_2$, N[( $C_1-C_4$)-Alkyl] [$(C_1-C_4)$-Alkyl]-COOH, NH-[C(H)(Aryl)]-CO-O$(C_1-C_4)$-Alkyl, NH-[C(H)(Aryl)]-COOH, NH-[C(H)(Aryl)]-$CONH_2$, NH- [C(H)(Heteroaryl)]-CO-O$(C_1-C_4)$-Alkyl, NH-[C(H)(Heteroaryl)]-COOH, NH- [C(H)(Hetetoaryl)]-$CONH_2$, NH-[$(C_3-C_6)$-Cycloalkyl]-CO-O$(C_1-C_4)$-Alkyl, NH- [$(C_3-C_6)$-Cycloalkyl]-COOH, NH-[$(C_3-C_6)$-Cycloalkyl]-$CONH_2$, NH-$(C_1-C_4)$- Alkyl-OH, NH-[( $C_1-C_4$)-Alkyl]-$SO_2$-$(C_1-C_4)$-Alkyl, NH-[$(C_1-C_4)$-Alkyl]- $SO_3H$, NH-[( $C_1-C_4$)-Alkyl]-$SO_2$-$NH_2$,
wobei die Alkohol (OH) - Funktionen durch F ersetzt sein können und wobei der Aryl- oder Heteroarylrest mit Halogen, CN, $(C_1-C_4)$-Alkyl, O-$(C_1-C_4)$-Alkyl, OH, $SO_2$-$NH_2$, COOH, $CONH_2$, CO-O$(C_1-C_4)$-Alkyl substituiert sein kann;

R18 $(CH_2)_n$-CR25R26-CO-O$(C_1-C_4)$-Alkyl, $(CH_2)_n$-CR25R26-CO-$NH_2$, $(CH_2)_n$- CR25R26-COOH;

R21 H, F, $CF_3$, $(C_1-C_4)$-Alkyl, $(C_3-C_6)$-Cycloalkyl, OH, O-$(C_1-C_4)$-Alkyl, O-$(C_3-C_6)$- Cycloalkyl, O-$(CH_2)_n$-Aryl, O-(CO)-$(C_1-C_4)$-Alkyl, O-(CO)-$(C_3-C_6)$-Cycloalkyl, O-(CO)-O-$(C_1-C_4)$-Alkyl, O-(CO)-O-$(C_3-C_6)$-Cycloalkyl, NH-[$(C_1-C_4)$-Alkyl]- Aryl, $NH_2$, NH-$(C_1-C_4)$-Alkyl, NH-(CO)-$(C_1-C_4)$-Alkyl;

R22 H, $CF_3$, $(C_1-C_4)$-Alkyl, Aryl, [$(C_1-C_4)$-Alkyl]-Aryl;

R25, R26 unabhängig voneinander H, F, $(C_1-C_4)$-Alkyl, Aryl, [$(C_1-C_4)$-Alkyl]-Aryl, wobei der Aryl mit Halogen, CN, OH, O-$(C_1-C_4)$-Alkyl substituiert sein kann oder die Reste R25 und R26 bilden zusammen mit dem an sie gebundenen Kohlenstoffatom einen drei- bis siebengliedrigen Carbocyclus, bei welchem ein Kohlenstoffatom durch O, $S(O)_m$, NH, N[$(C_1-C_4)$-Alkyl] oder CO ersetzt sein kann;

A', D', E', G', L' unabhängig voneinander CH oder N, wobei bei der Bedeutung N der entsprechende Substituent R30, R31 oder R32 entfällt, wenn er an das Stickstoffatom gebunden wäre;

R30, R31, R32 unabhängig voneinander R11, F, Cl, Br, J, CN, $CF_3$, $(CH_2)_n$-O-R11, O- R13, $OCF_3$,

$(CH_2)_n$-NH-R11, $(CH_2)_n$-N[$(CH_2)_q$-CO-O($C_1$-$C_4$)-Alkyl]$_2$, $(CH_2)_n$- N[$(CH_2)_q$-COOH]$_2$, $(CH_2)_n$-N[$(CH_2)_q$-$CONH_2$]$_2$, $(CH_2)_n$-NH-R13, $(CH_2)_n$- N(R13)$_2$, $(CH_2)_n$-NH-$SO_2$-R16, $(CH_2)_n$-NH-$(CH_2)_n$-$SO_2$-R12, $(CH_2)_n$-NR12-CO-R16, (CH2)$_n$-NR12-CO-NR12R13, $(CH_2)_n$-NR12-CO-N(R12)$_2$, $(CH_2)_n$- NR12-CO-NHR11, $(CH_2)_n$-NH-C(=NH)-$NH_2$, $(CH_2)_n$-NH-C(=NH)-R16, $(CH_2)_n$-NH-C(=NH)-NHR12, $(CH_2)_n$-NH-$(CH_2)_n$ -CO-NH-[($C_1$-$C_4$)-Alkyl), $(CH_2)_n$-NH-$(CH_2)_n$ -CO-N[($C_1$-$C_4$)-Alkyl]$_2$, $(CH_2)_n$-NH-C$(CH_3)_2$-CO-O($C_1$-$C_4$)- Alkyl, $(CH_2)_n$-NH-C$(CH_3)_2$-CO-O($C_3$-$C_6$)-Cycloalkyl, $(CH_2)_n$-NH-C$(CH_3)_2$-CO- O-$(CH_2)_r$-$NH_2$, $(CH_2)_n$-NH-C$(CH_3)_2$-CO-O-$(CH_2)_n$-Aryl, $(CH_2)_n$-NH-C$(CH_3)_2$- CO-O-$(CH_2)_n$-Heteroaryl, $(CH_2)_n$-NH-C$(CH_3)_2$-CO-$NH_2$, $(CH_2)_n$-NH-C$(CH_3)_2$- CO-NH-[($C_1$-$C_4$)-Alkyl], $(CH_2)_n$-NH-C$(CH_3)_2$-CO-N[($C_1$-$C_4$)-Alkyl]$_2$, $(CH_2)_n$- NH-C$(CH_3)_2$-COOH, $S(O)_m$-R12, $SO_2$-R16, $SO_2$-N=CH-N$(CH_3)_2$, $SO_2$-NH-CO- R12, $SO_2$-NHR12, $SO_2$-N[($C_1$-$C_4$)-Alkyl]$_2$, $SF_5$, COOH, CO-$NH_2$, $(CH_2)_q$-CN, $(CH_2)_n$-CO-NH-piperidin-1-yl, $(CH_2)_n$-CO-NH-$SO_2$-NHR12, $(CH_2)_n$-CO-NH-$SO_2$-R18, $(CH_2)_n$-C(=NH)-NHOH, $(CH_2)_n$-C(=NR13)NHR12, $(CH_2)_n$- C(=NR12)NR12R13, $(CH_2)_n$-C(=$NSO_2$-R12)$NH_2$, $(CH_2)_n$-P(O)(OH)[O-($C_1$-$C_4$)- Alkyl], $(CH_2)_n$-P(O)[O-($C_1$-$C_4$)-Alkyl]$_2$, $(CH_2)_n$-P(O)(O-$CH_2$-Aryl)$_2$, $(CH_2)_n$- P(O)(OH)$_2$, wobei die Alkyl- und Cycloalkylreste mit Fluoratomen substituiert sein können und wobei die Aryl- oder Heteroarylreste mit Halogen, CN, ($C_1$- $C_4$)-Alkyl, O-($C_1$-$C_4$)-Alkyl, $S(O)_m$-($C_1$-$C_4$)-Alkyl, $SO_2$-$NH_2$, COOH, $CONH_2$, CO-O($C_1$-$C_4$)-Alkyl substituiert sein können und wobei die Alkylreste mit Fluoratomen substituiert sein können;

wobei die Verbindung N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)harnstoff ausgenommen ist,
sowie deren physiologisch verträgliche Salze.

**6.** Verbindungen der Formel Ia, gemäß Anspruch 5, **dadurch gekennzeichnet, dass** darin bedeuten

R, R' ($C_1$-$C_4$)-Alkyl;
oder R und R' bilden gemeinsam einen Ring mit drei bis acht Kohlenstoffatomen;
m 0, 1, 2;
n 0, 1, 2;
A, D, E, G, L C;
Q C=O, $SCO_2$;
R1, R2, R3, R4, R5 unabhängig voneinander H, F, Cl, Br, CN, $CF_3$, ($C_1$-$C_8$)-Cycloalkyl, O- Phenyl;
R7, R8, R9, R10 unabhängig voneinander H, F, Cl, Br, $CF_3$, -$OCH_3$;
A', E' unabhängig voneinander CH oder N, wobei bei der Bedeutung N der entsprechende Substituent R30, R31 oder R32 dann entfällt, wenn er an das Stickstoffatom gebunden wäre,
R30, R31, R32 unabhängig voneinander H, F, Cl, Br, $CF_3$, OH, $NO_2$, $NH_2$, $CONH_2$, COOH , -COO-($C_1$-$C_8$)-Alkyl, $(CH_2)_n$-P(O)(OH)[O-($C_1$-$C_4$)-Alkyl], $(CH_2)_n$- P(O)[O-($C_1$-$C_4$)-Alkyl]$_2$, $(CH_2)_n$-P(O)(OH)$_2$, $S(O)_m$-($C_1$-$C_4$)-Alkyl, $S(O)_m$-($CH_2$)- COOH, $S(O)R_m$-($CH_2$)-COO-($C_1$-$C_4$)-Alkyl, $SO_2$-Cl, $SO_2$-$NH_2$, $SO_3H$;

sowie deren physiologisch verträgliche Salze.

**7.** Verbindungen der Formel Ia, gemäß Anspruch 6, **dadurch gekennzeichnet, dass** darin bedeuten

R, R' ($C_1$-$C_4$)-Alkyl;
oder R und R' bilden gemeinsam einen Ring mit drei bis acht Kohlenstoffatomen;
m 0, 1, 2;
n 0, 1, 2;
A, D, E, G, L C;
Q C=O, $SO_2$;
R1, R2, R3, R4, R5 unabhängig voneinander H, F, Cl, Br, CN, $CF_3$, ($C_1$-$C_8$)-Cycloalkyl, O- Phenyl;
R7, R8, R9, R10 unabhängig voneinander H, F, Cl, Br, $CF_3$, $OCH_3$;
A', E' unabhängig voneinander CH oder N, wobei bei der Bedeutung N der entsprechende Substituent R30, R31 oder R32 dann entfällt, wenn er an das Stickstoffatom gebunden wäre,
R30, R31, R32 unabhängig voneinander H, F, Cl, Br, $CF_3$, OH, $NO_2$, $NH_2$, $CONH_2$, COOH , -COO-($C_1$-$C_8$)-Alkyl, $(CH_2)_n$-P(O)(OH)[O-($C_1$-$C_4$)-Alkyl], $(CH_2)_n$-P(O)[O-($C_1$-$C_4$)-Alkyl]$_2$, $(CH_2)_n$-P(O)(OH)$_2$, $S(O)_m$-($C_1$-$C_4$)- Alkyl, $S(O)_m$-($CH_2$)-COOH, $S(O)_m$-($CH_2$)-COO-($C_1$-$C_4$)-Alkyl, $SO_2$-Cl, $SO_2$-$NH_2$, $SO_3H$;

sowie deren physiologisch verträgliche Salze.

**8.** Verbindungen gemäß einem oder mehreren der Ansprüche 1 bis 7, zur Anwendung als Arzneimittel.

**9.** Arzneimittel enthaltend eine oder mehrere der Verbindungen gemäß einem oder mehreren der Ansprüche 1 bis 7.

**10.** Arzneimittel enthaltend eine oder mehrere der Verbindungen gemäß einem oder mehreren der Ansprüche 1 bis 7 und mindestens einen weiteren Wirkstoff.

**11.** Arzneimittel, gemäß Anspruch 8, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Antagonisten, MC4-Agonisten, Orexin-Antagonisten, H3-Antagonisten, TNF-Agonisten, CRF-Antagonisten, CRF BP-Antagonisten, Urocortin-Agonisten, β3-Agonisten, CB1-Rezeptor Antagonisten, MSH (Melanocyt-stimulierendes Hormon)-Agonisten, MCH-Antagonisten,CCK-Agonisten, Serotonin-Wiederaufnahme-Inhibitoren, gemischte Sertonin- und noradrenerge Verbindungen, 5HT-Modulatoren, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

**12.** Verwendung der Verbindungen gemäß einem oder mehreren der Ansprüche 1 bis 7 zur Herstellung eines Medikamentes zur Behandlung des metabolischen Syndroms.

**13.** Verwendung der Verbindungen gemäß einem oder mehreren der Ansprüche 1 bis 7 zur Herstellung eines Medikamentes zur Behandlung des Diabetes.

**14.** Verwendung der Verbindungen gemäß einem oder mehreren der Ansprüche 1 bis 7 zur Herstellung eines Medikamentes zur Behandlung von Adipositas.

**15.** Verwendung der Verbindungen gemäß einem oder mehreren der Ansprüche 1 bis 7 zur Herstellung eines Medikamentes zur Gewichtsreduktion.

**16.** Verwendung der Verbindungen gemäß einem oder mehreren der Ansprüche 1 bis 7 zur Herstellung eines Medikamentes zur Behandlung von Nikotinabhängigkeit.

**17.** Verwendung der Verbindungen gemäß einem oder mehreren der Ansprüche 1 bis 7 zur Herstellung eines Medikamentes zur Behandlung von Alkoholabhängigkeit.

**18.** Verwendung der Verbindungen gemäß einem oder mehreren der Ansprüche 1 bis 7 zur Herstellung eines Medikamentes zur Behandlung von ZNS-Erkrankungen.

**19.** Verwendung der Verbindungen gemäß einem oder mehreren der Ansprüche 1 bis 7 zur Herstellung eines Medikamentes zur Behandlung von Schizophrenie.

**20.** Verwendung der Verbindungen gemäß einem oder mehreren der Ansprüche 1 bis 7 zur Herstellung eines Medikamentes zur Behandlung von Alzheimer.

**21.** Verfahren zur Herstellung eines Arzneimittels enthaltend eine oder mehrere der Verbindungen gemäß einem oder mehreren der Ansprüche 1 bis 7, **dadurch gekennzeichnet, dass** der Wirkstoff mit einem pharmazeutisch geeigneten Träger vermischt wird und diese Mischung in eine für die Verabreichung geeignete Form gebracht wird.

## Claims

**1.** A compound of the formula I

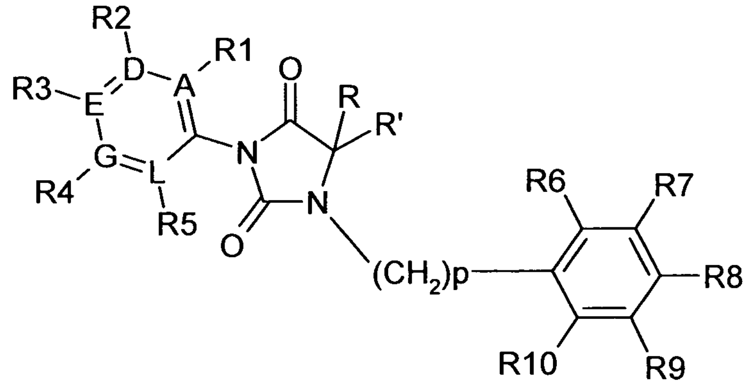

I

in which

R, R' are each independently H, $(CH_2)_n$-aryl, $(C_1-C_6)$- alkyl, where $(C_1-C_6)$-alkyl or the aryl radical may be substituted by halogen, O-R14, $S(O)_m$- R12 or NR13R15;
or R and R' together form a ring having from three to eight carbon atoms, where one carbon atom may be replaced by 0, $S(O)_m$, NR13 or NR15;
m is 0, 1, 2;
n is 0, 1, 2, 3, 4;
p is 1, 2, 3, 4, 5;
q is 1, 2, 3, 4;
r is 2, 3, 4, 5, 6;
v is 0, 1, 2, 3, 4;
A, D, E, are each independently C or N, where, when they are defined as N, the corresponding substituent R1, R2, R3, R4, R5 is absent,
G, L or R2-D=E-R3 or R4-G=L-R5 are defined as S or O and where the five-membered or six-membered ring may be fused to - $(CH_2)_3$- or - $(CH_2)_4$- or -CH=CH-CH=CH- to form a bicyclic system;
R1, R2, R3, R4, R5 are each independently H, F, Cl, Br, I, CN, $N_3$, NC, $NO_2$, $CF_3$, $(C_1-C_8)$ -alkyl, $(C_3-C_8)$ -cycloalkyl, $(CH_2)_q$- [$(C_3-C_8)$- cycloalkyl], $(CH_2)_n$-[$(C_3-C_8)$ -cycloalkenyl] , $(CH_2)_n$- [$(C_7-C_{12})$ -bicycloalkyl] , $(CH_2)_n$- [$(C_7-C_{12})$- tricycloalkyl], adamantan-1-yl, adamantan-2- yl, $(CH_2)_n$-aryl, $(CH_2)_n$-heteroaryl, $OCF_3$, O-R11, NR13R15, NH-CN, $S(O)_m$-R12, $SO_2$-$NH_2$, $SO_2$- N=CH-N $(CH_3)_2$, $SO_2$-NH- [$(C_1-C_8)$ -alkyl] , $SO_2$-NH- [ $(C_3-C_8)$ -cycloalkyl] , $SO_2$-NH- $(CH_2)_n$-aryl , $SO_2$- NH- $(CH_2)_n$-heteroaryl, $SO_2$-N [$(C_1-C_8)$ -alkyl]$_2$, $SO_2$-R16, $SF_5$, CO-O [$(C_1-C_8)$ -alkyl] , CO-O[$(C_3-C_8)$-cycloalkyl] , CO-O-$(CH_2)_n$-aryl, CO- O-$(CH_2)_n$-heteroaryl, CO-$NH_2$, CO-NH-CN, CO-NH- [ $(C_1-C_8)$ -alkyl] , CO-N [$(C_1-C_8)$ -alkyl]$_2$, CO-NH- [ $(C_3-C_8)$ -cycloal kyl ] , CO-N [$(C_3-C_8)$-cycloalkyl]$_2$, C(=NH)-O-[$(C_1-C_6$-alkyl)], C(=NH)-$NH_2$, C (=NH) -R16, C (=NR13) -NR12R13, $(CH_2)_n$-C(=$NSO_2$-R12) $NH_2$, CO-R16, COOH, CO- ($C_1$- C8) -alkyl, CO- $(C_3-C_8)$ -cycloalkyl, CO-aryl, CO- heteroaryl, CH(OH)-aryl, CH(OH)-heteroaryl, CH [O- $(C_1-C_6)$ -alkyl] -aryl, CH[O- $(C_1-C_6)$-alkyl]- heteroaryl, CHF-aryl, CHF-heteroaryl, $CF_2$- aryl, $CF_2$-heteroaryl, CHO, $CH_2$-OH, $CH_2$-CN, $CH_2$- O-R12 , $CH_2$-O- $(CH_2)_n$-CO-O [$(C_1-C_8)$ -alkyl] , $CH_2$-O- $(CH_2)_n$-CO-$NH_2$, $CH_2$-O- $(CH_2)_q$-COOH,
where the alkyl, cycloalkyl, cycloalkenyl, bicycloalkyl and tricycloalkyl radicals may be substituted by fluorine atoms, and where the aryl or heteroaryl radicals may be substituted by halogen, CN, $(C_1-C_6)$-alkyl, $(C_3-C_6)$ -cycloalkyl, O- $(C_1-C_6)$ -alkyl, $OCF_3$, OH, O- $(CH_2)_n$-aryl, $(CH_2)_n$-aryl, S $(O)_m$- $(C_1-C_6)$ -alkyl, $SO_2$-$NH_2$, SH, NR12R13, NH-CO- [$(C_1-C_6)$ -alkyl] , NH-CO- $(CH_2)_n$-aryl, $(CH_2)_n$-COOH, $(CH_2)_n$-$CONH_2$, $(CH_2)_n$-CO-O $(C_1-C_6)$ -alkyl, $(CH_2)_n$-CO- $(C_1-C_6)$- alkyl, and where the alkyl radicals may be substituted by fluorine atoms;
R6, R7, R8, R9, R10 are each independently T-bicyclic heterocycle, T-aryl or T-heteroaryl, where the bicyclic

heterocycle or the aryl or heteroaryl radical may be fused to a 5- or 6-membered aromatic or nonaromatic carbon ring in which one or more CH or $CH_2$ groups may be replaced by oxygen atoms and where the 5- or 6-membered aromatic or nonaromatic carbon ring may be substituted by F, =O or -($C_1$-$C_6$)- alkyl and where the bicyclic heterocycle may contain from 9 to 12 ring members and up to five CH or $CH_2$ groups may each independently be replaced by N, NR20, O, $S(O)_m$ or C=O and where the aryl or heteroaryl radical or bicyclic may heterocycle may be unsubstituted or be mono- or polysubstituted by

R11, F, Cl, Br, I, CN, $N_3$, NC, $NO_2$, $CF_3$, $(CH_2)_n$-O-R11, $(CH_2)_n$-O- $(CH_2)_r$-OH, $(CH_2)_n$- O-CH $(CH_2OH)_2$, $(CH_2)_n$-O- $(CH_2)_n$-CO-O- $(CH_2)_r$- $NH_2$, $(CH_2)_n$-O- $(CH_2)_n$-CO-NH- $(CH_2)_r$-OH, O- R13, $OCF_3$, $(CH_2)_n$-O-$(CH_2)_r$-$NH_2$, $(CH_2)_n$-NH- R11, $(CH_2)_n$-N[$(CH_2)_q$-CO-O ($C_1$-$C_6$) -alkyl]$_2$, $(CH_2)_n$-N [ $(CH_2)_q$-COOH ]$_2$, $(CH_2)_n$-N [ $(CH_2)$q- $CONH_2$]$_2$, $(CH_2)_n$-NH-R13, $(CH_2)_n$-N $(R13)_2$, $(CH_2)_n$-NH-CN, $(CH_2)_n$-NH-$SO_2$-R16, $(CH_2)_n$- NH- $(CH_2)_n$-$SO_2$-R12, $(CH_2)_n$-NR12-CO-R16, $(CH_2)_n$-NR12-CO-NR12R13, $(CH_2)_n$-NR12-CO- N $(R12)_2$, $(CH_2)_n$-NR12-CO-NHR11, $(CH_2)_n$-NH- C (=NH) -$NH_2$, $(CH_2)_n$-NH-C (=NH) -R16, $(CH_2)_n$-NH-C(=NH)-NHR12, $(CH_2)_n$-NR12-C(=NR13)- NHR12, $(CH_2)_n$-NR12-C(=NR12)-NR12R13, $(CH_2)_n$-NH-$(CH_2)_n$-CO-O- $(CH_2)$r-NH2 , $(CH_2)_n$- NH- $(CH_2)$n -CO-NH-[($C_1$-$C_8$)-alkyl], $(CH_2)_n$- NH- $(CH_2)_n$-CO-NH-$(CH_2)_r$-OH, $(CH_2)_n$-NH- $(CH_2)_n$ -CO-N [($C_1$-$C_8$-alkyl]$_2$, $(CH_2)_n$-NH- $(CH_2)_n$ -CO-NH- [ ($C_3$-$C_8$) -cycloalkyl] , $(CH_2)_n$-NH- $(CH_2)_n$ -CO-N [ ($C_3$-$C_8$)- cycloalkyl]$_2$, $(CH_2)_n$-NH-C $(CH_3)_2$-CO-O ($C_1$- $C_8$) -alkyl, $(CH_2)_n$-NH-C $(CH_3)_2$-CO-O ($C_3$-$C_8$)- cycloalkyl, $(CH_2)_n$-NH-C $(CH_3)_2$-CO-O- $(CH_2)_r$-$NH_2$ , $(CH_2)_n$-NH-C $(CH_3)_2$-CO-O-$(CH_2)_n$- aryl, $(CH_2)_n$-NH-C $(CH_3)_2$-CO-O- $(CH_2)$n- heteroaryl, $(CH_2)_n$-NH-C $(CH_3)_2$-CO-$NH_2$, $(CH_2)_n$-NH-C $(CH_3)_2$-CO-NH- [ ($C_1$-$C_8$) -al kyl ] , $(CH_2)_n$-NH-C $(CH_3)_2$-CO-NH- $(CH_2)_r$-OH, $(CH_2)_n$- NH-C $(CH_3)_2$-CO-N [($C_1$-$C_8$) -alkyl ] $_2$, $(CH_2)_n$- NH- $(CH_3)_2$-CO-NH- [($C_3$-$C_8$) -cycloalkyl] , $(CH_2)_n$-NH-C $(CH_3)_2$-CO-N [ ($C_3$-$C_8$)-cycloalkyle]$_2$, $(CH_2)_n$-NH-C $(CH_3)_2$-COOH, S $(O)_m$-R12, $SO_2$-R16, $SO_2$-N=CH-N $(CH_3)_2$,

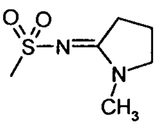
,

$SO_2$-NH-CO-R12 , $SO_2$-NHR12, $SO_2$-NH- $(CH_2)_r$-OH, $SO_2$-N [ ($C_1$-$C_8$) -alkyl ]$_2$, $SO_2$-NH-$(CH_2)_r$-$NH_2$, $SF_5$, COOH, CO-$NH_2$, $(CH_2)_q$-CN, $(CH_2)_n$-CO-NH-CN , $(CH_2)_n$-CO-NH- piperidin-1-yl, $(CH_2)_n$-CO-NH-$SO_2$-NHR12, $(CH_2)_n$-CO-NH-$SO_2$-R18 , $(CH_2)_n$-CHO, $(CH_2)_n$- C (=NH) -$NH_2$, $(CH_2)_n$-C (=NH) -NHOH, $(CH_2)_n$- C (=NH) - [NH-O- ($C_1$-$C_6$) -alkyl] , $(CH_2)_n$- C (=NH) (R16) , $(CH_2)_n$-C (=NR13) NHR12, $(CH_2)_n$-C (=NR12) NR12R13, $(CH_2)_n$-C (=$NSO_2$- R12)$NH_2$, $(CH_2)_n$-C(=NH)O[ ($C_1$-$C_6$)-alkyl], where the alkyl and cycloalkyl radicals may be substituted by fluorine atoms and where the aryl or heteroaryl radicals may be substituted by halogen, CN, ($C_1$-$C_6$) -alkyl, ($C_3$-$C_6$) -cycloalkyl, O- ($C_1$-$C_6$) -alkyl, S $(O)_m$- ($C_1$-$C_6$) -alkyl, $SO_2$- $NH_2$, COOH, $CONH_2$, CO-O ($C_1$-$C_6$) -alkyl, CO- ($C_1$-$C_6$)-alkyl and where the alkyl radicals may be substituted by fluorine atoms,

and where, when R3 is CN, $NO_2$ or halogen and R4 is $CF_3$ or halogen and R and R' are each methyl, the X-aryl radical is provided with at least one of the abovementioned substituents other than hydrogen;

H, F, Cl, Br, I, CN, $N_3$, NC, $NO_2$, $CF_3$, ($C_1$-$C_8$)-alkyl, ($C_2$-$C_{10}$)-alkenyl, ($C_2$-$C_{10}$)- alkynyl, ($C_3$-$C_8$) -cycloalkyl, aryl, heteroaryl,

$(CH_2)_n$-CO-[O-($C_1$-$C_8$)-alkyl], $(CH_2)_n$-CO-[O-($C_3$- $C_8$)-cycloalkyl], $(CH_2)_n$-CO-[($C_1$-$C_8$)-alkyl], $(CH_2)_n$-CO-[($C_3$-$C_8$)-cycloalkyl],

$(CH_2)_n$-CO-[O-$(CH_2)_v$-aryl],

$(CH_2)_n$-CO-NH2, $(CH_2)_n$-COOH, $(CH_2)_n$-CO-NH-CN,

$(CH_2)_n$-P(O)(OH)[O-($C_1$-$C_6$) -alkyl], $(CH_2)_n$- P(O)[O-($C_1$-$C_6$)-alkyl]$_2$, $(CH_2)_n$-P(O)(OH)(O-$CH_2$- aryl) , $(CH_2)_n$-P(O)(O-$CH_2$-aryl)$_2$, $(CH_2)_n$- P(O)$(OH)_2$,

$(CH_2)_n$-$SO_3H$, $(CH_2)_n$-$SO_2$-$NH_2$,

$(CH_2)_n$-CO-NH- [ ($C_1$-$C_8$) -alkyl] , $(CH_2)_n$-CO-N [ ($C_1$- $C_8$) -alkyl]$_2$, $(CH_2)_n$-CO-NH-[($C_3$-$C_8$) -cycloalkyl] , ($C_2$-$C_{10}$) -alkenyl-CO-O [($C_1$-$C_6$) -alkyl] , ($C_2$-$C_{10}$)- alkenyl-$CONH_2$, ($C_2$-$C_{10}$) -alkenyl-COOH, ($C_2$-$C_{10}$)- alkynyl-CO-O [ ($C_1$-$C_6$) -alkyl] , ($C_2$-$C_{10}$) -alkynyl- $CONH_2$, ($C_2$-$C_{10}$)-alkynyl-COOH,

$(CH_2)_n$-CO-R16,

$(CH_2)_n$-OH, $(CH_2)_n$-O- ($C_1$-$C_8$) -alkyl, $(CH_2)_n$-O- ($C_2$- $C_{10}$) -alkenyl, $(CH_2)_n$-O- ($C_2$-$C_{10}$) -alkynyl, $(CH_2)_n$- O-($C_3$-$C_8$) -cycloalkyl, $(CH_2)_n$-O- $(CH_2)$q- [ ($C_3$-$C_8$)- cycloalkyl] , $(CH_2)_n$-O-$(CH_2)_n$-CO-[O-($C_1$-$C_8$)- alkyl], $(CH_2)_n$-O-$(CH_2)_n$-CO-[O-($C_3$-$C_8$)- cycloalkyl] , $(CH_2)_n$-O-$(CH_2)_n$-CO-[($C_1$-$C_8$)- alkyl], $(CH_2)_n$-O-$(CH_2)_n$-CO-[($C_3$-$C_8$)-cycloalkyl] , $(CH_2)_n$-O-$(CH_2)_n$-CO-[O-$(CH_2)_v$- aryl] , $(CH_2)_n$-O- $(CH_2)_n$-CO- [O- $(CH_2)_v$- heteroaryl] ,

$(CH_2)_n$-O-$(CH_2)_q$-CO-$NH_2$, $(CH_2)_n$-O- $(CH_2)_q$-COOH, $(CH_2)_n$-O- $(CH_2)_q$-CO-NH-CN, $(CH_2)_n$-O- $(CH_2)_n$-P(O)(OH)[O- $(C_1$-$C_6)$ -alkyl], $(CH_2)_n$-O- $(CH_2)_n$-P (O) [O- $(C_1$-$C_6)$ -alkyl]$_2$, $(CH_2)_n$-O- $(CH_2)_n$- P (O) (OH) (O-$CH_2$-aryl) , $(CH_2)_n$-O- $(CH_2)_n$-P (O) (O- $CH_2$-aryl)$_2$, $(CH_2)_n$-O- $(CH_2)_n$-P (O) $(OH)_2$, $(CH_2)_n$-O- $(CH_2)_n$-$SO_3H$, $(CH_2)_n$-O- $(CH_2)_n$-$SO_2$-$NH_2$, $(CH_2)_n$-O- $(CH_2)_n$-CO-NH-[$(C_1$-$C_8)$-alkyl] , $(CH_2)_n$-O-$(CH_2)_n$- CO-N [ $(C_1$-$C_8)$ -alkyl]$_2$, $(CH_2)_n$-O- $(CH_2)_n$-CO-NH- [ $(C_3$-$C_8)$-cycloalkyl] , $(CH_2)_n$-O- $(CH_2)_n$-CR21R22- CO-O[$(C_1$-$C_6)$-alkyl] , $(CH_2)_n$-O-$(CH_2)_n$-CR21R22- CONH$_2$, $(CH_2)_n$-O- $(CH_2)_n$-CR21R22-COOH , $(CH_2)_n$-O- $(CH_2)_n$-CO-R16, $(CH_{2\ n}$-O- $(CH_2)_r$-OH, $(CH_2)_n$-O- CH $(CH_2OH)_2$, (-$CH_2)_n$-O- $(CH_2)_n$-CO-O- $(CH_2)_r$-$NH_2$ , $(CH_2)_n$-O-$(CH_2)_n$-CO-NH-$(CH_2)_r$-OH, O-R13 , $OCF_3$,

$(CH_2)_n$-$NH_2$, $(CH_2)_n$-NH- $(C_1$-$C_8)$ -alkyl, $(CH_2)_n$-NH- $(C_3$-$C_8)$ -cycloalkyl, $(CH_2)_n$-NH- $(CH_2)_n$-CO- [O- $(C_3$- $C_8)$ -cycloalkyl] , $(CH_2)_n$-NH- $(CH_2)_n$-CO- [ $(C_1$-$C_8)$ - alkyl], (-$CH_2)_n$-NH-$(CH_2)_n$-CO- [$(C_3$-$C_8)$ - cycloalkyl] , (-$CH_2)_n$-NH-$(CH_2)_n$-CO- [O-$(CH_2)_v$- aryl] , (($CH_2)_n$-NH- $(CH_2)_n$-CO- [O- $(CH_2)_v$- heteroaryl] , $(CH_2)_n$-NH-$(CH_2)_q$-CO-NH-CN,

$(CH_2)_n$-NH- $(CH_2)_n$-P (O) $(OH)_2$ ,

$(CH_2)_n$-NH- $(CH_2)_n$-$SO_3H$,

$(CH_2)_n$-NH- $(CH_2)_n$-$SO_2$-$NH_2$,

$(CH_2)_n$-NH- $(CH_2)_n$-CR21R22-CO-O [$(C_1$-$C_6)$ -alkyl] ,

$(CH_2)_n$-NH- $(CH_2)_n$-CR21R22-CONH$_2$, $(CH_2)_n$-NH- $(CH_2)_n$-CR21R22-COOH,

$(CH_2)_n$-NH- $(CH_2)_n$-CO-R16 ,

$(CH_2)_n$-NH- $(CH_2)_n$-$SO_2$- [ $(C_1$-$C_8)$ -alkyl] , $(CH_2)_n$-NH- $(CH_2)_n$-$SO_2$- [$(C_3$-$C_8)$ -cycloalkyl] , $(CH_2)_n$-NH-$SO_2$-$(CH_2)_n$-$NH_2$, $(CH_2)_n$-NH-$SO_2$-$(CH_2)_n$-NH-$(C_1$-$C_8)$- alkyl, $(CH_2)_n$-NH-$SO_2$-$(CH_2)_n$-NH-$(C_3$-$C_8)$- cycloalkyl, $(CH_2)_n$-NH-$SO_2$-$(CH_2)_n$-N[$(C_1$-C8)- alkyl]$_2$,

$(CH_2)_n$-NH-CN, $(CH_2)_n$-NH-$SO_2$-R16,

$(CH_2)_n$-NR12-CO-NH- $(C_1$-$C_8)$ -alkyl, $(CH_2)$ $_n$-NR12- CO-NH- $(C_3$-$C_8)$ -cycloalkyl, $(CH_2)_n$-NR12-CO-$NH_2$, $(CH_2)_n$-NR12-CO-NH-$SO_2$- $(C_1$-$C_8)$ -alkyl, $(CH_2)_n$- NR12-CO-NH-$SO_2$- $(C_3$-$C_8)$ -cycloalkyl, $(CH_2)_n$-NR12-CO-N[$(C_1$-$C_8)$-alkyl]$_2$, $(CH_2)$n-NH-CO-NH- $(CH_2)_n$- CO-[O-$(C_1$-$C_8)$-alkyl] , $(CH_2)$n-NH-CO-NH- $(CH_2)_q$-CO-$NH_2$, $(CH_2)$n-NH-CO-NH- $(CH_2)_q$-COOH, $(CH_2)_n$- NH-C ( =NH ) -$NH_2$, $(CH_2)_n$-NH-C ( =NH ) -R16 , $(CH_2)_n$-NH- C (=NH)-NH[$(C_1$-$C_8)$-alkyl], (CHF2)$_n$-NH-C(=N-$SO_2$- $(C_1$-$C_8)$-alkyl) -$NH_2$, $(CH_2)_n$-NH-C(=N-$SO_2$-$(C_1$-$C_8)$-alkyl)-NH[$(C_1$-$C_8)$-alkyl], $(CH_2)_n$-NH-C(=N-$SO_2$- $NH_2$)-$NH_2$, $(CH_2)_n$-NH-C(=N-$SO_2$-$NH_2$)-NH[$(C_1$-$C_8)$- alkyl] , $(CH_2)_n$-NH-C(=NH) -N [$(C_1$-$C_8)$ -alkyl]$_2$, $(CH_2)_n$-NH-C(=N-$SO_2$-$(C_1$-$C_8)$-alkyl)-N[$(C_1$-$C_8)$- alkyl]$_2$, $(CH_2)_n$-NH-$(CH_2)_n$-CO-O-$(CH_2)_r$-$NH_2$, $(CH_2)_n$-NH-$(CH_2)_n$ -CO-NH-[$(C_1$-$C_8$-alkyl], $(CH_2)_n$- NH-$(CH_2)_n$ -CO-NH- $(CH_2)_r$-OH, $(CH_2)_n$-NH-$(CH_2)_n$- CO-N [$(C_1$-$C_8)$ -alkyl]$_2$, $(CHF_2)_n$-NH-$(CHF_2)_n$ -CO-NH- [$(C_3$-$C_8)$-cycloalkyl], $(CH_2)_n$-NH-C$(CH_3)_2$-CO-O$(C_3$- $C_8)$-cycloalkyl, $(CH_2)_n$-NH-C$(CH_3)_2$-CO-O-$(CH_2)_r$- $NH_2$, $(CH_2)_n$-NH-C$(CH_3)_2$-CO-O-$(CH_2)_n$-aryl, $(CH_2)_n$- NH-C$(CH_3)$2-CO-O-$(CH_2)_n$-heteroaryl, (-$CH_2)_n$-NH- C $(CH_3)_2$-CO-NH-[$(C_1$-$C_8)$-alkyl], $(CH_2)_n$-NH-C $(CH_3)_2$-CO-NH-$(CH_2)$ $_r$-OH, (-$CH_2)_n$-NH-C$(CH_3)_2$-CO- N[$(C_1$-$C_8)$-alkyl]$_2$, (-$CH_2)_n$-NH-$(CH_3)_2$-CO-NH-[$(C_3$-$C_8)$-cycloalkyl], $(CH_2)_n$-NH-C$(CH_3)_2$-CO-N[$(C_3$-$C_8)$- cycloalkyle]$_2$,

$(CH_2)_n$-S$(O)_m$-$(C_1$-$C_8)$-alkyl, $(CH_2)_n$-S$(O)_m$-$(C_3$-$C_8)$- cycloalkyl, $(CH_2)_n$-$SO_2$-R16, $SO_2$-N=CH-N$(CH_3)_2$,

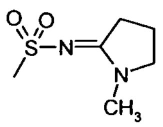
,

$(CH_2)_n$-$SO_2$-NH-CO-$(C_1$-$C_8)$-alkyl, $(CH_2)_n$-$SO_2$-NH-CO-$(C_3$-$C_8)$-cycloalkyl, $(CH_2)$n-$SO_2$- NH- $(C_1$-$C_8)$-alkyl, $(CH_2)_n$-$SO_2$-NH-$(C_3$-$C_8)$- cycloalkyl, $(CH_2)_n$-$SO_2$-N[$(C_1$-$C_8)$-alkyl]$_2$, $SO_2$- NH-$(CH_2)_r$-OH, $SO_2$-NH-$(CH_2)$ $_r$-$NH_2$, $SF_5$,

$(CH_2)_q$-CN,

$(CH_2)_n$-CO-NH-piperidin-1-yl,

$(CH_2)_n$-CO-NH-$SO_2$-NHR12, $(CH_2)_n$-CO-NH-$SO_2$-$(C_1$- $C_8)$-alkyl, $(CH_2)_n$-CO-NH-$SO_2$-$(C_3$-$C_8)$-cycloalkyl, $(CH_2)_n$-CHO, $(CH_2)_n$-C(=NH)$NH_2$, $(CH_2)_n$-C(=NH)NHOH, $(CH_2)_n$-C(=NH) (R16), $(CH_2)_n$-C(=NR13)NHR12, $(CH_2)_n$-C(=NR12) NR12R13, $(CH_2)_n$-C(=NH)O[$(C_1$-$C_6)$- alkyl], where the alkyl and cycloalkyl radicals may be substituted by fluorine atoms and where the aryl or heteroaryl radicals may be substituted by halogen, CN, $(C_1$-$C_6)$-alkyl, $(C_3$-$C_6)$-cycloalkyl, O-$(C_1$-$C_6)$-alkyl, S$(O)_m$-$(C_1$-$C_6)$-alkyl, $SO_2$-$NH_2$, COOH, CONH$_2$, CO-[O$(C_1$-$C_6)$-alkyl], CO-$(C_1$-$C_6)$-alkyl and where the alkyl radicals may be substituted by fluorine atoms;

where at least one of the R6, R7, R8, R9 and R10 radicals is always defined as T-bicyclic heterocycle, T-aryl or T-heteroaryl and

where one of the four radical pairs of R6 and R7, or R7 and R8, or R8 and R9, or R9 and R10 may in each case together form the $-CH_2-CH_2-CH_2-$ or $-CH_2-CH_2$ $-CH_2-CH_2-$ groups in which up to two $-CH_2-$ groups may be replaced by -O- and where the $-CH_2-CH_2-CH_2-$ or $-CH_2-CH_2-CH_2-CH_2-$ groups may be substituted by F, $(C_1-C_8)$-alkyl or =O;

T is NR23-CO-NR24, NR23-$SO_2$-NR24, $SO_2$-NR23-$SO_2$, CO-NR23-CO, NR23-C(=NR13)-NR24, NR23-C(=NR22)-NR24, CO-NR23-CR22R23, CO-CR22R23- CO, CR22R23-CO-CR22R24, NR23-CO-CR22R24, NR23-$SO_2$-CR22R24, CR22R24-CO-NR23, CR22R24- $SO_2$-NR23, CR22R23-NR23-$SO_2$, $SO_2$-CR22R23-NR23, $SO_2$-NR23-CR22R23-, NR23-CR22R23-$SO_2$, CO-NR23- $SO_2$, $SO_2$-NR23-CO, CO-CR22R23-$SO_2$, $SO_2$-CR22R23- CO, CR23R24-CR23R24-CR23R24, CR23R24-NR23- CR23R24;

R11 is H, $(C_1-C_8)$-alkyl, $(C_2-C_{10})$-alkenyl, $(C_2-C_{10})$- alkynyl, $(C_3-C_8)$ -cycloalkyl, $(CH_2)$q-[ $(C_3-C_8)$- cycloalkyl], $(CH_2)_n$-aryl, $(CH_2)_n$-CO-[O-$(C_1-C_8)$- alkyl] , $(CH_2)_n$-CO-[O-$(C_3-C_8)$-cycloalkyl], $(CH_2)_n$-CO-[$(C_1-C_8)$-alkyl], $(CH_2)_n$-CO-[$(C_3-C_8)$- cycloalkyl] , $(CH_2)_n$-CO-aryl, $(CH_2)_n$-CO- heteroaryl, $(CH_2)_q$-CO-$NH_2$, $(CH_2)_q$-COOH, $(CH_2)$n- P(O) (OH) [O-$(C_1-C_6)$-alkyl], $_{(CH2)}{}_n$-P(O) [O-$(C_1$- $C_6)$-alkyl]$_2$, $(CH_2)_n$-P(O)(OH) (O-$CH_2$-aryl), $(CH_2)_n$-P(O) (O-$CH_2$-aryl)$_2$. $(CH_2)_n$-P(O) $(OH)_2$, $(CH_2)_n$-$SO_3H$, $(CH_2)_n$-$SO_2$-$NH_2$, $(CH_2)_n$-CO-NH-[$(C_1-C_8)$-alkyl], $(CH_2)_n$-CO-N[$(C_1-C_8)$-alkyl]$_2$, $(CH_{2)n}$- CO-NH- [$(C_3-C_8)$-cycloalkyl], $(C_2-C_{10})$-alkenyl-CO-O[$(C_1-C_6)$-alkyl], $(C_2-C_{10})$-alkenyl-$CONH_2$, $(C_2-C_{10})$-alkenyl-COOH, $(C_2-C_{10})$-alkynyl-CO- O [$(C_1-C_6)$-alkyl], $(C_2-C_{10})$-alkynyl-$CONH_2$, $(C_2$- $C_{10})$-alkynyl-COOH, $(CH_2$ $)_n$-CR21[(CO-O$(C_1-C_6)$- alkyl)]$_2$, $(CH_2)_n$-CR21$(CONH_2)_2$, $(CH_2)_n$- CR21$(COOH)_2$, $(CH_2)_n$-CR21R22-CO-O[$(C_1-C_6)$- alkyl] , $(CH_2)_n$-CR21R22-$CONH_2$, $(CH_2)_n$-CR21R22- CO-NH-[$(C_1-C_8)$-alkyl], $(CH_2)_n$-CR21R22-CO-N[$(C_1$- $C_8)$-alkyl]$_2$, $(CH_2)_n$-CR21R22-COOH, $(CH_2)_n$-CO- R16, $(CH_2)_n$-CO-NH-C$(CH_3)_2$-CO-O[$(C_1-C_8)$ -alkyl], $(CH_2)_n$-CO-NH-C$(CH_3)_2$-$CONH_2$, $(CH_2)_n$-CO-NH- C$(CH_3)_2$-COOH, where the alkyl, alkenyl, alkynyl and cycloalkyl radicals may be substituted by fluorine atoms and where the aryl or heteroaryl radical may be substituted by halogen, CN, $(C_1-C_6)$-alkyl, $(C_3-C_6)$- cycloalkyl, O-$(C_1-C_6)$-alkyl, S $(O)_m$-$(C_1-C_6)$- alkyl, $SO_2$-$NH_2$, COOH, $CONH_2$, CO-O$(C_1-C_6)$-alkyl, CO-$(C_1-C_6)$-alkyl and where the alkyl radicals may be substituted by fluorine atoms;

R12 is H, $(C_1-C_8)$ -alkyl, $(C_3-C_8)$ -cycloalkyl, $(CH_2)_n$-aryl, $(CH_2)_n$-heteroaryl, where the alkyl or cycloalkyl radicals may be substituted by fluorine atoms,

and where the aryl or heteroaryl radical may be substituted by halogen, CN, $(C_1-C_6)$-alkyl, O-$(C_1-C_6)$-alkyl, $SO_2$-$NH_2$, COOH, $CONH_2$, CO-O$(C_1$- $C_6)$-alkyl, CO-$(C_1-C_6)$-alkyl and where the alkyl radicals may be substituted by fluorine atoms;

R13 is H, $SO_2$-[$(C_1-C_8)$-alkyl], $SO_2$-[$(C_3-C_8)$ cycloalkyl], $SO_2$-$(CH_2)_n$-aryl, $SO_2$- $(CH_2)_n$-heteroaryl,

where the alkyl and cycloalkyl radicals may be substituted by fluorine atoms and where the aryl or heteroaryl radical may be substituted by halogen, CN, $CF_3$, $(C_1-C_6)$- alkyl, $(C_3-C_6)$-cycloalkyl, O-[$(C_1-C_6)$-alkyl] , $S(O)_m$-[$(C_1-C_6)$-alkyl], $SO_2$-$NH_2$, COOH, $CONH_2$, CO-[O$(C_1-C_6)$-alkyl], CO-$(C_1-C_6)$-alkyl and where the alkyl radicals may be substituted by fluorine atoms;

R14 is H, $(C_1-C_8)$-alkyl, $(C_3-C_8)$ -cycloalkyl, $(CH_2)_n$-aryl, $(CH_2)_n$-heteroaryl, $(CH_2)_n$-CO-[O- $(C_1-C_8)$-alkyl], $(CH_2)_n$-CO-[O-$(CH_2$ $)_n$-aryl], $(CH_2)_n$-CO-[$(C_1-C_8)$-alkyl], $(CH_2)_n$-CO-[$(C_3-C_8)$- cycloalkyl], $(CH_2)_n$-CO-aryl, $(CH_2)$ n-CO- heteroaryl, $(CH_2)_q$-COOH,

where the alkyl and cycloalkyl radicals may be substituted by fluorine atoms and where the aryl or heteroaryl radical may be substituted by halogen, CN, $(C_1-C_6)$-alkyl, $(C_3-C_6)$ -cycloalkyl, O-$(C_1-C_6)$-alkyl, $S(O)_m$-$(C_1-C_6)$-alkyl, $SO_2$-$NH_2$, COOH, $CONH_2$, CO-O$(C_1-C_6)$- alkyl, CO-$(C_1-C_6)$-alkyl and where the alkyl radicals may be substituted by fluorine atoms;

R15 is H, $(C_1-C_8)$-alkyl, $(C_3-C_8)$-cycloalkyl, $(CH_2)_n$-aryl, $(CH_2)_n$-heteroaryl, $(CH_2)_n$-CO-[O- $(C_1-C_8)$-alkyl], CO-[$(C_1-C_8)$-alkyl], CO-[ $(C_3$- C8)-cycloalkyl], CO-aryl, CO-heteroaryl, $(CH_2)_n$-CO-$NH_2$, $(CH_2)_q$-COOH, $(CH_2)_n$-$SO_2$-$NH_2$, $(CH_2)_n$-C$(CH_3)_2$-COOH,

where the alkyl and cycloalkyl radicals may be substituted by fluorine atoms and where the aryl or heteroaryl radical may be substituted by halogen, CN, $(C_1-C_6)$-alkyl, O- $(C_1-C_6)$-alkyl, $SO_2$-$NH_2$, COOH, $CONH_2$, CO-O$(C_1-C_6)$-alkyl, CO-$(C_1-C_6)$-alkyl

and where the alkyl radicals may be substituted by fluorine atoms;

R16 is aziridin-1-yl, azetidin-1-yl, 3- hydroxyazetidin-1-yl, piperidin-1-yl, pyrrolidin-1-yl, 3-pyrrolidinol-1-yl, morpholin-N-yl, piperazin-1-yl, 4-[$(C_1-C_6)$- alkyl]piperazin-1-yl, thiomorpholin-4-yl, thiomorpholine-1,1-dioxid-4-yl, NH-$(CH_2)_r$-OH, NH-CH$(CH_2OH)_2$, NH-C$(CH_2OH)_3$, N[$(C_1-C_6)$-alkyl- OH]$_2$, N[$(C_1-C_6)$-alkyl] [$(C_1-C_6)$-alkyl-OH], D- glucamin-N-yl, N-methyl-D-glucamin-N-yl, NH- [$(C_1-C_8)$-alkyl]-CO-O$(C_1-C_6)$-alkyl, NH-[$(C_1-C_8)$- alkyl]-COOH, NH-[$(C_1-C_8)$-alkyl]-$CONH_2$, N[$(C_1$- $C_6)$-alkyl] [$(C_1-C_8)$-alkyl] -CO-O $(C_1-C_6)$ -alkyl,

N[$(C_1-C_6)$-alkyl][$(C_1-C_8)$-alkyl]-COOH, N[$(C_1$- $C_6)$-alkyl] [$(C_1-C_8)$-alkyl] -$CONH_2$, NH- [C(H)(aryl)]-CO-O$(C_1-C_6)$-alkyl, NH- [C(H)(aryl)]-COOH, NH-[C(H)(aryl)]-$CONH_2$, N[( $C_1-C_6)$-alkyl] [C(H)(aryl)]-CO-O$(C_1-C_6)$-alkyl,

N[$(C_1-C_6)$-alkyl] [C (H) (aryl)]-COOH, N[$(C_1-C_6)$- alkyl] [C (H) (aryl)]-$CONH_2$, NH- [C (H) (heteroaryl) ] -CO-O $(C_1-C_6)$ -alkyl, NH- [C(H)(heteroaryl)]-COOH, NH- [C(H)(heteroaryl)]-$CONH_2$, N[$(C_1-C_6)$- alkyl] [C(H) (heteroar-

yl) -CO-O($C_1$-$C_6$) -alkyl,
N [($C_1$-$C_6$) -alkyl] [C (H) (heteroaryl)]-COOH, N [($C_1$-$C_6$)-alkyl] [C (H) (heteroaryl)]-$CONH_2$, N[( $C_1$-$C_6$)-alkyl] [($C_3$-$C_8$)-cycloalkyl]-CO-O($C_1$-$C_6$)- alkyl, N [($C_1$-$C_6$)-alkyl] [($C_3$-$C_8$)-cycloalkyl]- COOH, N[($C_1$-$C_6$) -alkyl] [($C_3$-$C_8$)-cycloalkyl]- $CONH_2$, NH- [($C_3$-$C_8$)-cycloalkyl]-CO-O($C_1$-$C_6$)- alkyl, NH-[($C_3$-$C_8$)-cycloalkyl]-COOH, NH-[($C_3$- $C_8$)-cycloalkyl]-$CONH_2$, NH-($C_1$-$C_8$)-alkyl-OH, NH- [($C_1$-$C_6$)-alkyl]-$SO_2$-(C1-C6)-alkyl, NH-[($C_1$-$C_6$)-alkyl]-$SO_3H$, NH-[($C_1$-$C_6$)-alkyl]-$SO_2$-$NH_2$, N [($C_1$-$C_6$)-alkyl] [($C_1$-$C_6$) -alkyl] -$SO_3H$},
where the alcohol (OH) functions may be replaced by F and where the aryl or heteroaryl radical may be substituted by halogen, CN, ($C_1$-$C_6$) -alkyl, O-($C_1$-$C_6$) -alkyl, OH, $SO_2$-$NH_2$, COOH, $CONH_2$, CO-O($C_1$-$C_6$)-alkyl, CO-($C_1$-$C_6$)-alkyl;
R18 is $(CH_2)_n$-CR25R26-CO-O($C_1$-$C_6$) -alkyl, $(CH_2)_n$- CR25R26-CO-$NH_2$, $(CH_2)_n$-CR25R26-COOH;
R20 is H, ($C_1$-$C_6$)-alkyl, ($C_3$-$C_8$)-cycloalkyl, aryl, [($C_1$-$C_6$)-alkyl]-aryl;
R21 is H, F, $CF_3$, ($C_1$-$C_6$) -alkyl, ($C_3$-$C_8$)- cycloalkyl, OH, 0- ($C_1$-$C_6$) -alkyl, O- ($C_3$-$C_8$)- cycloalkyl, O-$(CH_2)_n$-aryl, O-(CO)-($C_1$-$C_6$)- alkyl, O-(CO)-($C_3$-$C_8$)-cycloalkyl, O-(CO)-O- ($C_1$-$C_6$)-alkyl, O-(CO)-O-($C_3$-$C_8$)-cycloalkyl, NH-[($C_1$-$C_6$)-alkyl] -aryl, $NH_2$, NH- ($C_1$-$C_6$)-alkyl, NH-(CO)-($C_1$-$C_6$)-alkyl;
R22 is H, $CF_3$, ($C_1$-$C_6$)-alkyl, aryl, [($C_1$-$C_6$)-alkyl]-aryl;
R23, R24 are each independently H, ($C_1$-$C_6$) -alkyl, ($C_3$- $C_8$)-cycloalkyl, [($C_1$-$C_6$)-alkyl]-[($C_3$-$C_8$)- cycloalkyl], aryl, ($C_1$-$C_6$)-alkyl]-aryl
or R23 and R24 together form a -CH=CH-, -$CH_2$- $CH_2$-, -$CH_2$-$CH_2$-$CH_2$- or -$CH_2$-$CH_2$-$CH_2$-$CH_2$- unit in which one $CH_2$ moiety may be replaced by C=O, CHF or $CF_2$, and in which up to four hydrogen atoms may be replaced by a ($C_1$-$C_6$)-alkyl radical;
R25, R26 are each independently H, F, ($C_1$-$C_6$)-alkyl, aryl, [($C_1$-$C_6$)-alkyl]-aryl,
where the aryl may be substituted by halogen, CN, OH, O-($C_1$-$C_6$)-alkyl,
or the R25 and R26 radicals, together with the carbon atom bonded to them, form a three- to seven-membered carbocycle in which one carbon atom may be replaced by 0, $S(O)_m$, NH, N[($C_1$-$C_6$)-alkyl] or CO;

excluding the compound N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)urea,
and the physiologically compatible salts thereof.

**2.** A compound of the formula I as claimed in claim 1, wherein

R, R' are each independently H, $(CH_2)_n$-aryl, ($C_1$-$C_6$)- alkyl, where ($C_1$-$C_6$)-alkyl or the aryl radical may be substituted by halogen;
or R and R' together form a ring having from three to eight carbon atoms, where one carbon atom may be replaced by 0, $S(O)_m$, NR13 or NR15;
m is 0, 1, 2;
n is 0, 1, 2, 3;
p is 1, 2, 3, 4;
q is 1, 2, 3;
r is 2, 3, 4, 5;
v is 0, 1, 2, 3;
A, D, E, G, L are each independently C or N, where, when they are defined as N, the corresponding substituent R1, R2, R3, R4, R5 is absent,
or R2-D=E-R3 or R4-G=L-R5 are defined as S or O and where the five-membered or six-membered ring may be fused to -$(CH_2)_3$- or -$(CH_2)_4$- or -CH=CH-CH=CH- to form a bicyclic system;
R1, R2, R3, R4, R5 are each independently H, F, Cl, Br, I, CN, $CF_3$, ($C_1$-$C_8$)-alkyl, ($C_3$-$C_8$)- cycloalkyl, $(CH_2)_q$-[($C_3$-$C_8$)-cycloalkyl], $(CH_2)_n$-[($C_7$-$C_{12}$)-bicycloalkyl], $(CH_2)_n$-[($C_7$-$C_{12}$)- tricycloalkyl], adamantan-1-yl, adamantan-2- yl, $(CH_2)_n$-aryl, $(CH_2)_n$-heteroaryl, $OCF_3$, O- R11, NR13R15, NH-CN, $S(O)_m$-R12, $SO_2$-$NH_2$, $SO_2$-N=CH-$(CH_3)_2$, $SO_2$-NH-[($C_1$-$C_8$)-alkyl], $SO_2$-NH- [($C_3$-$C_8$)-cycloalkyl], $SO_2$-NH-$(CH_2)_n$-aryl, $SO_2$- NH-$(CH_2)_n$-heteroaryl, $SO_2$-N[($C_1$-$C_8$)-alkyl]$_2$, $SO_2$-R16, $SF_5$, CO-O[($C_1$-$C_8$)-alkyl], CO-O[($C_3$- $C_8$)-cycloalkyl], CO-O-$(CH_2)_n$-aryl, CO-O-$(CH_2)_n$- heteroaryl, CO-$NH_2$, CO-NH-CN, CO-NH-[($C_1$-$C_8$)- alkyl], CO-N [($C_1$-$C_8$)-alkyl]$_2$, CO-NH- [($C_3$-$C_8$)- cycloalkyl], C(=NH)-O-[($C_1$-$C_6$-alkyl)], C(=NH)- $NH_2$, C(=NH)-R16, C(=NR13)-NR12R13, $(CH\text{-}2)_n$- C(=N$SO_2$-R12)$NH_2$, CO-R16, COOH, CO-($C_1$-$C_8$)- alkyl, CO-($C_3$-$C_8$)-cycloalkyl, CO-aryl, CO- heteroaryl, CH(OH)-aryl, CH(OH)-heteroaryl, CH[O-($C_1$-$C_6$)-alkyl]-aryl, CH[O-($C_1$-$C_6$)-alkyl]- heteroaryl, CHF-aryl, CHF-heteroaryl, $CF_2$- aryl, $CF_2$-heteroaryl, CHO, $CH_2$-OH, $CH_2$-CN, $CH_2$- O-R12 , $CH_2$-O-$(CH_2)_q$-COOH,
where the alkyl, cycloalkyl, cycloalkenyl, bicycloalkyl and tricycloalkyl radicals may be substituted by fluorine atoms, and where the aryl or heteroaryl radicals may be substituted by halogen, CN, ($C_1$-$C_6$)-alkyl, ($C_3$-$C_6$)-cy-

cloalkyl, O-($C_1$-$C_6$)-alkyl, $OCF_3$, OH, O-(CH2)n-aryl, $(CH_2)_n$-aryl, $S(O)_m$-($C_1$-$C_6$)-alkyl, $SO_2$-$NH_2$, SH, NR12R13, NH-CO- [($C_1$-$C_6$)-alkyl], NH-CO- $(CH_2)_n$-aryl, $(CH_2)_n$-COOH, $(CH_2)_n$-$CONH_2$, $(CH_2)_n$-CO-O($C_1$-$C_6$)-alkyl, $(CH_2)_n$-CO-($C_1$-$C_6$)- alkyl, and where the alkyl radicals may be substituted by fluorine atoms;

R6, R7, R8, R9, R10 are each independently T-bicyclic heterocycle, T-aryl or T-heteroaryl, where the bicyclic heterocycle or the aryl or heteroaryl radical may be fused to a 5- or 6- membered aromatic or nonaromatic carbon ring in which one or more CH or $CH_2$ groups may be replaced by oxygen atoms and where the 5- or 6-membered aromatic or nonaromatic carbon ring may be substituted by F, =O or -($C_1$-$C_6$)- alkyl and where the bicyclic heterocycle may contain from 9 to 12 ring members and up to five CH or $CH_2$ groups may each independently be replaced by N, NR20, O, $S(O)_m$ or C=O and where the aryl or heteroaryl radical or bicyclic heterocycle may be unsubstituted or may be mono- or polysubstituted by

R11, F, C1, Br, I, CN, $N_3$, NC, $NO_2$, $CF_3$, $(CH_2)_n$-O-R11, $(CHF_2)_n$-O-$(CH_2)_r$-OH, $(CH_2)_n$- O-CH$(CH_2OH)_2$, $(CH_2)_n$-O-$(CH_2)_n$-CO-O-$(CH_2)_r$- $NH_2$, $(CH_2)_n$-O- $(CH_2)_n$-CO-NH-$(CH_2)_r$-OH, O- R13, $OCF_3$, $(CH_2)_n$-O-$(CH_2)_r$-$NH_2$, $(CH_2)_n$-NH- R11 $(CH_2)_n$-N[$(CH_2)_q$-CO-O ($C_1$-$C_6$)-alkyl$]_2$, $(CH_2)_n$-N[$(CH_2)_q$-COOH$]_2$, $(CH_2)_n$-N[(CH2)q- $CONH_2]_2$, $(CH_2)_n$-NH-R13 , $(CH_2)_n$-N$(R13)_2$, $(CH_2)_n$-NH-CN, $(CH_2)_n$-NH-$SO_2$-R16, $(CH_2)_n$- NH- $(CH_2)_n$-$SO_2$-R12, $(CH_2)_n$-NR12-CO-R16, $(CH_2)_n$-NR12-CO-NR12R13, $(CH_2)_n$-NR12-CO-N$(R12)_2$, $(CH_2)_n$-NR12-CO-NHR11, $(CH_2)_n$-NH- C(=NH)-$NH_2$, $(CH_2)_n$-NH-C(=NH)-R16, $(CH_2)_n$- NH-C(=NH)-NHR12, $(CH_2)_n$-NR12-C(=NR13)- NHR12, $(CH_2)_n$-NR12-C(=NR12)-NR12R13, ($(CH_2)_n$-NH-$(CH_2)_n$-CO-O-$(CH_2)_r$-$NH_2$, $(CH_2)_n$- NH-$(CH_2)_n$ -CO-NH-[($C_1$-$C_8$)-alkyl), $(CH_2)_n$- NH-$(CH_2)_n$ -CO-NH-$(CH_2)_r$-OH, $(CH_2)_n$-NH- $(CH_2)_n$ -CO-N[($C_1$-$C_8$)-alkyl$]_2$, $(CH_2)_n$-NH- $(CH_2)_n$ -CO-NH- [ ($C_3$-$C_8$)-cycloalkyl], $(CH_2)_n$-NH-$(CH_2)_n$-CO-N[($C_3$-$C_8$)-cycloalkyl$]_2$ $(CH_2)$ n-NH-C $(CH_3)_2$-CO-O($C_1$-$C_8$)-alkyl, $(CH_2)_n$-NH-C$(CH_3)_2$-CO-O($C_3$-$C_8$)-cycloalkyl, $(CH_2)_n$-NH-C $(CH_3)_2$-CO-O-$(CH_2)$ r-$NH_2$, $(CH_2)_n$- NH-C $(CH_3)_2$-CO-O-$(CH_2)_n$-aryl, $(CH_2)_n$-NH- C$(CH_3)_2$-CO-O-$(CH_2)_n$-heteroaryl, $(CH_2)_n$- NH-C(CH3)2-CO-$NH_2$, $(CH_2)_n$-NH-C $(CH_3)_2$-CO- NH- [($C_1$-$C_8$)-alkyl], (-$CH_2)_n$-NH-C$(CH_3)_2$-CO- NH- $(CH_2)_r$-OH, $(CH_2)_n$-NH-C $(CH_3)_2$-CO-N [($C_1$- $C_8$)-alkyl$]_2$, $(CH_2)_n$-NH-$(CH_3)_2$-CO-NH[($C_3$- $C_8$)-cycloalkyl], $(CH_2)_n$-NH-C $(CH_3)_2$-CO-N ($C_3$-$C_8$)-cycloalkyl$]_2$, $(CH_2)_n$-NH-C$(CH_3)_2$- COOH, $S(O)_m$-R12, $SO_2$-R16, $SO_2$-N=CH- N$(CH_{3)2}$,

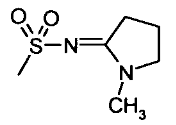,

$SO_2$-NH-CO-R12, $SO_2$- NHR12, $SO_2$-NH-$(CH_2)_r$-OH, $SO_2$-N [($C_1$-$C_8$)- alkyl]2, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH-piperidin-1-yl, $(CH_2)_n$-CO- NH-$SO_2$-NHR12, $(CH_2)_n$-CO-NH-$SO_2$-R18, $(CH_2)_n$-CHO, $(CH_2)_n$-C (=NH)$NH_2$, $(CH_2)_n$- C(=NH)-NHOH, $(CH_2)_n$-C(=NH)-[NH-O-($C_1$-$C_6$)- alkyl] , $(CH_2)_n$-C(=NH) (R16), $(CH_2)_n$- C(=NR13)NHR12, $(CH_2)_n$-C(=NR12)NR12R13, $(CH_2)_n$-C (=N$SO_2$-R12) $NH_2$, $(CH_2)_n$- C(=NH)O[($C_1$-$C_6$)-alkyl], where the alkyl and cycloalkyl radicals may be substituted by fluorine atoms and where the aryl or heteroaryl radicals may be substituted by halogen, CN, ($C_1$-$C_6$)- alkyl, ($C_3$-$C_6$) -cycloalkyl, O- ($C_1$-$C_6$)- alkyl, S $(O)_m$-($C_1$-$C_6$) -alkyl, $SO_2$-$NH_2$, COOH, $CONH_2$, CO-O($C_1$-$C_6$)-alkyl, CO-($C_1$- $C_6$)-alkyl and where the alkyl radicals may be substituted by fluorine atoms,

and where, when R3 is CN, $NO_2$ or halogen and R4 is $CF_3$ or halogen and R and R' are each methyl, the X-aryl radical is provided with at least one of the abovementioned substituents other than hydrogen;

H, F, Cl, Br, I, CN, $N_3$, NC, $NO_2$, $CF_3$,
($C_1$-$C_8$) -alkyl, ($C_2$-$C_{10}$) -alkenyl, ($C_2$-$C_{10}$)- alkynyl, ($C_3$-$C_8$)-cycloalkyl, aryl, heteroaryl,
$(CH_2)_n$-CO-[O-($C_1$-$C_8$)-alkyl], $(CH_2)_n$-CO-[O-($C_3$- $C_8$)-cycloalkyl] , $(CH_2)_n$-CO-[($C_1$-$C_8$)-alkyl] , $(CH_2)_n$-CO-[($C_3$-$C_8$)-cycloalkyl],
$(CH_2)_n$-CO-[O-$(CH_2)_v$-aryl],
$(CH_2)_n$-CO-$NH_2$, $(CH_2)_n$-COOH, $(CH_2)_n$-CO-NH-CN,
$(CH_2)_n$-P(O) (OH) [O-($C_1$-$C_6$-alkyl], $(CH_2)_n$- P(O)[O-($C_1$-$C_6$)-alkyl$]_2$, $(CH_2)_n$-P(O) (OH) (O-$CH_2$- aryl) , $(CH_2)_n$-P(O) (O-$CH_2$-aryl$)_2$, $(CH_2)_n$- P (O) $(OH)_2$,
$(CH_2)_n$-$SO_3H$, $(CH_2)_n$-$SO_2$-$NH_2$,
$(CH_2)_n$-CO-NH- [($C_1$-$C_8$)-alkyl], $(CH_2)_n$-CO-N[($C_1$- $C_8$)-alkyl$]_2$, $(CH_2)_n$-CO-NH-[($C_3$-$C_8$)-cycloalkyl], ($C_2$-$C_{10}$)-alkenyl-CO-O[($C_1$-$C_6$)-alkyl], ($C_2$-$C_{10}$)- alkenyl-$CONH_2$, ($C_2$-$C_{10}$)-alkenyl-COOH, ($C_2$-$C_{10}$)- al kynyl-CO-O [($C_1$-$C_6$)-alkyl], ($C_2$-$C_{10}$)-alkynyl- $CONH_2$, ($C_2$-$C_{10}$)-alkynyl-COOH,
$(CH_2)_n$-CO-R16,

$(CH_2)_n$-OH, $(CH_2)_n$-O-($C_1$-$C_8$)-alkyl, $(CH_2)_n$-O-($C_2$- $C_{10}$) -alkenyl, $(CH_2)_n$-O-($C_2$-$C_{10}$) -alkynyl, $(CH_2)_n$- O- ($C_3$-$C_8$)-cycloalkyl, $(CH_2)_n$-O-$(CH_2)_q$-[($C_3$-$C_8$)- cycloalkyl] , $(CH_2)_n$-O-$(CH_2)_n$-CO-[O-($C_1$-$C_8$)- alkyl], $(CH_2)_n$-O-$(CH_2)$ $_n$-CO-[O-($C_3$-$C_8$)- cycloalkyle, $(CH_2)_n$-O-$(CH_2)_n$-CO-[($C_1$-$C_8$)- alkyl], $(CH_2)_n$-O-$(CH_2)_n$-CO-[($C_3$-$C_8$)- cycloalkyl], $(CH_2)_n$-O-$(CH_2)_n$-CO-[O-$(CH_2)_v$- aryl], $(CH_2)_n$-O-$(CH_2)_n$-CO- [O-$(CH_2)_v$- heteroaryl], $(CH_2)_n$-O-$(CH_2)_q$-CO-$NH_2$, $(CH_2)_n$-O- $(CH_2)_q$-COOH, $(CH_2)_n$-O- $(CH_2)$q-CO-NH-CN, $(CH_2)$ $_n$-O- $(CH_2)_n$-P(O)(OH)[O-($C_1$-$C_6$)-alkyl], $(CH_2)_n$-O- $(CH_2)_n$-P (O) [O- ($C_1$-$C_6$)-alkyl]$_2$, $(CH_2)_n$-O- $(CH_2)_n$- P(O) (OH) (O-$CH_2$-aryl), $(CH_2)_n$-O-$(CH_2)$n-P(O)(O- $CH_2$-aryl)$_2$, $(CH_2)_n$-O-(CH2)$_n$-P(O)$(OH)_2$, $(CH_2)_n$-O- $(CH_2)_n$-$SO_3H$, $(CH_2)_n$-O-$(CH_2)_n$-$SO_2$-$NH_2$, $(CH_2)_n$-O- $(CH_2)_n$-CO-NH-[($C_1$-$C_8$)-alkyl], $(CH_2)_n$-O-$(CH_2)_n$- CO-N[($C_1$-$C_8$)-alkyl]$_2$, $(CH_2)_n$-O-$(CH_2)_n$-CO-NH- [($C_3$-$C_8$)-Cycloalkyl], $(CH_2)_n$-O-$(CH_2)_n$-CR21R22- CO-O[($C_1$-$C_6$) -alkyl], (-$CH_2)_n$-O-$(CH_2)_n$-CR21R22- $CONH_2$, $(CH_2)_n$-O-$(CH_2)_n$-CR21R22-COOH, $(CH_2)_n$-O- $(CH_2)_n$-CO-R16, $(CH_2)_n$-O-$(CH_2)_r$-OH, $(CH_2)_n$-O- CH $(CH_2OH)_2$, $(CH_2)_n$-O-$(CH_2)_n$-CO-O-$(CH_2)_r$-NH2, $(CH_2)_n$-O-$(CH_2)_n$-CO-NH-$(CH_2)_r$-OH, O-R13, $OCF_3$,

$(CH_2)_n$-$NH_2$, (-$CH_2)_n$-NH- ($C_1$-$C_8$) -alkyl, $(CH_2)_n$-NH- ($C_3$-$C_8$) -cycloalkyl, (CHF2)$_n$-NH-$(CH_2)_n$-CO-[O-($C_3$-$C_8$)-cycloalkyl], $(CH_2)_n$-NH-$(CH_2)_n$-CO-[($C_1$-$C_8$)- alkyl], $(CH_2)_n$-NH-$(CH_2)_n$-CO-[($C_3$-$C_8$)- cycloalkyl], $(CH_2)_n$-NH-$(CH_2)_n$-CO-[O-$(CH_2)_v$- aryl], $(CH_2)_n$-NH-$(CH_2)_n$-CO-[O-$(CH_2)_v$- heteroaryl], $(CH_2)_n$-NH-$(CH_2)_q$-CO-NH-CN,

$(CH_2)_n$-NH-$(CH_2)_n$-P(O)$(OH)_2$,

$(CH_2)_n$-NH-$(CH_2)_n$-$SO_3H$,

$(CH_2)_n$-NH-$(CH_2)_n$-$SO_2$-$NH_2$,

$(CH_2)_n$-NH-$(CH_2)$n-CR21R22-CO-O[($C_1$-$C_6$)-alkyl],

$(CH_2)_n$-NH-$(CH_2)_n$-CR21R22-$CONH_2$, $(CH_2)_n$-NH- $(CH_2)_n$-CR21R22-COOH,

$(CH_2)_n$-NH-$(CH_2)_n$-CO-R16,

$(CH_2)_n$-NH-$(CH_2)_n$-$SO_2$-[($C_1$-$C_8$)-alkyl, $(CH_2)_n$-NH- $(CH_2)_n$-$SO_2$-[($C_3$-$C_8$)-cycloalkyl], $(CH_2)_n$-NH-$SO_2$-$(CH_2)_n$-$NH_2$, $(CH_2)_n$-NH-$SO_2$-$(CH_2)_n$-NH-($C_1$-$C_8$)- alkyl, (-$CH_2)_n$-NH-$SO_2$-$(CH_2)_n$-NH-($C_3$-$C_8$)- cycloalkyl, $(CH_2)_n$-NH-$SO_2$-$(CH_2)_n$-N[($C_1$-$C_8$)- alkyl]$_2$,

$(CH_2)_n$-NH-CN, $(CH_2)_n$-NH-$SO_2$-R16, $(CH_2)_n$-NR12-CO-NH-($C_1$-$C_8$) -alkyl, $(CH_2)_n$-NR12- CO-NH- ($C_3$-$C_8$) -cycloalkyl, $(CH_2)_n$-NR12-CO-NH2, $(CH_2)_n$-NR12-CO-NH-$SO_2$-($C_1$-$C_8$)-alkyl, $(CH_2)_n$- NR12-CO-NH-$SO_2$- ($C_3$-$C_8$)-cycloalkyl, $(CH_2)_n$-NR12- CO-N[($C_1$-$C_8$)-alkyl]$_2$, $(CH_2)$ n-NH-CO-NH-$(CH_2)$n- CO-[O-($C_1$-$C_8$)-alkyl], $(CH_2)$n-NH-CO-NH- $(CH_2)$q- CO-$NH_2$, $(CH_2)$ n-NH-CO-NH- $(CH_2)_q$-COOH, $(CH_2)_n$- NH-C(=NH)-$NH_2$, $(CH_2)_n$-NH-C(=NH)-R16, $(CH_2)_n$-NH- C(=NH)-NH[($C_1$-$C_8$)-alkyl], $(CH_2)_n$-NH-C(=N-$SO_2$- ($C_1$-$C_8$)-alkyl)-$NH_2$, $(CH_2)_n$-NH-C (=N-$SO_2$-($C_1$-$C_8$)- alkyl) -NH[($C_1$-$C_8$)-alkyl], $(CH_2)_n$-NH-C (=N-$SO_2$- $NH_2$) -$NH_2$, $(CH_2)$ $_n$-NH-C(=N-$SO_2$-$NH_2$) -NH[($C_1$-$C_8$)- alkyl], $(CH_2)_n$-NH-C(=NH)-N[($C_1$-$C_8$)-alkyl]$_2$, $(CH_2)_n$-NH-C(=N-$SO_2$-($C_1$-$C_8$)-alkyl)-N[($C_1$-$C_8$)-alkyl]$_2$, $(CH_2)_n$-NH-$(CH_2)_n$-CO-O-$(CH_2)_r$-NH2, $(CH_2)_n$-NH-$(CH_2)_n$ -CO-NH-[($C_1$-$C_8$)-alkyl], $(CH_2)_n$- NH-$(CH_2)_n$ -CO-NH- $(CH_2)_r$-OH, $(CH_2)_n$-NH-$(CH_2)_n$ -CO-N[($C_1$-$C_8$)-alkyl]$_2$, $(CH_2)_n$-NH- $(CH_2)_n$ -CO-NH- [($C_3$-$C_8$)-cycloalkyl], $(CH_2)_n$-NH-C $(CH_3)_2$-CO-O ($C_3$- $C_8$) -cycloalkyl, $(CH_2)_n$-NH-C$(CH_3)_2$-CO-O- $(CH_2)_r$- $NH_2$, $(CH_2)$ $_n$-NH-C $(CH_3)_2$-CO-O-$(CH_2)_n$-aryl, $(CH_2)_n$- NH-C $(CH_3)_2$-CO-O-$(CH_2)_n$-heteroaryl, $(CH_2)_n$-NH-C$(CH_3)_2$-CO-NH-[($C_1$-$C_8$)-alkyl], $(CH_2)_n$-NH- C$(CH_3)_2$-CO-NH-$(CH_2)_r$-OH, $(CH_2)_n$-NH-C$(CH_3)_2$-CO-N[($C_1$-$C_8$)-alkyl]$_2$, $(CH_2)_n$-NH-$(CH_3)_2$-CO-NH-[($C_3$- $C_8$)-cycloalkyl], $(CH_2)_n$-NH-C$(CH_3)_2$-CO-N[($C_3$-$C_8$) - cycloalkyle]$_2$, $(CH_2)_n$-S$(O)_m$-($C_1$-$C_8$)-alkyl, $(CH_2)_n$-S$(O)_m$-($C_3$-$C_8$)- cycloalkyl, $(CH_2)_n$-$SO_2$-R16, $SO_2$-N=CH-N$(CH_3)_2$,

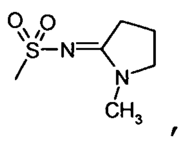
,

$(CH_2)_n$-$SO_2$-NH-CO-($C_1$-$C_8$)-alkyl, $(CH_2)_n$-$SO_2$-NH-CO-($C_3$-$C_8$) -cycloalkyl, $(CH_2)_n$-$SO_2$- NH- ($C_1$-$C_8$) -alkyl, $(CH_2)_n$-$SO_2$-NH-($C_3$-$C_8$)- cycloalkyl, $(CH_2)_n$-$SO_2$-N[($C_1$-$C_8$) -alkyl]$_2$, $SO_2$- NH-$(CH_2)_r$-OH, $SO_2$-NH- $(CH_2)_r$-$NH_2$, $SF_5$,

$(CH_2)_q$-CN,

$(CH_2)_n$-CO-NH-piperidin-1-yl,

$(CH_2)_n$-CO-NH-$SO_2$-NHR12, $(CH_2)_n$-CO-NH-$SO_2$-($C_1$- $C_8$)-alkyl, $(CH_2)_n$-CO-NH-$SO_2$- ($C_3$-$C_8$)-cycloalkyl, $(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13) NHR12, $(CH_2)_n$-C(=NR12) NR12R13, $(CH_2)_n$-C(=NH)O[($C_1$-$C_6$)- alkyl], where the alkyl and cycloalkyl radicals may be substituted by fluorine atoms and where the aryl or heteroaryl radicals may be substituted by halogen, CN, ($C_1$-$C_6$)-alkyl, ($C_3$-$C_6$)-cycloalkyl, O-($C_1$-$C_6$)-alkyl, S$(O)_m$-($C_1$- $C_6$)-alkyl, $SO_2$-$NH_2$, COOH, $CONH_2$, CO-[O($C_1$-$C_6$)- alkyl], CO-($C_1$-$C_6$)-alkyl and where the alkyl radicals may be substituted by fluorine atoms;

where at least one of the R6, R7, R8, R9 and R10 radicals is always defined as T-bicyclic heterocycle, T-aryl or T-heteroaryl and

where one of the four radical pairs of R6 and R7, or R7 and R8, or R8 and R9, or R9 and R10 may in each case together form the $-CH_2-CH_2-CH_2-$ or $-CH_2-CH_2-CH_2-CH_2-$ groups in which up to two $-CH_2-$ groups may be replaced by -0- and where the $-CH_2-CH_2-CH_2-$ or $-CH_2-CH_2-CH_2-CH_2-$ groups may be substituted by F, $(C_1-C_8)$-alkyl or =0;

T is NR23-CO-NR24, NR23-$SO_2$-NR24, $SO_2$-NR23-$SO_2$, CO-NR23-CO, NR23-C(=NR13)-NR24, NR23-C(=NR22)-NR24, CO-NR23-CR22R23, CO-CR22R23- CO, CR22R23-CO-CR22R24, NR23-CO-CR22R24, NR23-$SO_2$-CR22R24, CR22R24-CO-NR23, CR22R24- $SO_2$-NR23, CR22R23-NR23-$SO_2$, $SO_2$-CR22R23-NR23, $SO_2$-NR23-CR22R23-, NR23-CR22R23-$SO_2$, CO-NR23- $SO_2$, $SO_2$-NR23-CO, CO-CR22R23-$SO_2$, $SO_2$-CR22R23- CO, CR23R24-CR23R24-CR23R24, CR23R24-NR23- CR23R24;

R11 is H, $(C_1-C_8)$-alkyl, $(C_2-C_{10})$-alkenyl, $(C_2-C_{10})$- alkynyl, $(C_3-C_8)$ -cycloalkyl, $(CH_2)_q$- [$(C_3-C_8)$- cycloalkyl], $(CH_2)_n$-aryl, $(CH_2)_n$-CO- [O- $(C_1-C_8)$- alkyl], $(CH_2)_n$-CO-[O-$(C_3-C_8)$-cycloalkyl], $(CH_2)_n$-CO-[$(C_1-C_8)$ -alkyl], $(CH_2)_n$-CO-[$(C_3-C_8)$- cycloalkyl], $(CH_2)_n$-CO-aryl, $(CH_2)_n$-CO- heteroaryl, $(CH_2)_q$-CO-$NH_2$, $(CH_2)_q$-COOH, $(CH_2)_n$- P(O)(OH)[O-$(C_1-C_6)$-alkyl], $(CH_2)_n$-P(O)[O-$(C_1$- $C_6)$-alkyl]$_2$, $(CH_2)_n$-P(O) (OH) (O-$CH_2$-aryl), $(CH_2)_n$-P(O) (O-$CH_2$-aryl)$_2$, $(CH_2)_n$-P(O) $(OH)_2$, $(CH_2)_n$-$SO_3H$, $(CH_2)_n$-$SO_2$-$NH_2$, $(CH_2)_n$-CO-NH- [$(C_1$-$C_8)$-alkyl],

$(CH_2)_n$-CO-N[$(C_1-C_8)$-alkyl]$_2$, $(CH_2)_n$-CO-NH- [$(C_3$- $C_8)$-cycloalkyl], $(C_2-C_{10})$-alkenyl-CO-O[$(C_1-C_6)$- alkyl], $(C_2-C_{10})$-alkenyl-$CONH_2$, $(C_2-C_{10})$- alkenyl-COOH, $(C_2-C_{10})$ -alkynyl-CO-O [$(C_1-C_6)$- alkyl], $(C_2-C_{10})$ -alkynyl-$CONH_2$, $(C_2-C_{10})$- alkynyl-COOH, $(CH_2)_n$-CR21[(CO-O$(C_1-C_6)$- alkyl)]$_2$, $(CH_2)_n$-CR21$(CONH_2)_2$, $(CH_2)_n$-CR21$(COOH)_2$, $(CH_2)_n$-CR21R22-CO-O[$(C_1-C_6)$- alkyl], $(CH_2)_n$-CR21R22-$CONH_2$,$(CH_2)_n$-CR21R22- CO-NH-[$(C_1-C_8)$-alkyl], $(CH_2)_n$-CR21R22-CO-N[$C_1$- $C_8)$-alkyl]$_2$, $(CH_2)_n$-CR21R22-COOH, $(CH_2)_n$-CO- R16, $(CH_2)_n$-CO-NH-C$(CH_3)_2$-CO-O[$(C_1-C_8)$ -alkyl], $(CH_2)_n$-CO-NH-C $(CH_3)_2$-$CONH_2$, $(CH_2)_n$-CO-NH- C$(CH_3)_2$-COOH, where the alkyl, alkenyl, alkynyl and cycloalkyl radicals may be substituted by fluorine atoms and where the aryl or heteroaryl radical may be substituted by halogen, CN, $(C_1-C_6)$-alkyl, $(C_3-C_6)$- cycloalkyl, O-$(C_1-C_6)$-alkyl, $S(O)_m$-$(C_1-C_6)$- alkyl, $SO_2$-$NH_2$, COOH, $CONH_2$, CO-O$(C_1-C_6)$-alkyl, CO-$(C_1-C_6)$-alkyl, and where the alkyl radicals may be substituted by fluorine atoms;

R12 is H, $(C_1-C_8)$-alkyl, $(C_3-C_6)$ -cycloalkyl, $(CH_2)_n$-aryl, $(CH_2)_n$-heteroaryl, where the alkyl or cycloalkyl radicals may be substituted by fluorine atoms,

and where the aryl or heteroaryl radical may be substituted by halogen, CN, $(C_1-C_6)$-alkyl, O-$(C_1-C_6)$-alkyl, $SO_2$-$NH_2$, COOH, $CONH_2$, CO-O$(C_1$- $C_6)$-alkyl, CO-$(C_1-C_6)$-alkyl and where the alkyl radicals may be substituted by fluorine atoms;

R13 is H, $SO_2$-[$(C_1-C_8)$-alkyl], $SO_2$-[$(C_3-C_8)$- cycloalkyl], $SO_2$-$(CH_2)_n$-aryl, $SO_2$- $(CH_2)_n$-heteroaryl,

where the alkyl and cycloalkyl radicals may be substituted by fluorine atoms and where the aryl or heteroaryl radical may be substituted by halogen, CN, $CF_3$, $(C_1-C_6)$- alkyl, $(C_3-C_6)$-cycloalkyl, O-[$(C_1-C_6)$-alkyl], $S(O)_m$-[$(C_1-C_6)$-alkyl], $SO_2$-$NH_2$, COOH, $CONH_2$, CO-[O$(C_1-C_6)$-alkyl], CO-$(C_1-C_6)$-alkyl, and where the alkyl radicals may be substituted by fluorine atoms;

R15 is $(C_1-C_8)$-alkyl,

where the alkyl radical may be substituted by fluorine atoms;

R16 is aziridin-1-yl, azetidin-1-yl, 3- hydroxyazetidin-1-yl, piperidin-1-yl, pyrrolidin-1-yl, 3-pyrrolidinol-1-yl, morpholin-N-yl, piperazin-1-yl, 4-[$(C_1-C_6)$- alkyllpiperazin-1-yl, thiomorpholin-4-yl, thiomorpholine-1,1-dioxid-4-yl, NH-$(CH_2)_r$-OH, NH-CH $(CH_2OH)_2$, NH-C $(CH_2OH)_3$, N [$(C_1-C_6)$-alkyl- OH]$_2$, D-glucamin-N-yl, N-methyl-D-glucamin-N- yl, NH-[$(C_1-C_8)$-alkyl]-CO-O$(C_1-C_6)$-alkyl, NH- [$(C_1-C_8)$-alkyl]-COOH, NH-[$(C_1-C_8)$-alkyl]-$CONH_2$, N[$(C_1-C_6)$-alkyl] [$(C_1-C_8)$ -alkyl] -COOH, NH- [C (H) (aryl)] -CO-O$(C_1-C_6)$ -alkyl, NH- [C (H) (aryl)]-COOH, NH-[C (H) (aryl)]-$CONH_2$, NH- [C(H) (heteroaryl)]-CO-O$(C_1-C_6)$-alkyl, NH- [C(H)(heteroaryl)]-COOH, NH- [C(H)(heteroaryl)]-$CONH_2$, NH-[$(C_3-C_8)$- cycloalkyl] -CO-O $(C_1-C_6)$ -alkyl, NH-[$(C_3-C_8)$- cycloalkyl]-COOH, NH-[$(C_3-C_8)$-cycloalkyl]- $CONH_2$, NH-$(C_1-C_6)$-alkyl-OH, NH-[$(C_1-C_6)$-alkyl]- $SO_2$-$(C_1-C_6)$ -alkyl, NH-[$(C_1-C_6)$-alkyl]-$SO_3H$, NH- [$(C_1-C_6)$-alkyl]-$SO_2$-NH2,

where the alcohol (OH) functions may be replaced by F and where the aryl or heteroaryl radical may be substituted by halogen, CN, $(C_1-C_6)$-alkyl, O-$(C_1-C_6)$-alkyl, OH, $SO_2$-$NH_2$, COOH, $CONH_2$, CO-O$(C_1-C_6)$-alkyl, CO-$(C_1-C_6)$-alkyl;

R18 is $(CH_2)_n$-CR25R26-CO-O$(C_1-C_4)$ -alkyl, $(CH_2)_n$- CR25R26-CO-$NH_2$, $(CH_2)_n$-CR25R26-COOH;

R20 is H, $(C_1-C_6)$-alkyl, $(C_3-C_8)$-cycloalkyl, aryl, [$(C_1-C_6)$-alkyl]-aryl;

R21 is H, F, $CF_3$, $(C_1-C_6)$-alkyl, $(C_3-C_8)$- cycloalkyl, OH, O-$(C_1-C_6)$-alkyl, O-$(C_3-C_8)$- cycloalkyl, O-$(CH_2)_n$-aryl, O-(CO)-$(C_1-C_6)$- alkyl, O-(CO)-$(C_3-C_8)$-cycloalkyl, O-(CO)-O- $(C_1-C_6)$-alkyl, O-(CO)-O-$(C_3-C_8)$-cycloalkyl, NH-[$(C_1-C_6)$-alkyl]-aryl, $NH_2$, NH-$(C_1-C_6)$-alkyl, NH-(CO)-$(C_1-C_6)$-alkyl;

R22 is H, $CF_3$, $(C_1-C_6)$-alkyl, aryl, [$(C_1-C_6)$-alkyl]-aryl;

R23, R24 are each independently H, $(C_1-C_6)$-alkyl, $(C_3$- $C_6)$-cycloalkyl, [$(C_1-C_4)$-alkyl]-[$(C_3-C_6)$- cycloalkyl], aryl,

$[(C_1-C_4)$-alkyl]-aryl
or R23 and R24 together form a -CH=CH-, $-CH_2-$ $CH_2-$, $-CH_2-CH_2-CH_2-$ or $-CH_2-CH_2-CH_2-CH_2-$ unit in which one $CH_2$ moiety may be replaced by C=O, CHF or $CF_2$, and in which up to four hydrogen atoms may be replaced by a $(C_1-C_4)$-alkyl radical;
R25, R26 are each independently H, F, $(C_1-C_4)$-alkyl, aryl, $[(C_1-C_4)$-alkyl]-aryl,
where the aryl may be substituted by halogen, CN, OH, O-$(C_1-C_4)$-alkyl,
or the R25 and R26 radicals, together with the carbon atom bonded to them, form a three- to seven-membered carbocycle in which one carbon atom may be replaced by O, $S(O)_m$, NH, $N[(C_1-C_9)$-alkyl] or CO;

excluding the compound N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)urea,
and the physiologically compatible salts thereof.

**3.** A compound of the formula I as claimed in claim 1 or 2, wherein

R, R' are each independently H, $(CH_2)_n$-aryl, $(C_1-C_6)$- alkyl, where $(C_1-C_6)$-alkyl or the aryl radical may be substituted by halogen;
or R and R' together form a ring having three to eight carbon atoms where one carbon atom may be replaced by O, $S(O)_m$, NR13 or NR15;
m is 0, 1, 2;
n is 0, 1, 2;
p is 1, 2, 3;
q is 1, 2;
r is 2, 3, 4;
v is 0, 1, 2;
A, D, E, G, L are each independently C or N, where, when they are defined as N, the corresponding substituent R1, R2, R3, R4, R5 is absent, or R2-D=E-R3 or R4-G=L-R5 are defined as S or O and where the five-membered or six-membered ring may be fused to $-(CH_2)_3-$ or -CH=CH-CH=CH- to form a bicyclic system;
R1, R2, R3, R4, R5 are each independently H, F, Cl, Br, I, CN, $CF_3$, $(C_1-C_8)$-alkyl, $(C_3-C_8)$- cycloalkyl, adamantan-1-yl, adamantan-2-yl, $(CH_2)_n$-aryl, $(CH_2)_n$-heteroaryl, $OCF_3$, O-R11, NR13R15, $S(O)_m$-R12, $SO_2-NH_2$, $SO_2-N=CH-N(CH_3)_2$, $SO_2$-NH-$[(C_1-C_8)$-alkyl], $SO_2$-NH-$[(C_3-C_8)$- cycloalkyl], $SO_2$-NH-$(CH_2)_n$-aryl, $SO_2$-NH-$(CH_2)_n$- heteroaryl, $SO_2-N[(C_1-C_8)\text{-alkyl}]_2$, $SO_2$-R16, $SF_5$, CO-O$[(C_1-C_8)$-alkyl],
CO-O$[(C_3-C_6)$-cycloalkyl], CO-NH2, CO-NH-$[(C_1$- $C_4)$-alkyl], CO-N $[(C_1-C_9)\text{ -alkyl}]_2$, CO-NH-$[(C_3$- $C_6)$-cycloalkyl], C (=NH)-O-$[(C_1-C_6$-alkyl)], C(=NH)-$NH_2$, C(=NH)-R16, C(=NR13)-NR12R13, $(CH_2)_n$-C(=$NSO_2$-R12)$NH_2$, CO-R16, COOH, CO-$(C_1$- $C_4)$-alkyl, CO-$(C_3-C_6)$-cycloalkyl, CO-aryl, CO- heteroaryl, CH(OH)-aryl, CH(OH)-heteroaryl, CHF-aryl, CHF-heteroaryl, $CF_2$-aryl, $CF_2$- heteroaryl, $CH_2$-OH, $CH_2$-CN, $CH_2$-O-R12, $CH_2$-O- $(CH_2)_q$-COOH,
where the alkyl and cycloalkyl radicals may be substituted by fluorine atoms, and where the aryl or heteroaryl radicals may be substituted by halogen, CN, $(C_1-C_4)$-alkyl, $(C_3-C_6)$-cycloalkyl, O-$(C_1-C_4)$-alkyl, $OCF_3$, OH, O-$(CH_2)_n$-aryl, $(CH_2)_n$-aryl, $S(O)_m$-$(C_1-C_4)$-alkyl, $SO_2-NH_2$, SH, NR12R13, NH-CO- $[(C_1-C_4)$ -alkyl], NH-CO-$(CH_2)_n$-aryl, $(CH_2)_n$-COOH, $(CH_2)_n$-$CONH_2$, $(CH_2)_n$-CO-O$(C_1-C_9)$ -alkyl, $(CH_2)_n$-CO-$(C_1-C_4)$- alkyl, and where the alkyl radicals may be substituted by fluorine atoms;
R6, R7, R8, R9, R10 are each independently T-bicyclic heterocycle, T-aryl or T-heteroaryl, where the bicyclic heterocycle or the aryl or heteroaryl radical may be fused to a 5- or 6-membered aromatic or nonaromatic carbon ring in which one or more CH or $CH_2$ groups may be replaced by oxygen atoms and where the 5- or 6-membered aromatic or nonaromatic carbon ring may be substituted by F, =0 or -$(C_1-C_6)$- alkyl and where the bicyclic heterocycle may contain from 9 to 12 ring members and up to five CH or $CH_2$ groups may each independently be replaced by N, NR20, 0, $S(O)_m$ or C=O and where the aryl or heteroaryl radical or bicyclic heterocycle may be unsubstituted or may be mono- or polysubstituted by

R11, F, Cl, Br, I, CN, $N_3$, NC, $NO_2$, $CF_3$, $(CH_2)_n$-O-R11, $(CH_2)_n$-O- $(CH_2)_r$-OH, $(CH_2)_n$- O-$CH(CH_2OH)_2$, $(CH_2)_n$-O-$(CH_2)_n$-CO-O-$(CH_2)_r$- $NH_2$, $(CH_2)_n$-O-$(CH_2)_n$-CO-NH-$(CH_2)_n$-OH, O- R13, $OCF_3$, $(CH_2)_n$-O-$(CH_2)_r$-$NH_2$, $(CH_2)_n$-NH- R11, $(CH_2)_n$-$N[(CH_2)_q\text{-CO-O}(C_1-C_6)\text{-alkyl}]_2$, $(CH_2)_n$-N $[(CH_2)_q$-COOH]2 , $(CH_2)_n$-N[$(CH_2)$q- $CONH_2]_2$, $(CH_2)_n$-NH-R13, $(CH_2)_n$-$N(R13)_2$, $(CH_2)_n$-NH-CN, $(CH_2)_n$-NH-$SO_2$-R16, $(CH_2)_n$- NH- $(CH_2)_n$-$SO_2$-R12, $(CH_2)_n$-NR12-CO-R16, $(CH_2)_n$-NR12-CO-NR12R13, (CH2)$_n$-NR12-CO-$N(R12)_2$, $(CH_2)_n$-NR12-CO-NHR11, $(CH_2)_n$-NH- C(=NH)-$NH_2$, $(CH_2)_n$-NH-C (=NH) -R16, $(CH_2)_n$- NH-C(=NH)-NHR12, $(CH_2)_n$-NR12-C(=NR13)- NHR12, $(CH_2)_n$-NR12-C(=NR12) -NR12R13, $(CH_2)_n$-NH-$(CH_2)_n$-CO-O-$(CH_2)_r$-$NH_2$, $(CH_2)_n$- NH-$(CH_2)_n$-CO-NH-$[(C_1-C_8)$-alkyl], $(CH_2)_n$- NH-$(CH_2)_n$ -CO-NH-

$(CH_2)_r$-OH, $(CH_2)_n$-NH- $(CH_2)_n$ -CO-N[(C1-C8)-alkyl]$_2$, $(CH_2)_n$-NH- (CH2)$_n$ -CO-NH-[($C_3$-$C_8$)-cycloalkyl], $(CH_2)_n$-NH-$(CH_2)_n$-CO-N[($C_3$-$C_8$)-cycloalkyl]$_2$ $(CH_2)_n$-NH-C$(CH_3)_2$-CO-O($C_1$-$C_8$)-alkyl, $(CH_2)_n$-NH-C$(CH_3)_2$-CO-O($C_3$-$C_8$)-cycloalkyl, $(CH_2)_n$-NH-C$(CH_3)_2$-CO-O-$(CH_2)_r$-$NH_2$, $(CH_2)_n$- NH-C$(CH_3)_2$-CO-O-$(CH_2)_n$-aryl, $(CH_2)_n$-NH- C$(CH_3)_2$-CO-O-$(CH_2)_n$-heteroaryl, $(CH_2)_n$- NH-C $(CH_3)$-CO-$NH_2$, (-$CH_2$)$_n$-NH-C$(CH_3)_2$-CO- NH- [($C_1$-$C_8$) -alkyl], $(CH_2)_n$-NH-C $(CH_3)_2$-CO- NH-$(CH_2)_r$-OH, $(CH_2)_n$-NH-C$(CH_3)_2$-CO-N[($C_1$- $C_8$)-alkyl]$_2$, $(CH_2)_n$-NH-$(CH_3)_2$-CO-NH-[($C_3$- $C_8$)-cycloalkyl], $(CH_2)_n$-NH-C$(CH_3)_2$-CO-N[($C_3$-$C_8$)-cycloalkyl]$_2$, $(CH_2)_n$-NH- C$(CH_3)_2$-COOH, S(O)$_m$-R12, $SO_2$-R16, $SO_2$- N=CH-N $(CH_3)_2$,

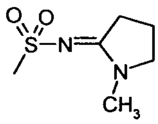,

$SO_2$-NH-CO-R12, $SO_2$-NHR12, $SO_2$-NH-$(CH_2)_r$-OH, $SO_2$-N[($C_1$- $C_8$)-alkyl]$_2$, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH-piperidin-1-yl, $(CH_2)_n$-CO- NH-$SO_2$-NHR12, $(CH_2)_n$-CO-NH-$SO_2$-R18, $(CH_2)_n$-CHO, $(CH_2)_n$-C (=NH) $NH_2$, $(CH_2)_n$- C(=NH)-NHOH, $(CH_2)_n$-C (=NH)- [NH-O-($C_1$-$C_6$)- alkyl], $(CH_2)_n$-C (=NH) (R16), $(CH_2)_n$- C(=NR13)NHR12, $(CH_2)_n$-C(=NR12)NR12R13, $(CH_2)_n$-C(=N$SO_2$-R12)$NH_2$, $(CH_2)_n$- C(=NH)O[($C_1$-$C_6$)-alkyl], where the alkyl and cycloalkyl radicals may be substituted by fluorine atoms and where the aryl or heteroaryl radicals may be substituted by halogen, CN, ($C_1$-$C_6$)- alkyl, ($C_3$-$C_6$)-cycloalkyl, O-($C_1$-$C_6$)- alkyl, S (O)$_m$-($C_1$-$C_6$) -alkyl, $SO_2$-NH2, COOH, CONH$_2$, CO-O($C_1$-$C_6$)-alkyl, CO-($C_1$- $C_6$)-alkyl, and where the alkyl radicals may be substituted by fluorine atoms,

and where, when R3 is CN, $NO_2$ or halogen and R4 is $CF_3$ or halogen and R and R' are each methyl, the X-aryl radical is provided with at least one of the abovementioned substituents other than hydrogen;

H, F, Cl, Br, I, CN, $N_3$, NC, $NO_2$, $CF_3$,
($C_1$-$C_8$)-alkyl, ($C_2$-$C_{10}$) -alkenyl, ($C_2$-$C_{10}$)- alkynyl, ($C_3$-$C_8$)-cycloalkyl,
aryl, heteroaryl,
$(CH_2)_n$-CO-[O-($C_1$-$C_8$)-alkyl], $(CH_2)_n$-CO-[O-($C_3$- $C_8$)-cycloalkyl], $(CH_2)_n$-CO-[($C_1$-$C_8$)-alkyl], $(CH_2)_n$-CO-[($C_3$-$C_8$)-Cycloalkyl],
$(CH_2)_n$-CO-[O-$(CH_2)_v$-aryl],
$(CH_2)_n$-CO-$NH_2$, $(CH_2)_n$-COOH, $(CH_2)_n$-CO-NH-CN, $(CH_2)_n$-P (O) (OH) [O-($C_1$-$C_6$)-alkyl], $(CH_2)_n$- P(O) [O-($C_1$-$C_6$)-alkyl]$_2$, $(CH_2)_n$-P (O) (OH)(O-$CH_2$- aryl), $(CH_2)_n$-P(O)(O-$CH_2$-aryl)$_2$, $(CH_2)_n$- P (O) $(OH)_2$,
$(CH_2)_n$-$SO_3H$, $(CH_2)_n$-$SO_2$-$NH_2$,
$(CH_2)_n$-CO-NH-[($C_1$-$C_8$)-alkyl], $(CH_2)_n$-CO-N[($C_1$- $C_8$)-alkyl]$_2$, $(CH_2)_n$-CO-NH-[($C_3$-$C_8$)-cycloalkyl], ($C_2$-$C_{10}$)-alkenyl-CO-O[($C_1$-$C_6$) -alkyl], ($C_2$-$C_{10}$)- alkenyl-CONH$_2$, ($C_2$-$C_{10}$)-alkenyl-COOH, ($C_2$-$C_{10}$)- alkynyl-CO-O[($C_1$-$C_6$)-alkyl], ($C_2$-$C_{10}$) -alkynyl- CONH$_2$, ($C_2$-$C_{10}$) -alkynyl-COOH,
$(CH_2)_n$-CO-R16,
$(CH_2)_n$-OH, $(CH_2)_n$-O-($C_1$-$C_8$)-alkyl, $(CH_2)_n$-O-($C_2$- $C_{10}$) -alkenyl, $(CH_2)_n$-O-($C_2$-$C_{10}$) -alkynyl, $(CH_2)_n$- O-($C_3$-$C_8$) -cycloalkyl, $(CH_2)_n$-O-$(CH_2)_q$-[($C_3$-$C_8$)- cycloalkyl], $(CH_2)_n$-O-$(CH_2)_n$-CO-[O-($C_1$-$C_8$)- alkyl], $(CH_2)_n$-O-$(CH_2)_n$-CO-[O-($C_3$-$C_8$)- cycloalkyl], $(CH_2)_n$-O-$(CH_2)_n$-CO-($C_1$-$C_8$)- alkyl], $(CH_2)_n$-O-$(CH_2)_n$-CO-[($C_3$-$C_8$)- Cycloalkyl], $(CH_2)_n$-O-$(CH_2)_n$-CO-[O-$(CH_2)_v$- aryl], $(CH_2)_n$-O- $(CH_2)_n$-CO-[O-$(CH_2)_v$- heteroaryl], $(CH_2)_n$-O-$(CH_2)_q$-CO-$NH_2$,$(CH_2)_n$-O- $(CH_2)_q$-COOH, $(CHF_2)_n$-O-$(CH_2)_q$-CO-NH-CN, $(CH_2)_n$-O- $(CH_2)_n$-P(O)(OH)[O-($C_1$-$C_6$)-alkyl], $(CH_2)_n$-O- $(CH_2)_n$-P(O)[O-($C_1$-$C_6$)-alkyl]$_2$, $(CH_2)_n$-O-$(CH_2)_n$- P(O)(OH)(O-$CH_2$-aryl), $(CH_2)_n$-O-$(CH_2)_n$-P(O)(O- $CH_2$-aryl)$_2$, $(CH_2)_n$-O-$(CH_2)_n$-P(O)$(OH)_2$, $(CH_2)_n$-O- $(CH_2)_n$-$SO_3H$, $(CH_2)_n$-O-$(CH_2)_n$-$SO_2$-$NH_2$, $(CH_2)_n$-O- $(CH_2)_n$-CO-NH-[($C_1$-$C_8$)-alkyl], $(CH_2)_n$-O-$(CH_2)_n$- CO-N[($C_1$-$C_8$)-alkyl]$_2$, $(CH_2)_n$-O-$(CH_2)_n$-CO-NH- [($C_3$-$C_8$)-cycloalkyl], $(CH_2)_n$-O-$(CH_2)_n$-CR21R22- CO-O[($C_1$-$C_6$)-alkyl], $(CH_2)_n$-O-$(CH_2)_n$-CR21R22- CO-$NH_2$, $(CH_2)_n$-O-$(CH_2)_n$-CR21R22-COOH, $(CH_2)_n$-O- $(CH_2)_n$-CO-R16, $(CH_2)_n$-O-$(CH_2)_r$-OH, $(CH_2)_n$-O- CH$(CH_2OH)_2$, $(CH_2)_n$-O-$(CH_2)_n$-CO-O- $(CH_2)_r$-$NH_2$, $(CH_2)_n$-O-$(CH_2)_n$-CO-NH-$(CH_2)_r$-OH, O-R13 , $OCF_3$,
$(CH_2)_n$-$NH_2$, $(CH_2)_n$-NH-($C_1$-$C_8$)-alkyl, $(CH_2)_n$-NH- ($C_3$-$C_8$)-cycloalkyl, $(CH_2)_n$-NH-$(CH_2)_n$-CO-[O-($C_3$- $C_8$)-cycloalkyl], $(CH_2)_n$-NH-$(CH_2)_n$-CO-[($C_1$-$C_8$)- alkyl], $(CH_2)_n$-NH-$(CH_2)_n$-CO-[($C_3$-$C_8$)- cycloalkyl], $(CH_2)_n$-NH-$(CH_2)_n$-CO-[O-$(CH_2)_v$- aryl], $(CH_2)_n$-NH-$(CH_2)_n$-CO-[O-(CH2)$_v$- heteroaryl], $(CH_2)_n$-NH-$(CH_2)_q$-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-NH2,
$(CH_2)_n$-NH-$(CH_2)_n$-CR21R22-CO-O[($C_1$-$C_6$)-alkyl],

$(CH_2)_n$-NH-$(CH_2)_n$-CR21R22-$CONH_2$, $(CH_2)_n$-NH- $(CH_2)_n$-CR21R22-COOH,
$(CH_2)_n$-NH-$(CH_2)_n$-CO-R16,
$(CH_2)_n$-NH-$(CH_2)_n$-$SO_2$-[($C_1$-$C_8$)-alkyl], $(CH_2)_n$-NH- $(CH_2)_n$-$SO_2$-[($C_3$-$C_8$)-Cycloalkyl], $(CH_2)_n$-NH-$SO_2$-$(CH_2)_n$-$NH_2$, $(CH_2)_n$-NH-$SO_2$-$(CH_2)_n$-NH-($C_1$-$C_8$)- alkyl, $(CH_2)_n$-NH-$SO_2$-$(CH_2)_n$-NH-($C_3$-$C_8$)- cycloalkyl, $(CH_2)_n$-NH-$SO_2$-$(CH_2)_n$-N[($C_1$-$C_8$)- alkyl]$_2$,
$(CH_2)_n$-NH-CN, $(CH_2)_n$-NH-$SO_2$-R16,
$(CH_2)_n$-NR12-CO-NH-($C_1$-$C_8$)-alkyl, $(CH_2)_n$-NR12- CO-NH-($C_3$-$C_8$)-cycloalkyl, $(CH_2)_n$-NR12-CO-NH2, $(CH_2)_n$-NR12-CO-NH-$SO_2$-($C_1$-$C_8$)-alkyl, $(CH_2)_n$- NR12-CO-NH-$SO_2$-($C_3$-$C_8$)-cycloalkyl, $(CH_2)_n$-NR12-CO-N[($C_1$-$C_8$)-alkyl]$_2$, $(CH_2)_n$-NH-CO-NH-$(CH_2)_n$- CO-[O-($C_1$-$C_8$)-alkyl], $(CH_2)_n$-NH-CO-NH-$(CH_2)_q$- CO-$NH_2$, $(CH_2)$n-NH-CO-NH-$(CH_2)_q$-COOH, $(CH_2)_n$- NH-C(=NH)-$NH_2$, $(CH_2)_n$-NH-C(=NH)-R16, $(CH_2)_n$-NH-C(=NH)-NH[($C_1$-$C_8$)-alkyl], $(CH_2)_n$-NH-C(=N-$SO_2$- ($C_1$-$C_8$)-alkyl)-$NH_2$, $(CH_2)_n$-NH-C(=N-$SO_2$-($C_1$-$C_8$)-alkyl)-NH[($C_1$-$C_8$)-alkyl], $(CH_2)_n$-NH-C(=N-$SO_2$- $NH_2$)-$NH_2$, $(CH_2)_n$-NH-C(=N-$SO_2$-$NH_2$)-NH[($C_1$-$C_8$)- alkyl], $(CH_2)_n$-NH-C(=NH)-N[($C_1$-$C_8$)-alkyl]$_2$, $(CH_2)_n$-NH-C(=N-$SO_2$-($C_1$-$C_8$)-alkyl)-N[($C_1$-$C_8$)- alkyl]$_2$, $(CH_2)_n$-NH-$(CH_2)_n$-CO-O-$(CH_2)_r$-$NH_2$, $(CH_2)_n$-NH-$(CH_2)_n$-CO-NH-[($C_1$-$C_8$)-alkyl], $(CH_2)_n$- NH-$(CH_2)_n$-CO-NH-$(CH_2)_r$-OH, $(CH_2)_n$-NH-$(CH_2)_n$- CO-N[($C_1$-$C_8$)-alkyl]$_2$, $(CH_2)_n$-NH$(CH_2)_n$ -CO-NH- [($C_3$-$C_8$)-cycloalkyl], $(CH_2)_n$-NH-C$(CH_3)_2$-CO-O($C_3$- $C_8$)-cycloalkyl, $(CH_2)_n$-NH-C$(CH_3)_2$-CO-O-$(CH_2)_r$- $NH_2$, $(CH_2)_n$-NH-C$(CH_3)_2$-CO-O-$(CH_2)_n$-aryl, $(CH_2)_n$- NH-C$(CH_3)_2$-CO-O-$(CH_2)_n$-heteroaryl, $(CH_2)_n$-NH- C$(CH_3)_2$-CO-NH-[($C_1$-$C_8$)-alkyl], $(CH_2)_n$-NH- C$(CH_3)_2$-CO-NH-$(CH_2)_r$-OH, $(CH_2)_n$-NH-C$(CH_3)_2$-CO- N[($C_1$-$C_8$)-alkyl $_2$, $(CH_2)_n$-NH-( $CH_3)_2$-CO-NH-[($C_3$- $C_8$)-cycloalkyl], $(CH_2)_n$-NH-C$(CH_3)_2$-CO-N[($C_3$-$C_8$) cycloalkyl]$_2$,
$(CH_2)_n$-S(O)$_m$-($C_1$-$C_8$)-alkyl, $(CH_2)_n$-S(O)$_m$-($C_3$-$C_8$)- cycloalkyl, $(CH_2)_n$-$SO_2$-R16, $SO_2$-N=CH-N$(CH_3)_2$,

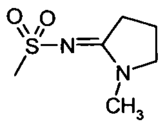
,

$(CH_2)_n$-$SO_2$-NH-CO-($C_1$-$C_8$)-alkyl, $(CH_2)_n$-$SO_2$-NH-CO-($C_3$-$C_8$)-cycloalkyl, $(CH_2)_n$-$SO_2$- NH-($C_1$-$C_8$)-alkyl, $(CH_2)_n$-$SO_2$-NH-($C_3$-$C_8$)- cycloalkyl, $(CH_2)_n$-$SO_2$-N[($C_1$-$C_8$)-alkyl]$_2$, $SO_2$- NH-$(CH_2)_r$-OH, $SO_2$-NH-$(CH_2)_r$-$NH_2$, $SF_5$,
$(CH_2)_q$-CN,
$(CH_2)_n$-CO-NH-piperidin-1-yl,
$(CH_2)_n$-CO-NH-$SO_2$-NHR12, $(CH_2)_n$-CO-NH-$SO_2$- ($C_1$- $C_8$)-alkyl, $(CH_2)_n$-CO-NH-$SO_2$-($C_3$-$C_8$)-cycloalkyl, $(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[($C_1$-$C_6$)- alkyl],
where the alkyl and cycloalkyl radicals may be substituted by fluorine atoms and where the aryl or heteroaryl radicals may be substituted by halogen, CN, ($C_1$-$C_6$)-alkyl, ($C_3$-$C_6$)-cycloalkyl, O-($C_1$-$C_6$)-alkyl, S(O)$_m$-($C_1$-$C_6$)-alkyl, $SO_2$-$NH_2$, COOH, $CONH_2$, CO-[O($C_1$-$C_6$)- alkyl], CO-($C_1$-$C_6$)-alkyl and where the alkyl radicals may be substituted by fluorine atoms;

where at least one of the R6, R7, R8, R9 and R10 radicals is always defined as T-bicyclic heterocycle, T-aryl or T-heteroaryl and
where one of the four radical pairs of R6 and R7, or R7 and R8, or R8 and R9, or R9 and R10 may in each case together form the -$CH_2$-$CH_2$-$CH_2$- or -$CH_2$-$CH_2$-$CH_2$-$CH_2$- groups in which up to two -$CH_2$- groups may be replaced by -O- and where the -$CH_2$-$CH_2$-$CH_2$- or -$CH_2$-$CH_2$-$CH_2$-$CH_2$- groups may be substituted by F, ($C_1$-$C_8$)-alkyl or =0;

T is NR23-CO-NR24, NR23-$SO_2$-NR24, $SO_2$-NR23-$SO_2$, CO-NR23-CO, NR23-C(=NR13)-NR24, NR23-C(=NR22)-NR24, CO-NR23-CR22R23, NR23-CO- CR22R24, NR23-$SO_2$-CR22R24, CR22R24-CO-NR23, CR22R24-$SO_2$-NR23, CR22R23-NR23-$SO_2$, $SO_2$- CR22R23-NR23, $SO_2$-NR23-CR22R23-, NR23-CR22R23-$SO_2$, CR23R24-CR23R24-CR23R24, CR23R24-NR23- CR23R24;
R11 is H, ($C_1$-$C_8$)-alkyl, ($C_2$-$C_{10}$)-alkynyl, ($C_3$-$C_6$)- cycloalkyl, $(CH_2)_n$-aryl, $(CH_2)_n$-CO-[O-($C_1$-$C_6$)- alkyl], $(CH_2)_n$-CO-[O-($C_3$-$C_6$)-cycloalkyl], $(CH_2)_n$-CO-[($C_1$-$C_6$)-alkyl], $(CH_2)_n$-CO-[($C_3$-$C_6$)- cycloalkyl], $(CH_2)_n$-CO-aryl, $(CH_2)_n$-CO- heteroaryl, $(CH_2)_q$-CO-$NH_2$, $(CH_2)_q$-COOH, $(CH_2)_n$- P(O)(OH)[O-($C_1$-$C_6$)-alkyl], $(CH_2)_n$-P(O)[O-($C_1$- $C_4$)-alkyl]$_2$, $(CH_2)_n$-P(O)(O-$CH_2$-aryl)$_2$, $(CH_2)_n$- P(O)$(OH)_2$, $(CH_2)_n$-$SO_3H$, $(CH_2)_n$-$SO_2$-$NH_2$, $(CH_2)_n$- CO-NH-[($C_1$-$C_4$)-alkyl], $(CH_2)_n$-CO-N[($C_1$-$C_4$) alkyl]$_{2,}$ $(CH_2)_n$-CO-NH-[($C_3$-$C_6$)-cycloalkyl], ($C_2$-$C_6$)-alkenyl-CO-O[($C_1$-$C_4$)-alkyl], ($C_2$-$C_6$)- alkenyl-$CONH_2$, ($C_2$-$C_6$)-alkenyl-COOH, ($C_2$-$C_6$)- alkynyl-CO-O[($C_1$-$C_4$)-alkyl] , ($C_2$-$C_6$)-alkynyl- $CONH_2$, ($C_2$-$C_6$)-alkynyl-COOH, $(CH_2)_n$-CR21[(CO- O($C_1$-$C_4$)-alkyl)]$_2$, $(CH_2)_n$-CR21$(CONH_2)_2$, $(CH_2)_n$- CR21$(COOH)_2$, $(CH_2)_n$-CR21R22-CO-O[($C_1$-$C_4$)- alkyl], $(CH_2)_n$-CR21R22-

$CONH_2$, $(CH_2)_n$-CR21R22- CO-NH-[($C_1$-$C_4$)-alkyl], $(CH_2)_n$-CR21R22-CO-N[($C_1$- $C_4$)-alkyl]$_2$, $(CH_2)_n$-CR21R22-COOH, $(CH_2)_n$-CO- R16, $(CH_2)_n$-CO-NH-C$(CH_3)_2$-CO-O[($C_1$-$C_8$)-alkyl], $(CH_2)_n$-CO-NH-C$(CH_3)_2$-$CONH_2$, $(CH_2)_n$-CO-NH- C$(CH_3)_2$-COOH,

where the alkyl, alkenyl, alkynyl and cycloalkyl radicals may be substituted by fluorine atoms and where the aryl or heteroaryl radical may be substituted by halogen, CN, ($C_1$-$C_4$)-alkyl, O-($C_1$-$C_4$)-alkyl, S(O)$_m$-($C_1$-$C_4$)-alkyl, $SO_2$-$NH_2$, COOH, $CONH_2$, CO- O($C_1$-$C_4$)-alkyl, and where the alkyl radicals may be substituted by fluorine atoms;

R12 is H, ($C_1$-$C_8$)-alkyl, ($C_3$-$C_8$)-cycloalkyl, $(CH_2)_n$-aryl, $(CH_2)_n$-heteroaryl, where the alkyl or cycloalkyl radicals may be substituted by fluorine atoms,

and where the aryl or heteroaryl radical may be substituted by halogen, CN, ($C_1$-$C_4$)-alkyl, O-($C_1$-$C_4$)-alkyl, $SO_2$-$NH_2$, COOH, $CONH_2$, CO-O($C_1$- $C_4$)-alkyl and where the alkyl radicals may be substituted by fluorine atoms;

R13 is H, $SO_2$[($C_1$-$C_8$)-alkyl], $SO_2$-($C_3$-$C_8$)- cycloalkyl], $SO_2$-(CH2)$_n$-aryl, $SO_2$-$(CH_2)_n$-heteroaryl

where the alkyl and cycloalkyl radicals may be substituted by fluorine atoms and where the aryl or heteroaryl radical may be substituted by halogen, CN, $CF_3$, ($C_1$-$C_4$)- alkyl, O-[($C_1$-$C_4$)-alkyl], S(O)$_m$-[($C_1$-$C_6$)- alkyl], $SO_2$-$NH_2$, COOH, $CONH_2$, CO-[O($C_1$-$C_4$)- alkyl] and where the alkyl radicals may be substituted by fluorine atoms;

R15 is ($C_1$-$C_6$)-alkyl,

where the alkyl radical may be substituted by fluorine atoms;

R16 is aziridin-1-yl, azetidin-1-yl, 3- hydroxyazetidin-1-yl, piperidin-1-yl, pyrrolidin-1-yl, 3-pyrrolidinol-1-yl, morpholin-N-yl, piperazin-1-yl, 4-[($C_1$-$C_6$)- alkyl]piperazin-1-yl, thiomorpholine-1,1- dioxid-4-yl, NH-$(CH_2)_r$-OH, NH-CH$(CH_2OH)_2$, NH- C$(CH_2OH)_3$, N [($C_1$-$C_4$)-alkyl-OH]$_2$, D-glucamin-N- yl, N-methyl-D-glucamin-N-yl, NH-($C_1$-$C_4$)- alkyl]-COOH, NH-[($C_1$-$C_4$)-alkyl]-$CONH_2$, N[($C_1$- $C_4$)-alkyl][($C_1$-$C_4$)-alkyl]-COOH, NH- [C(H)(aryl)]-CO-O ($C_1$-$C_4$)-alkyl, NH- [C(H)(aryl)]-COOH, NH-[C(H)(aryl)]-$CONH_2$, NH- [C(H)(heteroaryl)]-CO-O($C_1$-$C_4$)-alkyl, NH- [C(H)(heteroaryl)]-COOH, NH- [C(H)(heteroaryl)]-$CONH_2$, NH-[($C_3$-$C_6$)- cycloalkyl]-CO-O ($C_1$-$C_4$)-alkyl, NH-[($C_3$-$C_6$)- cycloalkyl]-COOH, NH-[($C_3$-$C_6$)-cycloalkyl]- $CONH_2$, NH-($C_1$-$C_4$)-alkyl-OH, NH-[($C_1$-$C_4$)-alkyl]- $SO_2$-($C_1$-$C_4$)-alkyl, NH-[($C_1$-$C_4$)-alkyl]-$SO_3H$, NH-[($C_1$-$C_4$)-alkyl]-$SO_2$-$NH_2$,

where the alcohol (OH) functions may be replaced by F and where the aryl or heteroaryl radical may be substituted by halogen, CN, ($C_1$-$C_4$)-alkyl, O-($C_1$-$C_4$)-alkyl, OH, $SO_2$-$NH_2$, COOH, $CONH_2$, CO-O($C_1$-$C_4$)-alkyl;

R18 is $(CH_2)_n$-CR25R26-CO-O($C_1$-$C_4$) -alkyl, $(CH_2)_n$- CR25R26-CO-$NH_2$, $(CH_2)_n$-CR25R26-COOH;

R20 is H, ($C_1$-$C_4$-alkyl, ($C_3$-$C_6$)-cycloalkyl, aryl, [($C_1$-$C_4$)-alkyl]-aryl;

R21 is H, F, $CF_3$, ($C_1$-$C_4$)-alkyl, ($C_3$-$C_6$)- cycloalkyl, OH, O-($C_1$-$C_4$)-alkyl, O-($C_3$-$C_6$)- cycloalkyl, O-$(CH_2)_n$-aryl, O-(CO)-($C_1$-$C_4$)- alkyl, O-(CO)-($C_3$-$C_6$)-cycloalkyl, O-(CO)-O- ($C_1$-$C_4$) -alkyl, O-(CO)-O-($C_3$-$C_6$)-cycloalkyl, NH-[($C_1$-$C_4$) -alkyl] -aryl, $NH_2$, NH- ($C_1$-$C_4$) -alkyl, NH-(CO)-($C_1$-$C_4$)-alkyl;

R22 is H, $CF_3$, ($C_1$-$C_4$)-alkyl, aryl, [($C_1$-$C_4$)-alkyl]-aryl;

R23, R24 are each independently H, ($C_1$-$C_4$)-alkyl, ($C_3$- $C_6$)-cycloalkyl, [($C_1$-$C_4$)-alkyl]-[($C_3$-$C_6$)- cycloalkyl], aryl, [($C_1$-$C_4$)-alkyl]-aryl or R23 and R24 together form a -CH=CH-, -$CH_2$- $CH_2$-, -$CH_2$-$CH_2$-$CH_2$- or -$CH_2$-$CH_2$-$CH_2$-$CH_2$- unit in which one $CH_2$ moiety may be replaced by C=O, CHF or $CF_2$, and in which up to four hydrogen atoms may be replaced by a ($C_1$-$C_{74}$)-alkyl radical;

R25, R26 are each independently H, F, ($C_1$-$C_4$)-alkyl, aryl, [($C_1$-$C_4$)-alkyl]-aryl,

where the aryl may be substituted by halogen, CN, OH, O-($C_1$-$C_4$)-alkyl,

or the R25 and R26 radicals, together with the carbon atom bonded to them, form a three- to seven-membered carbocycle in which one carbon atom may be replaced by 0, S(O)$_m$, NH, N[($C_1$-$C_4$)-alkyl] or CO;

excluding the compound N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)urea,
and the physiologically compatible salts thereof.

**4.** A compound of the formula I as claimed in one or more of claims 1 to 3, wherein

R, R' are each independently H, aryl, ($C_1$-$C_4$)-alkyl, where ($C_1$-$C_4$)-alkyl or the aryl radical may be substituted by halogen;

or R and R' together form a ring having from three to eight carbon atoms, where one carbon atom may be replaced by O, S(O)$_m$, NR13 or NR15;

m is 0, 1, 2;

n is 0, 1, 2;

p is 1, 2, 3;

q is 1, 2;

r is 2, 3;
v is 0, 1, 2;
A, D, E, G, L are each independently C or N, where, when they are defined as N, the corresponding substituent R1, R2, R3, R4, R5 is absent,
or R2-D=E-R3 or R4-G=L-R5 are defined as S or O and where the five-membered or six-membered ring may be fused to $-(CH_2)_3-$ or $-(CH_2)_4-$ or -CH=CH-CH=CH- to form a bicyclic system;
R1, R2, R3, R4, R5 are each independently H, F, Cl, Br, I, CN, $CF_3$, $(C_1-C_8)$-alkyl, $(C_3-C_8)$- cycloalkyl, $(CH_2)_n$-aryl, $(CH_2)_n$-heteroaryl, $OCF_3$, O-R11, NR13R15, $S(O)_m$-R12, $SO_2-NH_2$, $SO_2$- NH-$[(C_1-C_8)$-alkyl], $SO_2$-NH-$[(C_3-C_8)$-cycloalkyl], $SO_2$-NH-$(CH_2)_n$-aryl, $SO_2$-NH-$(CH_2)_n$- heteroaryl, $SO_2$-N$[(C_1-C_8)$-alkyl$]_2$, $SO_2$-R16, $SF_5$, CO-O$[(C_1-C_8)$-alkyl],
CO-O$[(C_3-C_6)$-cycloalkyl], CO-$NH_2$, CO-NH-$[(C_1$- $C_4)$-alkyl], CO-N$[(C_1-C_4)$-alkyl$]_2$, C(=NH)-$NH_2$, C(=NH)-R16, $(CH_2)_n$-C(=$NSO_2$-R12)$NH_2$, CO-R16, COOH, CO-$(C_1-C_4)$-alkyl, CO-$(C_3-C_6)$-cycloalkyl, CO-aryl, CO-heteroaryl, CH(OH)-aryl, CH(OH)- heteroaryl, CHF-aryl, CHF-heteroaryl, $CF_2$- aryl, $CF_2$-heteroaryl, $CH_2$-OH, $CH_2$-CN, $CH_2$-O- R12, $CH_2$-O-$(CH_2)_q$-COOH,
where the alkyl and cycloalkyl radicals may be substituted by fluorine atoms, and where the aryl or heteroaryl radicals may be substituted by halogen, CN, $(C_1-C_4)$-alkyl, O- $(C_1-C_4)$-alkyl, $OCF_3$, OH, O-$(CH_2)_n$-aryl, $(CH_2)_n$-aryl, $S(O)_m$-$(C_1-C_4)$-alkyl, $SO_2-NH_2$, SH, NR12R13, NH-CO-$[(C_1-C_4)$-alkyl], NH-CO-$(CH_2)_n$- aryl, $(CH_2)_n$-COOH, $(CH_2)_n$-$CONH_2$, $(CH_2)_n$-CO-O $(C_1$- $C_4)$-alkyl, $(CH_2)_n$-CO-$(C_1-C_4)$-alkyl, and where the alkyl radicals may be substituted by fluorine atoms;
R6, R7, R8, R9, R10 are each independently T-bicyclic heterocycle, T-aryl or T-heteroaryl, where the bicyclic heterocycle or the aryl or heteroaryl radical may be fused to a 5- or 6-membered aromatic or nonaromatic carbon ring in which one or more CH or $CH_2$ groups may be replaced by oxygen atoms and where the 5- or 6-membered aromatic or nonaromatic carbon ring may be substituted by F, =O or -$(C_1-C_6)$- alkyl and where the bicyclic heterocycle may contain from 9 to 12 ring members and up to five CH or $CH_2$ groups may each independently be replaced by N, NR20, O, $S(O)_m$ or C=O and where the aryl or heteroaryl radical or bicyclic heterocycle may be unsubstituted or may be mono- or polysubstituted by

R11, F, C1, Br, I, CN, $N_3$, NC, $NO_2$, $CF_3$, $(CH_2)_n$-O-R11, $(CH_2)_n$-O-$(CH_2)_r$-OH, $(CH_2)_n$- O-CH$(CH_2OH)_2$, $(-CH_2)_n$-O-$(CH_2)_n$-CO-O-$(CH_2)_r$- $NH_2$, $(CH_2)_n$-O-$(CH_2)_n$-CO-NH-$(CH_2)_r$-OH, O- R13, $OCF_3$, $(CH_2)_n$-O-$(CH_2)_r$-$NH_2$, $(CH_2)_n$-NH- R11, $(CH_2)_n$-N$[(CH_2)_q$-CO-O$(C_1-C_6)$-alkyl$]_2$, $(CH_2)_n$-N$[(CH_2)_q$-COOH$]_2$, $(CH_2)_n$-N$[(CH_2)_q$- $CONH_2]_2$, $(CH_2)_n$-NH-R13, $(CH_2)_n$-N$(R13)_2$, $(CH_2)_n$-NH-CN, $(CH_2)_n$-NH-$SO_2$-R16, $(CH_2)_n$- NH-$(CH_2)_n$-$SO_2$-R12, $(CH_2)_n$-NR12-CO-R16, $(CH_2)_n$-NR12-CO-NR12R13, $(CH_2)_n$-NR12-CO-N$(R12)_2$, $(CH_2)_n$-NR12-CO-NHR11, $(CH_2)_n$-NH- C(=NH)-$NH_2$, $(CH_2)_n$-NH-C(=NH)-R16, $(CH_2)_n$- NH-C(=NH)-NHR12, $(CH_2)_n$-NR12-C(=NR13)- NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$- NH-$(CH_2)_n$-CO-NH-$[(C_1-C_8)$-alkyl], $(CH_2)_n$- NH-$(CH_2)_n$-CO-NH-$(CH_2)_r$-OH, $(CH_2)_n$-NH- $(CH_2)_n$-CO-N$[(C_1-C_8)$-alkyl$]_2$, $(CH_2)_n$-NH- $(CH_2)_n$-CO-NH-$[(C_3-C_8)$-cycloalkyl], $(CH_2)_n$-NH-$(CH_2)_n$ -CO-N$[(C_3-C_8)$- cycloalkyl$]_2$, $(CH_2)_n$-NH-C$(CH_3)_2$-CO-O$(C_1$- $C_8)$-alkyl, $(CH_2)_n$-NH-C$(CH_3)_2$-CO-O$(C_3-C_8)$- cycloalkyl, $(CH_2)_n$-NH-C$(CH_3)_2$-CO-O- $(CH_2)_r$-$NH_2$, $(CH_2)_n$-NH-C$(CH_3)_2$-CO-O-$(CH_2)_n$- aryl, $(CH_2)_n$-NH-C$(CH_3)_2$-CO-O-$(CH_2)_n$- heteroaryl, $(CH_2)_n$-NH-C$(CH_3)_2$-CO-$NH_2$, $(CH_2)_n$-NH-C$(CH_3)_2$-CO-NH-$[(C_1-C_8)$-alkyl], $(CH_2)_n$-NH-C$(CH_3)_2$-CO-NH-$(CH_2)_r$-OH, $(CH_2)_n$- NH-C$(CH_3)_2$-CO-N$[(C_1-C_8)$-alkyl$]_2$, $(CH_2)_n$- NH-$(CH_3)_2$-CO-NH-$[(C_3-C_8)$-cycloalkyl $(CH_2)_n$-NH-C$(CH_3)_2$-CO-N$[(C_3-C_8)$-cycloalkyl$]_2$, $(CH_2)_n$-NH-C$(CH_3)_2$-COOH, $S(O)_m$-R12, $SO_2$-R16, $SO_2$N=CH-N $(CH_3)_2$,

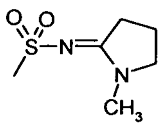
,

$SO_2$-NH-CO-R12, $SO_2$-NHR12, $SO_2$-NH-$(CH_2)_r$-OH, $SO_2$-N$[(C_1-C_8)$-alkyl$]_2$, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH- piperidin-1-yl, $(CH_2)_n$-CO-NH-$SO_2$-NHR12, $(CH_2)_n$-CO-NH-$SO_2$-R18, $(CH_2)_n$-CHO, $(CH_2)_n$- C(=NH)-$NH_2$, $(CH_2)_n$-C(=NH)-NHOH, $(CH_2)_n$- C(=NH)-[NH-O-$(C_1-C_6)$-alkyl], $(CH_2)_n$- C(=NH)(R16), $(CH_2)_n$-C(=NR13)NHR12, $(CH_2)_n$-C(=NR12)NR12R13, $(CH_2)_n$-C(=$NSO_2$- R12)$NH_2$, $(CH_2)_n$-C(=NH)O$[(C_1-C_6)$-alkyl], where the alkyl and cycloalkyl radicals may be substituted by fluorine atoms and where the aryl or heteroaryl radicals may be substituted by halogen, CN, $(C_1-C_6)$-alkyl, $(C_3-C_6)$-cycloalkyl, O- $(C_1-C_6)$-alkyl, $S(O)_m$-$(C_1-C_6)$-alkyl, $SO_2$-$NH_2$, COOH, $CONH_2$, CO-O$(C_1-C_6)$-alkyl, CO- $(C_1-C_6)$-alkyl and where the alkyl radicals may be substituted

by fluorine atoms,
and where, when R3 is CN, $NO_2$ or halogen and R4 is $CF_3$ or halogen and R and R' are each methyl, the X-aryl radical is provided with at least one of the abovementioned substituents other than hydrogen;

H, F, Cl, Br, I, CN, $N_3$, NC, $NO_2$, $CF_3$,
$(C_1-C_8)$-alkyl, $(C_2-C_{10})$-alkenyl, $(C_2-C_{10})$- alkynyl, $(C_3-C_8)$-cycloalkyl,
aryl, heteroaryl,
$(CH_2)_n$-CO-[O-$(C_1-C_8)$-alkyl], $(CH_2)_n$-CO-[O-$(C_3$- $C_8)$-cycloalkyl], $(CH_2)_n$-CO-[$(C_1-C_8)$-alkyl], $(CH_2)_n$-CO-[(3-$C_8)$-cycloalkyl],
$(CH_2)_n$-CO-[O-$(CH_2)_v$-aryl],
$(CH_2)_n$-CO-$NH_2$, $(CH_2)_n$-COOH, $(CH_2)_n$-CO-NH-CN,
$(CH_2)_n$-P(O)(OH)[O-$(C_1-C_6)$-alkyl], $(CH_2)_n$- P(O)[O-$(C_1-C_6)$-alkyl$]_2$, $(CH_2)_n$-P(O)(OH)(O-$CH_2$- aryl), $(CH_2)_n$-P(O)(O-$CH_2$-aryl$)_2$, $(CH_2)_n$- P(O)$(OH)_2$,
$(CH_2)_n$-$SO_3H$, $(CH_2)_n$-$SO_2$-$NH_2$,
$(CH_2)_n$-CO-NH-[$(C_1-C_8)$-alkyl], $(CH_2)_n$-CO-N[($C_1$- $C_8)$-alkyl$]_2$, $(CH_2)_n$-CO-NH- [$(C_3-C_8)$-cycloalkyl], $(C_2-C_{10})$-alkenyl-CO-O[$(C_1-C_6)$-alkyl], $(C_2-C_{10})$- alkenyl-CON$H_2$, $(C_2-C_{10})$-alkenyl-COOH, $(C_2-C_{10})$- alkynyl-CO-O[$(C_1-C_6)$-alkyl], $(C_2-C_{10})$-alkynyl- CON$H_2$, $(C_2-C_{10})$-alkynyl-COOH,
$(CH_2)_n$-CO-R16,
$(CH_2)_n$-OH, $(CH_2)_n$-O-$(C_1-C_8)$-alkyl, $(CH_2)_n$-O-($C_2$- $C_{10})$-alkenyl, $(CH_2)_n$-O-$(C_2-C_{10})$-alkynyl, $(CH_2)_n$- O-$(C_3-C_8)$-cycloalkyl, (-$CH_2)_n$-O-$(CH_2)_q$-[$(C_3-C_8)$- cycloalkyl], $(CH_2)_n$-O-$(CH_2)_n$-CO-[O-$(C_1-C_8)$- alkyl], (-$CH_2)_n$-O-$(CH_2)_n$-CO-[O-$(C_3-C_8)$- cycloalkyl], $(CH_2)_n$-O-$(CH_2)_n$-CO-[$(C_1-C_8)$- alkyl], $(CH_2)_n$-O-$(CH_2)_n$-CO-[$(C_3-C_8)$- cycloalkyl], $(CH_2)_n$-O-$(CH_2)_n$-CO-[O-$(CH_2)_v$- aryl], $(CH_2)_n$-O-$(CH_2)_n$-CO-[O-$(CH_2)_v$- heteroaryl], $(CH_2)_n$-O-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O- $(CH_2)_n$-P(O)(OH)[O-$(C_1-C_6)$-alkyl], $(CH_2)_n$-O- $(CH_2)_n$-P(O)[O-$(C_1-C_6)$-alkyl$]_2$, $(CH_2)_n$-O-$(CH_2)_n$- P(O)(OH)(O-$CH_2$-aryl), $(CH_2)_n$-O-$(CH_2)_n$-P(O)(O- $CH_2$-aryl$)_2$, $(CH_2)_n$-O-(CH2$)_n$-P(O)$(OH)_2$, $(CH_2)_n$-O- $(CH_2)_n$-$SO_3H$, $(CH_2)_n$-O-$(CH_2)_n$-$SO_2$-$NH_2$, $(CH_2)_n$-O- $(CH_2)_n$-CO-NH-[$(C_1-C_8)$-alkyl], $(CH_2)_n$-O-$(CH_2)_n$- CO-N[$(C_1-C_8)$-alkyl$]_2$, $(CH_2)_n$-O-$(CH_2)_n$-CO-NH- [$(C_3-C_8)$-cycloalkyl] (-$CH_2)_n$-O-$(CH_2)_n$-CR21R22- CO-O[$(C_1-C_6)$-alkyl], $(CH_2)_n$-O-$(CH_2)_n$-CR21R22- CON$H_2$, $(CH_2)_n$-O-$(CH_2)_n$-CR21R22-COOH, $(CH_2)_n$-O- $(CH_2)_n$-CO-R16, $(CH_2)_n$-O-$(CH_2)_r$-OH, $(CH_2)_n$-O- CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$,
$(CH_2)_n$-$NH_2$, $(CH_2)_n$-NH-$(C_1-C_8)$-alkyl, $(CH_2)_n$-NH- $(C_3-C_8)$-cycloalkyle, $(CH_2)_n$-NH-$(CH_2)_n$-CO-[O-($C_3$- $C_8)$-cycloalkyle], $(CH_2)_n$-NH-$(CH_2)_n$-CO-[$(C_1-C_8)$- alkyl], $(CH_2)_n$-NH-$(CH_2)_n$-CO-[$(C_3-C_8)$- cycloalkyl], $(CH_2)_n$-NH-$(CH_2)_n$-CO-[O-$(CH_2)_v$- aryl], $(CH_2)_n$-NH-$(CH_2)_n$-CO-[O-$(CH_2)$v- heteroaryl], $(CH_2)_n$-NH-$(CH_2)_q$-CO-NH-CN, $(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[$(C_1-C_6)$-alkyl],
$(CH_2)_n$-NH-$(CH_2)_n$-CR21R22-CON$H_2$, $(CH_2)_n$-NH- $(CH_2)_n$-CR21R22-COOH,
$(CH_2)_n$-NH-$(CH_2)_n$-CO-R16,
$(CH_2)_n$-NH-$(CH_2)_n$-$SO_2$-[$(C_1-C_8)$-alkyl], $(CH_2)_n$-NH- $(CH_2)_n$-$SO_2$-[$(C_3-C_8)$-cycloalkyl], $(CH_2)_n$-NH-$SO_2$-$(CH_2)_n$-$NH_2$, $(CH_2)_n$-NH-$SO_2$-$(CH_2)_n$-NH-$(C_1-C_8)$- alkyl, $(CH_2)_n$-NH-$SO_2$-$(CH_2)_n$-NH-$(C_3-C_8)$- cycloalkyle, $(CH_2)_n$-NH-$SO_2$-$(CH_2)_n$-N[$(C_1-C_8)$- alkyl$]_2$,
$(CH_2)_n$-NH-CN, $(CH_2)_n$-NH-$SO_2$-R16,
$(CH_2)_n$-NR12-CO-NH-$(C_1-C_8)$-alkyl, $(CH_2)_n$-NR12- CO-NH-$(C_3-C_8)$-cycloalkyl, $(CH_2)_n$-NR12-CO-NH2, $(CH_2)_n$-NR12-CO-NH-$SO_2$-$(C_1-C_8)$-alkyl, $(CH_2)_n$- NR12-CO-NH-$SO_2$-$(C_3-C_8)$-cycloalkyl, $(CH_2)_n$-NR12-CO-N[$(C_1-C_8)$-alkyl$]_2$, $(CH_2)$n-NH-CO-NH-$(CH_2)_n$- CO-[O-$(C_1-C_8)$-alkyl], $(CH_2)$n-NH-CO-NH- $CH_2)_q$- CO-$NH_2$, $(CH_2)$n-NH-CO-NH- $(CH_2)_q$-COOH, $(CH_2)_n$- NH-C(=NH)-$NH_2$, $(CH_2)_n$-NH-C(=NH)-R16, $(CH_2)_n$-NH-C(=NH)-NH[$(C_1-C_8)$-alkyl], $(CH_2)_n$-NH-C(=N-$SO_2$- $(C_1-C_8)$-alkyl)-$NH_2$, $(CH_2)_n$-NH-C(=N-$SO_2$-$(C_1-C_8)$-alkyl)-NH[$(C_1-C_8)$-alkyl], $(CH_2)_n$-NH-C(=N-$SO_2$- $NH_2$)-$NH_2$, $(CH_2)_n$-NH-C(=N-$SO_2$-$NH_2$)-NH[$(C_1-C_8)$- alkyl], $(CH_2)_n$-NH-C(=NH)-N[$(C_1-C_8)$-alkyl$]_2$, $(CH_2)_n$-NH-C(=N-$SO_2$-$(C_1-C_8)$-alkyl)-N[ $(C_1-C_8)$- alkyl$]_2$, $(CH_2)_n$-NH-$(CH_2)_n$-CO-O-$(CH_2)_r$-$NH_2$, $(CH_2)_n$-NH-$(CH_2)_n$-CO-NH-[$(C_1-C_8)$-alkyl], $(CH_2)_n$- NH-$(CH_2)_n$-CO-NH-$(CH_2)_r$-OH, $(CH_2)_n$-NH-$(CH_2)_n$- CO-N[$(C_1-C_8)$-alkyl$]_2$, $(CH_2)_n$-NH-$(CH_2)_n$-CO-NH- [$(C_3-C_8)$-cycloalkyl], $(CH_2)_n$-NH-C$(CH_3)_2$-CO-O($C_3$- $C_8)$-cycloalkyl, $(CH_2)_n$-NH-C$(CH_3)_2$-CO-O-$(CH_2)_r$- $NH_2$, $(CH_2)_n$-NH-C$(CH_3)_2$-CO-O-$(CH_2)_n$-aryl, $(CH_2)_n$- NH-C$(CH_3)_2$-CO-O-$(CH_2)_n$-heteroaryl, $(CH_2)_n$-NH-C$(CH_3)_2$-CO-NH-[$(C_1-C_8)$-alkyl], $(CH_2)_n$-NH- C$(CH_3)_2$-CO-NH-$(CH_2)_r$-OH, $(CH_2)_n$-NH-C$(CH_3)_2$-CO-N[$(C_1-C_8)$-alkyl$]_2$, $(CH_2)_n$-NH- $(CH_3)_2$-CO-NH-[($C_3$- $C_8)$-cycloalkyl, $(CH_2)_n$-NH-C$(CH_3)_2$-CO-N[$(C_3-C_8)$- cycloalkyl$]_2$,
$(CH_2)_n$-S$(O)_m$-$(C_1-C_8)$-alkyl, $(CH_2)_n$-S$(O)_m$-$(C_3-C_8)$- cycloalkyl, $(CH_2)_n$-$SO_2$-R16, $SO_2$-N=CH-N$(CH_3)_2$,

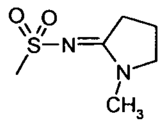

,

$(CH_2)_n$-$SO_2$-NH-CO-($C_1$-$C_8$)-alkyl, $(CH_2)_n$-$SO_2$-NH-CO-($C_3$-$C_8$)-cycloalkyl, $(CH_2)_n$-$SO_2$- NH- ($C_1$-$C_8$)-alkyl, $(CH_2)_n$-$SO_2$-NH-($C_3$-$C_8$)- cycloalkyl, $(CH_2)_n$-$SO_2$-N[($C_1$-$C_8$)-alkyl]$_2$, $SO_2$- NH-$(CH_2)_r$-OH, $SO_2$-NH-$(CH_2)_r$-$NH_2$, $SF_5$,
$(CH_2)_q$-CN,
$(CH_2)_n$-CO-NH-piperidin-1-yl,
$(CH_2)_n$-CO-NH-$SO_2$-NHR12, $(CH_2)_n$-CO-NH-$SO_2$-($C_1$- $C_8$)-alkyl, $(CH_2)_n$-CO-NH-$SO_2$-($C_3$-$C_8$)-cycloalkyl, $(CH_2)_n$-CHO, $(CH_2)_n$-C(=NH)$NH_2$,$(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[($C_1$-$C_6$)- alkyl], where the alkyl and cycloalkyl radicals may be substituted by fluorine atoms and where the aryl or heteroaryl radicals may be substituted by halogen, CN, ($C_1$-$C_6$)-alkyl, ($C_3$-$C_6$)-cycloalkyl, O-($C_1$-$C_6$)-alkyl, $S(O)_m$-($C_1$- $C_6$)-alkyl, $SO_2$-$NH_2$, COOH, $CONH_2$, CO-[O($C_1$-$C_6$)- alkyl], CO-($C_1$-$C_6$)-alkyl and where the alkyl radicals may be substituted by fluorine atoms;

where at least one of the R6, R7, R8, R9 and R10 radicals is always defined as T-bicyclic heterocycle, T-aryl or T-heteroaryl and
where one of the four radical pairs of R6 and R7, or R7 and R8, or R8 and R9, or R9 and R10 may in each case together form the -$CH_2$-$CH_2$-$CH_2$- or -$CH_2$-$CH_2$-$CH_2$-$CH_2$- groups in which up to two -$CH_2$- groups may be replaced by -O- and where the -$CH_2$-$CH_2$-$CH_2$- or -$CH_2$-$CH_2$-$CH_2$-$CH_2$- groups may be substituted by F, ($C_1$-$C_8$)-alkyl or =O;

T is NR23-CO-NR24, NR23-$SO_2$-NR24, $SO_2$-NR23-$SO_2$, CO-NR23-CO, NR23-C(=NR13)-NR24, NR23-C(=NR22)-NR24, CO-NR23-CR22R23, NR23-$SO_2$- CR22R24, CR22R24-$SO_2$-NR23, CR22R23-NR23-$SO_2$, $SO_2$-CR22R23-NR23, $SO_2$-NR23-CR22R23-, NR23- CR22R23-$SO_2$, CR23R24-CR23R24-CR23R24;
R11 is H, ($C_1$-$C_8$)-alkyl, ($C_3$-$C_6$)-cycloalkyl, $(CH_2)_n$- aryl, $(CH_2)_n$-CO-[O-($C_1$-$C_6$)-alkyl] , $(CH_2)_n$-CO- [O-($C_3$-$C_6$)-cycloalkyl] , $(CH_2)_n$-CO-[($C_1$-$C_6$)- alkyl], $(CH_2)_n$-CO-[($C_3$-$C_6$)-cycloalkyl], $(CH_2)_n$- CO-aryl, $(CH_2)_n$-CO-heteroaryl, $(CH_2)_q$-CO-$NH_2$, $(CH_2)_q$-COOH, $(CH_2)_n$-P(O)(OH) [O-($C_1$-$C_4$) -alkyl] , $(CH_2)_n$-P(O)[O-($C_1$-$C_4$)-alkyl]$_2$, $(CH_2)_n$-P(O)(O- $CH_2$-aryl)$_2$, $(CH_2)_n$-P(O)(OH)$_2$, $(CH_2)_n$-$SO_3H$, $(CH_2)_n$-$SO_2$-$NH_2$, $(CH_2)_n$-CO-NH-[($C_1$-$C_4$)-alkyl], $(CH_2)_n$-CO-N[($C_1$-$C_4$)-alkyl]$_2$, $(CH_2)_n$-CO-NH-[($C_3$- $C_6$)-cycloalkyl], ($C_2$-$C_6$)-alkenyl-CO-O[($C_1$-$C_4$)- alkyl], ($C_2$-$C_6$)-alkenyl-$CONH_2$, ($C_2$-$C_6$)-alkenyl- COOH, ($C_2$-$C_6$)-alkynyl-CO-O[($C_1$-$C_4$)-alkyl], ($C_2$- $C_6$)-alkynyl-$CONH_2$, ($C_2$-$C_6$) -alkynyl-COOH, $(CH_2)_n$-CR21[(CO-O($C_1$-$C_4$)-alkyl)]$_2$, $(CH_2)$n- CR21($CONH_2$)$_2$, $(CH_2)_n$-CR21(COOH)$_2$, $(CH_2)_n$- CR21R22-CO-O[($C_1$-$C_4$)-alkyl] , $(CH_2)_n$-CR21R22- $CONH_2$, $(CH_2)_n$-CR21R22-CO-NH-[($C_1$-$C_4$)-alkyl], $(CH_2)_n$-CR21R22-CO-N[($C_1$-$C_4$)-alkyl]$_2$, $(CH_2)_n$- CR21R22-COOH, $(CH_2)_n$-CO-R16, $(CH_2)_n$-CO-NH- C($CH_3$)$_2$CO-O[($C_1$-$C_8$)-alkyl], $(CH_2)_n$-CO-NH- C($CH_3$)$_2$-$CONH_2$, $(CH_2)_n$-CO-NH-C($CH_3$)$_2$-COOH, where the alkyl, alkeny, alkynyl and cycloalkyl radicals may be substituted by fluorine atoms and where the aryl or heteroaryl radical may be substituted by halogen, CN, ($C_1$-$C_4$)-alkyl, O- ($C_1$-$C_4$) -alkyl, $S(O)_m$-($C_1$-$C_4$)-alkyl, $SO_2$-$NH_2$, COOH, $CONH_2$, CO- O($C_1$-$C_4$)-alkyl and where the alkyl radicals may be substituted by fluorine atoms;
R12 is H, ($C_1$-$C_4$)-alkyl, ($C_3$-$C_6$) -cycloalkyl, $(CH_2)_n$-aryl, $(CH_2)_n$-heteroaryl, where the alkyl or cycloalkyl radicals may be substituted by fluorine atoms, and where the aryl or heteroaryl radical may be substituted by halogen, CN, ($C_1$-$C_4$)-alkyl, O-($C_1$-$C_4$)-alkyl, $SO_2$-$NH_2$, COOH, $CONH_2$, CO-O($C_1$-$C_4$)-alkyl, and where the alkyl radicals may be substituted by fluorine atoms;
R13 is H, $SO_2$-[($C_1$-$C_4$)-alkyl], $SO_2$-[($C_3$-$C_6$)- cycloalkyl], $SO_2$-$(CH_2)_n$-aryl, $SO_2$-$(CH_2)_n$-heteroaryl,
where the alkyl and cycloalkyl radicals may be substituted by fluorine atoms and where the aryl or heteroaryl radical may be substituted by halogen, CN, $CF_3$, ($C_1$-$C_4$)- alkyl, O-[($C_1$-$C_4$)-alkyl], $S(O)_m$-[($C_1$-$C_6$)- alkyl], $SO_2$-$NH_2$, COOH, $CONH_2$, CO-[O($C_1$-$C_4$)- alkyl] and where the alkyl radicals may be substituted by fluorine atoms;
R15 is ($C_1$-$C_6$)-alkyl,
where the alkyl radical may be substituted by fluorine atoms;
R16 is aziridin-1-yl, azetidin-1-yl, 3- hydroxyazetidin-1-yl, piperidin-1-yl, pyrrolidin-1-yl, 3-pyrrolidinol-1-yl, morpholin-N-yl, piperazin-1-yl, 4-[($C_1$-$C_6$)- alkyl]piperazin-1-yl, thiomorpholine-1,1- dioxid-4-yl, NH-$(CH_2)_r$-OH, NH-CH($CH_2$OH)$_2$, NH- C($CH_2$OH)$_3$, N[($C_1$-$C_4$)-alkyl-OH]$_2$, D-glucamin-N- yl, N-methyl-D-glucamin-N-yl, NH-[($C_1$-$C_4$)- alkyl]-COOH, NH-[($C_1$-$C_4$)-alkyl]-$CONH_2$, N [($C_1$- $C_4$)-alkyl][($C_1$-$C_4$)-alkyl]-COOH, NH- [C(H)(aryl)]-CO-O($C_1$-$C_4$)-alkyl, NH- [C(H)(aryl)]-COOH, NH-[C(H)(aryl)]-$CONH_2$, NH- [C(H)(heteroaryl)]-CO-O($C_1$-$C_4$)-alkyl, NH- [C(H)(heteroaryl)]-COOH, NH- [C (H) (heteroaryl)]-$CONH_2$, NH-[($C_3$-$C_6$)- cy-

cloalkyl]-CO-O($C_1$-$C_4$)-alkyl, NH-[($C_3$-$C_6$)- cycloalkyl]-COOH, NH-[($C_3$-$C_6$)-cycloalkyl]- $CONH_2$, NH-($C_1$-$C_4$)-alkyl-OH, NH-[($C_1$-$C_4$)- alkyl]-$SO_2$-($C_1$-$C_4$)-alkyl, NH-[($C_1$-$C_4$)-alkyl]- $SO_3H$, NH- [($C_1$-$C_4$)-alkyl]-$SO_2$-$NH_2$, where the alcohol (OH) functionalities may be replaced by F and where the aryl or heteroaryl radical may be substituted by halogen, CN, ($C_1$-$C_4$)-alkyl, O-($C_1$-$C_4$)-alkyl, OH, $SO_2$-$NH_2$, COOH, $CONH_2$, CO-O($C_1$-$C_4$)-alkyl;

R18 is $(CH_2)_n$-CR25R26-CO-O($C_1$-$C_4$)-alkyl, $(CH_2)_n$- CR25R26-CO-$NH_2$, $(CH_2)_n$-CR25R26-COOH;

R20 is H, ($C_1$-$C_4$)-alkyl, ($C_3$-$C_6$)-cycloalkyl, aryl, [($C_1$-$C_4$)-alkyl]-aryl;

R21 is H, F, $CF_3$, ($C_1$-$C_4$)-alkyl, ($C_3$-$C_6$)- cycloalkyl, OH, O-($C_1$-$C_4$)-alkyl, O-($C_3$-$C_6$)- cycloalkyl, O-$(CH_2)_n$-aryl, O-(CO)-($C_1$-$C_4$)- alkyl, O-(CO)-($C_3$-$C_6$)-cycloalkyl, O-(CO)-O- ($C_1$-$C_4$)-alkyl, O-(CO)-O-($C_3$-$C_6$)-cycloalkyl, NH-[($C_1$-$C_4$)-alkyl] -aryl, $NH_2$, NH- ($C_1$-$C_4$)-alkyl, NH-(CO)-($C_1$-$C_4$)-alkyl;

R22 is H, $CF_3$, ($C_1$-$C_4$)-alkyl, aryl, [($C_1$-$C_4$)-alkyl]-aryl;

R23, R24 are each independently H, ($C_1$-$C_4$)-alkyl, ($C_3$- $C_6$)-cycloalkyl, [($C_1$-$C_4$)-alkyl]-[($C_3$-$C_6$)- cycloalkyl], aryl, [($C_1$-$C_4$)-alkyl]-aryl or R23 and R24 together form a -CH=CH-, -$CH_2$- $CH_2$-, -$CH_2$-$CH_2$-$CH_2$- or -$CH_2$-$CH_2$-$CH_2$-$CH_2$- unit in which one $CH_2$ moiety may be replaced by C=O, CHF or $CF_2$, and in which up to four hydrogen atoms may be replaced by a ($C_1$-$C_4$)-alkyl radical;

R25, R26 are each independently H, F, ($C_1$-$C_4$)-alkyl, aryl, [($C_1$-$C_4$)-alkyl]-aryl, where the aryl may be substituted by halogen, CN, OH, O-($C_1$-$C_4$)-alkyl, or the R25 and R26 radicals, together with the carbon atom bonded to them, form a three- to seven-membered carbocycle in which one carbon atom may be replaced by O, $S(O)_m$, NH, N[($C_1$-$C_4$)-alkyl] or CO;

excluding the compound N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)urea, and the physiologically compatible salts thereof.

**5.** A compound of the formula Ia as claimed in claim 1

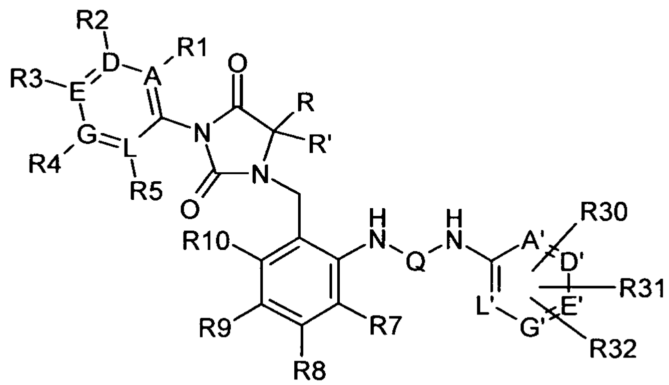

Ia

in which

R, R' are each independently H, aryl, ($C_1$-$C_4$)-alkyl, where ($C_1$-$C_4$)-alkyl or the aryl radical may be substituted by halogen;

or R and R' together form a ring having from three to eight carbon atoms, where one carbon atom may be replaced by O, $S(O)_m$, NR13 or NR15;

m is 0, 1, 2;

n is 0, 1, 2;

q is 1, 2;

r is 2, 3;

v is 0, 1, 2;

A, D, E, G, L are each independently C or N, where, when they are defined as N, the corresponding substituent R1, R2, R3, R4, R5 is absent,

or R2-D=E-R3 or R4-G=L-R5 is defined as S or O and where the five- or six-membered ring may be fused to -$(CH_2)_3$- or -$(CH_2)_4$- or -CH=CH- CH=CH- to form a bicyclic system;

Q is C=O, $SO_2$;

R1, R2, R3, R4, R5 are each independently H, F, Cl, Br, I, CN, $CF_3$, $(C_1-C_8)$-alkyl, $(C_3-C_8)$- cycloalkyl, $(CH_2)_n$-aryl, $(CH_2)_n$-heteroaryl, $OCF_3$, O-R11, NR13R15, $S(O)_m$-R12, $SO_2-NH_2$, $SO_2$- NH- $[(C_1-C_8)$-al kyl ] , $SO_2$-NH-$[(C_3-C_8)$- cycloalkyl], $SO_2NH-(CH_2)_n$-aryl, $SO_2-NH-(CH_2)_n$- heteroaryl, $SO_2-N[(C_1-C_8)$-alkyl$]_2$, $SO_2$-R16, $SF_5$, CO-O$[(C_1-C_8)$-alkyl],
CO-O$[(C_3-C_6)$-cycloalkyl], CO-$NH_2$, CO-NH-$[(C_1- C_4)$-alkyl], CO-N $[(C_1-C_4)$-alkyl$]_2$, C(=NH)-$NH_2$, C(=NH)-R16, $(CH_2)_n$-C(=$NSO_2$-R12)$NH_2$, CO-R16, COOH, CO-$(C_1-C_4)$-alkyl, CO-$(C_3-C_6)$-cycloalkyl, CO-aryl, CO-heteroaryl, CH(OH)-aryl, CH(OH)- heteroaryl, CHF-aryl, CHF-heteroaryl, $CF_2$- aryl, $CF_2$-heteroaryl, $CH_2$-OH, $CH_2$-CN, $CH_2$-O- R12, $CH_2-O-(CH_2)_q$-COOH,
where the alkyl and cycloalkyl radicals may be substituted by fluorine atoms, and where the aryl or heteroaryl radicals may be substituted by halogen, CN, $(C_1-C_4)$-alkyl, O- $(C_1-C_4)$-alkyl, $OCF_3$, OH, O-$(CH_2)_n$-aryl, $(CH_2)_n$-aryl, $S(O)_m-(C_1-C_4)$-alkyl, $SO_2-NH_2$, SH, NR12R13, NH-CO- $[(C_1-C_4)$-alkyl], NH-CO- $(CH_2)_n$- aryl, $(CH_2)_n$-COOH, $(CH_2)_n$-CON$H_2$, $(CH_2)_n$-CO-O$(C_1- C_4)$-alkyl, $(CH_2)_n$-CO-$(C_1-C_4)$-alkyl, and where the alkyl radicals may be substituted by fluorine atoms;
R7, R8, R9, R10 are each independently H, F, Cl, Br, I, CN, N3, NC, $NO_2$, $CF_3$,
$(C_1-C_8)$-alkyl, $(C_2-C_{10})$-alkenyl, $(C_2-C_{10})$- alkynyl, $(C_3-C_8)$-cycloalkyl, aryl, heteroaryl,
$(CH_2)_n$-CO-[O-$(C_1-C_8)$-alkyl], $(CH_2)_n$-CO-[O-$(C_3$- $C_8)$-cycloalkyl], $(CH_2)_n$-CO-$[(C_1-C_8)$-alkyl], $(CH_2)_n$-CO-$[(C_3-C_8)$-cycloalkyl],
$(CH_2)_n$-CO-[O-$(CH_2)_v$-aryl],
$(CH_2)_n$-CO-$NH_2$, $(CH_2)_n$-COOH, $(CH_2)_n$-CO-NH-CN,
$(CH_2)_n$-P(O)(OH)[O-$(C_1-C_6)$-alkyl] , $(CH_2)_n$- P(O)[O-$(C_1-C_6)$ alkyl$]_2$, $(CH_2)_n$-P(O)(OH) (O-$CH_2$- aryl) , $(CH_2)_n$-P(O)(O-$CH_2$-aryl$)_2$, $(CH_2)_n$- P(O)$(OH)_2$,
$(CH_2)_n$-$SO_3H$, $(CH_2)_n$-$SO_2$-$NH_2$,
$(CH_2)_n$-CO-NH-$[(C_1-C_8)$-alkyl], $(CH_2)_n$-CO-N$[(C_1$- $C_8)$-alkyl$]_2$, $(CH_2)_n$-CO-NH-$[(C_3-C_8)$-cycloalkyl], $(C_2-C_{10})$-alkenyl-CO-O$[(C_1-C_6)$-alkyl], $(C_2-C_{10})$- alkenyl-CON$H_2$, $(C_2-C_{10})$-alkenyl-COOH, $(C_2-C_{10})$- alkynyl-CO-O$[(C_1-C_6)$-alkyl] , $(C_2-C_{10})$-alkynyl- CON$H_2$, $(C_2-C_{10})$-alkynyl-COOH,
$(CH_2)_n$-CO-R16,
$(CH_2)_n$-OH, $(CH_2)_n$-O-$(C_1-C_8)$-alkyl, $(CH_2)_n$-O-$(C_2$- $C_{10})$-alkenyl, $(CH_2)_n$-O-$(C_2-C_{10})$-alkynyl, $(CH_2)_n$- O-$(C_3-C_8)$-cycloalkyl, $(CH_2)_n$-O-$(CH_2)_q$- $[(C_3-C_8)$- cycloalkyl], $(CH_2)_n$-O-$(CH_2)_n$-CO-[O-$(C_1-C_8)$- alkyl], $(CH_2)_n$-O-$(CH_2)_n$-CO-[O-$(C_3-C_8)$- cycloalkyl], $(CH_2)_n$-O-$(CH_2)_n$-CO-$[(C_1-C_8)$- alkyl], $(CH_2)_n$-O-$(CH_2)_n$-CO-$(C_3-C_8)$- cycloalkyl], $(CH_2)_n$-O-$(CH_2)_n$-CO-[O-$(CH_2)$v- aryl], $(CH_2)_n$-O-$(CH_2)_n$-CO-[O-$(CH_2)$v- heteroaryl], $(CH_2)_n$-O-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O- $(CH_2)_n$-P(O)(OH)[O-$(C_1-C_6)$-alkyl], $(CH_2)_n$-O- $(CH_2)_n$-P(O)[O-$(C_1-C_6)$-alkyl$]_2$, $(CH_2)$n-O-$(CH_2)_n$- P(O)(OH)(O-$CH_2$-aryl), $(CH_2)_n$-O-$(CH_2)_n$-P(O)(O- $CH_2$-aryl$)_2$, $(CH_2)_n$-O-$(CH_2)_n$-P(O)$(OH)_2$, $(CH_2)_n$-O- $(CH_2)_n$-$SO_3H$, $(CH_2)_n$-O-$(CH_2)_n$-$SO_2$-$NH_2$, $(CH_2)_n$-O- $(CH_2)_n$-CO-NH-$[C_1-C_8)$-alkyl], $(CH_2)_n$-O-$(CH_2)_n$- CO-N$[(C_1-C_8)$-alkyl$]_2$, $(CH_2)_n$-O-$(CH_2)_n$-CO-NH- $[(C_3-C_8)$-cycloalkyl], $(CH_2)_n$-O-$(CH_2)_n$-CR21R22- CO-O$[(C_1-C_6)$-alkyl], $(CH_2)_n$-O-$(CH_2)_n$-CR21R22- CON$H_2$, $(CH_2)_n$-O-$(CH_2)_n$-CR21R22-COOH, $(CH_2)_n$-O- $(CH_2)_n$-CO-R16, $(CH_2)_n$-O-$(CH_2)_r$-OH, $(CH_2)_n$-O- CH$(CH_2OH)_2$, $(CH_2)_n$-O-$(CH_2)_n$-CO-O-$(CH_2)_r$-$NH_2$, $(CH_2)_n$-O-$(CH_2)$n-CO-NH-$(CH_2)_r$-OH, O-R13, $OCF_3$, $(CH_2)_n$-$NH_2$, $(CH_2)_n$-NH-$(C_1-C_8)$-alkyl, $(CH_2)_n$-NH- $(C_3-C_8)$-cycloalkyl, $(CH_2)_n$-NH-$(CH_2)_n$-CO-[O-$(C_3$- $C_8)$-cycloalkyl], $(CH_2)_n$-NH-$(CH_2)_n$-CO-$[(C_1-C_8)$- alkyl], $(CH_2)_n$-NH- $(CH_2)_n$-CO-$[(C_3-C_8)$- cycloalkyl], $(CH_2)$n-NH-$(CH_2)_n$-CO-[O-$(CH_2)_v$- aryl], $(CH_2)_n$-NH-$(CH_2)_n$-CO-[O-$(CH_2)_v$- heteroaryl], $(CH_2)_n$-NH-$(CH_2)_q$-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O$[(C_1-C_6)$-alkyl,
$(CH_2)_n$-NH-$(CH_2)_n$-CR21R22-CON$H_2$, $(CH_2)_n$-NH- $(CH_2)_n$-CR21R22-COOH,
$(CH_2)_n$-NH-$(CH_2)_n$-CO-R16,
$(CH_2)_n$-NH-$(CH_2)_n$-$SO_2$-$[(C_1-C_8)$-alkyl], $(CH_2)_n$-NH- $(CH_2)_n$-$SO_2$-$[(C_3-C_8)$-cycloalkyl], $(CH_2)_n$-NH-$SO_2$-$(CH_2)_n$-$NH_2$, $(CH_2)_n$-NH-$SO_2$-$(CH_2)_n$-NH-$(C_1-C_8)$- alkyl, $(CH_2)_n$-NH-$SO_2$-$(CH_2)_n$-NH-$(C_3-C_8)$- cycloalkyl, $(CH_2)_n$-NH-$SO_2$-$(CH_2)_n$-N$[(C_1-C_8)$- alkyl$]_2$,
$(CH_2)_n$-NH-CN, $(CH_2)_n$-NH-$SO_2$-R16,
$(CH_2)_n$-NR12-CO-NH-$(C_1-C_8)$-alkyl, $(CH_2)_n$-NR12- CO-NH- $(C_3-C_8)$-cycloalkyl, $(CH_2)_n$-NR12-CO-$NH_2$, $(CH_2)_n$-NR12-CO-NH-$SO_2$-$(C_1-C_8)$-alkyl, $(CH_2)$n- NR12-CO-NH-$SO_2$-$(C_3-C_8)$-cycloalkyl, $(CH_2)_n$-NR12-CO-N$[(C_1-C_8)$-alkyl$]_2$, $(CH_2)$n-NH-CO-NH- $(CH_2)_n$- CO-[O-$(C_1-C_8)$-alkyl] , $(CH_2)$n-NH-CO-NH- $(CH_2)_q$- CO-$NH_2$, $(CH_2)$n-NH-CO-NH- $(CH_2)_q$-COOH, $(CH_2)_n$- NH-C(=NH)-$NH_2$, $(CH_2)$n-NH-C(=NH)-R16, $(CH_2)_n$-NH- C (=NH) -NH [ $(C_1-C_8)$-al kyl ] , $(CH_2)_n$-NH-C(=N-$SO_2$- $(C_1-C_8)$-alkyl) -$NH_2$, $(CH_2)_n$-NH-C(=N-$SO_2$-$(C_1-C_8)$-alkyl)-NH $[(C_1-C_8)$-alkyl], $(CH_2)_n$-NH-C(=N-$SO_2$- $NH_2$)-$NH_2$, $(CH_2)_n$-NH-C(=N-$SO_2$-$NH_2$)-NH [ $(C_1-C_8)$- alkyl] , $(CH_2)_n$-NH-C(=NH)-N[ $C_1-C_8)$-alkyl$]_2$, $(CH_2)_n$-NH-C(=N-$SO_2$-$(C_1-C_8)$-alkyl)-N$[(C_1-C_8)$- alkyl$]_2$, $(CH_2)_n$-NH-$(CH_2)_n$-CO-O-$(CH_2)_r$-$NH_2$, $(CH_2)_n$-NH-$(CH_2)_n$ -CO-NH-$[(C_1-C_8)$-alkyl], $(CH_2)_n$- NH-$(CH_2)_n$-CO-NH- $(CH_2)_r$-OH,

$(CH_2)_n$-NH-$(CH_2)_n$ -CO-N[$(C_1$-$C_8)$-alkyl]$_2$, $(CH_2)_n$-NH-$(CH_2)_n$ -CO-NH- [$(C_3$-$C_8)$-cycloalkyl], $(CH_2)_n$-NH-$C(CH_3)_2$-CO-O($C_3$- $C_8$)-cycloalkyl, $(CH_2)_n$-NH-$C(CH_3)_2$-CO-O-$(CH_2)_r$- $NH_2$, $(CH_2)_n$-NH-$C(CH_3)_2$-CO-O-$(CH_2)_n$-aryl, $(CH_2)_n$- NH-$C(CH_3)_2$-CO-O-$(CH_2)_n$-heteroaryl, $(CH_2)_n$-NH- $C(CH_3)_2$-CO-NH-[$(C_1$-$C_8)$-alkyl], $(CH_2)_n$-NH- $C(CH_3)_2$-CO-NH-$(CH_2)_r$-OH, $(CH_2)_n$-NH-$C(CH_3)_2$-CO- N[$(C_1$-$C_8)$-alkyl]$_2$, $(CH_2)_n$-NH-$(CH_3)_2$-CO-NH-[($C_3$- $C_8$)-cycloalkyl], $(CH_2)_n$-NH-$C(CH_3)_2$-CO-N [$(C_3$-$C_8)$- cycloalkyl]$_2$,

$(CH_2)_n$-$S(O)_m$-$(C_1$-$C_8)$-alkyl, $(CH_2)_n$-$S(O)_m$-($C_3$- $C_8$)-cycloalkyl, $(CH_2)_n$-$SO_2$-R16, $SO_2$-N=CH- $N(CH_3)_2$,

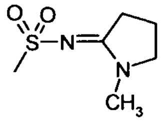,

$(CH_2)_n$-$SO_2$-NH-CO-$(C_1$-$C_8)$- alkyl, $(CH_2)$n-$SO_2$-NH-CO-$(C_3$-$C_8)$-cycloalkyl, $(CH_2)_n$-$SO_2$-NH-$(C_1$-$C_8)$-alkyl, $(CH_2)_n$-$SO_2$-NH-($C_3$- $C_8$)-cycloalkyl, $(CH_2)_n$-$SO_2$-N[$(C_1$-$C_8)$-alkyl]$_2$, $SO_2$-NH-$(CH_2)_r$-OH, $SO_2$-NH-$(CH_2)_r$-$NH_2$, $SF_5$,

$(CH_2)_q$-CN,

$(CH_2)_n$-CO-NH-piperidin-1-yl,

$(CH_2)_n$-CO-NH-$SO_2$-NHR12 , $(CH_2)_n$-CO-NH-$SO_2$-($C_1$- $C_8$)-alkyl, $(CH_2)_n$-CO-NH-$SO_2$-$(C_3$-$C_8)$-cycloalkyl, $(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[$(C_1$-$C_6)$- alkyl], where the alkyl and cycloalkyl radicals may be substituted by fluorine atoms, and where the aryl or heteroaryl radicals may be substituted by halogen, CN, $(C_1$-$C_6)$-alkyl, $(C_3$-$C_6)$ -cycloalkyl, O-$(C_1$-$C_6)$ - alkyl, $S(O)_m$-$(C_1$-$C_6)$-alkyl, $SO_2$-$NH_2$, COOH, $CONH_2$, CO-[O$(C_1$-$C_6)$-alkyl], CO-$(C_1$-$C_6)$-alkyl, and where the alkyl radicals may be substituted by fluorine atoms;

where one of the three radical pairs of R7 and R8, or R8 and R9, or R9 and R10 may in each case together form the -$CH_2$-$CH_2$-$CH_2$- or -$CH_2$-$CH_2$-$CH_2$-$CH_2$- groups in which up to two -$CH_2$ groups may be replaced by -O- and where the- $CH_2$-$CH_2$-$CH_2$- or -$CH_2$-$CH_2$-$CH_2$-$CH_2$- groups may be substituted by F, $(C_1$-$C_8)$-alkyl or =O;

R11 is H, $(C_1$-$C_8)$-alkyl, $(C_3$-$C_6)$ -cycloalkyl, $(CH_2)_n$-aryl, $(CH_2)_n$-CO-[O-$(C_1$-$C_6)$-alkyl] , $(CH_2)_n$-CO-[O-$(C_3$-$C_6)$-cycloalkyl], $(CH_2)_n$-CO- [$(C_1$-$C_6)$-alkyl], $(CH_2)_n$-CO-[$(C_3$-$C_6)$- cycloalkyl], $(CH_2)_n$-CO-aryl, $(CH_2)_n$-CO- heteroaryl, $(CH_2)_q$-CO-$NH_2$, $(CH_2)_q$-COOH, $(CH_2)_n$- P(O)(OH)[O-$(C_1$-$C_4)$-alkyl] , $(CH_2)_n$-P(O)[O-($C_1$- $C_4$)-alkyl]$_2$, $(CH_2)_n$- P(O) (O-$CH_2$aryl)$_2$, $(CH_2)_n$- $P(O)(OH)_2$, $(CH_2)_n$-$SO_3H$, $(CH_2)_n$-$SO_2$-$NH_2$, $(CH_2)_n$- CO-NH-[$(C_1$-$C_4)$-alkyl], $(CH_2)_n$-CO-N[$(C_1$-$C_4)$- alkyl]$_2$, $(CH_2)_n$-CO-NH-[$(C_3$-$C_6)$-cycloalkyl], $(C_2$-$C_6)$-alkenyl-CO-O[$(C_1$-$C_4)$-alkyl], $(C_2$-$C_6)$ - alkenyl-$CONH_2$, $(C_2$-$C_6)$-alkenyl-COOH, $(C_2$-$C_6)$- alkynyl-CO-O[$(C_1$-$C_4)$-alkyl], $(C_2$-$C_6)$ -alkynyl- $CONH_2$, $(C_2$-$C_6)$-alkynyl-COOH, $(CH_2)_n$-CR21[(CO- O$(C_1$-$C_4)$-alkyl)]$_2$, $(CH_2)_n$-CR21$(CONH_2)_2$, $(CH_2)_n$- CR21$(COOH)_2$, $(CH_2)_n$-CR21R22-CO-O [$(C_1$-$C_4)$ - alkyl], $(CH_2)_n$-CR21R22-$CONH_2$, $(CH_2)_n$-CR21R22- CO-NH-[$(C_1$-$C_4)$-alkyl] , $(CH_2)_n$-CR21R22-CO-N [($C_1$- $C_4$)-alkyl]$_2$, $(CH_2)_n$-CR21R22-COOH, $(CH_2)_n$-CO- R16, $(CH_2)_n$-CO-NH-$C(CH_3)_2$-CO-O[$(C_1$-$C_8)$-alkyl], $(CH_2)_n$-CO-NH-$C(CH_3)_2$-$CONH_2$, $(CH_2)_n$-CO-NH- $C(CH_3)_2$-COOH, where the alkyl, alkenyl, alkynyl and cycloalkyl radicals may be substituted by fluorine atoms, and where the aryl or heteroaryl radical may be substituted by halogen, CN, $(C_1$-$C_4)$-alkyl, O-$(C_1$-$C_4)$-alkyl, $S(O)_m$-$(C_1$-$C_4)$-alkyl, $SO_2$-$NH_2$, COOH, $CONH_2$, CO- O $(C_1$-$C_4)$-alkyl, and where the alkyl radicals may be substituted by fluorine atoms;

R12 is H, $(C_1$-$C_4)$-alkyl, $(C_3$-$C_6)$ -cycloalkyl, $(CH_2)_n$-aryl, $(CH_2)_n$-heteroaryl, where the alkyl or cycloalkyl radicals may be substituted by fluorine atoms,

and where the aryl or heteroaryl radical may be substituted by halogen, CN, $(C_1$-$C_4)$-alkyl, O-$(C_1$-$C_4)$-alkyl, $SO_2$-$NH_2$, COOH, $CONH_2$, CO-O($C_1$- $C_4$)-alkyl, and where the alkyl radicals may be substituted by fluorine atoms;

R13 is H, $SO_2$-[$(C_1$-$C_4)$-alkyl], $SO_2$-[$(C_3$-$C_6)$- cycloalkyl], $SO_2$-$(CH_2)_n$-aryl, $SO_2$-$(CH_2)_n$-heteroaryl,

where the alkyl and cycloalkyl radicals may be substituted by fluorine atoms, and where the aryl or heteroaryl radical may be substituted by halogen, CN, $CF_3$, $(C_1$-$C_4)$ - alkyl, O-[$(C_1$-$C_4)$-alkyl], $S(O)_m$-[$(C_1$-$C_6)$ - alkyl], $SO_2$-$NH_2$, COOH, $CONH_2$, CO-[O$(C_1$-$C_4)$ - alkyl], and where the alkyl radicals may be substituted by fluorine atoms;

R15 is $(C_1$-$C_6)$-alkyl,

where the alkyl radical may be substituted by fluorine atoms;

R16 is aziridin-1-yl, azetidin-1-yl, 3- hydroxyazetidin-1-yl, piperidin-1-yl, pyrrolidin-1-yl, 3-pyrrolidinol-1-yl, morpholin-N-yl, piperazin-1-yl, 4-[$(C_1$-$C_6)$- alkyl]piperazin-1-yl, thiomorpholine-1,1- dioxid-4-yl, NH-$(CH_2)_r$-OH,

NH-$CH(CH_2OH)_2$, NH- $C(CH_2OH)_3$, N[($C_1$-$C_4$)-alkyl-OH]$_2$, D-glucamin-N- yl, N-methyl-D-glucamin-N-yl, NH-[($C_1$-$C_4$)- alkyl]-COOH, NH-[($C_1$-$C_4$)-alkyl]-$CONH_2$, N[($C_1$- $C_4$)-alkyl][($C_1$-$C_4$)-alkyl]-COOH, NH- [C(H)(aryl)]-CO-O($C_1$-$C_4$)-alkyl, NH- [C(H)(aryl)]-COOH, NH-[C(H)(aryl)]-$CONH_2$, NH- [C(H)(heteroaryl)]-CO-O($C_1$-$C_4$)-alkyl, NH- [C(H)(heteroaryl)]-COOH, NH- [C(H)(heteroaryl)]-$CONH_2$, NH-[($C_3$-$C_6$)- cycloalkyl]-CO-O($C_1$-$C_4$)-alkyl, NH- [($C_3$-$C_6$)- cycloalkyl]-COOH, NH-[($C_3$-$C_6$)-cycloalkyl]- $CONH_2$, NH-($C_1$-$C_4$)-alkyl-OH, NH-[($C_1$-$C_4$)-alkyl]- $SO_2$-($C_1$- $C_4$)-alkyl, NH- [($C_1$-$C_4$)-alkyl] -S03H, NH- [($C_1$-$C_4$) -alkyl] -$SO_2$-$NH_2$,
where the alcohol (OH) functions may be replaced by F and where the aryl or heteroaryl radical may be substituted by halogen, CN, ($C_1$-$C_4$)-alkyl, O-($C_1$-$C_4$)-alkyl, OH, $SO_2$-$NH_2$, COOH, $CONH_2$, CO-O($C_1$-$C_4$)-alkyl;
R18 is $(CH_2)_n$-CR25R26-CO-O($C_1$-$C_4$) -alkyl, $(CH_2)_n$- CR25R26-CO-$NH_2$, $(CH_2)_n$-CR25R26-COOH;
R21 is H, F, $CF_3$, ($C_1$-$C_4$)-alkyl, ($C_3$-$C_6$)- cycloalkyl, OH, O-($C_1$-$C_9$)-alkyl, O-($C_3$-$C_6$)- cycloalkyl, O-$(CH_2)_n$-aryl, O-(CO)-($C_1$-$C_4$)- alkyl, O-(CO)-($C_3$-$C_6$)-cycloalkyl, O-(CO)-O- ($C_1$-$C_4$)-alkyl, O-(CO)-O($C_3$-$C_6$)-cycloalkyl, NH-[($C_1$-$C_4$)-alkyl]-aryl, $NH_2$, NH-($C_1$-$C_4$)-alkyl, NH-(CO)-($C_1$-$C_4$)-alkyl;
R22 is H, $CF_3$, ($C_1$-$C_4$) -alkyl, aryl, [($C_1$-$C_4$)-alkyl]-aryl;
R25, R26 are each independently H, F, ($C_1$-$C_4$)-alkyl, aryl, [($C_1$-$C_4$)-alkyl]-aryl,
where the aryl may be substituted by halogen, CN, OH, O-($C_1$-$C_4$)-alkyl,
or the R25 and R26 radicals, together with the carbon atom bonded to them, form a three- to seven-membered carbocycle in which one carbon atom may be replaced by 0, $S(O)_m$, NH, N[($C_1$-$C_4$)-alkyl] or CO;
A', D', E', G', L' are each independently CH or N, where, when they are defined as N, the corresponding substituent R30, R31 or R32 is absent if it would be bonded to the nitrogen atom;
R30, R31, R32 are each independently R11, F, Cl, Br, I, CN, $CF_3$, $(CH_2)_n$-O-R11, O-R13, $OCF_3$, $(CH_2)_n$- NH-R11, $(CH_2)_n$-N[$(CH_2)_q$-CO-O($C_1$-$C_4$)-alkyl]$_2$, $(CH_2)_n$-N[$(CH_2)_q$-COOH]$_2$, $(CH_2)_n$-N[$(CH_2)_q$-$CONH_2$]$_2$, $(CH_2)_n$-NH-R13, $(CH_2)_n$-N(R13)$_2$, $(CH_2)_n$-NH-$SO_2$- R16, ($CH_2$)n-NH-$(CH_2)_n$-$SO_2$-R12, $(CH_2)_n$-NR12-CO- R16, $(CH_2)_n$-NR12-CO-NR12R13, $(CH_2)_n$-NR12-CO- N(R12)$_2$, $(CH_2)_n$-NR12-CO-NHR11, $(CH_2)_n$-NH- C(=NH)-$NH_2$, $(CH_2)_n$-NH-C(=NH)-R16, $(CH_2)_n$-NH- C(=NH)-NHR12 , $(CH_2)_n$-NH-$(CH_2)_n$-CO-NH-[($C_1$-$C_4$)- alkyl], $(CH_2)_n$-NH-$(CH_2)_n$-CO-N[($C_1$-$C_4$)-alkyl]$_2$, $(CH_2)_n$-NH-$C(CH_3)_2$-CO-O($C_1$-$C_4$)-alkyl, $(CH_2)_n$-NH- $C(CH_3)_2$-CO-O($C_3$-$C_6$)-cycloalkyl, $(CH_2)_n$-NH- C $(CH_3)_2$-CO-O-$(CH_2)_r$-$NH_2$, $(CH_2)_n$-NH-$C(CH_3)_2$-CO-O- $(CH_2)_n$-aryl, $(CH_2)_n$-NH-$C(CH_3)_2$-CO-O-$(CH_2)_n$- heteroaryl, $(CH_2)_n$-NH-$C(CH_3)_2$-CO-$NH_2$, $(CH_2)_n$-NH- $C(CH_3)_2$-CO-NH-[($C_1$-$C_4$)-alkyl], $(CH_2)_n$-NH-$C(CH_3)_2$-CO-N[($C_1$-$C_4$)-alkyl]$_2$, $(CH_2)_n$-NH-$C(CH_3)_2$- COOH, $S(O)_m$-R12, $SO_2$-R16, $SO_2$-N=CH-N$(CH_3)_2$, $SO_2$-NH-CO-R12 , $SO_2$-NHR12, $SO_2$-N[($C_1$-$C_4$)- alkyl]$_2$, $SF_5$, COOH, CO-$NH_2$, $(CH_2)_q$-CN, $(CH_2)_n$- CO-NH-piperidin-1-yl, $(CH_2)_n$-CO-NH-$SO_2$-NHR12, $(CH_2)_n$-CO-NH-$SO_2$-R18 , $(CH_2)_n$-C(=NH)-NHOH, $(CH_2)_n$-C(=NR13)NHR12, $(CH_2)_n$-C(=NR12)NR12R13, $(CH_2)_n$-C(=N$SO_2$-R12)$NH_2$, $(CH_2)_n$-P(O)(OH)[O-($C_1$- $C_4$)-alkyl], $(CH_2)_n$-P(O)[O-($C_1$-$C_4$)-alkyl]$_2$, $(CH_2)_n$-P(O)(O-$CH_2$aryl)$_2$, $(CH_2)_n$-P(O)(OH)$_2$, where the alkyl and cycloalkyl radicals may be substituted by fluorine atoms, and where the aryl or heteroaryl radicals may be substituted by halogen, CN, ($C_1$-$C_4$)-alkyl, O- ($C_1$-$C_4$) -alkyl, $S(O)_m$-($C_1$-$C_4$)-alkyl, $SO_2$-$NH_2$, COOH, $CONH_2$, CO-O($C_1$-$C_4$)-alkyl, and where the alkyl radicals may be substituted by fluorine atoms;

excluding the compound N-[3-t-butyl-1-(4-methylphenyl)-1H-pyrazol-5-yl]-N'-(4-{[3-(2-methylphenyl)-2,4-dioxo-1-imidazolidinyl]methyl]}phenyl)urea,
and the physiologically compatible salts thereof.

**6.** A compound of the formula Ia as claimed in claim 5, wherein

R, R' are each ($C_1$-$C_4$) -alkyl;
or R and R' together form a ring having from three to eight carbon atoms;
m is 0, 1, 2;
n is 0, 1, 2;
A, D, E, G, L are each C;
Q is C=O, $SO_2$;
R1, R2, R3, R4, R5 are each independently H, F, Cl, Br, CN, $CF_3$, ($C_1$-$C_8$)-cycloalkyl, O- phenyl;
R7, R8, R9, R10 are each independently H, F, Cl, Br, $CF_3$, -$OCH_3$;
A', E' are each independently CH or N, where, when they are defined as N, the corresponding substituent R30, R31 or R32 is absent if it would be bonded to the nitrogen atom,
R30, R31, R32 are each independently H, F, Cl, Br, $CF_3$, OH, $NO_2$, $NH_2$, $CONH_2$, COOH, -COO-($C_1$-$C_8$)- alkyl, $(CH_2)_n$-P(O)(OH)[O-($C_1$-$C_4$)-alkyl], $(CH_2)_n$-P(O)[O-($C_1$-$C_4$)-alkyl]$_2$, $(CH_2)_n$-P(O)(OH)$_2$, $S(O)_m$-($C_1$-$C_4$)-alkyl, $S(O)_m$-($CH_2$)-COOH, $S(O)_m$- ($CH_2$)-COO-($C_1$-$C_4$)-alkyl, $SO_2$-Cl, $SO_2$-$NH_2$, $SO_3H$;

and the physiologically compatible salts thereof.

**7.** A compound of the formula Ia as claimed in claim 6, wherein

R, R' are each ($C_1$-$C_4$)-alkyl;
or R and R' together form a ring having from three to eight carbon atoms;
m is 0, 1, 2;
n is 0, 1, 2;
A, D, E, G, L are each C;
Q is C=O, $SO_2$;
R1, R2, R3, R4, R5 are each independently H, F, Cl, Br, CN, $CF_3$, ($C_1$-$C_8$)-cycloalkyl, O- phenyl;
R7, R8, R9, R10 are each independently H, F, Cl, Br, $CF_3$, $-OCH_3$;
A', E' are each independently CH or N, where, when they are defined as N, the corresponding substituent R30, R31 or R32 is absent if it would be bonded to the nitrogen atom,
R30, R31, R32 are each independently H, F, Cl, Br, $CF_3$, OH, $NO_2$, $NH_2$, $CONH_2$, COOH, -COO- ($C_1$- $C_8$)-alkyl, $(CH_2)_n$-P(O) (OH) [O- ($C_1$-$C_4$)- alkyl] , $(CH_2)_n$-P(O)[O-($C_1$-$C_4$)-alkyl]$_2$, $(CH_2)_n$-P(O) $(OH)_2$, S $(O)_m$- ($C_1$-$C_9$)-alkyl, $S(O)_m$-($CH_2$)-COOH, $S(O)_m$-($CH_2$)-COO-($C_1$-$C_4$)- alkyl, $SO_2$-Cl, $SO_2$-$NH_2$, $SO_3H$;

and the physiologically compatible salts thereof.

**8.** A compound as claimed in one or more of claims 1 to 7 for use as a medicament.

**9.** A medicament comprising one or more of the compounds as claimed in one or more of claims 1 to 7.

**10.** A medicament comprising one or more of the compounds as claimed in one or more of claims 1 to 7 and at least one further active ingredient.

**11.** The medicament as claimed in claim 8, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, MTP inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, $\alpha$-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, modulators of GPR40, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY antagonists, MC4 agonists, orexin antagonists, H3 antagonists, TNF agonists, CRF antagonists, CRF BP antagonists, urocortin agonists, $\beta$3 agonists, CB1 receptor antagonists, MSH (melanocyte-stimulating hormone) agonists, MCH antagonists, CCK agonists, serotonin reuptake inhibitors, mixed serotoninergic and noradrenergic compounds, 5HT modulators, bombesin agonists, galanin antagonists, growth hormones, growth hormone-releasing compounds, TRH agonists, decoupling protein 2 or 3 modulators, leptin agonists, DA agonists (bromocriptin, doprexin), lipase/amylase inhibitors, PPAR modulators, RXR modulators or TR-$\beta$-agonists or amphetamines.

**12.** The use of the compounds as claimed in one or more of claims 1 to 7 for producing a medicament for the treatment of metabolic syndrome.

**13.** The use of the compounds as claimed in one or more of claims 1 to 7 for producing a medicament for the treatment of diabetes.

**14.** The use of the compounds as claimed in one or more of claims 1 to 7 for producing a medicament for the treatment of obesity.

**15.** The use of the compounds as claimed in one or more of claims 1 to 7 for producing a medicament for weight reduction.

**16.** The use of the compounds as claimed in one or more of claims 1 to 7 for producing a medicament for the treatment of nicotine dependence.

**17.** The use of the compounds as claimed in one or more of claims 1 to 7 for producing a medicament for the treatment of alcohol dependence.

**18.** The use of the compounds as claimed in one or more of claims 1 to 7 for producing a medicament for the treatment of CNS disorders.

**19.** The use of the compounds as claimed in one or more of claims 1 to 7 for producing a medicament for the treatment of schizophrenia.

**20.** The use of the compounds as claimed in one or more of claims 1 to 7 for producing a medicament for the treatment of Alzheimer's.

**21.** A process for producing a medicament comprising one or more of the compounds as claimed in one or more of claims 1 to 7, which comprises mixing the active ingredient with a pharmaceutically suitable carrier and bringing this mixture into a form suitable for administration.

## Revendications

**1.** Composés de formule I

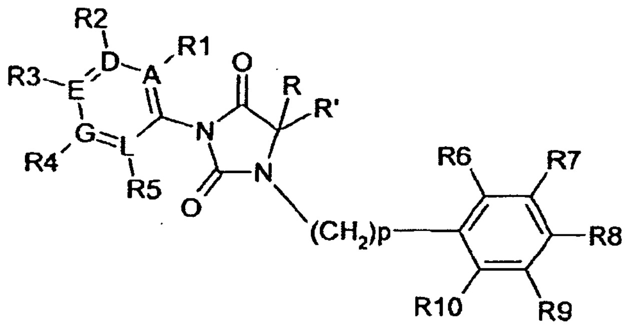

I

dans laquelle

R, R' représentent chacun indépendamment de l'autre H, un radical $(CH_2)_n$-aryle, alkyle en $C_1$-$C_6$, le radical alkyle en $C_1$-$C_6$ ou le radical aryle pouvant être substitué par un ou des substituants halogéno, O-R14, $S(O)_m$-R12 ou NR13R15 ;
ou R et R' forment ensemble un noyau ayant trois à huit atomes de carbone, un atome de carbone pouvant être remplacé par O, $S(O)_m$, NR13 ou NR15 ;
m vaut 0, 1, 2 ;
n vaut 0, 1, 2, 3, 4 ;
p vaut 1, 2, 3, 4, 5 ;
q vaut 1, 2, 3, 4 ;
r vaut 2, 3, 4, 5, 6 ;
v vaut 0, 1, 2, 3, 4 ;
A, D, E, G, L représentent chacun indépendamment des autres C ou N, où, pour la signification N, le substituant correspondant R1, R2, R3, R4, R5 disparaît, ou R2-D=E-R3 ou R4-G=L-R5 représentent S ou O, ou encore le noyau à cinq ou six chaînons peut être condensé à un radical $-(CH_2)_3-$ ou $-(CH_2)_4-$ ou -CH=CH-CH=CH- pour donner un radical bicyclique ;
R1, R2, R3, R4, R5 représentent chacun indépendamment des autres H, F, Cl, Br, I, CN, $N_3$, NC, $NO_2$, $CF_3$, alkyle en $C_1$-$C_8$, cycloalkyle en $C_3$-$C_8$, $(CH_2)_q$-[cycloalkyle en $C_3$-$C_8$], $(CH_2)_n$, - [cycloalcényle en $C_3$-$C_8$] ,

$(CH_2)_n$-[bicycloalkyle en $C_7$-$C_{12}$], $(CH_2)_n$-[tricycloalkyle en $C_7$-$C_{12}$], adamantan-1-yle, adamantan-2-yle, $(CH_2)_n$-aryle, $(CH_2)_n$-hétéroaryle, $OCF_3$, O-R11, NR13R15, NH-CN, $S(O)_m$-R12, $SO_2NH_2$, $SO_2$-N=CH-N$(CH_3)_2$, $SO_2$-NH-[alkyle en $C_1$-$C_8$] , $SO_2$-NH-[cycloalkyle en $C_3$-$C_8$] , $SO_2$-NH- $(CH_2)_n$-aryle, $SO_2$-NH-$(CH_2)_n$-hétéroaryle, $SO_2$-N[alkyle en $C_1$-$C_8$]$_2$, $SO_2$-R16, $SF_5$, CO-O [alkyle en $C_1$-$C_8$], CO-O[cycloalkyle en $C_3$-$C_8$], CO-O-$(CH_2)_n$-aryle, CO-O-$(CH_2)_n$-hétéroaryle, CO-$NH_2$, CO-NH-CN, CO-NH-[alkyle en $C_1$-$C_8$] , CO-N[alkyle en $C_1$-$C_8$]$_2$, CO-NH- [cycloalkyle en $C_3$-$C_8$], CO-N [cycloalkyle en $C_3$-$C_8$]$_2$, C(=NH)-O-[alkyle en $C_1$-$C_6$], C (=NH)-$NH_2$, C(=NH)-R16, C(=NR13)-NR12R13, $(CH_2)_n$-C(=$NSO_2$-R12)$NH_2$, CO-R16, COOH, CO-(alkyle en $C_1$-$C_8$), CO-(cycloalkyle en $C_3$-$C_8$) , CO-aryle, CO-hétéroaryle, CH(OH)-aryle, CH(OH)-hétéroaryle, CH[O-(alkyle en $C_1$-$C_6$)]-aryle, CH[O-(alkyle en $C_1$-$C_6$)]-hétéroaryle, CHF-aryle, CHF-hétéroaryle, $CF_2$-aryle, $CF_2$-hétéroaryle, CHO, $CH_2$-OH, $CH_2$-CN, $CH_2$-O-R12, $CH_2$-O-$(CH_2)_n$-CO-O[alkyle en $C_1$-$C_8$], $CH_2$-O-$(CH_2)_n$-CO-$NH_2$, $CH_2$-O-$(CH_2)_q$-COOH,
les radicaux alkyle, cycloalkyle, cycloalcényle, bicycloalkyle et tricycloalkyle pouvant être substitués par des atomes de fluor, les radicaux aryle ou hétéroaryle pouvant être substitués par un ou des substituants halogéno, CN, alkyle en $C_1$-$C_6$, cycloalkyle en $C_3$-$C_6$, O-(alkyle en $C_1$-$C_6$) , $OCF_3$, OH, O-$(CH_2)_n$-aryle, $(CH_2)_n$-aryle, $S(O)_m$- (alkyle en $C_1$-$C_6$), $SO_2$-$NH_2$, SH, NR12R13, NH-CO-[alkyle en $C_1$-$C_6$] , NH-CO-$(CH_2)_n$-aryle, $(CH_2)_n$-COOH, $(CH_2)_n$-$CONH_2$, $(CH_2)_n$-CO-O-(alkyle en $C_1$-$C_6$) , $(CH_2)_n$-CO-(alkyle en $C_1$-$C_6$) , et les radicaux alkyle pouvant être substitués par des atomes de fluor ;
R6, R7, R8, R9, R10 représentent chacun indépendamment des autres un radical hétérocyclique T-bicyclique, T-aryle ou T-hétéroaryle, le radical hétérocyclique bicyclique ou le radical aryle ou hétéroaryle pouvant être condensé à un noyau carboné aromatique ou non aromatique à 5 ou 6 chaînons, où un ou plusieurs groupes CH ou $CH_2$ peuvent être remplacés par des atomes d'oxygène, et le noyau carboné aromatique ou non aromatique à 5 ou 6 chaînons peut être substitué par un ou des substituants F, =O ou alkyle en $C_1$-$C_6$, le radical hétérocyclique bicyclique pouvant contenir 9 à 12 chaînons nucléaires, et jusqu'à cinq groupes CH ou $CH_2$ pouvant, indépendamment les uns des autres, être remplacés par N, NR20, O, $S(O)_m$ ou C=O, et le radical aryle ou hétéroaryle ou le radical hétérocyclique bicyclique pouvant être non substitué ou une ou plusieurs fois substitué par les substituants suivants :

R11, F, Cl, Br, I, CN, $N_3$, NC, $NO_2$, $CF_3$, $(CH_2)_n$-OR11, $(CH_2)_n$-O-$(CH_2)$-OH , $(CH_2)_n$-O-CH $(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O(alkyle en $C_1$-$C_6$)]$_2$, $(CH_2)_n$-N[$(CH_2)_q$-COOH]$_2$, $(CH_2)_n$-N[$(CH_2)_q$-$CONH_2$]$_2$, $(CH_2)_n$-NR-R13, $(CH_2)_n$-N$(R13)_2$, $(CH_2)_n$-NH-CN, $(CH_2)_n$-NH-$SO_2$-R16, $(CH_2)_n$-NH-(CH2)$_n$-$SO_2$-R12, $(CH_2)_n$-NR12-COR16, $(CH_2)_n$-NR12-CO-NR12R13, $(CH_2)_n$-NR12-CO-N$(R12)_2$, $(CH_2)_n$-NR12-CO-NHR11, $(CH_2)_n$-NH-C (=NH) -$NH_2$, $(CH_2)_n$-NH-C-(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)$-$NH_2$, $(CH_2)_n$-NH-$(CH_2)_n$-CO-NH-[alkyle en $C_1$-$C_8$] , $(CH_2)_n$-NH-$(CH_2)_n$-CO-NH-$(CH_2)_r$-OH, $(CH_2)_n$-NH-$(CH_2)_n$-CO-N [alkyle en $C_1$-$C_8$]$_2$, $(CH_2)_n$-NH-$(CH_2)_n$-CO-NH-[cycloalkyle en $C_3$-$C_8$], $(CH_2)_n$-NH-$(CH_2)_n$-CO-N[cycloalkyle en $C_3$-$C_8$]$_2$, $(CH_2)_n$-NH-C$(CH_3)_2$-CO-O(alkyle en $C_1$-$C_8$) , $(CH_2)_n$-NH-C$(CH_3)_2$-CO-O(cycloalkyle en $C_3$-$C_8$) , $(CH_2)_n$-NH-C$(CH_3)$ $_2$-CO-O-$(CH_2)_r$-$NH_2$, $(CH_2)_n$-NH-C$(CH_3)_2$-CO-O-$(CH_2)_n$-aryle, $(CH_2)_n$-NH-C$(CH_3)_2$-CO-O-$(CH_2)_n$-hétéroaryle, $(CH_2)_n$-NH-C$(CH_3)_2$-CO-$NH_2$, $(CH_2)_n$-NH-C$(CH_3)_2$-CO-NH-[alkyle en $C_1$-$C_8$], $(CH_2)_n$-NH-C$(CH_3)_2$-CO-NH-$(CH_2)_r$-OH, $(CH_2)_n$-NH-C$(CH_3)_2$-CO-N [alkyle en $C_1$-$C_8$]$_2$, $(CH_2)_n$-NH-$(CH_3)_2$-CO-NH-[cycloalkyle en $C_3$-$C_8$], $(CH_2)_n$-NH-C $(CH_3)_2$-CON[cycloalkyle en $C_3$-$C_8$]$_2$, $(CH_2)_n$-NH-C$(CH_3)_2$-COOH, $S(O)_m$-R12, $SO_2$-R16, $SO_2$-N=CH-N$(CH_3)_2$,

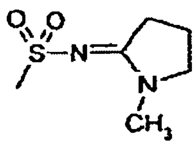

$SO_2$-NH-CO-R12, $SO_2$-NHR12, $SO_2$-NH-$(CH_2)_r$-OH, $SO_2$-N[alkyle en $C_1$-$C_8$]$_2$, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH-pipéridin-1-yle, $(CH_2)_n$-CO-NH-$SO_2$-NHR12, $(CH_2)_n$-CO-NH-$SO_2$-R18, $(CH_2)_n$-CHO, $(CH_2)_n$-C(=NH)$NH_2$, $(CH_2)_n$-C(=NH)-NHOH, $(CH_2)_n$-C(=NH) - [NH-O- (alkyle en $C_1$-$C_6$)] , $(CH_2)_n$-C(=NH)(R16) , $(CH_2)_n$-C (=NR13)NHR12, $(CH_2)_n$-C(=NR12)NR12R13, $(CH_2)_n$-C(=$NSO_2$-R12) $NH_2$, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O-(alkyle

en $C_1$-$C_6$), $S(O)_m$-(alkyle en $C_1$-$C_6$), $SO_2$-$NH_2$, COOH, $CONH_2$, CO-O(alkyle en $C_1$-$C_6$) , CO-(alkyle en $C_1$-$C_6$), et les radicaux alkyle pouvant être substitués par des atomes de fluor, et, quand R3 est CN, $NO_2$ ou un groupe halogéno et R4 est $CF_3$ ou un groupe halogéno et R est R' qui est un groupe méthyle, alors le radical X-aryle peut être pourvu d'au moins l'un des substituants mentionnés ci-dessus, différents de l'azote ;

H, F, Cl, Br, I, CN, $N_3$, NC, $NO_2$, $CF_3$,
alkyle en $C_1$-$C_8$, alcényle en $C_2$-$C_{10}$, alcynyle en $C_2$-$C_{10}$, cycloalkyle en $C_3$-$C_8$),
aryle, hétéroaryle,
$(CH_2)_n$-CO-[O-(alkyle en $C_1$-$C_8$)] , $(CH_2)_n$,-CO-[O-(cycloalkyle en $C_3$-$C_8$)], $(CH_2)_n$-CO-[alkyle en $C_1$-$C_8$] , $(CH_2)_n$-CO-[cycloalcyleen$C_3$-$C_8$],
$(CH_2)_n$-CO-[O-$(CH_2)_v$-aryle],
$(CH_2)_n$-CO-$NH_2$,$(CH_2)_n$-COOH, $(CH_2)_n$-CO-NH-CN,
$(CH_2)_n$-P(O)(OH)[O-(alkyleen$C_1$-$C_6$)], $(CH_2)_n$-P(O)[O-(alkyle en $C_1$-$C_6$)]$_2$, $(CH_2)_n$-P(O)(OH)(O-$CH_2$-aryle) , $(CH_2)_n$-P(O)(O-$CH_2$-aryle)$_2$, $(CH_2)_n$-P(O)$(OH)_2$, $(CH_2)_n$-$SO_3H$, $(CH_2)_n$-$SO_2$-$NH_2$,
$(CH_2)_n$-CO-NH-[alkyle en $C_1$-$C_8$] , $(CH_2)_n$-CO-N [alkyle en $C_1$-$C_8$]$_2$, $(CH_2)_n$-CO-NH-[cycloalkyle en $C_3$-$C_8$], (alcényle en $C_2$-$C_{10}$)-CO-O [alkyle en $C_1$-$C_6$], (alcényle en $C_2$-$C_{10}$)-$CONH_2$, (alcényle en $C_2$-$C_{10}$)-COOH, (alcynyle en $C_2$-$C_{10}$)-CO-O[alkyle en $C_1$-$C_6$], (alcynyle en $C_2$-$C_{10}$)-$CONH_2$, (alcynyle en $C_2$-$C_{10}$)-COOH,
$(CH_2)_n$-CO-R16,
$(CH_2)_n$-OH, $(CH_2)_n$-O- (alkyle en $C_1$-$C_8$), $(CH_2)_n$-O-(alcényle en $C_2$-$C_{10}$), $(CH_2)_n$-O-(alcynyle en $C_2$-$C_{10}$), $(CH_2)$n-O-(cycloalkyle en $C_3$-$C_8$), $(CH_2)_n$-O-$(CH_2)_q$-[cycloalkyle en $C_3$-$C_8$], $(CH_2)_n$-O-$(CH_2)_n$-CO-[O-(alkyle en $C_1$-$C_8$)], $(CH_2)_n$-O-$(CH_2)_n$-CO-[O-(cycloalkyle en $C_3$-$C_8$)], $(CH_2)_n$-O-$(CH_2)$ $_n$-CO-[alkyle en $C_1$-$C_8$], $(CH_2)_n$-O-$(CH_2)_n$-CO-[cycloalkyle en $C_3$-$C_8$], $(CH_2)_n$-O-$(CH_2)_n$-CO-[O-$(CH_2)_v$-aryle], $(CH_2)_n$-O-$(CH_2)$n-CO-[O-$(CH_2)_v$-hétéroaryle], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN , $(CH_2)_n$-O-$(CH_2)_n$-P(O)(OH)[O-(alkyleen$C_1$-$C_6$)], $(CH_2)_n$-O-$(CH_2)_n$-P(O)[O-(alkyle en $C_1$-$C_6$) ]$_2$, $(CH_2)_n$-O-$(CH_2)_n$-P(O)(OH) (O-$CH_2$-aryle) , $(CH_2)_n$-O-$(CH_2)_n$-P(O)(O-$CH_2$-aryle)$_2$, $(CH_2)_n$-O-$(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$-O-$(CH_2)_n$-CO-NH-[alkyle en $C_1$-$C_8$], $(CH_2)_n$-O-$(CH_2)_n$-CO-N [alkyle en $C_1$-$C_8$]$_2$, $(CH_2)_n$-O-$(CH_2)_n$-CO-NH-[cycloalkyle en $C_3$-$C_8$)], $(CH_2)_n$-O-$(CH_2)_n$-CR21R22-CO-O[alkyle en $C_1$-$C_6$] , $(CH_2)_n$-O-$(CH_2)_n$-CR21R22-$CONH_2$, $(CH_2)_n$-O-$(CH_2)_n$-CR21R22-COOH, $(CH_2)_n$-O-$(CH_2)_n$-CO-R16 , $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$CH_2)_r$-OH, O-R13, $OCF_3$,
$(CH_2)_n$-$NH_2$, $(CH_2)_n$-NH- (alkyle en $c_1$-$C_8$) , $(CH_2)_n$-NH-(cycloalkyle en $C_3$-$C_8$), $(CH_2)_n$-NH-$(CH_2)_n$-CO- [O-(cycloalkyle en $C_3$-$C_8$) ], $(CH_2)$ $_n$-NH- $(CH_2)$ $_n$-CO- [alkyle en $C_1$-$C_8$ ] , $(CH_2)$ $_n$-NH-$(CH_2)$ $_n$-CO- [ cycloalkyle en $C_3$-$C_8$ ] , $(CH_2)_n$-NH-$(CH_2)_n$-CO-[O-$(CH_2)_v$-aryle], $(CH_2)_n$-NH-$(CH_2)_n$-CO-[O-$(CH_2)_v$-hétéroaryle] , $(CH_2)_n$-NH-$(CH_2)_q$-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[alkyle en $C_1$-$C_6$], ( $CH_2$ ) $_n$-NH-$(CH_2)_n$-CR21R22-$CONH_2$, $(CH_2)$ $_n$-NH-$(CH_2)$ $_n$-CR21R22-COOH,
$(CH_2)_n$-NH- $(CH_2)$ $_n$-CO-R16 ,
$(CH_2)_n$-NH- $(CH_2)$ $_n$-$SO_2$-[alkyle en $C_1$-$C_8$ ] , $(CH_2$ $)_n$-NH-$(CH_2)_n$$SO_2$- [ cycloalkyle en $C_3$-$C_8$ ] , $(CH_2)_n$-NH-$SO_2$-$(CH_2)_n$-$NH_2$, $(CH_2)$ $_n$-NH-$SO_2$- $(CH_2)$ $_n$-NH- ( alkyle en $C_1$-$C_8$) , ( $CH_2$ ) $_n$-NH-$SO_2$-$(CH_2)_n$-NH- ( cycloalkyle en $C_3$-$C_8$ ), $(CH_2)$ $_n$-NH-$SO_2$- $(CH_2)$ $_n$-N[alkyle en $C_1$-$C_8$] $_2$,
$(CH_2)_n$-NH-CN, $(CH_2)_n$-NH-$SO_2$-R16,
$(CH_2)_n$-NR12-CO-NH-(alkyleen$C_1$-$C_8$), $(CH_2)$ $_n$-NR12-CO-NH-(cycloalkyle en $C_3$-$C_8$), $(CH_2)_n$-NR12-CO-$NH_2$, $(CH_2)_n$-NR12-CO-NH-$SO_2$-(alkyle en $C_1$-$C_8$) , $(CH_2)$ $_n$-NR12-CO-NH-$SO_2$-(cycloalkyle en $C_3$-$C_8$) , $(CH_2)$ $_n$-NR12-CO-N [alkyle en $C_1$-$C_2$ ] $_2$, $(CH_2)$ $_n$-NH-CO-NH-$(CH_2)_n$-CO- [ O- ( alkyle en $C_1$-$C_8$ ) ] , $(CH_2)$ $_n$-NH-CO-NH-$(CH_2)_q$-CO-$NH_2$, $(CH_2)_n$-NH-CO-NH- ( $CH_2$ ) q-COOH, ( $CH_2$ ) $_n$-NH-C (=NH ) -$NH_2$, $(CH_2)_n$-NH-C (=NH )-R16, ( $CH_2$ ) $_n$-NH-C (=NH) -NH [alkyle en $C_1$-$C_8$] , $(CH_2)$ $_n$-NH-C (=N-$SO_2$- (alkyle en $C_1$-$C_8$) -$NH_2$, $(CH_2)$ $_n$-NH-C (=N-$SO_2$- (alkyle en $c_1$-$c_8$) - NH [alkyle en $C_1$-$C_8$ ] , $(CH_2)_n$-NH-C(=N-$SO_2$-$NH_2$ -$NH_2$ $(CH_2)$ n-NH-C (=N-$SO_2$-$NH_2$ ) -NH [ alkyle en $C_1$-$C_8$ ] , $(CH_2)$ $_n$-NH-C (=NH ) - N[alkyle en $C_1$-$C_8$]$_2$, $(CH_2)$ $_n$-NH-C ( =N-$SO_2$ ) - ( alkyle en $C_1$-$C_8$)-N[alkyle en $C_1$-$C_8$ ] $_2$ , $(CH_2)$ $_n$-NH- $(CH_2)_n$-CO-O-$(CH_2)_r$-$NH_2$, $(CH_2)_n$-NH-$(CH_2)_n$-CO-NH-[ alkyle en $C_1$-$C_8$ ] , ( $CH_2$ ) $_n$-NH- ( $CH_2$ ) $_n$-CO-NH-$(CH_2)_r$-OH, $(CH_2)_n$-NH-$(CH_2)_n$-CO-N [ alkyle en $C_1$-C8 ]$_2$ , $(CH_2)_n$-NH-$(CH_2)_n$-CO-NH-[cycloalkyle en $C_3$-$C_8$], $(CH_2)$n-NH-C$(CH_3)_2$-CO-O(cycloalkyle en $C_3$-$C_8$), $(CH_2)_n$-NH-C$(CH_3)_2$-CO-O-$(CH_2)_r$-$NH_2$, $(CHF_2)_n$-NH-C$(CH_3)_2$-CO-O-$(CH_2)$n-aryle, $(CH_2)_n$-NH-C$(CH_3)_2$-CO-O-$(CH_2)_n$-hétéroaryle, $(CHF_2)_n$-NH-C$(CH_3)_2$-CO-NH-[alkyle en $C_1$-$C_8$ ], $(CH_2)_n$-NH-C$(CH_3)_2$-CO-NH-$(CH_2)_r$-OH, $(CH_2)_n$-NH-C$(CH_3)_2$-CO-N[alkyle en $C_1$-$C_8$]$_2$, $(CH_2)_n$-NH-$(CH_3)_2$-CO-NH-[cycloalkyle en $C_3$-$C_8$], $(CH_2)_n$-NH-

$C(CH_3)_2$-CON[cycloalkyle en $C_3$-$C_8$]$_2$, $(CH_2)_n$-$S(O)_m$-(alkyle en $C_1$-$C_8$) , $(CH_2)_n$-S (O)$_m$- (cycloalkyle en $C_3$-$C_8$) , $(CH_2)_n$-$SO_2$-

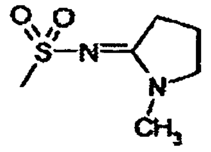

R16, $SO_2$-N=CH-N$(CH_3)_2$, $(CH_2)_n$-$SO_2$NH-CO- (alkyle en $C_1$-$C_8$ ) , $(CH_2)_n$-$SO_2$-NH-CO-(cycloalkyle en $C_3$-$C_8$), $(CH_2)$n-$SO_2$-NH-(alkyle en $C_1$-$C_8$), $(CH_2)_n$-$SO_2$-NH-(cycloalkyle en $C_3$-$C_8$), ( $CH_2$ )$_n$-$SO_2$-N [alkyle en $C_1$-$C_8$]$_2$, $SO_2$-NH- ( $CH_2$ )$_r$-OH, $SO_2$-NH-$(CH_2)$ r-$NH_2$, $SF_5$,
( $CH_2$ )$_q$-CN ,
$(CH_2)_n$-CO-NH-pipéridin-1-yle,
$(CH_2)_n$-CO-NH-$SO_2$-NHR12, $(CH_2)_n$-CO-NH-$SO_2$-(alkyle en $C_1$-$C_8$ ) , $(CH_2)_n$-CO-NH-$SO_2$-(cycloalkyle en $C_3$-$C_8$),
$(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O- (alkyle en $C_1$-$C_6$) , S (O)$_m$- (alkyle en $C_1$-$C_6$) , $SO_2$-$NH_2$, COOH, $CONH_2$, CO- [O (alkyle en $C_1$-$C_6$) ] , CO- (alkyle en $C_1$-$C_6$), et les radicaux alkyle pouvant être substitués par des atomes de fluor,
où toujours au moins l'un des radicaux R6, R7, R8, R9 et R10 représente un radical hétérocyclique T-bicyclique, T-aryle ou T-hétéroaryle, et
où l'une des quatre paires de radicaux R6 et R7, ou R7 et R8, ou R8 et R9, ou R9 et R10 peut chacune former ensemble les groupes -$CH_2$-$CH_2$-$CH_2$- ou -$CH_2$-$CH_2$-$CH_2$-$CH_2$-, où jusqu'à deux groupes -$CH_2$- peuvent être remplacés par -O-, et les groupes -$CH_2$-$CH_2$-$CH_2$- ou -$CH_2$-$CH_2$-$CH_2$-$CH_2$- pouvant être substitués par un ou des substituants F, alkyle en $C_1$-$C_8$ ou =O ;
T est
NR23-CO-NR24, NR23-$SO_2$-NR24, $SO_2$-NR23-$SO_2$, CO-NR23-CO, NR23-C(=NR13)-NR24, NR23-C(=NR22)-NR24, CO-NR23-CR22R23, CO-CR22R23-CO. CR22R23-CO-CR22R24, NR23-CO-CR22R24, NR23-$SO_2$-CR22R24, CR22R24-CO-NR23, CR22R24-$SO_2$-NR23, CR22R23-NR23-$SO_2$, $SO_2$-CP22R23-NR23, $SO_2$-R23-R22R23-, NR23-CR22R23-$SO_2$, CO-NR23 $SO_2$, $SO_2$-NR23-CO, CO-CR22R23-$SO_2$, $SO_2$-CR22R23-CO, CR23R24-CR23R24-CR23$R_{24}$. CR23R24-NR23-CR23R24;
R11 est H, un radical alkyle en $C_1$-$C_8$, alcényle en $C_2$-$C_{10}$, alcynyle en $C_2$-$C_{10}$, cycloalkyle en $C_3$-$C_8$, $(CH_2)_q$-[cycloalkyle en $C_3$-$C_8$] , $(CH_2)_n$-aryle, $(CH_2)_n$-CO-[O-(alkyle en $C_1$-$C_8$) ] , $(CH_2)_n$-CO- [O- (cycloalkyle en $C_3$-$C_8$) ] , $(CH_2)_n$-CO- [alkyle en $C_1$-$C_8$] , $(CH_2)_n$-CO- [cycloalkyle en $C_3$-$C_8$], $(CH_2)_n$-CO-aryle, $(CH_2)_n$-CO-hétéroaryle, $(CH_2)_q$-CO-$NH_2$, $(CH_2)_q$-COOH, $(CH_2)_n$-P(O) (OH) [O- (alkyle en $C_1$-$C_6$) ] , $(CH_2)_n$-P(O)[O-(alkyle en $C_1$-$C_6$)]$_2$, $(CH_2)_n$-P(O)(OH)(O-$CH_2$-aryle) , $(CH_2)_n$-P (O) (O-$CH_2$-aryle)$_2$, $(CH_2)_n$- P (O) $(OH)_2$, $(CH_2)_n$-$SO_3H$, $(CH_2)_n$-$SO_2$-$NH_2$, $(CH_2)_n$-CO-NH-[alkyle en $C_1$-$C_8$], $(CH_2)_n$-CO-N[alkyle en $C_1$-$C_8$]$_2$, $(CH_2)_n$-CO-NH-[cycloalkyle en $C_3$-$C_8$], (alcényle en $C_2$-$C_{10}$)-CO-O[alkyle en $C_1$-$C_{6]}$ , (alcényle en $C_2$-$C_{10}$) -$CONH_2$, (alcényle en $C_2$-$C_{10}$) -COOH, (alcynyle en $C_2$-$C_{10}$) -CO-O [alkyle en $C_1$-$C_6$] , (alcynyle en $C_2$-$C_{10}$) -$CONH_2$, (alcynyle en C2-$C_{10}$) -COOH, $(CH_2)$ n-CR21 [CO-O (alkyle en $C_1$-$C_6$) 12, $(CH_2)_n$-CR21 $(CONH_2)_2$, $(CH_2)_n$-CR21 $(COOH)_2$, $(CH_2)_n$-CR21R22-CO-O [alkyle en $C_1$-$C_6$] , $(CH_2)_n$-CR21R22-$CONH_2$, $(CH_2)_n$-CR21R22-CO-NH- [alkyle en $C_1$-$C_8$], $(CH_2)_n$-CR21R22-CO-N [alkyle en $C_1$-$C_8$]$_2$, ($CH_2$ n-CR21R22-COOH, $(CH_2)_n$-CO-$R_{16}$, $(CH_2)_n$-CO-NH-C ( $CH_3$ )$_2$-CO-O[alkyle en $C_1$-$C_8$], $(CH_2)_n$-CO-NH-C$(CH_3)_2$-$CONH_2$, $(CH_2)_n$-CO-NH-C$(CH_3)_2$-COOH, les radicaux alkyle, alcényle, alcynyle et cycloalkyle pouvant être substitués par des atomes de fluor, et le radical aryle ou hétéroaryle pouvant être substitué par un ou des substituants halogéno, CN, alkyle en $C_1$-$C_6$, cycloalkyle en $C_3$-$C_6$, O- (alkyle en $C_1$-$C_6$) , $S(O)_m$-(alkyle en $C_1$-$C_6$) , $SO_2$-$NH_2$, COOH, $CONH_2$, CO-O(alkyle en $C_1$-$C_6$) , CO- (alkyle en $C_1$-$C_6$) , et les radicaux alkyle pouvant être substitués par des atomes de fluor ;
R12 est H, un radical alkyle en $C_1$-$C_8$, cycloalkyle en $C_3$-$C_8$, $(CH_2)_n$-aryle, $(CH_2)_n$-hétéroaryle, les radicaux alkyle et cycloalkyle pouvant être substitués par des atomes de fluor, et le radical aryle ou hétéroaryle pouvant être substitué par un ou des substituants halogéno, CN, alkyle en $C_1$-$C_6$, O- (alkyle en $C_1$-$C_6$) , $SO_2$-$NH_2$, COOH, $CONH_2$, CO-O (alkyle en $C_1$-$C_6$) , CO- (alkyle en $C_1$-$C_6$), et les radicaux alkyle pouvant être substitués par des atomes de fluor ;
R13 est H, un radical $SO_2$- [alkyle en $C_1$-$C_8$], $SO_2$-[cycloalkyle en $C_3$-$C_8$], $SO_2$-$(CH_2)_n$-aryle, $SO_2$-$(CH_2)_n$-hétéroaryle,

les radicaux alkyle et cycloalkyle pouvant être substitués par des atomes de fluor, et le radical aryle ou hétéroaryle pouvant être substitué par un ou des substituants halogéno, CN, $CF_3$, alkyle en $C_1$-$C_6$, cycloalkyle en $C_3$-$C_6$, O-[alkyle en $C_1$-$C_6$], $S(O)_m$-[alkyle en $C_1$-$C_6$] , $SO_2$-$NH_2$, COOH, $CONH_2$, CO- [O (alkyle en $C_1$-$C_6$)], CO-(alkyle en $C_1$-$C_6$), et les radicaux alkyle pouvant être substitués par des atomes de fluor ;

R14 est H, un radical alkyle en $C_1$-$C_8$, cycloalkyle en $C_3$-$C_8$, $(CH_2)_n$-aryle, $(CH_2)_n$-hétéroaryle, $(CH_2)_n$-CO-[O-(alkyle en $C_1$-$C_8$) ], $(CH_2)_n$-CO-[O-$(CH_2)_n$-aryle], $(CH_2)_n$-CO- [alkyle en $C_1$-$C_8$], $(CH_2)_n$-CO-[cycloalkyle en $C_3$-$C_8$] , $(CH_2)_n$-CO-aryle, $(CH_2)_n$-CO-hétéroaryle, $(CH_2)$ q-COOH, les radicaux alkyle et cycloalkyle pouvant être substitués par des atomes de fluor, et le radical aryle ou hétéroaryle pouvant être substitué par un ou des substituants halogéno, CN, alkyle en $C_1$-$C_6$, cycloalkyle en $C_3$-$C_6$, O- (alkyle en $C_1$-$C_6$) , S (O) m- (alkyle en $C_1$-$C_6$) , $SO_2$-$NH_2$, COOH, $CONH_2$, CO-O (alkyle en $C_1$-$C_6$) , CO- (alkyle en $C_1$-$C_6$), et les radicaux alkyle pouvant être substitués par des atomes de fluor ;

R15 est H, un radical alkyle en $C_1$-$C_8$, cycloalkyle en $C_3$-$C_8$, $(CH_2)_n$-aryle, $(CH_2)_n$-hétéroaryle, $(CH_2)_n$-CO-[O-(alkyle en $C_1$-$C_8$)], CO- [alkyle en $C_1$-$C_8$], CO- [cycloalkyle en $C_3$-$C_8$] , CO-aryle, CO-hétéroaryle, $(CH_2)_n$-CO-$NH_2$, $(CH_2)_q$-COOH, $(CH_2)_n$-$SO_2$-$NH_2$, $(CH_2)_n$-$C(CH_3)_2$-COOH,
les radicaux alkyle et cycloalkyle pouvant être substitués par des atomes de fluor, et le radical aryle ou hétéroaryle pouvant être substitué par un ou des substituants halogéno, CN, alkyle en $C_1$-$C_6$, O- (alkyle en $C_1$-$C_6$) , $SO_2$-$NH_2$, COOH, $CONH_2$, CO-O (alkyle en $C_1$-$C_6$) , CO-(alkyle en $C_1$-$C_6$), et les radicaux alkyle pouvant être substitués par des atomes de fluor ;

R16 est le groupe aziridin-1-yle, azétidin-1-yle, 3-hydroxy-azétidin-1-yle, pipéridin-1-yle, pyrrolidin-1-yle, 3-pyrrolidinol-1-yle, morpholin-N-yle, pipérazin-1-yle, un radical 4-[alkyle en $C_1$-$C_6$] pipérazin-1-yle, le groupe thiomorpholin-4-yle, thiomorpholin-1,1-dioxyde-4-yle, un radical NH- $(CH_2)_r$-OH, NH-CH ($CH_2$OH) 2, NH-C ($CH_2$OH) $_3$, N- [ (alkyle en $C_1$-$C_6$) -OH] $_2$, N-[alkyle en $C_1$-$C_6$] [(alkyle en $C_1$-$C_6$)-OH], D-glucamin-N-yle, N-méthyl-D-glucamin-N-yle, NH- [alkyle en $C_1$-$C_8$]-CO-O(alkyle en $C_1$-$C_6$) , NH- [alkyle en $C_1$-$C_8$] -COOH, NH- [alkyle en $C_1$-$C_8$] - $CONH_2$, N[alkyle en $C_1$-$C_6$][alkyle en $C_1$-$C_8$]-CO-O(alkyle en $C_1$-$C_6$) , N [alkyle en $C_1$-$C_6$] [alkyle en $C_1$-$C_8$] -COOH, N [alkyle en $C_1$-$C_6$] [alkyle en $C_1$-$C_8$] -$CONH_2$, NH-[C (H) (aryle) ] -CO-O (alkyle en $C_1$-$C_6$] , NH- [C (H) (aryle) ] - COOH, NH- [C (H) (aryle) ] -$CONH_2$, N [alkyle en $C_1$-$C_6$] [C (H) (aryle) ] -CO-O (alkyle en $C_1$-$C_6$) , N [alkyle en $C_1$-$C_6$] [C (H) (aryle) ] -COOH, N [alkyle en $C_1$-$C_6$] [C (H) (aryle) ] - $CONH_2$, NH-[C(H)(hétéroaryle)]-CO-O(alkyle en $C_1$-$C_6$), NH-[C(H)(hétéroaryle)]-COOH, NH-[C(H)(hétéroaryle)]-$CONH_2$, N[alkyle en $C_1$-$C_6$] [C(H)(hétéroaryle)]-CO-O(alkyle en $C_1$-$C_6$), N[alkyle en $C_1$-$C_6$] [C(H)(hétéroaryle)]-COOH, N[alkyle en $C_1$-$C_6$][C(H)(hétéroaryle)]-$CONH_2$, N[alkyle en $C_1$-$C_6$] [cycloalkyle en $C_3$-$C_8$] -CO-O (alkyle en $C_1$-$C_6$) , N [alkyle en $C_1$-$C_6$] [cycloalkyle en $C_3$-$C_8$] -COOH, N [alkyle en $C_1$-$C_6$] [cycloalkyle en $C_3$-$C_8$]-$CONH_2$, NH-[cycloalkyle en $C_3$-$C_8$]-CO-O(alkyle en $C_1$-$C_6$) , NH- [cycloalkyle en $C_3$-$C_8$] - COOH, NH-[cycloalkyle en $C_3$-$C_8$]-$CONH_2$, NH-(alkyle en $C_1$-$C_8$)-OH, NH-[alkyle en $C_1$-$C_6$] -$SO_2$- (alkyle en $C_1$-$C_6$) , NH-[alkyle en $C_1$-$C_6$]-$SO_3H$, NH-[alkyle en $C_1$-$C_6$] -$S0_2$-$NH_2$, N [alkyle en $C_1$-$C_6$] {[alkyle en $C_1$-$C_6$]-$SO_3H$),
les fonctions alcool (OH) pouvant être remplacées par F, et le radical aryle ou hétéroaryle pouvant être substitué par un ou des substituants halogéno, CN, alkyle en $C_1$-$C_6$, O-alkyle en $C_1$-$C_6$, OH, SO2-$NH_2$, COOH, $CONH_2$, CO-O (alkyle en $C_1$-$C_6$) , CO- (alkyle en $C_1$-$C_6$) ;

R18 est un radical $(CH_2)_n$-CR25R26-CO-O(alkyle en $C_1$-$C_6$), ($CH_{2\,n}$-CR25R26-CO-$NH_2$, ( $CH_2$ ) $_n$-$CR_2$ 5$R_2$ 6-COOH ;

R20 est H, un radical alkyle en $C_1$-$C_6$, cycloalkyle en $C_3$-$C_8$, aryle, [alkyle en $C_1$-$C_6$]-aryle ;

R21 est H, F, $CF_3$, un radical alkyle en $C_1$-$C_6$, cycloalkyle en $C_3$-$C_8$, OH, O-(alkyle en $C_1$-$C_6$) , 0-(cycloalkyle en $C_3$-$C_8$), O-$(CH_2)_n$-aryle, O-(CO)-(alkyle en $C_1$-$C_6$) , O-(CO) - (cycloalkyle en $C_3$-$C_8$), O-(CO) -O- (alkyle en $C_1$-$C_6$) , O- (CO) -O- (cycloalkyle en $C_3$-$C_8$), NH- [alkyle en $C_1$-$C_6$]-aryle, $NH_2$, NH- (alkyle en $C_1$-$C_6$) , NH- (CO) - (alkyle en $C_1$-$C_6$) ;

R22 est H, $CF_3$, un radical alkyle en $C_1$-$C_6$, aryle, [alkyle en $C_1$-$C_6$]-aryle ;

R23, R24 représentent chacun indépendamment de l'autre H, un radical alkyle en $C_1$-$C_6$, cycloalkyle en $C_3$-$C_8$, [alkyle en $C_1$-$C_6$]-[cycloalkyle en $C_3$-$C_8$], aryle, [alkyle en $C_1$-$C_6$] -aryle,
ou R23 et R24 forment ensemble un motif -CH=CH-, -$CH_2$-$CH_2$-, -$CH_2$-$CH_2$-$CH_2$- ou -$CH_2$-$CH_2$-$CH_2$-$CH_2$-, où un groupement $CH_2$ peut être remplacé par C=O, CHF ou $CF_2$, et jusqu' à quatre atomes d'hydrogène pouvant être remplacés par un radical alkyle en $C_1$-$C_6$ ;

R25, R26 représentent chacun indépendamment de l'autre H, F, un radical alkyle en $C_1$-$C_6$, aryle, [alkyle en $C_1$-$C_6$]-aryle, le radical aryle pouvant être substitué par un substituant halogéno, CN, OH, O- (alkyle en $C_1$-$C_6$) , ou les radicaux R25 et 26 forment, avec l'atome de carbone auquel ils sont liés, un radical carbocyclique à trois à sept chaînons, dans lequel un atome de carbone peut être remplacé par O, S (O) $_m$, NH, un radical N-[alkyle en $C_1$-$C_6$] ou CO ;

à l'exception du composé N-[3-tert-butyl-1-(4-méthylphényl)-1H-pyrazol-5-yl]-N'(4-{[3-(2-méthylphényl)-2,4-dioxo-1-imidazolidinyl]méthyl]}-phényl)urée,
ainsi que leurs sels physiologiquement tolérés.

**2.** Composés de formule I selon la revendication 1, **caractérisés en ce que**, dans cette formule,

R, R' représentent chacun indépendamment de l'autre H, un radical $(CH_2)_n$-aryle, alkyle en $C_1$-$C_6$, le radical alkyle en $C_1$-$C_6$ ou le radical aryle pouvant être substitué par un ou des substituants halogéno ;
ou R et R' forment ensemble un noyau ayant trois à huit atomes de carbone, un atome de carbone pouvant être remplacé par 0, $S(O)_m$, NR13 ou NR15 ;
m vaut 0, 1, 2 ;
n vaut 0, 1, 2, 3 ;
p vaut 1, 2, 3, 4 ;
q vaut 1, 2, 3 ;
r vaut 2, 3, 4, 5 ;
v vaut 0, 1, 2, 3 ;
A, D, E, G, L représentent chacun indépendamment des autres C ou N, où, pour la signification N, le substituant correspondant R1, R2, R3, R4, R5 disparaît, ou R2-D=E-R3 ou R4-G=L-R5 représentent S ou 0, et le noyau à cinq ou six chaînons peut être condensé à un radical $-(CH_2)_3-$ ou $-(CH_2)_4-$ ou -CH=CH-CH=CH- pour donner un radical bicyclique ;
R1, R2, R3, R4, R5 représentent chacun indépendamment des autres H, F, C1, Br, I, CN, $CF_3$, un radical alkyle en $C_1$-$C_8$, cycloalkyle en $C_3$-$C_8$, $(CH_2)_q$-[cycloalkyle en $C_3$-$C_8$], $(CH_2)_n$- [bicycloalkyle en $C_7$-$C_{12}$], $(CH_2)_n$-[tricycloalkyle en $C_7$-$C_{12}$], adamantan-1-yle, adamantan-2-yle, $(CH_2)_n$-aryle, $(CH_2)_n$-hétéroaryle, $OCF_3$, O-R11, NR13R15, NH-CN, S (O) $_m$-R12, $SO_2$-$NH_2$, $SO_2$-N=CH-N $(CH_3)$ 2, $SO_2$-NH-[alkyle en $C_1$-$C_{81}$, $SO_2$-NH-[cycloalkyle en $C_3$-$C_{8]}$ , $SO_2$-NH- $(CH_2)$ n-aryle, $SO_2$-NH- $(CH_2)_n$-hétéroaryle, $SO_2$-N [alkyle en $C_1$-$C_8$]$_2$, $SO_2$-R16, $SF_5$, CO-O [alkyle en $C_1$-$C_8$], CO-O[cycloalkyle en $C_3$-$C_8$], CO-O-$(CH_2)_n$-aryle, CO-O-$(CH_2)_n$-hétéroaryle, CO-$NH_2$, CO-NH-CN, CO-NH-[alkyle en $C_1$-$C_8$], CO-N [alkyle en $C_1$-$C_8$]$_2$, CO-NH- [cycloalkyle en $C_3$-$C_8$] , C(=NH)-O-[alkyle en $C_1$-$C_6$], C(=NH)-$NH_2$, C(=NH)-R16, C(=NR13)-NR12R13, $(CH_2)_n$-C(=$NSO_2$-R12)$NH_2$, CO-R16, COOH, CO-(alkyle en $C_1$-$C_8$), CO-(cycloalkyle en $C_3$-$C_8$), CO-aryle, CO-hétéroaryle, CH(OH)-aryle, CH(OH)-hétéroaryle, CH[O-(alkyle en $C_1$-$C_6$) -aryle, CH[O-(alkyle en $C_1$-$C_6$]-hétéroaryle, CHF-aryle, CHF-hétéroaryle, $CF_2$-aryle, $CF_2$-hétéroaryle, CHO, $CH_2$-OH, $CH_2$-CN, $CH_2$-O-R12, $CH_2$-O-$(CH_2)_q$-COOH,
les radicaux alkyle, cycloalkyle, cycloalcényle, bicycloalkyle et tricycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O- (alkyle en $C_1$-$C_6$), $OCF_3$, OH, O- $(CH_2)_n$-aryle, $(CH_2)_n$-aryle; S $(O)_m$- (alkyle en $C_1$-$C_6$), $SO_2$-$NH_2$, SH, NR12R13, NH-CO- [alkyle en $C_1$-$C_6$] , NH-CO- $(CH_2)_n$-aryle, $(CH_2)_n$-COOH, $(CH_2)_n$-$CONH_2$,$(CH_2)_n$-CO-O(alkyle en $C_1$-$C_6$), $(CH_2)_n$-CO-(alkyle en $C_1$-$C_6$), et les radicaux alkyle pouvant être substitués par des atomes de fluor ;
R6, R7, R8, R9, R10 représentent chacun indépendamment des autres un radical hétérocyclique T-bicyclique, T-aryle ou T-hétéroaryle, le radical hétérocyclique bicyclique ou le radical aryle ou hétéroaryle pouvant être condensé à un noyau carboné aromatique ou non aromatique à 5 ou 6 chaînons, dans lequel un ou plusieurs groupes CH ou $CH_2$ peuvent être remplacés par des atomes d'oxygène, et le noyau carboné aromatique ou non aromatique à 5 ou 6 chaînons pouvant être substitué par un ou des substituants F, =O ou -(alkyle en $C_1$-$C_6$) , et le radical hétérocyclique bicyclique pouvant contenir 9 à 12 chaînons, et jusqu'à cinq groupes CH ou $CH_2$ peuvent, indépendamment les uns des autres, être remplacés par N, NR20, O, $S(O)_m$ ou C=O, et le radical aryle ou hétéroaryle ou le radical hétérocyclique bicyclique pouvant être non substitué, ou une ou plusieurs fois substitué par les substituants suivantes :

R11, F, C1, Br, I, CN, $N_3$, NC, $NO_2$, $CF_3$, $(CH_2)_n$-OR11, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)$r-$NH_2$, $(CH_2)$n-O-$(CH_2)_n$-CO-NH-$(CH_2)_r$-OH, O-R13 , $OCF_3$ , $(CH_2)_n$-O-$(CH_2)_r$-$NH_2$, $(CH_2)_n$-NH-R11, $(CH_2)_n$-N[$(CH_2)_q$-CO-O (alkyle en $C_1$-$C_6$] $_2$, $(CH_2)_n$-N[$(CH_2)$q-COOH]$_2$, $(CH_2)_n$-N[$(CH_2)_q$-$CONH_2$]$_2$, $(CH_2)_n$-NH-R13, $(CH_2)_n$-N$(R_{13})_2$, $(CH_2)_n$-NH-CN,$(CH_2)_n$-NH-$SO_2$-R16, $(CH_2)_n$-NH-$(CH_2)$n-$SO_2$-R12, $(CH_2)_n$-NR12-COR16, $(CH_2)_n$-NR12-CO-NR12R13, $(CH_2)_n$-NR12-CO-N $(R12)_2$, $(CH_2)_n$-NR12-CO-NHR11, $(CH_2)_n$-NH-C(=NH)-$NH_2$, $(CH_2)_n$-NH-C(=NH)-R16, $(CH_2)_n$-NH-C(=NH)-NHR12 , $(CH_2)_{2n}$-NR12-C(=NR13)-$NHR1_2$, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$-CO-NH- [ alkyle en $C_1$-$C_8$], $(CH_2)_n$-NH-$(CH_2)_n$-CO-NH-$(CH_2)_r$-OH, $(CH_2)_n$-NH- $(CH_2)_n$-CO- N [ alkyle en $C_1$-$C_8$]$_2$, $(CH_2)_n$-NH- $(CH_2)_n$-CO-NH- [ cycloalkyle en $C_3$-$C_8$ ] , $(CH_2)_n$-NH-$(CH_2)_n$-CO-N [cycloalkyle en $C_3$-$C_8$] $_2$, $(CH_2)_n$-NH-C $(CH_3)_2$-CO-O ( alkyle en $C_1$-$C_8$), $(CH_2)_n$-NH-C$(CH_3)_2$-CO-O ( cycloalkyle en $C_3$-$C_8$ ) , $(CH_2)_n$-NH-C$(CH_3)_2$-CO-O-$(CH_2)_r$-$NH_2$, $(CH_2)_n$-NH-C$(CH_3)_2$-CO-O-$(CH_2)_n$-aryle, $(CH_2)_n$-NH-C$(CH_3)_2$-CO-O-$(CH_2)_n$-hétéroaryle, $(CH_2)_n$-NH-C$(CH_3)_2$-CO-$NH_2$, $(CH_2)_n$-NH-C$(CH_3)_2$-CO-NH-[alkyle en $C_1$-$C_8$], $(CH_2)_n$-NH-C$(CH_3)_2$-CO-NH-$(CH_2)_r$-OH,$(CH_2)_n$-NH-C$(CH_3)_2$-CO-N[alkyle en $C_1$-$C_8$]$_2$, $(CH_2)_n$-NH-$(CH_3)_2$-CO-NH- [ cycloalkyle en $C_3$-$C_8$ ] , $(CH_2)$n-NH-

$C(CH_3)_2$-CON [cycloalkyle en $C_3$-$C_8$] $_2$, $(CH_2)_n$-NH-$C(CH_3)_2$-COOH, S $(O)_m$-R12, $SO_2$-$R_{16}$, $SO_2$-N=CH-$N(CH_3)_2$,

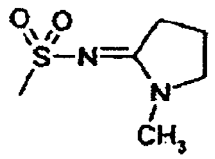

$SO_2$-NH-CO-R12 , $SO_2$-NHR12, $SO_2$-NH-$(CH_2)_r$-OH, $SO_2$-N [alkyle en $C_1$-$C_8$] $_2$, $SO_2$-NH- $(CH_2)_r$-$NH_2$, $SF_5$, COOH, CO-$NH_2$,$(CH_2)_q$-CN, $(CH_2)_n$-CO-NH-CN, $(CH_2)_n$-CO-NH-pipéridin-1-yle, $(CH_2)_n$-CO-NH-$SO_2$-NHR12, $(CH_2)_n$-CO-NH-$SO_2$-R18 , $(CH_2)_n$-CHO, $(CH_2)_n$-C(=NH)$NH_2$,$(CH_2)_n$-C(=NH)-NHOH, $(CH_2)_n$-C(=NH)-[NH-O-(alkyle en $C_1$-$C_6$)], $(CH_2)_n$-C (=NH) (R16) , $(CH_2)_n$-C (=NR13) NHR12, $(CH_2)_n$-C(=NR12)NR12R13, $(CH_2)_n$-C(=$NSO_2$-R12)$NH_2$, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O- (alkyle en $C_1$-$C_6$) , S $(O)_m$- (alkyle en $C_1$-$C_6$) , $SO_2$-$NH_2$, COOH, $CONH_2$, CO-O (alkyle en $C_1$-$C_6$) , CO- (alkyle en $C_1$-$C_6$), et les radicaux alkyle pouvant être substitués par des atomes de fluor, et, quand R3 est CN, $NO_2$ ou un groupe halogéno et R4 est $CF_3$ ou un groupe halogéno et R est R' et est le groupe méthyle, alors le radical X-aryle est pourvu d'au moins l'un des substituants mentionnés ci-dessus, différents de l'hydrogène ;

H, F, C1, Br, I, CN, $N_3$, NC, $NO_2$, $CF_3$,
alkyle en $C_1$-$C_8$, alcényle en $C_2$-$C_{10}$, alcynyle en $C_2$-$C_{10}$, cycloalkyle en $C_3$-$C_8$,
aryle, hétéroaryle,
$(CH_2)_n$-CO-[O-(alkyle en $C_1$-$C_8$)], $(CH_2)_n$, -CO-[O-(cycloalkyle en $C_3$-$C_8$) ] , $(CH_2)_n$-CO- [alkyle en $C_1$-$C_8$], $(CH_2)_n$-CO-[cycloalkyle en $C_3$-$C_8$] ,
$(CH_2)_n$-CO-[O-$(CH_2)_v$-aryle],
$(CH_2)_n$-CO-$NH_2$, $(CH_2)_n$-COOH, $(CH_2)_n$-CO-NH-CN,
$(CH_2)_n$-P (O) (OH) [O- (alkyle en $C_1$-$C_6$) ] , $(CH_2)_n$-P (0) [O-(alkyle en $C_1$-$C_6$)]$_2$, $(CH_2)_n$-P (O) (OH) (O-$CH_2$-aryle) , $(CH_2)_n$-P (O) (O-$CH_2$-aryle) $_2$, $(CH_2)_n$-P (O) (OH) $_2$, $(CH_2)_n$-$SO_3H$, $(CH_2)_n$-$SO_2$-$NH_2$,
$(CH_2)_n$-CO-NH- [alkyle en $C_1$-$C_8$] , $(CH_2)_n$-CO-N [alkyle en $C_1$-$C_8$] $_2$, $(CH_2)_n$-CO-NH- [cycloalkyle en $C_3$-$C_8$] , (alcényle en $C_2$-$C_{10}$) -CO-O [alkyle en $C_1$-$C_6$] , (alcényle en $C_2$-$C_{10}$) -$CONH_2$, (alcényle en $C_2$-$C_{10}$) -COOH, (alcynyle en $C_2$-$C_{10}$)-CO-O[alkyle en $C_1$-$C_6$], (alcynyle en $C_2$-$C_{10}$)-$CONH_2$, (alcynyle en $C_2$-$C_{10}$)-COOH,
$(CH_2)_n$-CO-R16,
$(CH_2)_n$-OH, $(CH_2)_n$-O-(alkyle en $C_1$-$C_8$), $(CH_2)_n$-O-(alcényle en $C_2$-$C_{10}$), $(CH_2)_n$-O-(alcynyle en $C_2$-$C_{10}$), $(CH_2)_n$-O-(cycloalkyle en $C_3$-$C_8$), $(CH_2)_n$-O-$(CH_2)_q$-[cycloalkyle en $C_3$-$C_8$], $(CH_2)_n$-O-$(CH_2)_n$-CO-[O-(alkyle en $C_1$-$C_8$)], $(CH_2)_n$-O-$(CH_2)_n$-CO-[O-(cycloalkyle en $C_3$-$C_8$)], $(CH_2)_n$-O-$(CH_2)_n$-CO-[alkyle en $C_1$-$C_8$], $(CH_2)_n$-O-$(CH_2)_n$-CO-[cycloalkyle en $C_3$-$C_8$], $(CH_2)_n$-O-$(CH_2)_n$-CO-[O-$(CH_2)_v$-aryle], $(CH_2)_n$-O-$(CH_2)_n$-CO-[O-$(CH_2)_v$-hétéroaryle], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P (O) (OH) [O- (alkyle en $C_1$-$C_6$) ] , $(CH_2)_n$-O- $(CH_2)_n$-P (O) [ O-(alkyle en $C_1$-$C_6$)]$_2$, $(CH_2)_n$-O-$(CH_2)_n$-P(O)(OH)(O-$CH_2$-aryle) , $(CH_2)_n$-O- $(CH_2)_n$-P (O) (O-$CH_2$-aryle) $_2$, $(CH_2)_n$-O-$(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$-O- $(CH_2)_n$-CO-NH- [ alkyle en $C_1$-$C_8$ ] , $(CH_2)_n$-O-$(CH_2)_n$-CO-N [ alkyle en $C_1$-$C_8$ ] $_2$ , $(CH_2)_n$-O-$(CH_2)_n$-CO-NH-[cycloalkyle en $C_3$-$C_8$] , $(CH_2)_n$-O-$(CH_2)_n$-CR21R22-CO-O [alkyle en $C_1$-$C_6$] , $(CH_2)$ n-O- $(CH_2)$ n-CR21R22-$CONH_2$, $(CH_2)_n$-O- $(CH_2)_n$-CR21R22-COOH, $(CH_2)_n$-O- $(CH_2)_n$-CO-R16, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$, $(CH_2)_n$-$NH_2$, $(CH_2)_n$-NH-(alkyle en $C_1$-$C_8$), $(CH_2)_n$-NH-(cycloalkyle en $C_3$-$C_8$), $(CH_2)_n$-NH-$(CH_2)_n$-CO-[O-(cycloalkyle en $C_3$-$C_8$) ], $(CH_2)_n$-NH-$(CH_2)$n-CO-[alkyle en $C_1$-$C_8$], $(CH_2)_n$-NH-$(CH_2)_n$-CO-[cycloalkyle en $C_3$-$C_8$], $(CH_2)_n$-NH-$(CH_2)_n$-CO-[O-$(CH_2)_v$-aryle], $(CH_2)_n$-NH-$(CH_2)_n$-CO-[O-$(CH_2)_v$-hétéroaryle], $(CH_2)_n$-NH-$(CH_2)_q$-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH- $(CH_2)_n$-$SO_2$-$NH_2$,
$(CH_2)_n$-NH- $(CH_2)_n$-CR21R22-CO-O[alkyle en $C_1$-$C_6$], $(CH_2)_n$-NH-$(CH_2)_n$-CR21R22-$CONH_2$, $(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[alkyle en $C_1$-$C_8$], $(CH_2)_n$-NH-$(CH_2)_n$-$SO_2$-[cycloalkyle en $C_3$-$C_8$], $(CH_2)_n$-NH-$SO_2$-

$(CH_2)_n$-$NH_2$, $(CH_2)_n$-NH-$SO_2$-$(CH_2)_n$-NH-(alkyle en $C_1$-$C_8$), $(CH_2)_n$-NH-$SO_2$-$(CH_2)_n$-NH-(cycloalkyle en $C_3$-$C_8$), $(CH_2)_n$-NH-$SO_2$-$(CH_2)_n$-N[alkyle en $C_1$-$C_8$]$_2$,
$(CH_2)_n$-NH-CN, $(CH_2)_n$-NH-$SO_2$-$R_{16}$,
(CH2)$_n$-NR12-CO-NH- (alkyle en $C_1$-$C_8$), $(CH_2)_n$-NR12-CO-NH-(cycloalkyle en $C_3$-$C_8$), $(CH_2)_n$-NR12-CO-$NH_2$, $(CH_2)_n$-NR12-CO-NH-$SO_2$- (alkyle en $C_1$-$C_8$), $(CH_2)_n$-NR12-CO-NH-$SO_2$-(cycloalkyle en $C_3$-$C_8$), $(CH_2)_n$-NR12-CO-N[alkyle en $C_1$-$C_8$]$_2$, $(CH_2)_n$-NH-CO-NH-$(CH_2)_n$-CO-[O-(alkyle en $C_1$-$C_8$)], $(CH_2)_n$-NH-CO-NH-$(CH_2)_q$-CO-$NH_2$, $(CH_2)_n$-NH-CO-NH-$(CH_2)_q$-COOH, $(CH_2)_n$-NH-C(=NH)-$NH_2$, $(CH_2)_n$-NH-C(=NH)-R16, $(CH_2)_n$-NH-C(=NH)-NH [alkyle en $C_1$-$C_8$], $(CH_2)_n$-NH-C(=N-$SO_2$- (alkyle en $C_1$-$C_8$)-$NH_2$, $(CH_2)_n$-NH-C(=N-$SO_2$-(alkyle en $C_1$-$C_8$)-NH[alkyle en $C_1$-$C_8$], $(CH_2)_n$-NH-C (=N-$SO_2$-$NH_2$)-$NH_2$, $(CH_2)_n$-NH-C(=N-$SO_2$-$NH_2$)-NH[alkyle en $C_1$-$C_8$], $(CH_2)_n$-NH-C(=NH)-N[alkyle en $C_1$-$C_8$]$_2$, $(CH_2)_n$-NH-C(=N-$SO_2$)-(alkyle en $C_1$-$C_8$)-N[alkyle en $C_1$-$C_8$]$_2$, $(CH_2)_n$-NH-$(CH_2)_n$-CO-O-$(CH_2)_r$-$NH_2$, $(CH_2)_n$-NH-$(CH_2)_n$-CO-NH-[alkyle, en $C_1$-$C_8$], $(CH_2)_n$-NH-$(CH_2)_n$-CO-NH- $(CH_2)_r$-OH, $(CH_2)_n$-NH-$(CH_2)_n$-CO-N[alkyle en $C_1$-$C_8$]$_2$, $(CH_2)_n$-NH-$(CH_2)_n$-CO-NH-[cycloalkyle en $C_3$-$C_8$], $(CH_2)_n$-NH-$C(CH_3)_2$-CO-O(cycloalkyle en $C_3$-$C_8$), $(CH_2)_n$-NH-$C(CH_3)_2$-CO-O-$(CH_2)_r$-$NH_2$, $(CH_2)_n$-NH-$C(CH_3)_2$-CO-O-$(CH_2)_n$-aryle, $(CH_2)_n$-NH-$C(CH_3)_2$-CO-O-$(CH_2)_n$-hétéroaryle, $(CH_2)_n$-NH-$C(CH_3)_2$-CO-NH-[alkyle en $C_1$-$C_8$], $(CH_2)_n$-NH-$C(CH_3)_2$-CO-NH-$(CH_2)_r$-OH, $(CH_2)_n$-NH-$C(CH_3)_2$-CO-N[alkyle en $C_1$-$C_8$]$_2$, $(CH_2)_n$-NH-$(CH_3)_2$-CO-NH-[cycloalkyle en $C_3$-$C_8$], $(CH_2)_n$-NH-$C(CH_3)_2$-CON[cycloalkyle en $C_3$-$C_8$]$_2$, $(CH_2)_n$-$S(O)_m$-(alkyle en $C_1$-$C_8$), $(CH_2)_n$-$S(O)_m$-(cycloalkyle en $C_3$-$C_8$), $(CH_2)_n$-$SO_2$-

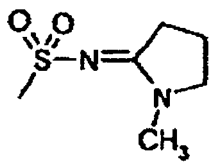

R16, $SO_2$-N=CH-N$(CH_3)_2$, $(CH_2)_n$-$SO_2$-NH-CO-(alkyle en $C_1$-$C_8$), $(CH_2)_n$-$SO_2$-NH-CO-(cycloalkyle en $C_3$-$C_8$), $(CH_2)_n$-$SO_2$-NH-(alkyle en $C_1$-$C_8$), $(CH_2)_n$-$SO_2$-NH-(cycloalkyle en $C_3$-$C_8$), $(CH_2)_n$-$SO_2$-N[alkyle en $C_1$-$C_8$]$_2$, $SO_2$-NH-$(CH_2)_r$-OH, $SO_2$-NH-$(CH_2)_r$-$NH_2$, $SF_5$,
$(CH_2)_q$-CN,
$(CH_2)_n$-CO-NH-pipéridin-1-yle,
$(CH_2)_n$-CO-NH-$SO_2$-NHR12, $(CH_2)_n$-CO-NH-$SO_2$- (alkyle en $C_1$-$C_8$), $(CH_2)_n$-CO-NH-$SO_2$-(cycloalkyle en $C_3$-$C_8$),
$(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O-(alkyle en $C_1$-$C_6$), $S(O)_m$-(alkyle en $C_1$-$C_6$), $SO_2$-$NH_2$, COOH, $CONH_2$, CO-[O(alkyle en $C_1$-$C_6$)], CO-(alkyle en $C_1$-$C_6$), et les radicaux alkyle pouvant être substitués par des atomes de fluor,
où toujours au moins l'un des radicaux R6, R7, R8, R9 et R10 représente un radical hétérocyclique T-bicyclique, T-aryle ou T-hétéroaryle, et
l'une des quatre paires de radicaux R6 et R7, ou R7 et R8, ou R8 et R9, ou R9 et R10, peut chacune former ensemble les groupes -$CH_2$-$CH_2$-$CH_2$- ou -$CH_2$-$CH_2$-$CH_2$-$CH_2$-, où jusqu'à deux groupes -$CH_2$- peuvent être remplacés par -O-, et les groupes -$CH_2$-$CH_2$-$CH_2$- ou -$CH_2$-$CH_2$-$CH_2$-$CH_2$- pouvant être substitués par un ou des substituants F, alkyle en $C_1$-$C_8$ ou =O ;
T est
NR23-CO-NR24, NR23-$SO_2$-NR24, $SO_2$NR23-$SO_2$, CO-NR23-CO, NR23-C(=NR13)-NR24, NR23-C(=NR22)-NR24, CO-NR23-CR22R23, CO-CR22R23-CO, CR22R23-CO-CR22R24, NR23-CO-CR22R24, NR23-$SO_2$-CR22R24, CR22R24-CO-NR23, CR22R24-$SO_2$-NR23, CR22R23-NR23-$SO_2$, $SO_2$-CR22R23-NR23, $SO_2$-NR23-CR22R23-, NR23-CR22R23-$SO_2$ CO-$NR_{23}$-$SO_2$, $SO_2$-NR23-CO, CO-CR22R23-$SO_2$, $SO_2$-CR22R23-CO, CR23R24-CR23R24-CR23R24, CR23R24-NR23-CR23R24;
R11 est H, un radical alkyle en $C_1$-$C_8$, alcényle en $C_2$-$C_{10}$, alcynyle en $C_2$-$C_{10}$, cycloalkyle en $C_3$-$C_8$, $(CH_2)_q$-[cycloalkyle en $C_3$-$C_8$], $(CH_2)_n$-aryle, $(CH_2)_n$-CO-[O-(alkyle en $C_1$-$C_8$)], $(CH_2)_n$-CO-[O-(cycloalkyle en $C_3$-$C_8$)], $(CH_2)_n$-CO-[alkyle en $C_1$-$C_8$], $(CH_2)_n$-CO-[cycloalkyle en $C_3$-$C_8$], (CH$_2$)n-CO-aryle, $(CH_2)_n$-CO-hétéroaryle, $(CH_2)_q$-CO-$NH_2$, $(CH_2)_q$-COOH, $(CH_2)_n$-P(O)(OH)[O-(alkyle en $C_1$-$C_6$)], $(CH_2)_n$-P(O)[O-(alkyle en $C_1$-$C_6$)]$_2$, $(CH_2)_n$-P(O)(OH)(O-$CH_2$-aryle), $(CH_2)_n$-P(O)(O-$CH_2$-aryle)$_2$, $(CH_2)_n$-P(O)$(OH)_2$, $(CH_2)_n$-$SO_3H$, $(CH_2)_n$-$SO_2$-$NH_2$, $(CH_2)_n$-CO-NH-[alkyle en $C_1$-$C_8$], $(CH_2)_n$-CO-N[alkyle en $C_1$-$C_8$]$_2$, $(CH_2)_n$-CO-NH-[cycloalkyle en $C_3$-$C_8$], (alcényle en $C_2$-$C_{10}$)-CO-O[alkyle en $C_1$-$C_6$], (alcényle en $C_2$-$C_{10}$)-$CONH_2$, (alcényle en

$C_2$-$C_{10}$)-COOH, (alcynyle en $C_2$-$C_{10}$)-CO-O[alkyle en $C_1$-$C_6$], (alcynyle en $C_2$-$C_{10}$)-$CONH_2$, (alcynyle en $C_2$-$C_{10}$)-COOH, $(CH_2)_n$-CR21[CO-O(alkyle en $C_1$-$C_6$)]$_2$, $(CH_2)_n$-CR21$(CONH_2)_2$, $(CH_2)_n$-CR21$(COOH)_2$, $(CH_2)_n$-CR21R22-CO-O[alkyle en $C_1$-$C_6$], $(CH_2)_n$-CR21R22-$CONH_2$, $(CH_2)_n$-CR21R22-CO-NH-[alkyle en $C_1$-$C_8$], $(CH_2)_n$-CR21R22-CO-N[alkyle en $C_1$-$C_8$]$_2$, $(CH_2)_n$-CR21R22-COOH, $(CH_2)_n$-CO-R16, $(CH_2)_n$-CO-NH-C$(CH_3)_2$-CO-O[alkyle en $C_1$-C8], $(CH_2)_n$-CO-NH-C$(CH_3)_2$-$CONH_2$, $(CH_2)_n$-CO-NH-C$(CH_3)_2$-COOH, les radicaux alkyle, alcényle, alcynyle et cycloalkyle pouvant être substitués par des atomes de fluor, et le radical aryle ou hétéroaryle pouvant être substitué par un ou des substituants halogéno, CN, alkyle en $C_1$-$C_6$, cycloalkyle en $C_3$-$C_6$, O-(alkyle en $C_1$-$C_6$), $S(O)_m$-(alkyle en $C_1$-$C_6$), $SO_2$-$NH_2$, COOH, $CONH_2$, CO-O(alkyle en $C_1$-$C_6$), CO-(alkyle en $C_1$-$C_6$), et les radicaux alkyle pouvant être remplacés par des atomes de fluor ;

R12 est H, un radical alkyle en $C_1$-$C_8$, cycloalkyle en $C_3$-$C_8$, $(CH_2)_n$-aryle, $(CH_2)_n$-hétéroaryle, les radicaux alkyle ou cycloalkyle pouvant être substitués par des atomes de fluor, et le radical aryle ou hétéroaryle pouvant être substitué par un ou des substituants halogéno, CN, alkyle en $C_1$-$C_6$, O-(alkyle en $C_1$-$C_6$), $SO_2$-$NH_2$, COOH, $CONH_2$, CO-O(alkyle en $C_1$-$C_6$), CO-(alkyle en $C_1$-$C_6$), et les radicaux alkyle pouvant être substitués par des atomes de fluor ;

R13 est H, un radical $SO_2$-[alkyle en $C_1$-$C_8$], $SO_2$-[cycloalkyle en $C_3$-$C_8$], $SO_2$-$(CH_2)_n$-aryle, $SO_2$-$(CH_2)_n$-hétéroaryle,

où les radicaux alkyle et cycloalkyle pouvant être substitués par des atomes de fluor, et le radical aryle ou hétéroaryle pouvant être substitué par un ou des substituants halogéno, CN, $CF_3$, alkyle en $C_1$-$C_6$, cycloalkyle en $C_3$-$C_6$, O-[alkyle en $C_1$-$C_6$], $S(O)_m$-[alkyle en $C_1$-$C_6$], $SO_2$-$NH_2$, COOH, $CONH_2$, CO-[O(alkyle en $C_1$-$C_6$)], CO-(alkyle en $C_1$-$C_6$), et les radicaux alkyle pouvant être substitués par des atomes de fluor ;

R15 est un radical alkyle en $C_1$-$C_8$,

le radical alkyle pouvant être substitué par des atomes de fluor ;

R16 est un groupe aziridin-1-yle, azétidin-1-yle, 3-hydroxy-azétidin-1-yle, pipéridin-1-yle, pyrrolidin-1-yle, 3-pyrrolidinol-1-yle, morpholin-N-yle, pipérazin-1-yle, un radical 4-[alkyle en $C_1$-$C_6$]pipérazin-1-yle, thiomorpholin-4-yle, thiomorpholin-1,1-dioxyde-4-yle, NH-$(CH_2)_r$-OH, NH-CH$(CH_2OH)_2$, NH-C$(CH_2OH)_3$, N[(alkyle en $C_1$-$C_6$)-OH]$_2$, D-glucamin-N-yle, N-méthyl-D-glucamin-N-yle, NH-[alkyle en $C_1$-$C_8$]-CO-O(alkyle en $C_1$-$C_6$), NH-[alkyle en $C_1$-$C_8$]-COOH, NH-[alkyle en $C_1$-$C_8$]-$CONH_2$, N [alkyle en $C_1$-$C_{6]}$ [alkyle en $C_1$-$C_8$]-COOH, NH-[C(H)(aryle)]-CO-O(alkyle en $C_1$-$C_6$], NH-[C(H)(aryle)]-COOH, NH-[C(H)(aryle)]-$CONH_2$, NH-[C(H)(hétéroaryle)]-CO-0(alkyle en $C_1$-$C_6$), NH-[C(H)(hétéroaryle)]-COOH, NH-[C(H)(hétéroaryle)]-$CONH_2$, NH-[cycloalkyle en $C_3$-$C_8$]-CO-O(alkyle en $C_1$-$C_6$), NH-[cycloalkyle en $C_3$-$C_8$]-COOH, NH-[cycloalkyle en $C_3$-$C_8$]-$CONH_2$, NH-(alkyle en $C_1$-$C_6$)-OH, NH-[alkyle en $C_1$-$C_6$]-$SO_2$-(alkyle en $C_1$-$C_6$), NH-[alkyle en $C_1$-$C_6$]-$SO_3H$, NH-[alkyle en $C_1$-$C_6$]-$SO_2$-$NH_2$,

les fonctions alcool (OH) pouvant être remplacées par F, et le radical aryle ou hétéroaryle pouvant être substitué par un ou des substituants halogéno, CN, alkyle en $C_1$-$C_6$, O-(alkyle en $C_1$-$C_6$), OH, $SO_2$-$NH_2$, COOH, $CONH_2$, CO-O(alkyle en $C_1$-$C_6$), CO-(alkyle en $C_1$-$C_6$) ;

R18 est $(CH_2)_n$-CR25R26-CO-O (alkyle en $C_1$-$C_4$), $(CH_2)_n$-CR25R26-CO-$NH_2$, $(CH_2)_n$-CR25R26-COOH ;

R20 est H, un radical alkyle en $C_1$-$C_6$, cycloalkyle en $C_3$-$C_8$, aryle, [alkyle en $C_1$-$C_6$]-aryle ;

R21 est H, F, $CF_3$, un radical alkyle en $C_1$-$C_6$, cycloalkyle en $C_3$-$C_8$, OH, O-(alkyle en $C_1$-$C_6$), O-(cycloalkyle en $C_3$-$C_8$), O-$(CH_2)_n$-aryle, O-(CO)-(alkyle en $C_1$-$C_6$), O-(CO)-(cycloalkyle en $C_3$-$C_8$), O-(CO)-O-(alkyle en $C_1$-$C_6$), O-(CO)-O-(cycloalkyle en $C_3$-$C_8$), NH-[alkyle en $C_1$-$C_6$]-aryle, $NH_2$, NH-(alkyle en $C_1$-$C_6$), NH-(CO)-(alkyle en $C_1$-$C_6$) ;

R22 est H, $CF_3$, un radical alkyle en $C_1$-$C_6$, aryle, [alkyle en $C_1$-$C_6$]-aryle ;

R23, R24 représentent chacun indépendamment de l'autre H, un radical alkyle en $C_1$-$C_6$, cycloalkyle en $C_3$-$C_6$, [alkyle en $C_1$-$C_4$]-[cycloalkyle en $C_3$-$C_6$], aryle, [alkyle en $C_1$-$C_4$]-aryle,

ou R23 et R24 forment ensemble un motif -CH=CH-, -$CH_2$-$CH_2$-, -$CH_2$-$CH_2$-$CH_2$- ou -$CH_2$-$CH_2$-$CH_2$-$CH_2$-, où un groupement $CH_2$ peut être remplacé par C=O, CHF ou $CF_2$, et où jusqu'à quatre atomes d'hydrogène pouvant être remplacés par un radical alkyle en $C_1$-$C_4$ ;

R25, R26 représentent chacun indépendamment de l'autre H, F, un radical alkyle en $C_1$-$C_4$, aryle, [alkyle en $C_1$-$C_4$]-aryle, le radical aryle pouvant être substitué par un ou des substituants halogéno, CN, OH, O-(alkyle en $C_1$-$C_4$), ou les radicaux R25 et 26 forment, avec l'atome de carbone auquel ils sont liés, un radical carbocyclique à trois à sept chaînons, dans lequel un atome de carbone peut être remplacé par O, $S(O)_m$, NH, N-[alkyle en $C_1$-$C_4$] ou CO ;

à l'exception du composé N-[3-tert-butyl-1-(4-méthylphényl)-1H-pyrazol-5-yl]-N'(4-{[3-(2-méthylphényl)-2,4-dioxo-1-imidazolidinyl]méthyl]}-phényl)urée,

ainsi que leurs sels physiologiquement acceptables.

3. Composés de formule I selon la revendication 1 ou 2, **caractérisés en ce que**, dans cette formule :

R, R' représentent chacun indépendamment de l'autre H, un radical $(CH_2)_n$-aryle, alkyle en $C_1$-$C_6$, le radical alkyle en $C_1$-$C_6$ ou le radical aryle pouvant être substitué par un ou des substituants halogéno ;
ou R et R' forment ensemble un noyau ayant trois à huit atomes de carbone, un atome de carbone pouvant être remplacé par 0, $S(O)_m$, NR13 ou NR15 ;
m vaut 0, 1, 2 ;
n vaut 0, 1, 2 ;
p vaut 1, 2, 3 ;
q vaut 1, 2 ;
r vaut 2, 3, 4 ;
v vaut 0, 1, 2 ;
A, D, E, G, L représentent chacun indépendamment de l'autre C ou N, où, pour la signification N, le substituant correspondant R1, R2, R3, R4, R5 disparaît, ou R2-D=E-R3 ou R4-G=L-R5 représentent S ou 0, le noyau à cinq ou six chaînons pouvant être condensé à un radical $-(CH_2)_3$- ou -CH=CH-CH=CH-, pour donner un radical bicyclique ;
R1, R2, R3, R4, R5 représentent chacun indépendamment des autres H, F, Cl, Br, I, CN, $CF_3$, un radical alkyle en $C_1$-$C_8$, cycloalkyle en $C_3$-$C_8$, adamantan-1-yle, adamantan-2-yle, $(CH_2)_n$-aryle, $(CH_2)_n$-hétéroaryle, $OCF_3$, O-R11, NR13R15, $S(O)_m$-R12, $SO_2$-$NH_2$, $SO_2$-N=CH-$N(CH_3)_2$, $SO_2$-NH-[alkyle en $C_1$-$C_8$], $SO_2$-NH-[cycloalkyle en $C_3$-$C_8$], $SO_2$-NH-$(CH_2)_n$-aryle, $SO_2$-NH-$(CH_2)_n$-hétéroaryle, $SO_2$-N[alkyle en $C_1$-$C_8$]$_2$, $SO_2$-R16, $SF_5$, CO-O[alkyle en $C_1$-$C_8$)],
CO-O[cycloalkyle en $C_3$-$C_6$], CO-$NH_2$, CO-NH-[alkyle en $C_1$-$C_4$], CO-N[alkyle en $C_1$-$C_4$]$_2$, CO-NH-[cycloalkyle en $C_3$-$C_6$], C(=NH)-O-[alkyle en $C_1$-$C_6$], C(=NH)-$NH_2$, C(=NH)-R16, C(=NR13)-NR12R13, $(CH_2)_n$-C(=$NSO_2$-R12)$NH_2$, CO-R16, COOH,
CO-(alkyle en $C_1$-$C_4$), CO-(cycloalkyle en $C_3$-$C_6$), CO-aryle, CO-hétéroaryle, CH(OH)-aryle, CH(OH)-hétéroaryle, CHF-aryle, CHF-hétéroaryle, $CF_2$-aryle, $CF_2$-hétéroaryle, $CH_2$-OH, $CH_2$-CN, $CH_2$-O-R12, $CH_2$-O-$(CH_2)_q$-COOH,
les radicaux alkyle et cycloalkyle pouvant être substitués par des atomes de fluor, et les radicaux aryle ou hétéroaryle pouvant être substitués par un ou des radicaux halogéno, CN, alkyle en $C_1$-$C_4$, cycloalkyle en $C_3$-$C_6$, O-(alkyle en $C_1$-$C_4$), $OCF_3$, OH, O-$(CH_2)_n$-aryle, $(CH_2)_n$-aryle, $S(O)_m$- (alkyle en $C_1$-$C_4$) $SO_2$-$NH_2$, SH, NR12R13, NH-CO-[alkyle en $C_1$-$C_4$], NH-CO-$(CH_2)_n$-aryle, $(CH_2)_n$-COOH, $(CH_2)_n$-$CONH_2$, $(CH_2)_n$-CO-O-(alkyle en $C_1$-$C_4$), $(CH_2)_n$-CO-(alkyle en $C_1$-$C_4$), et les radicaux alkyle pouvant être substitués par des atomes de fluor ;
R6, R7, R8, R9, R10 représentent chacun indépendamment des autres un radical hétérocyclique T-bicyclique, T-aryle ou T-hétéroaryle, le radical hétérocyclique bicyclique ou le radical aryle ou hétéroaryle pouvant être condensé à un noyau carboné aromatique ou non aromatique à 5 ou 6 chaînons, dans lequel un ou plusieurs groupes CH ou $CH_2$ peuvent être remplacés par des atomes d'oxygène, et le noyau carboné aromatique ou non aromatique à 5 ou 6 chaînons peut être substitué par un ou des substituants F, =O ou -(alkyle en $C_1$-$C_6$), et le radical hétérocyclique bicyclique pouvant contenir 9 à 12 chaînons, et jusqu'à cinq groupes CH ou $CH_2$ pouvant, indépendamment les uns des autres, être remplacés par N, NR20, O, $S(O)_m$ ou C=O, et le radical aryle ou hétéroaryle ou le radical hétérocyclique bicyclique peut être non substitué, ou une ou plusieurs fois substitué par les substituants suivantes :

R11, F, Cl, Br, I, CN, $N_3$, NC, $NO_2$, $CF_3$, $(CH_2)_n$-OR1, $(CH_2)_n$-O-$(CH_2)_r$-OH, $(CH_2)_n$-O-CH$(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O(alkyle en $C_1$-$C_6$)]$_2$, $(CH_2)_n$-N[$(CH_2)_q$-COOH]$_2$, $(CH_2)_n$-N[$(CH_2)_q$-$CONH_2$]$_2$, $(CH_2)_n$-NR-R13, $(CH_2)_n$-$N(R13)_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-COR16, $(CH_2)_n$-NR12-CO-NR12R13, $(CH_2)_n$-NR12-CO-$N(R12)_2$, $(CH_2)_n$-NR12-CO-NHR11, $(CH_2)_n$-NH-C(=NH)-$NH_2$, $(CH_2)_n$-NH-C-(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$-CO-NH-[alkyle en $C_1$-$C_8$], $(CH_2)_n$-NH-$(CH_2)_n$-CO-NH-$(CH_2)_r$-OH, $(CH_2)_n$-NH-$(CH_2)_n$-CO-N[alkyle en $C_1$-$C_8$]$_2$, $(CH_2)_n$-NH-$(CH_2)_n$-CO-NH-[cycloalkyle en $C_3$-$C_8$)], $(CH_2)_n$-NH-$(CH_2)_n$-CO-N[cycloalkyle en $C_3$-$C_8$]$_2$, $(CH_2)_n$-NH-C$(CH_3)_2$-CO-O(alkyle en $C_1$-$C_8$), $(CH_2)_n$-NH-C$(CH_3)_2$-CO-O(cycloalkyl en $C_3$-$C_8$), $(CH_2)_n$-NH-C$(CH_3)_2$-CO-O-$(CH_2)_r$-$NH_2$, $(CH_2)_n$-NH-C$(CH_3)_2$-CO-O-$(CH_2)_n$-aryle, $(CH_2)_n$-NH-C$(CH_3)_2$-CO-O-$(CH_2)_n$-hétéroaryle, $(CH_2)_n$-NH-C$(CH_3)_2$-CO-$NH_2$, $(CH_2)_n$-NH-C $(CH_3)_2$-CO-NH- [alkyle en $C_1$-$C_8$], $(CH_2)_n$-NH-C $(CH_3)_2$-CO-NH- $(CH_2)_r$-OH, $(CH_2)_n$-NH-C$(CH_3)_2$-CO-N[alkyle en $C_1$-$C_8$]$_2$, $(CH_2)_n$-NH-$(CH_3)_2$-CO-NH-[cycloalkyle en $C_3$-$C_8$], $(CH_2)_n$-NH-C $(CH_3)_2$-CON[cycloalkyle en $C_3$-$C_8$]$_2$, $(CH_2)_n$-NH-C$(CH_3)_2$-COOH, $S(O)_m$-R12, $SO_2$-R16, $SO_2$-N=CH-$N(CH_3)_2$,

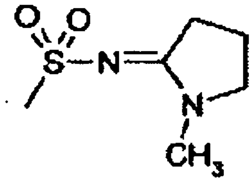

$SO_2$-NH-CO-R12 , $SO_2$-NHR12, $SO_2$-NH- $(CH_2)_r$-OH, $SO_2$-N[alkyle en $C_1$-$C_8$]$_2$, $SO_2$-NH-$(CH_2)_r$-$NH_2$, $SF_5$, COOH, CO-$NH_2$, $(CH_2)_q$-CN, $(CH_2)_n$-CO-NH-CN, $(CH_2)_n$-CO-NH-pipéridin-1-yle, $(CH_2)_n$-CO-NH-$SO_2$-NHR12, $(CH_2)_n$-CO-NH-$SO_2$-R18, $(CH_2)_n$-CHO, $(CH_2)_n$-C(=NH)$NH_2$, $(CH_2)_n$-C(=NH)-NHOH, $(CH_2)_n$-C(=NH)-[NH-O-(alkyle en $C_1$-$C_6$)], $(CH_2)_n$-C (=NH) (R16), $(CH_2)_n$-C (=NR13) NHR12, $(CH_2)_n$-C(=NR12)NR12R13, $(CH_2)_n$-C(=$NSO_2$-R12)$NH_2$, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O-(alkyle en $C_1$-$C_6$), $S(O)_m$-(alkyle en $C_1$-$C_6$), $SO_2$-$NH_2$, COOH, $CONH_2$, CO-O(alkyle en $C_1$-$C_6$), CO-(alkyle en $C_1$-$C_6$), et les radicaux alkyle pouvant être substitués par des atomes de fluor, et où, quand R3 est CN, $NO_2$ ou un groupe halogéno et R4 est $CF_3$ ou un groupe halogéno et R est égal à R' et est le groupe méthyle, alors le radical X-aryle est pourvu d'au moins l'un des substituants mentionnés ci-dessus, différents de l'hydrogène ;

H, F, Cl, Br, I, CN, $N_3$, NC, $NO_2$, $CF_3$,
alkyle en $C_1$-$C_8$, alcényle en $C_2$-$C_{10}$, alcynyle en $C_2$-$C_{10}$, cycloalkyle en $C_3$-$C_8$,
aryle, hétéroaryle,
$(CH_2)_n$-CO-[O-(alkyle en $C_1$-$C_8$)], $(CH_2)_n$, -CO-[O-(cycloalkyle en $C_3$-$C_8$)], $(CH_2)_n$-CO-[alkyle en $C_1$-$C_8$], $(CH_2)_n$)-CO-[cycloalkyle en $C_3$-$C_8$],
$(CH_2)_n$-CO-[O-$(CH_2)_v$-aryle],
$(CH_2)_n$-CO-$NH_2$, $(CH_2)_n$-COOH, $(CH_2)_n$-CO-NH-CN,
$(CH_2)_n$-P(O)(OH)[O-(alkyle en $C_1$-$C_6$)], $(CH_2)_n$-P(O)[O-(alkyle en $C_1$-$C_6$)]$_2$, $(CH_2)_n$-P(O) (OH) (O-$CH_2$-aryle), $(CH_2)_n$-P(O)(O-$CH_2$-aryle)$_2$, $(CH_2)_n$-P(O)$(OH)_2$, $(CH_2)$ $_n$-$SO_3H$, $(CH_2)$ $_n$-$SO_2$-NH2,
$(CH_2)_n$-CO-NH-[alkyle en $C_1$-$C_8$], $(CH_2)_n$-CO-N[alkyle en $C_1$-$C_8$]$_2$, $(CH_2)_n$-CO-NH-[cycloalkyle en $C_3$-$C_8$], (alcényle en $C_2$-$C_{10}$)-CO-O[alkyle en $C_1$-$C_6$], (alcényle en $C_2$-$C_{10}$)-$CONH_2$, (alcényle en $C_2$-$C_{10}$)-COOH, (alcynyle en $C_2$-$C_{10}$)-CO-O[alkyle en $C_1$-$C_6$], (alcynyle en $C_2$-$C_{10}$)-$CONH_2$, (alcynyle en $C_2$-$C_{10}$)-COOH,
$(CH_2)_n$-CO-R16,
$(CH_2)_n$-OH, $(CH_2)_n$-O-(alkyle en $C_1$-$C_8$), $(CH_2)_n$-O-(alcényle en $C_2$-$C_{10}$), $(CH_2)_n$-O- (alcynyle en $C_2$-$C_{10}$), $(CH_2)_n$-O-(cycloalkyle en $C_3$-$C_8$), $(CH_2)_n$-O-$(CH_2)_q$-[cycloalkyle en $C_3$-$C_8$], $(CH_2)_n$-O-$(CH_2)_n$-CO-[O-(alkyle, en $C_1$-$C_8$)], $(CH_2)_n$-O-$(CH_2)_n$-CO-[O-(cycloalkyle en $C_3$-$C_8$)], $(CH_2)_n$-O- $(CH_2)$ $_n$-CO-[alkyle en $C_1$-$C_8$], $(CH_2)_n$-O-$(CH_2)_n$-CO-[cycloalkyle en $C_3$-$C_8$], $(CH_2)_n$-O- $(CH_2)_n$-CO-[O-$(CH_2)_v$-aryle], $(CH_2)_n$-O- $(CH_2)_n$-CO-[O-$(CH_2)_v$-hétéroaryle], $(CH_2)_n$-O-$(CH_2)_q$-CO-$NH_2$, $(CH_2)_n$-O- $(CH_2)_q$-COOH, $(CH_2)_n$-O-$(CH_2)_q$-CO-NH-CN, $(CH_2)_n$-O-$(CH_2)_n$-P(O)(OH)[O-(alkyle en $C_1$-$C_6$)], $(CH_2)_n$-O-$(CH_2)_n$-P(O)[O-(alkyle en $C_1$-$C_6$)]$_2$, $(CH_2)_n$-O-$(CH_2)_n$-P(O)(OH)(O-$CH_2$-aryle), $(CH_2)_n$-O-$(CH_2)_n$-P(O)(O-$CH_2$-aryle)$_2$, $(CH_2)_n$-O-$(CH_2)_n$P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$-O-$(CH_2)_n$-CO-NH-[alkyle en $C_1$-$C_8$], $(CH_2)_n$-O-$(CH_2)_n$-CO-N[alkyle en $C_1$-$C_8$]$_2$, $(CH_2)_n$-O-$(CH_2)_n$-CO-NH-[cycloalkyle en $C_3$-$C_8$], $(CH_2)_n$-O-$(CH_2)_n$-CR21R22-CO-O[alkyle en $C_1$-$C_6$], $(CH_2)_n$-O-$(CH_2)_n$-CR21R22-$CONH_2$, $(CH_2)_n$-O-$(CH_2)_n$-CR21R22-COOH, $(CH_2)_n$-O-$(CH_2)_n$-CO-R16, $(CH_2)_n$-O-$(CH_2)$ $_r$-OH, $(CH_2)_n$-O-CH$(CH_2OH)_2$, $(CH_2)$n-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$, $(CH_2)_n$-$NH_2$, $(CH_2)_n$-NH- (alkyle en $C_1$-$C_8$), $(CH_2)_n$-NH-(cycloalkyle en $C_3$-$C_8$), $(CH_2)_n$-NH- $(CH_2)_n$-CO- [O-(cycloalkyle en $C_3$-$C_8$)], $(CH_2)_n$-NH-$(CH_2)_n$-CO-[alkyle en $C_1$-$C_8$], $(CH_2)_n$-NH-$(CH_2)_n$-CO-[cycloalkyle en $C_3$-$C_8$], $(CH_2)_n$-NH-$(CH_2)_n$-CO-[O-$(CH_2)_v$-aryle], $(CH_2)_n$-NH- $(CH_2)$ $_n$-CO-[O-$(CH_2)_v$-hétéroaryle], $(CH_2)_n$-NH-$(CH_2)_q$-CO-NH-CN,
$(CH_2)_n$-NH-$(CH_2)_n$-P(O)$(OH)_2$,
$(CH_2)_n$-NH-$(CH_2)_n$-$SO_3H$,
$(CH_2)_n$-NH-$(CH_2)_n$-$SO_2$-$NH_2$,
$(CH_2)_n$-NH-$(CH_2)_n$-CR21R22-CO-O[alkyle en $C_1$-$C_6$], $(CH_2)_n$-NH-$(CH_2)_n$-CR21R22-$CONH_2$, $(CH_2)$n-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[alkyle en $C_1$-$C_8$], $(CH_2)$ $_n$-NH- $(CH_2)$ $_n$ $SO_2$ [cycloalkyle en $C_3$-$C_8$], $(CH_2)_n$-NH-$SO_2$-$(CH_2)$ $_n$-NH2, $(CH_2)_n$-NH-$SO_2$-$(CH_2)_n$-NH-(alkyle en $C_1$-$C_8$), $(CH_2)$ $_n$-NH-$SO_2$-$(CH_2)_n$-NH-(cycloalkyle en $C_3$-$C_8$), $(CH_2)_n$-NH-$SO_2$-$(CH_2)_n$-N[alkyle en $C_1$-$C_8$]$_2$,

$(CH_2)_n$-NH-CN, $(CH_2)_n$-NH-$SO_2$-R16, $(CH_2)_n$-NR12-CO-NH- (alkyle en $C_1$-$C_8$), $(CH_2)_n$-NR12-CO-NH-(cycloalkyle en $C_3$-$C_8$), $(CH_2)_n$-NR12-CO-$NH_2$, $(CH_2)_n$-NR12-CO-NH-$SO_2$-(alkyle en $C_1$-$C_8$), $(CH_2)_n$-NR12-CO-NH-$SO_2$-(cycloalkyle en $C_3$-$C_8$), $(CH_2)_n$-NR12-CO-N[alkyle en $C_1$-$C_8$]$_2$, $(CH_2)_n$-NH-CO-NH-$(CH_2)_n$-CO-[O-(alkyle en $C_1$-$C_8$)], $(CH_2)_n$-NH-CO-NH-$(CH_2)_q$-CO-$NH_2$, $(CH_2)_n$-NH-CO-NH-$(CH_2)_q$-COOH, $(CH_2)_n$-NH-C(=NH)-$NH_2$, $(CH_2)_n$-NH-C(=NH)-R16, $(CH_2)_n$-NH-C (=NH) -NH [alkyle en $C_1$-$C_8$], $(CH_2)_n$-NH-C (=N-$SO_2$-(alkyle en $C_1$-$C_8$))-$NH_2$, $(CH_2)_n$-NH-C (=N-$SO_2$-(alkyle en $C_1$-$C_8$))-NH[alkyle en $C_1$-$C_8$], $(CH_2)_n$-NH-C (=N-$SO_2$-$NH_2$)-$NH_2$, $(CH_2)_n$-NH-C (=N-$SO_2$-$NH_2$) -NH [alkyle en $C_1$-$C_8$], $(CH_2)_n$-NH-C (=NH)-N[alkyle en $C_1$-$C_8$]$_2$, $(CH_2)_n$-NH-C(=N-$SO_2$)-(alkyle en $C_1$-$C_8$)-N[alkyle en $C_1$-$C_8$]$_2$, $(CH_2)_n$-NH- $(CH_2)_n$-CO-O- $(CH_2)_r$-$NH_2$, $(CH_2)_n$-NH- $(CH_2)_n$-CO-NH- [alkyle en $C_1$-$C_8$], $(CH_2)_n$-NH-$(CH_2)_n$-CO-NH-$(CH_2)_r$-OH, $(CH_2)_n$-NH-$(CH_2)_n$-CO-N[alkyle en $C_1$-$C_8$]2, $(CH_2)_n$-NH-$(CH_2)_n$-CO-NH-[cycloalkyle en $C_3$-$C_8$], $(CH_2)_n$-NH-$C(CH_3)_2$-CO-O(cycloalkyle en $C_3$-$C_8$), $(CH_2)_n$-NH-$C(CH_3)_2$-CO-O-$(CH_2)_r$-$NH_2$, $(CH_2)_n$-NH-C $(CH_3)_2$-CO-O-$(CH_2)_n$-aryle, $(CH_2)_n$-NH-$C(CH_3)_2$-CO-O-$(CH_2)_n$-hétéroaryle, $(CH_2)_n$-NH-$C(CH_3)_2$-CO-NH-[alkyle en $C_1$-$C_8$], $(CH_2)_n$-NH-$C(CH_3)_2$-CO-NH-$(CH_2)_r$-OH, $(CH_2)_n$-NH-$C(CH_3)_2$-CO-N[alkyle en $C_1$-$C_8$]$_2$, $(CH_2)_n$-NH-$(CH_3)_2$-CO-NH-[cycloalkyle en $C_3$-$C_8$], $(CH_2)_n$-NH-C $(CH_3)_2$-CON[cycloalkyle en $C_3$-$C_8$]$_2$, $(CH_2)_n$-S(O)$_m$-(alkyle en $C_1$-$C_8$), $(CH_2)_n$-S(O)$_m$-(cycloalkyle en $C_3$-$C_8$), $(CH_2)_n$-$SO_2$-

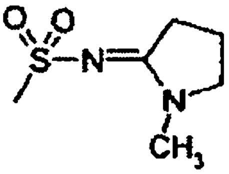

R16, $SO_2$-N=CH-N$(CH_3)_2$, $(CH_2)_n$-$SO_2$-NH-CO-(alkyle en $C_1$-$C_8$), $(CH_2)_n$-$SO_2$-NH-CO-(cycloalkyle en $C_3$-$C_8$), $(CH_2)_n$-$SO_2$-NH-(alkyle en $C_1$-$C_8$), $(CH_2)_n$-$SO_2$-NH-(cycloalkyle en $C_3$-$C_8$), $(CH_2)_n$-$SO_2$-N[alkyle en $C_1$-$C_8$]$_2$, $SO_2$-NH-$(CH_2)_r$-OH, $SO_2$-NH-$(CH_2)_r$-$NH_2$, $SF_5$,
$(CH_2)_q$-CN,
$(CH_2)_n$-CO-NH-pipéridin-1-yle,
$(CH_2)_n$-CO-NH-$SO_2$-NHR12, $(CH_2)_n$-CO-NH-$SO_2$-(alkyle en $C_1$-$C_8$), $(CH_2)_n$-CO-NH-$SO_2$-(cycloalkyle en $C_3$-$C_8$),
$(CH_2)_n$-CHO, $(CH_2)_n$-C (=NH) $NH_2$, $(CH_2)_n$-C (=NH) NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O-(alkyle en $C_1$-$C_6$), S(O)$_m$-(alkyle en $C_1$-$C_6$), $SO_2$-$NH_2$, COOH, $CONH_2$, CO-[O(alkyle en $C_1$-$C_6$)], CO-(alkyle en $C_1$-$C_6$), et les radicaux alkyle pouvant être substitués par des atomes de fluor,
où toujours au moins l'un des radicaux R6, R7, R8, R9 et R10 représente un radical hétérocyclique T-bicyclique, T-aryle ou T-hétéroaryle, et
l'une des quatre paires de radicaux R6 et R7, ou R7 et R8, ou R8 et R9, ou R9 et R10, peut chacune former ensemble les groupes -$CH_2$-$CH_2$-$CH_2$- ou -$CH_2$-$CH_2$-$CH_2$-$CH_2$-, jusqu'à deux groupes -$CH_2$- pouvant être remplacés par -O-, et les groupes -$CH_2$-$CH_2$-$CH_2$- ou -$CH_2$-$CH_2$-$CH_2$-$CH_2$- pouvant être substitués par un ou des substituants F, alkyle en $C_1$-$C_8$ ou =O ;
T est
**NR23-CO-NR24, NR23-$SO_2$-NR24, $SO_2$-NR23-$SO_2$, CO-NR23-CO, NR23-C(=NR13)-NR24, NR23-C(=NR22)-NR24, CO-NR23-CR22R23, NR23-CO-CR22R24, NR23-$SO_2$-CR22R24, CR22R24-CO-NR23, CR22R24-$SO_2$-NR23, CR22R23-NR23-$SO_2$, $SO_2$-CR22R23-NR23, $SO_2$-NR23-CR22R23-, NR23-CR22R23-$SO_2$, CR23R24-CR23R24-CR23R24, CR23R24-NR23-CR23R24;**
R11 est H, un radical alkyle en $C_1$-$C_8$, alcynyle en $C_2$-$C_{10}$, cycloalkyle en $C_3$-$C_6$, $(CH_2)_n$-aryle, (CH2)n-CO-[O-(alkyle en $C_1$-$C_6$)], $(CH_2)_n$-CO-[O-(cycloalkyle en $C_3$-$C_6$)], $(CH_2)_n$-CO-[alkyle en $C_1$-$C_6$], $(CH_2)_n$-CO-[cycloalkyle en $C_3$-$C_6$], $(CH_2)_n$-CO-aryle, $(CH_2)_n$-CO-hétéroaryle, $(CH_2)_q$-CO-$NH_2$, $(CH_2)_q$-COOH, $(CH_2)_n$-P(O) (OH) [O- (alkyle en $C_1$-$C_6$)], $(CH_2)_n$-P(O) [O-(alkyle en $C_1$-$C_4$)]$_2$, $(CH_2)_n$-P(O) (O-$CH_2$-aryle)2, $(CH_2)_n$-P(O) $(OH)_2$, $(CH_2)_n$-$SO_3H$, $(CH_2)_n$-$SO_2$-$NH_2$, $(CH_2)_n$-CO-NH-[alkyle en $C_1$-$C_4$], $(CH_2)_n$-CO-N [alkyle en $C_1$-$C_4$]$_2$, $(CH_2)_n$-CO-NH-[cycloalkyle en $C_3$-$C_6$], (alcényle en $C_2$-$C_6$)-CO-O [alkyle en $C_1$-$C_4$], (alcényle en $C_2$-$C_6$)-$CONH_2$, (alcényle en $C_2$-$C_6$)-COOH, (alcynyle en $C_2$-$C_6$)-CO-O[alkyle en $C_1$-$C_4$], (alcynyle en $C_2$-$C_6$)-$CONH_2$, (alcynyle en $C_2$-$C_6$)-COOH, $(CH_2)_n$-CR21[CO-O (alkyle en $C_1$-$C_4$)]$_2$, $(CH_2)_n$-CR21$(CONH_2)_2$, $(CH_2)_n$-CR21 $(COOH)_2$, $(CH_2)_n$-CR21R22-CO-O[alkyle en $C_1$-$C_4$], $(CH_2)_n$-CR21R22-$CONH_2$, $(CH_2)_n$-CR21R22-CO-NH-[alkyle en $C_1$-$C_4$], $(CH_2)_n$-CR21R22-CO-N [alkyle en $C_1$-$C_4$]$_2$, $(CH_2)_n$-CR21R22-COOH,

$(CH_2)_n$-CO-R16, $(CH_2)_n$-CO-NH-C $(CH_3)_2$-CO-O[alkyle en $C_1$-$C_8$], $(CH_2)_n$-CO-NH-C$(CH_3)_2$-$CONH_2$, $(CH_2)_n$-CO-NH-C$(CH_3)_2$-COOH, les radicaux alkyle, alcényle, alcynyle et cycloalkyle pouvant être substitués par des atomes de fluor, et le radical aryle ou hétéroaryle pouvant être substitué par un ou des substituants halogéno, CN, alkyle en $C_1$-$C_4$, O-(alkyle en $C_1$-$C_4$), $S(O)_m$-(alkyle en $C_1$-$C_4$), $SO_2$-$NH_2$, COOH, $CONH_2$, CO-O(alkyle en $C_1$-$C_4$), et les radicaux alkyle pouvant être substitués par des atomes de fluor ;

R12 est H, un radical alkyle en $C_1$-$C_8$, cycloalkyle en $C_3$-$C_8$, $(CH_2)_n$-aryle, $(CH_2)_n$-hétéroaryle, les radicaux alkyle ou cycloalkyle pouvant être substitués par des atomes de fluor, et le radical aryle ou hétéroaryle pouvant être substitué par un ou des substituants halogéno, CN, alkyle en $C_1$-$C_4$, O-(alkyle en $C_1$-$C_4$), $SO_2$-$NH_2$, COOH, $CONH_2$, CO-O(alkyle en $C_1$-$C_4$), et les radicaux alkyle pouvant être substitués par des atomes de fluor ;

R13 est H, $SO_2$-[alkyle en $C_1$-$C_8$], $SO_2$-[cycloalkyle en $C_3$-$C_8$], $SO_2$-$(CH_2)_n$-aryle, $SO_2$-$(CH_2)_n$-hétéroaryle, les radicaux alkyle et cycloalkyle pouvant être substitués par des atomes de fluor, et le radical aryle ou hétéroaryle pouvant être substitué par un ou des substituants halogéno, CN, $CF_3$, alkyle en $C_1$-$C_4$, 0-[alkyle en $C_1$-$C_4$], $S(O)_m$-[alkyle en $C_1$-$C_6$], $SO_2$-$NH_2$, COOH, $CONH_2$, CO-[O(alkyle en $C_1$-$C_4$)], et les radicaux alkyle pouvant être substitués par des atomes de fluor ;

R15 est un radical alkyle en $C_1$-$C_6$,

le radical alkyle pouvant être substitué par des atomes de fluor ;

R16 est un radical aziridin-1-yle, azétidin-1-yle, 3-hydroxy-azétidin-1-yle, pipéridin-1-yle, pyrrolidin-1-yle, 3-pyrrolidinol-1-yle, morpholin-N-yle, pipérazin-1-yle, 4-[alkyle en $C_1$-$C_6$]pipérazin-1-yle, thiomorpholin-1,1-dioxyde-4-yle, NH-$(CH_2)_r$-OH, NH-CH$(CH_2OH)_2$, NH-C$(CH_2OH)_3$, N-[(alkyle en $C_1$-$C_4$)-OH]$_2$, D-glucamin-N-yle, N-méthyl-D-glucamin-N-yle, NH-[alkyle en $C_1$-$C_4$]-COOH, NH-[alkyle en $C_1$-$C_4$]-$CONH_2$, N [alkyle en $C_1$-$C_4$] [alkyle en $C_1$-$C_4$] -COOH, NH-[C(H)(aryle)]-CO-O(alkyle en $C_1$-$C_4$), NH-[C(H)(aryle)]-COOH, NH-[C(H)(aryle)]-$CONH_2$, NH-[C(H)(hétéroaryle)]-CO-O(alkyle en $C_1$-$C_4$), NH-[C(H)(hétéroaryle)]-COOH, NH-[C(H)(hétéroaryle)]-$CONH_2$, NH-[cycloalkyle en $C_3$-$C_6$]-CO-O(alkyle en $C_1$-$C_4$), NH-[cycloalkyle en $C_3$-$C_6$]-COOH, NH-[cycloalkyle en $C_3$-$C_6$]-$CONH_2$, NH-(alkyle en $C_1$-$C_4$)-OH, NH-[alkyle en $C_1$-$C_4$]-$SO_2$-(alkyle en $C_1$-$C_4$), NH-[alkyle en $C_1$-$C_4$]-$SO_3H$, NH-[alkyle en $C_1$-$C_4$]-$SO_2$-$NH_2$,

les fonctions alcool (OH) pouvant être remplacées par F, et le radical aryle ou hétéroaryle pouvant être substitué par un ou des substituants halogéno, CN, alkyle en $C_1$-$C_4$, O-(alkyle en $C_1$-$C_4$), OH, $SO_2$-$NH_2$, COOH, $CONH_2$, CO-O(alkyle en $C_1$-$C_4$) ;

R18 est un radical $(CH_2)_n$-CR25R26-CO-O(alkyle en $C_1$-$C_4$), $(CH_2)_n$-CR25R26-CO-$NH_2$, $(CH_2)_n$-CR25R26-COOH ;

R20 est H, un radical alkyle en $C_1$-$C_4$, cycloalkyle en $C_3$-$C_6$, aryle, [alkyle en $C_1$-$C_4$]-aryle ;

R21 est H, F, $CF_3$, un radical alkyle en $C_1$-$C_4$, cycloalkyle en $C_3$-$C_6$, OH, O-(alkyle en $C_1$-$C_4$), O-(cycloalkyle en $C_3$-$C_6$), O-$(CH_2)_n$-aryle, O-(CO)-(alkyle en $C_1$-$C_4$), O-(CO)-(cycloalkyle en $C_3$-$C_6$), O-(CO)-O-(alkyle en $C_1$-$C_4$), O-(CO)-O-(cycloalkyle en $C_3$-$C_6$), NH-[alkyle en $C_1$-$C_4$]-aryle, $NH_2$, NH- (alkyle en $C_1$-$C_4$), NH-(CO)-(alkyle en $C_1$-$C_4$) ;

R22 est H, $CF_3$, un radical alkyle en $C_1$-$C_4$, aryle, [alkyle en $C_1$-$C_4$]-aryle ;

R23, R24 représentent chacun indépendamment de l'autre H, un radical alkyle en $C_1$-$C_4$, cycloalkyle en $C_3$-$C_6$, [alkyle en $C_1$-$C_4$]-[cycloalkyle en $C_3$-$C_6$], aryle, [alkyle en $C_1$-$C_4$]-aryle,

ou R23 et R24 forment ensemble un motif -CH=CH-, -$CH_2$-$CH_2$-, -$CH_2$-$CH_2$-$CH_2$- ou -$CH_2$-$CH_2$-$CH_2$-$CH_2$-, où un groupement $CH_2$ peut être remplacé par C=O, CHF ou $CF_2$, et jusqu'à quatre atomes d'hydrogène pouvant être remplacés par un radical alkyle en $C_1$-$C_4$ ;

R25, R26 représentent chacun indépendamment de l'autre H, F, un radical alkyle en $C_1$-$C_4$, aryle, [alkyle en $C_1$-$C_4$]-aryle, le radical aryle pouvant être substitué par un ou des substituants halogéno, CN, OH, O-(alkyle en $C_1$-$C_4$), ou les radicaux R25 et 26 forment, ensemble avec l'atome de carbone auquel ils sont liés, un radical carbocyclique à trois à sept chaînons, dans lequel un atome de carbone peut être remplacé par O, $S(O)_m$, NH, N-[alkyle en $C_1$-$C_4$] ou CO ;

à l'exception du composé N-[3-tert-butyl-1-(4-méthylphényl)-1H-pyrazol-5-yl]-N'(4-{[3-(2-méthylphényl)-2,4-dioxo-1-imidazolidinyl]méthyl]}-phényl)urée,

ainsi que leurs sels physiologiquement tolérés.

**4.** Composés de formule I, selon l'une ou plusieurs des revendications 1 à 3, **caractérisés en ce que**, dans cette formule :

R, R' représentent chacun indépendamment de l'autre H, un radical aryle, alkyle en $C_1$-$C_4$, le radical alkyle en $C_1$-$C_4$ ou le radical aryle pouvant être substitué par un ou des substituants halogéno ;

ou R et R' forment ensemble un noyau ayant trois à huit atomes de carbone, un atome de carbone pouvant être remplacé par O, $S(O)_m$, NR13 ou NR15 ;

m vaut 0, 1, 2 ;

n vaut 0, 1, 2 ;
p vaut 1, 2, 3 ;
q vaut 1, 2 ;
r vaut 2, 3 ;
v vaut 0, 1, 2 ;
A, D, E, G, L représentent chacun indépendamment des autres C ou N, où, pour la signification N, le substituant correspondant R1, R2, R3, R4, R5 disparaît,
ou R2-D=E-R3 ou R4-G=L-R5 représentent S ou O, et le noyau à cinq ou six chaînons peut être condensé à un radical $-(CH_2)_3-$ ou $-(CH_2)_4-$ ou -CH=CH-CH=CH-, pour donner un radical bicyclique ;
R1, R2, R3, R4, R5 représentent chacun indépendamment des autres H, F, Cl, Br, I, CN, $CF_3$, un radical alkyle en $C_1$-$C_8$, cycloalkyle en $C_3$-$C_8$, $(CH_2)_n$-aryle, $(CH_2)_n$-hétéroaryle, $OCF_3$, O-R11, NR13R15, $S(O)_m$-R12, $SO_2$-$NH_2$, $SO_2$-NH-[alkyle en $C_1$-$C_8$], $SO_2$-NH-[cycloalkyle en $C_3$-$C_8$], $SO_2$-NH-$(CH_2)_n$-aryle, $SO_2$-NH-$(CH_2)_n$-hétéroaryle, $SO_2$-N[alkyle en $C_1$-$C_8$]$_2$, $SO_2$-R16, $SF_5$, CO-O[alkyle en $C_1$-$C_8$], CO-O[cycloalkyle en $C_3$-$C_6$], CO-$NH_2$, CO-NH-[alkyle en $C_1$-$C_4$], CO-N[alkyle en $C_1$-$C_4$]$_2$, C(=NH)-$NH_2$, C(=NH)-R16, $(CH_2)_n$-C(=$NSO_2$-R12)$NH_2$, CO-R16, COOH, CO-(alkyle en $C_1$-$C_4$), CO-(cycloalkyle en $C_3$-$C_6$), CO-aryle, CO-hétéroaryle, CH(OH)-aryle, CH(OH)-hétéroaryle, CHF-aryle, CHF-hétéroaryle, $CF_2$-aryle, $CF_2$-hétéroaryle, $CH_2$-OH, $CH_2$-CN, $CH_2$-O-R12, $CH_2$-O-$(CH_2)_q$-COOH, les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_4$, O-alkyle en $C_1$-$C_4$, $OCF_3$, OH, O-$(CH_2)_n$-aryle, $(CH_2)_n$-aryle, $S(O)_m$-(alkyle en $C_1$-$C_4$) $SO_2$-$NH_2$, SH, NR12R13, NH-CO-[alkyle en $C_1$-$C_4$], NH-CO-$(CH_2)_n$-aryle, $(CH_2)_n$-COOH, $(CH_2)_n$-CONH2, $(CH_2)_n$-CO-O-(alkyle en $C_1$-$C_4$), $(CH_2)_n$-CO-(alkyle en $C_1$-$C_4$), et les radicaux alkyle pouvant être substitués par des atomes de fluor ;
R6, R7, R8, R9, R10 représentent chacun indépendamment des autres un radical hétérocyclique T-bicyclique, T-aryle ou T-hétéroaryle, le radical hétérocyclique bicyclique ou le radical aryle ou hétéroaryle pouvant être condensé à un noyau carboné aromatique ou non aromatique à 5 ou 6 chaînons, où un ou plusieurs groupes CH ou $CH_2$ peuvent être remplacés par des atomes d'oxygène, et le noyau carboné aromatique ou non aromatique à 5 ou 6 chaînons pouvant être substitué par un ou des substituants F, =O ou -(alkyle en $C_1$-$C_6$), et le radical hétérocyclique bicyclique pouvant contenir 9 à 12 chaînons, et jusqu'à cinq groupes CH- ou $CH_2$- peuvent, indépendamment les uns des autres, être remplacés par N, NR20, O, $S(O)_m$ ou C=O, et le radical aryle ou hétéroaryle ou le radical hétérocyclique bicyclique peut être non substitué, ou une ou plusieurs fois substitué par les substituants suivants :

R11, F, Cl, Br, I, CN, $N_3$, NC, $NO_2$, $CF_3$, $(CH_2)_n$-OR11, $(CH_2)_n$-O-$(CH_2)_r$-OH, $(CH_2)_n$-O-CH $(CH_2OH)_2$, $(CH_2)_n$-O-$(CH_2)_n$-CO-O-$(CH_2)_r$-$NH_2$, $(CHF_2)_n$-O-$(CH_2)_n$-CO-NH-$(CH_2)_r$-OH, O-R13, $OCF_3$, $(CH_2)_n$-O-$(CH_2)_r$-$NH_2$, $(CH_2)_n$-NH-R11, $(CH_2)_n$-N[$(CH_2)_q$-CO-O (alkyle en $C_1$-$C_6$)]$_2$, $(CH_2)_n$-N [$(CH_2)_q$-COOH]$_2$, $(CH_2)_n$-N[$(CH_2)_q$-$CONH_2$]$_2$, $(CH_2)_n$-NR-R13, $(CH_2)_n$-N$(R13)_2$, $(CH_2)_n$-NH-CN, $(CH_2)_n$-NH-$SO_2$-R16, $(CH_2)_n$-NH-$(CH_2)_n$-$SO_2$-R12, $(CH_2)_n$-NR12-COR16, $(CH_2)_n$-NR12-CO-NR12R13, $(CH_2)_n$-NR12-CO-N$(R12)_2$, $(CH_2)_n$-NR12-CO-NHR11, $(CH_2)_n$-NH-C (=NH)-$NH_2$, $(CH_2)_n$-NH-C-(=NH)-R16, $(CH_2)_n$-NH-C(=NH)-NHR12, $(CH_2)_n$-NR12-C(=NR13)-NHR12, $(CH_2)_n$-NR12-C(=NR12)-NR12R13, $(CH_2)_n$-NH-$(CH_2)_n$-CO-O-$(CH_2)_r$-$NH_2$, $(CH_2)_n$-NH-$(CH_2)_n$-CO-NH-[alkyle en $C_1$-$C_8$], $(CH_2)_n$-NH-$(CH_2)_n$-CO-NH-$(CH_2)_r$-OH, $(CH_2)_n$-NH-$(CH_2)_n$-CO-N [alkyle en $C_1$-$C_8$]$_2$, $(CH_2)_n$-NH-$(CH_2)_n$-CO-NH-[cycloalkyle en $C_3$-$C_8$], $(CH_2)_n$-NH-$(CH_2)_n$-CO-N[cycloalkyle en $C_3$-$C_8$ ]$_2$, $(CH_2)_n$-NH-C$(CH_3)_2$-CO-O-(alkyle en $C_1$-$C_8$), $(CH_2)_n$-NH-C$(CH_3)_2$-CO-O(cycloalkyl en $C_3$-$C_8$), $(CH_2)_n$-NH-C $(CH_3)_2$-CO-O-$(CH_2)_r$-$NH_2$, $(CH_2)_n$-NH-C $(CH_3)_2$-CO-O-$(CH_2)_n$-aryle, $(CH_2)_n$-NH-C $(CH_3)$ $_2$-CO-O-$CH_2)$ $_n$-hétéroaryle, $(CH_2)_n$-NH-C $(CH_3)_2$-CO-$NH_2$, $(CH_2)_n$-NH-C $(CH_3)_2$-CO-NH-[alkyle en $C_1$-$C_8$], $(CH_2)_n$-NH-C $(CH_3)_2$-CO-NH-$(CH_2)_r$-OH, $(CH_2)_n$-NH-C $(CH_3)_2$-CO-N [alkyle en $C_1$-$C_8$]$_2$, $(CH_2)_n$-NH-$(CH_3)_2$-CO-NH-[cycloalkyle en $C_3$-$C_8$], $(CH_2)_n$-NH-C $(CH_3)_2$-CON [cycloalkyle en $C_3$-$C_8$]$_2$, $(CH_2)_n$-NH-C $(CH_3)$ $_2$-COOH, $S(O)_m$-R12, $SO_2$-R16, $SO_2$-N=CH-N$(CH_3)_2$,

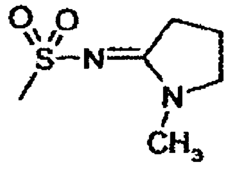

$SO_2$-NH-CO-R12, $SO_2$-NHR12, $SO_2$-NH-$(CH_2)_r$-OH, $SO_2$-N[alkyle en $C_1$-$C_8$]$_2$, $SO_2$-NH-$(CH_2)_r$-$NH_2$, $SF_5$, COOH, CO-$NH_2$, $(CH_2)_q$-CN, $(CH2)_n$-CO-NH-CN, $(CH_2)_n$-CO-NH-pipéridin-1-yle, $(CH_2)_n$-CO-NH-

$SO_2$-NHR12, $(CH_2)_n$-CO-NH-$SO_2$-R18, $(CH_2)_n$-CHO, $(CH_2)_n$-C(=NH)$NH_2$, $(CH_2)_n$-C(=NH)-NHOH, $(CH_2)_n$-C(=NH)-[NH-O-(alkyle en $C_1$-$C_6$)], $(CH_2)_n$-C(=NH)(R16), $(CH_2)_n$-C (=NR13) NHR12, $(CH_2)_n$-C(=NR12)NR12R13, $(CH_2)_n$-C(=$NSO_2$-R12)NH2, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O-(alkyle en $C_1$-$C_6$), $S(O)_m$-(alkyle en $C_1$-$C_6$), $SO_2$-$NH_2$, COOH, $CONH_2$, CO-O(alkyle en $C_1$-$C_6$), CO-(alkyle en $C_1$-$C_6$), et les radicaux alkyle pouvant être substitués par des atomes de fluor, et où, quand R3 est CN, $NO_2$ ou un groupe halogéno et R4 est $CF_3$ ou un groupe halogéno, et R est R' et est le groupe méthyle, alors le radical X-aryle est pourvu d'au moins l'un des substituants mentionnés ci-dessus, différents de l'hydrogène ;

H, F, Cl, Br, I, CN, $N_3$, NC, $NO_2$, $CF_3$,
alkyle en $C_1$-$C_8$, alcényle en $C_2$-$C_{10}$, alcynyle en $C_2$-$C_{10}$, cycloalkyle en $C_3$-$C_8$,
aryle, hétéroaryle,
$(CH_2)_n$-CO- [O- (alkyle en $C_1$-$C_8$)], $(CH_2)_n$-CO-[O-(cycloalkyle en $C_3$-$C_8$)], $(CH_2)_n$-CO-[alkyle en $C_1$-$C_8$], $(CH_2)_n$)-CO-[cycloalkyle en $C_3$-$C_8$],
$(CH_2)_n$-CO-[O-$(CH_2)_v$-aryle],
$(CH_2)_n$-CO-$NH_2$, $(CH_2)_n$-COOH, $(CH_2)_n$-CO-NH-CN,
$(CH_2)_n$-P(O) (OH) [O-(alkyle en $C_1$-$C_6$)], $(CH_2)_n$-P(O)[O-(alkyle en $C_1$-$C_6$)]$_2$, $(CH_2)_n$-P (O) (OH) (O-$CH_2$-aryle), $(CH_2)_n$-P(O)(O-$CH_2$-aryle)$_2$, $(CH_2)_n$-P (O) $(OH)_2$, $(CH_2)_n$-$SO_3H$, $(CH_2)_n$-$SO_2$-$NH_2$,
$(CH_2)_n$-CO-NH-[alkyle en $C_1$-$C_8$], $(CH_2)_n$-CO-N[alkyle en $C_1$-$C_8$]$_2$, $(CH_2)_n$-CO-NH-[cycloalkyle en $C_3$-$C_8$], (alcényle en $C_2$-$C_{10}$)-CO-O[alkyle en $C_1$-$C_6$], (alcényle en $C_2$-$C_{10}$)-$CONH_2$, (alcényle en $C_2$-$C_{10}$)-COOH, (alcynyle en $C_2$-$C_{10}$)-CO-O[alkyle en $C_1$-$C_6$], (alcynyle en $C_2$-$C_{10}$)-$CONH_2$, (alcynyle en $C_2$-$C_{10}$)-COOH,
$(CH_2)_n$-CO-R16,
$(CH_2)_n$-OH, $(CH_2)_n$-O-(alkyle en $C_1$-$C_8$), $(CH_2)_n$-O-(alcényle en $C_2$-$C_{10}$), $(CH_2)_n$-O-(alcynyle en $C_2$-$C_{10}$), $(CH_2)_n$-O-(cycloalkyle en $C_3$-$C_8$), $(CH_2)_n$-O-$(CH_2)_q$-[cycloalkyle en $C_3$-$C_8$], $(CH_2)_n$-O-$(CH_2)_n$-CO-[O-(alkyle en $C_1$-$C_8$)], $(CH_2)_n$-O-$(CH_2)_n$-CO-[O-(cycloalkyle en $C_3$-$C_8$)], $(CH_2)_n$-O-$(CH_2)_n$-CO-[alkyle en $C_1$-$C_8$], $(CH_2)_n$-O-$(CH_2)_n$-CO-[cycloalkyle en $C_3$-$C_8$], $(CH_2)_n$-O-$(CH_2)_n$-CO-[O-$(CH_2)_v$-aryle], $(CH_2)_n$-O-$(CH_2)_n$- CO-[O-$(CH_2)_v$-hétéroaryle], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P (O) (OH) [O- (alkyle en $C_1$-$C_6$)], $(CH_2)_n$-O-$(CH_2)_n$-P(O)[O-(alkyle en $C_1$-$C_6$)]$_2$, $(CH_2)_n$-O-$(CH_2)_n$-P(O) (OH) (O-$CH_2$-aryle), $(CH_2)_n$-O-$(CH_2)_n$-P(O)(O-$CH_2$-aryle)$_2$, $(CH_2)_n$-O-$(CH_2)_n$-P(O)$(OH)_2$, $(CH_2)_n$-O-$(CH_2)_n$-$SO_3H$, $(CH_2)_n$-O-$(CH_2)_n$-$SO_2$-$NH_2$, $(CH_2)_n$-O-$(CH_2)_n$-CO-NH-[alkyle en $C_1$-$C_8$], $(CH_2)_n$-O-$(CH_2)_n$-CO-N [alkyle en $C_1$-$C_8$]$_2$, $(CH_2)_n$-O-$(CH_2)_n$-CO-NH-[cycloalkyle en $C_3$-$C_8$], $(CH_2)_n$-O-$(CH_2)_n$-CR21R22-CO-O[alkyle en $C_1$-$C_6$], $(CH_2)_n$-O-$(CH_2)_n$-CR21R22-$CONH_2$, $(CH_2)_n$-O- $(CH_2)_n$-CR21R22-COOH, $(CH_2)_n$-O-$(CH_2)_n$-CO-R16, $(CH_2)_n$-O-$(CH_2)_r$-OH, $(CH_2)_n$-O-CH $(CH_2OH)_2$, $(CH_2)_n$-O-$(CH_2)_n$-CO-O-$(CH_2)_r$-$NH_2$, $(CH_2)_n$-O-$(CH_2)_n$-CO-NH-$CH_2)_r$-OH, O-R13, $OCF_3$, $(CH_2)$n-$NH_2$, $(CH_2)_n$-NH-(alkyle en $C_1$-$C_8$), $(CH_2)_n$-NH-(cycloalkyle en $C_3$-$C_8$), $(CH_2)_n$-NH-$(CH_2)_n$-CO-[O-(cycloalkyle en $C_3$-$C_8$)], $(CH_2)_n$-NH-$(CH_2)_n$-CO-[alkyle en $C_1$-$C_8$], $(CH_2)_n$-NH-$(CH_2)_n$-CO-[cycloalkyle en $C_3$-$C_8$], $(CH_2)_n$-NH-$(CH_2)_n$-CO-[O-$(CH_2)_v$-aryle], $(CH_2)_n$-NH-$(CH_2)_n$-CO-[O-$(CH_2)_v$-hétéroaryle], $(CH_2)_n$-NH-$(CH_2)_q$-CO-NH-CN,
$(CH_2)_n$-NH-$(CH_2)_n$-P (O) (OH) $_2$,
$(CH_2)_n$-NH-$(CH_2)_n$-$SO_3H$,
$(CH_2)_n$-NH-$(CH_2)_n$-$SO_2$-$NH_2$, $(CH_2)_n$-NH-$(CH_2)_n$-CR21R22-CO-O[alkyle en $C_1$-$C_6$], $(CH_2)_n$-NH-$(CH_2)_n$-CR21R22-$CONH_2$, $(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[alkyle en $C_1$-$C_8$], $(CH_2)_n$-NH-$(CH_2)_n$-$SO_2$-[cycloalkyle en $C_3$-$C_8$], $(CH_2)_n$-NH-$SO_2$-$(CH_2)_n$-$NH_2$, $(CH_2)_n$-NH-$SO_2$-$(CH_2)_n$-NH-(alkyle en $C_1$-$C_8$), $(CH_2)_n$-NH-$SO_2$-$(CH_2)_n$-NH-(cycloalkyle en $C_3$-$C_8$), $(CH_2)_n$-NH-$SO_2$-$(CH_2)_n$-N[alkyle en $C_1$-$C_8$]$_2$,
$(CH_2)_n$-NH-CN, $(CH_2)_n$-NH-$SO_2$-R16,
$(CH_2)_n$-NR12-CO-NH-(alkyle en $C_1$-$C_8$), $(CH_2)_n$-NR12-CO-NH-(cycloalkyle en $C_3$-$C_8$), $(CH_2)_n$-NR12-CO-$NH_2$, $(CH_2)_n$-NR12-CO-NH-$SO_2$-(alkyle en $C_1$-$C_8$), $(CH_2)_n$-NR12-CO-NH-SO2-(cycloalkyle en $C_3$-$C_8$), $(CH_2)_n$-NR12-CO-N [alkyle en $C_1$-$C_8$]$_2$, $(CH_2)_n$-NH-CO-NH-$(CH_2)_n$-CO-[O-(alkyle en $C_1$-$C_8$)], $(CH_2)$ $_n$-NH-CO-NH-$(CH_2)_q$-CO-$NH_2$, $(CH_2)_n$-NH-CO-NH-$(CH_2)_q$-COOH, $(CH_2)_n$-NH-C (=NH) -$NH_2$, $(CH_2)_n$-NH-C (=NH)-R16, $(CH_2)_n$-NH-C (=NH) -NH [alkyle en $C_1$-$C_8$], $(CH_2)_n$-NH-C(=N-$SO_2$-(alkyle en $C_1$-$C_8$)-$NH_2$, $(CH_2)_n$-NH-C (=N-$SO_2$-(alkyle en $C_1$-$C_8$)-NH [alkyle en $C_1$-$C_8$], $(CH_2)_n$-NH-C (=N-$SO_2$-$NH_2$) -$NH_2$, $(CH_2)_n$-NH-C (=N-$SO_2$-$NH_2$) -NH [alkyle en $C_1$-$C_8$], $(CH_2)_n$-NH-C (=NH)-N[alkyle en $C_1$-$C_8$]$_2$, $(CH_2)_n$-NH-C(=N-$SO_2$-(alkyle en $C_1$-$C_8$)-N[alkyle en $C_1$-$C_8$]$_2$, $(CH_2)_n$-NH-$(CH_2)_n$-CO-O-$(CH_2)_r$-$NH_2$, $(CH_2)_n$-NH-$(CH_2)_n$-CO-NH-[alkyle en $C_1$-$C_8$], $(CH_2)_n$-NH-$(CH_2)_n$-CO-NH- $(CH_2)_r$-OH, $(CH_2)_n$-NH-$(CH_2)_n$-CO-N[alkyle en $C_1$-$C_8$]$_2$, $(CH_2)_n$-NH-$(CH_2)_n$-CO-NH-[cycloalkyle en $C_3$-$C_8$], $(CH_2)_n$-NH-C$(CH_3)_2$-CO-O(cycloalkyle en $C_3$-$C_8$), $(CH_2)_n$-

NH-$C(CH_3)_2$-CO-O-$(CH_2)_r$-$NH_2$, $(CH_2)_n$-NH-C $(CH_3)_2$-CO-O-$(CH_2)_n$-aryle, $(CH_2)_n$-NH-C $(CH_3)_2$-CO-O-$(CH_2)_n$-hétéroaryle, $(CH_2)_n$-NH-$C(CH_3)_2$-CO-NH-[alkyle en $C_1$-$C_8$], $(CH_2)_n$-NH-$C(CH_3)_2$-CO-NH-$(CH_2)_r$-OH, $(CH_2)_n$-NH-$C(CH_3)_2$-CO-N[alkyle en $C_1$-$C_8$]$_2$, $(CH_2)_n$-NH-$(CH_3)_2$-CO-NH-[cycloalkyle en $C_3$-$C_8$], $(CH_2)_n$-NH-$C(CH_3)_2$-CON[cycloalkyle en $C_3$-$C_8$]$_2$, $(CH_2)_n$-$S(O)_m$-(alkyle en $C_1$-$C_8$), $(CH_2)_n$-$S(O)_m$-(cycloalkyle en $C_3$-$C_8$), $(CH_2)_n$-$SO_2$-

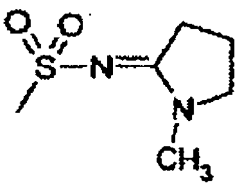

R16, $SO_2$-N=CH-$N(CH_3)_2$, $(CH_2)$n-$SO_2$-NH-CO-(alkyle en $C_1$-$C_8$), $(CH_2)_n$-$SO_2$-NH-CO-(cycloalkyle en $C_3$-$C_8$), $(CH_2)_n$-$SO_2$-NH-(alkyle en $C_1$-$C_8$), $(CH_2)_n$-$SO_2$-NH-(cycloalkyle en $C_3$-$C_8$), $(CH_2)_n$-$SO_2$-N[alkyle en $C_1$-$C_8$]$_2$, $SO_2$-NH-$(CH_2)_r$-OH, $SO_2$-NH-$(CH_2)_r$-$NH_2$, $SF_5$,

$(CH_2)_q$-CN,

$(CH_2)_n$-CO-NH-pipéridin-1-yle,

$(CH_2)_n$-CO-NH-$SO_2$-NHR12, $(CH_2)_n$-CO-NH-$SO_2$-(alkyle en $C_1$-$C_8$), $(CH_2)_n$-CO-NH-$SO_2$-(cycloalkyle en $C_3$-$C_8$),

$(CH_2)_n$-CHO, $(CH_2)_n$-C(=NH)$NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)$n-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O-(alkyle en $C_1$-$C_6$), $S(O)_m$-(alkyle en $C_1$-$C_6$), $SO_2$-$NH_2$, COOH, $CONH_2$, CO-[O(alkyle en $C_1$-$C_6$)], CO-(alkyle en $C_1$-$C_6$), et les radicaux alkyle pouvant être substitués par des atomes de fluor,

où toujours au moins l'un des radicaux R6, R7, R8, R9 et R10 représente un radical hétérocyclique T-bicyclique, T-aryle ou T-hétéroaryle, et

l'une des quatre paires de radicaux R6 et R7, ou R7 et R8, ou R8 et R9, ou R9 et R10, pouvant chacune former ensemble les groupes -$CH_2$-$CH_2$-$CH_2$-, ou -$CH_2$-$CH_2$-$CH_2$-$CH_2$-, où jusqu'à deux groupes -$CH_2$- peuvent être remplacés par -O-, et les groupes -$CH_2$-$CH_2$-$CH_2$- ou -$CH_2$-$CH_2$-$CH_2$-$CH_2$- peuvent être substitués par un ou des substituants F, alkyle en $C_1$-$C_8$ ou =O ;

T est

**NR23-CO-NR24, NR23-$SO_2$-NR24, $SO_2$-NR23-$SO_2$, CO-NR23-CO, NR23-C(-NR13)-NR24, NR23-C(=NR22)-NR24, CO-NR23-CR22R23, NR23-$SO_2$-GR22R24, CR22R24-$SO_2$-NR23, CR22R23-NR23-$SO_2$, $SO_2$-CR22R23-NR23, $SO_2$-NR23-CR22R23-, NR23-CR22R23-$SO_2$, CR23R24-CR23R24-CR23R24;**

R11 est H, un radical alkyle en $C_1$-$C_8$, cycloalkyle en $C_3$-$C_6$, $(CH_2)_n$-aryle, $(CH_2)_n$-CO-[O-(alkyle en $C_1$-$C_6$)], $(CH_2)_n$-CO-[O-(cycloalkyle en $C_3$-$C_6$)], $(CH_2)_n$-CO-[alkyle en $C_1$-$C_6$], $(CH_2)_n$-CO-[cycloalkyle en $C_3$-$C_6$], $(CH_2)_n$-CO-aryle, $(CH_2)_n$-CO-hétéroaryle, $(CH_2)_q$-CO-$NH_2$, $(CH_2)_q$-COOH, $(CH_2)_n$-P(O) (OH) [O-(alkyle en $C_1$-$C_4$)], $(CH_2)_n$-P(O)[O-(alkyle en $C_1$-$C_4$)]$_2$, $(CH_2)_n$-P(O)(O-$CH_2$-aryle)$_2$, $(CH_2)_n$-P(O) $(OH)_2$, $(CH_2)_n$-$SO_3H$, $(CH_2)_n$-$SO_2$-$NH_2$, $(CH_2)_n$-CO-NH-[alkyle en $C_1$-$C_4$], $(CH_2)_n$-CO-N [alkyle en $C_1$-$C_4$]$_2$, $(CH_2)_n$-CO-NH-[cycloalkyle en $C_3$-$C_6$], (alcényle en $C_2$-$C_6$)-CO-O[alkyle en $C_1$-$C_4$], (alcényle en $C_2$-$C_6$)-$CONH_2$, (alcényle en $C_2$-$C_6$)-COOH, (alcynyle en $C_2$-$C_6$)-CO-O [alkyle en $C_1$-$C_4$], (alcynyle en $C_2$-$C_6$)-$CONH_2$, (alcynyle en $C_2$-$C_6$)-COOH, $(CH_2)_n$-CR21[CO-O(alkyle en $C_1$-$C_4$)]$_2$, $(CH_2)_n$-CR21$(CONH_2)_2$, $(CH_2)_n$-CR21 (COOH) $_2$, $(CH_2)_n$-CR21R22-CO-O[alkyle en $C_1$-$C_4$], $(CH_2)_n$-CR21R22-$CONH_2$, $(CH_2)_n$-CR21R22-CO-NH-[alkyle en $C_1$-$C_4$], $(CH_2)_n$-CR21R22-CO-N[alkyle en $C_1$-$C_4$]$_2$, $(CH_2)_n$-CR21R22-COOH, $(CH_2)_n$-CO-R16, $(CH_2)_n$-CO-NH-C $(CH_3)_2$-CO-O[alkyle en $C_1$-$C_8$], $(CH_2)_n$-CO-NH-$C(CH_3)_2$-$CONH_2$, $(CH_2)_n$-CO-NH-$C(CH_3)_2$-COOH, les radicaux alkyle, alcényle, alcynyle et cycloalkyle pouvant être substitués par des atomes de fluor, et le radical aryle ou hétéroaryle pouvant être substitué par un ou des substituants halogéno, CN, alkyle en $C_1$-$C_4$, O-(alkyle en $C_1$-$C_4$), $S(O)_m$-(alkyle en $C_1$-$C_4$), $SO_2$-$NH_2$, COOH, $CONH_2$, CO-O(alkyle en $C_1$-$C_4$), et les radicaux alkyle pouvant être substitués par des atomes de fluor ;

R12 est H, un radical alkyle en $C_1$-$C_4$, cycloalkyle en $C_3$-$C_6$, $(CH_2)_n$-aryle, $(CH_2)_n$-hétéroaryle, les radicaux alkyle ou cycloalkyle pouvant être substitués par des atomes de fluor, et le radical aryle ou hétéroaryle pouvant être substitué par un ou des substituants halogéno, CN, alkyle en $C_1$-$C_4$, O-(alkyle en $C_1$-$C_4$), $SO_2$-$NH_2$, COOH, $CONH_2$, CO-O(alkyle en $C_1$-$C_4$), et les radicaux alkyle pouvant être substitués par des atomes de fluor ;

R13 est H, un radical $SO_2$-[alkyle en $C_1$-$C_4$], $SO_2$-[cycloalkyle en $C_3$-$C_6$], $SO_2$-$(CH_2)_n$-aryle, $SO_2$-$(CH_2)_n$-hétéroaryle,

les radicaux alkyle et cycloalkyle pouvant être substitués par des atomes de fluor, et le radical aryle ou hétéroaryle pouvant être substitué par un ou des substituants halogéno, CN, $CF_3$, alkyle en $C_1$-$C_4$, 0-[alkyle en $C_1$-$C_4$], $S(O)_m$-[alkyle en $C_1$-$C_6$], $SO_2$-$NH_2$, COOH, $CONH_2$, CO-[O(alkyle en $C_1$-$C_4$)], et les radicaux alkyle pouvant être substitués par des atomes de fluor ;

R15 est un radical alkyle en $C_1$-$C_6$,

le radical alkyle pouvant être substitué par des atomes de fluor ;

R16 est un radical aziridin-1-yle, azétidin-1-yle, 3-hydroxy-azétidin-1-yle, pipéridin-1-yle, pyrrolidin-1-yle, 3-pyrrolidinol-1-yle, morpholin-N-yle, pipérazin-1-yle, 4-[alkyle en $C_1$-$C_6$]pipérazin-1-yle, thiomorpholin-1,1-dioxyde-4-yle, NH-$(CH_2)_r$-OH, NH-CH$(CH_2OH)_2$, NH-C$(CH_2OH)_3$, N[(alkyle en $C_1$-$C_4$)-OH]$_2$, D-glucamin-N-yle, N-méthyl-D-glucamin-N-yle, NH-[alkyle en $C_1$-$C_4$]-COOH, NH-[alkyle en $C_1$-$C_4$]-$CONH_2$, N[alkyle en $C_1$-$C_4$][alkyle en $C_1$-$C_4$]-COOH, NH-[C(H)(aryle)]-CO-O(alkyle en $C_1$-$C_4$], NH-[C(H)(aryle)]-COOH, NH-[C(H)(aryle)]-$CONH_2$, NH-[C(H)(hétéroaryle)]-CO-O(alkyle en $C_1$-$C_4$), NH-[C(H)(hétéroaryle)]-COOH, NH-[C (H) (hétéroaryle)]-$CONH_2$, NH- [cycloalkyle en $C_3$-$C_6$]-CO-O(alkyle en $C_1$-$C_4$), NH-[cycloalkyle en $C_3$-$C_6$]-COOH, NH-[cycloalkyle en $C_3$-$C_6$]-$CONH_2$, NH-(alkyle en $C_1$-$C_4$)-OH, NH-[alkyle en $C_1$-$C_4$]-$SO_2$-(alkyle en $C_1$-$C_4$), NH-[alkyle en $C_1$-$C_4$]-$SO_3H$, NH-[alkyle en $C_1$-$C_4$]-$SO_2$-$NH_2$,

les fonctions alcool (OH) pouvant être remplacées par F, et le radical aryle ou hétéroaryle pouvant être substitué par un ou des substituants halogéno, CN, alkyle en $C_1$-$C_4$, O-(alkyle en $C_1$-$C_4$), OH, $SO_2$-$NH_2$, COOH, $CONH_2$, CO-O(alkyle en $C_1$-$C_4$) ;

R18 est $(CH_2)_n$-CR25R26-CO-O (alkyle en $C_1$-$C_4$), $(CH_2)_n$-CR25R26-CO-$NH_2$, $(CH_2)_n$-CR25R26-COOH ;

R20 est H, un radical alkyle en $C_1$-$C_4$, cycloalkyle en $C_3$-$C_6$, aryle, [alkyle en $C_1$-$C_4$]-aryle ;

R21 est H, F, $CF_3$, un radical alkyle en $C_1$-$C_4$, cycloalkyle en $C_3$-$C_6$, OH, O-(alkyle en $C_1$-$C_4$), 0-(cycloalkyle en $C_3$-$C_6$), O-$(CH_2)_n$-aryle, O-(CO)-(alkyle en $C_1$-$C_4$), O-(CO)-(cycloalkyle en $C_3$-$C_6$), O-(CO)-O-(alkyle en $C_1$-$C_4$), O-(CO)-O-(cycloalkyle en $C_3$-$C_6$), NH-[alkyle en $C_1$-$C_4$]-aryle, $NH_2$, NH-(alkyle en $C_1$-$C_4$), NH-(CO)-(alkyle en $C_1$-$C_4$) ;

R22 est H, $CF_3$, un radical alkyle en $C_1$-$C_4$, aryle, [alkyle en $C_1$-$C_4$]-aryle ;

R23, R24 représentent chacun indépendamment de l'autre H, un radical alkyle en $C_1$-$C_4$, cycloalkyle en $C_3$-$C_6$, [alkyle en $C_1$-$C_4$]-[cycloalkyle en $C_3$-$C_6$], aryle, [alkyle en $C_1$-$C_4$]-aryle,

ou R23 et R24 forment ensemble un motif -CH=CH-, -$CH_2$-$CH_2$-, -$CH_2$-$CH_2$-$CH_2$- ou -$CH_2$-$CH_2$-$CH_2$-$CH_2$-, où un groupement $CH_2$ peut être remplacé par C=O, CHF ou $CF_2$, et jusqu'à quatre atomes d'hydrogène pouvant être remplacés par un radical alkyle en $C_1$-$C_4$ ;

R25, R26 représentent chacun indépendamment de l'autre H, F, un radical alkyle en $C_1$-$C_4$, aryle, [alkyle en $C_1$-$C_4$]-aryle, le radical aryle pouvant être substitué par un ou des substituants halogéno, CN, OH, O-(alkyle en $C_1$-$C_4$), ou les radicaux R25 et 26 forment, ensemble avec l'atome de carbone auquel ils sont liés, un radical carbocyclique à trois à sept chaînons, dans lequel un atome de carbone peut être remplacé par O, $S(O)_m$, NH, N-[alkyle en $C_1$-$C_4$] ou CO ;

à l'exception du composé N-[3-tert-butyl-1-(4-méthylphényl)-1H-pyrazol-5-yl]-N'(4-{[3-(2-méthylphényl)-2,4-dioxo-1-imidazolidinyl]méthyl]}-phényl)urée,

ainsi que leurs sels physiologiquement tolérés.

**5.** Composés de formule la selon la revendication 1

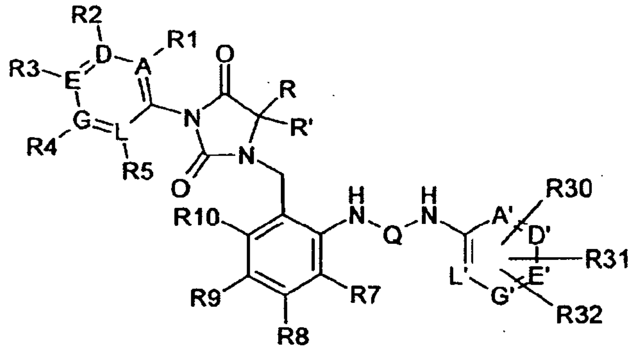

Ia

dans laquelle

R, R' représentent chacun indépendamment de l'autre H, un radical aryle, alkyle en $C_1$-$C_4$, le radical alkyle en $C_1$-$C_4$ ou le radical aryle pouvant être substitué par un ou des substituants halogéno ;
ou R et R' forment ensemble un noyau ayant trois à huit atomes de carbone, un atome de carbone pouvant être remplacé par O, $S(O)_m$, NR13 ou NR15 ;
m vaut 0, 1, 2 ;
n vaut 0, 1, 2 ;
q vaut 1, 2 ;
r vaut 2, 3 ;
v vaut 0, 1, 2 ;
A, D, E, G, L représentent chacun indépendamment des autres C ou N, où, pour la signification N, le substituant correspondant R1, R2, R3, R4, R5 disparaît, ou R2-D=E-R3 ou R4-G=L-R5 représentent S ou O, et le noyau à cinq ou six chaînons peut être condensé à $-(CH_2)_3-$ ou $-(CH_2)_4-$ ou -CH=CH-CH=CH-, pour donner un radical bicyclique ;
Q est C=O, $SO_2$ ;
R1, R2, R3, R4, R5 représentent chacun indépendamment des autres H, F, Cl, Br, I, CN, $CF_3$, un radical alkyle en $C_1$-$C_8$, cycloalkyle en $C_3$-$C_8$, $(CH_2)_n$-aryle, $(CH_2)_n$-hétéroaryle, $OCF_3$, O-R11, NR13R15, $S(O)_m$-R12, $SO_2$-$NH_2$, $SO_2$-NH-[alkyle en $C_1$-$C_8$], $SO_2$-NH-[cycloalkyle en $C_3$-$C_8$], $SO_2$-NH-$(CH_2)_n$-aryle, $SO_2$-NH-$(CH_2)_n$-hétéroaryle, $SO_2$-N[alkyle en $C_1$-$C_8$]$_2$, $SO_2$-R16, $SF_5$, CO-O[alkyle en $C_1$-$C_8$], CO-O[cycloalkyle en $C_3$-$C_6$], CO-$NH_2$, CO-NH-[alkyle en $C_1$-$C_4$], CO-N[alkyle en $C_1$-$C_4$)]$_2$, C(=NH)-$NH_2$, C (=NH) -R16, $(CH_2)_n$-C(=N$SO_2$-R12)$NH_2$, CO-R16, COOH, CO-(alkyle en $C_1$-$C_4$), CO-(cycloalkyle en $C_3$-$C_6$), CO-aryle, CO-hétéroaryle, CH(OH)-aryle, CH(OH)-hétéroaryle, CHF-aryle, CHF-hétéroaryle, $CF_2$-aryle, $CF_2$-hétéroaryle, $CH_2$-OH, $CH_2$-CN, $CH_2$-O-R12, $CH_2$-O-$(CH_2)_q$-COOH, les radicaux alkyle, et cycloalkyle pouvant être substitués par des atomes de fluor, et les radicaux aryle ou hétéroaryle pouvant être substitués par un ou des substituants halogéno, CN, alkyle en $C_1$-$C_4$, O-(alkyle en $C_1$-$C_4$), $OCF_3$, OH, O-$(CH_2)_n$-aryle, $(CH_2)_n$-aryle, $S(O)_m$-(alkyle en $C_1$-$C_4$), $SO_2$-$NH_2$, SH, NR12R13, NH-CO-[alkyle en $C_1$-$C_4$], NH-CO-$(CH_2)_n$-aryle, $(CH_2)_n$-COOH, $(CH_2)_n$-$CONH_2$, $(CH_2)_n$-CO-O(alkyle en $C_1$-$C_4$), $(CH_2)_n$-CO-(alkyle en $C_1$-$C_4$), et les radicaux alkyle pouvant être substitués par des atomes de fluor ;
R7, R8, R9, R10 représentent chacun indépendamment des autres H, F, Cl, Br, I, CN, $N_3$, NC, $NO_2$, $CF_3$, un radical alkyle en $C_1$-$C_8$, alcényle en $C_2$-$C_{10}$, alcynyle en $C_2$-$C_{10}$, cycloalkyle en $C_3$-$C_8$, aryle, hétéroaryle,
$(CH_2)_n$-CO-[O-(alkyle en $C_1$-$C_8$)], $(CH_2)_n$-CO-[O-(cycloalkyle en $C_3$-$C_8$)], $(CH_2)_n$-CO-[alkyle en $C_1$-$C_8$], $(CH_2)_n$)-CO-[cycloalkyle en $C_3$-$C_8$)],
$(CH_2)_n$-CO-[O-$(CH_2)_v$-aryle],
$(CH_2)_n$-CO-$NH_2$, $(CH_2)_n$-COOH, $(CH_2)_n$-CO-NH-CN,
$(CH_2)_n$-P(O) (OH) [O-(alkyle en $C_1$-$C_6$)], $(CH_2)_n$-P(O)[O-(alkyle en $C_1$-$C_6$)]$_2$, $(CH_2)_n$-P(O)(OH)(O-$CH_2$-aryle), $(CH_2)_n$-P(O) (O-$CH_2$-aryle)$_2$, $(CH_2)_n$-P(O)(OH) $_2$, $(CH_2)$ n-$SO_3$H, $(CH_2)$ n-$SO_2$-$NH_2$,
$(CH_2)_n$-CO-NH-[alkyle en $C_1$-$C_8$], $(CH_2)_n$-CO-N[alkyle en $C_1$-$C_8$]$_2$, $(CH_2)_n$-CO-NH-[cycloalkyle en $C_3$-$C_8$],
(alcényle en $C_2$-$C_{10}$)-CO-O[alkyle en $C_1$-$C_6$], (alcényle en $C_2$-$C_{10}$)-$CONH_2$, (alcényle en $C_2$-$C_{10}$)-COOH, (alcynyle en $C_2$-$C_{10}$)-CO-O[alkyle en $C_1$-$C_6$], (alcynyle en $C_2$-$C_{10}$)-$CONH_2$, (alcynyle en $C_2$-$C_{10}$)-COOH,
$(CH_2)_n$-CO-R16,
$(CH_2)_n$-OH, $(CH_2)_n$-O-(alkyle en $C_1$-$C_8$), $(CH_2)_n$-O-(alcényle en $C_2$-$C_{10}$), $(CH_2)_n$-O-( alcynyle en $C_2$-$C_{10}$), $(CH_2)_n$-O-(cycloalkyle en $C_3$-$C_8$), $(CH_2)_n$-O-$(CH_2)_q$-[cycloalkyle en $C_3$-$C_8$], $(CH_2)_n$-O-$(CH_2)_n$-CO-[O-(alkyle, en $C_1$-$C_8$)], $(CH_2)_n$-O-$(CH_2)_n$-CO-[O-(cycloalkyle en $C_3$-$C_8$)], $(CH_2)_n$-O-$(CH_2)_n$-CO-[alkyle en $C_1$-$C_8$], $(CH_2)_n$-O-$(CH_2)_n$-CO-[cycloalkyle en $C_3$-$C_8$], $(CH_2)_n$-O-$(CH_2)_n$-CO-[O-$(CH_2)_v$-aryle], $(CH_2)_n$-O-$(CH_2)_n$-CO-[O-$(CH_2)_v$-hétéroaryle], $(CH_2)_n$-O-$(CH_2)_q$-CO-$NH_2$, $(CH_2)_n$-O-$(CH_2)_q$-COOH, $(CH_2)_n$-O-$(CH_2)_q$-CO-NH-CN, $(CH_2)_n$-O-$(CH_2)_n$-P(O)(OH)[O-(alkyle en $C_1$-$C_6$)], $(CH_2)_n$-O-$(CH_2)_n$-P(O)[O-(alkyle en $C_1$-$C_6$)]$_2$, $(CH_2)_n$-O-$(CH_2)_n$-P(O)(OH) (O-$CH_2$-aryle) , $(CH_2)_n$-O-$(CH_2)_n$-P(O)(O-$CH_2$-aryle)$_2$, $(CH_2)_n$-O-$(CH_2)_n$-P(O)(OH)$_2$, $(CH_2)_n$-O-$(CH_2)_n$-$SO_3$H, $(CH_2)_n$-O-$(CH_2)_n$-$SO_2$-$NH_2$, $(CH_2)_n$-O-$(CH_2)_n$-CO-NH-[alkyle en $C_1$-$C_8$], $(CH_2)_n$-O-$(CH_2)_n$-CO-N[alkyle en $C_1$-$C_8$]2, $(CH_2)_n$-O-$(CH_2)_n$-CO-NH-[cycloalkyle en $C_3$-$C_8$)], $(CH_2)_n$-O-$(CH_2)_n$-CR21R22-CO-O[alkyle en $C_1$-$C_6$)], $(CH_2)_n$-O-$(CH_2)_n$-CR21R22-$CONH_2$, $(CH_2)_n$-O-$(CH_2)_n$-CR21R22-COOH, $(CH_2)_n$-O-$(CH_2)_n$-CO-R16, $(CH_2)_n$-O-$(CH_2)_r$-OH, $(CH_2)_n$-O-CH($CH_2$OH)$_2$, $(CH_2)_n$-O-$(CH_2)_n$-CO-O-$(CH_2)_r$-$NH_2$, $(CH_2)_n$-O-$(CH_2)_n$-CO-NH-$(CH_2)_r$-OH, O-R13, $OCF_3$,
$(CH_2)_n$-$NH_2$, $(CH_2)_n$-NH-(alkyle en $C_1$-$C_8$), $(CH_2)_n$-NH-(cycloalkyle en $C_3$-$C_8$), $(CH_2)_n$-NH-$(CH_2)_n$-CO-[O-(cycloalkyle en $C_3$-$C_8$)], $(CH_2)_n$-NH-$(CH_2)_n$-CO-[alkyle en $C_1$-$C_8$], $(CH_2)_n$-NH-$(CH_2)_n$-CO-[cycloalkyle en $C_3$-$C_8$], $(CH_2)_n$-NH- $(CH_2)_n$-CO-[O-$(CH_2)$ $_v$-aryle], $(CH_2)_n$-NH-$(CH_2)_n$-CO-[0-$(CH_2)_v$-hétéroaryle], $(CH_2)_n$-NH-$(CH_2)_q$-CO-NH-CN,
$(CH_2)_n$-NH-$(CH_2)_n$-P(O) (OH) 2,
$(CH_2)_n$-NH-$(CH_2)_n$-$SO_3$H,
$(CH_2)_n$-NH-$(CH_2)_n$-$SO_2$-$NH_2$,

$(CH_2)_n$-NH-$(CH_2)_n$-CR21R22-CO-O[alkyle en $C_1$-$C_6$], $(CH_2)_n$-NH-$(CH_2)_n$-CR21R22-CONH$_2$, $(CH_2)_n$-NH-$(CH_2)_n$-CR21R22-COOH,
$(CH_2)_n$-NH-$(CH_2)_n$-CO-R16,
$(CH_2)_n$-NH-$(CH_2)_n$-$SO_2$-[alkyle en $C_1$-$C_8$], $(CH_2)_n$-NH-$(CH_2)_n$-$SO_2$-[cycloalkyle en $C_3$-$C_8$], $(CH_2)_n$-NH-$SO_2$-$(CH_2)_n$-$NH_2$, $(CH_2)_n$-NH-$SO_2$-$(CH_2)_n$-NH-(alkyle en $C_1$-$C_8$), $(CH_2)_n$-NH-$SO_2$-$(CH_2)_n$-NH-(cycloalkyle en $C_3$-$C_8$), $(CH_2)_n$-NH-$SO_2(CH_2)_n$-N [alkyle en $C_1$-$C_8]_2$,
$(CH_2)_n$-NH-CN, $(CH_2)_n$-NH-$SO_2$-R16,
$(CH_2)_n$-NR12-CO-NH-(alkyle en $C_1$-$C_8$), $(CH_2)_n$-NR12-CO-NH-(cycloalkyle en $C_3$-$C_8$), $(CH_2)_n$-NR12-CO-$NH_2$, $(CH_2)_n$-NR12-CO-NH-$SO_2$-(alkyle en $C_1$-$C_8$), $(CH_2)_n$-NR12-CO-NH-$SO_2$-(cycloalkyle en $C_3$-$C_8$), $(CH_2)_n$-NR12-CO-N[alkyle en $C_1$-$C_8]_2$, $(CH_2)_n$-NH-CO-NH-$(CH_2)_n$-CO-[O-(alkyle en $C_1$-$C_8$)], $(CH_2)_n$-NH-CO-NH-$(CH_2)_q$-CO-$NH_2$, $(CH_2)_n$-NH-CO-NH-$(CH_2)_q$-COOH, $(CH_2)_n$-NH-C(=NH)-$NH_2$, $(CH_2)_n$-NH-C(=NH)-R16, $(CH_2)_n$-NH-C (=NH) -NH [alkyle en $C_1$-$C_8$], $(CH_2)_n$-NH-C(=N-$SO_2$-(alkyle en $C_1$-$C_8$)-$NH_2$, $(CH_2)_n$-NH-C(=N-$SO_2$-(alkyle en $C_1$-$C_8$)-NH[alkyle en $C_1$-$C_8$], $(CH_2)_n$-NH-C(=N-$SO_2$-$NH_2$)-$NH_2$, $(CH_2)_n$-NH-C (=N-$SO_2$-$NH_2$) -NH [alkyle en $C_1$-$C_8$], $(CH_2)_n$-NH-C(=NH)-N[alkyle en $C_1$-$C_8]_2$, $(CH_2)_n$-NH-C(=N-$SO_2$)-(alkyle en $C_1$-$C_8$)-N[alkyle en $C_1$-$C_8]_2$, $(CH_2)_n$-NH-$(CH_2)_n$-CO-O-$(CH_2)_r$-$NH_2$, $(CH_2)_n$-NH-$(CH_2)_n$-CO-NH-[alkyle en $C_1$-$C_8$], $(CH_2)_n$-NH-$(CH_2)_n$-CO-NH-$(CH_2)_r$-OH, $(CH_2)_n$-NH-$(CH_2)_n$-CO-N[alkyle en C1-$C_8]_2$, $(CH_2)_n$-NH-$(CH_2)_n$-CO-NH-[cycloalkyle en $C_3$-$C_8$], $(CH_2)_n$-NH-$C(CH_3)_2$-CO-O(cycloalkyle en $C_3$-$C_8$), $(CH_2)_n$-NH-$C(CH_3)_2$-CO-O-$(CH_2)_r$-$NH_2$, $(CH_2)_n$-NH-$C(CH_3)_2$-CO-O-$(CH_2)_n$-aryle, $(CH_2)_n$-NH-C $(CH_3)_2$-CO-O-$(CH_2)_n$-hétéroaryle, $(CH_2)_n$-NH-$C(CH_3)_2$-CO-NH-[alkyle en $C_1$-$C_8$], $(CH_2)_n$-NH-$C(CH_3)_2$-CO-NH-$(CH_2)_r$-OH, $(CH_2)_n$-NH-$C(CH_3)_2$-CO-N[alkyle en $C_1$-$C_8]_2$, $(CH_2)_n$-NH-$(CH_3)_2$-CO-NH-[cycloalkyle en $C_3$-$C_8$], $(CH_2)_n$-NH-$C(CH_3)_2$-CON[cycloalkyle en $C_3$-$C_8]_2$, $(CH_2)_n$-$S(O)_m$-(alkyle en $C_1$-$C_8$), $(CH_2)_n$-$S(O)_m$-(cycloalkyle en $C_3$-$C_8$), $(CH_2)_n$-$SO_2$-

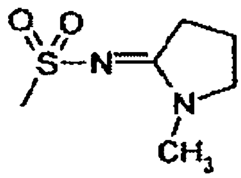

R16, $SO_2$-N=CH-$N(CH_3)_2$, $(CH_2)_n$-$SO_2$-NH-CO-(alkyle en $C_1$-$C_8$), $(CH_2)_n$-$SO_2$NH-CO- (cycloalkyle en $C_3$-$C_8$), $(CH_2)_n$-$SO_2$-NH-(alkyle en $C_1$-$C_8$), $(CH_2)_n$-$SO_2$-NH-(cycloalkyle en $C_3$-$C_8$), $(CH_2)_n$-$SO_2$N[alkyle en $C_1$-$C_8]_2$, $SO_2$-NH-$(CH_2)_r$-OH, $SO_2$NH-$(CH_2)_r$-$NH_2$, $SF_5$,
$(CH_2)_q$-CN,
$(CH_2)_n$-CO-NH-pipéridin-1-yle,
$(CH_2)_n$-CO-NH-$SO_2$-NHR12, $(CH_2)_n$-CO-NH-$SO_2$-(alkyle en $C_1$-$C_8$), $(CH_2)_n$-CO-NH-$SO_2$-(cycloalkyle en $C_3$-$C_8$),
$(CH_2)_n$-CHO, $(CH_2)_n$-C (=NH) $NH_2$, $(CH_2)_n$-C(=NH)NHOH, $(CH_2)_n$-C(=NH)(R16), $(CH_2)_n$-C(=NR13)NHR12, $(CH_2)_n$-C(=NR12)NR12R13, $(CH_2)_n$-C(=NH)O[alkyle en $C_1$-$C_6$], les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_6$, cycloalkyle en $C_3$-$C_6$, O- (alkyle en $C_1$-$C_6$), $S(O)_m$-(alkyle en $C_1$-$C_6$), $SO_2$-$NH_2$, COOH, $CONH_2$, CO-[O(alkyle en $C_1$-$C_6$)], CO-(alkyle en $C_1$-$C_6$), et les radicaux alkyle pouvant être substitués par des atomes de fluor ;
l'une des trois paires de radicaux R7 et R8 ou R8 et R9, ou R9 et R10, pouvant chacune former ensemble les groupes -$CH_2$-$CH_2$-$CH_2$- ou -$CH_2$-$CH_2$-$CH_2$-$CH_2$-, où jusgu'à deux groupes -$CH_2$- peuvent être remplacés par -O-, et les groupes -$CH_2$-$CH_2$-$CH_2$- ou -$CH_2$-$CH_2$-$CH_2$-$CH_2$- pouvant être substitués par un ou des substituants F, alkyle en $C_1$-$C_8$ ou =O ;
R11 est H, un radical alkyle en $C_1$-$C_8$, cycloalkyle en $C_3$-$C_6$, $(CH_2)_n$-aryle, $(CH_2)_n$-CO-[O-(alkyle en $C_1$-$C_6$)], $(CH_2)_n$-CO-[O-(cycloalkyle en $C_3$-$C_6$)], $(CH_2)_n$-CO-[alkyle en $C_1$-$C_6$], $(CH_2)_n$-CO-[cycloalkyle en $C_3$-$C_6$], $(CH_2)_n$-CO-aryle, $(CH_2)_n$-CO-hétéroaryle, $(CH_2)_q$-CO-$NH_2$, $(CH_2)_q$-COOH, $(CH_2)_n$-P(O)(OH) [O-(alkyle en $C_1$-$C_4$)], $(CH_2)_n$-P(O)[O-(alkyle en $C_1$-$C_4$)]$_2$, $(CH_2)_n$-P(O)(O-$CH_2$-aryle)$_2$, $(CH_2)_n$-P(O) $(OH)_2$, $(CH_2)_n$-$SO_3H$, $(CH_2)_n$-$SO_2$-$NH_2$, $(CH_2)_n$-CO-NH-[alkyle en $C_1$-$C_4$], $(CH_2)_n$-CO-N[alkyle en $C_1$-$C_4]_2$, $(CH_2)_n$-CO-NH-[cycloalkyle en $C_3$-$C_6$], (alcényle en $C_2$-$C_6$)-CO-O[alkyle en $C_1$-$C_4$], (alcényle en $C_2$-$C_6$)-$CONH_2$, (alcényle en $C_2$-$C_6$)-COOH, (alcynyle en $C_2$-$C_6$)-CO-O[alkyle en $C_1$-$C_4$], (alcynyle en $C_2$-$C_6$)-$CONH_2$, (alcynyle en $C_2$-$C_6$)-COOH, $(CH_2)_n$-CR21[CO-O(alkyle en $C_1$-$C_4$)]$_2$, $(CH_2)_n$-CR21 $(CONH_2)_2$, $(CH_2)_n$-CR21$(COOH)_2$, $(CH_2)_n$-CR21R22-CO-O[alkyle en $C_1$-$C_4$], $(CH_2)_n$-CR21R22-$CONH_2$, $(CH_2)_n$-CR21R22-CO-NH-[alkyle en $C_1$-$C_4$], (CH2)n-CR21R22-CO-N[alkyle en $C_1$-$C_4]_2$, $(CH_2)_n$-CR21R22-COOH, $(CH_2)_n$-CO-R16, $(CH_2)_n$-CO-NH-C $(CH_3)_2$-CO-O[alkyle en $C_1$-$C_8$], $(CH_2)_n$-CO-NH-$C(CH_3)_2$-$CONH_2$, $(CH_2)_n$-CO-NH-$C(CH_3)_2$-COOH,

les radicaux alkyle, alcényle, alcynyle et cycloalkyle pouvant être substitués par des atomes de fluor, et le radical aryle ou hétéroaryle pouvant être substitué par un ou des substituants halogéno, CN, alkyle en $C_1$-$C_4$, O-(alkyle en $C_1$-$C_4$), $S(O)_m$-(alkyle en $C_1$-$C_4$), $SO_2$-$NH_2$, COOH, $CONH_2$, CO-O(alkyle en $C_1$-$C_4$), et les radicaux alkyle pouvant être substitués par des atomes de fluor ;

R12 est H, un radical alkyle en $C_1$-$C_4$, cycloalkyle en $C_3$-$C_6$, $(CH_2)_n$-aryle, $(CH_2)_n$-hétéroaryle, les radicaux alkyle ou cycloalkyle pouvant être substitués par des atomes de fluor, et le radical aryle ou hétéroaryle pouvant être substitué par un ou des substituants halogéno, CN, alkyle en $C_1$-$C_4$, O-(alkyle en $C_1$-$C_4$), $SO_2$-$NH_2$, COOH, $CONH_2$, CO-O(alkyle en $C_1$-$C_4$), et les radicaux alkyle pouvant être substitués par des atomes de fluor ;

R13 est H, un radical $SO_2$-[alkyle en $C_1$-$C_4$], $SO_2$-[cycloalkyle en $C_3$-$C_6$], $SO_2$-$(CH_2)_n$-aryle, $SO_2$- $(CH_2)_n$-hétéroaryle,

les radicaux alkyle et cycloalkyle pouvant être substitués par des atomes de fluor, et le radical aryle ou hétéroaryle pouvant être substitué par un ou des substituants halogéno, CN, $CF_3$, alkyle en $C_1$-$C_4$, 0-[alkyle en $C_1$-$C_4$], $S(O)_m$-[alkyle en $C_1$-$C_6$], $SO_2$-$NH_2$, COOH, $CONH_2$, CO-[O(alkyle en $C_1$-$C_4$)], et les radicaux alkyle pouvant être substitués par des atomes de fluor ;

R15 est un radical alkyle en $C_1$-$C_6$,

le radical alkyle pouvant être substitué par des atomes de fluor ;

R16 est un radical aziridin-1-yle, azétidin-1-yle, 3-hydroxy-azétidin-1-yle, pipéridin-1-yle, pyrrolidin-1-yle, 3-pyrrolidinol-1-yle, morpholin-N-yle, pipérazin-1-yle, 4-[alkyle en $C_1$-$C_6$]pipérazin-1-yle, thiomorpholin-1,1-dioxyde-4-yle, NH-$(CH_2)_r$-OH, NH-$CH(CH_2OH)_2$, NH-C $(CH_2OH)_3$, N[(akyle en $C_1$-$C_4$)-OH]$_2$, D-glucamin-N-yle, N-méthyl-D-glucamin-N-yle, NH-[alkyle en $C_1$-$C_4$]-COOH, NH-[alkyle en $C_1$-$C_4$]-$CONH_2$, N [alkyle en $C_1$-$C_4$] [alkyle en $C_1$-$C_4$]-COOH, NH-[C(H)(aryle)]-CO-O (alkyle en $C_1$-$C_4$], NH-[C(H) (aryle)]-COOH, NH-[C(H)(aryle)]-$CONH_2$, NH-[C(H)(hétéroaryle)]-CO-O(alkyle en $C_1$-$C_4$), NH-[C(H) (hétéroaryle)]-COOH, NH-[C(H) (hétéroaryle)]-$CONH_2$, NH- [cycloalkyle en $C_3$-$C_6$]-CO-O(alkyle en $C_1$-$C_4$), NH-[cycloalkyle en $C_3$-$C_6$]-COOH, NH-[cycloalkyle en $C_3$-$C_6$]-$CONH_2$, NH-(alkyle en $C_1$-$C_4$)-OH, NH-[alkyle en $C_1$-$C_4$]-$SO_2$-(alkyle en $C_1$-$C_4$), NH-[alkyle en $C_1$-$C_4$]-$SO_3H$, NH- [alkyle en $C_1$-$C_4$]-$SO_2$-$NH_2$,

les fonctions alcool (OH) pouvant être remplacées par F, et le radical aryle ou hétéroaryle pouvant être substitué par un ou des substituants halogéno, CN, alkyle en $C_1$-$C_4$, O-(alkyle en $C_1$-$C_4$), OH, $SO_2$-$NH_2$, COOH, $CONH_2$, CO-O(alkyle en $C_1$-$C_4$);

R18 est un radical $(CH_2)_n$-CR25R26-CO-O (alkyle en $C_1$-$C_4$), $(CH_2)_n$-CR25R26-CO-$NH_2$, $(CH_2)_n$-CR25R26-COOH ;

R21 est H, F, $CF_3$, un radical alkyle en $C_1$-$C_4$, cycloalkyle en $C_3$-$C_6$, OH, O-(alkyle en $C_1$-$C_4$), 0-(cycloalkyle en $C_3$-$C_6$), O-$(CH_2)_n$-aryle, O-(CO)-(alkyle en $C_1$-$C_4$), O-(CO)-(cycloalkyle en $C_3$-$C_6$), O-(CO)-O-(alkyle en $C_1$-$C_4$), O-(CO)-O-(cycloalkyle en $C_3$-$C_6$), NH-[alkyle en $C_1$-$C_4$]-aryle, $NH_2$, NH- (alkyle en $C_1$-$C_4$), NH-(CO)-(alkyle en $C_1$-$C_4$);

R22 est H, $CF_3$, un radical alkyle en $C_1$-$C_4$, aryle, [alkyle en $C_1$-$C_4$]-aryle ;

R25, R26 représentent chacun indépendamment de l'autre H, F, un radical alkyle en $C_1$-$C_4$, aryle, [alkyle en $C_1$-$C_4$]-aryle, le radical aryle pouvant être substitué par un ou des substituants halogéno, CN, OH, O-(alkyle en $C_1$-$C_4$), ou les radicaux R25 et R26 forment ensemble avec l'atome de carbone auquel ils sont liés, un radical hétérocyclique à trois à sept chaînons, dans lequel un atome de carbone peut être remplacé par O, $S(O)_m$, NH, un radical N[alkyle en $C_1$-$C_4$] ou CO ;

A', D', E', G', L' représentent chacun indépendamment des autres CH ou N, où, pour la signification N, le substituant correspondant R30, R31 ou R32 disparaît, s'il était lié à l'atome d'azote ;

R30, R31, R32 représentent chacun indépendamment des autres R11, F, Cl, Br, I, CN, $CF_3$, un radical (CH2)n-OR11, O-R13 , $OCF_3$, $(CH_2)_n$-NH-R11, $(CH_2)_n$-N[$(CH_2)_q$-CO-O (alkyle en $C_1$-$C_4$)]$_2$, $(CH_2)_n$-N [$(CH_2)_q$-COOH]$_2$, $(CH_2)_n$-N[$(CH_2)_q$-$CONH_2$]$_2$, $(CH_2)_n$-NR-R13, $(CH_2)_n$-N$(R13)_2$, $(CH_2)_n$-NH-$SO_2$-R16, $(CH_2)_n$-NH-(CH2)n-$SO_2$-R12, $(CH_2)_n$-NR12-CO-R16, $(CH_2)_n$-NR12-CO-NR12R13, $(CH_2)_n$-NR12-CO-N$(R12)_2$, $(CH_2)_n$-NR12-CO-NHR11, $(CH_2)_n$-NH-C(=NH)-$NH_2$, $(CH_2)_n$-NH-C(=NH)-R16, $(CH_2)_n$-NH-C (=NH)-NHR12, $(CH_2)_n$-NH- $(CH_2)_n$-CO-NH-[alkyle en $C_1$-$C_4$], $(CH_2)_n$-NH-$(CH_2)_n$-CO-N [alkyle en $C_1$-$C_4$)]$_2$, $(CH_2)_n$-NH-C$(CH_3)_2$-CO-0(alkyle en $C_1$-$C_4$), $(CH_2)_n$-NH-C$(CH_3)_2$-CO-O(cycloalkyle en $C_3$-$C_6$), $(CH_2)_n$-NH-C$(CH_3)_2$-CO-O-$CH_2)_r$-$NH_2$, $(CH_2)_n$-NH-C $(CH_3)_2$-CO-O-$(CH_2)_n$-aryle, $(CH_2)_n$-NH-C $(CH_3)_2$-CO-O- $(CH_2)_n$-hétéroaryle, $(CH_2)_n$-NH-C $(CH_3)_2$-CO-$NH_2$, (CH2)n-NH-$(CH_3)_2$-CO-NH- [alkyle en $C_1$-$C_4$], $(CH_2)_n$-NH-C $(CH_3)_2$-CO-N[alkyle en $C_1$-$C_4$]$_2$, $(CH_2)_n$-NH-C$(CH_3)_2$-COOH, $S(O)_m$-R12, $SO_2$-R16, $SO_2$-N=CH-N$(CH_3)_2$, $SO_2$-NH-CO-R12, $SO_2$-NHR12, $SO_2$-N[alkyle en $C_1$-$C_4$]$_2$, $SF_5$, COOH, CO-$NH_2$, $(CH_2)_q$-CN, $(CH_2)_n$-CO-NH-pipéridin-1-yle, $(CH_2)_n$-CO-NH-$SO_2$-NHR12, $(CH_2)_n$-CO-NH-$SO_2$-R18, $(CH_2)_n$-C (=NH) -NHOH, $(CH_2)_n$-C(=NR13)NHR12, $(CH_2)_n$-C (=NR12)NR12R13, $(CH_2)_n$-C (=$NSO_2$-R12)$NH_2$, (CH2)n-P(O)(OH)[O-(alkyle en $C_1$-$C_4$)], (CH2)n-P(O)[O-(alkyle en $C_1$-$C_4$)]$_2$, $(CH_2)_n$-P(O) (O-$CH_2$-aryle)$_2$, $(CH_2)_n$-P(O)$(OH)_2$, les radicaux alkyle et cycloalkyle pouvant être substitués par des atomes de fluor, et les radicaux aryle ou hétéroaryle pouvant être substitués par un ou des substituants halogéno, CN, alkyle en $C_1$-$C_4$, O-(alkyle en $C_1$-$C_4$), S $(O)_m$- (alkyle

en $C_1$-$C_4$), $SO_2$-$NH_2$, COOH, $CONH_2$, CO-O (alkyle en $C_1$-$C_4$), et les radicaux alkyle pouvant être substitués par des atomes de fluor ;
à l'exception du composé N-[3-tert-butyl-1-(4-méthylphényl)-1H-pyrazol-5-yl]-N'-(4-{[3-(2-méthylphényl)-2,4-dioxo-1-imidazolidinyl]méthyl]}-phényl)urée
ainsi que leurs sels physiologiquement tolérés.

**6.** Composés de formule Ia selon la revendication 5, **caractérisés en ce que**, dans cette formule

R, R' sont des radicaux alkyle en $C_1$-$C_4$ ;
ou R et R' forment ensemble un noyau ayant trois à huit atomes de carbone ;
m vaut 0, 1, 2 ;
n vaut 0, 1, 2 ;
A, D, E, G, L, C ;
Q est C=O-$SO_2$ ;
R1, R2, R3, R4, R5 représentent chacun indépendamment des autres H, F, Cl, Br, CN, $CF_3$, un radical cycloalkyle en $C_1$-$C_8$, O-phényle ;
R7, R8, R9, R10 représentent chacun indépendamment des autres H, F, Cl, Br, $CF_3$, -$OCH_3$ ;
A', E' représentent chacun indépendamment de l'autre CH ou N, où, pour la signification N, le substituant correspondant R30, R31 ou R32 disparaît s'il était lié à l'atome d'azote,
R30, R31, R32 représentent chacun indépendamment des autres H, F, Cl, Br, $CF_3$, OH, $NO_2$, $NH_2$, $CONH_2$, COOH, un radical -COO-(alkyle en $C_1$-$C_8$), $(CH_2)_n$-P(O)(OH)[O-(alkyle en $C_1$-$C_4$)], $(CH_2)_n$-P(O) [O-(alkyle en $C_1$-$C_4$)]$_2$, $(CH_2)_n$-P(O)$(OH)_2$, $S(O)_m$-(alkyle en $C_1$-$C_4$), S(O)m- ($CH_2$)-COOH, $S(O)_m$-($CH_2$)-COO-(alkyle en $C_1$-$C_4$), $SO_2$-Cl, $SO_2$-$NH_2$, $SO_3H$ ;
ainsi que leurs sels physiologiquement tolérés.

**7.** Composés de formule Ia selon la revendication 6, **caractérisés en ce que**, dans cette formule :

R, R' sont des radicaux alkyle en $C_1$-$C_4$ ;
ou R et R' forment ensemble un noyau ayant trois à huit atomes de carbone ;
m vaut 0, 1, 2 ;
n vaut 0, 1, 2 ;
A, D, E, G, L, C ;
Q est C=O, $SO_2$ ;
R1, R2, R3, R4, R5 représentent chacun indépendamment des autres H, F, Cl, Br, CN, $CF_3$, un radical cycloalkyle en $C_1$-$C_8$, O-phényle ;
R7, R8, R9, R10 représentent chacun indépendamment des autres H, F, Cl, Br, $CF_3$, $OCH_3$ ;
A', E' représentent chacun indépendamment de l'autre CH ou N, où, pour la signification N, le substituant correspondant R30, R31 ou R32 disparaît s'il était lié à l'atome d'azote,
R30, R31, R32 représentent chacun indépendamment des autres H, F, Cl, Br, $CF_3$, OH, $NO_2$, $NH_2$, $CONH_2$, COOH, -COO- (alkyle en $C_1$-$C_8$), $(CH_2)_n$-P(O)(OH)[O-(alkyle en $C_1$-$C_4$)], $(CH_2)_n$-P(O) [O-(alkyle en $C_1$-$C_4$)]$_2$, $(CH_2)_n$-P(O) $(OH)_2$, $S(O)_m$-(alkyle en $C_1$-$C_4$), $S(O)_m$-($CH_2$)-COOH, $S(O)_m$-($CH_2$)-COO-(alkyle en $C_1$-$C_4$), $SO_2$-Cl, $SO_2$-$NH_2$, $SO_3H$ ;
ainsi que leurs sels physiologiquement tolérés.

**8.** Composés selon l'une ou plusieurs des revendications 1 à 7, pour utilisation en tant que médicament.

**9.** Médicament contenant un ou plusieurs des composés selon l'une des revendications 1 à 7.

**10.** Médicament contenant un ou plusieurs des composés selon l'une ou plusieurs des revendications 1 à 7 et au moins un autre principe actif.

**11.** Médicament selon la revendication 8, **caractérisé en ce qu'**il contient, en tant qu'autre principe actif, un ou plusieurs antidiabétiques, principes actifs hypoglycémiques, inhibiteurs de la HMGCoA-réductase, inhibiteurs de la résorption du cholestérol, agonistes des récepteurs PPAR-gamma, agonistes des PPAR-alpha, agonistes des récepteurs PPAR-alpha/gamma, agonistes des récepteurs PPAR-delta, fibrates, inhibiteurs de la MTP, inhibiteurs de la résorption des acides biliaires, inhibiteurs de la MTP, inhibiteurs de la CETP, agents polymères adsorbant les acides biliaires, inducteurs des récepteurs des LDL, inhibiteurs de l'ACAT, antioxydants, inhibiteurs de la lipoprotéine lipase, inhibiteurs de l'ATP-citrate-lyase, inhibiteurs de la squalène synthétase, antagonistes de la lipoprotéine (a), agonistes

du récepteur HM74A, inhibiteurs de la lipase, insulines, sulfonylurées, biguanides, méglitinides, thiazolidinediones, inhibiteurs de l'α-glucosidase, principes actifs agissant sur le canal potassique ATP-dépendant, des cellules bêta, inhibiteurs de la glycogène phosphorylase, antagonistes du récepteur du glucagon, activateurs de la glucokinase, inhibiteurs de la gluconogenèse, inhibiteurs de la fructose-1,6-bisphosphatase, modulateurs du transporteur de glucose de type 4, inhibiteurs de la glutamine-fructose-6-phosphate-amidotransférase, inhibiteurs de la dipeptidylpeptidase-IV, inhibiteurs de la 11-bêta-hydroxystéroïde déshydrogénase-I, inhibiteurs de la protéine-tyrosine-phosphatase-1B, modulateurs du transporteur de glucose sodium-dépendant de type 1 ou 2, modulateurs du GPR40, inhibiteurs de la lipase sensible aux hormones, inhibiteurs de l'acétyl-CoA carboxylase, inhibiteurs de la phosphoénolpyruvatecarboxykinase, inhibiteurs de la glycogène synthase kinase-3 bêta, inhibiteurs de la protéine kinase C bêta, antagonistes du récepteur de l'endothéline A, inhibiteurs de la I kappaB kinase, modulateurs du récepteur des glucocorticoïdes, agonistes de CART, antagonistes de NPY, agonistes de MC4, antagonistes de l'orexine, antagonistes H3, agonistes du TNF, antagonistes du CRF, antagonistes du CRF-BP, agonistes de l'urocortine, agonistes β3, antagonistes du récepteur CB1, antagonistes du MSH (hormone mélanostimulante), antagonistes du MCH, agonistes du CCK, inhibiteurs de la recapture de la sérotonine, composés sérotonines et noradrénergiques mélangés, modulateurs 5HT, agonistes de la bombésine, antagonistes de la galanine, hormones de croissance, composés libérant l'hormone de croissance, agonistes TRH, modulateurs de découplage de la protéine 2 ou 3, agonistes de la leptine, agonistes des DA (bromocriptine, doprexine), inhibiteurs de la lipase/amylase, modulateurs des récepteurs PPAR, modulateurs des RXR, ou agonistes du TR-β, ou amphétamines.

**12.** Utilisation des composés selon l'une ou plusieurs des revendications 1 à 7 pour fabriquer un médicament destiné au traitement du syndrome métabolique.

**13.** Utilisation des composés selon l'une ou plusieurs des revendications 1 à 7 pour fabriquer un médicament destiné au traitement du diabète.

**14.** Utilisation des composés selon l'une ou plusieurs des revendications 1 à 7 pour fabriquer un médicament destiné au traitement de l'adiposité.

**15.** Utilisation des composés selon l'une ou plusieurs des revendications 1 à 7 pour fabriquer un médicament destiné à la réduction du poids.

**16.** Utilisation des composés selon l'une ou plusieurs des revendications 1 à 7 pour fabriquer un médicament destiné au traitement de la dépendance à la nicotine.

**17.** Utilisation des composés selon l'une ou plusieurs des revendications 1 à 7 pour fabriquer un médicament destiné au traitement de la dépendance à l'alcool.

**18.** Utilisation des composés selon l'une ou plusieurs des revendications 1 à 7 pour fabriquer un médicament destiné au traitement de maladies du système nerveux central.

**19.** Utilisation des composés selon l'une ou plusieurs des revendications 1 à 7 pour fabriquer un médicament destiné au traitement de la schizophrénie.

**20.** Utilisation des composés selon l'une ou plusieurs des revendications 1 à 7 pour fabriquer un médicament destiné au traitement de la maladie d'Alzheimer.

**21.** Procédé de fabrication d'un médicament contenant un ou plusieurs des composés selon une ou plusieurs des revendications 1 à 7, **caractérisé en ce qu'**on mélange le principe actif à un support pharmaceutiquement approprié, et on met ce mélange sous une forme convenant à l'administration.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5411981 A
- US 20070010529 A
- WO 2005005477 A
- US 6221633 B
- WO 2007128815 A
- WO 2007128817 A
- WO 2008034881 A
- WO 2008049711 A
- WO 2008061355 A
- WO 9808871 A
- WO 2005027978 A
- WO 2006037811 A
- WO 2006037810 A
- WO 0104156 A
- WO 0034331 A
- WO 2006124529 A
- WO 2007124461 A
- WO 2008062457 A
- WO 2008082274 A
- WO 2008101017 A
- WO 2008081418 A
- WO 2008112939 A
- WO 2008112941 A
- WO 2008113601 A
- WO 2008116294 A
- WO 2008116648 A
- WO 2008119238 A
- WO 2007104789 A
- WO 2007120899 A
- WO 2008022015 A
- WO 2008056726 A
- WO 2006121860 A
- WO 2008021560 A
- WO 9726265 A
- WO 9903861 A
- US 2007264331 A
- WO 2008050987 A
- WO 2008062273 A
- WO 2007095462 A
- WO 2007101060 A
- WO 2007105650 A
- WO 2007137008 A
- WO 2008020607 A
- WO 9741097 A
- WO 2005086904 A
- WO 2007060992 A
- WO 2007100027 A
- WO 2007103252 A
- WO 2007122970 A
- WO 2007138485 A
- WO 2008006319 A
- WO 2008006969 A
- WO 2008010238 A
- WO 2008017398 A
- WO 2008028188 A
- WO 2008066356 A
- WO 2008084303 A
- WO 2008089461 A
- WO 2008089464 A
- WO 2008093639 A
- WO 2008096769 A
- WO 2008096820 A
- WO 2008096829 A
- US 2008194617 A
- WO 2008099944 A
- WO 2008108602 A
- WO 2008109334 A
- WO 2008126731 A
- WO 2008126732 A
- WO 2001040207 A
- WO 2002096894 A
- WO 2005097076 A
- WO 2007056771 A
- WO 2007087448 A
- WO 2007089667 A
- WO 2007089557 A
- WO 2007102515 A
- JP 2007246474 B
- WO 2007118963 A
- WO 2007118964 A
- WO 2007126043 A
- WO 2008006043 A
- WO 2008006044 A
- WO 2008012470 A
- WO 2008035359 A
- WO 2008087365 A
- WO 2008087366 A
- WO 2008087367 A
- WO 2008117982 A
- WO 0064888 A
- WO 0064876 A
- WO 03020269 A
- WO 2004024726 A
- WO 2007099553 A
- US 2007276041 A
- WO 2007085135 A
- WO 2007085136 A
- WO 2007141423 A

- WO 2008016175 A
- WO 2008053331 A
- WO 2008109697 A
- WO 2008109700 A
- WO 2008108735 A
- WO 2006059744 A
- WO 2006084176 A
- WO 2006029699 A
- WO 2007039172 A
- WO 2007039178 A
- WO 2007071766 A
- WO 2007101864 A
- US 2007244094 A
- WO 2007119887 A
- US 2008004281 A
- WO 20080713111 A
- WO 2008084962 A
- US 2008176861 A
- WO 2007114532 A
- WO 2007140230 A
- US 2007287674 A
- US 2008103201 A
- WO 2008065796 A
- WO 2008082017 A
- FR 258900
- WO 2003084922 A
- WO 2004007455 A
- WO 200507322931 A
- WO 2005067932 A
- WO 2008062739 A
- WO 2008099000 A
- WO 2008113760 A
- WO 2004100875 A
- WO 2005065680 A
- WO 2006086488 A
- WO 2007047177 A
- WO 2007106181 A
- WO 2007111864 A
- WO 2007120270 A
- WO 2007120284 A
- WO 2007123581 A
- WO 2007136577 A
- WO 2008042223 A
- WO 2008098244 A
- WO 2004063179 A
- WO 2004072031 A
- WO 2004072066 A
- WO 2005080360 A
- WO 2005044801 A
- WO 2006016194 A
- WO 2006058923 A
- WO 2006112549 A
- WO 2006125972 A
- WO 2007017549 A
- WO 2007417649 A
- WO 2007007910 A
- WO 200700704042 A
- WO 200700676061 A
- WO 2007006814 A
- WO 2007007886 A
- WO 2007028135 A
- WO 2007031739 A
- WO 2007041365 A
- WO 2007041366 A
- WO 2007037534 A
- WO 2007043638 A
- WO 2007053345 A
- WO 2007051846 A
- WO 2007051845 A
- WO 2007053765 A
- WO 2007051847 A
- WO 2007061923 A
- WO 2007075847 A
- WO 2007089512 A
- WO 2007104034 A
- WO 2007117381 A
- WO 2007122482 A
- WO 2007125103 A
- WO 2007125105 A
- US 2007281942 A
- WO 2008005914 A
- WO 2008005964 A
- WO 2008043701 A
- WO 2008044777 A
- WO 2008047821 A
- US 2008096877 A
- WO 20008050117 A
- WO 2008050101 A
- WO 2008059625 A
- US 2008146625 A
- WO 2008078674 A
- WO 2008079787 A
- WO 2008084043 A
- WO 2008084044 A
- WO 2008084872 A
- WO 2008089892 A
- WO 2008091770 A
- WO 2008075073 A
- WO 2008084873 A
- JP 2008189659 B
- WO 2008104994 A
- WO 2008111473 A
- WO 2008116107 A
- WO 2008118718 A
- WO 2008120754 A
- FR 225654
- WO 2008053446 A
- WO 2006023515 A
- WO 2006104030 A
- WO 2007014619 A
- WO 2007137962 A
- WO 2008019309 A
- WO 2008037628 A
- WO 2004101528 A
- WO 2003074500 A
- WO 2003106456 A

- WO 2004037169 A
- WO 200450658 A
- WO 2005037828 A
- WO 2005058901 A
- WO 2005012312 A
- WO 2005012308 A
- WO 2006039325 A
- WO 2006058064 A
- WO 2006015691 A
- WO 2006015701 A
- WO 2006015699 A
- WO 2006015700 A
- WO 2006018117 A
- WO 2006099943 A
- WO 2006099941 A
- JP 2006160733 B
- WO 2006071752 A
- WO 2006065826 A
- WO 2006078676 A
- WO 2006073167 A
- WO 2006068163 A
- WO 2006085685 A
- WO 2006090915 A
- WO 2006104356 A
- WO 2006127530 A
- WO 2006111261 A
- US 2006890898 A
- US 2006803357 A
- US 2006303661 A
- WO 2007015767 A
- WO 2007024993 A
- WO 2007029086 A
- WO 2007063928 A
- WO 2007070434 A
- WO 2007071738 A
- WO 2007071576 A
- WO 2007077508 A
- WO 2007087231 A
- WO 2007097931 A
- WO 2007099385 A
- WO 2007100374 A
- WO 2007112347 A
- WO 2007112669 A
- WO 2007113226 A
- WO 2007113634 A
- WO 2007115821 A
- WO 2007116092 A
- US 2007259900 A
- EP 1852108 A
- US 2007270492 A
- WO 2007126745 A
- WO 2007136603 A
- WO 2007142253 A
- WO 2007148185 A
- WO 2008017670 A
- US 2008051452 A
- WO 2008027273 A
- WO 2008028662 A
- WO 2008029217 A
- JP 2008031064 B
- JP 2008063256 B
- WO 2008033851 A
- WO 2008040974 A
- WO 2008040995 A
- WO 2008060488 A
- WO 2008064107 A
- WO 2008066070 A
- WO 2008077597 A
- JP 2008156318 B
- WO 2008087560 A
- WO 2008089636 A
- WO 2008093960 A
- WO 2008096841 A
- WO 2008141953 A
- WO 2008118848 A
- WO 2008119005 A
- WO 2008119208 A
- WO 2008120813 A
- WO 2008121506 A
- WO 2007128801 A
- WO 2003097064 A
- WO 2007026761 A
- WO 2008045484 A
- WO 2004007517 A
- WO 200452903 A
- WO 200452902 A
- EP 2005005959 W
- WO 2005085237 A
- JP 2004359630 B
- WO 2005121161 A
- WO 2006018150 A
- WO 2006035796 A
- WO 2006062224 A
- WO 2006058597 A
- WO 2006073197 A
- WO 2006080577 A
- WO 2006087997 A
- WO 2006108842 A
- WO 2007000445 A
- WO 2007014895 A
- WO 2007080170 A
- WO 2007093610 A
- WO 2007126117 A
- WO 2007128480 A
- WO 2007129668 A
- US 2007275907 A
- WO 2007136116 A
- WO 2007143316 A
- WO 2007147478 A
- WO 2008001864 A
- WO 2008002824 A
- WO 2008013277 A
- WO 2008013280 A
- WO 2008013321 A
- WO 2008013322 A
- WO 2008016132 A

- WO 2008020011 A
- JP 2008031161 B
- WO 2008034859 A
- WO 2008042688 A
- WO 2008044762 A
- WO 2008046497 A
- WO 2008049923 A
- WO 2008055870 A
- WO 2008055940 A
- WO 2008069327 A
- WO 2008070609 A
- WO 2008071288 A
- WO 2008072726 A
- WO 2008083200 A
- WO 2008090209 A
- WO 2008090210 A
- WO 2008101586 A
- WO 2008101939 A
- WO 2008116179 A
- WO 2008116195 A
- US 2008242596 A
- WO 20019009094 A
- WO 200343999 A
- WO 2004112782 A
- WO 200344000 A
- WO 200344009 A
- WO 2004112779 A
- WO 2004113310 A
- WO 2004103980 A
- WO 2004112784 A
- WO 2003065983 A
- WO 2003104207 A
- WO 2003104208 A
- WO 2004106294 A
- WO 2004011410 A
- WO 2004033427 A
- WO 2004041264 A
- WO 2004037251 A
- WO 2004056744 A
- WO 2004058730 A
- WO 2004065351 A
- WO 2004089367 A
- WO 2004089380 A
- WO 200408947071 A
- WO 2004089896 A
- WO 2005016877 A
- WO 2005063247 A
- WO 2005097759 A
- WO 2006010546 A
- WO 2006012227 A
- WO 2006012173 A
- WO 2006017542 A
- WO 2006034804 A
- WO 2006040329 A
- WO 2006051662 A
- WO 2006048750 A
- WO 2006049952 A
- WO 2006048331 A
- WO 2006050908 A
- WO 2006024627 A
- WO 2006066109 A
- WO 2006074244 A
- WO 2006078006 A
- WO 2006106423 A
- WO 2006132436 A
- WO 2006134481 A
- WO 2006134467 A
- WO 2006135795 A
- WO 2006136502 A
- WO 2006138508 A
- WO 2006138695 A
- WO 2006133926 A
- WO 2007003521 A
- WO 2007007688 A
- US 2007066584 A
- WO 2007029021 A
- WO 2007047625 A
- WO 2007051811 A
- WO 2007051810 A
- WO 2007057768 A
- WO 2007058346 A
- WO 2007061661 A
- WO 2007068330 A
- WO 2007070506 A
- WO 2007087150 A
- WO 2007092435 A
- WO 2007089683 A
- WO 2007101270 A
- WO 2007105753 A
- WO 2007107470 A
- WO 2007107550 A
- WO 2007111921 A
- US 2007207985 A
- US 2007208001 A
- WO 2007115935 A
- WO 2007118185 A
- WO 2007122411 A
- WO 2007124329 A
- WO 2007124337 A
- WO 2007124254 A
- WO 2007127688 A
- WO 2007127693 A
- WO 2007127704 A
- WO 2007127726 A
- WO 2007127763 A
- WO 2007127765 A
- WO 2007127901 A
- US 2007270424 A
- JP 2007291075 B
- WO 2007130898 A
- WO 2007135427 A
- WO 2007139992 A
- WO 2007144394 A
- WO 2007145834 A
- WO 2007145835 A
- WO 2007146761 A

- WO 2008000950 A
- WO 2008000951 A
- WO 2008003611 A
- WO 2008005910 A
- WO 2008006702 A
- WO 2008006703 A
- WO 2008011453 A
- WO 2008012532 A
- WO 2008024497 A
- WO 2008024892 A
- WO 2008032164 A
- WO 2008034032 A
- WO 2008043544 A
- WO 2008044656 A
- WO 2008046758 A
- WO 2008052638 A
- WO 2008053194 A
- WO 2008071169 A
- WO 2008074384 A
- WO 2008076336 A
- WO 2008076862 A
- WO 2008078725 A
- WO 2008087654 A
- WO 2008088540 A
- WO 2008099145 A
- WO 2008101885 A
- WO 2008101886 A
- WO 2008101907 A
- WO 2008101914 A
- WO 2008106128 A
- WO 2008110196 A
- WO 2008119017 A
- WO 2008120655 A
- WO 2008127924 A
- WO 20011983031 A
- WO 200117516 A
- WO 2004506446 A
- WO 2005012295 A
- WO 2005116003 A
- WO 2006007959 A
- DE 102004060542
- WO 2007009911 A
- WO 2007028145 A
- WO 2007067612 A
- WO 2007081755 A
- WO 2007115058 A
- US 2008004325 A
- WO 2008033455 A
- WO 2008033931 A
- WO 2008033932 A
- WO 2008033934 A
- WO 2008089581 A
- WO 2004041274 A
- WO 2006045565 A
- WO 2006045564 A
- WO 2006069242 A
- WO 02006085108 A
- WO 2006085112 A
- WO 2006085113 A
- WO 2006124490 A
- WO 2006113150 A
- WO 2007017261 A
- WO 2007017262 A
- WO 2007017265 A
- WO 2007015744 A
- WO 2007027532 A
- WO 2007092364 A
- WO 2007120575 A
- WO 2007134986 A
- WO 2007150025 A
- WO 2007150026 A
- WO 2008016968 A
- WO 2008051403 A
- WO 2008086949 A
- WO 2008091338 A
- WO 2008097535 A
- WO 2008099448 A
- US 2008234277 A
- WO 2008127591 A
- WO 2008039882 A
- WO 2006067531 A
- WO 2006067532 A
- WO 2007013689 A
- WO 2007033002 A
- WO 2007106469 A
- US 2007265332 A
- WO 2007123225 A
- WO 2007131619 A
- WO 2007131620 A
- WO 2007131621 A
- WO 2007131622 A
- WO 2007136572 A
- WO 2008001931 A
- WO 2008030520 A
- WO 2008030618 A
- WO 2008054674 A
- WO 2008054675 A
- WO 2008066097 A
- US 2008176912 A
- WO 2004065380 A
- WO 2005061489 A
- WO 2006083491 A
- WO 200700396062 A
- WO 2007003964 A
- WO 2007035355 A
- WO 2007116229 A
- WO 2007116230 A
- WO 2008005569 A
- WO 2008005576 A
- WO 2008008887 A
- WO 2008008895 A
- WO 2008025798 A
- WO 2008025799 A
- WO 2008025800 A
- WO 2008070692 A
- WO 2008076243 A

- WO 200807692 A
- WO 2008081204 A
- WO 2008081205 A
- WO 2008081206 A
- WO 2008081207 A
- WO 2008081208 A
- WO 2008083238 A
- WO 2008085316 A
- WO 2008109702 A
- EP 1688138 A
- WO 2008066131 A
- WO 2008103500 A
- WO 2008103501 A
- WO 2005073199 A
- WO 2006074957 A
- WO 2006087309 A
- WO 2006111321 A
- WO 2007042178 A
- WO 2007119837 A
- WO 2008122352 A
- WO 2008122357 A
- WO 2007110216 A
- WO 2008048866 A
- WO 20080488867 A
- WO 2007119827 A
- US 2005222220 A
- WO 2005085230 A
- WO 2005111018 A
- WO 2003078403 A
- WO 2004022544 A
- WO 2003106410 A
- WO 2005058908 A
- US 2005038023 A
- WO 2005009997 A
- US 2005026984 A
- WO 2005000836 A
- WO 2004106343 A
- EP 1460075 A
- WO 2004014910 A
- WO 2003076442 A
- WO 2005087727 A
- WO 2004046117 A
- WO 2007073117 A
- WO 2007083978 A
- WO 2007120102 A
- WO 2007122634 A
- WO 2007125109 A
- WO 2007125110 A
- US 2007281949 A
- WO 2008002244 A
- WO 2008002245 A
- WO 2008016123 A
- WO 2008023239 A
- WO 2008044700 A
- WO 2008056266 A
- WO 2008057940 A
- WO 2008077138 A
- EP 1939191 A
- EP 1939192 A
- WO 2008078196 A
- WO 2008094992 A
- WO 2008112642 A
- WO 2008112651 A
- WO 2008113469 A
- WO 2008121063 A
- WO 2008121064 A
- WO 2004074288 A
- WO 2008027584 A
- WO 2008070150 A
- WO 2008125833 A
- WO 2008125835 A
- WO 2008125839 A
- WO 2006072354 A
- WO 2007093264 A
- WO 2008009335 A
- WO 2008086854 A
- WO 2008057855 A
- WO 2008057856 A
- WO 2008057857 A
- WO 2008057859 A
- WO 2008057862 A
- WO 2008059867 A
- WO 2008059866 A
- WO 2008059865 A
- WO 2008070507 A
- WO 2008124665 A
- WO 2008124745 A
- WO 2008104306 A
- WO 2008119918 A
- WO 2008096260 A
- WO 2008125945 A
- WO 2008088006 A
- WO 2007062568 A
- WO 2008006432 A
- WO 2008016278 A
- WO 2008016730 A
- WO 2008083124 A
- WO 2007112914 A
- WO 2007149865 A
- WO 2007104053 A
- WO 2007115822 A
- WO 2008008547 A
- WO 2008075741 A
- WO 2001000610 A
- WO 2001030774 A
- WO 2004022057 A
- WO 2004022553 A
- WO 2005097129 A
- WO 2005113544 A
- US 2007244140 A
- WO 2008099072 A
- WO 2008099073 A
- WO 2008099074 A
- WO 2008099075 A
- WO 2008016131 A
- US 2007249583 A

- WO 2008083551 A
- WO 2003099821 A
- WO 2005056554 A
- WO 2007052843 A
- WO 2007070796 A
- WO 2007092751 A
- JP 2007230909 B
- WO 2007095174 A
- WO 2007140174 A
- WO 2007140183 A
- WO 2008000643 A
- WO 2008002573 A
- WO 2008025539 A
- WO 2008025540 A
- JP 2008214222 B
- WO 2007092965 A
- WO 2008041003 A
- WO 2008049047 A
- WO 2008065754 A
- WO 2008073825 A
- US 2008242677 A
- WO 2008093655 A
- WO 2005042692 A
- WO 2005005453 A
- WO 2005021497 A
- WO 2005021495 A
- WO 2002066464 A
- WO 2005000353 A
- WO 2005044256 A
- WO 2005062824 A
- WO 2005061451 A
- WO 2005061452 A
- WO 2006017257 A
- WO 2005033100 A
- WO 2002050060 A
- WO 2002050068 A
- WO 2004000803 A
- WO 2004000804 A
- WO 2004000805 A
- WO 2004087655 A
- WO 2004097655 A
- WO 2005047248 A
- WO 2006086562 A
- WO 2006102674 A
- WO 2006116499 A
- WO 2006121861 A
- WO 2006122186 A
- WO 2006122216 A
- WO 2006127893 A
- WO 2006137794 A
- WO 2006137796 A
- WO 2006137782 A
- WO 2006137793 A
- WO 2006137797 A
- WO 2006137795 A
- WO 2006137792 A
- WO 2006138163 A
- WO 2007059871 A
- US 2007232688 A
- WO 2007126358 A
- WO 2008033431 A
- WO 2008033465 A
- WO 2008052658 A
- WO 2008057336 A
- WO 2008085300 A
- WO 2008033464 A
- US 6992067 B
- US 7205290 B
- WO 2006002342 A
- WO 2006010422 A
- WO 2006012093 A
- WO 2006073973 A
- WO 2006072362 A
- WO 2007088996 A
- WO 2007088999 A
- US 2007185058 A
- US 2007185113 A
- US 2007185154 A
- US 2007185182 A
- WO 2006097169 A
- WO 2007041494 A
- WO 2007090752 A
- WO 2007107243 A
- WO 2007120621 A
- US 2007265252 A
- US 2007265304 A
- WO 2007128568 A
- WO 2007132906 A
- WO 2008006257 A
- WO 2008009435 A
- WO 2008018529 A
- WO 2008058961 A
- WO 2008058967 A
- WO 2008059513 A
- WO 2008070496 A
- WO 2008115442 A
- WO 2008111604 A
- US 6245744 B
- US 6221897 B
- WO 0061568 A
- DE 102005033099
- DE 102005033100
- DE 102006053635
- DE 102006053637
- WO 200700965556 A
- WO 2008058628 A
- WO 2008058629 A
- WO 2008058630 A
- WO 2008058631 A
- US 20060199795 A
- WO 2007110237 A
- WO 2007127505 A
- WO 2008009407 A
- WO 2008067219 A
- WO 2008067222 A
- FR 2908310

- WO 2008091540 A
- WO 2008097976 A
- WO 2008097504 A
- WO 2005085226 A
- WO 2005121091 A
- WO 2006010423 A
- WO 2006113910 A
- WO 2007143164 A
- WO 2008049806 A
- WO 2008049808 A
- WO 2008090198 A
- WO 2008100423 A
- WO 2008030382 A
- WO 2008079398 A
- WO 2008032980 A
- WO 2006094682 A
- WO 2008087029 A
- WO 2008087030 A
- WO 2008095189 A
- WO 2007063012 A
- WO 2007096251 A
- WO 2008015081 A
- US 2008103182 A
- WO 2008074692 A
- WO 2008031032 A
- WO 2008046071 A
- WO 2008083280 A
- WO 2008084300 A
- WO 2005077907 A
- JP 2007022943 B
- WO 2008003424 A
- WO 2008092231 A
- US 6342512 B
- WO 2005097738 A
- WO 2008040651 A
- WO 2008099278 A
- WO 2006072393 A
- WO 2008062830 A
- EP 1258247 A
- EP 1375508 A
- WO 2008028590 A
- WO 2008077050 A
- WO 2007111954 A
- WO 2007121918 A
- WO 2007121921 A
- WO 2007121923 A
- WO 2008070661 A
- WO 2008064788 A
- WO 2008064789 A
- US 2007270433 A
- WO 2008027585 A
- WO 2008080461 A
- WO 199946262 A
- WO 200372197 A
- WO 2003072197 A
- WO 2005044814 A
- WO 2005108370 A
- JP 2006131559 B
- WO 2007011809 A
- WO 2007011811 A
- WO 2007013691 A
- WO 2007095601603 A
- WO 2007119833 A
- WO 2008065508 A
- WO 2008069500 A
- WO 2008072850 A
- WO 2008079610 A
- WO 2008088688 A
- WO 2008088689 A
- WO 2008088692 A
- US 2008171761 A
- WO 2008090944 A
- JP 2008179621 B
- US 2008200461 A
- WO 2008102749 A
- WO 2008103382 A
- WO 2008121592 A
- WO 2007100789 A
- WO 20070833 A
- WO 2008051873 A
- WO 2008051875 A
- WO 2008073623 A
- WO 2008094869 A
- WO 2008112022 A
- WO 2006001318 A
- WO 2007103295 A
- WO 2007125952 A
- WO 2008026563 A
- WO 2008026564 A
- WO 2008052769 A
- WO 2008092887 A
- WO 2008092888 A
- WO 2008092891 A
- WO 2007038942 A
- WO 2007038943 A
- WO 2005080424 A
- WO 2006095166 A
- WO 2008003947 A
- WO 2006096847 A
- EP 0656354 A
- WO 0015609 A
- WO 20016463264634 A
- WO 02076949 A
- WO 2005080345 A
- WO 2005080328 A
- WO 2005080343 A
- WO 2005075450 A
- WO 2005080357 A
- WO 200170700 A
- WO 200302664748 A
- WO 200302776 A
- WO 2003040107 A
- WO 2003007887 A
- WO 2003027069 A
- US 6509367 B
- WO 200132663 A

- WO 2003086288 A
- WO 2003087037 A
- WO 2004048317 A
- WO 2004058145 A
- WO 2003084930 A
- WO 2003084943 A
- WO 2004058744 A
- WO 2004013120 A
- WO 2004029204 A
- WO 2004035566 A
- WO 2004058249 A
- WO 2004058255 A
- WO 2004058727 A
- WO 2004069838 A
- US 20040214837 A
- US 20040214855 A
- US 20040214856 A
- WO 2004096209 A
- WO 2004096763 A
- WO 2004096794 A
- WO 2005000809 A
- WO 2004099157 A
- US 20040266845 A
- WO 2004110453 A
- WO 2004108728 A
- WO 2004000817 A
- WO 2005000820 A
- US 20050009870 A
- WO 200500974 A
- WO 200411103334 A
- WO 20041103839 A
- WO 2005016286 A
- WO 2005007111 A
- WO 2005007628 A
- US 20050054679 A
- WO 2005027837 A
- WO 2005028456 A
- WO 200506376162 A
- WO 2005061509 A
- WO 2005077897 A
- WO 2006018662 A
- WO 2006047516 A
- WO 2006060461 A
- WO 2006067428 A
- WO 2006067443 A
- WO 2006087480 A
- WO 2006087476 A
- WO 2006100208 A
- WO 2006106054 A
- WO 2006111849 A
- WO 2006113704 A
- WO 2007009705 A
- WO 2007017124 A
- WO 2007017126 A
- WO 2007018459 A
- WO 2007018460 A
- WO 2007016460 A
- WO 2007020502 A
- WO 2007026215 A
- WO 2007028849 A
- WO 2007031720 A
- WO 2007031721 A
- WO 2007036945 A
- WO 2007038045 A
- WO 2007039740 A
- US 20070015810 A
- WO 2007046548 A
- WO 2007047737 A
- WO 2007057687 A
- WO 2007062193 A
- WO 2007064272 A
- WO 2007079681 A
- WO 2007084319 A
- WO 2007084450 A
- WO 2007086080 A
- EP 1816125 A
- US 2007213302 A
- WO 2007095513 A
- WO 2007096764 A
- US 2007254863 A
- WO 2007119001 A
- WO 2007120454 A
- WO 2007121687 A
- WO 2007123949 A
- US 2007259934 A
- WO 2007131219 A
- WO 2007133820 A
- WO 2007136571 A
- WO 2007136607 A
- US 7297710 B
- WO 2007138050 A
- WO 2007139464 A
- WO 2007140385 A
- WO 2007140439 A
- WO 2007148061 A
- WO 2007148062 A
- US 2007293509 A
- WO 2008004698 A
- WO 2008017381 A
- US 2008021031 A
- WO 2008024284 A
- WO 2008031734 A
- WO 2008035356 A
- WO 2008036021 A
- WO 2008036022 A
- WO 2008039023 A
- WO 2998043544 A
- WO 2008044111 A
- WO 2008048648 A
- EP 1921072 A1
- WO 2008053341 A
- WO 2008056377 A
- WO 2008059207 A
- WO 2008059335 A
- WO 2008062424 A
- WO 2008068423 A

- WO 2008068424 A
- WO 2008070305 A
- WO 2008070306 A
- WO 2008074816 A
- WO 2008074982 A
- WO 2008075012 A
- WO 2008075013 A
- WO 2008075019 A
- WO 2008075118 A
- WO 2008076754 A
- WO 2008081009 A
- WO 2008084057 A
- EP 1944295 A
- US 2008090809 A
- US 2008090810 A
- WO 2008092816 A
- WO 2008094473 A
- WO 2008094476 A
- WO 2008099076 A
- WO 2008099139 A
- WO 02008101995 A
- US 2008207704 A
- WO 2008107179 A
- WO 2008109027 A
- WO 2008112674 A
- WO 2008115705 A
- WO 2008118414 A
- WO 2008119999 A
- WO 200812000 A
- WO 2008121257 A
- WO 2008127585 A
- WO 2007001939 A
- WO 2007044215 A
- WO 2007112399 A
- WO 2007112402 A
- WO 2008122618 A
- WO 2007140005 A
- WO 2008019357 A
- WO 2008021625 A
- WO 2008023720 A
- WO 2008030532 A
- WO 2008057585 A
- WO 2008059214 A
- WO 2008075064 A
- WO 2008075070 A
- WO 2008075077 A
- WO 2008120653 A
- WO 2007091948 A
- WO 2007129188 A
- WO 2007133637 A
- WO 2008007780 A
- WO 2008010061 A
- WO 2008007211 A
- WO 2008015335 A
- WO 2008018827 A
- WO 2008024433 A
- WO 2008024438 A
- WO 2008032204 A
- WO 2008050199 A
- WO 2008059339 A
- WO 2008059370 A
- WO 2008066664 A
- WO 20080755150 A
- WO 2008090382 A
- WO 2008090434 A
- WO 2008093024 A
- WO 2008107543 A
- WO 2008107544 A
- WO 2008110863 A
- WO 2007047397 A
- WO 2008021849 A
- WO 2008021851 A
- WO 2008032156 A
- US 2008249122 A
- WO 2008089201 A
- WO 2007111806 A
- WO 0191752 A
- WO 2005060985 A
- WO 2005009950 A
- WO 2004087159 A
- WO 2004078717 A
- WO 2004078716 A
- WO 2004024720 A
- US 20050124652 A
- WO 2005051391 A
- WO 2004112793 A
- WO US20050222014 A
- US 20050176728 A
- US 20050164914 A
- US 20050124636 A
- US 20050130988 A
- US 20044167201 A
- WO 2004005324 A
- WO 2004037797 A
- WO 2005042516 A
- WO 2005040109 A
- WO 2005030797 A
- US 20040224901 A
- WO 200501921 A
- WO 200509184 A
- WO 2005000339 A
- EP 1460069 A
- WO 2005047253 A
- WO 2005047251 A
- WO 2005118573 A
- EP 1538159 A
- WO 2004072076 A
- WO 2004072077 A
- WO 200602165557 A
- WO 2007009894 A
- WO 2007015162 A
- WO 2007041061 A
- WO 2007041052 A
- JP 2007131570 B
- EP 1842846 A
- WO 2007096186 A

- WO 2007096763 A
- WO 2007141343 A
- WO 2008007930 A
- WO 2008017852 A
- WO 2008039418 A
- WO 2008087186 A
- WO 2008087187 A
- WO 2008087189 A
- WO 2008087190 A
- WO 2008090357 A
- WO 200196302 A
- WO 200185693 A
- WO 2004085403 A
- WO 2005075458 A
- WO 2006067224 A
- WO 2007085718 A
- WO 2007088276 A
- WO 2007116374 A
- WO 2007122591 A
- WO 2007126934 A
- WO 2007126935 A
- WO 2008008517 A
- WO 2008008518 A
- WO 2008008551 A
- WO 2008020405 A
- WO 2008026149 A
- WO 2008038251 A
- US 2008132490 A
- WO 2008065626 A
- WO 2008078291 A
- WO 2008087611 A
- WO 2008081399 A
- WO 2008108991 A
- WO 2008107335 A
- US 2008249125 A
- WO 0063208 A
- WO 200064884 A
- WO 2005082893 A
- US 2005171181 A
- WO 2006107661 A
- WO 2007003804 A
- WO 2007016496 A
- WO 2007020213 A
- WO 2007049798 A
- WO 2007055418 A
- WO 2007057329 A
- WO 2007065820 A
- WO 2007068620 A
- WO 2007068641 A
- WO 2007075629 A
- WO 2007080140 A
- WO 2007082840 A
- WO 2007088450 A
- WO 2007088462 A
- WO 2007094962 A
- WO 2007099423 A
- WO 2007100990 A
- WO 2007105053 A
- WO 2007106349 A
- WO 2007110364 A
- WO 2007115938 A
- WO 2007131907 A
- WO 2007133561 A
- US 2007270440 A
- WO 2007135111 A
- WO 2007137955 A
- US 2007281923 A
- WO 2007137968 A
- WO 2007138431 A
- WO 2007146122 A
- WO 2008005338 A
- WO 2008012010 A
- WO 2008015125 A
- WO 2008045371 A
- EP 1757594 A
- WO 2008068173 A
- WO 2008068174 A
- US 20080171753 A
- WO 2008072703 A
- WO 2008072724 A
- US 2008188484 A
- US 2008188486 A
- US 2008188487 A
- WO 2008109333 A
- WO 2008109336 A
- WO 2008002816 A
- WO 2008002817 A
- WO 2008002818 A
- WO 2008002820 A
- WO 2007117399 A
- WO 0066585 A
- WO 2007105113 A
- WO 2007133756 A
- WO 2008036541 A
- WO 2008036579 A
- WO 2008083070 A
- WO 0183451 A
- JP 2006111553 B
- WO 2002038543 A
- WO 2002038544 A
- WO 2007048840843 A
- WO 2008015558 A
- EP 1947103 A
- WO 2005085200 A
- WO 2005019240 A
- WO 2004011438 A
- WO 2004012648 A
- WO 2003015769 A
- WO 2004072025 A
- WO 2005070898 A
- WO 2005070925 A
- WO 2004039780 A
- WO 2004092181 A
- WO 2003033476 A
- WO 2002006245 A
- WO 2002089729 A

- WO 2002002744 A
- WO 2003004027 A
- FR 2868780
- WO 2006010446 A
- WO 2006038680 A
- WO 2006044293 A
- WO 2006044174 A
- JP 2006176443 B
- WO 2006018280 A
- WO 2006018279 A
- WO 2006118320 A
- WO 2006130075 A
- WO 2007018248 A
- WO 2007012661 A
- WO 2007029847 A
- WO 2007024004 A
- WO 2007039462 A
- WO 2007042660 A
- WO 2007042668 A
- WO 2007042669 A
- US 2007093508 A
- US 2007093509 A
- WO 2007048802 A
- JP 2007091649 B
- WO 2007092416 A
- WO 2007093363366 A
- WO 2007114902 A
- WO 2007114916 A
- WO 2007141200 A
- WO 2007142217 A
- US 2007299062 A
- WO 2007146758 A
- WO 2007146759 A
- WO 2008001160 A
- WO 2008016811 A
- WO 2008020799 A
- WO 2008022979 A
- WO 2008038692 A
- WO 2008041090 A
- WO 2008044632 A
- WO 2008047544 A
- WO 2008061109 A
- WO 2008065021 A
- WO 2008068265 A
- WO 2008071646 A
- WO 2008076562 A
- JP 2008088120 B
- WO 2008086404 A
- WO 2008086409 A
- WO 9915525 A
- WO 0244150 A
- WO 2005116034 A
- WO 2007120655 A
- WO 2007120688 A
- WO 2007120718 A
- WO 2008091631 A
- WO 2007148341 A
- WO 2008034142 A
- WO 2008081477 A
- WO 2008120761 A
- WO 2008063673 A
- WO 0071549 A
- WO 0109111 A
- WO 2006085118 A
- WO 2008079838 A
- WO 2008079839 A
- WO 2008079847 A
- WO 2008079848 A
- US 2008076724 A
- WO 2007138343 A
- WO 200077010 A
- WO 20007700102 A
- WO 2005019180 A
- WO 2003064423 A
- WO 200242304 A
- WO 2005035533 A
- WO 2005082859 A
- WO 2006004937 A
- US 2006025601 A
- WO 2006028961 A
- WO 2006077025 A
- WO 2006103511 A
- WO 2007028132 A
- WO 2007084622 A
- US 2007249709 A
- WO 2007132841 A
- WO 2007140213 A
- WO 2008007661 A
- WO 2008007664 A
- WO 2008009125 A
- WO 2008010073 A
- WO 2008108445 A
- WO 2005058858 A
- WO 2007054257 A
- WO 2007107373 A
- WO 2007108569 A
- WO 2007108742744 A
- WO 2008003703 A
- WO 2008027073 A
- WO 2008034815 A
- WO 2008054288 A
- EP 1947085 A
- WO 2008084491 A
- WO 2008084492 A
- WO 2008092665 A
- WO 2008092666 A
- WO 2008101247 A
- WO 2008110598 A
- WO 2008116831 A
- WO 2008116833 A
- WO 2007131005 A
- WO 2008052709 A
- WO 2008109727 A
- WO 2007098953 A
- WO 2007098961 A
- WO 2008015266 A

- WO 2008055932 A
- WO 2008055933 A
- WO 2007110782 A
- WO 2008041184 A
- WO 2008051404 A
- WO 2008051405 A
- WO 2008051406 A
- WO 2008073311 A
- WO 0185695 A
- WO 2005030734 A
- WO 2007127457 A
- WO 2008008286 A
- WO 2006012577 A
- WO 2007079239 A
- WO 2008092681 A
- EP 0462884 A
- WO 2008059023 A
- WO 2008059024 A
- WO 2008059025 A
- WO 2008059026 A
- WO 0040569 A
- WO 2008107184 A
- US 20040224997 A
- WO 2004094618 A
- WO 200058491 A
- WO 2005044250 A
- WO 2005072740 A
- JP 2005206492 B
- WO 2005013907 A
- WO 2006004200 A
- WO 2006019020 A
- WO 2006064189 A
- WO 2006082952 A
- WO 2006120125 A
- WO 2006113919 A
- WO 2006134317 A
- WO 2007016538 A
- WO 2007060140 A
- JP 2007131584 B
- WO 2007071966 A
- WO 2007126957 A
- WO 2007137103 A
- WO 2007137107 A
- WO 2007138304 A
- WO 2007138311 A
- WO 2007141502 A
- WO 2007141517 A
- WO 2007141538 A
- WO 2007141545 A
- WO 2007144571 A
- WO 2008011130 A
- WO 2008011131 A
- WO 2008039007 A
- WO 2008048991 A
- WO 2008067257 A
- WO 2008099221 A
- WO 2008038768 A
- WO 2004005277 A
- WO 2008006113 A
- WO 2007009236 A
- WO 2007044085 A
- WO 2007046867 A
- WO 2007046868 A
- WO 20070501124 A
- WO 2007056846 A
- WO 2007071023 A
- WO 2007130075 A
- WO 2007134457 A
- WO 2007136746 A
- WO 2007143597 A
- WO 2007143823 A
- WO 2007143824 A
- WO 2008003753 A
- WO 2008017161 A
- WO 2008024390 A
- WO 2008029266 A
- WO 2008036715 A
- WO 2008043087 A
- WO 2008044767 A
- WO 2008046226 A
- WO 2008056687 A
- WO 2008062276 A
- WO 2008064474 A
- WO 2008074824 A
- WO 2008074832 A
- WO 2008074833 A
- WO 2008074834 A
- WO 2008074835 A
- WO 2008089580 A
- WO 2008096746 A
- WO 2008104524 A
- WO 2008116898 A
- US 2008249100 A
- WO 2008120744 A
- WO 2008120759 A
- WO 2008123469 A
- WO 2008127349 A
- WO 2008089310 A
- WO 2008039087 A
- WO 2006082978 A
- WO 2008105533 A
- WO 2007125946 A
- WO 2008038712 A
- WO 2008121009 A
- WO 20058279 A
- WO 200172692 A
- WO 200194293 A
- WO 2003084915 A
- WO 2004018421 A
- WO 2005092316 A
- WO 2007003419 A
- WO 2007009913 A
- WO 2007039125 A
- WO 2007110225 A
- WO 2007110226 A
- WO 2007128492 A

- WO 2007132475 A
- WO 2007134864 A
- WO 2008001959 A
- WO 2008106213 A
- WO 2008062469 A
- US 2008146523 A
- WO 2008092785 A
- WO 2008067270 A
- WO 2007065948 A
- WO 2008027557 A
- EP 1886695 A
- WO 2008119744 A
- WO 2007119463 A
- WO 2007125405 A
- WO 2008028860 A
- WO 2008118626 A
- WO 2005090336 A
- WO 2006071609 A
- WO 2006135826 A
- WO 2007105766 A
- WO 2008120661 A
- WO 2007019416 A
- WO 2008073451 A
- WO 2007107587 A
- WO 2007111994 A
- WO 2008106600 A
- WO 2008113796 A
- WO 2008097835 A
- WO 2007101146 A
- WO 2007133828 A
- WO 2007112069 A
- WO 2007099200 A
- WO 2007137874 A
- JP 2008024673 B
- WO 2007100104 A
- JP 2008106008 B
- US 7138107 B
- WO 2008028958 A
- WO 2008085711 A
- WO 2008033447 A
- WO 2008033356 A
- WO 2008033460 A
- WO 2008033468 A
- WO 2008073461 A
- WO 2008073934 A
- WO 2008073936 A
- WO 2008110008 A
- US 2008027049 A
- US 2008027090 A
- WO 2008040057 A
- WO 2008040058 A
- WO 2008046065 A
- WO 2008014360 A
- WO 2008014381 A
- WO 2008019967 A
- US 2008064697 A
- US 2008249101 A
- WO 2008000692 A
- WO 2008051272 A
- WO 2008042800 A
- WO 2008035305 A
- WO 2008035306 A
- WO 2008035686 A
- WO 2008036966 A
- WO 2008036967 A
- WO 2008058641 A
- WO 2008074413 A
- WO 2008062905 A
- WO 2008067378 A
- WO 2008064315 A
- WO 2008074820 A
- WO 2008074821 A
- WO 2008044770 A
- WO 2008090200 A
- WO 2008092091 A
- US 2008194658 A

**In der Beschreibung aufgeführte Nicht-Patentliteratur**

- *Behavioural Pharmacology,* 2005, vol. 16, 275-296
- **Ken Mackie.** *Annu. Rev. Pharmacol. Toxicol.,* 2006, vol. 46, 101-122
- **S. C. Black.** *Curr. Opin. Investig. Drugs,* 2004, vol. 5, 389-394
- **V. Di Marzio et al.** *Nat. Rev. Drug Discov.,* 2004, vol. 3, 771-784
- **B. Le Foll et al.** *J. Pharmacol. Exp. Ther.,* 2005, vol. 312, 875-883
- **L. Walter et al.** *Br. J. Pharmacol.,* 2004, vol. 141, 775-785
- *Pharmaceutical Research,* 1986, vol. 2 (6), 318
- **D. Chen et al.** *Proc. Natl. Acad. Sci. USA,* 2007, vol. 104, 943
- **R. D. Carr et al.** *Diabetes,* 2003, vol. 52, 2513.2518
- **J. B. Hansen et al.** *Current Medicinal Chemistry,* 2004, vol. 11, 1595-1615
- **T. M. Tagmose et al.** *J. Med. Chem.,* 2004, vol. 47, 3202-3211
- **M. J. Coghlan et al.** *J. Med. Chem.,* 2001, vol. 44, 1627-1653
- **J.P.Berger et al.** *TRENDS in Pharmacological Sciences,* 2005, vol. 28 (5), 244-251
- **von A. L. Handlon.** *Expert Opin. Ther. Patents,* 2005, vol. 15 (11), 1531-1540
- **Asakawa, A. et al.** Cocaine-amphetamine-regulated transcript influences energy metabolism, anxiety and gastric emptying in mice. *Hormone and Metabolic Research,* 2001, vol. 33 (9), 554-558

- **Lee, Daniel W. ; Leinung, Matthew C. ; Rozhavskaya-Arena, Marina ; Grasso, Patricia.** Leptin agonists as a potential approach to the treatment of obesity. *Drugs of the Future,* 2001, vol. 26 (9), 873-881
- **Salvador, Javier ; Gomez-Ambrosi, Javier ; Fruhbeck, Gema.** Perspectives in the therapeutic use of leptin. *Expert Opinion on Pharmacotherapy,* 2001, vol. 2 (10), 1615-1622
- **Zunft H J et al.** Carob pulp preparation for treatment of hypercholesterolemia. *ADVANCES IN THERAPY,* September 2001, vol. 18 (5), 230-6
- **S.L.Buchwald et al.** *Acc. Chem. Res.,* 1998, vol. 31, 805
- **J.F.Hartwig et al.** *J. Org. Chem.,* 1999, vol. 64, 5575-5580
- **J.P.Wolfe et al.** *J. Org. Chem.,* 2000, vol. 65, 144-1157
- **M.D.Charles et al.** *Org. Lett.,* 2005, vol. 7, 3965-68
- **J.-B.Lan et al.** *SYNLETT,* 2004, 1095-1097
- **D.M.T.Chan et al.** *Tetrahedron Lett.,* 1998, vol. 39, 2933-2936
- **J. Zhou ; G. C. Fu.** *J. Am. Chem. Soc.,* 2004, vol. 126, 1340-1341
- **F. Gonzäles-Bobes ; G. C. Fu.** *J. Am. Chem. Soc.,* 2006, vol. 128, 5360-5361
- **D. J. Wallace ; C.-Y. Chen.** *Tetrahedron Letters,* 2002, vol. 43, 6987-6990
- **T. E. Barder et al.** *J. Am. Chem. Soc.,* 2005, vol. 127, 4685-4696
- **D. W. Old et al.** *J. Am. Chem. Soc.,* 1998, vol. 120, 9722
- **T. E. Barder ; St. L. Buchwald.** *Org. Lett.,* 2004, vol. 6, 2649-2652
- **R. Frlan ; D. Kikelj.** *Synthesis,* 2006, vol. 14, 2271-2285
- **A. V. Vorogushin et al.** *J. Am. Chem. Soc.,* 2005, vol. 127, 8146-8149
- **F. Y. Kwong ; St. L. Buchwald.** *Org. Lett.,* 2002, vol. 4, 3517-3520
- **S. M. Nobre ; A. L. Monteiro.** *Tetrahedron Letters,* 2004, vol. 45, 8225-8228
- **S. Langle et al.** *Tetrahedron Letters,* 2003, vol. 44, 9255-9258
- **G. Dewynter et al.** *C. R. Acad. Sci. Paris, Ser. II,* 1992, vol. 315, 1675-1682
- **Cheng, Y.-C. ; Prusoff, W.H.** *Biochem. Pharmacol,* 1973, vol. 22, 3099-3108